(19)

Europäisches
Patentamt
European
Patent Office
Office européen
des brevets

(11)  **EP 4 295 840 A2**

(12)                          **EUROPEAN PATENT APPLICATION**

(43) Date of publication:
     **27.12.2023   Bulletin 2023/52**

(51) International Patent Classification (IPC):
     **_A61K 9/48_** _(2006.01)_

(21) Application number: **23203796.0**

(22) Date of filing: **06.12.2017**

(52) Cooperative Patent Classification (CPC):
     **A61K 31/716; A61P 1/00; A61P 1/16; A61P 9/00;**
     **A61P 13/12; A61P 43/00;** A61K 9/0031;
     A61K 9/0095; A61K 9/2086; A61K 9/4891

(84) Designated Contracting States:
     **AL AT BE BG CH CY CZ DE DK EE ES FI FR GB**
     **GR HR HU IE IS IT LI LT LU LV MC MK MT NL NO**
     **PL PT RO RS SE SI SK SM TR**

(30) Priority: **06.12.2016   US 201662430895 P**
               **06.12.2016   US 201662430849 P**
               **13.01.2017   US 201762446316 P**

(62) Document number(s) of the earlier application(s) in
     accordance with Art. 76 EPC:
     **17832599.9 / 3 551 194**

(71) Applicant: **DSM Nutritional Products, LLC**
     **Parsippany, NJ 07054 (US)**

(72) Inventors:
     • **GIBSON, Molly Krisann**
       **Cambridge, 02142 (US)**

     • **LIU, Christopher Matthew**
       **Lexington, 02421 (US)**
     • **VON MALTZAHN, Geoffrey A.**
       **Cambridge, 02142 (US)**
     • **YUAN, Han**
       **Arlington, 02474 (US)**

(74) Representative: **Maiwald GmbH**
     **Elisenhof**
     **Elisenstraße 3**
     **80335 München (DE)**

Remarks:
•The complete document including Reference
Table(s) and the Sequence Listing(s) can be
downloaded from the EPO website
•This application was filed on 16-10-2023 as a
divisional application to the application mentioned
under INID code 62.

(54)     **GLYCAN POLYMERS AND RELATED METHODS THEREOF**

(57)     Compositions of glycan polymers and methods of making and manufacturing the same are described herein.
Also provided are methods of treating a disease or disorder with a glycan polymer preparation.

**EP 4 295 840 A2**

**Description**

**RELATED APPLICATIONS**

This application claims priority to U.S. Serial No.: 62/430895 filed December 6, 2016, U.S. Serial No.: 62/446316 filed January 13, 2017, and U.S. Serial No.: 62/430849 filed December 6, 2016, the entire contents of which are incorporated herein by reference.

**BACKGROUND**

The microbiota of humans is complex, and varies by individual depending on genetics, age, sex, stress, nutrition and diet. The microbiota performs many activities and may influence the physiology of the host. Modulating the gut microbiota can alter community function and interaction with the host. A limited number of probiotic bacteria are known in the art, and some association with health benefits are documented when the probiotic bacteria are taken by humans. Some foods are considered 'prebiotic' foods that contain substances that may promote the growth of certain bacteria that are thought to be beneficial to the human host. The results of clinical tests with these substances are conflicted with respect to their efficacy, and their influence on human health is generally described as being modest. Thus, there is a need for novel inputs that can modulate the microbiota and improve human health.

**SUMMARY OF THE INVENTION**

Described herein are methods of treating a subject having a disease or disorder with a glycan polymer preparation, and compositions thereof.

Accordingly, in one aspect, the invention is directed to a method of treating a subject having a disease or disorder associated with an unwanted level of a metabolite (e.g., a short chain fatty acid (SCFA) (e.g., propionate or butyrate), ammonia, trimethylamine (TMA), trimethylamine N-oxide (TMAO), a uremic solute (e.g., p-cresol or indole), lipopolysaccharide (LPS), or a bile acid (e.g., a secondary bile acid)), comprising:

optionally, selecting a glycan polymer preparation on the basis that it modulates the production or level of the metabolite, and
administering an amount of the glycan polymer preparation effective to result in a modulation of the level of the metabolite, thereby treating the disease or disorder.

In another aspect, the invention is directed to method of treating a subject having a disease or disorder associated with an unwanted level of a metabolite (e.g., a short chain fatty acid (SCFA) (e.g., propionate or butyrate), ammonia, trimethylamine (TMA), trimethylamine N-oxide (TMAO), a uremic solute (e.g., p-cresol or indole), lipopolysaccharide (LPS), or a bile acid (e.g., a secondary bile acid)), comprising:

optionally, acquiring knowledge that a glycan polymer preparation modulates the production or level of the metabolite, and
administering an amount of the glycan polymer preparation effective to result in a modulation of the level of the metabolite, thereby treating the disease or disorder.

In another aspect, the invention is directed to a method of modulating the production or level of a product (e.g., a short chain fatty acid (SCFA), ammonia, trimethylamine (TMA), trimethylamine N-oxide (TMAO), a uremic solute, or a bile acid) in the body (e.g., the gut (colon, intestine), blood, urine, an organ (e.g. liver, kidney), the brain) of a subject comprising: administering (e.g. orally or rectally) an effective amount of a glycan polymer preparation to the subject sufficient to modulate the production or level of a product, optionally, wherein the glycan polymer is a substrate for a microbial constituent of the colon or intestine.

In another aspect, the invention is directed to a method of selecting a glycan polymer preparation for use as a substrate for a glycosidase enzyme (e.g. CAZy family) of a preselected human gut microbe (e.g. selected because of its glycosidase profile), comprising:

a) acquiring a value for the glycosidase (e.g. CAZy family) profile of a microbe,
b) identifying, designing, or selecting a glycan polymer capable of being a substrate of the microbe on the basis of the glycosidase (e.g. CAZy family) profile,
c) optionally,

i. assembling a panel of human gut microbes (e.g. single strains, designed communities of strains, or ex vivo communities, e.g. from fecal samples, which include the microbe of interest)
ii. contacting the panel of microbes with a test glycan preparation,
iii. assessing the growth of the human gut microbe (of interest)

d) selecting the glycan polymer preparation.

In another aspect, the invention is directed to a glycan preparation made or selected by a method described herein.

In another aspect, the invention is directed to a glycan polymer preparation comprising glycan polymers, e.g., wherein the preparation comprises at least .5, 1, 2, 5, 10, 50, or 100 kg, and, e.g., is at least 20, 30, 40, 50, 60, 70, 80, 90, 95 or 99 % pure, comprising:

i) a glucose, mannose, or galactose subunit, or a combination thereof and at least one alpha-glycosidic bond, or
ii) a glucose, mannose, or galactose subunit, or a combination thereof and at least one beta-glycosidic bond, and

which are a substrate of one or more, e.g., two, three, four, or more, human gut microbe glycosidase enzymes selected from:

i) GT5, GH94, GH13 subfamily 9, GH13 subfamily 39, GH13 subfamily 36, GH113 or GH112 CAZy family,
ii) GT2, GT4, GT5, GT35, GT51, GH1, GH2, GH3, GH4, GH13, GH13 subfamily 9, GH13 subfamily 31, GH18, GH23, GH25, GH28, GH31, GH32, GH36, GH51, GH73, GH77, or GH94 CAZy family,
iii) GT11, GT10, GH92, GH51, GH35, GH29, GH28, GH20, GH130, GH13 subfamily 8, or GH13 subfamily 14 CAZy family, or
iv) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, or GH77 CAZy family.

In another aspect, the invention is directed to a glycan polymer preparation, e.g., wherein the preparation comprises at least about 0.5, 1, 2, 5, 10, 50, or 100 kg, and, e.g., is at least 20, 30, 40, 50, 60, 70, 80, 90, 95 or 99 % pure, comprising glycan polymers comprising:

i) a xylose, arabinose, fucose or rhamnose subunit, or a combination thereof and at least one alpha-glycosidic bond, or
ii) a xylose, arabinose, fucose or rhamnose subunit, or a combination thereof and at least one beta-glycosidic bond, and

which are a substrate of one or more, e.g., two, three, four, or more, human gut microbe glycosidase enzymes selected from:

i) GT11, GT10, GH92, GH51, GH35, GH29, GH28, GH20, GH130, GH13 subfamily 8, or GH13 subfamily 14 CAZy family, or
ii) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, or GH77 CAZy family.

In another aspect, the invention is directed to a glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

i) a glucose or galactose subunit, or a combination thereof and at least one alpha-glycosidic bond, or
ii) a glucose or galactose subunit, or a combination thereof and at least one beta-glycosidic bond, and

which are a substrate of one or more, e.g., two, three, four, or more, human gut microbe glycosidase enzymes selected from:

i) GT3, GH97, GH43 subfamily 24, GH27, GH133, GH13 subfamily 8, GH13 CAZy family, or
ii) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, GH24 CAZy family.

In another aspect, the invention is directed to a glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

an arabinose, galactose, xylose, or glucose subunit, or a combination thereof and at least one alpha-glycosidic bond, and
which are a substrate of one or more, e.g., two, three, four, or more, human gut microbe glycosidase enzymes selected from:

i) GH13 subfamily 3, GH13 subfamily 30, GH30 subfamily 2, GH30 subfamily 5, GH43 subfamily 22, GH43 subfamily 8, or GH84 CAZy family, or
ii) GH3, GH106, GH105, GH2, GH20, GH28, GH76, GH97, or GH92 CAZy family.

In another aspect, the invention is directed to a glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

a glucose and at least one alpha-glycosidic bond, and
which are a substrate of one or more, e.g., two, three, four, or more, human gut microbe glycosidase enzymes selected from:

i) GH13 subfamily 19, GH13 subfamily 21, GH23, GH33, GH37 or GH104 CAZy family, or
ii) GH23, GH24, or GH33 CAZy family.

In another aspect, the invention is directed to a glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

i) a glucose or xylose subunit, or a combination thereof and at least one alpha-glycosidic bond, or
ii) a glucose or xylose subunit, or a combination thereof and at least one beta-glycosidic bond, and

which are a substrate of one or more, e.g., two, three, four, or more, human gut microbe glycosidase enzymes selected from:

i) GH13 subfamily 20, GH13 subfamily 31, GH13 subfamily 39, GH39, GH43 subfamily 11, GH5 subfamily 44, or GH94 CAZy family, or
ii) GH2, GH31, GH23, GH13, or GH24 CAZy family.

In another aspect, the invention is directed to a glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

a glucose, xylose, arabinose, or galactose subunit, or a combination thereof and at least one alpha-glycosidic bond, and
which are a substrate of one or more, e.g., two, three, four, or more, human gut microbe glycosidase enzymes selected from:

i) GH13 subfamily 3, GH13 subfamily 30, GH121, GH15, GH43 subfamily 27, GH43 subfamily 34, or GH43 subfamily 8 CAZy family, or
ii) GH92, GH97, GH76, GH28, GH20, GH105, GH2, GH50, GH3, or GH106 CAZy family.

In another aspect, the invention is directed to a unit dosage from comprising a glycan preparation described herein.
In another aspect, the invention is directed to a pharmaceutical composition comprising a glycan preparation described herein.

4

In another aspect, the invention is directed to a set of pharmaceutical compositions, each comprising a glycan polymer preparation, or a portion thereof, described herein, wherein collectively, the set comprises at least 0.1, 0.5, 1, 2, 5, 10, or 100 kilograms of the preparation.

In another aspect, the invention is directed to a medical food comprising a glycan preparation described herein.

In another aspect, the invention is directed to a set of medical food portions, each comprising a glycan polymer preparation, or a portion thereof, described herein, wherein collectively, the set comprises at least 0.1, 0.5, 1, 2, 5, 10, or 100 kilograms of the preparation

In another aspect, the invention is directed to a dietary supplement comprising a glycan preparation described herein.

In another aspect, the invention is directed to a set of dietary supplement portions, each comprising a glycan polymer preparation, or a portion thereof, described herein, wherein collectively, the set comprises at least 0.1, 0.5, 1, 2, 5, 10, or 100 kilograms of the preparation.

In another aspect, the invention is directed to a food ingredient comprising a glycan preparation described herein.

In another aspect, the invention is directed to a set of food ingredient portions, each comprising a glycan polymer preparation, or a portion thereof, described herein, wherein collectively, the set comprises at least 0.1, 0.5, 1, 2, 5, 10, or 100 kilograms of the preparation.

In another aspect, the invention is directed to a method of making a co-preparation comprising:

> providing a preparation of a human gut microbe,
> providing a glycan polymer preparation described herein,

wherein the glycan polymer is a substrate of the human gut microbe, and
combining the human gut microbe comprising with the glycan polymer.

In another aspect, the invention is directed to a synbiotic co-preparation comprising a preparation of a human gut microbe and a preparation of a glycan polymer described herein.

In another aspect, the invention is directed to a method of engrafting a human gut microbe in the colon or large intestine of a human subject in need thereof, comprising: administering a synbiotic co-preparation described herein to the subject in an amount and for a time effective to engraft the human gut microbe.

In another aspect, the invention is directed to a method of treating a subject having a dysbiosis, comprising: administering a composition comprising a glycan polymer preparation described herein and a preparation of a microbe in an amount effective to treat the dysbiosis.

In another aspect, the invention is directed to a glycan polymer preparation described herein comprising glycan polymers which are a substrate of a human gut microbe glycosidase enzyme of a spore-forming microbe (e.g. spore-forming bacterial taxa).

In another aspect, the invention is directed to a glycan polymer preparation, optionally, e.g., wherein the preparation comprises at least about 0.5, 1, 2, 5, 10, 50, or 100 kg, and/or, further optionally, e.g., is at least 20, 30, 40, 50, 60, 70, 80, 90, 95 or 99 % pure, comprising glycan polymers comprising:

> a. a xylose or arabinose subunit, or a combination thereof and at least one alpha-glycosidic bond,
> b. a xylose or arabinose subunit, or a combination thereof and at least one beta-glycosidic bond,
> c. a galactose, xylose, or arabinose subunit, or a combination thereof and at least one alpha-glycosidic bond,
> d. a galactose, xylose, or arabinose subunit, or a combination thereof and at least one beta-glycosidic bond,
> e. a glucose, xylose, or arabinose subunit, or a combination thereof and at least one alpha-glycosidic bond,
> f. a glucose, xylose, or arabinose subunit, or a combination thereof and at least one beta-glycosidic bond,
> g. a xylose, arabinose, glucose or galactose subunit, or a combination thereof and at least one alpha-glycosidic bond,
> h. a xylose, arabinose, glucose or galactose subunit, or a combination thereof and at least one beta-glycosidic bond,
> or a combination thereof and at least one beta-glycosidic bond, and

which are a substrate of a human gut microbe glycosidase enzyme of one of: GT5, GT35, GT3, GH97, GH95, GH92, GH89, GH88, GH78, GH77, GH57, GH51, GH43 subfamily 34, GH43 subfamily 24, GH43 subfamily 10, GH42, GH36, GH35, GH33, GH32, GH31, GH3, GH29, GH28, GH27, GH24, GH20, GH2, GH16, GH133, GH130, GH13 subfamily 8, GH13 subfamily 38, GH13 subfamily 14, GH13, GH123, GH115, GH109, or GH105 CAZy family.

In another aspect, the invention is directed to a method of making a co-preparation comprising:

> providing a preparation of a spore-forming microbe (e.g. a spore-forming human gut microbe),
> providing the glycan polymer preparation (described herein),

wherein the glycan polymer is a substrate of the spore-forming microbe, and

combining the preparation of the spore-forming microbe with the glycan polymer preparation.

In another aspect, the invention is directed to a method of making a preparation of a glycan polymer, e.g., a glycan polymer that is a substrate for a glycosidase enzyme present in a human gut microbe, comprising:

providing a plurality of glycan subunits, e.g., a sugar monomer or a sugar dimer, suitable for the production of the glycan polymer; and

contacting the glycan subunits of the plurality with a glycosidase enzyme molecule, e.g. derived from a human gut microbe, under conditions that result in the incorporation, e.g., by a condensation reaction, of the glycan subunits into a glycan polymer,

thereby making a glycan polymer preparation that is a substrate for a human gut microbe, optionally wherein:

i) the glycan polymer preparation comprises at least about 0.25, 0.5, 1, 5, 10, 20, 50, 100, 200, 300, 400 or 500 kilograms of glycan polymer, and/or
ii) the glycan polymer preparation is produced at a yield of at least about 15%, 30%, 45%, 60%, or of about 75% (as determined on a weight/weight basis as a percentage of input glycan subunits).

In another aspect, the invention is directed to a method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT5, GH94, GH13 subfamily 9, GH13 subfamily 39, GH13 subfamily 36, GH113 or GH112 CAZy family;

under conditions that result in making a glycan polymer preparation, wherein a glycan polymer of the preparation is a substrate for a human gut microbe comprising a glycosidase enzyme of a GT5, GH94, GH13 subfamily 9, GH13 subfamily 39, GH13 subfamily 36, GH113 or GH112 CAZy family.

In another aspect, the invention is directed to a method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT2, GT4, GT5, GT35, GT51, GH1, GH2, GH3, GH4, GH13.0, GH13.9, GH13.31, GH18, GH23, GH25, GH28, GH31, GH32, GH36, GH51, GH73, GH77, or GH94 CAZy family,

under conditions that result in making a glycan polymer preparation, wherein a glycan polymer of the preparation is a substrate for a human gut microbe comprising a glycosidase enzyme of a GT2, GT4, GT5, GT35, GT51, GH1, GH2, GH3, GH4, GH13.0, GH13.9, GH13.31, GH18, GH23, GH25, GH28, GH31, GH32, GH36, GH51, GH73, GH77, GH94 CAZy family.

In another aspect, the invention is directed to a method of making a glycan polymer preparation, comprising:

providing a plurality of xylose, arabinose, galactose and/or glucose containing glycan subunits (e.g., monomers or dimers);

contacting the plurality of glycan subunits with a glycosidase enzyme selected from one of GH13 subfamily 3, GH13 subfamily 30, GH30 subfamily 2, GH30 subfamily 5, GH43 subfamily 22, GH43 subfamily 8, or GH84 CAZy family,

under conditions that result in making a glycan polymer preparation, wherein a glycan polymer of the preparation is a substrate for a human gut microbe comprising a glycosidase enzyme of a GH13 subfamily 3, GH13 subfamily 30, GH30 subfamily 2, GH30 subfamily 5, GH43 subfamily 22, GH43 subfamily 8, or GH84 CAZy family.

In another aspect, the invention is directed to a method of making a glycan polymer preparation, comprising:

providing a plurality of xylose, arabinose, galactose and/or glucose containing glycan subunits (e.g., monomers or dimers);

contacting the plurality of glycan subunits with a glycosidase enzyme selected from one of GH3, GH106, GH105, GH2, GH20, GH28, GH76, GH97, or GH92 CAZy family,

under conditions that result in making a glycan polymer preparation, wherein a glycan polymer of the preparation is a substrate for a human gut microbe comprising a glycosidase enzyme of a GH3, GH106, GH105, GH2, GH20, GH28, GH76, GH97, or GH92 CAZy family.

In another aspect, the invention is directed to a method of making a glycan polymer preparation, comprising:

providing a plurality of glucose and/or sialic acid containing glycan subunits (e.g., monomers or dimers);

contacting the plurality of glycan subunits with a glycosidase enzyme selected from one of GH13 subfamily 19, GH13 subfamily 21, GH23, GH33, GH37 or GH104 CAZy family,

under conditions that result in making a glycan polymer preparation, wherein a glycan polymer of the preparation is a substrate for a human gut microbe comprising a glycosidase enzyme of a GH13 subfamily 19, GH13 subfamily 21, GH23, GH33, GH37 or GH104 CAZy family.

In another aspect, the invention is directed to a method of making a glycan polymer preparation, comprising:

providing a plurality of glucose and/or sialic acid containing glycan subunits (e.g., monomers or dimers);

contacting the plurality of glycan subunits with a glycosidase enzyme selected from one of GH23, GH24, or GH33 CAZy family,

under conditions that result in making a glycan polymer preparation, wherein a glycan polymer of the preparation is a substrate for a human gut microbe comprising a glycosidase enzyme of a GH23, GH24, or GH33 CAZy family.

In another aspect, the invention is directed to a method of making a glycan polymer preparation, comprising:

providing a plurality of glucose, xylose, mannose, arabinose, and/or galactose containing glycan subunits (e.g., monomers or dimers);

contacting the plurality of glycan subunits with a glycosidase enzyme selected from one of GH13 subfamily 20, GH13 subfamily 31, GH13 subfamily 39, GH39, GH43 subfamily 11, GH5 subfamily 44, or GH94 CAZy family,

under conditions that result in making a glycan polymer preparation, wherein a glycan polymer of the preparation is a substrate for a human gut microbe comprising a glycosidase enzyme of a GH13 subfamily 20, GH13 subfamily 31, GH13 subfamily 39, GH39, GH43 subfamily 11, GH5 subfamily 44, or GH94 CAZy family.

In another aspect, the invention is directed to a method of making a glycan polymer preparation, comprising:

providing a plurality of glucose, xylose, mannose, arabinose, and/or galactose containing glycan subunits (e.g., monomers or dimers);

contacting the plurality of glycan subunits with a glycosidase enzyme selected from one of GH2, GH31, GH23, GH13, or GH24 CAZy family,

under conditions that result in making a glycan polymer preparation, wherein a glycan polymer of the preparation is a substrate for a human gut microbe comprising a glycosidase enzyme of a GH2, GH31, GH23, GH13, or GH24 CAZy family.

In another aspect, the invention is directed to a method of making a glycan polymer preparation, comprising:

providing a plurality of glucose, xylose, arabinose, and/or galactose containing glycan subunits (e.g., monomers or dimers);

contacting the plurality of glycan subunits with a glycosidase enzyme selected from one of GH13 subfamily 3, GH13 subfamily 30, GH121, GH15, GH43 subfamily 27, GH43 subfamily 34, or GH43 subfamily 8 CAZy family,

under conditions that result in making a glycan polymer preparation, wherein a glycan polymer of the preparation is a substrate for a human gut microbe comprising a glycosidase enzyme of a GH13 subfamily 3, GH13 subfamily 30, GH121, GH15, GH43 subfamily 27, GH43 subfamily 34, or GH43 subfamily 8 CAZy family.

In another aspect, the invention is directed to a method of making a glycan polymer preparation, comprising:

providing a plurality of glucose, xylose, arabinose, and/or galactose containing glycan subunits (e.g., monomers or dimers);

contacting the plurality of glycan subunits with a glycosidase enzyme selected from one of GH92, GH97, GH76, GH28, GH20, GH105, GH2, GH50, GH3, or GH106 CAZy family,

under conditions that result in making a glycan polymer preparation, wherein a glycan polymer of the preparation is a substrate for a human gut microbe comprising a glycosidase enzyme of a GH92, GH97, GH76, GH28, GH20, GH105, GH2, GH50, GH3, or GH106 CAZy family.

In another aspect, the invention is directed to a method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT11, GT10, GH92, GH51, GH35, GH29, GH28, GH20, GH130, GH13 subfamily 8, GH13 subfamily 14 CAZy family

under conditions that result in making a glycan polymer preparation, wherein a glycan polymer of the preparation is a substrate for a human gut microbe comprising a glycosidase enzyme of a GT11, GT10, GH92, GH51, GH35, GH29, GH28, GH20, GH130, GH13 subfamily 8, GH13 subfamily 14 CAZy family.

In another aspect, the invention is directed to a method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family,

under conditions that result in making a glycan polymer preparation, wherein a glycan polymer of the preparation is a substrate for a human gut microbe comprising a glycosidase enzyme of a GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family.

In another aspect, the invention is directed to a method of making a glycan polymer preparation, comprising:

providing a plurality of xylose, arabinose, fucose and/or rhamnose containing glycan subunits (e.g., monomers or dimers);

contacting the plurality of glycan subunits with a glycosidase enzyme selected from one of GT11, GT10, GH92, GH51, GH35, GH29, GH28, GH20, GH130, GH13 subfamily 8, GH13 subfamily 14 CAZy family

under conditions that result in making a glycan polymer preparation, wherein a glycan polymer of the preparation is a substrate for a human gut microbe comprising a glycosidase enzyme of a GT11, GT10, GH92, GH51, GH35, GH29, GH28, GH20, GH130, GH13 subfamily 8, GH13 subfamily 14 CAZy family.

In another aspect, the invention is directed to a method of making a glycan polymer preparation, comprising:

providing a plurality of glycan subunits of a substrate of column E of Table 23, e.g., monomers or dimers;
contacting the plurality of glycan subunits of a substrate with a glycosidase enzyme of column A of the same row as the substrate;
under conditions that result in making a glycan polymer preparation, e.g., conditions of columns F, G, H, I, J, K, and/or L of the same row as the substrate and glycosidase enzyme.

In another aspect, the invention is directed to method of making a glycan polymer preparation, comprising:

providing a plurality of xylose, arabinose, fucose and/or rhamnose containing glycan subunits (e.g., monomers or dimers);

contacting the plurality of glycan subunits with a glycosidase enzyme selected from one of GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family,

under conditions that result in making a glycan polymer preparation, wherein a glycan polymer of the preparation is a substrate for a human gut microbe comprising a glycosidase enzyme of a GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family.

In another aspect, the invention is directed to a method of making a glycan polymer preparation, comprising:

providing a plurality of glucose and/or galactose containing glycan subunits (e.g., monomers or dimers);

contacting the plurality of glycan subunits with a glycosidase enzyme selected from one of GT3, GH97, GH43 subfamily 24, GH27, GH133, GH13 subfamily 8, GH13 CAZy family,

under conditions that result in making a glycan polymer preparation, wherein a glycan polymer of the preparation is a substrate for a human gut microbe comprising a glycosidase enzyme of a GT3, GH97, GH43 subfamily 24, GH27, GH133, GH13 subfamily 8, GH13 CAZy family.

In another aspect, the invention is directed to a method of making a glycan polymer preparation, comprising:

providing a plurality of glucose and/or galactose containing glycan subunits (e.g., monomers or dimers);

contacting the plurality of glycan subunits with a glycosidase enzyme selected from one of GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, GH24 CAZy family,

under conditions that result in making a glycan polymer preparation, wherein a glycan polymer of the preparation is a substrate for a human gut microbe comprising a glycosidase enzyme of a GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, GH24 CAZy family.

In another aspect, the invention is directed to a glycan polymer preparation made by, producible by, or makeable by, a method disclosed herein, e.g., by a method described herein.
In another aspect, the invention is directed to glycan polymer preparation selected by, or selectable by, a method disclosed herein, e.g., by a method described herein.
In another aspect, the invention is directed to a therapeutic nutrition product comprising a glycan polymer preparation described herein.
In another aspect, the invention is directed to a reaction mixture, described herein, e.g., generated by any one of the methods described herein, comprising:

a plurality of glycan subunits, e.g., a sugar monomer or a sugar dimer, suitable for the production of the glycan polymer; and

a glycosidase enzyme molecule (e.g., Tables 4 (column 2), 23 (column A), 24 (column A), or 22 (column 1); or one or more glycosidase enzymes associated with glycotaxa class 1, class 2, class3, class 4, class 5, class 6, or class 7),

in amounts suitable to produce a glycan polymer preparation comprising at least 0.25, 0.5, 1, 5, 10, 20, 50, 100, 200, 300, 400 or 500 kilograms of glycan polymer and/or under conditions suitable to obtain a yield of at least about 15%, 30%, 45%, 60%, or of about 75% (as determined on a weight/weight basis as a % of input glycan subunits).

In another aspect, the invention is directed to a method of making a pharmaceutical composition, a medical food, a dietary supplement, a food ingredient, or a therapeutic nutrition product, comprising formulating a preparation described herein into a pharmaceutical composition, a medical food, a dietary supplement, a food ingredient, or a therapeutic nutrition product.

In another aspect, the invention is directed to a fraction, e.g., a molecular weight fraction, of a glycan polymer preparation described herein.

In another aspect, the invention is directed to a method of making, evaluating, selecting, classifying, or providing a preparation of a glycan polymer made or makeable by a method described herein, comprising

acquiring a candidate preparation;

acquiring, e.g., by performing an assay, a value for a parameter related to the preparation, e.g., a physical parameter, e.g., molecular weight, e.g., average molecular weight or molecular weight distribution, glycan subunit composition, or purity or a parameter related to a biological property, e.g., the ability to modulate growth of the human gut microbe, the ability to modulate a microbial metabolite produced by a microbe, e.g., in an ex vivo assay, or the ability to modulate a biomarker, e.g., an inflammatory or immune biomarker, a toxic or waste compound, a bacterial compound) e.g., in a human subject; and

comparing the value with a reference value;

thereby making, evaluating, selecting, classifying, or providing a preparation of a glycan polymer.

In another aspect, the invention is directed to a method of making a pharmaceutical composition that modulates a target human gut microbe, comprising

providing a plurality of glycan subunits;
contacting the glycan subunits of the plurality with a glycosidase enzyme composition having a glycosidase activity present in the target gut microbe, under conditions that result in the incorporation of the glycan subunits into a glycan polymer,
optionally purifying the glycan polymer, and
formulating the glycan polymer as a pharmaceutical composition for administration to the gut and modulation of the gut microbe, thereby making a pharmaceutical composition that modulates the target human gut microbe.

In another aspect, the invention is directed to a purified preparation of glycosidase enzyme molecules comprising a glycosidase enzyme encoded by a nucleic acid sequence that is at least 80, 85, 90, 91, 92, 93, 94, 95, 96, 97, 98, 99, or 100% identical to a nucleic acid sequence selected from one or more of SEQ ID NOs: 1-124, wherein the glycosidase enzyme is present in a human gut microbe.

In another aspect, the invention is directed to a vector comprising a nucleic acid sequence that is at least 80, 85, 90, 91, 92, 93, 94, 95, 96, 97, 98, 99, or 100% identical to a nucleic acid sequence selected from one or more of SEQ ID NOs: 1-124, wherein the nucleic acid encodes a glycosidase enzyme present in a human gut microbe, and wherein the vector is capable of being used to express the glycosidase enzyme.

In another aspect, the invention is directed to a reaction mixture comprising:

a glycosidase enzyme encoded by a nucleic acid sequence selected from one or more of SEQ ID NOs: 1-124, and
a substrate, e.g., glycan subunits, e.g., monomers or dimers, of the glycosidase enzyme,
wherein the substrate is present in a sufficient amount to form, e.g., by condensation, a glycan polymer.

## BRIEF DESCRIPTION OF THE DRAWINGS

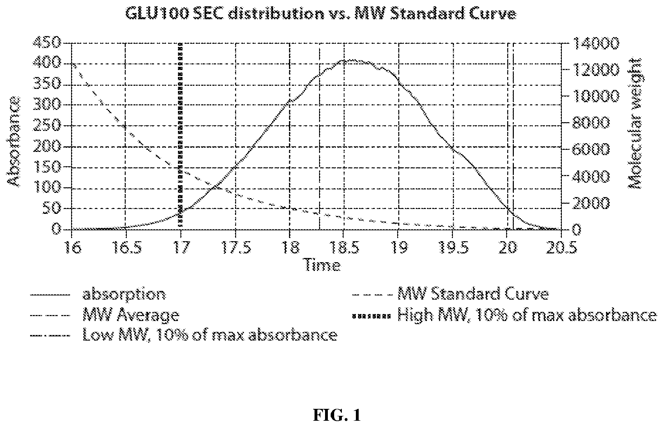

FIG. 1 is a representative SEC curve between 16 and 20.5 minutes of a glu100 sample showing the average MW and the MW at 10% of maximum absorption on both the leading and trailing edges of the curve.

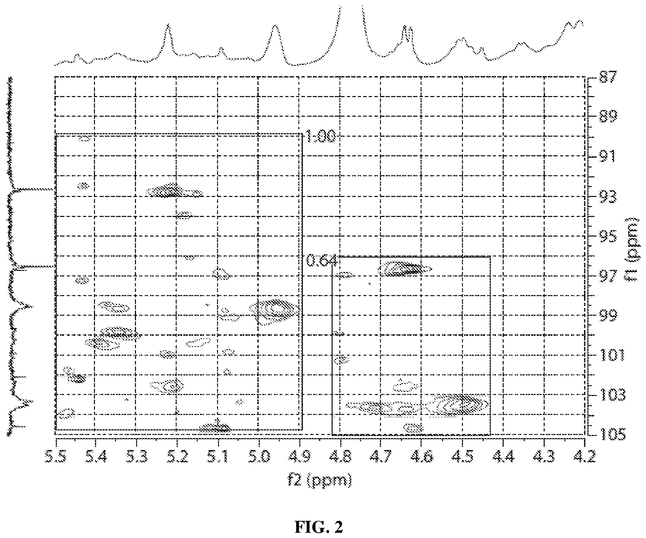

**FIG. 2** is a representative anomeric region of an $^1$H-$^{13}$C HSQC spectrum of a glu100 sample showing the signal distribution of alpha- and beta-glycosidic bonds

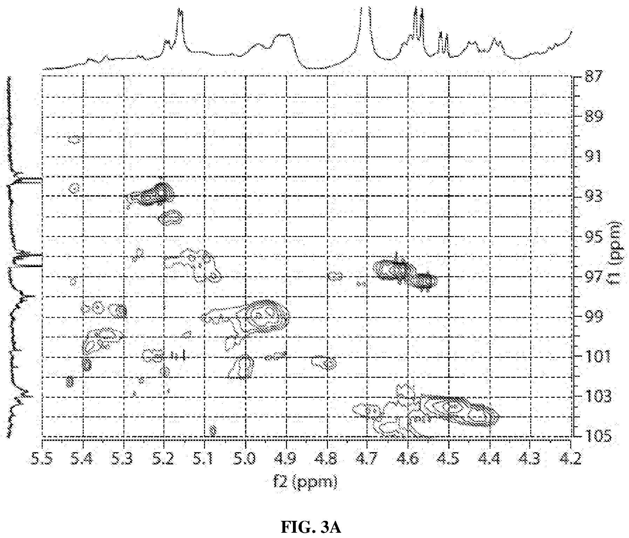

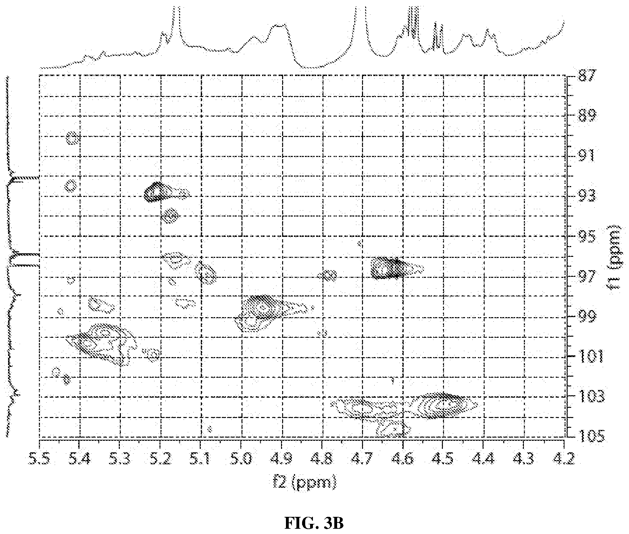

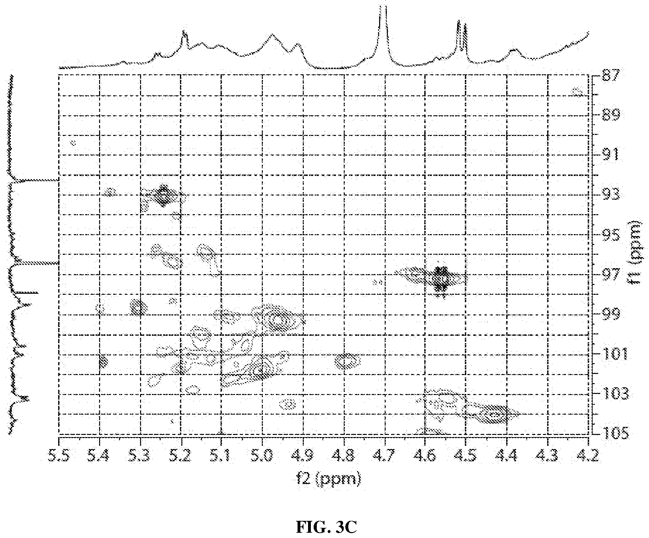

**FIGS. 3A-3C** are representative anomeric region of an $^1$H-$^{13}$C HSQC spectrum of glu50gal50 (FIG. 3A), glu100 (FIG. 3B), and gal100 (FIG. 3C) samples, demonstrating the additive effect of the fingerprint peaks.

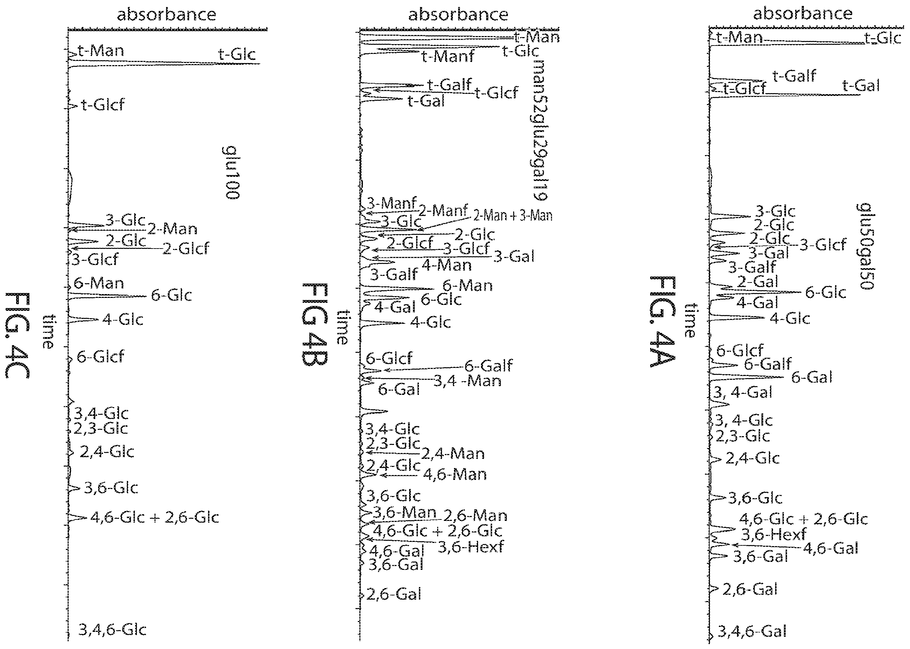

**FIGS. 4A-4C** are representative GC chromatograms of three representative permethylated and hydrolyzed glycans, glu50gal50 (FIG. 4A), man52glu29gal19 (FIG. 4B), and glu100 (FIG. 4C), showing distribution of regiochemistry as assigned by comparison to known standards.

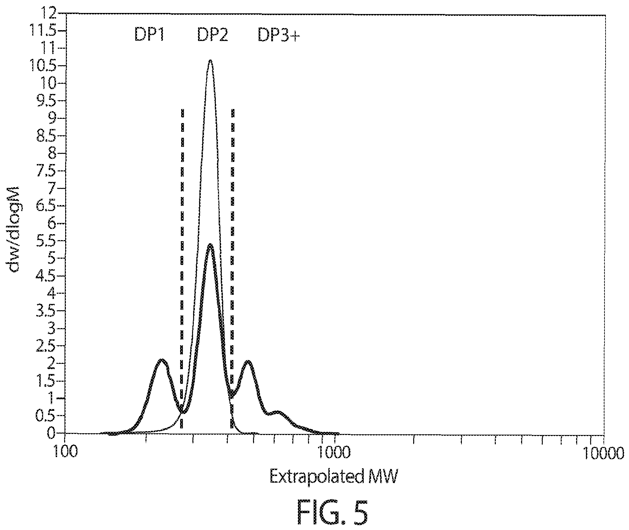

**FIG. 5** is a graph showing a processed SEC trace comparing lactose (gray, beta-galacto-1,4-glucose) to a glycan made by the treatment of lactose with beta-galactosidase as described in Example 2 (black).

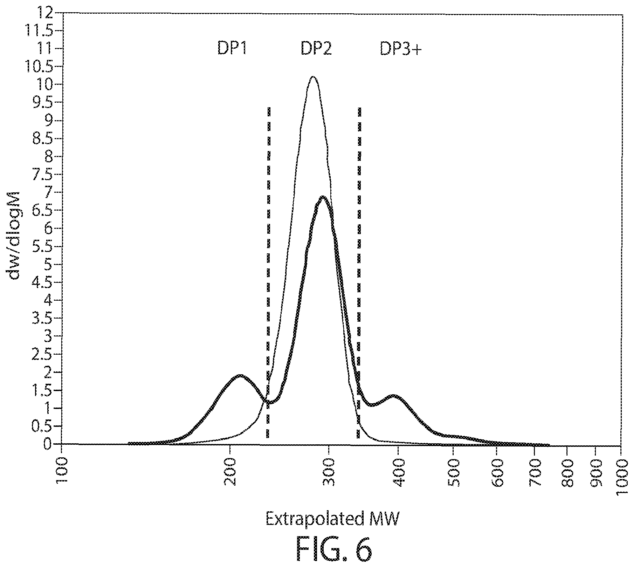

**FIG. 6** is a graph showing a processed SEC trace comparing cellobiose (gray, beta-gluco-1,4-glucose) to a glycan made by the treatment of cellobiose with beta-glucosidase as described in Example 4 (black). The shift in maximum peak intensity of DP2 materials is caused by the formation of allo-cellobioses (e.g. beta-gluco-1,6-glucose) which causes the average apparent Mw of DP2 materials to shift slightly.

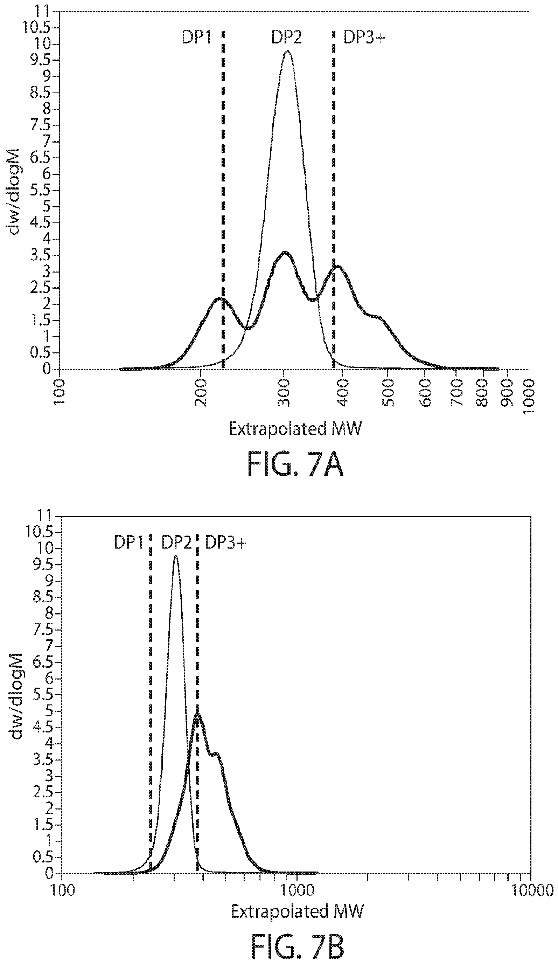

**FIGS. 7A-7B** are graphs showing processed SEC traces comparing (FIG. 7A) maltobiose (gray, alpha-gluco-1,4-glucose) to a glycan made by the treatment of maltobiose with alpha-glucosidase as described in Example 5 (black), and (FIG. 7B) maltobiose (gray) to a glycan from Example 18 purified by yeast fermentation as described in Example 9 (black). Although maltose is digestible by yeast, some DP2 materials remain due to trans-glycosylation in which maltose (alpha-gluco-1,4-glucose) is converted to allo-maltoses (e.g. alpha-gluco-1,6-glucose; alpha-gluco-1,3-glucose) which are less efficiently digested by yeast.

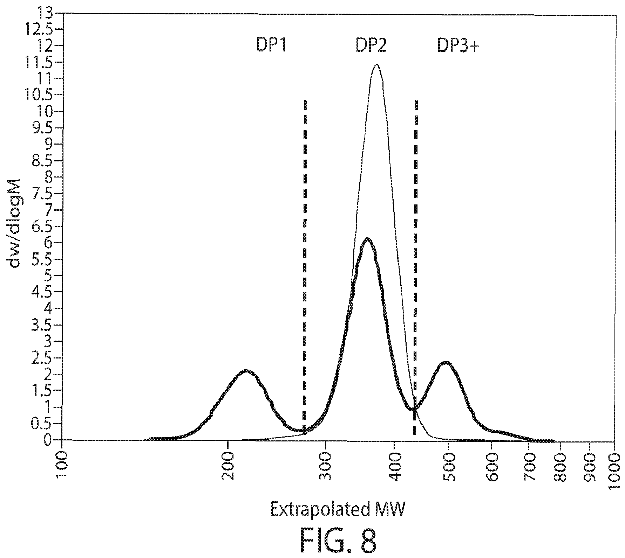

**FIG. 8** is a graph showing a processed SEC trace comparing melibiose (gray, alpha-galacto-1,6-glucose) to a glycan made by the treatment of melibiose with alpha-galactosidase as described in Example 3 (black). The shift in maximum peak intensity of DP2 materials is caused by the formation of allo-melibioses (e.g. alpha-galacto-1,4-glucose) which causes the average apparent Mw of DP2 materials to shift slightly.

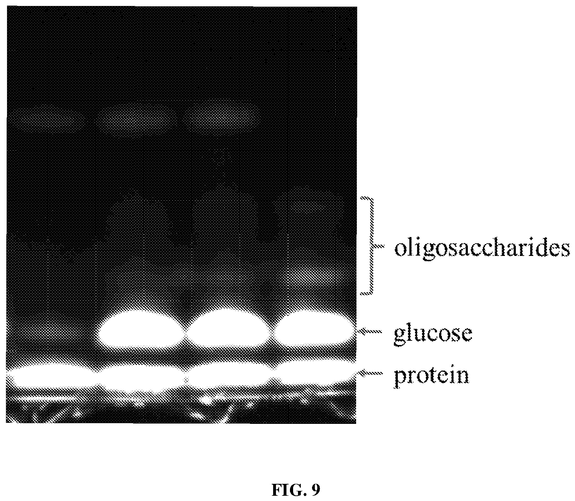

**FIG. 9** is an image showing the fluorophore-assisted carbohydrate electrophoresis (FACE) analysis of reaction mixture from reverse hydrolysis of glucose by beta-glucosidase. Lane 1 is pure protein, and lanes 2-4 are reactions in trimethyl phosphate, diethylene glycol dimethyl ether, and tetraethylene glycol dimethyl ether respectively as described in Example 7.

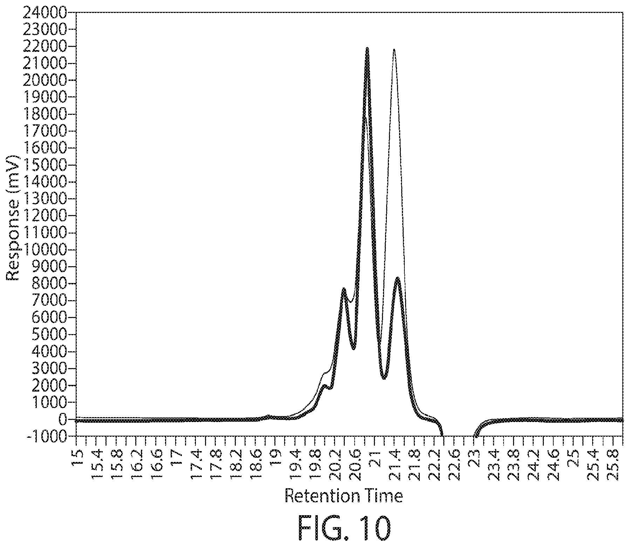

**FIG. 10** is a graph showing raw data SEC comparison of a glycan made by treating lactose with beta-galactosidase after 300 minutes with a glycan made by treating lactose with beta-galactosidase in the presence of d-galactose after 1200 minutes (i.e. at maximum conversion to DP≥3) as described in Example 8. The trace shows that the addition of D-galactose slows the reaction significantly but also shifts the product distribution towards increased amounts of DP≥3 oligosaccharides

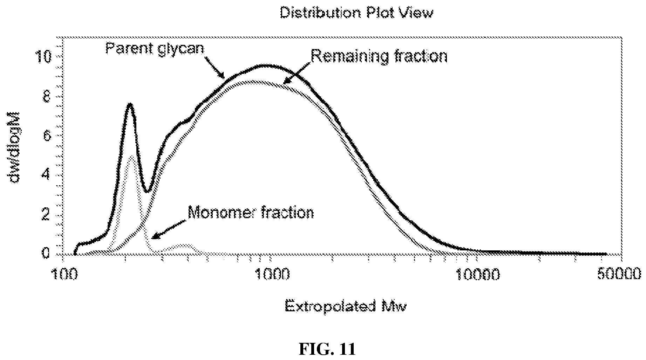

**FIG. 11** is a graph showing processed SEC data relating to the results of charcoal fractionation of a glycan with intent to remove monomer from a sample without further fractionation. The three curves represent parent glycan, the monomer fraction removed from the parent (apparent peak m.w. ~200) by 1% EtOH elution, and the remaining fraction isolated by a 50% EtOH elution.

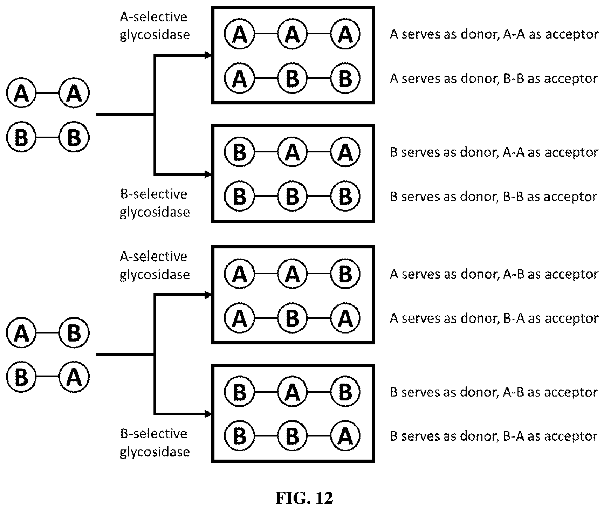

**FIG. 12** is a schematic representation of oligosaccharide synthesis via substrate-selective transglycosylation as described in Example 6. In each reaction, the enzyme selectivity for transglycosylation of the non-reducing end monomer leads to discrete mixtures of products. In this diagram, "A" and "B" could represent different monomers, different stereochemistries of glycosidic bond, different regiochemistries of glycosidic bond, or any combination thereof.

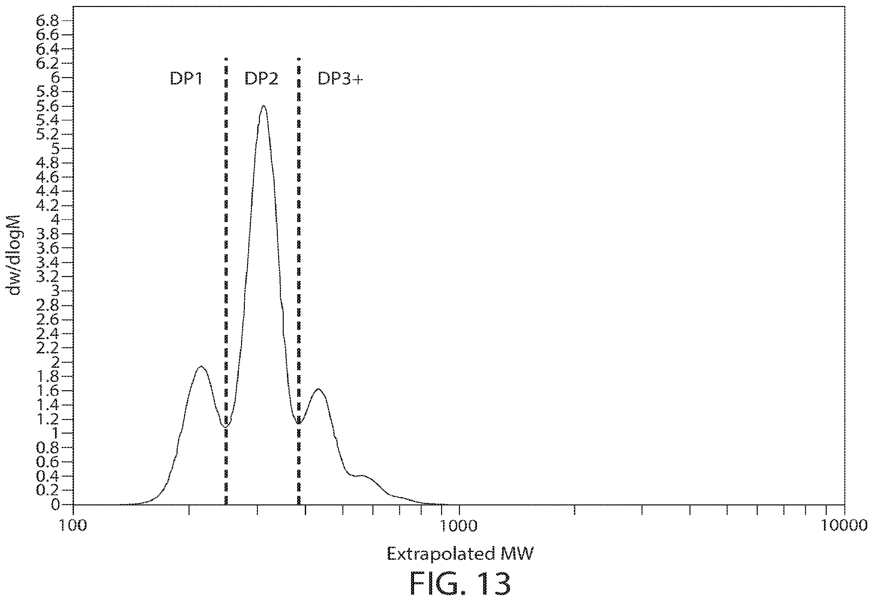

**FIG. 13** is a graph showing an SEC curve of a glycan made by treating lactose with beta-galactosidase after 300 minutes as described in Examples 11-18.

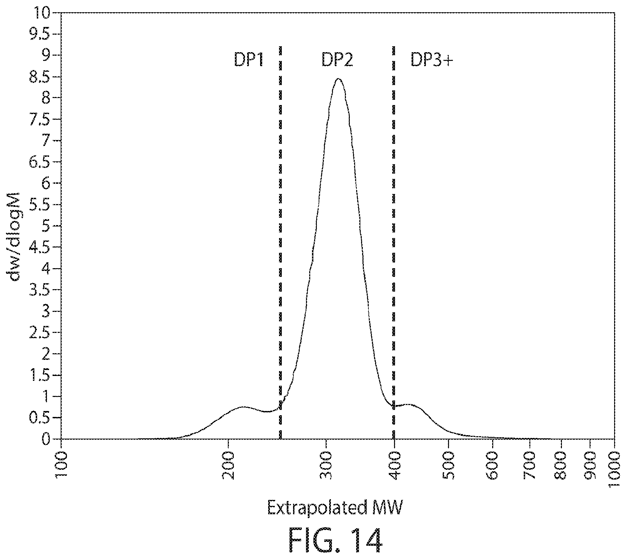

**FIG. 14** is a graph showing an SEC curve of a glycan made by treating lactose with beta-glucosidase after 300 minutes as described in Examples 11-18.

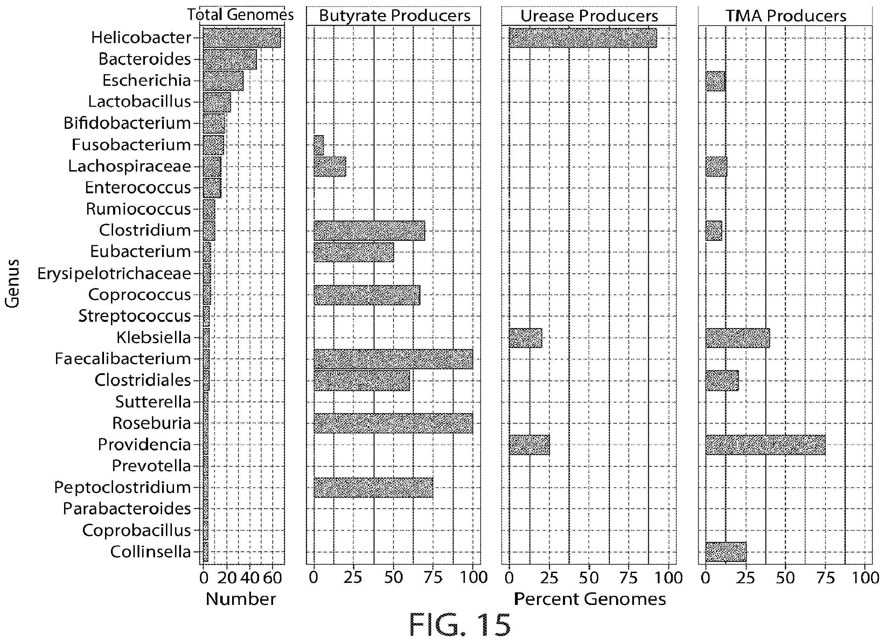

**FIG. 15** is a chart showing the total genomes annotated and used in genome analysis from the Human Microbiome Project and the percentage of genomes by genera that encode each of the indicated metabolites.

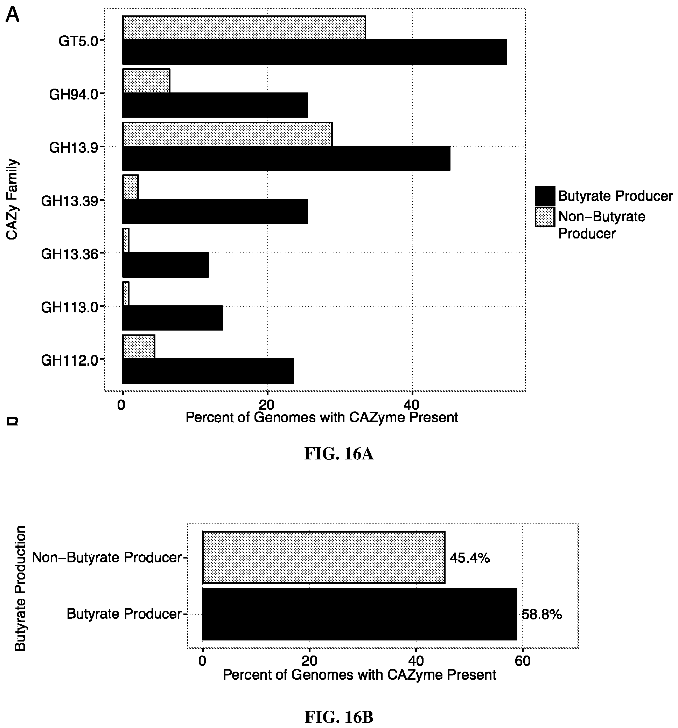

**FIGS. 16A-16B** are charts showing the percentage of genomes encoding CAZy families significantly enriched in butyrate producers (P<0.001, Wilcox Rank Sum, FDR corrected and identified in > 10% of butyrate producers). (FIG. 16A) Percentage of butyrate and non-butyrate producers that encode at least 1 enzyme from the indicated family. (FIG. 16B) Percentage of butyrate and non-butyrate producers that encode any CAZyme that is significantly enriched individually in butyrate producers.

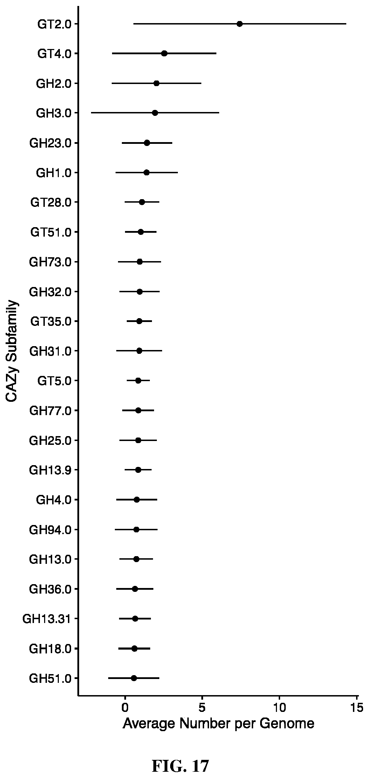

**FIG. 17** is a chart showing the most abundant families in butyrate producers, ordered by average gene count. Chart represents mean +/- s.d.

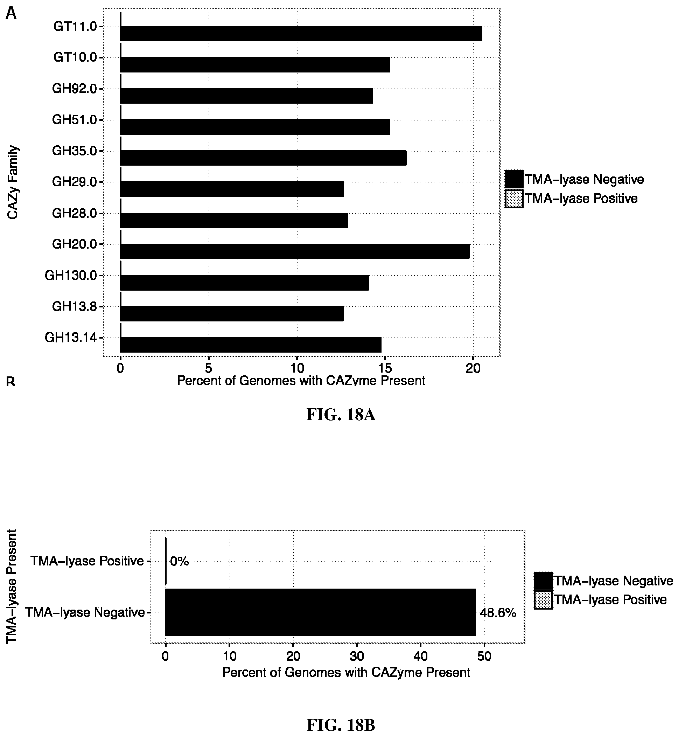

**FIGS. 18A-18B** are charts showing the percentage of genomes encoding CAZy families significantly depleted in TMA-lyase positive genomes (P<0.05, Wilcox Rank Sum, FDR corrected). (FIG. 18A) Percentage of TMA-lyase positive and negative genomes that encode at least 1 enzyme from the indicated family. (FIG. 18B) Percentage of TMA-lyase positive and negative genomes that encode any CAZyme that is significantly depleted in TMA-lyase positive genomes.

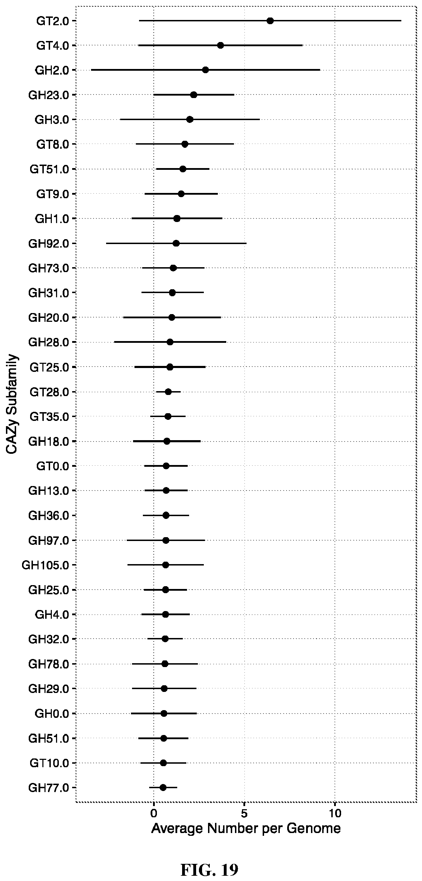

**FIG. 19** is a chart showing the most abundant families in TMA-lyase negative genomes, ordered by average gene count. Chart represent mean +/- s.d.

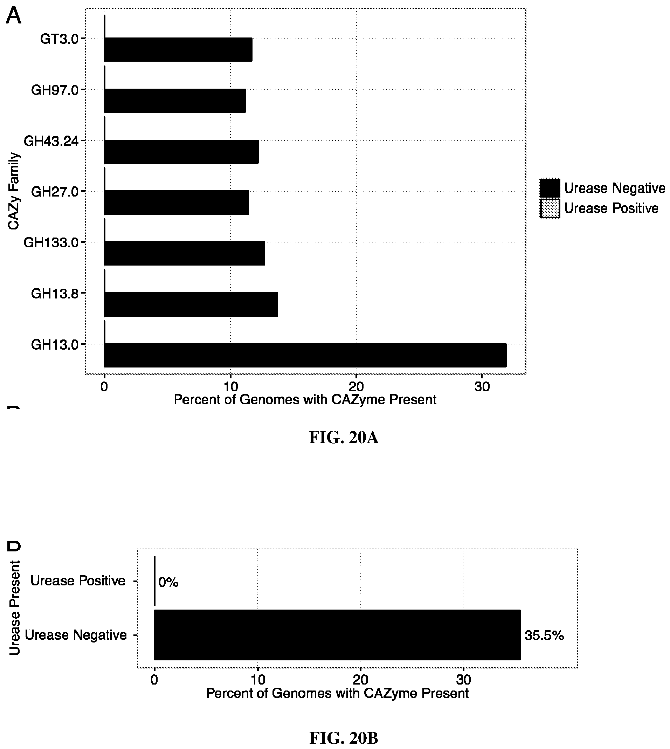

**FIGS. 20A-20B** are charts showing the percentage of genomes encoding CAZy families significantly depleted in urease positive genomes ($P < 0.05$, Wilcox Rank Sum, FDR corrected). (FIG. 20A) Percentage of urease positive and negative genomes that encode at least 1 enzyme from the indicated family. (FIG. 20B) Percentage of urease positive and negative genomes that encode any CAZyme that is significantly depleted in urease positive genomes.

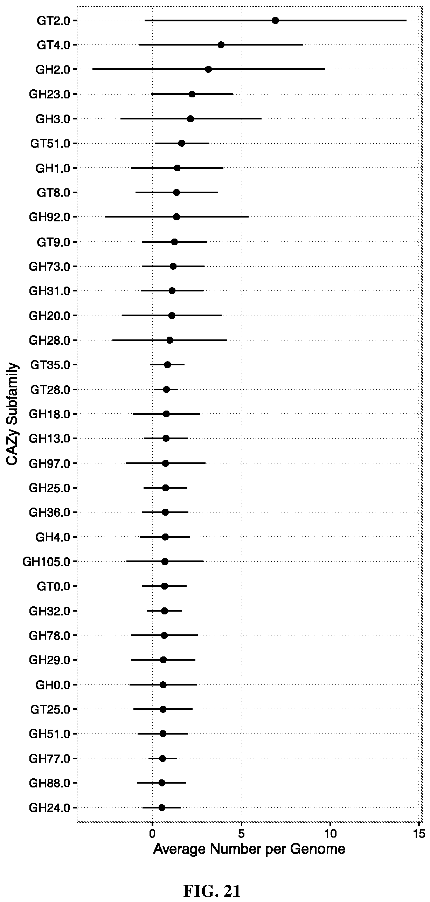

**FIG. 21** is a chart showing the most abundant families in urease negative genomes, ordered by average gene count. Chart represent mean +/- s.d.

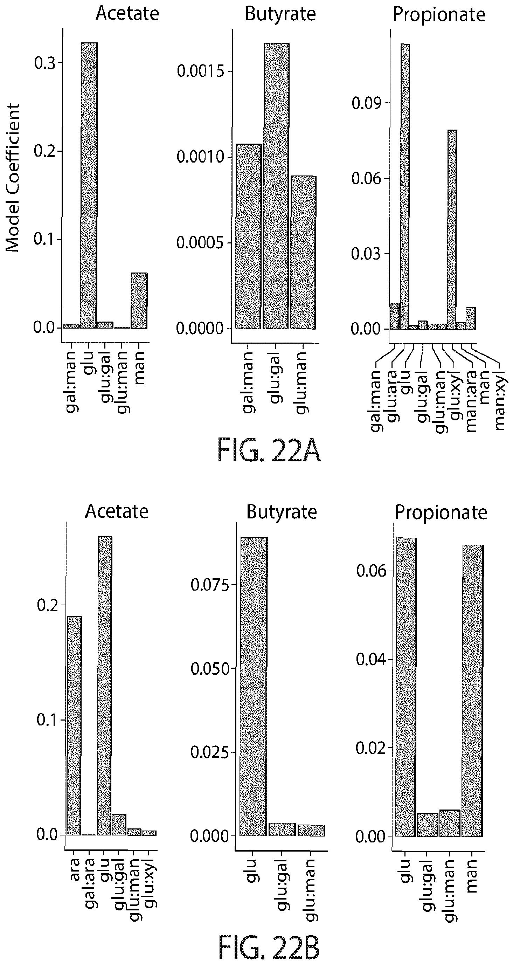

**FIGS. 22A-22B** are graphs showing the results of LASSO linear regression model of SCFA production as a function of glycan composition, allowing all 2nd order interaction terms. SCFA production in an (FIG. 22A) ex vivo model and from a (FIG. 22B) defined community.

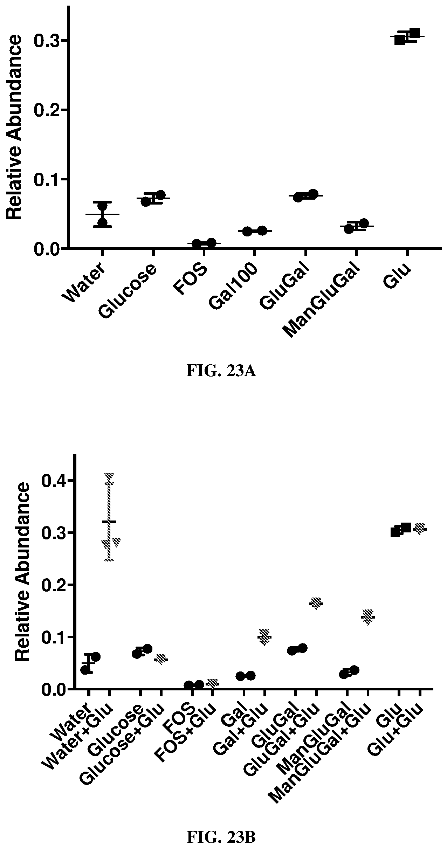

**FIGS. 23A-23B** are graphs showing relative abundance of a *Bacteroides cellulolyticus* strain in a defined community composed of 15 strains, grown in the presence of carbohydrates for 48 hours (FIG. 23A, and FIG. 23B black circles), or in the presence of indicated carbohydrates with an added glycan polymer preparation (eg, Glu100) at 18 hours (FIG. 23B, grey triangles). Shown is average relative abundance $\pm$ st.dev.

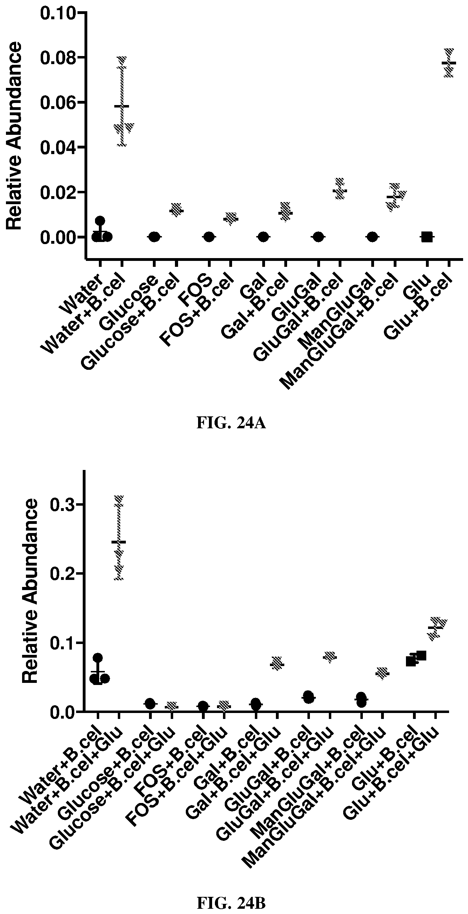

**FIGS. 24A-24B** are graphs showing relative abundance of a *Bacteroides cellulolyticus* strain in a defined community composed of 14 strains. FIG. 24A shown the relative abundance of *B. cellulolyticus* grown in the presence of various carbohydrates for 48 hours (black circles), or in the presence of indicated carbohydrates with added *B. cellulolyticus* at 18 hours (grey triangles). FIG. 24B shows the relative abundance of *B. cellulolyticus* grown in the same defined community composed of 14 strains, in the presence of various carbohydrates and added *B.cellulolyticus* at 18 hours (black circles), or in the presence of indicated carbohydrates with added *B. cellulolyticus* at 18 hours and added glycan polymer preparation (Glu, grey triangles). Shown is average relative abundance $\pm$ st.dev.

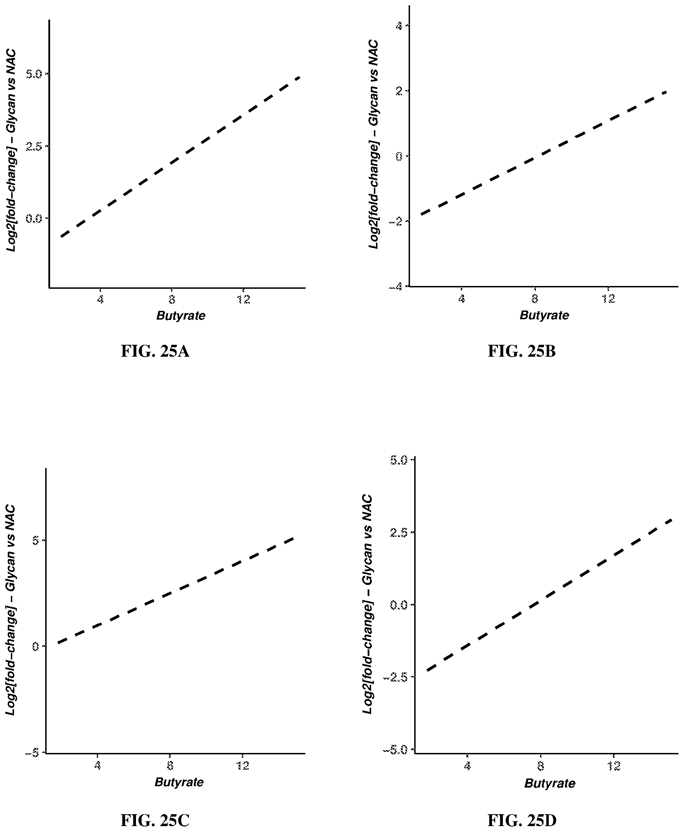

**FIGS. 25A-25D** are graphs showing 16S rRNA sequencing analysis results for the panel of bacteria screened in Example 23 and correlation with butyrate production. As shown, several taxa are highly correlated with butyrate levels. (FIG. 25A) Clostridiaceae (rho = 0.406 p.value 0.003), (FIG. 25B) Lachnospiraceae roseburia (rho = 0.333 p.value 0.018), (FIG. 25C) Bacteroides fragilis (rho = 0.483 p.value 0), (FIG. 25D) Turicibacteraceae turicibacter (rho = 0.554 p.value = 0).

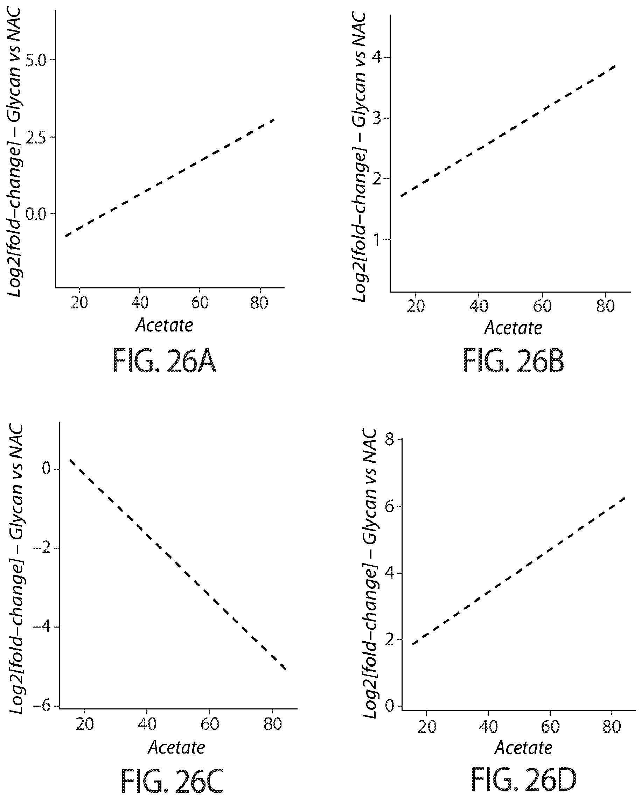

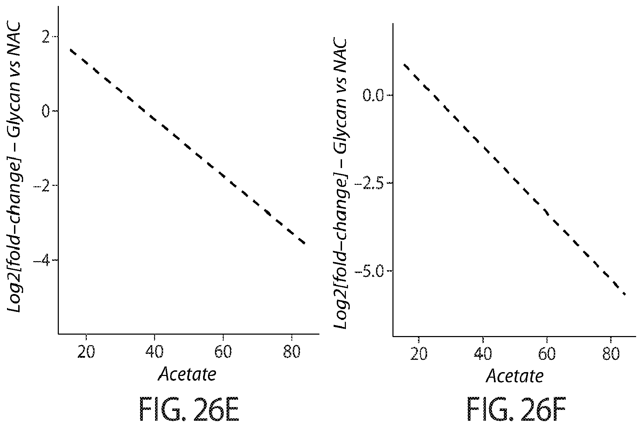

**FIGS. 26A-26F** are graphs showing 16S rRNA sequencing analysis results for the panel of bacteria screened in Example 23 and correlation with acetate production. In the ex vivo assay, several taxa are highly correlated with acetate levels. As shown, several taxa are highly correlated with acetate levels. (FIG. 26A) Clostridiaceae (rho = 0.428 p.value = 0.002), (FIG. 26B) Bacteroides uniformis (rho = 0.525 p.value = 0), (FIG. 26C) Ruminococcaceae Oscillospira (rho = -0.791 p.value = 0), (FIG. 26D) Bacteroides ovatus (rho = 0.405 p.value 0.004), (FIG. 26E) Bacteroidales Rikenellaceae (rho = -0.739 p.value = 0), (FIG. 26F) Clostridiales Ruminococcaceae (rho = -0.83 p.value = 0).

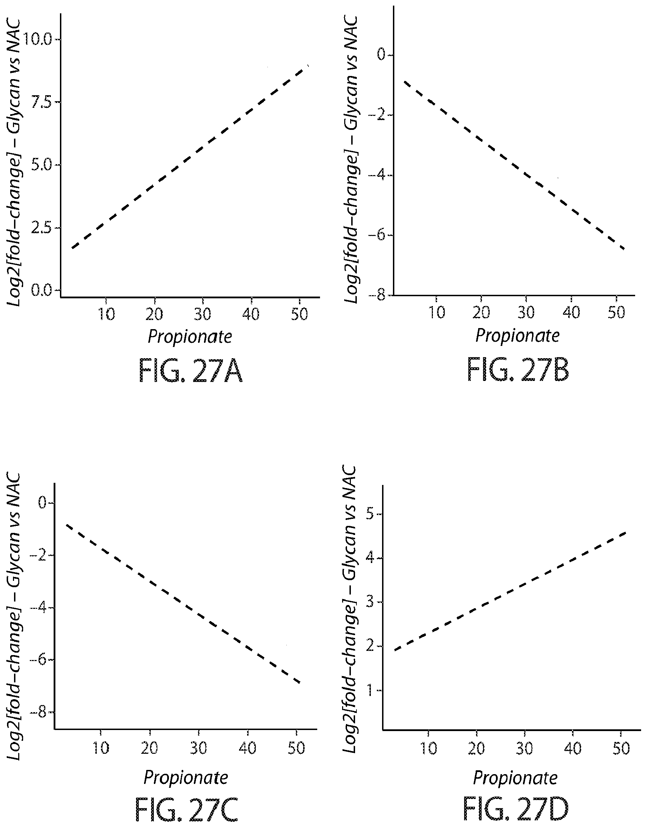

**FIGS. 27A-27D** are graphs showing 16S rRNA sequencing analysis results for the panel of bacteria screened in Example 23 and correlation with propionate production. As shown, several taxa are highly correlated with propionate levels. (FIG. 27A) Bacteroides ovatus (rho = 0.678 p.value 0), (FIG. 27B) Bifidobacterium (rho = -0.781 p.value =0), (FIG. 27C) Ruminococcus bromii rho = -0.72 p.value 0), (FIG. 27D) Bacteroides uniformis (rho = 0.559 p.value = 0).

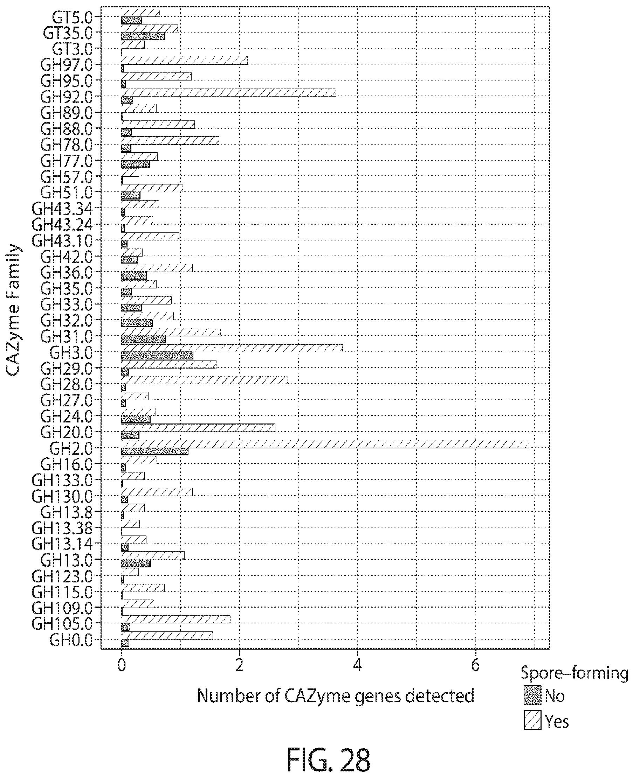

**FIG. 28.** Number of CAZyme genes detected in spore-forming and non-spore-forming bacteria (mean) for each CAZyme family and subfamily. Only families where genes were significantly enriched in spore-forming bacteria and detected in >10% of spore-forming bacterial genomes are shown ($P < 0.05$, Wilcox Rank Sum, FDR corrected).

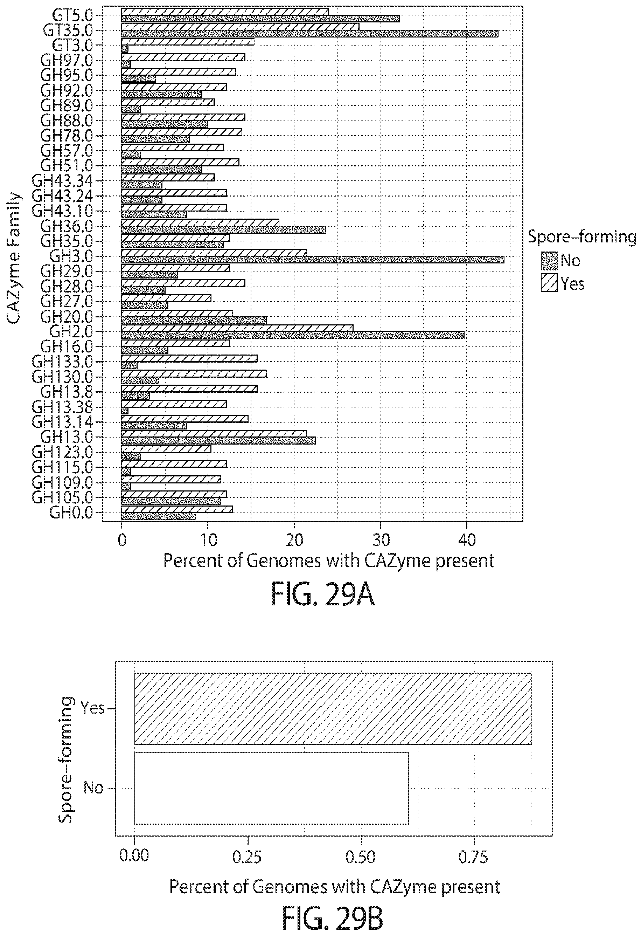

**FIGS. 29.** Percentage of genomes encoding CAZy families significantly enriched in genomes of spore formers vs. non-spore forming bacteria ($P < 0.001$, Wilcox Rank Sum, FDR corrected and identified in >10% of spore-formers). (**A**) Percentage of spore-forming and non-spore forming bacteria that encode at least 1 enzyme from the indicated family. (**B**) Percentage of spore-forming and non-spore forming bacteria that encode any CAZyme family or subfamily that is significantly enriched individually in spore-forming bacteria.

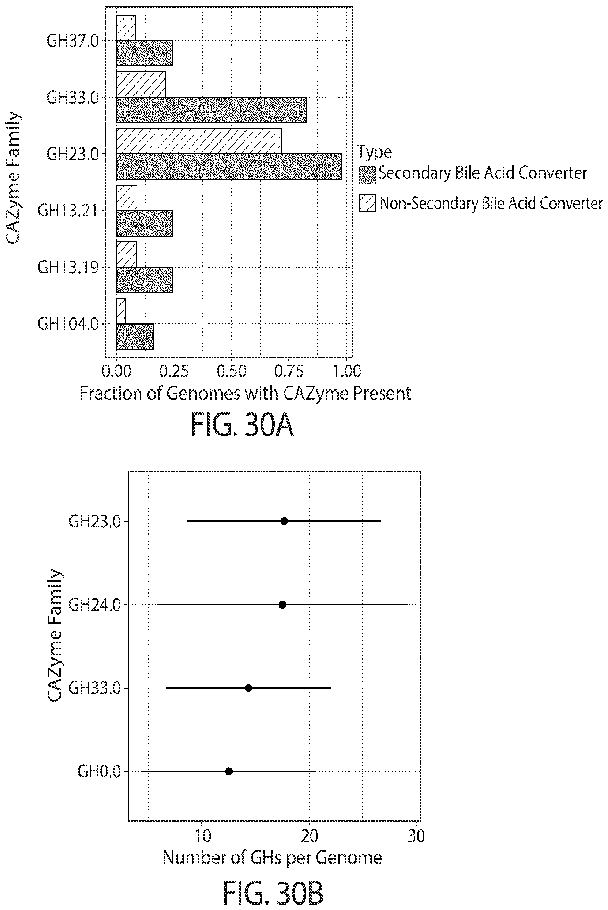

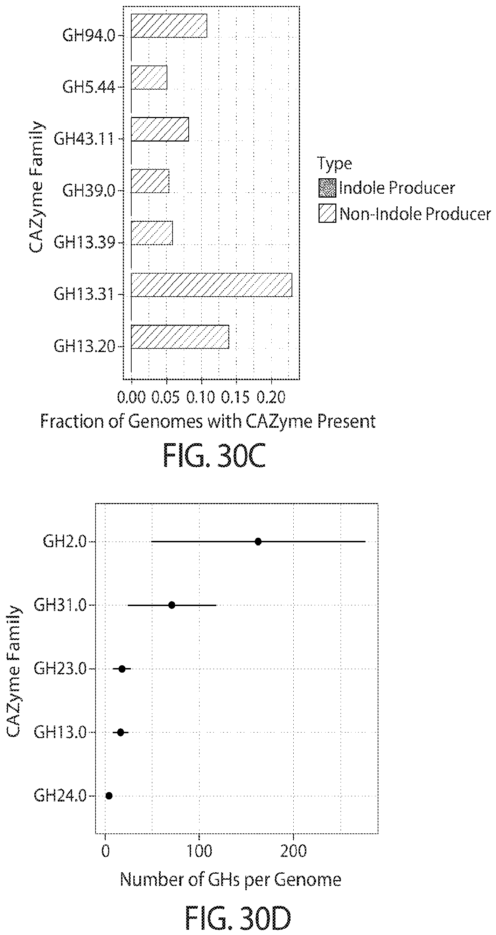

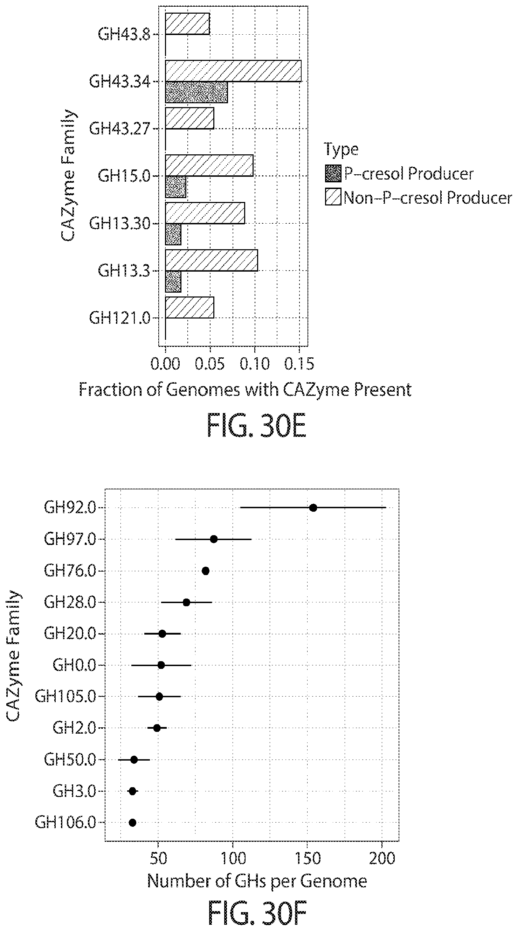

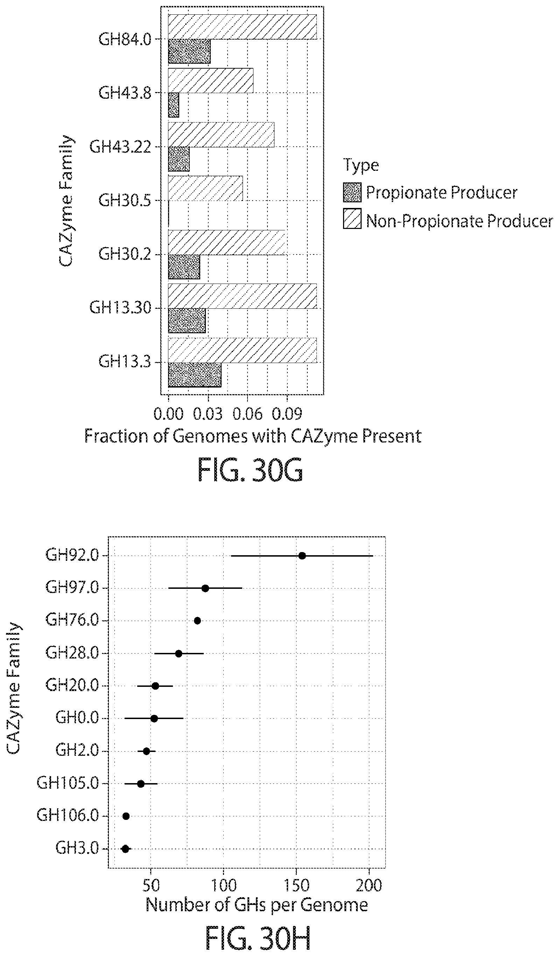

**FIGS. 30A-30H** are graphs showing the percentage of genomes encoding CAZy families significantly enriched in metabolite converter genomes (FIGS. 30A, 30C, 30E, 30G) and charts showing the most abundant families in metabolite converter genomes, ordered by average gene count (FIGS. 30B, 30D, 30F, 30H). The percentage of: secondary bile acid converter and non-converter genomes (FIG. 30A), genomes encoding CAZy families exclusively encoded in non-indole producing bacteria (FIG. 30C), genomes encoding CAZy families significantly depleted in p-cresol producing genomes (FIG. 30E), and genomes encoding CAZy families significantly depleted in prodpionate producing genomes (FIG. 30G) are depicted. Charts showing the most abundant families in: secondary bile acid converter genomes (FIG. 30B), indole negative genomes (FIG. 30D), p-cresol negative genomes (FIG. 30F), and propionate negative genomes (FIG. 30H) are depicted. Chart represent mean +/- s.e.

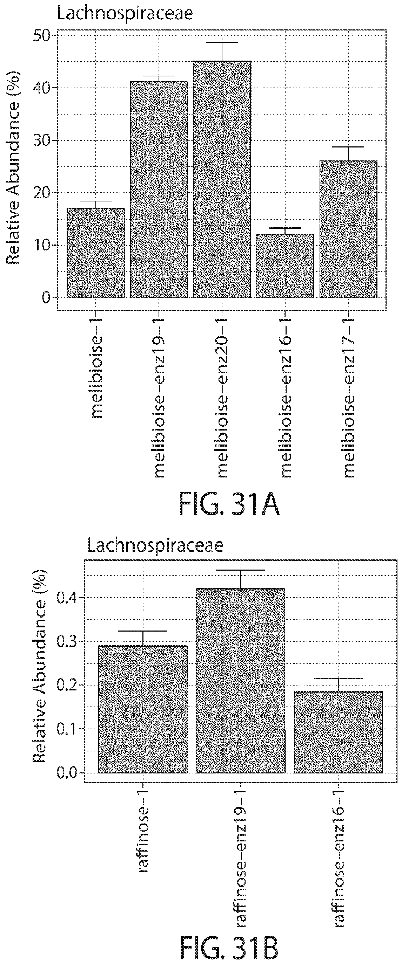

**FIGS 31A and 31B** are graphs showing the growth of Lachnospiraceae bacteria relative abundance in an ex vivo community when grown in the presence of melibiose (e.g., melibiose-1) (FIG. 31A) or raffinose (e.g., raffinose-1) (FIG. 31B) with alpha-galactooligosaccharides synthesized via alpha-galactosidase and either melibiose or raffinose. FIG. 31A depicts enzymes 19 and 20 are alpha-galactosidases encoded in bacterial genomes from Lachnospiraceae and showed a specific enrichment for those taxa (melibiose-enz19-1 and melibiose-enz20-1) compared to alpha-galactosidases that originated on other species (melibiose-enz16-1 and melibiose-enz17-1), which did not show the same specific enrichment for Lachnospiraceae bacteria. FIG. 31B depicts enzyme 19 is an alpha-galactosidases encoded in bacterial genomes from Lachnospiraceae and showed a specific enrichment for those taxa (raffinose-enz 19-1) compared to an alpha-galactosidases that originated in a different species (raffinose-enz16-1), which did not show the same specific enrichment for Lachnospiraceae bacteria.

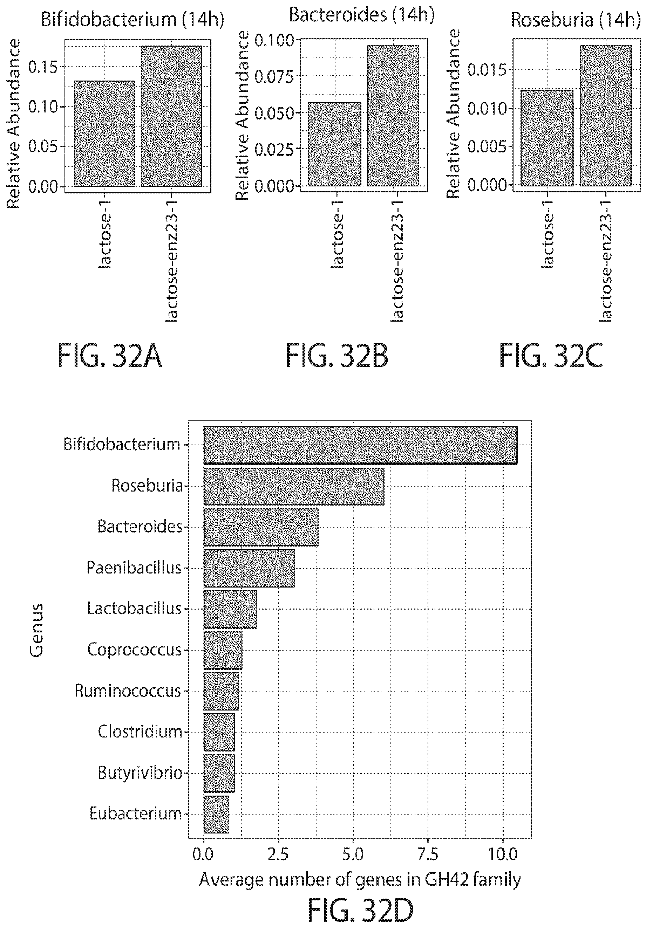

**FIGS. 32A-32D** are graphs showing the growth of Bifidobacterium (FIG. 32A), Bacteroides (FIG. 32B), and Roseburia (FIG. 32C) bacteria relative abundance in an *ex vivo* community when grown in the presence of lactulose (lactulose-1) and beta-galactooligosaccharides synthesized via GH42 beta-galactosidase (enz23) and lactulose (lactulose-enz23-1). Enzyme 23 is a beta-galactosidase encoded in the bacterial genome from a Bifidobacteria species and beta-galactooligosaccharides synthesized uing this enzyme (lactulose-enz23-1) showed enrichment of Bifidobacterium, Roseburia, and Bacteroides compared to lactulose alone. GH42 beta-galactooligosaccharides show enrichment in GH42 glycosidases from Bacteroides and Firmicute genomes (FIG. 32D), common gut microbiome commensals.

## DETAILED DESCRIPTION OF THE INVENTION

The present invention features, at least in part, methods of treating a subject having a disease or disorder (e.g., as described herein) with a glycan polymer preparation. In embodiments, the glycan polymer preparation is selected on the basis that it modulates the production or level (e.g., an unwanted level) of a metabolite (e.g., a short chain fatty acid (SFCA), (e.g., propionate or butyrate), ammonia, trimethylamine (TMA), trimethylamine N-oxide (TMAO), a uremic solute (e.g., p-cresol or indole), lipopolysaccharide (LPS), or a bile acid (e.g., a secondary bile acid)). The unwanted level of metabolite may be too high or too low. In some embodiments, the metabolite is associated with a desired (e.g. beneficial) effect on the subject's health. In other embodiments, the metabolite is associated with an unwanted (e.g. deleterious) effect on the subject's health. In some embodiments, the methods described herein include increasing a metabolite. In other embodiments, the methods include decreasing a metabolite. In some embodiments, the metabolite is a microbial (e.g. bacterial) metabolite. In some embodiments, a first metabolite is modulated (e.g. produced by taxa A) to modulate a second metabolite (e.g. produced by taxa B). In some embodiments, the second metabolite is associated with a disease or disorder. The unwanted level of metabolite may occur anywhere in subject's body (e.g. the GI tract, including the colon and intestines, fecal matter, the blood, the brain, the nervous system, an organ, including the heart, liver and kidneys, urine, and elsewhere). In some embodiments, metabolite production of the microbiota (e.g. in the gut) is modulated and has a local effect on the levels of the metabolite (e.g. a local decease or increase of the metabolite). In some embodiments, metabolite production of the microbiota (e.g. in the gut) is modulated and has a systemic effect on the levels of the metabolite (e.g. a systemic decease or increase of the metabolite). In some embodiments, modulation of a first metabolite (e.g., metabolite A, e.g., in the gut) leads to a modulation of a second metabolite (e.g., metabolite B, e.g., in a non-gut site of the body). In some embodiments, glycan polymer preparations are administered to subjects in need thereof, wherein the glycan polymers are substrates (e.g. preferred substrates) for a specific glycosidase machinery of a class of microbial metabolite producers. In some embodiments, glycan polymer preparations are administered to subjects in need thereof, wherein the glycan polymers are substrates (e.g. preferred substrates) for a specific glycosidase machinery of a class of non-producers of a microbial metabolite. In some embodiments, the balance (e.g., the relative abundance of microbial taxa in a body site, such as, e.g. the gut) of metabolite producers to metabolite non-producers is modulated to modulate the levels of metabolite produced by the site. In some embodiments, modulating the balance of producers to non-producers to modulate metabolite levels treats a disease or disorder that is associated with a dysregulation of the metabolite. In some embodiments, the subject has a dysbiosis of the site, such as the gut. Further provided herein are glycan polymer prepartions that are substrates (e.g., preferred substrates) of microbial metabolite producers or non-producers. In some embodiements, the glycan polymer preparations are tailored to the glycosidase enzyme profile of a microbial taxa or metabolite producers or non-producers, respectively, that is the glycan polymers are substrates (e.g., preferred substrates) of the glycosidases present in the genome of the producer or non-producer. In some embodiments, the glycosidases are enriched or exclusive to the one class (e.g. the metabolite producer) with respect to the other class (e.g., the non-producer). Further provided herein a coformulations (e.g. synbiotics) of tailored glycan polymers and a microbial taxa with a glycosidase repertoire (glycosidase profile) capable of (preferentially) using the glycan polymers as a substrate. In some embodiments, the co-formulations are used to increase engraftment of a microbial taxa in a microbial site, such as, e.g. the gut.

The glycan polymers described herein may be tailored to target a particular gut microbe, e.g., a human gut microbe. In some embodiments, glycoside hydrolase (glycosidase) enzymes are selected to tailor a glycan polymer to a particular microbe. In some embodiments, the glycoside hydrolase (glycosidase) profile of a microbe is determined and a glycan polymer is tailored thereto, e.g., using (e.g., *in vitro*) one or more glycoside hydrolase (glycosidase) so identified to produce a glycan polymer preparation under conditions that are suitable to produce glycan polymers. The glycoside hydrolases may be isolated (and optionally immobilized, e.g., on a suitable substrate). In some embodiments, glycoside hydrolases may be extracted from a microbe (e.g. a microbial extract comprising glycoside hydrolases). In some embodiments, microbial cells (e.g. bacteria) that comprise glycoside hydrolases on their surface and/or intracellulary may be used). In some embodiments, supernatants comprising glycoside hydrolases (e.g. from microbial cultures) may be used. In some embodiments, the glycoside hydrolase (glycosidase) profile of a particular microbe is not known or has not been determined but enzymes derived from the microbe are used (e.g. in isolated, extracted, whole cell, supernatant form, etc.) to produce the glycan polymers in the methods described herein. In some embodiments, the glycan polymer preparations produced as described herein are specific substrates for a particular microbe (or a group of microbes, e.g. a group of microbes with a comparable or similar glycosidase profile) and its glycosidase machinery. In some embodiments, the glycan polymer preparations are specifically fermented by the microbe or group of microbes, e.g. in the GI tract of a human subject (e.g. the glycan polymers are fermented at a faster rate or to a higher degree when compared to another microbe (or group of microbes), e.g. with a different glycosidase profile). In some embodiments, the glycan polymer preparations confer a growth advantage to the particular microbe. In some embodiments, the glycan polymers may be utilized to modulate the production of a microbial metabolite, e.g. a metabolite that is made by the particular microbe, or a microbial metabolite that is not made by the particular microbe. In the latter case, the particular microbe may compete with another microbe, one that produces a microbial metabolite that is undesired, and successful competition by the particular microbe may lead to lower levels of the microbial metabolite. In some embodiments, the glycan polymers may be used to promote engraftment into the microbiota of a subject (e.g. the gut microbiota, e.g. colonic microbiota) of a particular microbe that is administered to a subject in need of engraftment. In some embodiments, the glycan polymers confer a growth advantage on the particular microbe that lets it successfully compete for, e.g., space and nutrients, to more successfully engraft in the existing microbiota of the engraftment site (e.g. the gut).

**Definitions**

The present invention will be described with respect to particular embodiments and with reference to certain figures but the invention is not limited thereto but only by the claims. Terms as set forth hereinafter are generally to be understood in their common sense unless indicated otherwise.

"Abundance" of a microbial taxa as used herein is a relative term and refers to the relative presence of a microbial taxa to other taxa in a community in a defined microbial niche, such as the GI tract, or in the entire host organism (e.g. a human or a laboratory animal model of disease).

"Acquire" or "acquiring" as the terms are used herein, refer to obtaining possession of a value, e.g., a numerical value, or image, or a physical entity (e.g., a sample), by "directly acquiring" or "indirectly acquiring" the value or physical entity. "Directly acquiring" means performing a process (e.g., performing a synthetic or analytical method or protocol) to obtain the value or physical entity. "Indirectly acquiring" refers to receiving the value or physical entity from another party or source (e.g., a third party laboratory that directly acquired the physical entity or value). Directly acquiring a value or physical entity includes performing a process that includes a physical change in a physical substance or the use of a machine or device. Examples of directly acquiring a value include obtaining a sample from a human subject. Directly acquiring a value includes performing a process that uses a machine or device, e.g., an NMR spectrometer to obtain an NMR spectrum.

"Distinct" as used herein, e.g. with reference to a species in a glycan polymer, is meant to denote that it is chemically and/or structurally different from another. For example, two sugars are "distinct" if they are chemically different, e.g. a fucose and a xylose, or structurally different, e.g. cyclic vs. acyclic, L- vs. D-form. Two dimers are distinct if they consist of the same two monomers but one pair contains alpha-1,4 bond and the other contains a beta-1,6 bond. Distinct entities may have any other suitable distinguishing characteristic or property that can be detected by methods known in the art and/or described herein.

As used herein, a "dosage regimen", "dosing regimen", or "treatment regimen" is a modality of drug administration that achieves a therapeutic objective. A dosage regimen includes definition of one, two, three, or four of: a route of administration, a unit dose, a frequency of dosage, and a length of treatment.

"Dysbiosis" refers to an imbalanced state of the microbiota, e.g., within the GI tract, in which the normal diversity, proportion of a first bacterial taxa to a second bacterial taxa and/or function (e.g., the production of a metabolite) of the ecological network is disrupted or disturbed. This undesired, e.g., unhealthy, state can be due to a number of factors including, but not limited to, a decrease or increase in the diversity of the microbiota (e.g., bacterial taxa), the overgrowth of one or more pathogens or pathobionts, or the shift to an ecological microbial community that no longer provides an essential function to the host subject, and, in an embodiment, therefore no longer promotes health or, which is associated with unwanted symptoms in the subject. In one embodiment, the production of a metabolite is modulated so as to contribute to the development of a disease or disorder.

By the terms "effective amount" and "therapeutically effective amount" of a composition (such as, e.g., a pharmaceutical composition) or a drug agent is meant a sufficient amount of the composition or agent to provide the desired effect. In some embodiments, a physician or other health professional decides the appropriate amount and dosage regimen. An effective amount also refers to an amount of a composition (such as, e.g., a pharmaceutical composition) or a drug agent that prevents the development or relapse of a medical condition.

"Microbial Engraftment" or simply "engraftment" refers to the establishment (e.g. growth) of microbial taxa in a target niche (e.g. the human gut, such as the colon or intestines) that are either underrepresented (e.g. relative to a healthy reference subject) or absent (e.g. undetectable) in a human subject prior to engraftment (e.g. by administering the microbial taxa to the subject, e.g. in form of a synbiotic described herein). Engrafted microbial taxa can establish for a transient period, or demonstrate long-term stability in the microbiota that populates the subject post engraftment of the microbial taxa. In some embodiments, the engrafted microbial taxa can induce an environmental shift in the target niche representing a shift from dysbiosis to a health state.

"Fructooligosaccharide" or "FOS", as the terms are used herein, refer to a fructose polymer, optionally comprising terminal glucose, of the following sequence: (Fru)n-Glc consisting of one or more of: beta 2,1, beta 2,6, alpha 1,2 and beta-1,2 glycosidic bonds, wherein n typically is 3-10. Variants include Inulin type $\beta$-1,2 and Levan type $\beta$-2,6 linkages between fructosyl units in the main chain. In an embodiment, FOS is made by a method described in any of references 8,24,25, 61,67,69, 72,170, or 176-186, or 21,29, 170, 176, or 222 of Meyer, Biotechnological Production of Oligosaccharides - Applications in the Food Industry, Chapter two, Food technology and Industry, 2015, (Meyer 2015) which, together with each of its references referred to herein, is hereby incorporated by reference. In an embodiment, FOS is a FOS described in, or made by a method described in, Sangeetha et al. 2005 , 2014 found in Diez-Municio et al., 2014, Synthesis of novel bioactive lactose-derived oligosaccharides by microbial glycoside hydrolases, 2014, Microbial Biotecnhology, 7:315-313 (Diez-Municio et al. 2014), which together with each of its references referred to herein, is hereby incorporated by reference. In an embodiment FOS is made from an enzyme from B. macerans, Z. mobilis, L. reutri, A. niger, A. japonicas, A. foetidus, A. sydowi,bA. Pullans, C. purpurea, F. oxysporum P. citrinum, P. frequentans, P. spinulosum, P. rigulosum, P. parasitica S. brevicaulis, S. cerevisiae, or K. marxianus. In embodiments FOS is produced by enzymatic action of a Fructosyltransferase, $\beta$-fructofuranosidase (EC 3.2.1.26), inulosuscrase (EC 2.4.1.9) levansucrase (EC 2.4.1.10), or endoinulinase.

"Galactooligosacharride" or "GOS", as the terms are used herein, refer to a mixture of substances produced from lactose, with two to eight saccharide units, in which one of the units is a terminal glucose and the remaining units are galactose and disaccharides comprising two units of galactose. In an embodiment GOS is a mixture of galactopyranosyl oligomers (DP= 3-8) linked mostly by $\beta$-(1,4) or $\beta$-(1,6) bonds, although low proportions of $\beta$ (1,2) or $\beta$-(1,3) linkages may also be present. Terminal glucosyl residues are linked by $\beta$-(1,4) bonds to galactosyl units. GOS is synthesized by the reverse action of $\beta$-galactosidases (EC 3.2.1.23) on lactose at relatively high concentrations of lactose. In an embodiment GOS is synthesized by enzymatic action of a $\beta$-galactosidase from Bifidobacterium, e.g., Bifidobacterium longum, Kluyveromyces sp., Kluyveromyces marxianus, Aspergillus sp., e.g., Aspergillus oryzae, Escherichia coli K-12, Bacillus circulans, Lactobacillus bulgaricus, S. singularis, S. thermophiles, or C. laurentii. In an embodiment, GOS, is a GOS disclosed in, or made by a method described in, any of references 8,105, or 196-206 or 105,120, 198, 202-205, or 223- 227 of Meyer 2015, which together with each of its references referred to herein, is hereby incorporated by reference. In an embodiment GOS is a GOS described in, or made by a method described in, Panesar et al. 2006 or Torres et al 2010, 2014 found in Diez-Municio et al. 2014, which together with each of its references referred to herein, is hereby incorporated by reference.

"Glucooligosaccharide" or "GLOS", as the terms are used herein, refer to a polymer of glucose subunits. The main linkages in GLOS are (Glc)n [$\alpha(\rightarrow 2)$, $\alpha(1\rightarrow 3)$, $\alpha(1\rightarrow 4)$, and $\alpha(1\rightarrow 6)$]. In and embodiment GLOS is made with Dextransucrase (EC 2.4.1.5). In an embodiment, enzymes from Bacteria (L. mesenteroides; L. citreum) can be used to produce GLOS. In an embodiment GLOS is a GLOS described in, or made by a method described in, any of references Remaud et al., 1992, Chung and Day, 2002 or Kim et al., 2014 found in Diez-Municio et al., 2014, Synthesis of novel bioactive lactose-derived oligosaccharides by microbial glycoside hydrolases, 2014, Microbial Biotecnhology, 7:315-313, which together with each of its references referred to herein, is hereby incorporated by reference.

As used herein, a "glycan polymer preparation" (also referred to as a "preparation of glycan polymers", "glycan preparation" or "glycan polymer") is a preparation comprising glycan polymers that exhibits a desired effect (e.g. a therapeutic effect). In some embodiments, preparations of glycan polymers do not contain one or more naturally occurring oligosaccharide, including: glucooligosaccharide, mannanoligosaccharide, inulin, lychnose, maltotretraose, nigerotetraose, nystose, sesemose, stachyose, isomaltotriose, nigerotriose, maltotriose, melezitose, maltotriulose, raffinose, kestose, fructooligosaccharide, 2'-fucosyllactose, galactooligosaccharide, glycosyl, idraparinux, isomaltooligosaccharide, maltodextrin, xylooligosaccharide, agar, agarose, alginic acid, alguronic acid, alpha glucan, amylopectin, amylose, arabioxylan, beta-glucan, callose, capsulan, carrageenan, cellodextrin, cellulin, cellulose, chitin, chitin nanofibril, chitinglucan complex, chitosan, chrysolaminarin, curdlan, cyclodextrin, alpha-cylcodextrin, dextran, dextrin, dialdehyde starch, ficoll, fructan, fucoidan, galactoglucomannan, galactomannan, galactosamineogalactan, gellan gum, glucan, glucomannan, glucoronoxyland, glycocalyx, glycogen, hemicellulose, hypromellose, icodextrin, kefiran, laminarin, lentinan, levan polysaccharide, lichenin, mannan, mucilage, natural gum, paramylon, pectic acid, pectin, pentastarch, phytoglycogen, pleuran, poligeenan, polydextrose, porphyran, pullulan, schizophyllan, sepharose, sinistrin, sizofiran, sugammadex, welan gum, xantham gum, xylan, xyloglucan, zymosan, and the like. In some embodiments, a glycan polymer exists as a salt, e.g., a pharmaceutically acceptable salt.

A "glycan subunit" as used herein refers to the individual unit of a glycan species disclosed herein, e.g., the building blocks from which the glycan species is made. In an embodiment, a glycan subunit is a monomer. In an embodiment, a glycan subunit is a dimer. In an embodiment, a glycan subunit is a monosaccharide. In an embodiment, a glycan subunit is a disaccharide. In some embodiments, the glycan subunit is a carbohydrate and may be selected from a sugar alcohol, a short-chain fatty acid, a sugar acid, an imino sugar, a deoxy sugar, and an amino sugar. In some embodiments, the glycan subunit is erythrose, threose, erythulose, arabinose, lyxose, ribose, xylose, ribulose, xylulose, allose, altrose, galactose, glucose, gulose, idose, mannose, talose, fructose, psicose, sorbose, tagatose, fucose, fuculose, rhamnose, mannoheptulose, sedoheptulose, and the like. In some embodiments, the glycan subunit is glucose, galactose, arabinose, mannose, fructose, xylose, fucose, or rhamnose. In embodiments, a glycan comprises distinct glycan subunits, e.g., a first and a second monosaccharide, or a first and a second disaccharide. In embodiments, a glycan comprises distinct glycan subunits, e.g., a first, a second, a third, a fourth, and/or a fifth distinct glycan subunit.

As used herein, "a glycosidase enzyme molecule" comprises a polypeptide that retains or has an activity of the glycosidase enzyme, e.g., it retains or has at least about 40%, about 50%, about 60%, about 70%, about 80%, about 90%, about 95%, about 99%, about 99.9% of the turnover rate of the glycosidase enzyme, or it retains or has at least about 40%, about 50%, about 60%, about 70%, about 80%, about 90%, about 95%, about 99%, about 99.9% of the specificity of the glycosidase enzyme, or it retains or has at least about 40%, about 50%, about 60%, about 70%, about 80%, about 90%, about 95%, about 99%, about 99.9% of the affinity for a glycan subunit of the glycosidase enzyme. In some embodiments, a glycosidase enzyme molecule comprises a polypeptide that has an activity of the glycosidase enzyme that is at least about 110%, 120%, 130%, 140%, 150%, 160%, 170%, 180%, 190%, 200%, 300%, 400%, or 500% of the turnover rate of the glycosidase enzyme or it has at least 110%, 120%, 130%, 140%, 150%, 160%, 170%, 180%, 190%, 200%, 300%, 400%, or 500% of the affinity for a glycan subunit of the glycosidase enzyme. In some embodiments, the glycosidase enzyme molecule is a fragment (e.g., an active fragment) of the glycosidase enzyme. In some embodiments, the glycosidase enzyme molecule differs by at least 1, 2, 3, 4, 5, 10, 25, 50, 75, 100 or more amino acid residues compared with the glycosidase enzyme. In some embodiments, the glycosidase enzyme molecule comprises at least 1, 2, 3, 4, 5, 10, 25, 50, 75, 100 amino acid mutations (e.g., deletions, additions, or substitutions) compared with the glycosidase enzyme.

"Glycosidase enzymes" as used herein include glycosidases (also referred to as "glycoside hydrolase" (GH)), glycosyltransferases (GT) and lysases.

As used herein, "glycotaxa" refers to bacterial microbes (e.g., human gut microbes) grouped according to the presence or absence (e.g., lack of) a metabolic (e.g., enzymatic) function. In some embodiments, taxa may be grouped according CAZy glycosidase/glycohydrolase (GH) or CAZy glycosyltransferase (GT) enzyme function. In some embodiments, bacterial taxa may fall into any one of glycotaxa class 1, glycotaxa class 2, glycotaxa class 3, glycotaxa class 4, glycotaxa class 5, glycotaxa class 6, or glycotaxa class 7. In some embodiments, glycotaxa class 1 contains the *but* and/or *buk* gene-containing bacterial taxa. In some embodiments, glycotaxa class 2 contains *cutC* gene-negative bacterial taxa. In some embodiments, glycotaxa class 3 contains *urease* gene-negative bacterial taxa. In some embodiments, glycotaxa class 4 excludes one or more propionate production associated enzymes chosen from propionate kinase, propionate CoA-transferase, propionate-CoA ligase, propionyl-CoA carboxylase, methylmalonyl-CoA carboxytransferase, (S)-methylmalonyl-CoA decarboxylase, methylmalonate-semialdehyde dehydrogenase, and propanal dehydrogenase. In some embodiments, glycotaxa class 5 contains bile acid production (e.g., secondary bile acid production) associated enzymes chosen from 7alpha-hydroxysteroid dehydrogenase, 12alpha-hydroxysteroid dehydrogenase, 7beta-hydroxysteroid dehydrogenase (NADP+), 2beta-hydroxysteroid dehydrogenase, 3beta-hydroxycholanate 3-dehydrogenase (NAD+), 3alpha-hydroxycholanate dehydrogenase (NADP+), 3beta-hydroxycholanate 3-dehydrogenase (NADP+), 3alpha-hydroxy bile acid-CoA-ester 3-dehydrogenase, 3alpha-hydroxycholanate dehydrogenase (NAD+), bile acid CoA-transferase, bile-acid 7alpha-dehydratase, and bile acid CoA ligase. In some embodiments, glycotaxa class 6 excludes one or more indole production associated enzymes (e.g., tryptophanase). In some embodiments, glycotaxa class 7 excludes one or more p-cresol production associated enzymes chosen from 4-hydroxyphenylacetate decarboxylase and aldehyde ferredoxin oxidoreductase.

"Isomaltooligosaccharide" or "IMOS", as the terms are used herein, refer to a mixture of oligosaccharides with predominantly $\alpha$-(1,6)-linked glucose residues with a degree of polymerization (DP) ranging from 2- 6, and oligosaccharides with a mixture of $\alpha$-(1,6) and occasionally $\alpha$-(1,4) glycosidic bonds such as panose. In an embodiment IMOS comprises glucosyl residues linked to maltose or isomaltose by $\alpha$-(1,6) glycosidic bonds. In an embodiment an IMOS is produced using starch as the raw material. In an embodiment it is produced from cornstarch and consists of isomaltose, isomaltotriose and panose. In an embodiment IMOS is the product of an enzymatic transfer reaction, using a combination of immobilized enzymes wherein starch is liquefied using α-amylase (EC 3.2.1.1) and pullulanase (EC 3.2.1.41), and, in a second stage, the intermediary product is processed by both β-amylase (EC 3.2.1.2) and α-glucosidase (EC 3.2.1.20). Beta-amylase first hydrolyzes the liquefied starch to maltose. The transglucosidase activity of α-glucosidase then produces isomaltooligosaccharides mixtures which contain oligosaccharides with both α-(1,6)- and α-(1,4)-linked glucose residues. In an embodiment IMOS is an IMOS described in, or made by a method described in, any of references 2, or 217- 219 or 12, 152, 159, or 236 of Meyer 2015, which together with each of its references referred to herein, is hereby incorporated by reference. In an embodiment IMOS is a IMOS described in, or made by a method described in, Panesar et al. 2006 or Torres et al 2010, 2014 found in Diez-Municio et al., 2014, which together with each of its references referred to herein, is hereby incorporated by reference. In an embodiment IMOS is synthesized by a the enzymatic hydrolysis of starch by an α-amylase or or pullulanase; or a β-amylase and α-glucosidase in sequence. In an embodiment IMOS is synthesized by an enzyme from A. niger,

Bacillus spp., B.subtilis, B. stearothermophilus, T. maritime, A. carbonarious, or L. mesenteroides

As used herein, an "isolated" or "purified" glycan polymer preparation is substantially pure and free of contaminants, e.g. pathogens, enzymes or otherwise unwanted biological material, or toxic or otherwise unwanted organic or inorganic compounds. In some embodiments, pure or isolated compounds, compositions or preparations may contain traces of solvents and/or salts (such as less than 10%, 9%, 8%, 7%, 6%, 5%, 4%, 3%, 2%, 1%, less than 0.5% or 0.1% by w/w, w/v, v/v or molar %). Purified compounds are or preparations contain at least about 60% (by w/w, w/v, v/v or molar %), at least about 75%, at least about 90%, at least about 95%, at least about 97%, at least about 98%, or at least about 99% by w/w, w/v, v/v or molar % the compound(s) of interest. For example, a purified (substantially pure) or isolated preparation of glycan polymers is one that is at least 80%, 85%, 90%, 91%, 92%, 93%, 94%, 95%, 98%, 99%, 99.5%, 99.8%, 99.9% or 100% of the glycan polymer by w/w, w/v, v/v or molar % (e.g., not including any solvent, such as e.g. water, in which the glycan polymer preparation may be dissolved) and separated from the components that accompany it, e.g. during manufacture, extraction/purification and/or processing (e.g. such that the glycan polymer is substantially free from undesired compounds). Purity may be measured by any appropriate standard method, for example, by column chromatography (e.g., size-exclusion chromatography (SEC)), thin layer chromatography (TLC), gas chromatography (GC), high-performance liquid chromatography (HPLC) or nuclear magnatic resonance (NMR) spectroscopy. Purified or purity may also define a degree of sterility that is safe for administration to a human subject, e.g., lacking viable infectious or toxic agents.

"Microbiome" as used herein refers to the genetic content of the communities of microbes ("microbiota") that live in and on a subject (e.g., a human subject), both sustainably and transiently, including eukaryotes, archaea, bacteria, and viruses (including bacterial viruses (e.g., phage)), wherein "genetic content" includes genomic DNA, RNA such as ribosomal RNA and messenger RNA, the epigenome, plasmids, and all other types of genetic information. In some embodiments, microbiome specifically refers to genetic content of the communities of microorganisms in a niche.

"Microbiota" as used herein refers to the community of microorganisms that occur (sustainably or transiently) in and on a subject (e.g., a human subject), including eukaryotes, archaea, bacteria, and viruses (including bacterial viruses, e.g. phage). In some embodiments, microbiota specifically refers to the microbial community in a niche.

"Pathobionts" or "(Opportunistic) Pathogens" as used herein refer to symbiotic organisms able to cause disease only when certain genetic and/or environmental conditions are present in a subject (e.g., a human subject).

As used herein, the term "pathogenic" (e.g. "pathogenic bacteria") refers to a substance, microorganism or condition that has the capability to cause a disease. In certain contexts, pathogens also include microbes (e.g. bacteria) that are associated with a disease or condition but for which a (direct) causative relationship has not been established or has yet to be established. As used herein, the term "pathogens" refers to viruses, parasites and bacteria or other pathogens that may cause infections in a subject, e.g. a human.

As used herein, a "pharmaceutical composition" is a composition or preparation, having pharmacological activity or other direct effect in the mitigation, treatment, or prevention of disease, and/or a finished dosage form or formulation thereof and is for human use. A pharmaceutical composition is typically produced under good manufacturing practices (GMP) conditions. Pharmaceutical compositions may be sterile or non-sterile. If non-sterile, such pharmaceutical compositions typically meet the microbiological specifications and criteria for non-sterile pharmaceutical products as described in the U.S. Pharmacopeia (USP) or European Pharmacopoeia (EP). Pharmaceutical compositions may further comprise or may be co-administered with additional active agents, such as, e.g. additional therapeutic agents. Pharmaceutical compositions may also comprise e.g. additional therapeutic agents, polyphenols, prebiotic substances, probiotic bacteria, pharmaceutically acceptable excipients, solvents, carriers or any combination thereof. Any glycan polymer preparation described herein may be formulated as a pharmaceutical composition.

The term "subject" (in some cases "patient") as used herein refers to any human subject. The term does not denote any particular age or gender. Subjects may include pregnant women. Subjects may include a newborn (a preterm newborn, a full-term newborn), an infant up to one year of age, young children (e.g., 1 yr to 12 yrs), teenagers, (e.g., 13-19 yrs), adults (e.g., 20-64 yrs), and elderly adults (65 yrs and older). A subject does not include an agricultural animal, e.g., farm animals or livestock, e.g., cattle, horses, sheep, swine, chickens, etc.

A "substantial decrease" as used herein (e.g. with respect to a biomarker or metabolite) is a decrease of 5%, 10%, 20%, 30%, 40%, 50%, 60%, 70%, 80%, 90%, 95%, 97%, 98%, 99%, 99.9% or 100%.

A "substantial increase" as used herein (e.g. with respect to a biomarker or metabolite) is an increase of 10%, 20%, 30%, 40%, 50%, 60%, 70%, 80%, 90%, 100%, 150%, 200%, 250%, 300%, 350%, 400%, 450%, 500%, 550%, 600%, 650%, 700%, 750%, 800%, 850%, 900%, 950%, 1000%, or more than 1000%.

The term "substrate" as that term is used herein in connection with the terms glycosidase enzyme and/or glycosidase enzyme molecule, refers to a glycan polymer which is the product of, or has the structure of a glycan polymer made by a glycosidase enzyme molecule; and is the substrate of a glycosidase enzyme, e.g., a glycosidase expressed in a human gut microbe. In embodiments the glycosidase enzyme molecule, under the appropriate reaction conditions, catalyzes the polymerization of glycan subunits to form the substrate, and the glycosidase enzyme, under the appropriate reaction conditions, cleaves a bond between glycan subunits (in embodiments the same bond formed by the glycosidase enzyme molecule) of the substrate. In an embodiment the glycosidase enzyme molecule and the glycosidase enzyme have the same primary amino acid sequence, e.g., are the same enzyme. In embodiments, the substrate has one or more of the following properties:

i) it is sufficiently similar to a naturally occurring substrate of the glycosidase enzyme that the turnover rate for the substrate and the glycosidase enzyme is at least 10, 20, 30, 40, 50, 60, 70, 80, 90, 95, or 99% of that of at least one naturally occurring substrate of the glycosidase enzyme. Turnover rate can be expressed, e.g., in terms of cleaved glycosidic bonds per unit of time, e.g., per minute or hour, or rate of depolymerization of the glycan polymer per unit of time, e.g., hour or minute; ii) its binding constant for the glycosidase enzyme is at least 10, 20, 30, 40, 50, 60, 70, 80, 90, 95, or 99% that of at least one naturally occurring substrate of the glycosidase enzyme, and in embodiments is no more than 1, 2, 3, 4, 5, 10, 50, or 100 fold that of at least one naturally occurring substrate of the glycosidase enzyme; and iii) its binding motif for the glycosidase enzyme, its binding motif for the glycosidase enzyme molecule, and at least one naturally occurring substrate of the glycosidase enzyme share one or more of a specific glycan subunit, e.g., a specific sugar dimer, a specific sugar branching point, a specific alpha- or beta configuration, a specific regio-chemistry, e.g., an 1,2- 1,3- 1,4- 1,5- or 1,6-linkage; and iv) the substrate promotes the growth or metabolism of a human gut microbe that expresses the enzyme molecule.

"Synthetic" as used herein refers to a man-made compound or preparation, such as a glycan polymer preparation, that is not naturally occurring. In one embodiment, a non-enzymatic, polymeric catalyst described herein is used to synthesize the glycans of the preparation under suitable reaction conditions, e.g. by a polymerization reaction that creates oligomers from individual glycan subunits that are added to the reaction. In some embodiments, the non-enzymatic, polymeric catalyst acts as a hydrolysis agent and can break glycosidic bonds. In other embodiments, the non-enzymatic, polymeric catalyst can form glycosidic bonds. In one embodiment, a glycosidase enzyme molecule described herein is used to synthesize the glycans of the preparation under suitable reaction conditions, e.g. by a polymerization reaction that creates oligomers from individual glycan subunits that are added to the reaction. In some embodiments, the glycosidase enzyme molecule acts as a hydrolysis agent and can break glycosidic bonds. In other embodiments, the glycosidase enzyme molecule can form glycosidic bonds. In one embodiment, solid-phase oligosaccharide synthesis is used to synthesize the glycans of the preparation under suitable reaction conditions, e.g. by a polymerization reaction that creates oligomers from individual glycan subunits that are added to the reaction. Synthetic glycan polymer preparations may also include glycan polymers that are not isolated from a natural oligo- or polysaccharide source. It is to be understood that while the glycan polymer preparation is not isolated from a natural oligo- or polysaccharide source, the glycan subunits making up the glycan polymer can be and often are isolated from natural oligo- or polysaccharide sources, including those listed herein, or are synthesized de novo.

The terms "treating" and "treatment" as used herein refer to the administration of an agent or composition to a subject (e.g., a symptomatic subject afflicted with an adverse condition, disorder, or disease) so as to affect a reduction in severity and/or frequency of a symptom, eliminate a symptom and/or its underlying cause, and/or facilitate improvement or remediation of damage, and/or preventing an adverse condition, disorder, or disease in an asymptomatic subject (e.g., a human subject) who is susceptible to a particular adverse condition, disorder, or disease, or who is suspected of developing or at risk of developing the condition, disorder, or disease.

A "therapeutic nutrition product" is a food product that provides a therapeutic effect, either when administered solely or in combination with a second therapy (e.g., a drug therapy), in which case it provides an additive or synergistic therapeutic effect or alleviates or reduces negative effects of the second therapy (e.g., reduction of side effects). A therapeutic nutrition product forms part of a recommended diet (e.g., by a physician or dietitian or other expert in dietetics, human nutrition) and the regulation of a diet (e.g., based upon a subject's medical condition and individual needs).

"Xylooligosaccharide" or "XOS", as the terms are used herein, refer to sugar oligomers of xylose units linked by $\beta$-(1,4). The number of xylose residues varies from 2 to 10, but mainly consist of xylobiose, xylotriose and xylotetraose. Arabinofuranosyl, glucopyranosyl uronic acid or its 4-O-methyl derivative (2- or 3-acetyl or phenolic substituents) can also be present and results in branched XOS. In an embodiment the XOS is primarily linear $\beta$-(1,4)-linked XOS (mainly xylobiose, xylotriose and xylotetraose) as well as some oligosaccharides with branched arabinose residues. In an embodiment, XOS is made with β-xylanases from lignocellulosic materials. In an embodiment xylan is enzymatically hydrolysed to xylo-oligosaccharides by an endo-β-1,4-xylanase (EC 3.2.1.8) or by beta-Xylosidase (EC 3.2.1.9). In an embodiment XOS is made by the enzymatic degradation of xylans, e.g., by a Endo-β-1,4-xylanase, exo-β-1,4-xylosidase, α-glucuronosidase, α-L-arabinofuranosidase, acetylxylan esterase, ferulic acid esterase, or p-coumaric acid esterase. In an embodiment XOS, is a XOS disclosed in, or made by a method described in, any of references 152, 159, 162, 179, 214-216, or 232 of Meyer 2015, which together with each of its references referred to herein, is hereby incorporated by reference. In an embodiment XOS is a XOS described in, or made by a method described in, Casci and Rostal, 2006, found in Diez-Municio et al., 2014, which together with each of its references referred to herein, is hereby incorporated by reference. In an embodiment the XOS is synthesized by a xylanase from any of T. reesei, T. harzianu, T. viride, T. koningii, T. longibrachiatum, P. chyrosporium, G. trabeum, or A. oryzae.

Where the term "comprising" is used in the present description and claims, it does not exclude other elements. For the purposes of the present invention, the term "consisting of' is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. Claims and disclosure pertaining to methods of treatment or diagnosis are considered as an equivalent disclosure of embodiments and claims to "compound, composition, product, etc. for use in ..." or "use of a compound, composition, product, etc in the manufacture of a medicament, pharmaceutical composition, diagnostic composition, etc. for ..." and indicates that such compounds, compositions, products, etc. are to be used in diagnostic or therapeutic methods which may be practiced on the human or animal body. If an embodiment or a claim thus refers to a "method of treatment by administereing a compound to a human or animal being suspected to to suffer from a disease" this is considered to be also a disclosure of a "use of a compound in the manufacture of a medicament for treating a human or animal being suspected to to suffer from a disease" or "a compound for use in treating a human or animal being suspected to to suffer from a disease". As an example: a reference to a method of treating a subject having a disease or disorder associated with an unwanted level of a metabolite (e.g., a short chain fatty acid (SCFA), ammonia, trimethylamine (TMA), trimethylamine N-oxide (TMAO), a uremic solute, lipopolysaccharide (LPS), or a bile acid) by administering an amount of a glycan polymer preparation is considered to be a disclosure of (i) a glycan polymer preparation for use in treating a subject having a disease or disorder associated with an unwanted level of a metabolite (e.g., a short chain fatty acid (SCFA), ammonia, trimethylamine (TMA), trimethylamine N-oxide (TMAO), a uremic solute, lipopolysaccharide (LPS), or a bile acid) or (ii) use of a glycan composition in the manufacture of a medicament for treating a subject having a disease or disorder associated with an unwanted level of a metabolite (e.g., a short chain fatty acid (SCFA), ammonia, trimethylamine (TMA), trimethylamine N-oxide (TMAO), a uremic solute, lipopolysaccharide (LPS), or a bile acid).

Wherever reference is made to a method of treatment of an individual or a population of individuals by administering e.g. a glycan composition such a reference, in a preferred embodiment, contemplates an analytical, diagnostic step and the like in the course of such treatment which may help to determine e.g. whether an individual or population will be susceptible to a certain treatemt due to its microbiome composition, whether was succesful, etc. By way of example: a reference to a method of treating a subject having a disease or disorder associated with an unwanted level of a metabolite (e.g., a short chain fatty acid (SCFA), ammonia, trimethylamine (TMA), trimethylamine N-oxide (TMAO), a uremic solute, lipopolysaccharide (LPS), or a bile acid) by administering an amount of a glycan polymer preparation is considered to be also a disclosure of such a method, wherein in a preferred embodiment the subject e.g. (i) will be tested initally for the nature and level of the metabolite before commencing with the treatment, (ii) will be tested for the composition of its microbiome to adapt the administration of the glycan composition to the microbe glycosidase enzyme compoistion in the gut, (iii) will be tested in the course of treatment to monitor the effect of the administration of the glycan composition on the level of metabolite, etc.

The terms "obtainable by", "producible by" or the like are used to indicate that a claim or embodiment refers to compound, composition, product, *etc. per se,* i. e. that the compound, composition, product, etc. can be obtained or produced by a method which is described for manufacture of the compound, composition, product, etc., but that the compound, composition, product, etc. may be obtained or produced by other methods than the described one as well. The terms "obtained by", "produced by" or the like indicate that the compound, composition, product, is obtained or produced by a recited specific method. It is to be understood that the terms "obtainable by", "producible by" and the like also disclose the terms "obtained by", "produced by" and the like as a preferred embodiment of "obtainable by", "producible by" and the like.

It is to be further understood that the present disclosure, as preferred embodiments, also discloses how the individual aspects and embodiments described herein can be combined. For example,

Table 3 discloses an association between metabolites and phylae and strains whilst Table 5 discloses an association between metabolites and diseases. The person skilled in the art will thus consider this information together and understand which microorganisms must be influenced to e.g., lower the level of a metabolite in order to treat a certain disease.

As used herein, "homology" and "sequence identity" (used interchangeably herein) are measures of how similar a sequence (e.g., amino acid sequences or nucleic acid sequences) is to another sequence. Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frameshift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences.

**Methods of making glycan polymers**

A glycan polymer preparation may be produced using any method known in the art.

Glycan polymer compositions can comprise the glycans described herein, dietary fibers, such as, e.g., FOS (fructo-oligosaccharide), other sugars (e.g., monomers, dimers, such as, e.g., lactulose) and sugar alcohols, and optionally other components, such as, e.g., polyphenols, fatty acids, peptides, micronutrients, etc., such as those described in WO 2016/172658, "MICROBIOME REGULATORS AND RELATED USES THEREOF", and microbes, such as bacteria.

Glycan preparations described in WO 2016/122889 "GLYCAN THERAPEUTICS AND RELATED METHODS THEREOF" and WO 2016/172657, "GLYCAN THERAPEUTICS AND METHODS OF TREATMENT", which in their entirety are hereby incorporated by reference, are suitable for in the methods and compositions described herein.

Preparations comprising glycan polymers can be generated using a non-enzymatic catalyst, e.g., the polymeric catalyst described in WO 2012/118767, "POLYMERIC ACID CATALYSTS AND USES THEREOF" or by other suitable methods. Other acid catalysts (e.g. solid catalysts) may be used. Methods to prepare the polymeric and solid-supported catalysts described herein can be found in WO 2014/031956, "POLYMERIC AND SOLID-SUPPORTED CATALYSTS, AND METHODS OF DIGESTING CELLULOSIC MATERIALS USING SUCH CATALYSTS." The glycans generated, e.g., by using the catalyst, for example as described in WO 2016/007778, "OLIGOSACCHARIDE COMPOSITIONS AND METHODS FOR PRODUCING THEREOF" are suitable for the methods and compositions described herein. All patent applications are incorporated herein by reference in their entirety.

In some embodiments, glycan polymers are made using solid-phase oligosaccharide synthesis, e.g., using a variety of protection groups to accomplish glycan synthesis. Exemplary methods are described in "Solid-Phase Oligosaccharide Synthesis and Combinatorial Carbohydrate Libraries", Peter H. Seeberger and Wilm-Christian Haase, American Chemical Society, 2000; and "Opportunities and challenges in synthetic oligosaccharide and glycoconjugate research", Thomas J. Boltje et al., Nat Chem. 2009 November 1; 1(8): 611-622.

In some embodiments, glycan polymers may be synthesized using an enzyme catalyst (e.g., a glycosidase or glycosyltransferase, either isolated or expressed in bacteria), such as described herein, to synthesize the glycans by a polymerization reaction that creates oligomers from individual glycan subunits that are added to the reaction. Exemplary methods are described in "Synthesis and Purification of Galacto-Oligosaccharides: State of the Art", Carlos Vera et al., World J. Microbiol Biotechnol. 2016; 32:197; "Synthesis of Novel Bioactive Lactose-Derived Oligosaccharides by Microbial Glycoside Hydrolases", Marina Diez-Municio et al., Microbiol Biotechnol. 2014; 7(4), 315-331; and "Methods of Improving Enzymatic Trans-Glycosylation for Synthesis of Human Milk Oligosaccharide Biomimetics", Birgitte Zeuner et al., J. Agric. Food Chem. 2014, 62, 9615-9631, WO 2005/003329 "NOVEL GALACTOOLIGOSACCHARIDE COMPOSITION AND THE PREPARATION THEREOF", all of which are hereby incorporated by reference.

In some embodiments, glycan preparations may be prepared using glycan polymers, such as starch and other fibers, such as dietary fibers (such as described herein) and subject them to a catalyst (e.g., an acid catalyst, a solid or polymeric catalyst, an enzyme catalyst) to change one or more glycan (or fiber) properties, e.g., degree of polymerization (e.g. depolymerization), degree of branching (e.g. debranching), or glycosidic bond distribution (e.g., by adding new types of glycosidic bonds or removing existing bonds). An exemplary method for corn syrup is described in U.S. Patent Publication No. 2016/0007642, Example 101, which is incorporated by reference. Other methods, such as those used for preparation of resistant starch (e.g., described in M.G. Sajilata et al., "Resistant Starch - A Review," Comprehensive Reviews in Food Science and Food Safety - Vol. 5, 2006, and U.S. Patent Publication No. 2006/0257977, "Slowly digestible starch"), such as, e.g., heat treatment, enzymic treatment, chemical treatment, or a combination thereof, may be used to produce glycan preparations described herein.

**Glycan subunits**

The present invention features methods of making or methods of manufacturing a preparation of a glycan polymer that is a substrate for a gut microbe (e.g., a human gut microbe). The starting materials for said methods are glycan subunits that comprise sugar monomers (e.g., monosaccharides), sugar dimers (e.g., disaccharides), sugar trimers (e.g., trisaccharides), or combinations thereof.

The starting material may comprise a furanose sugar or a pyranose sugar. In some embodiments, the starting material comprises a tetrose, a pentose, a hexose, or a heptose. In some embodiments, the starting material comprises glucose, galactose, arabinose, mannose, fructose, xylose, fucose, and rhamnose. The glycan subunit starting materials may be in either their L- or D-form, in the alpha or beta configuration, and/or a deoxy-form, where applicable, and any combination thereof.

The glycan subunits used in the methods described herein may include a monosaccharide, such as a C5 monosaccharide or a C6 monosaccharide. In some embodiments, the monosaccharide is a C5 monosaccharide. In some embodiments, the monosaccharide is a C6 monosaccharide. The glycan subunits may include a disaccharide, such as a disaccharide comprising a C5 monosaccharide or a C6 monosaccharide. In some embodiments, the disaccharide comprises a C5 monosaccharide. In some embodiments, the disaccharide comprises two C5 monosaccharides. In some embodiments, the disaccharide comprises a C6 monosaccharide. In some embodiments, the disaccharide comprises two C6 monosaccharides. In some embodiments, the disaccharide comprises one of a C5 monosaccharide and one of a C6 monosaccharide.

The glycan subunit starting material used herein may be a monosaccharide selected from glycolaldehyde, glyceraldehyde, dihydroxyacetone, erythrose, threose, erythulose, arabinose, lyxose, ribose, xylose, ribulose, xylulose, allose, altrose, galactose, glucose, gulose, idose, mannose, talose, fructose, psicose, sorbose, tagatose, fucose, fuculose, rhamnose, mannoheptulose, sedoheptulose, neuraminic acid, N-acetylneuraminic acid, N-acetylgalactosamine, N-acetylglucosamine, fructosamine, galactosamine, glucosamine, sorbitol, glycerol, erythritol, threitol, arabitol, xylitol, mannitol, sorbitol, galactitol, fucitol, and lactic acid.

The glycan subunit starting material used herein may be a disaccharide or larger subunit selected from acarviosin, N-acetyllactosamine, allolactose, cellobiose, chitobiose, glactose-alpha-1,3-galactose, gentiobiose, isomalt, isomaltose, isomaltulose, kojibiose, lactitol, lactobionic acid, lactose, lactulose, laminaribiose, maltitol, maltose, mannobiose, melibiose, melibiulose, neohesperidose, nigerose, robinose, rutinose, sambubiose, sophorose, sucralose, sucrose, sucrose acetate isobutyrate, sucrose octaacetate, trehalose, turanose, vicianose, and xylobiose.

In some embodiments, the glycan subunit is an unactivated glycan subunit. In some embodiments, the glycan subunit is an activated glycan subunit, e.g., activated with a nucleoside, nucleotide (e.g., UTP, UDP, UMP, GTP, GDP, GMP, ATP, ADP, AMP, CTP, CDP, CMP), or phosphate group. In some embodiments, the glycan subunit is a UDP sugar or a UMP sugar.

In some embodiments, the glycan subunit is substituted or derivatized with an acetyl group, acetate ester, sulfate half-ester, phosphate ester, or a pyruvyl cyclic acetal group, or has been otherwise derivatized at, e.g., at one or more hydroxyl groups or amine groups.

In some embodiments, the glycan subunit comprises an amino sugar, deoxy sugar, imino sugar, sugar acid, or sugar alcohol. Exemplary amino sugars include acarbose, N-acetylemannosamine, N-acetylmuramic acid, N-acetylneuraminic acid, N-acetyletalosaminuronic acid, arabinopyranosyl-N-methyl-N-nitrosourea, D-fructose-L-histidine, N-glycolyneuraminic acid, ketosamine, kidamycin, mannosamine, 1B-methylseleno-N-acetyl-D-galactosamine, muramic acid, muramyl dipeptide, phosphoribosylamine, PUGNAc, sialyl-Lewis A, sialyl-Lewis X, validamycin, voglibose, N-acetylgalactosamine, N-acetylglucosamine, aspartylglucosamine, bacillithiol, daunosamine, desosamine, fructosamine, galactosamine, glucosamine, meglumine, and perosamine. Exemplary deoxy sugars include 1-5-ahydroglucitol, cladinose, colitose, 2-deoxy-D-glucose, 3-deoxyglucasone, deoxyribose, dideoxynucleotide, digitalose, fludeooxyglucose, sarmentose, and sulfoquinovose. Exemplary imino sugars inclue castanospermine, 1-deoxynojirimycin, iminosugar, miglitol, miglustat, and swainsonine. Exemplary sugar acids include N-acetylneuraminic acid, N-acetyltalosamnuronic acid, aldaric acid, aldonic acid, 3-deoxy-D-manno-oct-2-ulosonic acid, glucuronic acid, glucosaminuronic acid, glyceric acid, N-glycolylneuraminic acid, iduronic acid, isosaccharinic acid, pangamic acid, sialic acid, threonic acid, ulosonic acid, uronic acid, xylonic acid, gluconic acid, ascorbic acid, ketodeoxyoctulosonic acid, galacturonic acid, galactosaminuronic acid, mannuronic acid, mannosaminuronic acid, tartaric acid, mucic acid, saccharic acid, lactic acid, oxalic acid, succinic acid, hexanoic acid, fumaric acid, maleic acid, butyric acid, citric acid, glucosaminic acid, malic acid, succinamic acid, sebacic acid, and capric acid. Exemplary sugar alcohols include methanol, ethylene glycol, glycerol, erythritol, threitol, arabitol, ribitol, xylitol, mannitol, sorbitol, galactitol, iditol, volemitol, fucitol, inositol, maltotritol, maltotetraitol, and polyglycitol. In some embodiments, the glycan subunit starting material is a salt (e.g., a pharmaceutically acceptable salt), such as, e.g., a hydrochlorate, hydroiodate, hydrobromate, phosphate, sulfate, methanesulfate, acetate, formate, tartrate, malate, citrate, succinate, lactate, gluconate, pyruvate, fumarate, propionate, aspartate, glutamate, benzoate, ascorbate salt.

A glycan subunit used in a method described herein may be obtained from any commercially known source, or produced according to any known method in the art. In some embodiments, hydrolysis may be used to generate the constituent monosaccharides or oligosaccharides that are suitable to produce the glycans described herein. Glycan units, such as e.g. monosaccharides, may exist in many different forms, for example, conformers, cyclic forms, acyclic forms, stereoisomers, tautomers, anomers, and isomers.

**Making glycan polymers using a non-enzymatic, polymeric catalyst**

*Reaction conditions*

In some embodiments, the glycan unit and catalyst (e.g., polymeric catalyst or solid-supported catalyst) are allowed to react for at least 1 hour, at least 2 hours, at least 3 hours, at least 4 hours, at least 6 hours, at least 8 hours, at least 16 hours, at least 24 hours, at least 36 hours, or at least 48 hours; or between 1-24 hours, between 2-12 hours, between 3-6 hours, between 1-96 hours, between 12-72 hours, or between 12-48 hours.

In some embodiments, the degree of polymerization of the one or more oligosaccharides produced according to the methods described herein can be regulated by the reaction time. For example, in some embodiments, the degree of polymerization of the one or more oligosaccharides is increased by increasing the reaction time, while in other embodiments, the degree of polymerization of the one or more oligosaccharides is decreased by decreasing the reaction time.

*Reaction temperature*

In some embodiments, the reaction temperature is maintained in the range of about 25°C to about 150°C. In certain embodiments, the temperature is from about 30°C to about 125°C, about 60°C to about 120°C, about 80°C to about 115°C, about 90°C to about 110°C, about 95°C to about 105°C, or about 100°C to 110°C.

*Amount of Glycan Units*

The amount of the glycan unit used in the methods described herein relative to the amount solvent used may affect the rate of reaction and yield. The amount of the glycan unit used may be characterized by the dry solids content. In certain embodiments, dry solids content refers to the total solids of a slurry as a percentage on a dry weight basis. In some embodiments, the dry solids content of the glycan unit is between about 5 wt% to about 95 wt %, between about 10 wt% to about 80 wt %, between about 15 wt %, to about 75 wt %, or between about 15 wt %, to about 50 wt %.

*Amount of catalyst*

The amount of the catalyst used in the methods described herein may depend on several factors including, for example, the selection of the type of glycan unit, the concentration of the glycan unit, and the reaction conditions (e.g., temperature, time, and pH). In some embodiments, the weight ratio of the catalyst to the glycan unit is about 0.01 g/g to about 50 g/g, about 0.01 g/g to about 5 g/g, about 0.05 g/g to about 1.0 g/g, about 0.05 g/g to about 0.5 g/g, about 0.05 g/g to about 0.2 g/g, or about 0.1 g/g to about 0.2 g/g.

*Solvent*

In certain embodiments, the methods of using the catalyst are carried out in an aqueous environment. One suitable aqueous solvent is water, which may be obtained from various sources. Generally, water sources with lower concentrations of ionic species (e.g., salts of sodium, phosphorous, ammonium, or magnesium) are preferable, as such ionic species may reduce effectiveness of the catalyst. In some embodiments where the aqueous solvent is water, the water has a resistivity of at least 0.1 megaohm-centimeters, of at least 1 megaohm-centimeters, of at least 2 megaohm-centimeters, of at least 5 megaohm-centimeters, or of at least 10 megaohm-centimeters.

*Water content*

Moreover, as the dehydration reaction of the methods progresses, water is produced with each coupling of the one or more glycan units. In certain embodiments, the methods described herein may further include monitoring the amount of water present in the reaction mixture and/or the ratio of water to monomer or catalyst over a period of time. In some embodiments, the method further includes removing at least a portion of water produced in the reaction mixture (e.g., by removing at least about any of 10%, 20%, 30%, 40%, 50%, 60%, 70%, 80%, 90%, 95%, 97%, 99%, or 100%, such as by vacuum filtration). It should be understood, however, that the amount of water to monomer may be adjusted based on the reaction conditions and specific catalyst used.

Any method known in the art may be used to remove water in the reaction mixture, including, for example, by vacuum filtration, vacuum distillation, heating, and/or evaporation. In some embodiments, the method comprises including water in the reaction mixture.

In some aspects, provided herein are methods of producing an oligosaccharide composition, by: combining a glycan unit and a catalyst having acidic and ionic moieties to form a reaction mixture, wherein water is produced in the reaction mixture; and removing at least a portion of the water produced in the reaction mixture. In certain variations, at least a portion of water is removed to maintain a water content in the reaction mixture of less than 99%, less than 90%, less than 80%, less than 70%, less than 60%, less than 50%, less than 40%, less than 30%, less than 20%, less than 10%, less than 5%, or less than 1% by weight.

In some embodiments, the degree of polymerization of the one or more oligosaccharides produced according to the methods described herein can be regulated by adjusting or controlling the concentration of water present in the reaction mixture. For example, in some embodiments, the degree of polymerization of the one or more oligosaccharides is increased by decreasing the water concentration, while in other embodiments, the degree of polymerization of the one or more oligosaccharides is decreased by increasing the water concentration. In some embodiments, the water content of the reaction is adjusted during the reaction to regulate the degree of polymerization of the one or more oligosaccharides produced.

In one example, to a round bottom flask equipped with an overhead stirrer and a jacketed short-path condenser one or more mono-, dimer-, trimer or other oligosaccharides may be added along with 1-50% (1-10%, 1-20%, 1-30%, 1-40%, 1-60%, 1-70%) by dry weight of one or more of the catalysts described herein. Water or another compatible solvent (0.1-5 equiv, 1-5 equiv, 1-4 equiv, 0.1-4 equiv) may be added to the dry mixture and the slurry can be combined at slow speed (e.g. 10-100 rpm, 50-200 rpm, 100-200 rpm) using a paddle sized to match the contours of the selected round bottom flask as closely as possible. The mixture is heated to 70-180° C (70-160° C, 75-165° C, 80-160° C) under 10-1000 mbar vacuum pressure. The reaction may be stirred for 30 minutes to 6 hours, constantly removing water from the reaction. Reaction progress can be monitored by HPLC. The solid mass obtained by the process can be dissolved in a volume of water sufficient to create a solution of approximately 50 Brix (grams sugar per 100 g solution). Once dissolution is complete, the solid catalyst can be removed by filtration and the oligomer solution can be concentrated to approximately 50-75 Brix, e.g., by rotary evaporation. Optionally, an organic solvent can be used and water immiscible solvents can be removed by biphasic extraction and water miscible solvents can be removed, e.g., by rotary evaporation concomitant to the concentration step.

**Making glycan polymers using a glycosidase enzyme molecule**

_Reaction conditions_

A glycan polymer produced using the methods described herein may be generated by condensation (e.g., reverse hydrolysis) and/or transglycosylation of a glycosidic bond catalyzed by a glycosidase enzyme molecule (e.g., a hydrolase, transferase, or lyase). In some embodiments, a characteristic of a glycan polymer produced according to the methods described herein can be regulated by a reaction condition, e.g., reaction time, reaction temperature, concentration or amount of a glycan subunit, concentration or amount of a glycosidase enzyme molecule , solvent, or an additional processing step, e.g., as described herein. In some embodiments, the reaction conditions of the methods described herein reflect physiological conditions, e.g., pH between 5 and 7.5 and a temperature between 35 °C and 60 °C. In some embodiments, the reaction conditions of a method described herein deviate from physiological conditions.

_Reaction time_

In some embodiments, the glycosidase enzyme molecule and a starting material (e.g., a glycan subunit) are allowed to react for at least 5 minutes, at least 10 minutes, at least 15 minutes, at least 30 minutes, at least 1 hour, at least 2 hours, at least 3 hours, at least 4 hours, at least 6 hours, at least 8 hours, at least 16 hours, at least 24 hours, at least 36 hours, or at least 48 hours. In some embodiments, the glycosidase enzyme molecule and a starting material (e.g., a glycan subunit) are allowed to react between 1-24 hours, between 2-12 hours, between 3-6 hours, between 1-96 hours, between 12-72 hours, or between 12-48 hours. In some embodiments, the degree of polymerization (DP) of a glycan polymer produced according to the methods described herein can be regulated by the reaction time. For example, in some embodiments, the degree of polymerization of a glycan polymer is increased by increasing the reaction time, while in other embodiments, the degree of polymerization of a glycan polymer is decreased by decreasing the reaction time.

*Reaction temperature*

In some embodiments, the reaction temperature is maintained in the range of about 4°C to about 150°C. In certain embodiments, the temperature is from about 4°C to about 30°C, about 4°C to about 125°C, about 30°C to about 125°C, about 60°C to about 120°C, about 80°C to about 115°C, about 90°C to about 110°C, about 95°C to about 105°C, or about 100°C to 110°C. In some embodiments, the reaction temperature is room temperature (e.g., about 25°C). In some embodiments, the reaction temperature is physiological temperature (e.g., about 30°C). In some embodiments, the reaction temperature is about 60°C.

In some embodiments, the reaction is slowed or substantially stopped after a period of time by increasing the temperature, e.g., through denaturation of the enzyme. In some embodiments, the reaction is slowed or substantially stopped by increasing the temperature to greater than about 45°C, about 50°C, about 60°C about 70°C, about 80°C, about 90°C, about 100°C, about 110°C, or greater.

*Concentration or amount of a glycan subunit*

The concentration or amount of a glycan subunit used in the methods described herein relative to the amount solvent used may affect the rate of reaction and yield. In some embodiments, the concentration or amount of a glycan subunit is about 10 mg/mL, about 25 mg/mL, about 50 mg/mL, about 75 mg/mL, about 100 mg/mL, about 200 mg/mL, about 300 mg/mL, about 400 mg/mL, about 500 mg/mL, about 750 mg/mL, about 1 g/mL, or more.

The amount of the glycan subunit used may be characterized by the dry solids content. In certain embodiments, dry solids content refers to the total solids of a slurry as a percentage on a dry weight basis. In some embodiments, the dry solids content of the glycan subunit is between about 5 wt% to about 95 wt %, between about 10 wt% to about 80 wt %, between about 15 wt %, to about 75 wt %, or between about 15 wt %, to about 50 wt %.

*Concentration or amount of a glycosidase enzyme molecule*

The concentration or amount of the glycan enzyme molecule used in the methods described herein may depend on several factors including, for example, the selection of the type of glycan subunit, the concentration of the glycan subunit, and the reaction conditions (e.g., temperature, time, and pH). In some embodiments, the concentration or amount of the glycosidase enzyme molecule is about 0.1 U/mL, about 0.5 U/mL, about 1 U/mL, about 5 U/mL, about 10 U/mL, about 25 U/mL, about 50 U/mL, or higher. In some embodiments, the concentration or amount of the glycosidase enzyme molecule is between 0.1-5 U/mL, between 1-25 U/mL, or 1-50 U/mL. In some embodiments, the weight ratio of the glycosidase enzyme molecule to the glycan subunit is about 0.01 g/g to about 50 g/g, about 0.01 g/g to about 5 g/g, about 0.05 g/g to about 1.0 g/g, about 0.05 g/g to about 0.5 g/g, about 0.05 g/g to about 0.2 g/g, or about 0.1 g/g to about 0.2 g/g.

*Solvent*

In some embodiments, the solvent of the reaction is a biocompatible solvent. In certain embodiments, the solvent of the reaction is an aqueous solvent, e.g., water or a water mixture. In some embodiments, the solvent of the rection is water or a mixture of water and a miscible solvent such as acetone, ethanol, isopropanol, polyethylene glycol, t-butanol or another solvent. In some embodiments, the solvent of the reaction is an organic solvent (e.g., a pure organic solvent).

A solvent may be added to the reaction mixture in order to increase the reaction rate or overall reaction yield, e.g., through increasing the accessibility of a glycosidase enzyme molecule to a glycan subunit. Exemplary solvents include an organic solvent such as DMSO, and toluene.

*Addition reaction components*

The reaction mixture may comprise an additional component such as a salt, a detergent, a metal, a chelator, an acid, a base, a cofactor, a coenzyme, a vitamin, an amino acid, a prosthetic group, a nucleoside, a nucleotide, or any combination thereof. In some embodiments, the reaction mixture comprises a cofactor or coenzyme such as $NAD^+$, NADH, $NADP^+$, NADPH, FAD, FADH, coenzyme A, biotin, pyridoxal phosphate, or methylcobalamin. In some embodiments, inclusion of an additional compoeent improves the reaction yield, enzyme turnover rate, enzyme stability, glycan subunit stability, glycan polymer stability, or any combination thereof.

*Glycosidase enzymes*

Described herein are methods of making preparations of glycan polymers that are substrates for a glycosidase enzyme, e.g., a glycosidase enzyme present in a human gut microbe. In their natural environments, e.g., expressed by a gut bacterium in the gut of a subject, glycosidases use glycan polymers as substrates, e.g., they recognize specific glycan polymers and hydrolyze glycosidic bonds in the glycan polymer. This hydrolysis may lead to the liberation of monomers or dimers from the glycan polymer, a shortening of the glycan polymer, and/or a debranching (e.g. the removal of a glycosidic branching point of the glycan polymer). Glycosidase action provides a microbe with glycan breakdown products that it can convert to energy. This process is referred to glycan fermentation. Many glycosidases are specific, e.g. they have recognition motifs at the end of glycan chain (e.g., exo-glycosidases) or within (e.g. endo-glycosidases), they may recognize specific sugars or sugar combinations (e.g. glu-glu or glu-gal) and may further be selective in stereo- and/or regio-chemistry (e.g. recognition of alpha versus beta glycosidic bonds, and/or 1->2 versus 1->3 versus 1->6 linkages). Some glycosidase enzymes are more promiscuous, having a wider variety of glycan polymer substrates.

In artificial environments and under suitable conditions, glycosidase enzymes can produce glycan polymers, e.g. by condensation reaction and/or transglycosylation reactions. The glycan polymers that are produced can have a higher degree of polymerization than the imputs, can exhibit branching, and stereo- and/or regiochemical variety (eith respect to alpha-beta glycosidic bonds and linkages. Exemplary glycosidase enzymes include hydrolases, transferases, or lyases. A glycosidase enzyme may be characterized in a variety of ways, such as by its sequence, size, or function. In some embodiments, a glycosidase enzyme is associated with a bacterium from a particular taxa. In some embodiments, a glycosidase enzyme has a CAZy family designation (i.e., the family designation provided by the Carbohydrate Active enZYme database (http://www.cazy.org/)), e.g., glycosylhydrolase (GH) family or glycosyltransferase (GT) family, based on analysis of genomic, structural, and biochemical information. In some embodiments, a glycosidase enzyme (e.g. a naturally occurring glycosidase enzyme, e.g., expressed by a gut microbe) is a glycosidase enzyme molecule (e.g. a glycosidase used in the methods of making a glycan polymer describe herein). In some embodiments, the glycosidase enzyme molecule is 80%, 85%, 90%, 95%, 97%, 98% 99% or 100% identical to the glycosidase enzyme (e.g. by DNA sequence, RNA sequence or amino acid sequence). In other embodiments, the glycosidase enzyme molecule comprises a deletion, additional sequence, point mutation, conservative or non-conservative amino acid changes, codon optimization, purification tags, folding/stability promoting mutations, etc. compared to the glycosidase enzyme. In some embodiments, the glycosidase enzyme is a member of a GH CAZY family. In some embodiments, the glycosidase enzyme is a member of a GT CAZY family. In some embodiments, the glycosidase enzyme molecule is related to (or derivatized from) the glycosidase enzyme having one or more sequence (e.g. DNA, RNA or amino acid sequence) modifications, such as those described herein.

In some embodiments, the glycosidase enzyme or glycosidase enzyme molecule is present in a human gut microbe. The glycosidase enzyme may be isolated from the microbe. In some embodiments, the glycosidases are present in a microbial supernatant, present in a microbial extract, present in a microbial cell mass, or are isolated to essential purity (e.g. essentially pure enzymatic fraction). In some embodiments, the glycosidase enzyme is sourced from human gut microbe. In some embodiments, the glycosidase enzyme is sourced from a yeast, a fungus, or a bacterium. In one embodiment, the glycosidase enzyme is sourced from a bacterium, such as a human gut bacterium. In some embodiments, the bacterial taxa is one of Actinobacteria, Bacteroidetes, Firmicutes, Fusobacteria, Spirochaetes, Synergistetes, Tenericutes, Proteobacteria, Verrucomicrobia, Euroarchaeota, e.g., a bacterial taxa described in Table 2. In some embodiments, the human gut microbe is a species with the bacterial taxa Actinobacteria. In some embodiments, the human gut microbe is a species with the bacterial taxa Bacteroidetes. In some embodiments, the human gut microbe is a species with the bacterial taxa Firmicutes. In some embodiments, the human gut microbe is a species with the bacterial taxa Fusobacteria. In some embodiments, the human gut microbe is a species with the bacterial taxa Spirochaetes. In some embodiments, the human gut microbe is a species with the bacterial taxa Synergistetes. In some embodiments, the human gut microbe is a species with the bacterial taxa Tenericutes. In some embodiments, the human gut microbe is a species with the bacterial taxa Proteobacteria. In some embodiments, the human gut microbe is a species with the bacterial taxa Verrucomicrobia. In some embodiments, the human gut microbe is a species with the bacterial taxa Euroarchaeota. In some embodiments, the human gut microbe is other than a Bifidobacterium or a Lactobacillus. In some embodiments, the glycan polymer (e.g., produced by a method described herein) is a substrate for a human gut microbe glycosidase enzyme from a certain CAZy family (e.g., a glycosylhydrolase (GH) family or a glycosyltransferase (GT) family). In some embodiments, the glycan polymer is a substrate for a human gut microbe glycosidase enzyme from a certain glycosylhydrolase (GH) family (e.g. one of GH1 to GH135) or glycosyltransferase (GT) family (e.g. one of GT1 to GT101). In some embodiments, the glycan polymer preparations are selected to be substrates for a human gut microbe with a particular glycosidase profile (e.g. it expresses (or harbors in its genome) one or more glycosidase genes, e.g. from one or more CAZy families). In some embodiments, glycosidase enzyme molecules are used in the methods described herein to produce glycan polymers that comprise functions of one or more of those of the glycosidase enzymes present in the particular microbe (or group of microbes). In some embodiments, the glycosidase enzyme molecule comprises the same functions as the glcosidase enzyme (e.g., the enzyme present in the gut microbe). In some embodiments, the glycosidase enzyme molecule has structural similarity or a certain degree of sequence similarity with the glycosidase enzyme. A glycosidase enzyme molecule may be generated by any method known in the art, e.g., using standard cloning, genetics, protein expression, protein purification, or protein processing techniques.

Glycosidase enzyme molecules suitable for the methods of making glycan polymers described herein can be selected based on the basis of their glycosidase enzyme counterparts that are present in a microbe and thus the glycan polymer or prearation thereof can be tailored to the glycoidase enzyme (glycosidase enzyme profile) of the microbe as tailored substrates.

In some embodiments, the glycan polymer (e.g., produced by a method described herein) is a substrate for a human gut microbe glycosidase enzyme selected from GT5, GH94, GH13.9, GH13.39, GH13.36, GH113.0 and GH112 CAZy families. In some embodiments, the glycan polymer is a substrate for a human gut microbe glycosidase enzyme selected from GT2, GT4, GT5, GT35, GT51, GH1, GH2, GH3, GH4, GH13, GH13 subfamily 9, GH13 subfamily31, GH18, GH23, GH25, GH28, GH31, GH32, GH36, GH51, GH73, GH77, and GH94 CAZy families. In some embodiments, the glycan polymer is a substrate for a human gut microbe glycosidase enzyme selected from GT11, GT10, GH92, GH51, GH35, GH29, GH28, GH20, GH130, GH13 subfamily 8, and GH13 subfamily14 CAZy families. In some embodiments, the glycan polymer is a substrate for a human gut microbe glycosidase enzyme selected from GT2, GT4, GH2, GH23, GH3, GT8, GT51, GT9, GH1, GH92, GH73, GH31, GH20, GH28, GT25, GT28, GT35, GH18, GH13, GH36, GH97, GH105, GH25, GH4, GH32, GH78, GH29, GH51, GT10, and GH77 CAZy families. In some embodiments, the glycan polymer is a substrate for a human gut microbe glycosidase enzyme selected from GT3, GH97, GH43 subfamily24, GH27, GH133, GH13 subfamily 8, and GH13 CAZy families. In some embodiments, the glycan polymer is a substrate for a human gut microbe glycosidase enzyme selected from GT2, GT4, GH2, GH23, GH3, GT51, GH1, GT8, GH92, GT9, GH73, GH31, GH20, GH28, GT35, GT28, GH18, GH13, GH97, GH25, GH36, GH4, GH105, GH32, GH78, GH29, GT25, GH51, GH77, GH88, GH24 CAZy families.

In some embodiments, the glycosidase enzyme or glycosidase enzyme molecule is other than one of GH1, GH2, GH3, GH4, GH5, GH8, GH9, GH10, GH11, GH12, GH13, GH14, GH16, GH26, GH28, GH30, GH31, GH32, GH35, GH42, GH43, GH44, GH50, GH51, GH57, GH62, GH63, GH68, GH70, GH97, GH100, GH116, GH119, or GH122 CAZy family.

In some embodiments, the method described herein further comprises identification of a glycosidase profile (e.g. of CAZy family (e.g, a GT family or GH family)) of a particular microbe in silico. In some embodiments, the identification of a glycosidase profile is carried out according the methods of Examples 11-15. For example, a sequenced genome from an array of commensal bacterial species isolated from healthy human gut microbiomes, e.g., as a part of the Human Microbiome Project, may be predicted for their ability to modulate a metabolite, e.g., produce butyrate, convert urea to ammonia through urease, or convert choline to TMA.

In some embodiments, the glycan polymer is a substrate for a glycosidase enzyme present in a microbe (e.g. a human gut microbe) that modulates the level of (e.g., produces) a microbial metabolite. Exemplary metabolites include formic acid, acetic acid, propionic acid, butryic acid, isobutyric acid, valeric acid, isovaleric acid, ascorbic acid, lactic acid, tryptophan, serotonin, indole, succinic acid, trimethylamine (TMA), TMAO (trimethylamine N-oxide), deoxycholic acid, ethyphenyl sulfate, acetylaldehyde, hydrogen peroxide, ammonia, bile acids, lipopolysaccharide (LPS), and/or butanedione. In some embodiments, the metabolite is butyric acid (e.g., butyrate), trimethylamine (TMA) or ammonia. In some embodiments, the metabolite is butyric acid (e.g., butyrate). In some embodiments, the metabolite is acetic acid (e.g., acetate). In some embodiments, the metabolite is propionic acid (e.g., propionate). In some embodiments, the metabolite is trimethylamine (TMA). In some embodiments, the metabolite is ammonia. In some embodiments, the metabolite is lipopolysaccharide (LPS). In some embodiments, the metabolite is bile acid (e.g. a secondary bile acid). In some embodiments, a substantial increase or decrease in a metabolite may be detected. In some embodiments, the glycosidase enzyme or glycosidase enzyme molecule is other than alpha- or beta-galactosidase, alpha- or beta-glucosidase, alpha- or beta-xylosidase, alpha- or beta-mannosidase, or alpha- or beta-fructofuranosidase. In some embodiments, the glycosidase enzyme or the glycosidase enzyme molecule is other than alpha- or beta-galactosidase.

*Methods of generating glycosidase enzyme molecules*

Glycosidase enzyme molecules can be produced by expression in recombinant host cells, but also by other methods such as in vitro transcription and translation and chemical synthesis. For cellular expression, one or more nucleic acids (e.g., cDNA or genomic DNA) encoding a glycosidase enzyme molecule may be inserted into a replicable vector for cloning or for expression. The vector may, for example, be a plasmid, cosmid, viral genome, phagemid, phage genome, or other autonomously replicating sequence. The appropriate coding nucleic acid sequence may be inserted into the vector by a variety of procedures. For example, appropriate restriction endonuclease sites can be engineered (e.g., using PCR). Then restriction digestion and ligation can be used to insert the coding nucleic acid sequence at an appropriate location. Vector components generally include one or more of an origin of replication, one or more marker genes, an enhancer element, a promoter, and a transcription termination sequence.

The glycosidase enzyme molecule may be produced recombinantly optionally by fusion to one or more other components, such as a signal sequence, an epitope or purification moiety, or a label.

For bacterial expression, the glycosidase enzyme molecule can be produced with or without a signal sequence. For example, it can be produced within cells so that it accumulates in inclusion bodies, or in the soluble fraction. It can also be secreted, e.g., by addition of a prokaryotic signal sequence, e.g., an appropriate leader sequence. Exemplary bacterial host cells for expression include any transformable E. coli K-12 strain (such as E. coli BL21, C600, ATCC 23724; E. coli HB101 NRRLB-11371, ATCC-33694; E. coli MM294 ATCC-33625; E. coli W3110 ATCC-27325), strains of B. subtilis, Pseudomonas, and other bacilli. In some embodiments, the bacterial host cell is selected from a proteolytic taxa, e.g., a taxa expressing few or none endogenous glycosidase enzymes.

The glycosidase enzyme molecules can be expressed in a yeast host cell, e.g., Saccharomyces cerevisiae, Schizosaccharomyces pombe, Hanseula, or Pichia pastoris. For yeast expression, the glycosidase enzyme molecules can also be produced intracellularly or by secretion, e.g., using the yeast invertase leader or alpha factor leader (including Saccharomyces and Kluyveromyces forms), or the acid phosphatase leader, or the C. albicans glucoamylase leader (EP 362,179 published 4 Apr. 1990).

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria; the 2□ plasmid origin is suitable for yeast.

Expression and cloning vectors typically contain a selection gene or marker. Typical selection genes encode proteins that (a) confer resistance to antibiotics or other toxins, e.g., ampicillin, neomycin, methotrexate, or tetracycline, (b) complement auxotrophic deficiencies (such as the URA3 marker in Saccharomyces), or (c) supply critical nutrients not available from complex media, e.g., the gene encoding D-alanine racemase for Bacilli.

Expression and cloning vectors usually contain a promoter operably linked to the nucleic acid sequence encoding the glycosidase enzyme molecule to direct mRNA synthesis. Exemplary promoters suitable for use with prokaryotic hosts include the β-lactamase and lactose promoter systems (Chang et al., Nature, 275:615 (1978); Goeddel et al., Nature, 281:544 (1979)), alkaline phosphatase, a tryptophan (trp) promoter system (Goeddel, Nucleic Acids Res., 8:4057 (1980); EP 36,776), and hybrid promoters such as the tac promoter (deBoer et al., Proc. Natl. Acad. Sci. USA, 80:21-25 (1983)). Promoters for use in bacterial systems can also contain an appropriately located Shine-Dalgarno sequence. The T7 polymerase system can also be used to drive expression of a nucleic acid coding sequence placed under control of the T7 promoter.

Still other methods, vectors, and host cells suitable for adaptation to the synthesis of glycosidase enzyme molecules in recombinant cells are described in Molecular Cloning: A Laboratory Manual, Third Ed., Sambrook et al. (eds.), Cold Spring Harbor Press, (2001) (ISBN: 0879695773).

Once expressed in cells, glycosidase enzyme moleucles can be recovered from culture medium, inclusion bodies, or cell lysates. Cells can be disrupted by various physical or chemical means, such as freeze-thaw cycling, sonication, mechanical disruption, or cell lysing agents (e.g., detergents).

Glycosidase enzyme molecules can be purified from other cell proteins or polypeptides that can be found in cell lysates or in the cell medium. Various methods of protein purification may be employed and such methods are known in the art and described for example in Deutscher, Methods in Enzymology, 182 (1990); and Scopes, Protein Purification: Principles and Practice,

Springer-Verlag, New York (2010) (ISBN: 1441928332). Exemplary of purification procedures include: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and affinity columns (e.g., metal chelating columns to bind epitope-tagged forms of the protein and columns with various ligands to bind any purification moiety that is associated with the glycosidase enzyme). A purification method can include a combination of two different ion-exchange chromatography steps, e.g., cation exchange chromatograph followed by anion exchange chromatography, or vice versa. Glycosidase enzyme molecules can be eluted from ion exchange resin by a variety of methods include salt and/or pH gradients or steps. In some embodiments, the glycosidase enzyme molecules includes a purification moiety (such as epitope tags and affinity handles). Such moieties can be used for affinity chromatography and can be optionally removed by proteolytic cleavage.

Anionic or cationic substituents may be attached to matrices in order to form anionic or cationic supports for chromatography. Anionic exchange substituents include diethylaminoethyl (DEAE), quaternary aminoethyl (QAE) and quaternary amine (Q) groups. Cationic substitutents include carboxymethyl (CM), sulfoethyl (SE), sulfopropyl (SP), phosphate (P) and sulfonate (S). Cellulose ion exchange resins such as DE23, DE32, DE52, CM-23, CM-32 and CM-52 are available from Whatman Ltd. (Maidstone, Kent, U.K). SEPHADEX™ and other cross-linked ion exchangers are also known. For example, DEAE-, QAE-, CM-, and SP-SEPHADEX™ and DEAE-, Q-, CM- and S-SEPHAROSE™ and SEPHAROSE™ Fast Flow are available from Pharmacia AB. DEAE and CM derivatized ethylene glycol-methacrylate copolymer such as TOYOPEARL DEAE-650S or M and TOYOPEARL CM-650S or M are available from Toso Haas Co. (Philadelphia, PA, USA).

A cation exchange surface is an ion exchange surface with covalently bound negatively charged ligands, and which thus has free cations for exchange with cations in a solution in contact with the surface. Exemplary surfaces include cation exchange resins, such as those wherein the covalently bound groups are carboxylate or sulfonate. Commercially available cation exchange resins include CMC-cellulose, SP-Sephadex™ and Fast S-Sepharose™ (Pharmacia).

An anion exchange surface is an ion exchange surface with covalently bound positively charged groups, such as quaternary amino groups. An exemplary anion exchange surface is an anion exchange resin, such as DEAE cellulose, TMAE, QAE Sephadex™ and Fast Q Sepharose™ (Pharmacia).

An exemplary purification scheme for a glycosidase enzyme molecules includes lysing E. coli cells in lysis buffer following by depth filtration. The material is then subject to cation exchange chromatography (CEX). The CEX eluate is then flowed over anion exchange media in an anion exchange chromatography (AEX) step. The AEX FT can be subject to a polishing step. Material can then be processed by ultrafiltration/diafiltration, e.g., to concentrate or desalt the material. Ultrafiltration/diafiltration membranes may be selected based on nominal molecular weight cut-off ("NMWCO") so as to retain the protein in the retentate, while allowing low molecular weight materials such as salts to pass into the filtrate. Any buffering solution or sterile water may be used during the final buffer exchange step, e.g., depending on the desired final pH and conductivity of the product.

A glycosidase enzyme molecule may comprise one or more conservative sequence modifications. Such conservative modifications include amino acid substitutions, additions and deletions. Modifications can be introduced by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a glycosidase enzyme can be replaced with other amino acid residues from the same side chain family and the altered glycosidase enzyme molecule can be tested using the functional assays described herein.

**Additional processing steps**

Optionally, the preparation may undergo additional processing steps. Additional processing steps may include, for example, purification steps. Purification steps may include, for example, separation, dilution, concentration, filtration, desalting or ion-exchange, chromatographic separation, or decolorization, or any combination thereof.

*Decolorization*

In some embodiments, the methods described herein further include a decolorization step. A glycan polymer produced may undergo a decolorization step using any method known in the art, including, for example, treatment with an absorbent, activated carbon, chromatography (e.g., using ion exchange resin), hydrogenation, and/or filtration (e.g., microfiltration).

In certain embodiments, a glycan polymer produced is contacted with a color-absorbing material at a particular temperature, at a particular concentration, and/or for a particular duration of time. In some embodiments, the mass of the color absorbing species contacted with a glycan polymer is less than 50% of the mass of the glycan polymer, less than 35% of the mass of the glycan polymer, less than 20% of the mass of the glycan polymer, less than 10% of the mass of the glycan polymer, less than 5% of the mass of the glycan polymer, less than 2% of the mass glycan polymer, or less than 1% of the mass of the glycan polymer.

In some embodiments, a glycan polymer is contacted with a color absorbing material. In certain embodiments, a glycan polymer is contacted with a color absorbing material for less than 10 hours, less than 5 hours, less than 1 hour, or less than 30 minutes. In a particular embodiment, a glycan polymer is contacted with a color absorbing material for 1 hour.

In certain embodiments, the glycan polymer is contacted with a color absorbing material at a temperature from 20 to 100 degrees Celsius, 30 to 80 degrees Celsius, 40 to 80 degrees Celsius, or 40 to 65 degrees Celsius. In a particular embodiment, the glycan polymer is contacted with a color absorbing material at a temperature of 50 degrees Celsius.

In certain embodiments, the color absorbing material is activated carbon. In one embodiment, the color absorbing material is powdered activated carbon. In other embodiments, the color absorbing material is an ion exchange resin. In one embodiment, the color absorbing material is a strong base cationic exchange resin in a chloride form. In another embodiment, the color absorbing material is cross-linked polystyrene. In yet another embodiment, the color absorbing material is cross-linked polyacrylate. In certain embodiments, the color absorbing material is Amberlite FPA91, Amberlite FPA98, Dowex 22, Dowex Marathon MSA, or Dowex Optipore SD-2.

*Ion-exchange/de-salling (demineralization)*

In some embodiments, the glycan polymer produced is contacted with a material to remove salts, minerals, and/or other ionic species. In certain embodiments, the glycan polymer is flowed through an anionic/cationic exchange column pair. In one embodiment, the anionic exchange column contains a weak base exchange resin in a hydroxide form and the cationic exchange column contains a strong acid exchange resin in a protonated form.

*Separation and concentration*

In some embodiments, the methods described herein further include isolating the glycan polymers produced. In certain variations, isolating glycan polymers comprises separating at least a portion of the glycan polymers from at least a portion of the glycosidase enzyme molecule, using any method known in the art, including, for example, centrifugation, filtration (e.g., vacuum filtration, membrane filtration), and gravity settling. In some embodiments, isolating the glycan polymers comprises separating at least a portion of the glycan polymers from at least a portion of any unreacted sugar, using any method known in the art, including, for example, filtration (e.g., membrane filtration), chromatography (e.g., chromatographic fractionation), differential solubility, and centrifugation (e.g., differential centrifugation).

In some embodiments, the methods described herein further include a concentration step. For example, in some embodiments, the isolated glycan polymer is subjected to evaporation (e.g., vacuum evaporation) to produce a concentrated glycan polymer preparation. In other embodiments, the isolated glycan polymer is subjected to a spray drying step to produce an oligosaccharide powder. In certain embodiments, the isolated glycan polymer is subjected to both an evaporation step and a spray drying step.

*Fractionation*

In some embodiments, the methods described herein further include a fractionation step. Glycan polymers prepared and purified may be subsequently separated by molecular weight using any method known in the art, including, for example, high-performance liquid chromatography, adsorption/desorption (e.g. low-pressure activated carbon chromatography), or filtration (for example, ultrafiltration or diafiltration).

In certain embodiments, produced glycan polymers are fractionated by adsorption onto a carbonaceous material and subsequent desorption of fractions by washing the material with mixtures of an organic solvent in water at a concentration of 1%, 5%, 10%, 20%, 50%, or 100%. In one embodiment, the adsorption material is activated charcoal. In another embodiment, the adsorption material is a mixture of activated charcoal and a bulking agent such as diatomaceous earth or Celite 545 in 5%, 10%, 20%, 30%, 40%, or 50% portion by volume or weight.

In further embodiments, produced glycan polymers are separated by passage through a high-performance liquid chromatography system. In certain variations, produced glycan polymers are separated by ion-affinity chromatography, hydrophilic interaction chromatography, or size-exclusion chromatography including gel-permeation and gel-filtration.

In other embodiments, low molecular weight materials are removed by filtration methods. In certain variations, low molecular weight materials may be removed by dialysis, ultrafiltration, diafiltration, or tangential flow filtration. In certain embodiments, the filtration is performed in static dialysis tube apparatus. In other embodiments, the filtration is performed in a dynamic flow filtration system. In other embodiments, the filtration is performed in centrifugal force-driven filtration cartridges.

Other processing steps may include any one of those described in the Examples herein. In some embodiments, yeast fermentation is used to remove unreacted constituents, e.g. sugar monomers or dimers, or reaction byproducts, such as sugar monomers.

Glycan preparation properties

Glycan may have any one or more of the characteristics and properties disclosed in WO2016/122889, WO2016/172657, WO 2016/007778, and WO2016/172658, each of which is incorporated herein by reference in its entirety, and any characteristics and properties disclosed herein.

The glycans produced by the methods described herein may comprise oligosaccharides. In some embodiments, the glycans comprise homo-oligosaccharides (or homoglycans), wherein all the monosaccharides in a polymer are of the same type.

In some embodiments, the glycans comprise hetero-oligosaccharides (or heteroglycans), wherein more than one type of monosaccharide is present in the polymer. In some embodiments, the glycans have one or more of the properties described herein. In some embodiments, the glycan preparation has one or more of the bulk properties described herein.

*Degree of polymerization (DP)*

In some embodiments, glycan polymer preparations are produced, e.g., using a method described herein, that are polydisperse, exhibiting a range of degrees of polymerization.

Optionally, the preparations may be fractionated, e.g. representing 60%, 65%, 70%, 75%, 80%, 85%, 90%, 95%, 97%, 98%, or greater than 98% short (about DP 1-2), medium (about DP3-10), long (about DP11-18), or very long (about DP>18) species. In one embodiment, a polydisperse, fractionated glycan polymer preparation is provided comprising at least 85%, 90%, or at least 95% medium-length species with a DP of about 3-10. In one embodiment, a polydisperse, fractionated glycan polymer preparation is provided comprising at least 85%, 90%, or at least 95% long-length species with a DP of about 11-18. In one embodiment, a polydisperse, fractionated glycan polymer preparation is provided comprising at least 85%, 90%, or at least 95% very long-length species with a DP of about 18-30.

Optionally, the preparations may be fractionated, e.g. representing 60%, 65%, 70%, 75%, 80%, 85%, 90%, 95%, 97%, 98%, or greater than 98% short (about DP1-2) or medium (about DP3-10) glycans in the preparation. Alternatively, or in addition to fractionation, the small DP fraction (e.g. monomers and dimers) are subjected to enzymatic fermentation, e.g. with suitable yeasts to break down these sugars. In one embodiment, a polydisperse, fractionated glycan polymer preparation is prepared using a method described herein, comprising at least 85%, 90%, or at least 95% of glycans with a DP of about 3-10.

In some embodiments, about 55%, 60%, 65%, 70%, 75%, 80%, 85%, 90%, 95%, or about 97% of the glycan polymers of the glycan preparation have a DP of at least DP3, DP4, DP5, DP6 or DP7. In some embodiments, about 55%, 60%, 65%, 70%, 75%, 80%, 85%, 90%, 95%, or about 97% of the glycan polymers of the glycan preparation have a DP from about DP3 to about DP10, from about DP3 to about DP8, from about DP3 to about DP6, from about DP3 to about DP5, from about DP3 to about DP4, from about DP2 to about DP4, from about DP2 to about DP5, from about DP2 to about DP6, from about DP2 to about DP8, or from about DP2 to about DP 10.

In some embodiments, less than 1%, 2%, 3%, 5%, 10%, 15%, 20%, 25%, 30%, 40%, or less than 50% of the glycan polymers of the glycan preparation have a DP of DP2 or less.

In some embodiments, about 55%, 60%, 65%, 70%, 75%, 80%, 85%, 90%, 95%, or about 97% of the glycan polymer preparation has a DP of between 2 and 25, between 3 and 25, between 4 and 25, between 5 and 25, between 6 and 25, between 7 and 25, between 8 and 25, between 9 and 25, between 10 and 25, between 2 and 30, between 3 and 30, between 4 and 30, between 5 and 30, between 6 and 30, between 7 and 30, between 8 and 30, between 9 and 30, or between 10 and 30. In one embodiment, the glycan polymer preparation has a degree of polymerization (DP) of at least 3 and less than 30 glycan units.

In some embodiments, about 55%, 60%, 65%, 70%, 75%, 80%, 85%, 90%, 95%, or about 97% of the glycan polymer preparation has a DP of at least 5 and less than 30 glycan units. In some embodiments, about 55%, 60%, 65%, 70%, 75%, 80%, 85%, 90%, 95%, or about 97% of the glycan polymer preparation has a DP of at least 8 and less than 30 glycan units. In some embodiments, about 55%, 60%, 65%, 70%, 75%, 80%, 85%, 90%, 95%, or about 97% of the glycan polymer preparation has a DP of at least 10 and less than 30 glycan units. In some embodiments, about 55%, 60%, 65%, 70%, 75%, 80%, 85%, 90%, 95%, or about 97% of the glycan polymer preparation has a DP of between 3, 4, 5, 6, 7, 8 and 10, 11, 12, 13, 14, 15, 16, 17, 18, 19, 20 glycan units. In some embodiments, about 55%, 60%, 65%, 70%, 75%, 80%, 85%, 90%, 95%, or about 97% of the glycan polymer preparation has a DP of between 10, 11, 12, 13, 14, 15, 16, 17, 18, 19 and 20, 21, 22, 23, 24, 25, 26, 27, 28, 29, 30 glycan units. In some embodiments, about 55%, 60%, 65%, 70%, 75%, 80%, 85%, 90%, 95%, or about 97% of the glycan polymer preparation has a DP of between 3, 4, 5, 6, 7, 8, 9, 10 and 20, 21, 22, 23, 24, 25, 26, 27, 28 glycan units.

The yield of conversion for the one or more glycan units (e.g. sugars) in the methods described herein can be determined by any suitable method known in the art, including, for example, high performance liquid chromatography (HPLC). The average yield of conversion can be determined by methods known to the person skilled in the art, for example size-exclusion, ion-affinity, hydrophilic, or hydrophobic chemistry. These methods generally rely on chromatographic separation of materials by an HPLC system equipped with an appropriate column chemistry. Chromatographic separation of starting materials from products then allows the direct comparion of the area under the curve of those materials which can then be converted into a percent yield of conversion. Example 15 describes specific IAC and SEC approaches which can be used to determine the yield of conversion. In a preferred embodiment, the conversion as mentioned herein is determined by the SEC method of Example 15.

In some embodiments, the yield of conversion to a glycan polymer preparation with glycan polymers of a DP of greater than DP1 (DP>1) after combining the one or more glycan subunits with the glycosidase enzyme molecule is greater than or equal to about 1%, 2%, 3%, 4%, 5%, 10%, 15%, 20%, 25%, 30%, 35%, 40%, 45%, 50%, 55%, 60%, 65%, 70%, 75% (as determined on a weight/weight basis as a percentage of input glycan subunits). In some embodiments, the yield of conversion to a glycan polymer preparation with glycan polymers of a DP of at least DP2 after combining the one or more glycan subunits with the glycosidase enzyme molecule is greater than or equal to about 1%, 2%, 3%, 4%, 5%, 10%, 15%, 20%, 25%, 30%, 35%, 40%, 45%, 50%, 55%, 60%, 65%, 70%, 75% (as determined on a weight/weight basis as a percentage of input glycan subunits).

In some embodiments, the yield of conversion to a glycan polymer preparation with glycan polymers of a DP of at least DP3 after combining the one or more glycan subunits with the glycosidase enzyme molecule is greater than or equal to about 1%, 2%, 3%, 4%, 5%, 10%, 15%, 20%, 25%, 30%, 35%, 40%, 45%, 50%, 55%, 60%, 65%, 70%, 75% (as determined on a weight/weight basis as a percentage of input glycan subunits).

In some embodiments, the yield of conversion to a glycan polymer preparation with DP > 1 after combining the one or more glycan units with the catalyst (e.g., at 2, 3, 4, 8, 12, 24, or 48 hours after combining the one or more glycan units with the catalyst) is greater than about 50% (e.g., greater than about 55%, 60%, 65%, 70%, 75%, 80%, 85%, 90%, 95%, or 98%). In some embodiments, the yield of conversion to a glycan polymer preparation with > DP2 after combining the one or more glycan units with the catalyst (e.g., at 2, 3, 4, 8, 12, 24, or 48 hours after combining the one or more glycan units with the catalyst) is greater than 30% (e.g., greater than 35%, 40%, 45%, 50%, 55%. 60%, 65%, 70%, 75%, 80%, 85%, 90%, 95%, or 98%).

In one embodiment, about 55%, 60%, 65%, 70%, 75%, 80%, 85%, 90%, 95%, or about 97% of the glycan polymer preparation has a DP of at least 2. In one embodiment, about 55%, 60%, 65%, 70%, 75%, 80%, 85%, 90%, 95%, or about 97% of the glycan polymer preparation has a DP of at least 3.

*Average DP*

In some embodiments, the glycan polymer preparation has an average degree of polymerization (average DP) of about DP2, DP3, DP4, DP5, DP6, DP7, DP8, or DP9. In some embodiments, the glycan polymer preparation has an average degree of polymerization (average DP) of between about 2 and about 10, between about 2 and about 8, between about 2 and about 6, between about 2 and about 4, between about 3 and about 10, between about 3 and about 8, between about 3 and about 6, or between about 3 and about 4.

In some embodiments, about 55%, 60%, 65%, 70%, 75%, 80%, 85%, 90%, 95%, or about 97% of the glycan polymer preparation has an average degree of polymerization (DP) of about DP5, DP6, DP7, DP8, DP9, DP10, DP11, or DP12. In some embodiments, the average DP of the glycan polymer preparation is between about DP5 and DP10, between about DP6 and DP10, between about DP6 and DP12, between about DP6 and DP14, between about DP8 and DP12, between about DP8 and DP14, between about DP8 and DP16, between about DP10 and DP16 between about DP10 and DP18, between about DP4 and DP18, between about DP6 and DP18, or between about DP8 and DP18.

The distribution of (or average) degree of polymerization (DP) of a glycan polymer preparation can be determined by methods known to the person skilled in the art, for example using ion-affinity (IAC) or size-exclusion chromatography (SEC) measurements of molecular weight (MW) followed by a mathematical conversion into average DP. These methods generally rely on chromatographic separation of materials based on an HPLC system equipped with a mass-sensitive column chemistry such as size-exclusion or ion-affinity columns followed by a computational conversion of that distribution into an average MW by comparison to a set of standards with known MW. Once the average MW is determined, division of that value by the average weight of the glycan's repeat unit allows the calculation of average DP. Example 15 describes specific IAC and SEC approaches which can be used to determine the average DP as mentioned herein. In a preferred embodiment, the average DP as mentioned herein is determined by the SEC method of Example 15.

*Average Molecular weight*

In some embodiments, about 55%, 60%, 65%, 70%, 75%, 80%, 85%, 90%, 95%, or about 97% of the glycan polymers of the preparation have an average molecular weight of about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800 g/mol and less than 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, and 5000 g/mol.

The average molecular weight (MW) can be determined by methods known to the person skilled in the art, for example ion-affinity chromatography (IAC) or size exclusion chromatography (SEC). These methods generally rely on chromatographic separation of materials based on an HPLC system equipped with a mass-sensitive column chemistry such as size-exclusion or ion-affinity columns followed by a computational conversion of that distribution into an average MW by comparison to a set of standards with known MW. Example 15 describes specific IAC and SEC approaches which can be used to determine the average MW as mentioned herein. In a preferred embodiment, the average MW as mentioned herein is determined by the SEC method of Example 15.

*Degree of branching (DB)*

In some embodiments, the glycan preparations range in structure from linear to branched. Branched glycans may contain at least one glycan subunit being linked via an alpha or a beta glycosidic bond so as to form a branch. The branching rate or degree of branching (DB) may vary, such that the glycan polymers of a preparation comprise at least 1, at least 2, at least 3, at least 4, at least 5, or at least about 6 branching points in the glycan polymer. In some embodiments, the glycan polymers of the glycan preparation are unbranched (DB=0).

In some embodiments, the glycan preparations (e.g. oligo- or polysaccharides) range in structure from linear to highly branched. Unbranched glycans may contain only alpha linkages or only beta linkages. Unbranched glycans may contain at least one alpha and at least one beta linkage. Branched glycans may contain at least one glycan unit being linked via an alpha or a beta glycosidic bond so as to form a branch. The branching rate or degree of branching (DB) may vary, such that about every $2^{nd}$, $3^{rd}$, $4^{th}$, $5^{th}$, $6^{th}$, $7^{th}$, $8^{th}$, $9^{th}$, $10^{th}$, $15^{th}$, $20^{th}$, $25^{th}$, $30^{th}$, $35^{th}$, $40^{th}$, $45^{th}$, $50^{th}$, $60^{th}$, or $70^{th}$ unit comprises at least one branching point. For example, animal glycogen contains a branching point approximately every 10 units.

In some embodiments, preparations of glycan polymer are provided, wherein the preparation comprises a mixture of branched glycans, wherein the avarage degree of branching (DB, branching points per residue) is 0, 0.01. 0.02, 0.03, 0.04, 0.05, 0.06, 0.07, 0.08, 0.09, 0.1, 0.2, 0.3, 0.4, 0.5, 0.6, 0.7, 0.8, 0.9, 0.95, 0.99, 1, or 2. In some embodiments, preparations of glycan polymers are provided, wherein the avarage degree of branching is at least 0.01, 0.05, 0.1, 0.2, 0.3, or at least 0.4. In some embodiments, preparations of glycan polymers are provided, wherein the avarage degree of branching is between about 0.01 and 0.1, 0.01 and 0.2, 0.01 and 0.3, 0.01 and 0.4, 0.01 and 0.5, 0.01 and 0.6, or between about 0.01 and 0.7. In some embodiments, preparations of glycan polymers are provided, wherein the avarage degree of branching is between about 0.05 and 0.1, 0.05 and 0.2, 0.05 and 0.3, 0.05 and 0.4, 0.05 and 0.5, 0.05 and 0.6, or between about 0.05 and 0.7. In some embodiments, preparations of glycan polymers are provided, wherein the avarage degree of branching is not 0. In some embodiments, preparations of glycan polymers are provided, wherein the avarage degree of branching is not between at least 0.1 and less than 0.4 or at least 0.2 and less than 0.4. In some embodiments, the preparations of glycan polymers comprise linear glycans. In some embodiments, the preparations of glycan polymers comprise glycans that exhibit a branched or branch-on-branch structure.

In some embodiments, preparations of glycan polymers are provided wherein the average degree of branching (DB) is not 0, but is at least 0.01, 0.05, 0.1, or at least 0.2, or ranges between about 0.01 and about 0.2 or between about 0.05 and 0.1.

The degree of branching (DB) of a glycan polymer preparation can be determined by methods known to the person skilled in the art, for example permethylation analysis. These methods generally rely on chemical functionalization of free hydroxyl groups of a glycan followed by total acid hydrolysis and GC-MS analysis of the isolated monomers. Thus, the fraction of monomers with multiple unfunctionalized hydroxyl groups can be interpreted to equal the fraction of polymer units that were bonded to more than one other unit, e.g., the branched fraction. Example 15 describes specific permethylation approaches which can be used to determine the DB as mentioned herein. In a preferred embodiment, the DB as mentioned herein is determined by the permethylation of Example 15.

*Glycosidic bonds and linkages*

Linkages between the individual glycan subunits found in preparations of glycan polymers may include alpha 1->2, alpha 1->3, alpha 1->4, alpha 1->5, alpha 1->6, alpha 2->1, alpha 2->3, alpha 2->4, alpha 2->6, beta 1->2, beta 1->3, beta 1->4, beta 1->5, beta 1->6, beta 2->1, beta 2->3, beta 2->4, and beta 2->6.

In some embodiments, the glycan polymer preparations comprise only alpha linkages. In some embodiments, the glycan polymers comprise only beta linkages. In some embodiments, the glycan polymers comprise mixtures of alpha and beta linkages.

In some embodiments, the alpha:beta glycosidic bond ratio in a preparation is about 1:1, 2:1, 3:1, 4:1, or 5:1.. In some embodiments, the beta:alpha glycosidic bond ratio in a preparation is about 1:1, 2:1, 3:1, 4:1, or 5:1.

In some embodiments, the alpha:beta glycosidic bond ratio in a preparation is about 0.1:1, 0.2:1, 0.3:1, 0.4:1, 0.5:1, 0.6:1, 0.7:1, 0.8:1, 0.9:1, 1:1, 1.2:1, 1.5:1, 1.7:1, 2:1, 2.2:1, 2.5:1, 2.7:1, 3:1, 4:1, 5:1, 6:1, 7:1, 8:1, 9:1, or about 10:1.

In some embodiments, the glycan polymers of the glycan polymer preparation comprise both alpha- and beta-glycosidic bonds selected from the group consisting of 1->2 glycosidic bond, a 1->3 glycosidic bond, a 1->4 glycosidic bond, a 1->5 glycosidic bond and a 1->6 glycosidic bond. In some embodiments, the glycan polymer preparation comprises at least two or at least three alpha and beta 1->2 glycosidic bonds, alpha and beta 1->3 glycosidic bonds, alpha and beta 1->4 glycosidic bonds, alpha and beta 1->5 glycosidic bonds, and/or alpha and beta 1->6 glycosidic bonds.

In some embodiments, the glycan polymers of the glycan preparation comprise substantially all alpha- or beta configured glycan subunits, optionally comprising about 1%, 2%, 3%, 4% 5%, 6%, 7%, 8%, 9%, 10%, 11%, 12%, 13%, 14%, 15%, 16%, 17%, 18%, 19%, or 20% of the respective other configuration.

In some embodiments, the preparations of glycan polymers comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% glycans with alpha glycosidic bonds. In some embodiments, the preparations of glycan polymers comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% glycans with beta glycosidic bonds. In some embodiments, preparations of glycan polymers are provided, wherein at least 10%, 15%, 20%, 25%, 30%, 35%, 40%, 45%, 50%, 55%, 60%, 65%, 70%, 75%, 80%, or at least 85% of glycans with glycosidic bonds that are alpha glycosidic bonds, at least 10%, 15%, 20%, 25%, 30%, 35%, 40%, 45%, 50%, 55%, 60%, 65%, 70%, 75%, 80%, or at least 85% of glycans with glycosidic bonds that are beta glycosidic bonds, and wherein the percentage of alpha and beta glycosidic bonds does not exceed 100%.

In some embodiments, preparations of glycan polymers are provided, wherein at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% of glycan glycosidic bonds are one or more of: 1->2 glycosidic bonds, 1->3 glycosidic bonds, 1->4 glycosidic bonds, and 1->6 glycosidic bonds. In some embodiments, preparations of glycan polymers are provided, wherein at least 1%, 2%, 3%, 4%, 5%, 6%, 7%, 8%, 9%, 10%, 15%, at least 20%, or 25% each of glycan glycosidic bonds are 1->2, 1->3, 1->4, and 1->6 glycosidic bonds. Optionally, the preparations of glycan polymers further comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, or at least 85% of glycan glycosidic bonds that are selected from the group consisting of: alpha 2->1, alpha 2->3, alpha 2->4, alpha 2->6, beta 2->1, beta 2->3, beta 2->4, and beta 2->6, glycosidic bonds.

In some embodiments, the glycan polymers of the glycan preparation comprise at least two glycosidic bonds selected from the group consisting of alpha 1->2 and alpha 1->3, alpha 1->2 and alpha 1->4, alpha 1->2 and alpha 1->6, alpha 1->2 and beta 1->2, alpha 1->2 and beta 1->3, alpha 1->2 and beta 1->4, alpha 1->2 and beta 1->6, alpha 1->3 and alpha 1->4, alpha 1->3 and alpha 1->6, alpha 1->3 and beta 1->2, alpha 1->3 and beta 1->3, alpha 1->3 and beta 1->4, alpha 1->3 and beta 1->6, alpha 1->4 and alpha 1->6, alpha 1->4 and beta 1->2, alpha 1->4 and beta 1->3, alpha 1->4 and beta 1->4, alpha 1->4 and beta 1->6, alpha 1->6 and beta 1->2, alpha 1->6 and beta 1->3, alpha 1->6 and beta 1->4, alpha 1->6 and beta 1->6, beta 1->2 and beta 1->3, beta 1->2 and beta 1->4, beta 1->2 and beta 1->6, beta 1->3 and beta 1->4, beta 1->3 and beta 1->6, and beta 1->4 and beta 1->6.

The distribution of the glycosidic bonds and linkages can be determined by methods known to the person skilled in the art, for example two-dimensional nuclear magnetic resonance spectroscopy (2D NMR). These methods generally rely on area under the curve (AUC) quantitations of peaks diagnostic to a given linkage type. Example 15 describes specific 2D NMR approaches which can be used to determine the glycosidic bonds and linkages as mentioned herein. In a preferred embodiment, the glycosidic bonds and linkages are determined using the 2D NMR method of Example 15.

*L- and D-forms*

In some embodiments, preparations of glycan polymers are provided, wherein at least one glycan subunit is a sugar in L-form. In some embodiments, preparations of glycans are provided, wherein at least one glycan subunit is a sugar in D-form. In some embodiments, preparations of glycans are provided, wherein the glycan subunits are sugars in L- or D-form as they naturally occur or are more common (e.g. D-glucose, D-xylose, L-arabinose).

In some embodiments, the preparation of glycan polymers (e.g. oligosaccharides and polysaccharides) comprises a desired mixture of L- and D-forms of glycan subunits, e.g. of a desired ratio, such as: 1:1, 1:2, 1:3, 1:4, 1:5 L- to D-forms or D- to L-forms.

In some embodiments, the preparation of glycan polymers comprises a desired mixture of L- and D-forms of glycan units, e.g. of a desired ratio, such as: 1:1, 1:2, 1:3, 1:4, 1:5, 1:6, 1:7, 1:8, 1:9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, 1:100, 1:150 L- to D-forms or D- to L-forms.

In some embodiments, the preparation of glycan polymers comprises glycans with substantially all L- or D-forms of glycan subunits, optionally comprising about 1%, 2%, 3%, 4% 5%, 6%, 7%, 8%, 9%, 10%, 11%, 12%, 13%, 14%, 15%, 16%, 17%, 18%, 19%, or 20% of the respective other form.

*Glycan unit content*

In some embodiments, preparations of glycan polymers are provided, wherein at least one glycan subunit is a tetrose, a pentose, a hexose, or a heptose. Optionally, the glycan subunits involved in the formation of the glycans of the glycan polymer preparation are varied. Examples of monosaccharide glycan subunits include hexoses, such as glucose, galactose, and fructose, and pentoses, such as xylose. Monosaccharides generally have the chemical formula: $C_x(H_2O)_y$, where conventionally $x \geq 3$. Monosaccharides can be classified by the number x of carbon atoms they contain, for example: diose (2) triose (3) tetrose (4), pentose (5), hexose (6), and heptose (7). The monosaccharide glycan subunits may exist in an acyclic (open-chain) form. Open-chain monosaccharides with same molecular graph may exist as two or more stereoisomers. The monosaccharides may also exist in a cyclic form through a nucleophilic addition reaction between the carbonyl group and one of the hydroxyls of the same molecule. The reaction creates a ring of carbon atoms closed by one bridging oxygen atom. In these cyclic forms, the ring usually has 5 (furanoses) or 6 atoms (pyranoses).

In some embodiments, the preparation of glycan polymers comprises a desired mixture of different monosaccharide glycan subunits, such as a mixture of a diose (2), a triose (3), tetrose (4), pentose (5), hexose (6), or heptose (7). In some embodiments, the glycan polymers of the glycan polymer preparation comprise a desired mixture of a pentose (5) and a hexose (6).

In some embodiments, the preparation of glycan polymers comprises a desired mixture of two, three, four or five different glycan subunits, such as a mixture of, e.g., i) one or more glycan subunits selected from monosaccharides, selected from glucose, a galactose, an arabinose, a mannose, a fructose, a xylose, a fucose, and a rhamnose; ii) one or more glycan subunits selected from disaccharides selected from acarviosin, n-acetyllactosamine, allolactose, cellobiose, chitobiose, glactose-alpha-1,3-galactose, gentiobiose, isomalt, isomaltose, isomaltulose, kojibiose, lactitol, lactobionic acid, lactose, lactulose, laminaribiose, maltitol, maltose, mannobiose, melibiose, melibiulose, neohesperidose, nigerose, robinose, rutinose, sambubiose, sophorose, sucralose, sucrose, sucrose acetate isobutyrate, sucrose octaacetate, trehalose, turanose, vicianose, and xylobiose; iii) one or more glycan subunits selected from amino sugars selected from acarbose, N-acetylemannosamine, N-acetylmuramic acid, N-acetylnueraminic acid, N-acetyletalosaminuronic acid, arabinopyranosyl-N-methyl-N-nitrosourea, D-fructose-L-histidine, N-glycolyneuraminic acid, ketosamine, kidamycin, mannosamine, 1B-methylseleno-N-acetyl-D-galactosamine, muramic acid, muramyl dipeptide, phosphoribosylamine, PUGNAc, sialyl-Lewis A, sialyl-Lewis X, validamycin, voglibose, N-acetylgalactosamine, N-acetylglucosamine, aspartylglucosamine, bacillithiol, daunosamine, desosamine, fructosamine, galactosamine, glucosamine, meglumine, and perosamine; iv) one or more glycan subunits selected from deoxy sugars selected from 1-5-ahydroglucitol, cladinose, colitose, 2-deoxy-D-glucose, 3-deoxyglucasone, deoxyribose, dideoxynucleotide, digitalose, fludeooxyglucose, sarmentose, and sulfoquinovose; v) one or more glycan subunits selected from imino sugars selected from castanospermine, 1-deoxynojirimycin, iminosugar, miglitol, miglustat, and swainsonine; one or more glycan subunits selected from sugar acids selected from N-acetylneuraminic acid, N-acetyltalosamnuronic acid, aldaric acid, aldonic acid, 3-deoxy-D-manno-oct-2-ulosonic acid, glucuronic acid, glucosaminuronic acid, glyceric acid, N-glycolylneuraminic acid, iduronic acid, isosaccharinic acid, pangamic acid, sialic acid, threonic acid, ulosonic acid, uronic acid, xylonic acid, gluconic acid, ascorbic acid, ketodeoxyoctulosonic acid, galacturonic acid, galactosaminuronic acid, mannuronic acid, mannosaminuronic acid, tartaric acid, mucic acid, saccharic acid, lactic acid, oxalic acid, succinic acid, hexanoic acid, fumaric acid, maleic acid, butyric acid, citric acid, glucosaminic acid, malic acid, succinamic acid, sebacic acid, and capric acid; vi) one or more glycan subunits selected from short-chain fatty acids selected from formic acid, acetic acid, propionic acid, butryic acid, isobutyric acid, valeric acid, and isovaleric acid; and vii) one or more glycan subunits selected from sugar alcohols selected from methanol, ethylene glycol, glycerol, erythritol, threitol, arabitol, ribitol, xylitol, mannitol, sorbitol, galactitol, iditol, volemitol, fucitol, inositol, maltotritol, maltotetraitol, and polyglycitol.

Exemplary glycans are described by a three-letter code representing the monomeric sugar component followed by a number out of one hundred reflecting the percentage of the material that monomer constitutes. Thus, 'glu100' is ascribed to a glycan generated from a 100% D-glucose (glycan unit) input and 'glu50gal50' is ascribed to a glycan generated from 50% D-glucose and 50% D-galactose (glycan units) input or, alternatively from a lactose dimer (glycan unit) input. As used herein: xyl = D-xylose; ara = L-arabinose; gal = D-galactose; glu = D-glucose; rha = L-rhamnose; fuc = L-fucose; man = D-mannose; sor = D-sorbitol; gly = D-glycerol; neu = NAc-neuraminic acid.

In some embodiments, the preparation of glycan polymers comprises one glycan unit A selected from i) to vii) above, wherein glycan unit A comprises 100% of the glycan unit input. For example, in some embodiments, the glycan polymer preparation is selected from the homo-glycans xyl100, rha100, ara100, gal100, glu100, and man100. In some embodiments, the glycan polymer preparation is selected from the homo-glycans fuc100 and fru100.

In some embodiments, the preparation of glycan polymers comprises a mixture of two glycan units A and B selected independently from i) to vii) above, wherein A and B may be selected from the same or a different group i) to vii) and wherein A and B may be selected in any desired ratio (e.g. anywhere from 1-99% A and 99-1% B, not exceeding 100%).

For example, in some embodiments, the glycan polymer preparation is selected from the heteroglycans ara50gal50, ara50gal50, xyl75gal25, ara80xyl20, ara60xyl40, ara50xyl50, glu80man20, glu60man40, man80glu20, man60glu40, xyl75ara25, gal75xyl25, Man80gal20, gal75xyl25, Man66gal33, Man75gal25, glu80gal20, glu60gal40, glu40gal60, glu20gal80, gal80man20, gal60man40, gal40man60, glu80xyl20, glu60xyl40, glu40xyl60, glu20xyl80, glu80ara20, glu60ara40, glu40ara60, glu20ara80, gal80xyl20, gal60xyl40, gal40xyl60, gal20xyl80, gal80ara20, gal60ara40, gal40ara60, gal20ara80, man80xyl20, man60xyl40, man40xyl60, man20xyl80, man80ara20, man60ara40, man40ara60, man20ara80, xyl80ara20, xyl60ara40, glu50gal50, and man62glu38.

In some embodiments, the preparation of glycan polymers comprises a mixture of three glycan units A, B and C selected independently from i) to vii) above, wherein A, B and C may be selected from the same or a different group i) to vii) and wherein A, B and C may be selected in any desired ratio (e.g. anywhere from 1-99% A, 1-99% B, 1-99% C, not exceeding 100%).

For example, in some embodiments, the glycan polymer preparation is selected from the heteroglycans xyl75glu12gal12, xyl33glu33gal33, xyl75glu12gal12, glu33gal33fuc33, glu33gal33nman33, glu33gal33xyl33, glu33gal33ara33, gal33man33xyl33, gal33man33ara33, man52glu29gal19, Glu33Man33Xyl33, Glu33Man33Ara33, Glu33Xyl33Ara33, Gal33Man33Xyl33, Gal33Man33Ara33, Gal33Xyl33Ara33, Man33Xyl33Ara33, Glu90Gal5Man5, Glu80Gal10Man10, Glu60Gal20Man20, Glu40Gal30Man30, Glu20Gal40Man40, Glu10Gal45Man45, Glu5Gal90Man5, Glu10Gal80Man10, Glu20Gal60Man20, Glu30Gal40Man30, Glu40Gal20Man40, Glu45Gal10Man45, Glu5Gal5Man90, Glu10Gal10Man80, Glu20Gal20Man60, Glu30Gal30Man40, Glu40Gal40Man20, and Glu45Gal45Man10.

In some embodiments, the preparation of glycan polymers comprises a mixture of four glycan units A, B, C and D selected independently from i) to vii) above, wherein A, B, C and D may be selected from the same or a different group i) to vii) and wherein A, B, C and D may be selected in any desired ratio (e.g. anywhere from 1-99% A, 1-99% B, 1-99% C, 1-99% D, not exceeding 100%).

In some embodiments, the preparation of glycan polymers comprises a mixture of five glycan units A, B, C, D and E selected independently from i) to vii) above, wherein A, B, C, D and E may be selected from the same or a different group i) to vii) and wherein A, B, C, D and E may be selected in any desired ratio (e.g. anywhere from 1-99% A, 1-99% B, 1-99% C, 1-99% D, 1-99% E, not exceeding 100%).

In some embodiments, preparationsof glycan polymers are provided, wherein at least one glycan subunit is selected from the group consisting of a glucose, a galactose, an arabinose, a mannose, a fructose, a xylose, a fucose, and a rhamnose.

In some embodiments, the preparation of glycan polymers comprises a desired mixture of two different monosaccharide glycan subunits, such as a mixture of, e.g., glucose and galactose, glucose and arabinose, glucose and mannose, glucose and fructose, glucose and xylose, glucose and fucose, glucose and rhamnose, galactose and arabinose, galactose and mannose, galactose and fructose, galactose and xylose, galactose and fucose, and galactose and rhamnose, arabinose and mannose, arabinose and fructose, arabinose and xylose, arabinose and fucose, and arabinose and rhamnose, mannose and fructose, mannose and xylose, mannose and fucose, and mannose and rhamnose, fructose and xylose, fructose and fucose, and fructose and rhamnose, xylose and fucose, xylose and rhamnose, and fucose and rhamnose, e.g. a in a ratio of 1:1, 1:2, 1:3, 1:4, or 1:5 or the reverse ratio thereof, or a in a ratio of 1:1, 1:2, 1:3, 1:4, 1:5, 1:6, 1:7, 1:8, 1:9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, or 1:100 or the reverse ratio thereof.

In some embodiments, the preparation of glycan polymers comprises a desired mixture of three different monosaccharide glycan subunits, such as a mixture of, e.g. for glucose-containing glycan preparations, glucose, galactose and arabinose; glucose, galactose and mannose; glucose, galactose and fructose; glucose, galactose and xylose; glucose, galactose and fucose, glucose, galactose and rhamnose; glucose, arabinose, and mannose; glucose, arabinose and fructose; glucose, arabinose and xylose; glucose, arabinose and fucose; glucose, arabinose and rhamnose; glucose, mannose and fructose; glucose, mannose and xylose; glucose, mannose and fucose; glucose, mannose rhamnose; glucose, fructose and xylose; glucose, fructose and fucose; glucose, fructose and rhamnose; glucose, fucose and rhamnose, e.g. a in a ratio of 1:1:1, 1:2:1, 1:3:1, 1:4:1, 1:5:1, 1:1:2, 1:2:2, 1:3:2, 1:4:2, 1:1:3, 1:2:3, 1:3:3, 1:1:4, 1:2:4, 1:1:5, 1:2:5, , etc., or . a in a ratio of 1:1:1, 1:2:1, 1:3:1, 1:4:1, 1:5:1, 1:6:1, 1:7:1, 1:8:1, 1:9:1, 1:10:1, 1:12:1, 1:14:1, 1:16:1, 1:18:1, 1:20:1, 1:1:2, 1:2:2, 1:3:2, 1:4:2, 1:5:2, 1:6:2, 1:7:2, 1:8:2, 1:9:2, 1:10:2, 1:1:3, 1:2:3, 1:3:3, 1:4:3, 1:5:3, 1:6:3, 1:7:3, 1:8:3, 1:9:3, 1:10:3, 1:1:4, 1:2:4, 1:3:4, 1:4:4, 1:5:4, 1:6:4, 1:7:4, 1:8:4, 1:9:4, 1:10:4, 1:1:5, 1:2:5, 1:3:5, 1:4:5, 1:5:5, 1:6:5, 1:7:5, 1:8:5, 1:9:5, 1:10:5, etc.

In some embodiments, the preparation of glycan polymers does not comprise N-acetylgalactosamine or N-acetylglucosamine. In some embodiments, the preparation of glycans does not comprise sialic acid. In some embodiments, the preparation of glycan polymers does not comprise a lipid and fatty acid. In some embodiments, the preparation of glycan polymers does not comprise an amino acid.

*Furanose: Pyranose*

In some embodiments, preparations of glycan polymers are provided, wherein at least one glycan subunit is a furanose sugar. In some embodiments, preparations of glycans are provided, wherein at least one glycan subunit is a pyranose sugar. In some embodiments, glycan polymers comprise mixtures of furanose and pyranose sugars. In some embodiments, the furanose: pyranose sugar ratio in a preparation is about 0.1:1, 0.2:1, 0.3:1, 0.4:1, 0.5:1, 0.6:1, 0.7:1, 0.8:1, 0.9:1, 1:1, 1.2:1, 1.5:1, 1.7:1, 2:1, 2.2:1, 2.5:1, 2.7:1, 3:1, 4:1, 5:1, or about 6:1 or the furanose: pyranose sugar ratio in a preparation is about 7:1, 8:1, 9:1, or about 10:1..

In some embodiments, the preparation of glycan polymers comprises substantially all furanose or pyranose sugar, optionally comprising 1%, 2%, 3%, 4% 5%, 6%, 7%, 8%, 9%, 10%, 11%, 12%, 13%, 14%, 15%, 16%, 17%, 18%, 19%, or 20% of the respective other sugar.

In some embodiments, the preparation of glycan polymers comprises substantially all pyranose sugar and no more than about 0.1%, 02%, 0.5%, 1%, 2%, 3%, 4%, or no more than 5% of glycan units in the preparation in furanose form. In some embodiments, no more than 3%, 2% or no more than 1% of monomeric glycan units in the preparation are in furanose form.

*Salts*

In some embodiments, the preparation of glycan polymers comprises a glycan subunit or plurality of glycan subunits present in a salt form (e.g., a pharmaceutically acceptable salt form), such as, e.g., a hydrochlorate, hydroiodate, hydrobromate, phosphate, sulfate, methanesulfate, acetate, formate, tartrate, malate, citrate, succinate, lactate, gluconate, pyruvate, fumarate, propionate, aspartate, glutamate, benzoate, ascorbate salt.

*Derivatization*

If desired, the monosaccharide or oligosaccharide glycan subunits of the glycans are further substituted or derivatized, e.g., hydroxyl groups can be etherified or esterified. For example, the glycans (e.g. oligo- or polysaccharide) can contain modified saccharide units, such as 2 '-deoxyribose wherein a hydroxyl group is removed, 2'-fluororibose wherein a hydroxyl group is replaced with a fluorine, or N- acetylglucosamine, a nitrogen-containing form of glucose (e.g., 2'-fluororibose, deoxyribose, and hexose). The degree of substitution (DS, average number of hydroxyl groups per glycosyl unit) can be 1, 2, or 3, or another suitable DS. In some embodiments, 1%, 2%, 3%, 4% 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 80%, 85%, 90%, 95%, 96%, 97%, 98%, 99%, or 100% of glycan subunits are substituted or derivatized. In some embodiments, the degree of substitution varies between subunits, e.g., a certain percentage is not derivatized, exhibits a DS of 1, exhibits a DS of 2, or exhibits a DS of 3. Any desired mixture can be generated, e.g. 0-99% of subunits are not derivatized, 0-99% of subunits exhibit a DS of 1, 0-99% of subunits exhibit a DS of 2, and 0-99% of subunits exhibit a DS of 3, with the total making up 100%. The degree of substitution can be controlled by adjusting the average number of moles of substituent added to a glycosyl moiety (molar substitution (MS)). The distribution of substituents along the length of the glycan oligo- or polysaccharide chain can be controlled by adjusting the reaction conditions, reagent type, and extent of substitution. In some embodiments, the monomeric subunits are substituted with one or more of an acetate ester, sulfate half-ester, phosphate ester, or a pyruvyl cyclic acetal group.

*Solubility*

In some embodiments, the glycan polymers in a preparation are highly soluble. In some embodiments, glycan polymer preparations can be concentrated to at least to 55 Brix, 65 Brix, 60 Brix, 65 Brix, 70 Brix, 75 Brix, 80 Brix, or at least 85 Brix without obvious solidification or crystallization at 23 °C (final solubility limit). In some embodiments, glycan polymer preparations are concentrated to at least about 0.5 g/ml, 1 g/ml, 1.5 g/ml, 2 g/ml, 2.5 g/ml, 3 g/ml, 3.5 g/ml or at least 4 g/ml without obvious solidification or crystallization at 23 °C (final solubility limit).

In some embodiments, the glycan polymer preparations (e.g. oligosaccharides) are branched, e.g. have an average DB of at least 0.01, 0.05, or 0.1 and has a final solubility limit in water of at least about 70 Brix, 75 Brix, 80 Brix, or at least about 85 Brix at 23 °C or is at least about 1 g/ml, 2 g/ml or at least about 3 g/ml.

In some embodiments, the preparation of glycan polymers has a final solubility limit of at least 0.001 g/L, 0.005 g/L, 0.01 g/L, 0.05 g/L, 0.1 g/L, 0.2 g/L, 0.3 g/L, 0.4 g/L, 0.5 g/L, 0.6 g/L, 0.7 g/L, 0.8 g/L, 0.9 g/L, 1g/L, 5 g/L, 10 g/L, 20 g/L, 30 g/L, 40 g/L, 50 g/L, 100 g/L, 200 g/L, 300 g/L, 400 g/L, 500 g/L, 600 g/L, 700 g/L, 800 g/L, 900 g/L, 1000 g/L in deionized water, or in a suitable buffer such as, e.g., phosphate-buffered saline, pH 7.4 or similar physiological pH) and at 20 °C. In some embodiments, the preparation of glycan polymers is greater than 50%, greater than 60%, greater than 70%, greater than 80%, greater than 90%, greater than 95%, greater than 96%, greater than 97%, greater than 98%, greater than 99%, or greater than 99.5% soluble with no precipitation observed at a concentration of greater than 0.001 g/L, 0.005 g/L, 0.01 g/L, 0.05 g/L, 0.1 g/L, 0.2 g/L, 0.3 g/L, 0.4 g/L, 0.5 g/L, 0.6 g/L, 0.7 g/L, 0.8 g/L, 0.9 g/L, 1g/L, 5 g/L, 10 g/L, 20 g/L, 30 g/L, 40 g/L, 50 g/L, 100 g/L, 200 g/L, 300 g/L, 400 g/L, 500 g/L, 600 g/L, 700 g/L, 800 g/L, 900 g/L, 1000 g/L in deionized water, or in a suitable buffer such as, e.g., phosphate-buffered saline, pH 7.4 or similar physiological pH) and at 20 °C.

*Sweetness*

In some embodiments, the preparation of glycan polymers has a desired degree of sweetness. For example, sucrose (table sugar) is the prototype of a sweet substance. Sucrose in solution has a sweetness perception rating of 1, and other substances are rated relative to this (e.g., fructose, is rated at 1.7 times the sweetness of sucrose). In some embodiments, the sweetness of the preparation of glycan polymers ranges from 0.1 to 500,000 relative to sucrose. In some embodiments, the relative sweetness is 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 80, 90, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, 10000, 25000, 50000, 75000, 100000, 150000, 200000, 250000, 300000, 350000, 40000, 450000, 500000, or more than 500,000 relative to sucrose (with sucrose scored as one). In some embodiments, the preparation of glycan polymers is mildly sweet, or both sweet and bitter.

In some embodiments, the preparation of glycan polymers, e.g. a preparation that is substantially DP2+ or DP3+ (e.g. at least 80%, 90%, or at least 95%, or a fractionated preparation of DP2+ or DP3+), is substantially imperceptible as sweet and the relative sweetness is about 0, 0.0001, 0.001, 0.005, 0.01, 0.05, 0.1, 0.2, 0.3, 0.4, 0.5, 0.6, 0.7, or about 0.8 relative to sucrose (with sucrose scored as one).

*Fermentability*

In some embodiments, glycan polymer preparations disclosed herein are screened to assess their fermetability. Fermentability of a glycan polymer is a function of the number or representation of hydrolysable glycosidic bonds in the glycan species of the preparation. In some embodiments, fermentability is tested using a glycosidase enzyme or a glycosidase enzyme molecule described herein. It is believed that a glycan polymer produced by the methods described herein, e.g., by utilizing a glycosidase enzyme molecule, is a substrate for a glycosidase enzyme (e.g. that of a human gut microbe) that is closely related to the glycosidase enzyme molecule (e.g. they share a high degree of relevant sequence homology, the glycosidase enzyme molecule is a derivative of the glycosidase enzyme, they share the same origin (e.g., microbial origin, they share the same glycosidic functionality, they are members of the glycoside hydrolase or glycoside transferase CAZy family, etc.).

In some embodiments, the degree of fermentability of the glycan polymer preparation is 30 minutes or less, 20 minutes or less, 15 minutes or less, 10 minutes or less, 5 minutes or less, 4 minutes or less, 3 minutes or less, 2 minutes or less or 1 minute or less. In some embodiments, the digestibility of the glycan polymer preparation is 30 minutes or more, 45 minutes or more, 1 hour or more, 2 hours or more, 3 hours or more, 4 hours or more, 5 hours or more, or 10 hours or more, in which 5%, 10%, 15%, 20%, 25%, 30%, 35%, 40%, 45%, 50%, 55%, 60%, 70%, 80%, 90%, 95%, 97%, 99% of the glycans of the preparation have been fermented, e.g., broken down, so that the glycan polymers of the preparation exhibit a reduction in average DP (e.g. DP=5 to DP=4) and/or a gain (or loss) of small molecular weight fractions (e.g. monomers, dimers, trimers) by standard methodology (e.g. size-exclusion chromatography). In some embodiments, the glycan polymers of the glycan polymer preparation comprise less than 1%, 2%, 3%, 4%, 5%, 6%, 7%, 8%, 9%, 10%, 12%, 14%, 16%, 18%, 20%, 30%, 40%, or less than 50% bonds that are hydrolyzable by a mammalian enzyme (e.g. amylase).

Suitable assays can be used to assess comparative fermentability (e.g., against a benchmark glycan) or to assess absolute digestibility.

**Identification and analysis of glycan polymers**

If desired, the glycan polymer preparations can be characterized. For example, the monomeric building blocks (e.g. the monosaccharide or glycan subunit composition), the anomeric configuration of side chains, the presence and location of substituent groups, degree of polymerization/molecular weight and the linkage pattern can be identified by standard methods known in the art, such as, e.g. methylation analysis, reductive cleavage, hydrolysis, GC-MS (gas chromatography-mass spectrometry), MALDI-MS (Matrix-assisted laser desorption/ionization-mass spectrometry), ESI-MS (Electrospray ionization- mass spectrometry), HPLC (High-Performance Liquid chromatography with ultraviolet or refractive index detection), HPAEC-PAD (High-Performance Anion-Exchange chromatography with Pulsed Amperometric Detection), CE (capillary electrophoresis), IR (infra red)/Raman spectroscopy, and NMR (Nuclear magnetic resonance) spectroscopy techniques. For polymers of crystalline consistency, the crystal structure can be solved using, e.g., solid-state NMR, FT-IR (Fourier transform infrared spectroscopy), and WAXS (wide-angle X-ray scattering). The DP, DP distribution, and polydispersity can be determined by, e.g., viscosimetry and SEC (SEC-HPLC, high performance size-exclusion chromatography). Alien groups, end groups and substituents can be identified, e.g., using SEC with labeling, aqueous analytics, MALDI-MS, FT-IR, and NMR. To identify the monomeric components of the glycans methods such as, e.g. acid-catalyzed hydrolysis, HPLC (high performance liquid chromatography) or GLC (gas-liquid chromatography) (after conversion to alditol acetates) may be used. To determine the linkages present in the glycans, in one example, the polysaccharide is methylated with methyl iodide and strong base in DMSO, hydrolysis is performed, a reduction to partially methylated alditols is achieved, an acetylation to methylated alditol acetates is performed, and the analysis is carried out by GLC/MS (gas-liquid chromatography coupled with mass spectrometry). In some embodiments, to determine the polysaccharide sequence a partial depolymerization is carried out using an acid or enzymes to determine the structures. Possible structures of the polysaccharide are compared to those of the hydrolytic oligomers, and it is determined which one of the possible structures could produce the oligomers. To identify the anomeric configuration, in one example, the intact polysaccharide or a preparation of oligosaccharides are subjected to enzymatic analysis, e.g. they are contacted with an enzyme that is specific for a particular type of linkage, e.g., β-galactosidase, or α-glucosidase, etc., and NMR may be used to analyze the products.

For example, the distribution of (or average) degree of polymerization (DP) of a glycan polymer preparation may be measured by injecting a sample with a concentration of, e.g., 10-100 mg/mL onto an Agilent 1260 BioPure HPLC (or similar) equipped with a 7.8x300 mm BioRad Aminex HPX-42A column (or similar) and RI detector as described, e.g., in Gómez et al. (Purification, Characterization, and Prebiotic Properties of Pectic Oligosaccharides from Orange Peel Wastes, J Agric Food Chem, 2014, 62:9769). Alternatively, a sample with a concentration may be injected into a Dionex ICS5000 HPLC (or similar) equipped with a 4x250 mm Dionex CarboPac PA1 column (or similar) and PAD detector as described, e.g., in Holck et al., (Feruloylated and nonferuloylated arabino-oligosaccharides from sugar beet pectin selectively stimulate the growth of bifidobacterium spp. in human fecal in vitro fermentations, Journal of Agricultural and Food Chemistry, 2011, 59(12), 6511-6519). Integration of the resulting spectrum compared against a standard solution of oligomers allows determination of the average DP.

Distribution of molecular weights can be measured, e.g, by MALDI mass spectrometry. Oligosaccharide concentration can be measured with a Mettler-Toledo sugar refractometer (or similar) with the final value adjusted against a standardized curve to account for refractive differences between monomers and oligomers.

Distribution of glycoside regiochemistry can be characterized, e.g., by a variety of 2D-NMR techniques including COSY, HMBC, HSQC, DEPT, and TOCSY analysis using standard pulse sequences and a Bruker 500 MHz spectrometer. Peaks can be assigned by correlation to the spectra of naturally occurring polysaccharides with known regiochemistry.

Monomeric compositions of oligomers may be measured, e.g., by the complete hydrolysis method in which a known amount of oligomer is dissolved into a strong acid at elevated temperature and allowed sufficient time for total hydrolysis to occur. The concentration of individual monomers may then be measured by the HPLC or GC methods described herein and known in the art to achieve relative abundance measurements as in Holck et al. Absolute amounts can be measured by spiking the HPLC sample with a known amount of detector active standard selected to prevent overlap with any of the critical signals.

The degree of branching in any given population may be measured by the methylation analysis method established, e.g, by Hakomori (J. Biochem. (Tokyo), 1964, 55, 205). With these data, identification of potential repeat units may be established by combining data from the total hydrolysis, average DP, and methylation analysis and comparing them against the DEPT NMR spectrum. Correlation of the number of anomeric carbon signals to these data indicates if a regular repeat unit is required to satisfy the collected data as demonstrated, e.g., in Harding, et al. (Carbohydr. Res. 2005, 340, 1107).

The molar percentage of species with a degree of polymerization (DP) of n (denoted here as DP(n)) in a population is determined by high performance liquid chromatography (HPLC), e.g., on an Agilent 1260 BioInert series instrument equipped with a refractive index (RI) detector and a variety of columns familiar to those skilled in the art using water as the mobile phase. The columns are selected from chemistries including, but not limited to, HILIC, metal coordination, and aqueous size-exclusion chromatography that best isolate the species of interest. Molar % DP(n) is determined by the formula:

$$\% \, DP(n) = 100 * AUC \, [DP(n)] \, / \, AUC \, [DP(total)],$$

where AUC is defined as the area under the curve for the species of interest as determined by calibration to known standards. The molar percentage of glycosidic bond isomers (% alpha and %beta) are determined by nuclear magnetic resonance (NMR) spectroscopy using a variety of 2D techniques familiar to those skilled in the art. Alpha- and beta-isomers may be distinguished, e.g., by their distinct shift on the NMR spectrum and the molar percentage is determined by the formula:

$$\% \, (glycosidic \, isomer \, n) \, of \, glycosidic \, bonds =$$
$$100 * AUC \, [shift \, (isomer \, n)] \, / \, AUC \, [shift \, (isomer \, alpha+ \, isomer \, beta)],$$

where AUC is defined as the area under the curve at a specific shift value known to represent the desired isomer n. The molar percentage of regiochemical isomers is determined in an analogous fashion using the formula:

$$\% \text{ (regioisomer n) of regioisomers} = 100 * AUC \text{ [shift (regioisomer n)] } / AUC \text{ [shift (all regioisomers)]}.$$

The relative percentage of monomeric sugars making up the oligomeric population is determined, e.g., by total acidic digestion of the oligomeric sample followed by conversion to the alditol acetate followed by gas chromatographic (GC) analysis of the resultant monomeric solutions compared against GC of known standards. The molar percentage of monomer(n), where n can be any sugar, is determined by the formula:

$$\% \text{ (sugar n)} = 100 * AUC \text{ [sugar n] } / AUC \text{ [total of all monomeric sugars]}.$$

In some embodiments, the solubility of the preparation of glycan polymers can be controlled, e.g. by selecting the charge, structure (e.g. DP, degree of branching), and/or derivatization of the glycan units.

Preparations of glycan polymers consisting of one type of sugar unit uniformly linked in linear chains are usually water insoluble at 23 °C even when the glycans have a low molecular weight with degrees of polymerization (DP) between 20 and 30. The degree of solubility of the glycan polymers can be adjusted, e.g. by the introduction of (1->6)-linkages and alternating glycosidic bonds in the glycans. The extra degrees of freedom provided by the rotation about the C-5 to C-6 bonds gives higher solution entropy values. Homoglycans with two types of sugar linkages or heteroglycans composed of two types of sugars are generally more soluble than homogeneous polymers. Ionization of linear homoglycans can add solubility, e.g. to that of gels. The viscosity of the solutions often depends on the tertiary structures of the glycans.

**Formulations and dosages of glycan polymers**

Provided herein are also methods of producing compositions (e.g., pharmaceutical compositions) comprising a glycan polymer preparation that meets one or more, two or more, three or more or four or more of the characteristics of the preparations described herein (including criteria (i)-(v) above). In particular, methods include providing a glycan polymer preparation and acquiring the value(s) for one or more, two or more, or three or more characteristics of the preparation, including, e.g., i) the degree of polymerization (DP), ii) the average degree of branching (DB, branching points per residue), iii) the ratio of alpha- glycosidic to beta-glycosidic bonds, iv) the identity of the glycan subunits, and v) the ratio of glycan subunits, and producing a pharmaceutical composition comprising a glycan polymer preparation if the desired or predetermined criteria of the preparation are met within a desired range of deviation.

Methods for formulating the glycan polymer preparation into a pharmaceutical composition, medical food or dietary supplement are known in the art and may include one or more, two or more, three or more, or four or more of the following steps: (i) formulating the preparation into drug product, (ii) packaging the preparation, (iii) labeling the packaged preparation, and (iv) selling or offering for sale the packaged and labeled preparation. Formulating the glycan polymer preparation into a drug product is known in the art and may include one or more, two or more, three or more, or four or more of the following steps: (i) removing unwanted constituents from the preparation, (ii) reducing the volume of the preparation, (iii) sterilizing the preparation, (iv) admixing the preparation with a pharmaceutically acceptable excipient or carrier, (v) admixing the preparation with a second drug or pharmaceutical agent, (vi) formulating the preparation into a suitable consistency, such as, e.g., aqueous diluted solution, a syrup or a solid, (vii) formulating the preparation into a suitable dosage form, e.g. into a tablet, pill or capsule.

In some embodiments, the glycan polymer preparation undergoes further processing to produce either glycan polymer syrup or powder. For example, in one variation, the glycan polymer preparation is concentrated to form a syrup. Any suitable methods known in the art to concentrate a solution may be used, such as the use of a vacuum evaporator. In another variation, the glycan polymer preparation is spray dried to form a powder. Any suitable methods known in the art to spray dry a solution to form a powder may be used.

Provided herein are pharmaceutical compositions, medical foods and dietary supplements comprising glycan polymer preparations. Optionally, the pharmaceutical compositions, medical foods and dietary supplements comprising glycan polymer preparations further comprise a second agent, e.g., a prebiotic substance and/or a probiotic bacterium. In some embodiments, the pharmaceutical compositions and medical foods and dietary supplements comprising glycan polymer preparations further comprise a micronutrient. In some embodiments, the pharmaceutical compositions and medical foods and dietary supplements comprising glycan polymer preparations do not contain a prebiotic substance.

In some embodiments, the pharmaceutical compositions and medical foods and dietary supplements comprising glycan polymer preparations do not contain a probiotic bacterium. Further, optionally, the pharmaceutical compositions and medical foods and dietary supplements comprising glycan polymer preparations comprise one or more excipients or carriers, including diluents, binders, disintegrants, dispersants, lubricants, glidants, stabilizers, surfactants, flavoring agents, and colorants.

In some embodiments, pharmaceutical compositions and medical foods and dietary supplements comprising glycan polymer preparations (and kits comprising same) comprise one or more micronutrient. In some embodiments, the micronutrient is selected from the group consisting of a trace mineral, choline, a vitamin, and a polyphenol. In some embodiments, the micronutrient is a trace metal. Trace minerals suitable as a micronutrient include, but are not limited to, boron, cobalt, chromium, calcium, copper, fluoride, iodine, iron, magnesium, manganese, molybdenum, selenium, and zinc. In some embodiments, the micronutrient is a vitamin. In some embodiments, the micronutrient is a polyphenol.

Further, if desired, the pharmaceutical compositions and medical foods and dietary supplements comprising glycan polymer preparations may comprise therapeutically active agents, prebiotic substances and/or probiotic bacteria. Alternatively or in addition, therapeutically active agents, prebiotic substances and/or probiotic bacteria may be administered separately (e.g. prior to, concurrent with or after administration of the glycan polymers) and not as a part of the pharmaceutical composition or medical food or dietary supplement (e.g. as a co-formulation) of glycan polymers. In some embodiments, pharmaceutical compositions or medical foods or dietary supplements comprising preparations of glycan polymers are administered in combination with a recommended or prescribed diet, e.g. a diet that is rich in probiotic and/or prebiotic-containing foods, such as it may be determined by a physician or other healthcare professional. Therapeutically active agents, prebiotic substances and/or probiotic bacteria may be administered to modulate the gut microbiome of the subject. In some embodiments, the combined effect (e.g. on the number or intensity of the microbial, genomic or functional shifts) is additive. In other embodiments, the combined effect (e.g. on the number or intensity of the microbial, genomic or functional shifts) is synergistic.

In some embodiments, the pharmaceutical compositions and medical foods and dietary supplements comprising glycan polymer preparations described herein further comprise a prebiotic substance or preparation thereof.

In some embodiments, prebiotics may be administered to a subject receiving the pharmaceutical compositions or medical foods or dietary supplements comprising glycan polymer preparations described herein. Prebiotics are non-digestible substances that when consumed may provide a beneficial physiological effect on the host by selectively stimulating the favorable growth or activity of a limited number of indigenous bacteria in the gut (Gibson G R, Roberfroid M B. Dietary modulation of the human colonic microbiota: introducing the concept of prebiotics. J Nutr. 1995 June; 125(6): 1401-12.). A prebiotic such as a dietary fiber or prebiotic oligosaccharide (e.g. crystalline cellulose, wheat bran, oat bran, corn fiber, soy fiber, beet fiber and the like) may further encourage the growth of probiotic and/or commensal bacteria in the gut by providing a fermentable dose of carbohydrates to the bacteria and increase the levels of those microbial populations (e.g. lactobacilli and bifidobacteria) in the gastrointestinal tract. Prebiotics include, but are not limited to, various galactans and carbohydrate based gums, such as psyllium, guar, carrageen, gellan, lactulose, and konjac. In some embodiments, the prebiotic is one or more of galactooligosaccharides (GOS), lactulose, raffinose, stachyose, lactosucrose, fructo-oligosaccharides (FOS, e.g. oligofructose or oligofructan), inulin, isomaltooligosaccharide, xylo-oligosaccharides (XOS), paratinose oligosaccharide, isomaltose oligosaccharides (IMOS), transgalactosylated oligosaccharides (e.g. transgalacto-oligosaccharides), transgalactosylate disaccharides, soybean oligosaccharides (e.g. soyoligosaccharides), chitosan oligosaccharide (chioses), gentiooligosaccharides, soy- and pectin-oligosaccharides, glucooligosaccharides, pecticoligosaccharides, palatinose polycondensates, difructose anhydride III, sorbitol, maltitol, lactitol, polyols, polydextrose, linear and branched dextrans, pullalan, hemicelluloses, reduced paratinose, cellulose, beta-glucose, beta-galactose, beta-fructose, verbascose, galactinol, xylan, inulin, chitosan, beta-glucan, guar gum, gum arabic, pectin, high sodium alginate, and lambda carrageenan, or mixtures thereof. Examples of suitable probiotics include, but are not limited to, organisms classified as genera Bacteroides, Blautia, Clostridium, Fusobacterium, Eubacterium, Ruminococcus, Peptococcus, Peptostreptococcus, Akkermansia, Faecalibacterium, Roseburia, Prevotella, Bifidobacterium, Lactobacillus, Bacillus, Enterococcus, Escherichia, Streptococcus, Saccharomyces, Streptomyces, and family Christensenellaceae. Non-exclusive examples of probiotic bacteria that can be used in the methods and compositions described herein include L. acidophilus, Lactobacillus species, such as L. crispatus, L. casei, L. rhamnosus, L. reuteri, L. fermentum, L. plantarum, L. sporogenes, and L. bulgaricus, as well as Bifidobacterum species, such as B. lactis, B. animalis, B. bifidum, B. longum, B. adolescentis, and B. infantis. Yeasts, such as Saccharomyces boulardii, are also suitable as probiotics for administration to the gut, e.g. via oral dosage forms or foods. In some embodiments, the probiotic bacterial taxa is not Bifidobacterium. In some embodiments, the probiotic bacterial taxa is not Lactobacillus. Beneficial bacteria for the modulation of the gastrointestinal microbiota may include bacteria that produce organic acids (lactic & acetic acids) or that produce cytotoxic or cytostatic agents (to inhibit pathogenic growth), such as, e.g., hydrogen peroxide ($H_2O_2$) and bacteriocins. Bacteriocins are small antimicrobial peptides which can kill both closely-related bacteria, or exhibit a broader spectrum of activity (e.g., nisin).

Beneficial bacteria may include one or more of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus. In some embodiments, the probiotic or commensal bacteria include one or more of the bacteria listed in Table 2.

The prebiotic substances and probiotic strains that may be combined with glycan polymers described herein to produce a composition or kit may be isolated at any level of purity by standard methods and purification can be achieved by conventional means known to those skilled in the art, such as distillation, recrystallization and chromatography. The cultivated bacteria to be used in the composition are separated from the culture broth with any method including, without limitations, centrifuging, filtration or decantation. The cells separated from the fermentation broth are optionally washed by water, saline (0.9% NaCl) or with any suitable buffer. The wet cell mass obtained may be dried by any suitable method, e.g., by lyophilization. In some embodiments, the probiotic bacteria are lyophilized vegetative cells. In some embodiments, the preparations of spores from sporulating probiotic bacteria are used.

In one embodiment, a glycan polymer preparation further comprises a prebiotic and probiotic. In one embodiment, the pharmaceutical composition comprises probiotics whose viability has been partially attenuated (e.g. a mixture comprising 10%, 20%, 30%, 40%, 50% or more non-viable bacteria), or probiotics consisting solely of non-viable microbes. The compositions may further comprise microbial membranes and/or cell walls that have been isolated and purified from killed microbes. If desired, the probiotic organism can be incorporated into the glycan polymer preparation as a culture in water or another liquid or semisolid medium in which the probiotic remains viable. In another technique, a freeze-dried powder containing the probiotic organism may be incorporated into a particulate material or liquid or semisolid material by mixing or blending.

In some embodiments, the pharmaceutical compositions and medical foods and dietary supplements comprising glycan polymer preparations further comprise a second therapeutic agent or preparation thereof. In some embodiments, the therapeutic agent is an antibiotic, an antifungal agent, an antiviral agent, or an anti-inflammatory agent (e.g. a cytokine, hormone, etc.).

The glycan polymer preparations described herein, other therapeutically active agents, prebiotic substances, micronutrients and probiotics may be comingled or mixed in a single pharmaceutical composition or medical food or dietary supplement. In other embodiments, they may be contained in separate containers (and/or in various suitable unit dosage forms) but packaged together in one or more kits. In some embodiments, the preparations or compositions are not packaged or placed together. A physician may then administer the preparations or compositions together, e.g. prior to, concomitant with, or after one another. In some embodiments, the preparations or compositions act synergistically in modulating the microbiota in a subject, e.g., in the GI tract.

In some embodiments, a glycan polymer composition comprises between 0.1% and 100% glycan polymer preparation by w/w, w/v, v/v or molar %. In another embodiment, a glycan polymer composition comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% 80%, 81%, 82%, 83%, 84%, 85%, 86%, 87%, 88%, 89%, 90%, 91%, 92%, 93%, 94%, 95%, 96%, 97%, 98%, 99%, 99.5%, or 100% of glycan polymer preparation by w/w, w/v, v/v or molar %. In one embodiment, a glycan polymer composition comprises about 1-90%, about 10-90%, about 20-90%, about 30-90%, about 40-90%, about 40-80%, about 40-70%, about 40-60%, about 40-50%, about 50-90%, about 50-80%, about 50-70%, about 50-60%, about 60-90%, about 60-80%, about 60-70%, about 70-90%, about 70-80%, about 70-90%, about 70-80%, about 80-90%, about 90-96%, about 93-96%, about 93-95%, about 94-98%, about 93-99%, or about 90-100% of glycan polymer preparation by w/w, w/v, v/v or molar %. A composition comprising a glycan polymer preparation can optionally comprise one or more excipients or carriers. The glycan polymer composition can comprise from about 1% to about 90% of the one or more excipients or carriers by w/w, w/v, v/v or molar %. For example, the glycan polymer composition can comprise about 1-90%, 1-75%, 1-60%, 1-55%, 1-50%, 1-45%, 1-40%, 1-25%, 1-15%, 1-10%, 10-90%, 10-75%, 10-60%, 10-55%, 10-50%, 10-45%, 10-40%, 10-25%, 10-15%, 15-90%, 15-75%, 15-60%, 15-55%, 15-50%, 15-45%, 15-40%, 15-25%, 25-90%, 25-75%, 25-60%, 25-55%, 25-50%, 25-45%, 25-40%, 40-90%, 40-75%, 40-60%, 40-55%, 40-50%, 40-45%, 45-90%, 45-75%, 45-60%, 45-55%, 45-50%, 50-90%, 50-75%, 50-60%, 50-55%, 55-90%, 55-75%, 55-60%, 60-90%, 60-75%, 75-90% of the one or more excipients or carriers by w/w, w/v, v/v or molar %.

Medical Food

Also provided herein are preparations of glycan polymers formulated as a medical food. Any glycan polymer preparation described herein may be formulated as a medical food as well as pharmaceutical compositions that comprise glycan polymer preparations.

A medical food is defined in section 5(b)(3) of the Orphan Drug Act (21 U.S.C. 360ee(b)(3)). Medical food is formulated to be consumed (oral intake) or administered enterally (e.g. feeding/nasogastric tube) under medical supervision, e.g. by a physician. It is intended for the specific dietary management of a disease or condition, such as, e.g. dysbiosis or a GI-tract disease. Medical foods as used herein do not include food that is merely recommended by a physician as part of an overall diet to manage the symptoms or reduce the risk of a disease or condition. Medical foods comprising a preparation of glycan polymers are foods that are synthetic (e.g., formulated and/or processed products, such as, being formulated for the partial or exclusive feeding of a patient by oral intake or enteral feeding by tube) and not naturally occurring foodstuff used in a natural state.

In some embodiments, the subject has limited or impaired capacity to ingest, digest, absorb, or metabolize ordinary foodstuffs or certain nutrients. In other embodiments, the subject has other special medically determined nutrient requirements, the dietary management of which cannot be achieved by the modification of the normal diet alone. Medical foods comprising a preparation of glycan polymers are administered to a subject in need thereof under medical supervision (which may be active and ongoing) and usually, the subject receives instructions on the use of the medical food. Medical foods may comprise one or more food additives, color additives, GRAS excipients and other agents or substances suitable for medical foods. Medical food preparations may be nutritionally complete or incomplete formulas.

Dietary Supplements

Any glycan polymer preparation described herein may be formulated as a dietary supplement, e.g, for use in a method described herein. Dietary supplements are regulated under the Dietary Supplement Health and Education Act (DSHEA) of 1994. A dietary supplement is a product taken by mouth that contains a "dietary ingredient" intended to supplement the diet. The "dietary ingredients" in these products may include, in addition to a glycan polymer preparation described herein, one or more of: vitamins, minerals, herbs or other botanicals, amino acids, and substances such as enzymes, organ tissues, glandulars, and metabolites. Dietary supplements can also be extracts or concentrates, and may be found in many forms such as tablets, capsules, softgels, gelcaps, liquids, or powders. They can also be in other forms, such as a bar, but if they are, information on their label must not represent the product as a conventional food or a sole item of a meal or diet. DSHEA requires that every supplement be labeled a dietary supplement and not as a general food.

Food Ingredient

Any glycan polymer preparation described herein may be formulated as a food ingredient or food additive, e.g, for use in a method described herein. Food ingredients may be generally recognized as safe (GRAS) or may require FDA authorization. Glycan polymer preparations can be added to any desireable food, e.g. beverages (incl., e.g., fruit juices), dairy products (e.g., milk, yogurt, cheese), cereals (any grain products), bread, spreads, etc.

The glycan polymer preparations described herein may be formulated into any suitable dosage form, e.g. for nasal, oral, rectal or gastric administration. In some embodiments, the glycan polymer preparations described herein may be formulated for enteral administration. In some embodiments, the glycan polymer preparations described herein may be formulated for tube feeding (e.g. naso-gastric, oral-gastric or gastric feeding). The dosage forms described herein can be manufactured using processes that are known to those of skill in the art.

The dosage form may be a packet, such as any individual container that contains a glycan polymer preparation in the form of, e.g., a liquid (wash/rinse), a gel, a cream, an ointment, a powder, a tablet, a pill, a capsule, a depository, a single-use applicator or medical device (e.g. a syringe). For example, provided is also an article of manufacture, such as a container comprising a unit dosage form of the glycan polymer preparation, and a label containing instructions for use of such glycan polymer.

Forms of the compositions that can be used orally include tablets, push-fit capsules made of gelatin, as well as soft, sealed capsules made of gelatin and a plasticizer, such as glycerol or sorbitol. Tablets can be made by compression or molding, optionally with one or more accessory ingredients. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservative, antioxidant, disintegrant (e.g., sodium starch glycolate, cross-linked povidone, cross-linked sodium carboxymethyl cellulose) or lubricating, surface active or dispersing agents. Molded tablets can be made by molding in a suitable machine a mixture of the powdered compound moistened with an inert liquid diluent. The tablets can optionally be coated or scored and can be formulated so as to provide slow or controlled release of the active ingredient therein. Tablets can optionally be provided with an enteric coating, to provide release in parts of the gut (e.g., colon, lower intestine) other than the stomach. All formulations for oral administration can be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds and/or other agents (e.g., prebiotics or probiotics) can be dissolved or suspended in suitable liquids, such as fatty oils, liquid paraffin, or liquid polyethylene glycols. In addition, stabilizers can be added. Dragee cores are provided with suitable coatings. For this purpose, concentrated sugar solutions can be used, which can optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments can be added to the tablets or dragee coatings for identification or to characterize different combinations of active compound doses. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethylene glycol or an oil medium, for example peanut oil, liquid paraffin, or olive oil.

In one embodiment, a provided glycan polymer preparation includes a softgel formulation. A softgel can contain a gelatin based shell that surrounds a liquid fill. The shell can be made of gelatin, plasticizer (e.g., glycerin and/or sorbitol), modifier, water, color, antioxidant, or flavor. The shell can be made with starch or carrageenan. The outer layer can be enteric coated. In one embodiment, a softgel formulation can include a water or oil soluble fill solution, or suspension of a composition covered by a layer of gelatin.

Solid formulations for oral use may comprise an enteric coating, which may control the location at which a glycan polymer preparation is absorbed in the digestive system. For example, an enteric coating can be designed such that a glycan polymer preparation does not dissolve in the stomach but rather travels to the small intestine, where it dissolves. An enteric coating can be stable at low pH (such as in the stomach) and can dissolve at higher pH (for example, in the small intestine). Material that can be used in enteric coatings includes, for example, alginic acid, cellulose acetate phthalate, plastics, waxes, shellac, and fatty acids (e.g., stearic acid, palmitic acid).

Formulations for oral use may also be presented in a liquid dosage from. Liquid preparations can be in the form of, for example, aqueous or oily suspensions, solutions, emulsions syrups or elixirs, or can be presented as a dry product for reconstitution with water or other suitable vehicle before use. Such liquid preparations can contain conventional additives, such as suspending agents, for example sorbitol, methyl cellulose, glucose syrup, gelatin, hydroxyethyl cellulose, carboxymethyl cellulose, aluminum stearate gel or hydrogenated edible fats, emulsifying agents, for example lecithin, sorbitan monooleate, acacia; nonaqueous vehicles (which can include edible oils), for example almond oil, oily esters such as glycerine, propylene glycol, or ethyl alcohol; preservatives, for example methyl or propyl p-hydroxybenzoate or sorbic acid, and, if desired, conventional flavoring or coloring agents. In some embodiments, liquid formulations can comprise, for example, an agent in water-in-solution and/or suspension form; and a vehicle comprising polyethoxylated castor oil, alcohol, and/or a polyoxyethylated sorbitan mono-oleate with or without flavoring. Each dosage form may comprise an effective amount of a glycan polymer and can optionally comprise pharmaceutically inert agents, such as conventional excipients, vehicles, fillers, binders, disintegrants, pH adjusting substances, buffer, solvents, solubilizing agents, sweeteners, coloring agents, and any other inactive agents that can be included in pharmaceutical dosage forms for administration. Examples of such vehicles and additives can be found in Remington's Pharmaceutical Sciences, 17th edition (1985).

The pharmaceutical compositions provided herein can be in unit-dosage forms or multiple-dosage forms. A unit-dosage form, as used herein, refers to physically discrete unit suitable for administration to human in need thereof. In an embodiment, the unit-dosage form is provided in a package. Each unit-dose can contain a predetermined quantity of an active ingredient(s) sufficient to produce the desired therapeutic effect, in association with other pharmaceutical carriers or excipients. Examples of unit-dosage forms include, but are not limited to, ampoules, syringes, and individually packaged tablets and capsules. Unit-dosage forms can be administered in fractions or multiples thereof. A multiple-dosage form is a plurality of identical unit-dosage forms packaged in a single container, which can be administered in segregated unit-dosage form. Examples of multiple-dosage forms include, but are not limited to, vials, bottles of tablets or capsules, or bottles of pints or gallons. In another embodiment, the multiple dosage forms comprise different pharmaceutically active agents. For example, a multiple dosage form can be provided which comprises a first dosage element comprising a composition comprising a glycan polymer and a second dosage element comprising a prebiotic, a therapeutic agent and/or a probiotic, which can be in a modified release form. In this example a pair of dosage elements can make a single unit dosage. In one embodiment, a kit is provided comprising multiple unit dosages, wherein each unit comprises a first dosage element comprising a composition comprising a glycan polymer preparation and a second dosage element comprising probiotic, a pharmaceutical agent, a prebiotic or a combination thereof, which can be in a modified release form. In another embodiment, the kit further comprises a set of instructions.

In some embodiments, the unit-dosage form comprises between about 1 mg to about 100 g of the glycan polymer preparation (e.g., a glycan polymer disclosed herein). For example, the unit-dosage form may comprise about 50 mg to about 50 g, about 500 mg to about 50 g, about 5 g to about 50 g, about 100 mg to about 100 g, about 1 g to about 100 g, about 10 g to about 100 g, about 1 g to about 10 g, about 1 g to about 20 g, about 1 g to about 30 g, about 1 g to about 40 g, about 1 g to about 50 g, about 1 g to about 60 g, about 1 g to about 70 g, about 1 g to about 80 g, about 1 g to about 90 g, about 1 g to about 100 g, about 1 g to about 150 g, about 1 g to about 200 g of the glycan polymer.

In other embodiments, the unit-dosage form comprises between about 0.001 mL to about 1000 mL of the glycan polymer (e.g., a glycan polymer disclosed herein). For example, the unit-dosage form may comprise about 0.001 mL to about 950 mL, about 0.005 mL to about 900 mL, about 0.01 mL to about 850 mL, about 0.05 mL to about 800 mL, about 0.075 mL to about 750 mL, about 0.1 mL to about 700 mL, about 0.25 mL to about 650 mL, about 0.5 mL to about 600 mL, about 0.75 mL to about 550 mL, about 1 mL to about 500 mL, about 2.5 mL to about 450 mL, about 5 mL to about 400 mL, about 7.5 mL to about 350 mL, about 10 mL to about 300 mL, about 12.5 mL to about 250 mL, about 15 mL to about 200 mL, about 17.5 mL to about 150 mL, about 20 mL to about 100 mL, or about 25 mL to about 75 mL of the glycan polymer.

In certain embodiments, the unit-dosage form comprises about 0.001 mL to about 10 mL, about 0.005 mL to about 7.5 mL, about 0.01 mL to about 5 mL, about 0.05 mL to about 2.5 mL, about 0.1 mL to about 1 mL, about 0.25 mL to about 1 mL, or about 0.5 mL to about 1 mL of the glycan polymer. In other embodiments, the unit-dosage form comprises about 0.01 mL to about 10 mL, about 0.025 mL to about 7.5 mL, about 0.05 mL to about 5 mL, or about 0.1 mL to about 2.5 mL of the glycan polymer. In other embodiments, the unit-dosage form comprises about 0.1 mL to about 10 mL, about 0.25 mL to about 7.5 mL, about 0.5 mL to about 5 mL, about 0.5 mL to about 2.5 mL, or about 0.5 mL to about 1 mL of the glycan polymer.

In some embodiments, the unit-dosage form, e.g., a tablet, capsule (e.g., a hard capsule, push-fit capsule, or soft capsule), or softgel, has a body length of between about 0.1 inches to about 1.5 inches (e.g., about 0.5 inches and about 1 inch), or about 5 mm to about 50 mm (e.g., about 10 mm to about 25 mm). In some embodiments, the unit-dosage form. e.g., a tablet, capsule (e.g., a hard capsule, push-fit capsule, or soft capsule), or softgel, has an external diameter of about 0.05 inches to about 1 inch (e.g., about 0.1 inches to about 0.5 inches), or about 1 mm to about 25 mm (e.g., about 5 mm to about 10 mm).

Each unit-dosage form of the glycan polymer may have a caloric value of between about 0.01 kcal and about 1000 kcal. For example, the unit-dosage form may have a caloric value of about 0.01 kcal to about 100 kcal, about 0.05 kcal to about 50 kcal, about 0.1 kcal to about 10 kcal, about 0.25 kcal to about 2.5 kcal, about 0.5 kcal to about 5 kcal, about 0.75 kcal to about 7.5 kcal, about 1 kcal to 10 kcal, about 5 kcal to about 50 kcal, or about 10 kcal to about 100 kcal. In certain embodiments, the unit-dosage form of the glycan polymer has a caloric value of between 10 kcal to about 500 kcal.. In certain embodiments, the unit-dosage form of the glycan polymer has a caloric value of between 1 kcal to about 100 kcal. In certain embodiments, the unit-dosage form of the glycan polymer has a caloric value of between 0.1 kcal to about 10 kcal. In still other embodiments, the unit-dosage form may have a caloric value of about 0.001 kcal to about 10 kcal, about 0.005 kcal to about 10 kcal, about 0.01 kcal to about 10 kcal, about 0.025 kcal to about 25 kcal, about 0.05 kcal to about 50 kcal, about 0.075 kcal to about 75 kcal, about 0.1 kcal to 100 kcal, about 0.25 kcal to about 10 kcal, about 0.5 kcal to about 5 kcal, about 0.25 kcal to about 25 kcal, or about 0.1 kcal to about 1 kcal.

The unit-dosage form of the glycan polymer may be formulated to dissolve in an aqueous solution (e.g., water, milk, juice, and the like) and is orally administered as a beverage, syrup, solution, or suspension. For example, the unit-form dosage of the glycan polymer may comprise a cube, packet, lozenge, pill, tablet, capsule, candy, powder, elixir, or concentrated syrup formulated for dissolving into an aqueous solution prior to oral administration. In other embodiments, the unit-dosage form of the glycan polymer may comprise a cube, packet, lozenge, pill, tablet, capsule, candy, powder, elixir, or concentrated syrup formulated to dissolve in vivo, e.g., in the mouth, stomach, intestine, or colon of the subject (e.g., a human subject) upon oral administration.

In some embodiments, the glycan polymer preparation is administered enterically. This preferentially includes oral administration, or by an oral or nasal tube (including nasogastric, nasojejunal, oral gastric, or oral jejunal). In other embodiments, administration includes rectal administration (including enema, suppository, or colonoscopy).

The dosage forms described herein can be manufactured using processes that are known to those of skill in the art. For example, for the manufacture of tablets, an effective amount of a prebiotic can be dispersed uniformly in one or more excipients or additives, for example, using high shear granulation, low shear granulation, fluid bed granulation, or by blending for direct compression. Excipients and additives include diluents, binders, disintegrants, dispersants, lubricants, glidants, stabilizers, surfactants, antiadherents, sorbents, sweeteners, and colorants, or a combination thereof. Diluents, also termed fillers, can be used to increase the bulk of a tablet so that a practical size is provided for compression. Non-limiting examples of diluents include lactose, cellulose, microcrystalline cellulose, mannitol, dry starch, hydrolyzed starches, powdered sugar, talc, sodium chloride, silicon dioxide, titanium oxide, dicalcium phosphate dihydrate, calcium sulfate, calcium carbonate, alumina and kaolin. Binders can impart cohesive qualities to a tablet formulation and can be used to help a tablet remain intact after compression. Non-limiting examples of suitable binders include starch (including corn starch and pregelatinized starch), gelatin, sugars (e.g., glucose, dextrose, sucrose, lactose and sorbitol), celluloses, polyethylene glycol, alginic acid, dextrin, casein, methyl cellulose, waxes, natural and synthetic gums, e.g., acacia, tragacanth, sodium alginate, gum arabic, xantan gum, and synthetic polymers such as polymethacrylates, polyvinyl alcohols, hydroxypropylcellulose, and polyvinylpyrrolidone. Lubricants can also facilitate tablet manufacture; non-limiting examples thereof include magnesium stearate, calcium stearate, stearic acid, glyceryl behenate, and polyethylene glycol. Disintegrants can facilitate tablet disintegration after administration, and non-limiting examples thereof include starches, alginic acid, crosslinked polymers such as, e.g., crosslinked polyvinylpyrrolidone, croscarmellose sodium, potassium or sodium starch glycolate, clays, celluloses (e.g., carboxymethylcelluloses (e.g., carboxymethylcellulose (CMC), CMC-Na, CMC-Ca)), starches, gums and the like. Non-limiting examples of suitable glidants include silicon dioxide, talc, and the like. Stabilizers can inhibit or retard drug decomposition reactions, including oxidative reactions. Surfactants can also include and can be anionic, cationic, amphoteric or nonionic. Exemplary sweeteners may include stevia extract, aspartame, sucrose, alitame, saccharin, and the like. If desired, the tablets can also comprise nontoxic auxiliary substances such as pH buffering agents, preservatives, e.g., antioxidants, wetting or emulsifying agents, solubilizing agents, coating agents, flavoring agents (e.g., mint, cherry, anise, peach, apricot, licorice, raspberry, vanilla), and the like. Additional excipients and additives may include aluminum acetate, benzyl alcohol, butyl paraben, butylated hydroxy toluene, calcium disodium EDTA, calcium hydrogen phosphate dihydrate, dibasic calcium phosphate, tribasic calcium phosphate, candelilla wax, carnuba wax, castor oil hydrogenated, cetylpyridine chloride, citric acid, colloidal silicone dioxide, copolyvidone, corn starch, cysteine HCl, dimethicone, disodium hydrogen phosphate, erythrosine sodium, ethyl cellulose, gelatin, glycerin, glyceryl monooleate, glyceryl monostearate, glycine, HPMC pthalate, hydroxypropylcellulose, hydroxyl propyl methyl cellulose, hypromellose, iron oxide red or ferric oxide, iron oxide yellow, iron oxide or ferric oxide, magnesium carbonate, magnesium oxide, magnesium stearate, methionine, methacrylic acid copolymer, methyl paraben, silicified microcrystalline cellulose, mineral oil, phosphoric acid, plain calcium phosphate, anhydrous calcium phosphate, polaxamer 407, polaxamer 188, plain polaxamer, polyethylene oxide, polyoxy140 stearate, polysorbate 80, potassium bicarbonate, potassium sorbate, potato starch, povidone, propylene glycol, propylene paraben, propyl paraben, retinyl palmitate, saccharin sodium, selenium, silica, silica gel, fumed silica, sodium benzoate, sodium carbonate, sodium citrate dihydrate, sodium crossmellose, sodium lauryl sulfate, sodium metabisulfite, sodium propionate, sodium starch, sodium starch glycolate, sodium stearyl fumarate, sorbic acid, sorbitol, sorbitan monooleate, pregelatinized starch, succinic acid, triacetin, triethyl citrate, vegetable stearin, vitamin A, vitamin E, vitamin C, or a combination thereof. The amounts of these excipients and additives can be properly selected based on their relation to other components and properties of the preparation and production method.

Immediate-release formulations of an effective amount of a glycan polymer preparation can comprise one or more combinations of excipients that allow for a rapid release of a pharmaceutically active agent (such as from 1 minute to 1 hour after administration).Controlled-release formulations (also referred to as sustained release (SR), extended-release (ER, XR, or XL), time-release or timed-release, controlled-release (CR), or continuous-release) refer to the release of a glycan polymer preparation from a dosage form at a particular desired point in time after the dosage form is administered to a subject (e.g., a human subject).

In one embodiment a controlled release dosage form begins its release and continues that release over an extended period of time. Release can occur beginning almost immediately or can be sustained. Release can be constant, can increase or decrease over time, can be pulsed, can be continuous or intermittent, and the like. In one embodiment, a controlled release dosage refers to the release of an agent from a composition or dosage form in which the agent is released according to a desired profile over an extended period of time. In one aspect, controlled-release refers to delayed release of an agent from a composition or dosage form in which the agent is released according to a desired profile in which the release occurs after a period of time. Pharmaceutical carriers or vehicles suitable for administration of the compounds provided herein include all such carriers known to those skilled in the art to be suitable for the particular mode of administration. In addition, the compositions can one or more components that do not impair the desired action, or with components that supplement the desired action, or have another action.

In a further aspect, the dosage form can be an effervescent dosage form. Effervescent means that the dosage form, when mixed with liquid, including water and saliva, evolves a gas. Some effervescent agents (or effervescent couple) evolve gas by means of a chemical reaction which takes place upon exposure of the effervescent disintegration agent to water or to saliva in the mouth. This reaction can be the result of the reaction of a soluble acid source and an alkali monocarbonate or carbonate source. The reaction of these two general compounds produces carbon dioxide gas upon contact with water or saliva. An effervescent couple (or the individual acid and base separately) can be coated with a solvent protective or enteric coating to prevent premature reaction. Such a couple can also be mixed with previously lyophilized particles (such as a glycan polymer). The acid sources can be any which are safe for human consumption and can generally include food acids, acid and hydrite antacids such as, for example: citric, tartaric, amalic, fumeric, adipic, and succinics. Carbonate sources include dry solid carbonate and bicarbonate salt such as sodium bicarbonate, sodium carbonate, potassium bicarbonate and potassium carbonate, magnesium carbonate and the like. Reactants which evolve oxygen or other gasses and which are safe for human consumption are also included. In one embodiment citric acid and sodium bicarbonate are used.

In another aspect, the dosage form can be in a candy form (e.g., matrix), such as a lollipop or lozenge. In one embodiment an effective amount of a glycan polymer is dispersed within a candy matrix. In one embodiment the candy matrix comprises one or more sugars (such as dextrose or sucrose). In another embodiment the candy matrix is a sugar-free matrix. The choice of a particular candy matrix is subject to wide variation. Conventional sweeteners (e.g., sucrose), sugar alcohols suitable for use with diabetic patients (e.g., sorbitol or mannitol), or other sweeteners (e.g., sweeteners described herein) may be employed. The candy base can be very soft and fast dissolving, or can be hard and slower dissolving. Various forms will have advantages in different situations.

A candy mass composition comprising an effective amount of the glycan polymer can be orally administered to a subject in need thereof so that an effective amount of the glycan polymer will be released into the subject's mouth as the candy mass dissolves and is swallowed. A subject in need thereof includes a human adult or child.

The dosage forms described herein can also take the form of pharmaceutical particles manufactured by a variety of methods, including but not limited to high-pressure homogenization, wet or dry ball milling, or small particle precipitation (e.g., nGimat's NanoSpray). Other methods useful to make a suitable powder formulation are the preparation of a solution of active ingredients and excipients, followed by precipitation, filtration, and pulverization, or followed by removal of the solvent by freeze-drying, followed by pulverization of the powder to the desired particle size. In one embodiment, the pharmaceutical particles have a final size of 3-1000 microns, such as at most 3, 4, 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950, 1000 microns. In another embodiment, the pharmaceutical particles have a final size of 10-500 microns. In another embodiment, the pharmaceutical particles have a final size of 50-600 microns. In another embodiment, the pharmaceutical particles have a final size of 100-800 microns.

In another aspect, the disclosure provides a method of making a unit-dosage form described herein, comprising providing a glycan polymer (e.g., a glycan polymer described herein); formulating the glycan polymer into a unit-dosage form (e.g., a unit-dosage form described herein), packaging the unit-dosage form, labelling the packaged unit-dosage form, and/or selling or offering for sale the packaged and labeled unit-dosage form.

The unit-dosage forms described herein may also be processed. In one embodiment, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on whether the predetermined threshold is met. In some embodiments, the processed dosage forms comprise a glycan polymer described herein.

In some embodiments, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on the determination.

In another embodiment, an oral dosage form is provided comprising a glycan polymer preparation, wherein the oral dosage form is a syrup. The syrup can comprise about 1%, 5%, 10%, 15%, 20%, 25%, 30%, 35%, 40%, 45%, 50%, 55%, 60%, 65%, 70%, 75%, 80%, or 85% solid. The syrup can comprise about 15%, 20%, 25%, 30%, 35%, 40%, 45%, or 50% liquid, for example, water. The solid can comprise a glycan polymer preparation. The solid can be, for example, about 1-96%, 10-96%, 20-96%, 30-96%, 40-96%, 50-96%, 60-96%, 70-96%, 80-96%, or 90-96% glycan polymer preparation. In another embodiment, a glycan polymer preparation is formulated as a viscous fluid.

In one embodiment, the composition comprises a foaming component, a neutralizing component, or a water-insoluble dietary fiber. A foaming component can be at least one member selected from the group consisting of sodium hydrogencarbonate, sodium carbonate, and calcium carbonate. In one embodiment a neutralizing component can be at least one member selected from the group consisting of citric acid, L-tartaric acid, fumaric acid, L-ascorbic acid, DL-malic acid, acetic acid, lactic acid, and anhydrous citric acid. In one embodiment a water-insoluble dietary fiber can be at least one member selected from the group consisting of crystalline cellulose, wheat bran, oat bran, cone fiber, soy fiber, and beet fiber. The formulation can contain a sucrose fatty acid ester, powder sugar, fruit juice powder, and/or flavoring material.

In some embodiments, the dosage forms are formulated to release the pharmaceutical compositions comprising glycan polymer preparations in a specific region(s) of the GI tract, such as the small or the large intestine. In some embodiments, the dosage forms are formulated to release the pharmaceutical compositions comprising glycan polymer preparations in a specific region(s) of the GI tract, such as the cecum, ascending colon, transverse colon, descending colon, sigmoid colon, and/or rectum.

In some embodiments, the dosage form for the glycan polymer preparations described herein is an enzyme-responsive delivery system. For example, trypsin responsive polymers can be made using hydrogels that are crosslinked by peptides that are degraded by trypsin. Trypsin is active in the small intestine. Trypsin-responsive delivery systems can be used to target delivery of the glycan polymer preparations to the small intestine. In another example, enzyme-digestible hydrogels consisting of poly(vinyl pyrrolidone) crosslinked with albumin are degraded in the presence of pepsin.

In some embodiments, the dosage form for the glycan polymer preparations described herein is a delivery device that enables prolonged retention at a specific site in the GI tract. For example, a gastroretentive delivery system enables prolonged release of the glycan polymer preparations to the stomach. Gastroretentive delivery may be used for the glycan polymer preparations that modulate bacteria in the stomach or in the upper small intestine.

In some embodiments, the dosage form for the glycan polymer preparations described herein is a mucoadhesive delivery system that adheres to the mucosal surfaces of the stomach. They are typically composed of polymers with numerous hydrogen-bonding groups, e.g., cross-linked polyacrylic acids, sodium carboxymethyl cellulose, sodium alginate, carrageenan, Carbopol 934P, or thiolated polycarbophil.

In some embodiments, the dosage form for the glycan polymer preparations described herein is an expanding delivery system that rapidly increases in size in the stomach, which slows its passage through the pylorus. Such systems include systems that unfold in the stomach. For example, geometric shapes such as tetrahedrons, rings, disks, etc. can be packed into a gelatin capsule. When the capsule dissolves, the shape unfolds. The systems can be composed of one or more erodible polymer (e.g., hydroxypropyl cellulose), one or more nonerodible polymer (e.g., polyolefins, polyamides, polyurethanes). The glycan polymer may then be dispersed within the polymer matrix. The retention times can be fine-tuned by the polymer blend. Alternatively, devices made out of elastic polymers that are stable in the acidic pH of the stomach but dissolve in the neutral/alkaline conditions further along the GI tract can be used. Such polymer formulations can prevent intestinal obstruction when the device exits the stomach. Supramolecular polymer gels crosslinked by hydrogen bonds between carboxyl groups may also be used, e.g. composed of poly(acryloyl 6-aminocaproic acid) (PA6ACA) and poly(methacrylic acid-co-ethyl acrylate) (EUDRAGIT L 100-55). Other systems include swellable excipients, such as collagen sponges. For example, a hydrogel matrix (e.g. a swellable core: polyvinyl pyrrolidone XL, Carbopol 934P, calcium carbonate) swells 2-50 times in the stomach. Superporous hydrogel composites swell to hundreds of times their original volume in a few minutes. Some systems exploit gas generation to achieve expansion, e.g. carbon dioxide-generating, expandable systems that are surrounded by a hydrophilic membrane.

In some embodiments, the dosage form for the glycan polymer preparations described herein is a density-controlled delivery system. These systems are designed to either float or sink in gastric fluids, which delays their emptying from the stomach. For example, high-density systems enable the device to settle to the bottom of the stomach, below the pylorus, and thus avoid stomach emptying. Other systems are low-density/floating systems. Such devices may, e.g., comprise entrapped air in hollow chambers or may incorporate low-density materials like fats, oils, or foam powder. Low density may be achieved through swelling, e.g. hydrocolloid containing capsules dissolve upon contacting gastric fluid and the hydrocolloids swell to form a mucous body. Alternative polymers include: chitosans, sodium alginate, and glycerol monooleate matrix. Low density may be achieved through gas generation. For example, tablets loaded with carbonate and optionally citric acid generate carbon dioxide after contact with acidic aqueous media. The carbon dioxide generated is entrapped within the gelling hydrocolloid causing the system to float. Hydrocolloids include hydroxypropyl methylcellulose and Carbopol 934P.

In some embodiments, the dosage form for the glycan polymer preparations described herein employs a design to retain a device in the small or large intestine. The location-specific nature of the device is provided by a specific triggering method, e.g. pH, enzyme, etc. These include systems designed for mucoadhesion and also microneedle pills. Microneedle pills comprise a drug reservoir spiked with microneedles that is encapsulated in a pH-responsive coating. When the pill reaches the desired location in the GI tract and the coating dissolves, the microneedles enable the pill to become stuck to the lining of the GI tract. In other embodiments, the microneedle pills comprise a capsule that consists of two chemical compartments filled with citric acid and sodium bicarbonate, respectively. As the pill dissolves in the digestive system, barriers between the two substances erode, allowing them to mix and create a chemical reaction that pushes micro-needles of saccharides through the outer layer of the capsule and into the lining of the small intestine. The saccharide needles can be filled with drugs that are delivered into nearby blood vessels as the saccharide is absorbed.

In some embodiments, the dosage form for the glycan polymer preparations described herein employs a pH sensitive polymer coating. For example, pH-dependent polymers (bi- or tri-phasic) can be insoluble at low pH levels (e.g. acid resistance in the stomach, pH 1-2) and become increasingly soluble as pH rises, e.g. to about 5.5 - 6.2 in the duodenum, to about pH 5.7 in the ascending colon, to about pH 6.4 in the cecum, to about pH 6.6 in the transverse colon, to about pH 7.0 in the descending colon, to about 7.2 - 7.5 in the ileum, or to about pH 7.5 in the distal small intestine. In one example, TARGIT™ technology may be used for site-specific delivery of the glycan polymer preparations in the gastrointestinal (GI) tract. The system employs pH-sensitive coatings onto injection-moulded starch capsules to target the terminal ileum and colon. In some embodiments, the dosage form for the glycan polymer preparations described herein is a delayed release system or time controlled release system. Such systems usually employ enteric coatings that may be combined with pH sensitive and time release functions. For example, ETP (enteric coated time-release press coated) tablets may be used that are composed of three components: a glycan polymer-containing core tablet (rapid release function), a press-coated, swellable hydrophobic polymer layer (e.g. hydroxypropyl cellulose layer (HPC), and a time release function. The duration of lag phase can be controlled either by weight or composition of polymer layer and an enteric coating layer (acid resistance function).

In some embodiments, the dosage form for the glycan polymer preparations described herein employs Eudragit® enteric coatings of tablets and capsules. Other suitable synthetic polymers include: Shellac, ethyl cellulose, cellulose acetate phthalate, hydroxypropylmethyl cellulose, polyvinyl acetate phthalate and poly glutamic acid coatings, such as poly-γ-glutamic acid (y-PGA). These coatings combine both mucoadhesive and pH-dependent release strategies. To enhance colon targeted delivery Eudragits® are methacrylic co-polymers with varying side group compositions that alter the pH at which they are soluble. For example, for Eudragit®-coated systems no significant drug release occurs in the stomach (e.g. at pH 1.4) and in the small intestine (e.g. at pH 6.3), while significant drug release can be seen at pH 7.8 in the ileocaecal region.

In some embodiments, the dosage form for the glycan polymer preparations described herein is a microbial-triggered system, such as a polysaccharide based delivery system. Polysaccharide based delivery systems contain biodegradable and mucoadhesive polymer coatings, including coatings of chitosan and pectin. Other suitable natural polymers include, e.g., guar gum, inulin, cyclodextrin, dextran, amylase, chondrotin sulphate, and locust bean gum. These delivery systems can be used to target the glycan polymer to the small intestine. Coatings with naturally occurring polysaccharides like guar gum, xanthan gum, chitosan, alginates, etc. are degraded by colonic gut microbiota, e.g. enzymes such as, xylosidase, arabinosidase, galactosidase etc. For example, CODES™ technology may be used to deliver the glycan polymer preparations. This system combines the polysaccharide coating with a pH-sensitive coating. In some embodiments, the system consists of a core tablet coated with three layers of polymer coatings: The outer coating is composed of Eudragit L. This coating gets dissolved in the duodenum and exposes the next coating. The next coating is composed of Eudragit E. This layer allows the release of lactulose present in the inner core. The lactulose gets metabolized into short chain fatty acids that lower the surrounding pH where the Eudragit E layer dissolves. The dissolving of Eudragit E results in the exposure of the glycan polymer. The bacteria present in the colon are responsible for the degradation of polysaccharides that are released from the core tablet. The degradation of polysaccharides may result in organic acids formation that lowers the pH of the contents surrounding the tablet.

In some embodiments, the dosage form for the glycan polymer preparations described herein is a pressure-controlled delivery system. The system employs the fact that higher pressures are encountered in the colon than in the small intestine. For example, for ethylcellulose systems that are insoluble in water, the release of glycan polymers occurs following disintegration of a water-insoluble polymer capsule as a result of pressure in the lumen of the colon. The release profile may be adjusted by varying the thickness of the ethylcellulose, the capsule size and/or density of the capsule.

In some embodiments, the dosage form for the glycan polymer preparations described herein is a pulsatile colon targeted delivery system. For example, the system can be a pulsincap system. The capsule which is employed comprises a plug that is placed in the capsule that controls the release of the glycan polymer. A swellable hydrogel (e.g. hydroxyl propyl methyl cellulose (HPMC), poly methyl methacrylate or polyvinyl acetate) seals the drug content. When the capsule gets in contact with a fluid the plug is pushed off from the capsule and the glycan polymer is released. The release profile can be controlled by varying the length and/or point of intersection of the plug with the capsule body. Another system is a port system. The capsule body is enclosed in a semi-permeable membrane. The insoluble plug consists of an osmotically active agent and the glycan polymer. When the capsule gets in contact with a fluid the semi-permeable membrane permits inflow of the fluid which increases pressure in the capsule body. This leads to an expelling of the plug and release of the glycan polymer.

In some embodiments, the dosage form for the glycan polymer preparations described herein is an osmotically controlled colon targeted delivery system. An exemplary system, OROS-CT, consists of osmotic units (up to 5 or 6 push pull units) encapsulated in a hard gelatin capsule. The push pull units are bilayered with outer enteric impermeable membrane and inner semi-permeable membrane. The internal, central part of the push pull consists of the drug layer and push layer. The glycan polymer is released through the semi-permeable membrane. The capsule body enclosing the push pull units is dissolved immediately after administration. In the GI tract the enteric impermeable membrane prevents water absorption. The enteric coating is dissolved in small intestine (higher pH, >7), water enters the unit through the semi-permeable membrane causing push layer to swell and force out the glycan polymer.

In some embodiments, the dosage form for the glycan polymer preparations described herein is "smart pill" which can be used to release the glycan polymer just before reaching the ileocecal valve.

In some embodiments, the dosage form for the glycan polymer preparations described herein is a rectally administered formulation. For example, enemas introduce a glycan polymer preparation in liquid formulation into the rectum. The volume administered is typically less than 10 mL. Suppositories introduce a glycan polymer preparation into the rectum. Suppositories are solid dosage forms that melt or dissolve when inserted into the rectum, releasing the glycan polymers. Typical excipients for suppository formulations include cocoa butter, polyethylene glycols, and agar.

**Kits**

Kits also are contemplated. For example, a kit can comprise unit dosage forms of the glycan polymer preparation, and a package insert containing instructions for use of the glycan polymer in treatment of a gastrointestinal disorder or condition. The kits include a glycan polymer preparation in suitable packaging for use by a subject (e.g., a human subject) in need thereof. Any of the compositions described herein can be packaged in the form of a kit. A kit can contain an amount of a glycan polymer preparation (optionally additionally comprising a prebiotic substance, a probiotic bacterium, and/or a second therapeutic agent) sufficient for an entire course of treatment, or for a portion of a course of treatment. Doses of a glycan polymer preparation can be individually packaged, or the glycan polymer preparation can be provided in bulk, or combinations thereof. Thus, in one embodiment, a kit provides, in suitable packaging, individual doses of a glycan polymer preparation that correspond to dosing points in a treatment regimen, wherein the doses are packaged in one or more packets.

In one embodiment, the glycan polymer preparation can be provided in bulk in a single container, or in two, three, four, five, or more than five containers. For example, \each container may contain enough of a glycan polymer preparation for a particular week of a treatment program that runs for a month. If more than one bulk container is provided, the bulk containers can be suitably packaged together to provide sufficient glycan polymer preparation for all or a portion of a treatment period. The container or containers can be labeled with a label indicating information useful to the subject in need thereof or the physician performing the treatment protocol, such as, e.g. dosing schedules.

The glycan polymer preparation can be packaged with other suitable substances, such as probiotic bacteria, prebiotic substances or other substances, as described herein. The other substance or substances can be packaged separately from the glycan polymer preparation, or mixed with the glycan polymer preparation, or combinations thereof. Thus, in one embodiment, kits include a dosage form containing all the ingredients intended to be used in a course of treatment or a portion of a course of treatment, e.g., a glycan polymer preparation and optionally buffers, excipients, etc., a probiotic, prebiotic or a polymer agent. In one embodiment, a glycan polymer preparation is packaged in one package or set of packages, and additional components, such as probiotic bacteria, prebiotics, and therapeutic agents are packaged separately from the glycan polymer preparation.

Kits can further include written materials, such as instructions, expected results, testimonials, explanations, warnings, clinical data, information for health professionals, and the like. In one embodiment, the kits contain a label or other information indicating that the kit is only for use under the direction of a health professional. The container can further include scoops, syringes, bottles, cups, applicators or other measuring or serving devices.

**Identification of bacterial constituents**

In some embodiments, the glycan polymer preparations described herein are administered to a subject (e.g., a human subject) to increase the growth of beneficial bacteria, decrease the growth of pathogens and/or modulate a (microbial) metabolite (such as, e.g., SCFAs, ammonia, TMA/TMAO, bile acids, LPS) in the GI tract. In some embodiments, the microbial community is shifted by the glycan polymer toward that of a healthy state. The microbial changes occurring in the GI tract can be analyzed using any number of methods known in the art and described herein. As one quantitative method for determining whether a glycan polymer preparation results in a shift of the population of bacteria in the GI tract, quantitative PCR (qPCR) can be performed. Genomic DNA can be extracted from samples using commercially-available kits, such as the Mo Bio Powersoil®-htp 96 Well Soil DNA Isolation Kit (Mo Bio Laboratories, Carlsbad, CA), the Mo Bio Powersoil® DNA Isolation Kit (Mo Bio Laboratories, Carlsbad, CA), orthe QIAamp DNA Stool Mini Kit (QIAGEN, Valencia, CA) according to the manufacturer's instructions, or by other standard methods known to those skilled in the art.

In some embodiments, qPCR can be conducted using HotMasterMix (5PRIME, Gaithersburg, MD) and primers specific for certain (e.g. beneficial or desired) bacteria and may be conducted on a MicroAmp® Fast Optical 96-well Reaction Plate with Barcode (0.1mL) (Life Technologies, Grand Island, NY) and performed on a BioRad C1000™ Thermal Cycler equipped with a CFX96™ Real-Time System (BioRad, Hercules, CA), with fluorescent readings of the FAM and ROX channels. The Cq value for each well on the FAM channel is determined by the CFX Manager™ software version 2.1. The $\log_{10}$(cfu/ml) of each experimental sample is calculated by inputting a given sample's Cq value into linear regression model generated from the standard curve comparing the Cq values of the standard curve wells to the known $\log_{10}$(cfu/ml) of those samples. The skilled artisan may employ alternative qPCR modes.

In some embodiments, the microbial constituents are identified by characterizing the DNA sequence of microbial 16S small subunit ribosomal RNA gene (16S rRNA gene). 16S rRNA gene is approximately 1,500 nucleotides in length, and in general is highly conserved across organisms, but contain specific variable and hypervariable regions (V1-V9) that harbor sufficient nucleotide diversity to differentiate species- and strain-level taxa of most organisms. These regions in bacteria are defined by nucleotides 69-99, 137-242, 433-497, 576-682, 822-879, 986-1043, 1117-1173, 1243-1294 and 1435-1465 respectively using numbering based on the E. coli system of nomenclature. (See, e.g., Brosius et al., Complete nucleotide sequence of a 16S ribosomal RNA gene from Escherichia coli, PNAS 75(10):4801-4805 (1978)).

Composition of a microbial community can be deduced by sequencing full 16S rRNA gene, or at least one of the V1, V2, V3, V4, V5, V6, V7, V8, and V9 regions of this gene or by sequencing of any combination of variable regions from this gene (e.g. V1-3 or V3-5). In one embodiment, the V1, V2, and V3 regions are used to characterize a microbiota.

In another embodiment, the V3, V4, and V5 regions are used to characterize a microbiota. In another embodiment, the V4 region is used to characterize a microbiota.

Sequences that are at least 97% identical to each other are grouped into Operational Taxonomic Units (OTUs). OTUs that contain sequences with 97% similarity correspond to approximately species level taxa. At least one representative sequence from each OTU is chosen, and is used to obtain a taxonomic assignment for an OTU by comparison to a reference database of highly curated 16S rRNA gene sequences (such as Greengenes or SILVA databases). Relationship between OTUs in a microbial community could be deduces by constructing a phylogenetic tree from representative sequences from each OTU.

Using known techniques, in order to determine the full 16S sequence or the sequence of any variable region of the 16S sequence, genomic DNA is extracted from a bacterial sample, the 16S rRNA (full region or specific variable regions) amplified using polymerase chain reaction (PCR), the PCR products are cleaned, and nucleotide sequences delineated to determine the genetic composition of 16S rRNA gene or a variable region of the gene. If full 16S sequencing is performed, the sequencing method used may be, but is not limited to, Sanger sequencing. If one or more variable regions is used, such as the V4 region, the sequencing can be, but is not limited to being performed using the Sanger method or using a next-generation sequencing method, such as an Illumina method. Primers designed to anneal to conserved regions of 16S rRNA genes (e.g., the 515F and 805R primers for amplification of the V4 region) could contain unique barcode sequences to allow characterizing multiple microbial communities simultaneously.

As another method to identify microbial composition is characterization of nucleotide markers or genes, in particular highly conserved genes (e.g., "house-keeping" genes), or a combination thereof, or whole genome shotgun sequence (WGS). Using defined methods, DNA extracted from a bacterial sample will have specific genomic regions amplified using PCR and sequenced to determine the nucleotide sequence of the amplified products. In the WGS method, extracted DNA will be fragmented into pieces of various lengths (from 300 to about 40,000 nucleotides) and directly sequenced without amplification. Sequence data can be generated using any sequencing technology including, but not limited to Sanger, Illumina, 454 Life Sciences, Ion Torrent, ABI, Pacific Biosciences, and/or Oxford Nanopore.

In addition to the 16S rRNA gene, a selected set of genes that are known to be marker genes for a given species or taxonomic group is analyzed to assess the composition of a microbial community. These genes are alternatively assayed using a PCR-based screening strategy. For example, various strains of pathogenic *Escherichia coli* are distinguished using genes that encode heat-labile (LTI, LTIIa, and LTIIb) and heat-stable (STI and STII) toxins, verotoxin types 1, 2, and 2e (VT1, VT2, and VT2e, respectively), cytotoxic necrotizing factors (CNF1 and CNF2), attaching and effacing mechanisms (*eaeA*), enteroaggregative mechanisms (Eagg), and enteroinvasive mechanisms (Einv). The optimal genes to utilize to determine the taxonomic composition of a microbial community by use of marker genes are familiar to one with ordinary skill in the art of sequence based taxonomic identification.

Sequencing libraries for microbial whole-genome sequencing (WGS) may be prepared from bacterial genomic DNA. For genomic DNA that has been isolated from a human or laboratory animal sample, the DNA may optionally enriched for bacterial DNA using commercially available kits, for example, the NEBNext Microbiome DNA Enrichment Kit (New England Biolabs, Ipswich, MA) or other enrichment kit. Sequencing libraries may be prepared from the genomic DNA using commercially available kits as well, such as the Nextera Mate-Pair Sample Preparation Kit, TruSeq DNA PCR-Free or TruSeq Nano DNA, or the Nextera XT Sample Preparation Kit (Illumina, San Diego, CA) according to the manufacturer's instructions. Alternatively, libraries can be prepared using other kits compatible with the Illumina sequencing platform, such as the NEBNext DNA Library Construction Kit (New England Biolabs, Ipswich, MA). Libraries may then be sequenced using standard sequencing technology including, but not limited to, a MiSeq, HiSeq or NextSeq sequencer (Illumina, San Diego, CA).

Alternatively, a whole-genome shotgun fragment library prepared using standard methods in the art. For example, the shotgun fragment library could be constructed using the GS FLX Titanium Rapid Library Preparation Kit (454 Life Sciences, Branford, CT), amplified using a GS FLX Titanium emPCR Kit (454 Life Sciences, Branford, CT), and sequenced following standard 454 pyrosequencing protocols on a 454 sequencer (454 Life Sciences, Branford, CT).

Bacterial RNA may be isolated from microbial cultures or samples that contain bacteria by commercially available kits, such as the RiboPure Bacterial RNA Purification Kit (Life Technologies, Carlsbad, CA). Another method for isolation of bacterial RNA may involve enrichment of mRNA in purified samples of bacterial RNA through remove of tRNA. Alternatively, RNA may be converted to cDNA, which used to generate sequencing libraries using standard methods such as the Nextera XT Sample Preparation Kit (Illumina, San Diego, CA).

Nucleic acid sequences are analyzed to define taxonomic assignments using sequence similarity and phylogenetic placement methods or a combination of the two strategies. A similar approach is used to annotate protein names, protein function, transcription factor names, and any other classification schema for nucleic acid sequences. Sequence similarity based methods include BLAST, BLASTx, tBLASTn, tBLASTx, RDP-classifier, DNAclust, RapSearch2, DIAMOND, USEARCH, and various implementations of these algorithms such as QIIME or Mothur. These methods map a sequence read to a reference database and select the best match. Common databases include KEGG, MetaCyc, NCBI non-redundant database, Greengenes, RDP, and Silva for taxonomic assignments. For functional assignments, reads are mapped to various functional databases such as COG, KEGG, BioCyc, MetaCyc, and the Carbohydrate-Active Enzymes (CAZy) database. Microbial clades are assigned using software including MetaPhlAn.

*Proteomic Analysis of MicrobialPopulations*

Preparations of glycan polymers may be selected based on their ability to increase the expression of microbial proteins associated with healthy states or to decrease the expression of microbial proteins associated with diseased states. Proteomic analysis of microbial populations can be performed following protocols known to one skilled in the art (e.g., Cordwell, Exploring and exploiting bacterial proteomes, Methods in Molecular Biology, 2004, 266:115). To identify differentially expressed proteins (for example, to identify changes in protein expression upon treatment of microbial populations with glycan polymers), proteomic analysis can be performed as described, e.g., in Juste et al. (Bacterial protein signals are associated with Crohn's disease, Gut, 2014, 63:1566). For example, the protein is isolated from the microbial lysates of two samples (for example, an untreated microbial population and a population that has been treated with glycan polymers). Each protein sample is labeled (e.g., with a fluorescent dye, e.g., Cy3 or Cy5 CyDye DIGE Fluor minimal dye, GE Healthcare) and analyzed by two-dimensional differential gel electrophoresis (2D-DIGE). Gels are stained and protein spots identified as being significantly different between the two samples are excised, digested, and analyzed by liquid chromatography-tandem mass spectrometry (LC-MS/MS). X!TandemPipeline (http://pappso.inra.fr/bioinfo/xtandempipeline/) can be used to identify differentially expressed proteins.

Preparations of glycan polymers may also be selected for administration to a human subject based on their effect on the presence of microbial products. For example, preparations of glycan polymers can be selected for their ability to induce or promote growth of bacteria that produce short chain fatty acids such as propionate (propionic acid), acetate, and/or butyrate (butyric acid).

Similarly, preparations of glycan polymers can be selected for their ability to induce or promote growth of bacteria that produce lactic acid, which can modulate the growth of other bacteria by producing an acidic environment and also is also utilized by butyrate producing taxa. Such analysis may also be used to pair probiotic bacteria with glycan polymers such that the glycan polymer is a substrate for the production of the desired fermentation products. In some embodiments, glycan polymers may also be selected for administration to a human subject based on their effect on bacterial taxa that do not produce an unwanted metabolite, such as, e.g. ammonia, a uremic solute, TMA and similar. In some embodiments, the glycan polymers increase growth of bacterial taxa that do not produce an unwanted metabolite thereby out-competing (e.g. for space and nutrients) bacterial taxa that produce the unwanted metabolite. By shifting the balance of non-producers to producers in favor of non-producers the overall level of the unwanted metabolite can be reduced. In some embodiments, the balance of SCFA producers to non-producer taxa is shifted toward SCFA producers to increase the level of SCFA production (e.g., butyrate, acetate, propionate). In some embodiments, the balance of ammonia producers to non-producer taxa is shifted toward non-producers (e.g. urease negative bacterial taxa) to decrease the level of ammonia production. In some embodiments, the balance of TMA producers to non-producer taxa is shifted toward non-producers to decrease the level of TMA production. The metabolites that are present in fresh or spent culture media or in biological samples collected from humans may be determined using methods described herein. Unbiased methods that may be used to determine the relative concentration of metabolites in a sample and are known to one skilled in the art, such as gas or liquid chromatography combined with mass spectrometry or $^1$H-NMR. These measurements may be validated by running metabolite standards through the same analytical systems.

In the case of gas chromatography-mass spectrometry (GC-MS) or liquid-chromatography-mass spectrometry (LC-MS) analysis, polar metabolites and fatty acids could be extracted using monophasic or biphasic systems of organic solvents and an aqueous sample and derivatized (Fendt et al., Reductive glutamine metabolism is a function of the $\alpha$-ketoglutarate to citrate ratio in cells, Nat Commun, 2013, 4:2236; Fendt et al., Metformin decreases glucose oxidation and increases the dependency of prostate cancer cells on reductive glutamine metabolism, Cancer Res, 2013, 73:4429; Metallo et al., Reductive glutamine metabolism by IDH1 mediates lipogenesis under hypoxia, Nature, 2011, 481:380). An exemplary protocol for derivatization of polar metabolites involves formation of methoxime-tBDMS derivatives through incubation of the metabolites with 2% methoxylamine hydrochloride in pyridine followed by addition of N-tert-butyldimethylsilyl-N-methyltrifluoroacetamide (MTBSTFA) with 1% tert-butyldimethylchlorosilane (t-BDMCS). Non-polar fractions, including triacylglycerides and phospholipids, may be saponified to free fatty acids and esterified to form fatty acid methyl esters, for example, either by incubation with 2% $H_2SO_4$ in methanol or by using Methyl-8 reagent (Thermo Scientific). Derivatized samples may then be analyzed by GC-MS using standard LC-MS methods, for example, a DB-35MS column (30 m x 0.25 mm i.d. x 0.25 $\mu$m, Agilent J&W Scientific) installed on a gas chromatograph (GC) interfaced with an mass spectrometer (MS). Mass isotopomer distributions may be determined by integrating metabolite ion fragments and corrected for natural abundance using standard algorithms, such as those adapted from Fernandez et al. (Fernandez et al., Correction of 13C mass isotopomer distributions for natural stable isotope abundance, J Mass Spectrom, 1996, 31 :255). In the case of liquid chromatography-mass spectrometry (LC-MS), polar metabolites may be analyzed using a standard benchtop LC-MS/MS equipped with a column, such as a SeQuant ZIC-pHILIC Polymeric column (2.1 × 150 mm; EMD Millipore). Exemplary mobile phases used for separation could include buffers and organic solvents adjusted to a specific pH value.

In combination or in the alternative, extracted samples may be analyzed by $^1$H-nuclear magnetic resonance ($^1$H-NMR). Samples may be combined with isotopically enriched solvents such as D2O, optionally in the presence of a buffered solution (e.g., $Na_2HPO_4$, $NaH_2PO_4$ in $D_2O$, pH 7.4). Samples may also be supplemented with a reference standard for calibration and chemical shift determination (e.g., 5 mM 2,2-dimethyl-2-silapentane-5-sulfonate sodium salt (DSS-d$_6$, Isotec, USA)). Prior to analysis, the solution may be filtered or centrifuged to remove any sediment or precipitates, and then transferred to a suitable NMR tube or vessel for analysis (e.g., a 5 mm NMR tube). $^1$H-NMR spectra may be acquired on a standard NMR spectrometer, such as an Avance II + 500 Bruker spectrometer (500 MHz) (Bruker, DE), equipped with a 5 mm QXI-Z C/N/P probe-head) and analyzed with spectra integration software (such as Chenomx NMR Suite 7.1; Chenomx Inc., Edmonton, AB). (Duarte et al., 1H-NMR protocol for exometabolome analysis of cultured mammalian cells, Methods Mol Biol, 2014:237-47).

Alternatively, $^1$H-NMR may be performed following other published protocols known in the art (Chassaing et al., Lack of soluble fiber drives diet-induced adiposity in mice, Am J Physiol Gastrointest Liver Physiol, 2015; Bai et al., Comparison of Storage Conditions for Human Vaginal Microbiome Studies, PLoS ONE, 2012:e36934).

## Administration

In some embodiments, a glycan polymer is administered to a subject (e.g., a human subject) in need thereof by enteral administration. In some embodiments, a glycan polymer is administered to a subject in need thereof by oral, nasal, gastric or rectal administration. In some embodiments, a glycan polymer is administered to a subject in need thereof by tube feeding.

In some embodiments, a glycan polymer is administered to a subject in need thereof immediately after one or more drug treatment(s) has ended (e.g. 1 hour, 6 hours, 12 hours, 24 hours, 36 hours, 48 hours, 3 days, 4 days, 5 days, 6 days, 7 days, 2 weeks, 3 weeks or 4 weeks after the antibiotic treatment has ended). During a course of drug treatment, the glycan polymer preparation may be provided prior to the initiation of drug treatment (e.g. 1, 2, 3, 4, 5, 6, 7 days prior); at the day of initiation of drug treatment; or shortly following antibiotic treatment, e.g. 1, 2, 3, 4, 5, 6, 7, or more days following treatment, and may optionally be provided only initially (e.g. for a short period) or throughout the duration of the drug-treatment, and may even be continued for a desired period after the drug treatment period has ended (e.g. for 1-7days, 1-14 days, or 1-21 days thereafter). In some embodiments, administration of the glycan polymer preparation is initiated or continued when one or more adverse effects occur and/or are diagnosed (e.g. digestive abnormalities or pathogen growth) in conjunction with the drug treatment. In some embodiments, the treatment agent causing a dysbiosis is not a drug but radiation treatment or surgery and the glycan polymer preparation may also be administered as described herein.

In some embodiments, the total number and duration of treatment periods is based on a subject's response to the treatment. For example, an individual can experience a reduction in symptoms after 1, 2, 3, 4, 5, 6, 7, 8, 9, 10, 11, 12, 13, or 14 days of treatment with a glycan polymer preparation. In another example, an individual can experience a reduction in symptoms after 1, 2, 3, 4, 5, 6, 7, 8, 9, 10, 11, 12 months of treatment with a glycan polymer preparation. Thus, the duration of treatment is determined by an individual subject's response to a glycan polymer preparation and the onset of relief from one or more symptoms. Thus, a subject can experience symptoms at a given dose of a glycan polymer preparation and can require that the subject stay at that dose, or a lower dose, until symptoms subside. Thus, in one embodiment, the duration of the treatment is not determined at the outset, but continues until the maximum dose of a glycan polymer preparation is achieved per day, or until the desired level of reduction in symptoms is achieved. In one embodiment, the treatment is continuous.

In one embodiment, a subject (e.g., a human subject) can be given one dose for the first treatment period during a treatment regimen and a second dose during a second treatment period. For example, a subject can be administered one dose of glycan polymer preparation for a one week period and a second dose for a subsequent one week period.

A subject may self-administer a glycan polymer preparation and the glycan polymer preparation is supplied or recommended (or prescribed) by a health professional, e.g., a physician or other qualified health professional and optionally test results (e.g. obtained for biomarkers from samples taken from the subject) and/or health changes and treatment endpoints are monitored by a health professional. In some embodiments, the glycan polymer preparation is administered by a health professional.

In one embodiment, a subject in need thereof can undergo repeated courses of treatment with a glycan polymer preparation. The course of treatment can be repeated when symptoms reappear or increase to an undesirable level. Alternatively, the course of treatment can be repeated at regular or predetermined intervals. Thus, treatment can be repeated after about one month, two months, three months, four months, six months, eight months, ten months, one year, 18 months, two years, three years, four years, five years, or more than five years, or any combination thereof (e.g.,

treatment can be repeated after one year, then every two to five years thereafter). The treatment can be repeated in the same form (e.g., duration, dosage, timing of dosage, additional substances, etc.) as used in the first treatment or it can be modified. For example, treatment duration can be shortened or lengthened, dosage can be increased or decreased.

In some embodiments, the pharmaceutical composition is administered one, two, or three times a day. In some embodiments, the pharmaceutical composition is administered twice a day. In some embodiments, the pharmaceutical composition is administered each day for a predetermined number of days (the treatment period). In some embodiments, the treatment period is 1, 2, 3, 4, 5, 6, 7, 8, 9, 10, 11, 12, 13, 14, 21, 28, 35, 42, 49, 56, 63, 70, 100, 200, 300 or 365 days. In some embodiments, the treatment period is 1, 2, 3, 4, 5, 6, 7, 8, 9, 10, 11, or 12 months. In some embodiments, the treatment period is 1, 2, 3, 4, 5, 6, 7, 8, 9, 10, 11, 12 years, or life-long.

In one embodiment the total duration of treatment periods for a gastrointestinal disease, disorder or condition can be from about one day to 10 years, one day to 1 year, 1 day to 6 months, 1 day to 3 months, 1 day to 1 months, one day to one week, one day to five days, one day to 10 days, one week to about 12 weeks, or about four weeks to about ten weeks, or about four weeks to about eight weeks, or about six weeks. The subject (e.g., a human subject) may undergo a suitable number of treatment periods, such as, e.g. 1, 2, 3, 4, 5, 6, 7, 8, 9, 10 or more than 10 treatment periods. During a treatment period, the subject takes a glycan polymer preparation described herein, optionally along with ingestion of prebiotic and/or probiotic containing food products. In one embodiment, a glycan polymer preparation can also be administered in combination with another substance (such as a probiotic or commensal beneficial bacteria, a prebiotic substance or a therapeutic agent), as described herein.

In some embodiments, the glycan polymer preparation may also be combined with an antibiotic that disrupts normal gastrointestinal microbiota growth. Typically durations for antibiotic treatments are 1-14 days, or 2-10 days, or 5-7 days. In some embodiments, a glycan polymer is administered to a subject in need thereof immediately after one or more antibiotic treatment(s) has ended (e.g. 1 hour, 6 hours, 12 hours, 24 hours, 36 hours, 48 hours, 3 days, 4 days, 5 days, 6 days, 7 days, 2 weeks, 3 weeks or 4 weeks after the antibiotic treatment has ended). During a course of antibiotic treatment, the glycan polymer preparation may be provided at the initiation of antibiotic treatment; shortly following antibiotic treatment, e.g. 1, 2, 3, 4, 5, 6, 7, or more days following treatment; or may be administered upon diagnosis of undesirable pathogen growth.

**Methods of Treatment**

Provided herein are methods for treating a subject (e.g., a human subject) having a disease or disorder. In some embodiments, the disease or disorder is associated with a level (e.g., an unwanted level) of a metabolite (e.g., a short chain fatty acid (SCFA), ammonia, trimethylamine (TMA), trimethylamine N-oxide (TMAO), a uremic solute, lipopolysaccharide (LPS) or a bile acid). The methods, in some embodiments, include administering to the human subject a glycan polymer preparation in an amount effective to treat the disease or disorder. In some embodiments, the glycan polymer preparation (e.g., described herein) is beneficial in the treatment of various diseases, disorders or conditions. Such disease, disorders or conditions may be associated with a dysbiosis of the microbiota. Disturbances in beneficial microbiota can occur due to a variety of factors (e.g., genetic or environmental) including, but not limited to, use of antibiotics, chemotherapeutics and other dysbiosis-inducing drugs or treatments (e.g., radiation treatment), pathogen infection, pathobiont activity, miscalibrated caloric intake (e.g., high-fat, high-sugar), miscalibrated (non-digestible) fiber intake (e.g. low or zero fiber), host factors (e.g. host genetic alterations), and similar. In some embodiments, the disease, disorder or condition is associated with a dysbiosis of the gastrointestinal microbiota. In some embodiments, by treating the dysbiosis the disease, disorder or condition is treated. Symptoms that may be associated with a dysbiosis of the gastrointestinal microbiota and/or with a gastrointestinal disease, disorder or condition include, but are not limited to gas, heartburn, stomach upset, bloating, flatulence, diarrhea, abdominal pain, cramping, nausea, and vomiting. Minor digestive problems related to the GI also include occasional bloating, diarrhea, constipation, gas, or stomach upset.

*Indications associated with metabolites*

In some embodiments, the disease or disorder is associated with a level (e.g., an unwanted level) of a metabolite. Metabolites, such as a short chain fatty acid (SCFA), ammonia, trimethylamine (TMA), trimethylamine N-oxide (TMAO), a uremic solute, lipopolysaccharide, or a bile acid, and the bacteria that produce them have been associated with a range of diseases. For example, reduced levels of butyrate-producing bacteria have been reported in Crohn's Disease (Takahashi et al., (2016)), and levels of butyrate and propionate are reportedly reduced and acetate is increased in fecal samples from patients with Crohn's Disease (Galecka et al., (2013)). Butyrate has been reported to decrease proinflammatory cytokine expression, which may play an important role in inflammatory bowel disease, including Crohn's Disease (Russo I. et al. PLoS One 2012). Other diseases associated with decreased levels of butyrate relative to healthy patient populations include ulcerative colitis (Kumari et al., 2013), Type 2 Diabetes (Qin et al., 2012), atopic dermatitis (Song et al., 2016), colorectal cancer (Wang et al., 2012) and Parkinson's disease (Keshavarzian et al., 2015). Administration of glycans that support the growth of microbiota positively associated with butyrate production, directly or indirectly, to individuals may increase butyrate levels *in vivo* and improve or prevent symptoms of Crohn's disease.

In some embodimens, it may also be beneficial to administer glycans that reduce production of one or more short chain fatty acids to treat some diseases. Butyrate producing bacteria are reportedly increased in obese patients relative to healthy individuals (Ross et al., 2015), and butyrate and propionate have been found to be increased in the stools of obese patients relative to healthy patients (Payne et al., 2011); likewise, increased levels of acetate have been associated with obesity (Gao et al., 2014). Administration of glycans that selectively decrease microbiota associated directly or indirectly with increased butyrate, propionate and/or acetate thus may be useful in treating or preventing obesity. Other diseases associated with relatively high levels of acetate include Malabsorption Syndrome (Bala et al., 2006), colorectal cancer (Weir et al., (2013) and Crohn's Disease (Galecka et al., 2013). Administration of glycans to individuals to selectively decrease microbiota associated directly or indirectly with increased acetate may be useful in treating or preventing diseases associated with increased levels of acetate, such as obesity, malabsorption syndrome, colorectal cancer and Crohn's disease.

In some embodiments, the disease or disorder is associated with a level (e.g., an unwanted level) of a short chain fatty acid, e.g., and is selected from acute pouchitis, allergic diseases, AIDS, atherosclerosis, asthma, atopic dermatitis, autism spectrum disorder, chronic functional constipation, celiac disease, chronic atrophic gastritis, chronic pouchitis, Clostridium difficile-associated disease (CDAD), celiac disease, pcolorectal adenoma, colorectal cancer, Crohn's disease, cystic fibrosis, depression, diabetes (Type I), diabetes (Type II), diarrhea, eczema, enterostomy, familial mediterranean fever, food hypersensitivity, graft-versus-host disease (GvHD), hepatic encephalopathy, hypertension, inflammatory bowel disease, irritable bowel disease, irritable bowel disease-constipation (IBS-C), lung cancer, microscopic colitis, multiple sclerosis, non-alcoholic fatty liver disease (NAFLD), non-alcoholic steatohepatitis (NASH), obesity-related asthma, Parkinson's disease (PD), radiation-induced acute intenstinal symptoms, Shigellosis, short bowel syndrome, spinal cord injury associated bowel dysfunction, systemic inflammatory response syndrome, systemic lupus erythematosus, and. ulcerative colitis. In some embodiments, methods of treatment are provided that include modulating the levels of SCFAs to treat the disease or disorder.

In some embodiments, the disease or disorder is associated with a level (e.g., an unwanted level) of a short chain fatty acid, e.g., butyrate, is diarrhea. In some embodiments, the disease or disorder is associated with a level (e.g., an unwanted level) of a short chain fatty acid, e.g., butyrate, is toxicity, e.g., drug toxicity. In some embodiments, methods of treatment are provided that include modulating the levels of SCFAs, e.g., butyrate to treat the disease or disorder, such as diarrhea associated symptoms, such as, e.g. caused by drug toxitcity.

In some embodiments, the disease or disorder is associated with a level (e.g., an unwanted level) of trimethylamine or trimethylamine N-oxide, e.g., and is selected from atherosclerosis, cardiovascular disease, cardiovascular risk in HIV, carotid atherosclerosis, chronic heart disease, chronic heart failure, chronic kidney disease (CKD), chronic vascular disease, colorectal cancer, coronary heart disease, coronary artery disease (CAD), diabetes (Type II), end stage renal disease, HIV, inflammatory bowel disease, ischemic attack, metabolic syndrome, non-alcoholic fatty liver disease (NAFLD), obesity, radiation-induced acute intestinal symptoms (RIAISs), and stroke. In some embodiments, methods of treatment are provided that include modulating the levels of TMA or TMAO to treat the disease or disorder.

In some embodiments, the disease or disorder is associated with a level (e.g., an unwanted level) of ammonia, e.g., and is selected from chronic kidney disease, Helicobacter pylori infection, hepatic encephalopathy, and liver cirrhosis with minimal hepatic encephalopathy (MHE). In one embodiment, the disease or disorder that is associated with a level (e.g., an unwanted level) of ammonia is hepatic encephalopathy (HE). In some embodiments, methods of treatment are provided that include modulating the levels of ammonia to treat the disease or disorder.

In some embodiments, the disease or disorder is associated with a level (e.g., an unwanted level) of a bile acid, e.g., and is selected from alcoholic liver cirrhosis, atherosclerosis, chronic pouchitis, cirrhosis, colorectal adenoma, colorectal cancer, colorectal cancer (postcholecystectony pateints), coronary artery disease, Crohn's disease, cystic fibrosis, inflammatory bowel disease, diabetes (Type II), intestinal failure-associated liver disease, irritable bowel disease, irritable bowel disease-constipation (IBS-C), malabsorption syndrome, non-alcoholic fatty liver disease (NAFLD), non-alcoholic steatohepatitis (NASH), obesity, obesity-related asthma, postcholecystectomy, primary biliary cirrhosis, primary sclerosing cholangitis (PSC), progressive familial intrahepatic cholestasis, reflux esophagitis, short bowel syndrome, Steven Johnson syndrome, ulcerative colitis, and uncomplicated diverticular disease. In some embodiments, methods of treatment are provided that include modulating the levels of a bile acid to treat the disease or disorder.

In some embodiments, the disease or disorder is associated with a level (e.g., an unwanted level) of lipopolysaccharide, e.g., and is selected from allergic diseases, atherosclerosis, autism spectrum disorder, autoimmune hepatitis, chronic fatigue syndroms (CFS), chronic kidney diseases, chronic vascular diseases, common variable immunodeficiency (CVID), Crohn's disease, depression, diabetes (Type II), hepatic encephalopathy, hepatitis B, hepatitis C, HIV, HIV-elite controllers, intestinal failure-associated liver diseases, irritable bowel disease, metabolic syndrome, neonatal necrotizing enterocolitis (NEC), obesity, Parkinson's disease (PD), and ulcerative colitis. In some embodiments, methods of treatment are provided that include modulating the levels of LPS to treat the disease or disorder.

*Drug toxicity/Digestive Abnormalities (including diarrhea)*

Provided herein are methods of reducing drug- or treatment-induced symptoms in a human subject through administration of a glycan polymer preparation (e.g., as described herein). In one embodiment, the methods include modulating the levels of SCFAs, including butyrate. Drug- or treatment-induced symptoms include any digestive abnormalities. Exemplary digestive abnormalies include, but are not limited to weight-gain, constipation, heartburn, upset stomach, gas, bloating, flatulence, diarrhea, abdominal pain, cramping, nausea, and vomiting. In some embodiments, the digestive abnormality is diarrhea. The method include administering to the human subject a pharmaceutical composition comprising a glycan polymer preparation preparation in an amount effective to reduce one or more symptoms induced by a drug or treatment. In one embodiment, the treatment is radiation treatment. In one embodiment, the treatment is chemotherapeutic treatment.

In one embodiment, the subject (e.g., a human subject) being identified to be suitable for treatment with a glycan polymer preparation has or is suspected of having drug-induced diarrhea, drug-induced constipation, drug-induced toxicity, drug-induced intolerance (e.g. to metformin, to chemotherapies, such as, e.g. irinotecan (camptosar) and/or 5-fluorouracil), drug-induced microbiome damage, drug-induced microbiome disease, drug-induced gastrointestinal disease, drug-induced enteritis or colitis or similar drug-induced disorder or condition.

In some embodiments, the pharmaceutical composition comprising a glycan polymer preparation is administered prior to, concomitant with or after administration of the drug (or radiation treatment), administration of which induces the symptoms.

Examplary drugs which often are associated with drug- or treatment-induced symptoms include, but are not limited to a cancer drug, an anti-diabetic, an immune-suppressive drug, an antimicrobial drug, a chemotherapeutic, an anti-psychotic, a proton pump inhibitor, tyrosine kinase inhibitors (TKIs, e.g., Dasatinib (Sprycel), Erlotinib (Tarceva), Gefitinib (Iressa), Imatinib (Gleevec), Lapatinib (Tykerb), Nilotinib (Tasigna), Sorafenib (Nexavar), Sunitinib (Sutent), Afatinib (Gilotrif), Alectinib (Alecensa), Axitinib (Inlyta), Bortezomib (Velcade), Bosutinib (Bosulif), Cabozantinib (Cometriq, Cabometyx), Carfilzomib (Kyprolis), Ceritinib (Zykadia), Cobimetinib (Cotellic), Crizotinib (Xalkori), Dabrafenib (Tafinlar), Dasatinib (Sprycel), Erlotinib (Tarceva), Gefitinib (Iressa), Ibrutinib (Imbruvica), Idelalisib (Zydelig), Imatinib (Gleevec), Ixazomib (Ninlaro), Lapatinib (Tykerb), Lenvatinib (Lenvima), Nilotinib (Tasigna), Niraparib (Zejula), Olaparib (Lynparza), Osimertinib (Tagrisso), Palbociclib (Ibrance), Pazopanib (Votrient), Pegaptanib (Macugen), Ponatinib (Iclusig), Regorafenib (Stivarga), Ribociclib (Kisqali), Rucaparib (Rubraca), Ruxolitinib (Jakafi), Sonidegib (Odomzo), Sorafenib (Nexavar), Sunitinib (Sutent), Tofacitinib (Xeljanz), Trametinib (Mekinist), Vandetanib (Caprelsa), Vemurafenib (Zelboraf), Vismodegib (Erivedge).) and a non-steroid anti-inflammatory drug (NSAID). Administration of these drugs generally is associated with dysbioses that can, e.g., occur during the treatment regimen. In some embodiments, the dysbiosis causes or amplifies the drug- or treatment-induced symptoms, such as digestive abnormalities, such as diarrhea. In some embodiments, administration of the glycan polymer preparation modulates the microbiome such that the drug- or treatment-induced symptoms are reduced (e.g. by modulating the levels of SCFAs, such as butyrate). In some embodiments, the glycan polymer preparation promotes the growth of commensal bacteria and/or supports the growth of beneficial microbial communities which would negatively be affected or lost in response to the drug treatment or which can complement commensal bacteria that have been negatively affected or lost in response to the drug treatment. In some embodiments, the glycan polymer preparation promotes the growth of SCFA producing bacterial taxa, such as, e.g. acetate, propionate or butyrate-producing taxa.

Specifc examples of drugs associated with digestive abnormalities symptoms of which can be reduced by administration of the glycan polymer preparation include, but are not limited to ciprofloxacin, clindamycin, amoxicillin-clavulanate, cefixime, ephalosporins, fluoroquinolones, azithromycin, clarithromycin, erythromycin, tetracycline, azithromycin, irinotecan (camptosar), 5-fluorouracil, leucovorin, oxaliplatin, bortezomib, imatinib, lenalidomide, imbruvica, ipilimumab, pertuzumab, capecitabine, docetaxel, lapatinib, erlotinib, carmustine, etoposide, aracytine, melphalan, cytarabine, daunorubicine, amsacrine, mitoxantrone, olanzapine, ranitidine, famotidine, cimetidine, omeprazole, sucralfate, esomeprazole, naproxen, diclofenac, indomethacin, ibuprofen, ketoprofen, piroxicam, celecoxib, nimesulid, aspirin, metformin, paroxetine, valproic acid, or clozapine.

In some embodiments, the digestive abnormalities are associated with treatment of the subject (e.g., a human subject) with a chemotherapeutic agent. In one embodiment, the digestive abnormality is diarrhea. In specific embodiments, the chemotherapeutic agent is irinotecan, 5-fluorouracil, leucovorin, or combinations thereof. In specific emobidments, the chemotherapeutic agent is oxaliplatin, leucovorin, 5-fluorouracil, or combinations thereof (e.g., FOLFIRI regimen). In specific embodiments, the chemotherapeutic agent is bortezomib, imatinib, lenalidomide, imbruvica, ipilimumab, pertuzumab, capecitabine, docetaxel, lapatinib, erlotinib, or combinations thereof. In some embodiments, the chemotherapeutic agent is carmustine, etoposide, aracytine, melphalan, or combinations thereof. In specific embodiments, the chemotherapeutic agent is cytarabine, daunorubicine, etoposide, or combinations thereof. In specific embodiments, the chemotherapeutic agent is amsacrine, cytarabine, etoposide, or combinations thereof. In specific embodiments, the chemotherapeutic agent is mitoxantrone, cytarabine, or combinations thereof.

In some embodiments, the digestive abnormalities are associated with treatment of the subject with an antibiotic. In one embodiment, the digestive abnormality is diarrhea. In specific embodiments, the antibiotic is ciprofloxacin, clindamycin, amoxicillin-clavulanate, cefixime, ephalosporins, fluoroquinolones, azithromycin, clarithromycin, erythromycin, tetracycline, or azithromycin.

In some embodiments, the digestive abnormalities are associated with treatment of the subject with an antipsychotic drug. In one embodiment, the digestive abnormality is weight gain. In one embodiment, the drug is olanzapine.

In some embodiments, the digestive abnormalities are associated with treatment of the subject with a proton-pump inhibitor drug. In one embodiment, the digestive abnormality is diarrhea. In specific embodiments, the drug is ranitidine, famotidine, cimetidine, omeprazole, sucralfate, or esomeprazole.

In some embodiments, the digestive abnormalities are associated with treatment of the subject with a non-steroidal anti-inflammatory drug (NSAID). In one embodiment, the digestive abnormality is diarrhea. In specific embodiments, the drug is naproxen, diclofenac, indomethacin, ibuprofen, ketoprofen, piroxicam, celecoxib, nimesulid, or aspirin.

In some embodiments, the digestive abnormalities are associated with treatment of the subject with metformin, paroxetine, valproic acid, or clozapine.

In one embodiment, reducing the one or more symptoms increases compliance by the subject to the treatment regimen. In one embodiment, reducing one or more symptom enables the physician to prescribe a higher-dose of the drug to be administered. In such embodiments, treatment of the underlying disease is more effective (e.g. increased reduction of symptoms, shorter period to achieve a disease or symptom-free state, or longer maintainance of a disease or symptom-free state, etc.).

*Chronic kidney disease (CKD)*

In some embodiments, subjects with chronic kidney disease (CKD) may be treated according to the methods provided herein. Subjects with CKD may present with fatigue, trouble concentrating, poor appetite, trouble sleeping, nocturnal muscle cramping, swollen feet and ankles, skin rash/itching, nausea, vomiting, a metallic taste in the mouth, shortness of breath, and/or increased urination. Diagnosis of kidney disease, including CKD, is performed by tests of the glomerular filtration rate (GFR), blood levels of urea and creatinine, urine levels of albumin, kidney biopsy, ultrasound, and/or CT scan. Patient populations include subjects with CKD caused by diabetic nephropathy; subjects with CKD caused by high blood pressure; subjects with polycystic kidney disease, pyelonephritis, or glomerulonephritis; subjects with kidney damage due to long-term use of kidney-damaging medicines; and subjects at risk of developing CKD due to the presence of risk factors such as diabetes, high blood pressure, or family history of kidney disease.

*Hepatic encephalopathy (HE)*

In some embodiments, subjects with hepatic encephalopathy (HE) may be treated according to the methods provided herein. Hepatic encephalopathy includes multiple adverse neurological symptoms that occur when the liver is unable to remove toxic substances such as ammonia from the blood. Subjects with HE may present with confusion, forgetfulness, anxiety or excitation, sudden changes in personality or behavior, changes in sleep patterns, disorientation, sweet or musty smelling breath, slurred speech, and/or difficulty controlling motor functions. Diagnosis of HE is performed by tests of liver function, serum ammonia levels, EEG, and other blood and neurological tests. Patient populations include subjects with mild HE, severe HE, overt HE, subjects who have previously experience one or more episodes of HE, and patients who are at risk for HE due to the presence of risk factors such as liver damage.

*Inflammatory bowel disease (IBD) /Crohn's disease (CD)/ ulcerative colitis (UC)*

Subjects with inflammatory bowel disease (IBD) may present with abdominal cramps and pain, diarrhea that may be bloody, urgency of bowel movements, constipation, nausea, vomiting, fever, weight loss, loss of appetite, and/or iron deficiency anemia due to blood loss. Symptoms of IBD may occur in flares, with alternating periods of symptomatic and asymptomatic disease. IBD may be diagnosed by a combination of tests, including stool exams (to eliminate the possibility of infectious causes of diarrhea, check for trace amounts of blood in the stool, and quantify biomarkers associated with IBD such as fecal calprotectin), a complete blood count to assess levels of inflammation, blood tests to assess biomarkers including C-reactive protein (CRP) and perinuclear anti-neutrophil cytoplasmic antibody (pANCA), barium X-ray, sigmoidoscopy, colonoscopy, and endoscopy. Patient populations include subjects with ulcerative colitis (UC; limited to the colon or large intestine), subjects with Crohn's disease (CD; affecting any segment of the gastrointestinal tract), and subjects with different disease severities (mild, moderate, severe).

*Type 2 diabetis/NASH/NAFLD*

In some embodiments, subjects with type 2 diabetes may be treated according to the methods provided herein. Subjects with type 2 diabetes may present with blurred vision, peripheral neuropathy, increased urination, increased thirst, fatigue, increased hunger, weight loss, or yeast, bladder, kidney, skin, or other infections. Type 2 diabetes is diagnosed by criteria described by the American Diabetes Association (ADA), including the following: fasting plasma glucose (FPG) of 126 mg/dL (7 mM) or higher, or a 2 hour plasma glucose level of 200 mg/dL (11.1 mM) or higher during a 75 g oral glucose tolerance test (OGTT), or a random plasma glucose of 200 mg/dL (11.1 mM) or higher in a patient with classic symptoms of hyperglycemia or hyperglycemic crisis, or a hemoglobin A1c (HbA1c) level of 6.5% or higher. Patient populations include adults and children with type 2 diabetes, subjects at risk for developing type 2 diabetes (e.g., subjects with prediabetes or subjects who are overweight), and subjects with type 2 diabetes in conjunction with conditions of metabolic syndrome including obesity, elevated blood pressure, elevated serum triglycerides, and low high-density lipoprotein (HDL) levels.

In some embodiments, subjects exhibiting non-alcoholic fatty liver disease (NAFLD) and/or non-alcoholic steatohepatitis (NASH) may be treated according to the methods provided herein. Non-alcoholic fatty liver disease (NAFLD) is characterized by an abnormal buildup of fat in the liver. NAFLD can progress to non-alcoholic steatohepatitis (NASH), which is characterized by liver inflammation, fibrosis, and cirrhosis. Subjects with NAFLD may be asymptomatic. Subjects with NAFLD or NASH may present with increased liver size (noted during physical exam), fatigue, weight loss, general weakness, and/or ache in the upper right of the belly. Diagnosis of NAFLD/NASH includes elevated blood levels of alanine aminotransferase (ALT) or aspartate aminotransferase (AST), enlarged liver and specific histopathologic markers (e.g. by liver biopsy, abdominal ultrasound, CT scan, or an MRI scan). Patient populations include subjects with NAFLD, subjects with NASH, subjects at risk of developing NAFLD/NASH (e.g., subjects who are overweight or have elevated cholesterol levels), and subjects with NAFLD/NASH in conjunction with conditions of metabolic syndrome including obesity, elevated fasting plasma glucose, elevated blood pressure, elevated serum triglycerides, and low high-density lipoprotein (HDL) levels.

*Obesity*

In some embodiments, obese subjects may be treated according to the methods provided herein. Obesity is a significant health concern, and may have a negative effect on health. For example, obesity may lead to reduced life expectancy and/or increased health problems, such as diabetes, high blood pressure, heart disease, stroke, high cholesterol, sleep apnea, and arthritis. Obese subjects present with a body mass index (BMI) of greater than 30 kg/m$^2$. Alternatively, obese subjects may be classified based on body fat percentage (greater than 25% for males or greater than 33% for females). Diagnosis may also include an evaluation of fasting lipid levels (cholesterol, triglycerides), liver function, glucose levels, insulin levels, glycosylated hemoglobin (HbA1c), and/or glucose tolerance. Patient populations include subjects with childhood obesity, moderate obesity, morbid/severe obesity, genetic causes of obesity (including Prader-Willi syndrome, Bardet-Biedl syndrome, Cohen syndrome, and MOMO syndrome), and obesity in conjunction with other conditions of metabolic syndrome (elevated blood pressure, elevated fasting plasma glucose, elevated serum triglycerides, and low high-density lipoprotein (HDL) levels).

*Clostridium difficile infection (CDI)-induced colitis*

In some embodiments, subjects with Clostridium difficile infection (CDI)-induced colitis may be treated according to the methods provided herein. Subjects with CDI-induced colitis may present with watery diarrhea, cramping, abdominal pain, anorexia, malaise, fever, dehydration, lower abdominal tenderness, and/or rebound tenderness. The presence of C. difficile in the stool of patients can be tested by stool culture, glutamate dehydrogenase enzyme immunoassay, PCR assay to detect genes for C. difficile toxins, stool cytotoxin assay, or enzyme immunoassay for C. difficile toxins A and B. Patient populations include subjects with primary CDI, subjects with recurrent CDI, subjects with different severities of CDI-associated diarrhea (mild, moderate, severe), and subjects at risk for CDI due to the presence of risk factors such as antibiotics treatment, broad-spectrum antibiotics treatment, residence in a hospital or long-term care facility, gastrointestinal tract surgery, diseases of the colon, a weakened immune system, chemotherapy, advanced age, kidney disease, or use of proton-pump inhibitors. Standard-of-care treatments for CDI include antibiotics such as metronidazole, fidaxomicin, or vancomycin. Treatments may also include probiotics, fecal transplant, and fluids to prevent dehydration. Resolution of disease is measured by abatement of diarrhea (e.g., the absence of a 24 hour period with more than three unformed stools) and resolution of other symptoms described above. Clearance of infection may be verified by the absence of a positive stool test for C. difficile.

In one embodiment, methods are provided to prevent, treat, ameliorate symptoms of, and/or prevent initial colonization or relapse of colonization by pathogens. In some embodiments, the replapse occurs during or after first-line or standard-of-care treatment regimen. In some cases, a pathogen load may initially lighten upon the standard-of-care treatment but then the load begins to increase again, potentially triggering a relapse of the disease. In some embodiments, glycan polymer preparations may be administered (e.g. at the beginning, during or after the initial treatment regimen) to prevent the relapse or treat one or more relapse symptoms. In some embodiments, disease-associated bacteria, pathobionts or pathogens are selected from the group consisting of the species Bilophila wadsworthia, Campylobacter jejuni, Citrobacter farmer, Clostridium difficile, Clostridium perfringens, Clostridium tetani, Collinsella aerofaciens, Enterobacter hormaechei, Enterococcus faecalis, Enterococcus faecium, Escherichia coli, Fusobacterium varium, Fusobacterium nucleatum, Haemophilus parainfluenzae, Klebsiella pneumonia, Peptostreptococcus stomatis, Porphyromonas asaccharolytica, Pseudomonas aeruginosa, Salmonella bongori, Salmonella enteric, Shigella boydii, Shigella dysenteriae, Shigella flexneri, Shigella sonnei, Staphylococcus aureus, Streptococcus infantarius, Vibrio cholera, and Yersinia enterocolitica.

In some embodiments, disease-associated bacteria, pathobionts or pathogens include the genera Bilophila, Campylobacter, Candidatus, Citrobacter, Clostridium, Collinsella, Desulfovibrio, Enterobacter, Enterococcus, Escherichia, Fusobacterium, Haemophilus, Klebsiella, Lachnospiraceae, Peptostreptococcus, Porphyromonas, Portiera, Providencia, Pseudomonas, Salmonella, Shigella, Staphylococcus, Streptococcus, Vibrio, and Yersinia.

*Vancomycin-resistant enterococci (VRE) colonization*

In some embodiments, subjects exhibiting vancomycin-resistant enterococci (VRE) colonization and infection may be treated according to the methods provided herein. Bacteria of the genus Enterococcus are common members of the gut microbiota. Vancomycin-resistant members of this genus, commonly E. faecalis and E. faecium, can cause vancomycin-resistant enterococci (VRE) colonization and infection. Subjects colonized with VRE may present with a VRE-positive stool sample, rectal swab, perirectal swab, or sample from another body site. Vancomycin resistance can be assessed by bacterial culture or by PCR-based assays that detect vancomycin resistance (Van) gene operons. Although colonized subjects may be asymptomatic, this population is at increased risk for infection with VRE. Subjects with VRE infection may present with diarrhea, fever, chills, urinary tract infection (UTI), bacteremia, endocarditis, intra-abdominal and pelvic infection, respiratory infection, or infection at another body site. Patient populations include subjects who are colonized with VRE, subjects suffering from a VRE infection, and subjects who are at risk for colonization or infection with VRE due to the presence of risk factors such as hospitalization, residence in a long-term care facility, long-term antibiotic use, immunosuppression, surgery, open wounds, indwelling devices (e.g., intravenous lines or urinary catheters), or employment as a health care worker.

*Atopic dermatitis (AD)*

In some embodiments, subjects with atopic dermatitis (AD) may be treated according to the methods provided herein. Subjects with atopic dermatitis (AD) may present with skin that is dry, itchy, and/or inflamed. Diagnosis and severity of AD may be determined by using the SCORAD index (Oranje, A. P., et al. British Journal of Dermatology 157.4 (2007): 645-648) or the Eczema Area and Severity Index (EASI) score (Hanifin et al., Experimental Dermatology, 2001, 10:11). AD may occur in flares, with alternating periods of symptomatic and asymptomatic disease. Staphylococcus aureus is commonly present on skin sites with AD, and biomarkers including IgE and inflammatory or Th2 cytokines and chemokines may also be elevated in the diseased skin or systemically. Patient populations include infants with early-onset AD, children with pediatric AD, adults with late-onset AD, pregnant women at risk for flares of AD ("atopic eruption of pregnancy"), subjects with mild, moderate, or severe AD flares, or subjects who are at risk of developing AD.

*Asthma*

In some embodiments, subjects with asthma may be treated according to the methods provided herein. Subjects with asthma may present with wheezing, coughing, shortness of breath, and/or chest tightness or pain. These symptoms are commonly episodic and may be triggered by factors such as exercise or exposure to allergens. Additionally, children with asthma may present with a history of recurrent bronchitis, bronchiolitis, or pneumonia or a persistent cough with colds.

Diagnosis of asthma is established by lung function testing with spirometry in the presence and absence of treatment with a bronchodilator. Patient populations include infants with asthma; subjects with childhood asthma; adult-onset asthma; intermittent, mild persistent, moderate persistent, or severe persistent asthma; exercise-induced asthma; allergic asthma; cough-variant asthma; occupational asthma; nocturnal asthma; and subjects who are at risk of developing asthma, for example, due to a family history of atopy.

*Inflammatory Diseases*

In some embodiments, administration of the glycan polymer preparation glycan polymer preparation reduces inflammation. In some embodiments, a subject is identified to be suitable for treatment if the subject has or is suspected of having a disease, disorder or condition including: gastrointestinal inflammatory diseases including inflammatory bowel disease (IBD), ulcerative colitis (UC), Crohn's disease (CD), idiopathic inflammation of the small bowel, indeterminatal colitis, pouchitis; irritable bowel syndrome (IBS), colon and liver cancers, necrotizing enterocolitis (NEC), intestinal inflammation, constipation, microscopic colitis, diarrhea; graft versus host disease (GVHD); (food) allergies; pseudomembranous colitis; indigestion or non-ulcer dyspepsia; diverticulosis or diverticulitis; ischemic colitis; radiation colitis or enteritis; collagenous colitis; gastroenteritis; and polyps.

In one embodiment, the subject being identified to be suitable for treatment with a glycan polymer preparation has or is suspected of having inflammatory bowel disease (IBD), ulcerative colitis (UC), Crohn's disease (CD), intestinal inflammation, microscopic colitis or similar disease, disorder or condition that is associated with inflammation of the intestine.

In one embodiment, the subject being identified to be suitable for treatment with a glycan polymer preparation has or is suspected of having idiopathic inflammation of the small bowel, indeterminatal colitis, pouchitis, pseudomembranous colitis, ischemic colitis, radiation colitis (enteritis), collagenous colitis or similar disease, disorder or condition that is associated with inflammation of the intestine.

In one embodiment, the subject being identified to be suitable for treatment with a glycan polymer preparation has or is suspected of having gastroenteritis; graft versus host disease (GVHD), or a (food) allergy.

In one embodiment, the subject being identified to be suitable for treatment with a glycan polymer preparation has or is suspected of having irritable bowel syndrome (IBS), constipation, diarrhea, indigestion, non-ulcer dyspepsia or similar disease, disorder or condition that is associated with an altered intestinal transit.

In one embodiment, the subject being identified to be suitable for treatment with a glycan polymer preparation has or is suspected of having colon cancer, liver cancers, necrotizing enterocolitis (NEC); diverticulosis or diverticulitis; polyps or similar disease, disorder or condition that is associated with structural alteration of the intestine.

*Metabolic Diseases*

In some embodiments, a subject is identified to be suitable for treatment if the subject has or is suspected of having a disease, disorder or condition including: obesity, pre-diabetes, type II diabetes, high blood cholesterol, high LDL, high blood pressure, high fasting blood sugar, high triglyceride levels, low HDL non-alcoholic fatty liver disease (NAFLD), nonalcoholic steatohepatitis (NASH); metabolic syndrome; hyperammonemia, essential nutrient deficiency, hemochromatosis, lactose intolerance, gluten intolerance; and acrodermatitis enteropathica.

In one embodiment, the subject being identified to be suitable for treatment with a glycan polymer preparation has or is suspected of having obesity, (insulin resistance) pre-diabetes, type II diabetes, high fasting blood sugar (hyperglycemia), metabolic syndrome or similar disease, disorder or condition associated with metabolic disease symptoms.

In one embodiment, the subject being identified to be suitable for treatment with a glycan polymer preparation has or is suspected of having high blood cholesterol, high LDL, high blood pressure (hypertension), high triglyceride levels, low HDL or similar cardiovascular risk factor.

In one embodiment, the subject being identified to be suitable for treatment with a glycan polymer preparation has or is suspected of having non-alcoholic fatty liver disease (NAFLD), nonalcoholic steatohepatitis (NASH), hyperammonemia or similar disease, disorder or condition of the liver.

In one embodiment, the subject being identified to be suitable for treatment with a glycan polymer preparation has or is suspected of having lactose intolerance, gluten intolerance or similar disease, disorder or condition that is associated with food intolerance.

In one embodiment, the subject being identified to be suitable for treatment with a glycan polymer preparation has or is suspected of having essential nutrient deficiency, hemochromatosis, acrodermatitis enteropathica or similar disease, disorder or condition that is associated with a nutrient mismanagement.

In one embodiment, provided is a method of treating a metabolic disorder in a human in need thereof, by: administering to the human a glycan polymer preparation composition to treat the metabolic disorder. In one embodiment, the metabolic disorder is selected from obesity, adiposity, insulin resistance, diabetes, and fatty liver syndrome.

Metabolic disorders may include disorders, diseases, and conditions that are caused or characterized by abnormal weight gain; energy use or consumption; altered responses to nutrients, energy sources, hormones, or other signaling molecules; or altered metabolism of carbohydrates, lipids, proteins, or nucleic acids, or a combination thereof. Examples of metabolic disorders include insulin resistance, insulin sensitivity, fatty liver syndrome, obesity, adiposity, and diabetes (e.g., type 1 diabetes, type 2 diabetes). In one variation, the methods provided herein treat obesity. Provided herein are methods for treating obesity in a subject in need thereof using a glycan polymer preparation composition that can alter gut microbiota of the subject in a way that results in weight loss and/or decreased body fat in the subject.

In one embodiment, provided is a method of reducing adiposity in a subject in need thereof, by: administering to the human a glycan polymer preparation composition in an amount effective to reduce adiposity. Adiposity may be determined using any appropriate method known in the art, including, for example, waist circumference, waist to hip ratio, skinfold thickness, bioelectric impedance, underwater weighing, air-displacement plethysmography, or hydrometry.

In one embodiment, provided is a method of improving glucose metabolism in a subject in need thereof, by: administering to the subject a glycan polymer preparation composition in an amount effective to improve glucose metabolism. Glucose metabolism may be determined by any appropriate method known in the art, including, for example, fasting blood sugar level, fasting insulin level, postprandial blood sugar test, postprandial insulin test, oral glucose tolerance test, intravenous glucose tolerance test, glycated hemoglobin level, or random blood sugar test.

In one embodiment, provided is a method of increasing insulin sensitivity in a human, by: administering to the subject a glycan polymer preparation composition in an amount effective to increase insulin sensitivity, wherein the human has an insulin sensitivity prior to the administration of the glycan polymer preparation and an insulin sensitivity after the administration of the glycan polymer preparation, and the insulin sensitivity of the human after the administration of the glycan polymer preparation is higher than the insulin sensitivity of the human prior to the administration of the glycan polymer preparation. Insulin sensitivity may be determined by any appropriate method known in the art, including, for example, fasting blood sugar level, fasting insulin level, postprandial blood sugar test, postprandial insulin test, oral glucose tolerance test, intravenous glucose tolerance test, glycated hemoglobin level, or random blood sugar test.

*Infectious Diseases*

In some embodiments, administration of the glycan polymer preparation reduces infection. In some embodiments, a subject is identified to be suitable for treatment if the subject has or is suspected of having a disease, disorder or condition including: gastrointestinal infectious diseases including Clostridium difficile infection (CDI); Vancomycin-resistant enterococci (VRE) infection, infectious colitis, and C. difficile colitis; mycoses, such as, e.g., Candida albicans infection, Campylobacter jejuni infection, Helicobacter pylori infection; diarrhea, such as, e.g., Clostridium difficile associated diarrhea (CDAD), antibiotic-associated diarrhea (AAD), antibiotic-induced diarrhea, travellers' diarrhea (TD), pediatric diarrhea, (acute) infectious diarrhea, colon and liver cancers, ameboma; necrotizing enterocolitis (NEC), and small intestine bacterial overgrowth (SIBO); indigestion or non-ulcer dyspepsia; anal fissures, perianal abscess and anal fistula; diverticulosis or diverticulitis; peptic ulcers; and gastroenteritis.

In one embodiment, the subject being identified to be suitable for treatment with a glycan polymer preparation has or is suspected of having a Clostridium difficile infection (CDI); a Vancomycin-resistant enterococci (VRE) infection, infectious colitis, or C. difficile colitis.

In one embodiment, the subject being identified to be suitable for treatment with a glycan polymer preparation has or is suspected of having mycoses, such as, e.g., Candida albicans infection, Campylobacter jejuni infection, or Helicobacter pylori infection.

In some embodiments, the GI tract infection is a bacterial or viral infection, such as an infection with, e.g., VRE, C. difficile, Escherichia coli, Salmonella, Shigella, Campylobacter, Vibrio cholera, Clostridium perfringes, Bacillus cereus, Vibrio parahemolyticus, Yersinia enterocolitica, Helicobacter pylori, rotavirus, or norovirus.

In some embodiments, the GI tract infection is a fungal infection, such as an infection with, e.g., Candida, Aspergillus, Mucor, Cryptococcus, Histoplasma, or Coccidioides.

In some embodiments, the GI tract infection is a protozoal infection, such as an infection with, e.g., Entamoeba histolytica, Giardia lamblia, Cryptosporidium parvum.

In one embodiment, the subject being identified to be suitable for treatment with a glycan polymer preparation has or is suspected of having diarrhea, such as, e.g., Clostridium difficile associated diarrhea (CDAD), antibiotic-associated diarrhea (AAD), antibiotic-induced diarrhea, travellers' diarrhea (TD), pediatric diarrhea, or (acute) infectious diarrhea.

In one embodiment, the subject being identified to be suitable for treatment with a glycan polymer preparation has or is suspected of having necrotizing enterocolitis (NEC); gastroenteritis; small intestine bacterial overgrowth (SIBO) or similar disease, disorder or condition associated with a GI tract infection.

In one embodiment, the subject being identified to be suitable for treatment with a glycan polymer preparation has or is suspected of having colon cancer, liver cancer, ameboma; indigestion or non-ulcer dyspepsia; anal fissures, perianal abscess and anal fistula; diverticulosis or diverticulitis; peptic ulcer or similar disease, disorder or condition associated with structural alterations of the GI tract.

*Other Diseases*

In some embodiments, a subject is identified to be suitable for treatment if the subject has or is suspected of having a disease, disorder or condition including: autoimmune arthritis, type I diabetes, atopic dermatitis, autism, asthma, cardiovascular disease, chronic kidney disease, multiple sclerosis, heart disease, psoriasis, hyperammonemia, hepatic encephalopathy, cachexia, Gout, drug intolerance (e.g., to metformin), low oral bioavailability of drugs, fecal incontinence, Hirschsprung's disease, anismus, colic, ileus, hemorrhoids, and intussusceptions.

In one embodiment, the subject being identified to be suitable for treatment with a glycan polymer preparation has or is suspected of having autoimmune arthritis, type I diabetes, multiple sclerosis, psoriasis or similar autoimmune disease, disorder or condition.

In one embodiment, the subject being identified to be suitable for treatment with a glycan polymer preparation hasor is suspected of having asthma, atopic dermatitis or similar environmental-driven allergy.

In one embodiment, the subject being identified to be suitable for treatment with a glycan polymer preparation has or is suspected of having chronic kidney disease, heart disease, cardiovascular disease or similar disease, disorder or condition that is associated with organ failure.

In one embodiment, the subject being identified to be suitable for treatment with a glycan polymer preparation has or is suspected of having autism, hyperammonemia, hepatic encephalopathy or similar disease, disorder or condition that is associated with neurological symptoms.

In one embodiment, the subject being identified to be suitable for treatment with a glycan polymer preparation has or is suspected of having cachexia, Gout or similar nutritional disorder.

In one embodiment, the subject being identified to be suitable for treatment with a glycan polymer preparation has or is suspected of having Hirschsprung's disease, ileus, anismus, intussusceptions, fecal incontinence, hemorrhoids or similar gastrointestinal disorder.

*Treatment effects*

In some embodiments, the subject experiences a reduction in at least one symptom of a disease or disorder following treatment. In some embodiments, a reduction in the severity of a symptom following treatment can be determined (e.g. by measuring a known biomarker) and is in the order of about 3%, 5%, 7%, 10%, 20%, 30%, 40%, 50%, 60%, 70%, 80%, 90%, 95%, or about 100%. In some embodiments, the symptoms, measured as described herein, are decreased by an average of about 10%, 20%, 30%, 40%, 50%, 60%, 70%, 80%, 90%, 95%, or about 100% when compared to symptoms prior to the administration of a glycan polymer preparation. In some embodiments, the reduction in the severity of the symptom persists for at least about a day, two days, three days, four days, five days, a week, two weeks, three weeks, a month, 3 months, 6 months, 9 months, a year, two years, five years, ten years after treatment or the reduction is permanent.

In one embodiment, a symptom of a disease, disorder or condition described herein remains partially, substantially, or completely eliminated or decreased in severity in a subject for at least about 1 day, 1 week, 1 month, 2 months, 3 months, 4 months, 5 months, 6 months, 9 months, one year, 18 months, two years, three years, four years, five years, ten years, or more than ten years after the termination of treatment. In another embodiment a symptom of a disease, disorder or condition described herein is permanently eliminated or decreased in severity in a subject after the termination of treatment.

In some embodiments, administration of the glycan polymer preparations improves the overall health of the host and/or the health of a specific niche, such as the GI tract, e.g. by modulating (e.g. increasing or decreasing) the growth or abundance of one or more members of the microbial community in the niche (such as resident commensal bacteria and/or acquired pathogens or pathobionts).

The glycan polymer preparations when administered to a subject in an effective amount may modulate one or more host pathways. The glycan polymer preparation treatment may result in increases or decreases of one or more biomarkers that can be determined by methods known in the art. An investigator can easily determine at which point or points during treatment the biomarker(s) should be measured, e.g. prior to treatment, at various intervals during treatment and/or after treatment. Any suitable sample, e.g. a gastrointestinal-specific sample such as, e.g. a tissue sample or biopsy, a swab, a gastrointestinal secretion (such as feces/a stool sample), etc. may be drawn from the subject and the sample may be analyzed. In some embodiments, a substantial increase or decrease in a biomarker may be detected.

In some embodiments, the glycan polymer preparation is digested by the gut microbiota (e.g. Clostridia), resulting, e.g., in the release of short-chain fatty acids such as butyrate, acetate, and propionate, which may act in an immunomodulatory capacity (e.g. anti-inflammatory) and other metabolites (e.g. bile acids, and lactate) that may confer beneficial health effects on the host.

To evaluate the effect of administered glycan polymer preparation compositions on SCFA production in the gut, fecal samples can be collected. SCFA levels, particularly acetate, propionate, and butyrate may be quantified. SCFAs, creatines, and hydroxy-SCFAs can be quantified by alkalinizing stool samples, obtaining fingerprints of the metabolic composition of the sample using, e.g., 1D 1H NMR spectrometer, and analyzing with supervised multivariate statistical methods. Inulin may serve as a positive control.

In some embodiments, microbial metabolite profiles of patient samples or microbes cultures from subject samples are used to identify risk factors for developing a gastrointestinal infectious and/or inflammatory disease, disorder or condition. Exemplary metabolites for the purposes of diagnosis, prognostic risk assessment, or treatment assessment purposes include those listed in Table 5. In some embodiments, microbial metabolite profiles are taken at different time points during a subject's disease and treatment in order to better evaluate the subject's disease state including recovery or relapse events. Such monitoring is also important to lower the risk of a subject developing a new gastrointestinal disease, disorder or condition. In some embodiments, metabolite profiles inform subsequent treatment.

Further, if determined useful by a treating physician or other healthcare provider, the glycan polymer preparation compositions described herein can be administered in combination with various other standard of care therapies. In some embodiments, the combination of administration of the glycan polymer preparation and the standard-of-care therapy agent has additive or synergistic treatment effects. The glycan polymer preparations may be administered prior to, concurrent with, or post treatment with standard of care therapies. In some instances, the therapies disrupt the composition and health of the GI tract's normal microbiota (e.g. use of antibacterial, anti-viral or anti-fungal agents), which may lead to the undesirable proliferation of harmful bacteria or pathogens, which may cause one or more of the symptoms described herein.

In some embodiments, administration of the glycan polymer preparations described herein is useful for alleviating those symptoms and improving the composition of the gastrointestinal microbial community.

*Combinations*

Additional substances can be given in conjunction with a glycan polymer preparation. In some embodiments, the glycan polymer preparation may also be combined with another agent (e.g., a therapeutic agent, micronutrient, prebiotic, probiotic, or synbiotic).

These substances can enhance the action of the doses of glycan polymer by, e.g., encouraging the growth of bacteria, e.g., in the gut that alleviate symptoms of a disease, disorder (e.g., described herein), increasing adhesion of probiotic or beneficial commensal bacteria in the niche or in the gut. These substances can be given prior to treatment with glycan polymer preparation, during treatment with glycan polymer preparation, after treatment with glycan polymer preparation, or any combination thereof. If administered during glycan polymer preparation treatment, they can be administered with the dose of glycan polymer preparation being given, or before or after the dose of glycan polymer preparation, or any combination thereof. In one embodiment substances of use in conjunction with a glycan polymer preparation include a probiotic microbe(s), prebiotics, therapeutic agents, or buffers/carriers/excipients. One or more of these substances can be used in combination with glycan polymer preparation at any suitable time before, during, after treatment, or some combination thereof.

In some embodiments, the additional agent is a therapeutic agent, e.g., a dysbiosis-causing drug, e.g. a drug that disrupts normal gastrointestinal microbiota growth, e.g. a chemotherapeutic drug, an anti-diabetic drug, an immune-suppressive drug, an antimicrobial drug, an anti-psychotic drug, a proton pump inhibitor drug, or a non-steroid anti-inflammatory drug (NSAID). The glycan polymer preparation, in some embodiments, reduces the drug- or treatment-induced symptoms in a human subject. The symptoms include digestive abnormalities, such as, e.g., weight-gain, constipation, heartburn, upset stomach, gas, bloating, flatulence, diarrhea, abdominal pain, cramping, nausea, and vomiting.

In some embodiments, the additional agent is a micronutrient. In some embodiments, the micronutrient is selected from the group consisting of a trace mineral, choline, a vitamin, and a polyphenol. In some embodiments, the micronutrient is a trace metal. Trace minerals suitable as a micronutrient include, but are not limited to, boron, cobalt, chromium, calcium, copper, fluoride, iodine, iron, magnesium, manganese, molybdenum, selenium, and zinc. In some embodiments, the micronutrient is a vitamin. Vitamins suitable as a micronutrient include, but are not limited to, Vitamin B complex, Vitamin B1 (thiamin), Vitamin B2 (riboflavin), Vitamin B3 (niacin), Vitamin B5 (pantothenic acid), Vitamin B6 group (pyridoxine, pyridoxal, pyridoxamine), Vitamin B7 (biotin), Vitamin B8 (ergadenylic acid), Vitamin B9 (folic acid), Vitamin B12 (cyanocobalamin), Choline, Vitamin A (retinol), Vitamin C (ascorbic acid), Vitamin D, Vitamin E (tocopherol), Vitamin K, carotenoids (alpha carotene, beta carotene, cryptoxanthin, lutein, lycopene) and zeaxanthin.

In some embodiments, the micronutrient is a polyphenol. Polyphenols are chemical compounds or molecules that are characterized by having at least one aromatic ring with one or more hydroxyl groups. In some embodiments, the polyphenol is a synthetic polyphenol or a naturally occurring polyphenol. In some embodiments, the polyphenol is a naturally occurring polyphenol and is derived from plant source material. In some embodiments, the polyphenol is a flavonoid or catechin. In some embodiments, the flavonoid or catechin is selected from anthocyanins, chalcones, dihydrochalcones, dihydroflavonols, flavanols, flavanones, flavones, flavonols and isoflavonoids. In some embodiments, the polyphenol is a lignan. In some embodiments, the polyphenol is selected from alkylmethoxyphenols, alkylphenols, curcuminoids, furanocoumarins, hydroxybenzaldehydes, hydroxybenzoketones, hydroxycinnamaldehydes, hydroxycoumarins, hydroxyphenylpropenes, methoxyphenols, naphtoquinones, phenolic terpenes, and tyrosols. In some embodiments, the polyphenol is a tannin or tannic acid. In some embodiments, the polyphenol is selected from hydroxybenzoic acids, hydroxycinnamic acids, hydroxyphenylacetic acids, hydroxyphenylpropanoic acids, and hydroxyphenylpentanoic acids. In some embodiments, the polyphenol is a stilbene.

In some embodiments, the pharmaceutical compositions and medical foods and dietary supplements comprising glycan polymer preparations described herein further comprise a prebiotic substance or preparation thereof.

In some embodiments, prebiotics may be administered to a subject receiving the pharmaceutical compositions or medical foods or dietary supplements comprising glycan polymer preparations described herein. Prebiotics are non-digestible substances that when consumed may provide a beneficial physiological effect on the host by selectively stimulating the favorable growth or activity of a limited number of indigenous bacteria in the gut (Gibson G R, Roberfroid M B. J Nutr. 1995 June; 125(6): 1401-12.). A prebiotic such as a dietary fiber or prebiotic oligosaccharide (e.g. crystalline cellulose, wheat bran, oat bran, corn fiber, soy fiber, beet fiber and the like) may further encourage the growth of probiotic and/or commensal bacteria in the gut by providing a fermentable dose of carbohydrates to the bacteria and increase the levels of those microbial populations (e.g. lactobacilli and bifidobacteria) in the gastrointestinal tract.

Prebiotics include, but are not limited to, various galactans and carbohydrate based gums, such as psyllium, guar, carrageen, gellan, lactulose, and konjac. In some embodiments, the prebiotic is one or more of galactooligosaccharides (GOS), lactulose, raffinose, stachyose, lactosucrose, fructo-oligosaccharides (FOS, e.g. oligofructose or oligofructan), inulin, isomaltooligosaccharide, xylo-oligosaccharides (XOS), paratinose oligosaccharide, isomaltose oligosaccharides (IMOS), transgalactosylated oligosaccharides (e.g. transgalacto-oligosaccharides), transgalactosylate disaccharides, soybean oligosaccharides (e.g. soyoligosaccharides), chitosan oligosaccharide (chioses), gentiooligosaccharides, soy- and pectin-oligosaccharides, glucooligosaccharides, pecticoligosaccharides, palatinose polycondensates, difructose anhydride III, sorbitol, maltitol, lactitol, polyols, polydextrose, linear and branched dextrans, pullalan, hemicelluloses, reduced paratinose, cellulose, beta-glucose, beta-galactose, beta-fructose, verbascose, galactinol, xylan, inulin, chitosan, beta-glucan, guar gum, gum arabic, pectin, high sodium alginate, and lambda carrageenan, or mixtures thereof.

Prebiotics can be found in certain foods, e.g. chicory root, Jerusalem artichoke, Dandelion greens, garlic, leek, onion, asparagus, wheat bran, wheat flour, banana, milk, yogurt, sorghum, burdock, broccoli, Brussels sprouts, cabbage, cauliflower, collard greens, kale, radish and rutabaga, and miso. In some embodiments, the glycan polymers described herein are administered to a subject in conjunction with a diet that includes foods rich in prebiotics. Suitable sources of soluble and insoluble fibers are commercially available.

In some embodiments, the pharmaceutical compositions and medical foods and dietary supplements comprising glycan polymer preparations further comprise a probiotic bacterium or preparation thereof, e.g., derived from bacterial cultures that are generally recognized as safe (GRAS) or known commensal or probiotic microbes. In some embodiments, to maximize the beneficial effect of endogenous commensal microbes or exogenously administered probiotic microorganisms, the pharmaceutical compositions and medical foods and dietary supplements comprising glycan polymer preparations are administered to stimulate the growth and/or activity of advantageous bacteria in the GI tract.

Examples of suitable probiotics include, but are not limited to, organisms classified as genera Bacteroides, Blautia, Clostridium, Fusobacterium, Eubacterium, Ruminococcus, Peptococcus, Peptostreptococcus, Akkermansia, Faecalibacterium, Roseburia, Prevotella, Bifidobacterium, Lactobacillus, Bacillus, Enterococcus, Escherichia, Streptococcus, Saccharomyces, Streptomyces, and family Christensenellaceae. Non-exclusive examples of probiotic bacteria that can be used in the methods and compositions described herein include L. acidophilus, Lactobacillus species, such as L. crispatus, L. casei, L. rhamnosus, L. reuteri, L. fermentum, L. plantarum, L. sporogenes, and L. bulgaricus, as well as Bifidobacterum species, such as B. lactis, B. animalis, B. bifidum, B. longum, B. adolescentis, and B. infantis. Yeasts, such as Saccharomyces boulardii, are also suitable as probiotics for administration to the gut, e.g. via oral dosage forms or foods. For example, yogurt is a product which already contains bacteria species, such as Lactobacillus bulgaricus and Streptococcus thermophilus.

Beneficial bacteria for the modulation of the gastrointestinal microbiota may include bacteria that produce organic acids (lactic & acetic acids) or that produce cytotoxic or cytostatic agents (to inhibit pathogenic growth), such as, e.g., hydrogen peroxide ($H_2O_2$) and bacteriocins. Bacteriocins are small antimicrobial peptides which can kill both closely-related bacteria, or exhibit a broader spectrum of activity (e.g., nisin).

Beneficial bacteria may include one or more of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus.

In some embodiments, combinations are provided comprising a bacterial taxa selected from column 1 of tables 19, 20 or 21 and a glycan preparation described herein. In some embodiments, the combination preparation comprises a microbial preparation of a microbe selected from column 1 of tables 19, 20 or 21 and a glycan preparation selected from columns 3-10 (Table 19) or columns 2-9 (Tables 20 and 21). In some embodiments, synbiotic combinations are provided suitable for the administration to a human subject in need thereof (e.g. oral or rectal administration). In some embodiments, the bacterial taxa selected for the combination is a spore-forming bacterial taxa. In some embodiments, the glycan preparation selected for the combination is a (fermentable) substrate (e.g. for a glycosidase enzyme) of the spore-forming bacterial taxa.

Further, if desired, the pharmaceutical compositions and medical foods and dietary supplements comprising glycan polymer preparations may comprise therapeutically active agents, prebiotic substances and/or probiotic bacteria. Alternatively or in addition, therapeutically active agents, prebiotic substances and/or probiotic bacteria may be administered separately (e.g. prior to, concurrent with or after administration of the glycan polymers) and not as a part of the pharmaceutical composition or medical food or dietary supplement (e.g. as a co-formulation) of glycan polymers. In some embodiments, pharmaceutical compositions or medical foods or dietary supplements comprising preparations of glycan polymers are administered in combination with a recommended or prescribed diet, e.g. a diet that is rich in probiotic and/or prebiotic-containing foods, such as it may be determined by a physician or other healthcare professional. Therapeutically active agents, prebiotic substances and/or probiotic bacteria may be administered to modulate the gut microbiome of the subject. In some embodiments, the combined effect (e.g. on the number or intensity of the microbial, genomic or functional shifts) is additive. In other embodiments, the combined effect (e.g. on the number or intensity of the microbial, genomic or functional shifts) is synergistic.

*Administration of glycan polymer preparations*

For any glycan polymer preparation composition used in a method described herein (e.g., a method of treatment of a disease, disorder or condition listed in Table 5), a therapeutically effective dose can be estimated initially from laboratory animal models known to those of skill in the art. Such information can be used to more accurately determine useful doses in humans.

Initial dosages can also be estimated from in vitro or in vivo data. Initial dosages can also be formulated by comparing the effectiveness of the compounds used in the methods described herein in model assays with the effectiveness of known compounds. For instance, initial dosages can be formulated by comparing the effectiveness of the glycan polymer preparation preparations in model assays with the effectiveness of other compounds that have shown efficacy in treating the present conditions. In this method, an initial dosage can be obtained by multiplying the ratio of effective concentrations obtained in the model assay for the glycan polymer preparation preparations used in methods described herein and the control compound by the effective dosage of the control compound. For example, if a preparation useful in a present method is twice as effective in a model assay as a known compound (e.g., the efficacious concentration ($EC_{50}$) of the glycan polymer preparation preparation is equal to one-half the $EC_{50}$ of the known compound in the same assay), an initial effective dosage of the glycan polymer preparation preparation would be one-half the known dosage for the known compound. Using these initial guidelines an effective dosage in subjects, such as humans, can be determined by one of ordinary skill. Dosage amount and interval may be adjusted individually to provide levels of the glycan polymer preparation preparation which are sufficient to maintain therapeutic effect. One of skill in the art will be able to optimize therapeutically effective local dosages without undue experimentation.

Depending upon the disorder and subject to be treated and the route of administration, the compositions may be administered at varying doses. In one embodiment, the smallest effective amount or dose of glycan polymer preparation is used. In some embodiments, the glycan polymer preparation is administered in a dose from about 0.01mg/kg to about 10,000 mg/kg, from about 0.1mg/kg to about 1,000 mg/kg, from about 1mg/kg to about 100 mg/kg, 0.05 mg/kg to about 5,000 mg/kg, from about 0.5 mg/kg to about 5,000 mg/kg, from about 5 mg/kg to about 500 mg/kg. This dose may be given as mg/kg/day and may be administered as an initial dose or may be increased or decreased over time (e.g., days or week) to reach a final dose.

In some embodiments, the glycan polymer preparation is administered in a total daily dose per subject from about 1 mg per day to about 100 grams per day; from about 10 mgs per day to about 10 grams per day; from about 100 mgs per day to about 10 grams per day; from about 1 gram per day to about 10 grams per day, from about 2 grams per day to about 20 grams per day; from about 5 grams per day to about 50 grams per day, from about 10 grams per day to about 100 grams per day, from about 10 grams per day to about 50 grams per day, from about 10 grams per day to about 75 grams per day, from about 20 grams per day to about 100 grams per day, from about 20 grams per day to about 50 grams per day, from about 20 grams per day to about 75 grams per day, from about 20 grams per day to about 100 grams per day, from about 50 grams per day to about 150 grams per day, or from about 50 grams per day to about 200 grams per day.

In some embodiments, a symptom of a gastrointestinal disease, disorder or condition in a subject exhibiting the symptoms is decreased or eliminated by administering to the subject increasing, decreasing or constant amounts (or doses) of a glycan polymer preparation composition for a period of time (e.g. a treatment period).

In one embodiment, the composition contains beneficial, commensal and/or probiotic bacterial strains in an amount comprised from $1\times10^7$ to $1\times10^{13}$ CFU/dose and bacterial strain, or from $1\times10^9$ to $1\times10^{11}$ CFU/dose and bacterial strain.

In some embodiments, the pharmaceutical composition is administered one, two, or three times a day. In some embodiments, the pharmaceutical composition is administered twice a day. In some embodiments, the pharmaceutical composition is administered each day for a predetermined number of days (the treatment period). In some embodiments, the treatment period is 1, 2, 3, 4, 5, 6, 7, 8, 9, 10, 11, 12, 13, 14, 21, 28, 35, 42, 49, 56, 63, 70, 100, 200, 300 or 365 days. In some embodiments, the treatment period is 1, 2, 3, 4, 5, 6, 7, 8, 9, 10, 11, or 12 months. In some embodiments, the treatment period is 1, 2, 3, 4, 5, 6, 7, 8, 9, 10, 11, 12 years, or life-long.

In one embodiment the total duration of treatment periods for a gastrointestinal disease, disorder or condition can be from about one day to 10 years, one day to 1 year, 1 day to 6 months, 1 day to 3 months, 1 day to 1 months, one day to one week, one day to five days, one day to 10 days, one week to about 12 weeks, or about four weeks to about ten weeks, or about four weeks to about eight weeks, or about six weeks. The subject may undergo a suitable number of treatment periods, such as, e.g. 1, 2, 3, 4, 5, 6, 7, 8, 9, 10 or more than 10 treatment periods. During a treatment period, the subject takes a glycan polymer preparation composition described herein, optionally along with ingestion of prebiotic and/or probiotic containing food products. In one embodiment, a glycan polymer preparation composition can also be administered in combination with another substance (such as a probiotic or commensal beneficial bacteria, a prebiotic substance or a therapeutic agent), as described herein.

In some embodiments, the glycan polymer preparation composition may also be combined with an antibiotic that disrupts normal gastrointestinal microbiota growth. Typically durations for antibiotic treatments are 1-14 days, or 2-10 days, or 5-7 days. In some embodiments, a glycan polymer preparation is administered to a subject in need thereof immediately after one or more antibiotic treatment(s) has ended (e.g. 1 hour, 6 hours, 12 hours, 24 hours, 36 hours, 48 hours, 3 days, 4 days, 5 days, 6 days, 7 days, 2 weeks, 3 weeks or 4 weeks after the antibiotic treatment has ended). During a course of antibiotic treatment, the glycan polymer preparation composition may be provided at the initiation of antibiotic treatment; shortly following antibiotic treatment, e.g. 1, 2, 3, 4, 5, 6, 7, or more days following treatment; or may be administered upon diagnosis of undesirable pathogen growth.

In some embodiments, the glycan polymer preparation composition may also be combined with a dysbiosis-causing drug, e.g. a drug that disrupts normal gastrointestinal microbiota growth, e.g. a chemotherapeutic drug, an anti-diabetic drug, an immune-suppressive drug, an antimicrobial drug, an anti-psychotic drug, a proton pump inhibitor drug, or a non-steroid anti-inflammatory drug (NSAID). The glycan polymer preparation composition, in some embodiments, reduces the drug- or treatment-induced symptoms in a human subject. The symptoms include digestive abnormalities, such as, e.g., weight-gain, constipation, heartburn, upset stomach, gas, bloating, flatulence, diarrhea, abdominal pain, cramping, nausea, and vomiting. In some embodiments, a glycan polymer preparation is administered to a subject in need thereof immediately after one or more drug treatment(s) has ended (e.g. 1 hour, 6 hours, 12 hours, 24 hours, 36 hours, 48 hours, 3 days, 4 days, 5 days, 6 days, 7 days, 2 weeks, 3 weeks or 4 weeks after the antibiotic treatment has ended). During a course of drug treatment, the glycan polymer preparation composition may be provided prior to the initiation of drug treatment (e.g. 1, 2, 3, 4, 5, 6, 7 days prior); at the day of initiation of drug treatment; or shortly following antibiotic treatment, e.g. 1, 2, 3, 4, 5, 6, 7, or more days following treatment, and may optionally be provided only initially (e.g. for a short period) or throughout the duration of the drug-treatment, and may even be continued for a desired period after the drug treatment period has ended (e.g. for 1-7days, 1-14 days, or 1-21 days thereafter). In some embodiments, administration of the glycan polymer preparation composition is initiated or continued when one or more adverse effects occur and/or are diagnosed (e.g. digestive abnormalities or pathogen growth) in conjunction with the drug treatment. In some embodiments, the treatment agent causing a dysbiosis is not a drug but radiation treatment or surgery and the glycan polymer preparation composition may also be administered as described herein.

In some embodiments, the total number and duration of treatment periods is based on a subject's response to the treatment. For example, an individual can experience a reduction in symptoms after 1, 2, 3, 4, 5, 6, 7, 8, 9, 10, 11, 12, 13, or 14 days of treatment with a glycan polymer preparation composition. In another example, an individual can experience a reduction in symptoms after 1, 2, 3, 4, 5, 6, 7, 8, 9, 10, 11, 12 months of treatment with a glycan polymer preparation composition. Thus, the duration of treatment is determined by an individual subject's response to a glycan polymer preparation composition and the onset of relief from one or more symptoms. Thus, a subject can experience symptoms at a given dose of a glycan polymer preparation composition and can require that the subject stay at that dose, or a lower dose, until symptoms subside. Thus, in one embodiment, the duration of the treatment is not determined at the outset, but continues until the maximum dose of a glycan polymer preparation composition is achieved per day, or until the desired level of reduction in symptoms is achieved. In one embodiment, the treatment is continuous.

In one embodiment, a subject can be given one dose for the first treatment period during a treatment regimen and a second dose during a second treatment period. For example, a subject can be administered one dose of glycan polymer preparation composition for a one week period and a second dose for a subsequent one week period.

A subject may self-administer a glycan polymer preparation composition and the glycan polymer preparation composition is supplied or recommended (or prescribed) by a health professional, e.g., a physician or other qualified health professional and optionally test results (e.g. obtained for biomarkers from samples taken from the subject) and/or health changes and treatment endpoints are monitored by a health professional. In some embodiments, the glycan polymer preparation composition is administered by a health professional.

In one embodiment, a subject in need thereof can undergo repeated courses of treatment with a glycan polymer preparation composition. The course of treatment can be repeated when symptoms reappear or increase to an undesirable level. Alternatively, the course of treatment can be repeated at regular or predetermined intervals. Thus, treatment can be repeated after about one month, two months, three months, four months, six months, eight months, ten months, one year, 18 months, two years, three years, four years, five years, or more than five years, or any combination thereof (e.g., treatment can be repeated after one year, then every two to five years thereafter). The treatment can be repeated in the same form (e.g., duration, dosage, timing of dosage, additional substances, etc.) as used in the first treatment or it can be modified. For example, treatment duration can be shortened or lengthened, dosage can be increased or decreased. Optionally, treatment with the glycan polymer preparation can occur in combination with a different number or compositions of agents, e.g., containing more or less of other substances, or fewer or more substances (such as, e.g., a prebiotic substance, a probiotic bacterium or a therapeutic agent) in addition to the glycan polymer preparation.

Additional substances can be given in conjunction with a glycan polymer preparation composition. These substances can enhance the action of the doses of glycan polymer preparation by, e.g., encouraging the growth of bacteria in the GI tract that alleviate symptoms of the gastrointestinal disease, disorder or condition, increasing adhesion of probiotic or beneficial commensal bacteria in the niche or in the gut. These substances can be given prior to treatment with glycan polymer preparation, during treatment with glycan polymer preparation, after treatment with glycan polymer preparation, or any combination thereof. If administered during glycan polymer preparation treatment, they can be administered with the dose of glycan polymer preparation being given, or before or after the dose of glycan polymer preparation, or any combination thereof. In one embodiment substances of use in conjunction with a glycan polymer preparation composition include a probiotic microbe(s), prebiotics, therapeutic agents, or buffers/carriers/excipients. One or more of these substances can be used in combination with glycan polymer preparation composition at any suitable time before, during, after treatment, or some combination thereof.

**Table 1: Exemplary glycan polymer characteristics.**

| Monomer content (one or more of) | Degree polymerization (DP) | Average DP preparation | Alpha-/beta-glycosidic bonds | Degree of branching (DB) | Glycosidic bonds |
|---|---|---|---|---|---|
| i) glucose, ii) galactose, iii) arabinose, iv) mannose, v) fructose, vi) fucose, vii) rhamnose , or viii) xylose, or any glycan subunit described herein | 2-4, 2-5, 2-6, 2-10, 2-15, 2-20, 3-6, 3-8,3-10,3-12,3-14, 3-16, 3-20,3-25, 3-30, or any DP described herein | 2, 3, 4, 5, 6, 2-5, 2-6, 4-10, 6-12, 6-14, 6-16, 8-16, 10-20, 10-25, or any average DP described herein | >80%, >90%, >95%, >98%, 100% alpha glycosidic bonds, or any alpha bond content described herein | DB 0 (non-branched) | Alpha: 1,2, Alpha: 1,3 Alpha: 1,4 Alpha: 1,5 Alpha: 1,6 Alpha: 2,1 Alpha: 2, 3 Alpha: 2,4 Alpha: 2,5 Alpha: 2,6, or any alpha bond described herein |
| | | | >80%, >90%, >95%, >98%, 100% beta glycosidic bonds, or any beta bond content described herein | DB (branched) : >0.01, >0.05, 0.01-0.6 0.05-0.5, or any DB described herein | Beta: 1,2, Beta: 1,3 Beta: 1,4 Beta: 1,5 Beta: 1,6 Beta: 2,1 Beta: 2, 3 Beta: 2,4 Beta: 2,5 Beta: 2,6, or any betabond described herein |
| | | | Alpha to beta bond ratio: 1:1, 1:2, 1:3, 1:4, 1:5, from 1:1-1:5, 1:1-1:4, 1:1-1:3, or any alpha:beta ratio described herein | | |
| | | | Beta to alpha bond ratio: 1:1, 1:2, 1:3, 1:4, 1:5, from 1:1-1:5, 1:1-1:4, 1:1-1:3, or any beta:alpha ratio described herein | | |

**Table 2: Genus level microbial constituents of the GI tract.**

| Phylum | Class | Genus |
|---|---|---|
| Actinobacteria | Actinobacteria | Actinomyces, Adlercreutzia, Atopobium, Bifidobacterium, Collinsella, Corynebacterium, Eggerthella, Mobiluncus, Propionibacterium, Rothia, Slackia |
| Bacteroidetes | Bacteroidia | Alistipes, Bacteroides, Dysgonomonas, Odoribacter, Parabacteroides, Porphyromonas, Prevotella, Tannerella |
| | Flavobacteria | Capnocytophaga |

(continued)

| Phylum | Class | Genus |
|---|---|---|
| Firmicutes | Bacilli | Bacillus, Enterococcus, Gemella, Granulicatella, Lactobacillus, Lactococcus, Staphylococcus, Streptococcus, Turicibacter, Weissella |
| | Clostridia | Acidaminococcus, Anaerococcus, Anaerofilum, Anaerofustis, Anaerostipes, Anaerotruncus, Anaerovorax, Bacteroides, Bacteroides, Blautia, Clostridium, Coprococcus, Dehalobacterium, Dialister, Dorea, Eubacterium, Faecalibacterium, Finegoldia, Lachnobacterium, Lachnospira, Megamonas, Megasphaera, Mitsuokella, Moryella, Oribacterium, Oscillospira, Peptococcus, Peptoniphilus, Peptostreptococcus, Phascolarctobacterium, Pseudobutyrivibrio, Roseburia, Ruminococcus, Ruminococcus, Selenomonas, Subdoligranulum, Veillonella |
| Fusobacteria | Fusobacteria | Fusobacterium, Leptotrichia |
| | Betaproteobacteria | Comamonas, Herbaspirillum, Lautropia, Neisseria, Oxalobacter, Sutterella |
| | Deltaproteobacteria | Bilophila, Desulfovibrio, LE30 |
| | Epsilon proteobacteria | Campylobacter, Helicobacter |
| | Gammaproteobacteria | Actinobacillus, Aggregatibacter, Citrobacter, Escherichia, Haemophilus, Klebsiella, Moraxella, Pseudomonas, Raoultella |
| Spirochaetes | Spirochaetes | Treponema |
| Synergistetes | Synergistetia | Cloacibacillus, Synergistes |
| Tenericutes | Erysipelotrichi | Bulleidia, Catenibacterium, Clostridium, Coprobacillus, Holdemania, RFN20 |
| | Mollicutes | Asteroleplasma, Mycoplasma |
| Verrucomicrobia | Verrucomicrobiae | Akkermansia |
| Euryarchaeota | Methanobacteria | Methanobrevibacter |

**Table 3: Phyla and strains associated with exemplary metabolites**

| Strain | phylum | butyrate | ammonia | TMA/ TMAO |
|---|---|---|---|---|
| Providencia rettgeri DSM 1131 | Proteobacteria | 0 | 1 | 1 |
| Proteus penneri ATCC 35198 | Proteobacteria | 0 | 1 | 1 |
| Proteus mirabilis WGLW6 | Proteobacteria | 0 | 1 | 1 |
| Desulfitobacterium hafniense DP7 | Firmicutes | 1 | 0 | 1 |
| Clostridium sporogenes ATCC 15579 | Firmicutes | 1 | 0 | 1 |
| Anaerococcus hydrogenalis DSM 7454 | Firmicutes | 1 | 0 | 1 |
| Collinsella tanakaei YIT 12063 | Actinobacteria | 0 | 0 | 1 |
| Lachnospiraceae [Clostridium | Firmicutes | 0 | 0 | 1 |
| Lachnospiraceae [Clostridium asparagiforme | Firmicutes | 0 | 0 | 1 |
| Clostridiales bacterium 1 7 47FAA | Firmicutes | 0 | 0 | 1 |
| Escherichia coli MS 60-1 | Proteobacteria | 0 | 0 | 1 |
| Escherichia coli MS 69-1 | Proteobacteria | 0 | 0 | 1 |
| Escherichia coli MS 153-1 | Proteobacteria | 0 | 0 | 1 |

(continued)

| Strain | phylum | butyrate | ammonia | TMA/ TMAO |
|---|---|---|---|---|
| Klebsiella sp. MS 92-3 | Proteobacteria | 0 | 0 | 1 |
| Yokenella regensburgei ATCC 43003 | Proteobacteria | 0 | 0 | 1 |
| Providencia alcalifaciens DSM 30120 | Proteobacteria | 0 | 0 | 1 |
| Klebsiella pneumoniae subsp. pneumoniae WGLW5 | Proteobacteria | 0 | 0 | 1 |
| Providencia rustigianii DSM 4541 | Proteobacteria | 0 | 0 | 1 |
| Escherichia coli MS 200-1 | Proteobacteria | 0 | 0 | 1 |
| Streptomyces sp. HGB0020 | Actinobacteria | 0 | 1 | 0 |
| Odoribacter laneus YIT 12061 | Bacteroidetes | 0 | 1 | 0 |
| Bacillus smithii 7 3 47FAA | Firmicutes | 0 | 1 | 0 |
| Paenibacillus sp. HGF5 | Firmicutes | 0 | 1 | 0 |
| Staphylococcus sp. HGB0015 | Firmicutes | 0 | 1 | 0 |
| Helicobacter pylori GAM246Ai | Proteobacteria | 0 | 1 | 0 |
| Citrobacter youngae ATCC 29220 | Proteobacteria | 0 | 1 | 0 |
| Helicobacter pylori GAM93Bi | Proteobacteria | 0 | 1 | 0 |
| Helicobacter pylori HP116Bi | Proteobacteria | 0 | 1 | 0 |
| Helicobacter pylori GAM83Bi | Proteobacteria | 0 | 1 | 0 |
| Helicobacter pylori GAM96Ai | Proteobacteria | 0 | 1 | 0 |
| Helicobacter pylori GAM101Biv | Proteobacteria | 0 | 1 | 0 |
| Helicobacter pylori HP250BFiii | Proteobacteria | 0 | 1 | 0 |
| Helicobacter pylori GAM252T | Proteobacteria | 0 | 1 | 0 |
| Helicobacter pylori HP250BSi | Proteobacteria | 0 | 1 | 0 |
| Helicobacter pylori GAM121Aii | Proteobacteria | 0 | 1 | 0 |
| Helicobacter pylori GAM239Bi | Proteobacteria | 0 | 1 | 0 |
| Pseudomonas sp. 2 1 26 | Proteobacteria | 0 | 1 | 0 |
| Helicobacter pylori GAM260BSi | Proteobacteria | 0 | 1 | 0 |
| Helicobacter pylori GAM260Bi | Proteobacteria | 0 | 1 | 0 |
| Klebsiella sp. 1 1 55 | Proteobacteria | 0 | 1 | 0 |
| Helicobacter pylori HP260ASii | Proteobacteria | 0 | 1 | 0 |
| Acinetobacter junii SH205 | Proteobacteria | 0 | 1 | 0 |
| Acinetobacter radioresistens SH164 | Proteobacteria | 0 | 1 | 0 |
| Enterobacter cloacae subsp. cloacae NCTC 9394 | Proteobacteria | 0 | 1 | 0 |
| Helicobacter pylori HP250AFiV | Proteobacteria | 0 | 1 | 0 |
| Helicobacter pylori HP260Bi | Proteobacteria | 0 | 1 | 0 |
| Helicobacter pylori GAM115Ai | Proteobacteria | 0 | 1 | 0 |
| Helicobacter pylori GAM71Ai | Proteobacteria | 0 | 1 | 0 |
| Helicobacter pylori GAM268Bii | Proteobacteria | 0 | 1 | 0 |
| Helicobacter pylori GAM270ASi | Proteobacteria | 0 | 1 | 0 |
| Ralstonia sp. 5 2 56FAA | Proteobacteria | 0 | 1 | 0 |

(continued)

| Strain | phylum | butyrate | ammonia | TMA/ TMAO |
|---|---|---|---|---|
| Helicobacter pylori GAMchJs114i | Proteobacteria | 0 | 1 | 0 |
| Helicobacter pylori GAMchJs124i | Proteobacteria | 0 | 1 | 0 |
| Helicobacter pylori GAM260ASi | Proteobacteria | 0 | 1 | 0 |
| Helicobacter pylori GAMchJs106B | Proteobacteria | 0 | 1 | 0 |
| Helicobacter pylori GAM252Bi | Proteobacteria | 0 | 1 | 0 |
| Helicobacter pylori GAM105Ai | Proteobacteria | 0 | 1 | 0 |
| Helicobacter pylori GAM244Ai | Proteobacteria | 0 | 1 | 0 |
| Helicobacter pylori GAM201Ai | Proteobacteria | 0 | 1 | 0 |
| Helicobacter pylori GAM265BSii | Proteobacteria | 0 | 1 | 0 |
| Helicobacter pylori GAM80Ai | Proteobacteria | 0 | 1 | 0 |
| Helicobacter pylori GAMchJs136i | Proteobacteria | 0 | 1 | 0 |
| Helicobacter pylori HP250AFiii | Proteobacteria | 0 | 1 | 0 |
| Helicobacter pylori GAM119Bi | Proteobacteria | 0 | 1 | 0 |
| Helicobacter pylon 83 | Proteobacteria | 0 | 1 | 0 |
| Helicobacter pylori 35A | Proteobacteria | 0 | 1 | 0 |
| Ralstonia sp. 5 7 47FAA | Proteobacteria | 0 | 1 | 0 |
| Helicobacter pylori GAM103Bi | Proteobacteria | 0 | 1 | 0 |
| Helicobacter pylori GAM112Ai | Proteobacteria | 0 | 1 | 0 |
| Helicobacter pylori HP250BFii | Proteobacteria | 0 | 1 | 0 |
| Helicobacter pylori GAMchJs 117 Ai | Proteobacteria | 0 | 1 | 0 |
| Helicobacter pylori GAM42Ai | Proteobacteria | 0 | 1 | 0 |
| Helicobacter pylori HP250ASii | Proteobacteria | 0 | 1 | 0 |
| Helicobacter pylori HP260AFi | Proteobacteria | 0 | 1 | 0 |
| Helicobacter pylori HP260AFii | Proteobacteria | 0 | 1 | 0 |
| Helicobacter pylori HP260BFii | Proteobacteria | 0 | 1 | 0 |
| Helicobacter pylori GAM250AFi | Proteobacteria | 0 | 1 | 0 |
| Helicobacter pylori GAM249T | Proteobacteria | 0 | 1 | 0 |
| Helicobacter pylori GAM245Ai | Proteobacteria | 0 | 1 | 0 |
| Helicobacter pylori GAM114Ai | Proteobacteria | 0 | 1 | 0 |
| Helicobacter pylori GAM264Ai | Proteobacteria | 0 | 1 | 0 |
| Helicobacter pylori GAM210Bi | Proteobacteria | 0 | 1 | 0 |
| Helicobacter pylori GAM231Ai | Proteobacteria | 0 | 1 | 0 |
| Helicobacter pylori GAM120Ai | Proteobacteria | 0 | 1 | 0 |
| Helicobacter pylori GAM118Bi | Proteobacteria | 0 | 1 | 0 |
| Helicobacter pylori GAM263BFi | Proteobacteria | 0 | 1 | 0 |
| Helicobacter pylori HP250BFiV | Proteobacteria | 0 | 1 | 0 |
| Helicobacter pylori HP250AFii | Proteobacteria | 0 | 1 | 0 |
| Helicobacter pylori GAM250T | Proteobacteria | 0 | 1 | 0 |

(continued)

| Strain | phylum | butyrate | ammonia | TMA/ TMAO |
|---|---|---|---|---|
| Helicobacter pylori HP250BFi | Proteobacteria | 0 | 1 | 0 |
| Helicobacter pylori GAM254Ai | Proteobacteria | 0 | 1 | 0 |
| Helicobacter pylori GAM100Ai | Proteobacteria | 0 | 1 | 0 |
| Helicobacter pylori HP250ASi | Proteobacteria | 0 | 1 | 0 |
| Citrobacter freundii 4 7 47CFAA | Proteobacteria | 0 | 1 | 0 |
| Helicobacter pylori GAM83T | Proteobacteria | 0 | 1 | 0 |
| Citrobacter sp. 30 2 | Proteobacteria | 0 | 1 | 0 |
| Coprococcus sp. ART55/1 | Firmicutes | 1 | 0 | 0 |
| Acidami0coccus sp. HPA0509 | Firmicutes | 1 | 0 | 0 |
| Clostridium sp. L2-50 | Firmicutes | 1 | 0 | 0 |
| Coprococcus eutactus ATCC 27759 | Firmicutes | 1 | 0 | 0 |
| Rumi0coccaceae bacterium D16 | Firmicutes | 1 | 0 | 0 |
| Acidami0coccus sp. D21 | Firmicutes | 1 | 0 | 0 |
| Clostridiales butyrate-producing bacterium SSC/2 | Firmicutes | 1 | 0 | 0 |
| Roseburia intestinalis XB6B4 | Firmicutes | 1 | 0 | 0 |
| Anaerostipes sp. 3 2 56FAA | Firmicutes | 1 | 0 | 0 |
| Eubacterium rectale M104/1 | Firmicutes | 1 | 0 | 0 |
| Roseburia intestinalis M50/1 | Firmicutes | 1 | 0 | 0 |
| Clostridium sp. M62/1 | Firmicutes | 1 | 0 | 0 |
| Lachnospiraceae bacterium 3 1 57FAA CT1 | Firmicutes | 1 | 0 | 0 |
| Lachnospiraceae bacterium 5 1 63FAA | Firmicutes | 1 | 0 | 0 |
| Lachnospiraceae bacterium 7 1 58FAA | Firmicutes | 1 | 0 | 0 |
| Faecalibacterium prausnitzii M21/2 | Firmicutes | 1 | 0 | 0 |
| Clostridium sp. SS2/1 | Firmicutes | 1 | 0 | 0 |
| Anaerostipes caccae DSM 14662 | Firmicutes | 1 | 0 | 0 |
| Anaerofustis stercorihominis DSM 17244 | Firmicutes | 1 | 0 | 0 |
| Anaerotruncus colihominis DSM 17241 | Firmicutes | 1 | 0 | 0 |
| Eubacterium ventriosum ATCC 27560 | Firmicutes | 1 | 0 | 0 |
| Eubacterium rectale DSM 17629 | Firmicutes | 1 | 0 | 0 |
| Coprococcus catus GD/7 | Firmicutes | 1 | 0 | 0 |
| Roseburia intestinalis L1-82 | Firmicutes | 1 | 0 | 0 |
| Faecalibacterium prausnitzii L2-6 | Firmicutes | 1 | 0 | 0 |
| Roseburia inulinivorans DSM 16841 | Firmicutes | 1 | 0 | 0 |
| Faecalibacterium prausnitzii A2-165 | Firmicutes | 1 | 0 | 0 |
| Clostridiales butyrate-producing bacterium SM4/1 | Firmicutes | 1 | 0 | 0 |
| Peptoclostridium difficile 70-100-2010 | Firmicutes | 1 | 0 | 0 |
| Peptoclostridium difficile NAP08 | Firmicutes | 1 | 0 | 0 |
| Subdoligranulum variabile DSM 15176 | Firmicutes | 1 | 0 | 0 |

(continued)

| Strain | phylum | butyrate | ammonia | TMA/ TMAO |
|---|---|---|---|---|
| Flavonifractor plautii ATCC 29863 | Firmicutes | 1 | 0 | 0 |
| Faecalibacterium cf. prausnitzii KLE1255 | Firmicutes | 1 | 0 | 0 |
| Butyrivibrio fibrisolvens 16/4 | Firmicutes | 1 | 0 | 0 |
| Clostridium sp. 7 3 54FAA | Firmicutes | 1 | 0 | 0 |
| Anaerostipes hadrus DSM 3319 | Firmicutes | 1 | 0 | 0 |
| Clostridium perfringens WAL-14572 | Firmicutes | 1 | 0 | 0 |
| Peptoclostridium difficile NAP07 | Firmicutes | 1 | 0 | 0 |
| Coprococcus comes ATCC 27758 | Firmicutes | 1 | 0 | 0 |
| Clostridiales butyrate-producing bacterium SS3/4 | Firmicutes | 1 | 0 | 0 |
| Faecalibacterium prausnitzii SL3/3 | Firmicutes | 1 | 0 | 0 |
| Clostridium sp. 7 2 43FAA | Firmicutes | 1 | 0 | 0 |
| Butyrivibrio crossotus DSM 2876 | Firmicutes | 1 | 0 | 0 |
| Fusobacterium mortiferum ATCC 9817 | Fusobacteria | 1 | 0 | 0 |
| Bilophila sp. 4 130 | Proteobacteria | 1 | 0 | 0 |
| Bilophila wadsworthia 3 1 6 | Proteobacteria | 1 | 0 | 0 |
| **Key:** "1" refers to positive correlation between a bacterial species and a metabolite. "0" refers to no correlation between a bacterial species and a metabolite. | | | | |

## Table 4: CAZy glycoside hydrolase (GH) and glycosyltransferase (GT) family activity prediction.

| Glycoside Hydrolase Family (CAZy) | Family members show glycosidase/glycoside hydrolase activities of: | Genera and glycosidase/glycohydrolase sequences |
|---|---|---|
| GH 1 | β-glucosidases, β-galactosidases; 6-phospho-β-glucosidase, 6-phospho-β-galactosidase, β-mannosidase, β-D-fucosidase and β-glucuronidase | Clostridioides; Enterococcus; Escherichia (Ruminococcus GH1.0 (SEQ ID NO: 31)) |
| GH 2 | β-galactosidases, β-glucuronidases, β-mannosidases, and exo-β-glucosaminidases | Bacteroides; Roseburia (Lactobacillus GH2.0 (SEQ ID NO: 2); Bifidobacterium GH2.0-5 (SEQ ID NO: 8); Bacteroides GH2.0-1 (SEQ ID NO: 11); Bacteroides GH2.0-3 (SEQ ID NO: 43); Bacteroides GH2.0-4 (SEQ ID NO: 44); Bacteroides GH2.0-2 (SEQ ID NO: 79); Bifidobacterium GH2.0-1 (SEQ ID NO: 88); Bifidobacterium |

| | | | |
|---|---|---|---|
| | | | GH2.0-2 (SEQ ID NO: 94); Bifidobacterium GH2.0-3 (SEQ ID NO: 105); Bifidobacterium GH2.0-4 (SEQ ID NO: 114); Bifidobacterium GH2.0-6 (SEQ ID NO: 115)) |
| GH 3 | exo-acting β-D-glucosidases, α-L-arabinofuranosidases, β-D-xylopyranosidases and N-acetyl-β-D-glucosaminidases | | Bacteroides; Escherichia (Bacteroides GH3.0-1 (SEQ ID NO: 12); Bacteroides GH3.0-5 (SEQ ID NO: 18); Bacteroides GH3.0-4 (SEQ ID NO: 48); Bacteroides GH3.0-2 (SEQ ID NO: 56); Bacteroides GH3.0-3 (SEQ ID NO: 64); Bacteroides GH3.0-8 (SEQ ID NO: 99); Bacteroides GH3.0-7 (SEQ ID NO: 110); Bacteroides GH3.0-6 (SEQ ID NO: 117)) |
| GH 4 | α-glucosidases, α-galactosidases, α-glucuronidases, 6-phospho-α-glucosidases, and 6-phospho-β-glucosidases | | Escherichia (Citrobacter GH4.0-2 (SEQ ID NO: 98); Citrobacter GH4.0-1 (SEQ ID NO: 109)) |
| GH 5 | endoglucanase (cellulase), endomannanase, exoglucanases, exomannanases, β-glucosidase, β-mannosidase, 1,6-galactanase, 1,3-mannanase, 1,4-xylanase, endoglycoceramidase, and xyloglucanases | | Bifidobacterium; Bacteroides (Ruminococcus GH5.8 (SEQ ID NO: 37); Paenibacillus GH5.8 (SEQ ID NO: 52)) |
| GH 6 | β-1,4-glucans, endoglucanase (EC 3.2.1.4) and cellobiohydrolase (EC 3.2.1.91) | | |
| GH 7 | endo-1,4-β-glucanase (EC 3.2.1.4), [reducing end-acting] cellobiohydrolase (EC 3.2.1.-), chitosanase (EC 3.2.1.132) and endo-1,3-1,4-β-glucanase (EC 3.2.1.73), cleave β-1,4 glycosidic bonds in cellulose/β-1,4-glucans, and show activity on xylan | | |
| GH 8 | chitosanase (EC 3.2.1.132), cellulase (EC 3.2.1.4), licheninase (EC 3.2.1.73), endo-1,4-β-xylanase (EC 3.2.1.8) and reducing-end-xylose releasing exo-oligoxylanase (EC 3.2.1.156), cleave β-1,4 linkages of β-1,4 glucans, xylans (or xylooligosaccharides), chitosans, and lichenans (1,3-1,4-β-D-glucan) | | Escherichia (Bacteroides GH8.0-2 (SEQ ID NO: 22); Bifidobacterium GH8.0-3 (SEQ ID NO: 26); Bacteroides GH8.0-3 (SEQ ID NO: 30); Bifidobacterium GH8.0-1 (SEQ ID NO: 41); Bacteroides GH8.0 (SEQ ID NO: 45); Bifidobacterium GH8.0-2 (SEQ ID NO: 96)) |
| GH 9 | endoglucanases (cellulases, EC 3.2.1.4) with activity toward xylan, 1,3-1,4-ß-glucan, xyloglucan, and glucomannan | | |
| GH 10 | endo-beta-1,3-xylanase, endo-beta-1,4-xylanases | | Bacteroides |
| GH 11 | endo-β-1,4-xylanases | | |
| GH 12 | endo-β-1,4-glucanase (EC 3.2.1.4), xyloglucan endo-hydrolase (EC 3.2.1.151), and endo-β-1,3-1,4-glucanase (EC 3.2.1.73). Xyloglucan endo-transglycosylase (XET, EC 2.4.1.207) | | |

| GH 13 | α-amylase (EC 3.2.1.1); oligo-1,6-glucosidase (EC 3.2.1.10); α-glucosidase (EC 3.2.1.20); pullulanase (EC 3.2.1.41); cyclomaltodextrinase (EC 3.2.1.54); maltotetraose-forming α-amylase (EC 3.2.1.60); isoamylase (EC 3.2.1.68); dextran glucosidase (EC 3.2.1.70); trehalose-6-phosphate hydrolase (EC 3.2.1.93); maltohexaose-forming α-amylase (EC 3.2.1.98); maltotriose-forming α-amylase (EC 3.2.1.116); maltogenic amylase (EC 3.2.1.133); neopullulanase (EC 3.2.1.135); malto-oligosyltrehalose trehalohydrolase (EC 3.2.1.141); limit dextrinase (EC 3.2.1.142); maltopentaose-forming α-amylase (EC 3.2.1.-); amylosucrase (EC 2.4.1.4); sucrose phosphorylase (EC 2.4.1.7); branching enzyme (EC 2.4.1.18); cyclomaltodextrin glucanotransferase (CGTase) (EC 2.4.1.19); 4-α-glucanotransferase (EC 2.4.1.25); isomaltulose synthase (EC 5.4.99.11); trehalose synthase (EC 5.4.99.16), Notably, a considerable number of GH13 members contain carbohydrate binding modules (CBMs) referred to as starch binding domains belonging to CBM20, CBM21, CBM25, CBM26, CBM34, CBM41, CBM45, CBM48, CBM53, and CBM58 | Bacteroides; Escherichia; Streptomyces; Lactobacillus; Enterococcus; Bifidobacterium; Propionibacterium; Roseburia; Fusobacterium (Streptococcus GH13.28-1 (SEQ ID NO: 19); Streptococcus GH13.5 (SEQ ID NO: 20); Streptococcus GH13.28-2 (SEQ ID NO: 21); Roseburia GH13.41-1 (SEQ ID NO: 23); Roseburia GH13.41-2 (SEQ ID NO: 24); Eubacterium GH13.41 (SEQ ID NO: 28); Bifidobacterium GH13.28 (SEQ ID NO: 68); Bifidobacterium GH13.30 (SEQ ID NO: 104); Butyrivibrio GH13.28 (SEQ ID NO: 124)) |
|---|---|---|
| GH 14 | Not annotated | |
| GH 15 | exo-acting, glucoamylase (EC 3.2.1.3), amyloglucosidase, glucodextranase (EC 3.2.1.70) and α,α-trehalase (EC 3.2.1.28), activity toward α-1,4-glycosidic bonds, α-1,6-, α-1,3- and α-1,2-bonds | |
| GH 16 | keratan-sulfate endo-1,4-β-galactosidases (EC 3.2.1.103), endo-1,3-β-galactanases (EC 3.2.1.-), endo-1,3-β-glucanases (EC 3.2.1.39), endo-1,3(4)-β-glucanases (EC 3.2.1.6), licheninases (EC 3.2.1.73), β-agarases (EC 3.2.1.81), β-porphyranases (EC 3.2.1.178), κ-carrageenases (EC 3.2.1.83), and endo-xyloglucanases (EC 3.2.1.151, a.k.a. xyloglucan endo-hydrolases, XEHs, xyloglucan:xyloglucosyltransferases (EC 2.4.1.207, a.k.a. xyloglucan endo-transglycosylases, XETs), and yeast chitin/beta-glucan crosslinking enzymes Crh1 and Crh2, activity toward β-1,4 or β-1,3 glycosidic bonds | Bacteroides |
| GH 17 | 1,3-β-D-glucan endohydrolases (EC 3.2.1.39) and 1,3;1,4-β-D-glucan endohydrolases (EC 3.1.2.73). A 1,3-β-D-glucan exohydrolase (EC 3.1.2.58), activity toward unbranched, internal 1,3-β-D-glucosidic linkages and 1,4-β-D-glucosidic linkages | |
| GH 18 | chitinases (EC 3.2.1.14) and endo-β-N-acetylglucosaminidases (EC 3.2.1.96) | Bacteroides; Enterococcus |
| GH 19 | chitinases (EC 3.2.1.14) | Escherichia |
| GH 20 | exo-acting β-N-acetylglucosaminidases, β-N-acetylgalactosamindase and β-6-SO3-N-acetylglucosaminidases, human isoenzymes hexosaminidase A and B, exo-acting lacto-N-biosidases, activity toward β-D-Gal-(1→3)-D-GlcNAc disaccharides | Bacteroides |
| GH 21 | Deleted family | |
| GH 22 | Not annotated | |

| GH 23 | lytic transglycosylases (peptidoglycan lyases), family G lysozymes (EC 3.2.1.17; muramidase, peptidoglycan N-acetylmuramoylhydrolase, 1,4-β-N-acetylmuramidase, N-acetylmuramoylhydrolase) | Bacteroides; Citrobacter |
|---|---|---|
| GH 24 | Not annotated | Bacteroides |
| GH 25 | Chalaropsis (CH) type lysozymes, activity toward β-1,4-glycosidic bond between N-acetylmuramic acid (NAM) and N-acetylglucosamine (NAG) | Bacteroides; Enterococcus |
| GH 26 | endo-β-1,4-mannanases, exo-acting β-mannanase, β-1,3:1,4-glucanase and β-1,3-xylanase | Bacteroides (Ruminococcus GH26.0-1 (SEQ ID NO: 32); Ruminococcus GH26.0-2 (SEQ ID NO: 33); Bifidobacterium GH26.0-1 (SEQ ID NO: 51); Bifidobacterium GH26.0-2 (SEQ ID NO: 55)) |
| GH 27 | α-galactosidase, α-N-acetylgalactosaminidase, isomaltodextranases | Bacteroides |
| GH 28 | Polygalacturonases, activity toward α-1,4 glycosidic linkage between galacturonate residues | Bacteroides |
| GH 29 | exo-acting α-fucosidases | Bacteroides |
| GH 30 | glucuronoxylan xylanohydrolases | Bacteroides |
| GH 31 | glycoside hydrolases, α-glucosidases, sucrase/isomaltase, lysosomal α-glucosidase, ER glucosidase II, α-xylosidases, isomaltosyltransferases, maltase/glucoamylases and sulfoquinovosidases | Bacteroides (Ruminococcus GH31.0 (SEQ ID NO: 4); Bacteroides GH31.0-7 (SEQ ID NO: 13); Bacteroides GH31.0-1 (SEQ ID NO: 14); Bacteroides GH31.0-4 (SEQ ID NO: 47); Bacteroides GH31.0-3 (SEQ ID NO: 50); Bacteroides GH31.0-2 (SEQ ID NO: 53); Bacteroides GH31.0-9 (SEQ ID NO: 66); Bacteroides GH31.0-5 (SEQ ID NO: 69); Bacteroides GH31.0-8 (SEQ ID NO: 70); Bacteroides GH31.0-6 (SEQ ID NO: 75); Bacteroides GH31.0-12 (SEQ ID NO: 100); Bacteroides GH31.0-10 (SEQ ID NO: 111); Bacteroides GH31.0-11 (SEQ ID NO: 112); Bacteroides GH31.0-13 (SEQ ID NO: 118)) |

| GH 32 | invertase (EC 3.2.1.26), inulinases (EC 3.2.1.7), exo-inulinases (EC 3.2.1.80), levanases (EC 3.2.1.65), β-2,6-fructan 6-levanbiohydrolases(EC 3.2.1.64), fructan β-(2,1)-fructosidase/1-exohydrolase (EC 3.2.1.153), fructan β-(2,6)-fructosidase/6-exohydrolases (EC 3.2.1.154), sucrose:sucrose 1-fructosyltransferases (EC 2.4.1.99), fructan:fructan 1-fructosyltransferase (EC 2.4.1.100), sucrose:fructan 6-fructosyltransferase (EC 2.4.1.10), fructan:fructan 6G-fructosyltransferase (EC 2.4.1.243) and levan fructosyltransferases (EC 2.4.1.-) | Bacteroides; Escherichia |
|---|---|---|
| GH 33 | sialidases (E.C. 3.2.1.18) and trans-sialidases, activity toward α(2,3) or α(2,6) linkage to galactose, N-acetylgalactosamine, and N-acetylglucosamine or an α(2,8) linkage to another sialic acid | Bacteroides |
| GH 34 | Not annotated | |
| GH 35 | β-galactosidases (EC 3.2.1.23), activity towards β-1,3-, β-1,6- or β-1,4-galactosidic linkages, pectic β-1,4-galactans, β-1,3- and β-1,6-galactosyl linkages of arabinogalactan | Bacteroides; Enterococcus |
| GH 36 | α-galactosidase and α-N-acetylgalactosaminidase | Bacteroides (Lactobacillus GH36.0-1 (SEQ ID NO: 1); Ruminococcus GH36.0 (SEQ ID NO: 3); Lactobacillus GH36.0-2 (SEQ ID NO: 6); Blautia GH36.0 (SEQ ID NO: 42); Lachnospiraceae GH36.0-2 (SEQ ID NO: 57); Lachnospiraceae GH36.0-1 (SEQ ID NO: 72)) |
| GH 37 | activity toward the disaccharide trehalose (α-D-glucopyranosyl-(1→1)-α-D-glucopyranoside), (EC 3.2.1.28) | Escherichia |
| GH 38 | Class II α-mannosidases, Golgi α-mannosidase (2A1) with activity toward α-1,6 and α-1,3-linked mannoses; and lysosomal mannosidases with activity toward α1,2, α1,3 and α1,6 linkages. | Clostridium |
| GH 39 | β-xylosidase and α-L-iduronidase, | Klebsiella |
| GH 40 | Deleted family | |
| GH 41 | Deleted family | |
| GH 42 | β-galactosidases (EC 3.2.1.23), α-L-arabinosidase (EC 3.2.1.55) and β-D-fucosidase (EC 3.2.1.38) | Bacteroides (Lactobacillus GH42.0 (SEQ ID NO: 5); Bifidobacterium GH42.0-2 (SEQ ID NO: 38); Bifidobacterium GH42.0-1 (SEQ ID NO: 39); Klebsiella GH42.0-2 (SEQ ID NO: 49); Escherichia GH42.0 (SEQ ID NO: 63); Klebsiella GH42.0-3 (SEQ ID NO: 83); Klebsiella GH42.0-4 (SEQ ID NO: 84); Klebsiella GH42.0-1 (SEQ ID NO: 92); Klebsiella GH42.0-5 (SEQ ID NO: 93)) |

| GH 43 | α-L-arabinofuranosidases, endo-α-L-arabinanases (or endo-processive arabinanases), β-D-xylosidases, exo α-1,3-galactanase | Bacteroides; Bifidobacterium (Bacteroides GH43.12-1 (SEQ ID NO: 15); Bacteroides GH43.12-8 (SEQ ID NO: 16); Bifidobacterium GH43.10-2 (SEQ ID NO: 25); Ruminococcus GH43.16 (SEQ ID NO: 34); Ruminococcus GH43.37 (SEQ ID NO: 35); Ruminococcus GH43.4 (SEQ ID NO: 36); Bifidobacterium GH43.10-1 (SEQ ID NO: 40); Bacteroides GH43.10-1 (SEQ ID NO: 46); Bacteroides GH43.12-3 (SEQ ID NO: 54); Bacteroides GH43.12-2 (SEQ ID NO: 60); Bacteroides GH43.12-5 (SEQ ID NO: 61); Bacteroides GH43.12-6 (SEQ ID NO: 62); Bacteroides GH43.12-4 (SEQ ID NO: 67); Bacteroides GH43.12-12 (SEQ ID NO: 71); Bacteroides GH43.12-7 (SEQ ID NO: 73); Bacteroides GH43.12-9 (SEQ ID NO: 74); Bacteroides GH43.12-10 (SEQ ID NO: 77); Bacteroides GH43.12-11 (SEQ ID NO: 80); Bacteroides GH43.4 (SEQ ID NO: 90); Bacteroides GH43.19-2 (SEQ ID NO: 101); Bacteroides GH43.10-2 (SEQ ID NO: 102); Bacteroides GH43.0 (SEQ ID NO: 119); Bacteroides GH43.19-1 (SEQ ID NO: 120)) |
| GH 44 | activity toward tetrasaccharide cellooligosaccharides and longer oligomers, carboxymethylcellulose, xylan, lichenan, and xyloglucan | |
| GH 45 | endoglucanases (EC 3.2.1.4), activity toward β-1,4 glucans | |
| GH 46 | endo-β-1,4-chitosanases (EC 3.2.1.132) | |
| GH 47 | exo-acting α-1,2-mannosidases, Class I mannosidases, ER-α-mannosidase I (ERMI), Golgi mannosidase I (Golgi MI) | |
| GH 48 | cellulase, endo-β-1,4-glucanase, chitinase, endo-processive cellulase and cellobiohydrolase | |
| GH 49 | dextranase (EC 3.2.1.11), Penicillium minioluteum Dex49A, Dextran 1,6-α-isomaltotriosidase (EC 3.2.1.95) and isopullulanase (EC 3.2.1.57), activity toward α-1,6-glucosidic linkages or α-1,4-glucosidic linkages | |
| GH 50 | β-agarases (EC 3.2.1.81) activity toward β-1,4 glycosidic bonds of agarose, exo-β-agarases: Aga50A and Aga50D from Saccharophagus degradans and Aga50B from Vibrio sp. | |

| GH 51 | β-1,4-endoglucanase activity towards carboxymethyl cellulose and xylan, activity toward L-arabinofuranosides side chains of hemicelluloses: arabinoxylan, arabinogalactan, and L-arabinan | Bacteroides (Bifidobacterium GH51.0-1 (SEQ ID NO: 7); Bifidobacterium GH51.0-10 (SEQ ID NO: 9); Bacteroides GH51.0-1 (SEQ ID NO: 17); Bifidobacterium GH51.0-8 (SEQ ID NO: 27); Bacteroides GH51.0-2 (SEQ ID NO: 65); Bifidobacterium GH51.0-4 (SEQ ID NO: 82); Bifidobacterium GH51.0-5 (SEQ ID NO: 89); Bacteroides GH51.0-3 (SEQ ID NO: 91); Bifidobacterium GH51.0-2 (SEQ ID NO: 95); Bifidobacterium GH51.0-7 (SEQ ID NO: 97); Bifidobacterium GH51.0-6 (SEQ ID NO: 106); Bifidobacterium GH51.0-9 (SEQ ID NO: 107); Bifidobacterium GH51.0-11 (SEQ ID NO: 108); Bifidobacterium GH51.0-3 (SEQ ID NO: 123)) |
| --- | --- | --- |
| GH 52 | Not annotated | |
| GH 53 | β-1,4-galactanase (EC 3.2.1.89) | Bacteroides |
| GH 54 | α-L-arabinofuranosidase (EC 3.2.1.55) and β-xylosidase (EC 3.2.1.37) | |
| GH 55 | β-1,3-glucanases, including both exo- and endo-enzymes, exo-glucan-1,3-β-glucosidases (EC 3.2.1.58) | |
| GH 56 | Not annotated | |
| GH 57 | α-amylase (EC 3.2.1.1), α-galactosidase (EC 3.2.1.22), amylopullulanase (EC 3.2.1.41), branching enzyme (EC 2.4.1.18) and 4-α-glucanotransferase (EC 2.4.1.25). | Bacteroides |
| GH 58 | endo-N-acetylneuraminidases (endo-sialidases) | |
| GH 59 | Not annotated | Lactobacillus |
| GH 60 | Deleted family | |
| GH 61 | Deleted family | |
| GH 62 | Arabinofuranosidases, activity toward α-1,2 or α-1,3-L-arabinofuranose side chains from xylans | |
| GH 63 | exo-acting α-glucosidases, processing α-glucosidase I enzymes (mannosyl-oligosaccharide glucosidase, EC 3.2.1.106), activity toward terminal α-1,2-glucosidic linkage, Escherichia coli YgjK, activity toward α-1,3-glucosidic linkage of nigerose (Glc-α-1,3-Glc), from Thermus thermophilus HB27 and Rubrobacter radiotolerans RSPS-4 with activity toward α-D-mannopyranosyl-1,2-D-glycerate (mannosylglycerate) and α-D-glucopyranosyl-1,2-D-glycerate (glucosylglycerate) | Escherichia |
| GH 64 | Not annotated | |

| GH 65 | phosphorylases; maltose (Glc-α-1,4-Glc) phosphorylase (EC 2.4.1.8), trehalose (Glc-α1,α1-Glc) phosphorylase (EC 2.4.1.64), kojibiose (Glc-α-1,2-Glc) phosphorylase (EC 2.4.1.230), and trehalose 6-phosphate (Glc-α1,α1-Glc6P) phosphorylase (EC 2.4.1.-). Notably α,α-trehalases (EC 3.2.1.28), activity toward α-glucosidic linkages | Bacteroides; Escherichia |
|---|---|---|
| GH 66 | endo-acting dextranase (Dex; EC 3.2.1.11) and cycloisomaltooligosaccharide glucanotransferase (CITase; EC 2.4.1.248), activity toward α-1,6 linkages of dextran, (Type I) Dexs, (Type II) Dexs with low CITase activity, and (Type III) CITases | |
| GH 67 | alpha-glucuronidase, uncapping decorated xyloooligosaccharides, making these molecules available to beta-xylosidases | |
| GH 68 | levansucrase (sucrose:2,6-β-D-fructan 6-β-D-fructosyltransferase; EC 2.4.1.10), β-fructofuranosidase (EC 3.2.1.26), and inulosucrase (EC 2.4.1.9) | |
| GH 69 | Deleted family | |
| GH 70 | Transglucosylases, glucansucrases, activity toward α-1,2; α-1,3; α-1,4; and/or α-1,6, dextransucrase (sucrose:1,6-α-D-glucosyltransferase; EC 2.4.1.5), alternansucrase (sucrose:1,6(1,3)-α-D-glucan-6(3)-α-D-glucosyltransferase, EC 2.4.1.140), mutansucrase (sucrose:1,3-α-D-glucan-3-α-D-glucosyltransferase; EC 2.4.1.125), and reuteransucrase (sucrose:1,4(6-α-D-glucan-4(6)-α-D-glucosyltransferase; EC 2.4.1.-), production of D-glucans | |
| GH 71 | Not annotated | |
| GH 72 | transglycosylases (Aspergillus fumigatus and yeasts), activity toward 1,3-β-glucan | |
| GH 73 | β-N-acetylglucosaminidases, activity toward β-1,4-glycosidic linkage between N-acetylglucosaminyl (NAG) and N-acetylmuramyl (NAM) moieties | Bacteroides; Enterococcus |
| GH 74 | Oligoxyloglucan reducing end-specific cellobiohydrolase (OXG-RCBH, EC 3.2.1.150)" from Geotrichum sp. M128 and "oligoxyloglucan reducing end-specific xyloglucanobiohydrolase (OREX)" from Emericella nidulans (formerly known as Aspergillus nidulans), xyloglucanase; xyloglucan specific endo-β-1,4-glucanases: XEG; and xyloglucan hydrolases: Xgh, (EC 3.2.1.151), activity toward xyloglucans and/or xyloglucan-oligosaccharides, β-1,4-linkages, branched and unbranched | |
| GH 75 | beta-1,4-chitosanases, with endo-splitting activity, GlcN-GlcN and GlcNAc-GlcN links | |
| GH 76 | endo-acting α-mannanases, activity toward α-1,6-mannans (Bacteroides thetaiotaomicron) | (Bacteroides GH76.0-4 (SEQ ID NO: 10); Bacteroides GH76.0-6 (SEQ ID NO: 58); Bacteroides GH76.0-7 (SEQ ID NO: 59); Bacteroides GH76.0-5 (SEQ ID NO: 76); Bacteroides GH76.0-3 (SEQ ID NO: 78); Bacteroides GH76.0-1 (SEQ ID NO: 85); |

| | | Bacteroides GH76.0-2 (SEQ ID NO: 86)) |
|---|---|---|
| GH 77 | α-amylase clan GH-H, 4-α-glucanotransferase (EC 2.4.1.25), disproportionating enzyme (D-enzyme) (plants), amylomaltase (bacteria), glucan-chain transfer from/to α-1,4-glucan | Bacteroides; Escherichia |
| GH 78 | α-L-rhamnosidases with activity toward α-L-rhamnosyl-linkages in L-rhamnosides (EC 3.2.1.40), naringin, hesperidin and rutin, rhamnogalacturonan and arabinogalactan, rhamnogalacturonan hydrolase, naringinase | Bacteroides |
| GH 79 | β-glucuronidase (EC 3.2.1.31), β-4-O-methyl-glucuronidase (EC 3.2.1.-), baicalin β-glucuronidase (EC 3.2.1.167), heparanase (EC 3.2.1.166), and hyaluronidase (EC 3.2.1.-) | |
| GH 80 | endo-acting β-1,4-chitosanases of bacterial origin | |
| GH 81 | Not annotated | |
| GH 82 | Activity toward β-1,4 galactosidic bonds (of the marine algal polysaccharide iota-carrageenan), iota-carrageenase | |
| GH 83 | Not annotated | |
| GH 84 | β-N-acetylglucosaminidases, β-N-acetylhyaluronidases, O-GlcNAcase | Bacteroides |
| GH 85 | Endo-β-N-acetylglucosaminidases (ENGse), Endo-H, Endo-A, Endo-Fsp, Endo-F1, Endo-D and Endo-E, Endo-F2 and Endo-F3, Endo-M, Arthrobacter protophormiae (ApGH85) and Endo-M from Mucor hiemalis (MhGH85) | Bifidobacterium |
| GH 86 | β-agarases (EC 3.2.1.81), activity toward β-1,4 glycosidic bonds of agarose, AgrA (Pseudoalteromonas atlantica), AgaO (Microbulbifer thermotolerans) JAMB-A94, Aga86E (Saccharophagus degradans 2-40) | |
| GH 87 | Not annotated | |
| GH 88 | unsaturated glucuronyl hydrolases, activity toward β-1,3- or β-1,4-linked bonds, Clostridium perfringens | Bacteroides |
| GH 89 | N-acetylglucosaminidases, human lysosomal enzyme, NAGLU, activity toward heparan sulfate, CpGH89 (Clostridium perfringens) | Bacteroides |
| GH 90 | Not annotated | |
| GH 91 | di-fructofuranose 1,2':2,3' dianhydride hydrolase, DFA-IIIase | |
| GH 92 | exo-acting α-mannosidases, α-1,2-mannosidase (Microbacterium sp. M-90), Bacteroides thetaiotaomicron, activity toward α-1,2-mannosidase, α-1,3-mannosidase, α-1,4-mannosidase and α-1,6-mannosidase, CcGH92_5 (Cellulosimicrobium cellulans (formerly Arthrobacter luteus)), activity toward mannose-1-phosphate-6-mannosides | Bacteroides (Bacteroides GH92.0-5 (SEQ ID NO: 29); Bacteroides GH92.0-6 (SEQ ID NO: 81); Bacteroides GH92.0-4 (SEQ ID NO: 87); Bacteroides GH92.0-3 (SEQ ID NO: 113); Bacteroides GH92.0-1 (SEQ ID NO: 121); Bacteroides GH92.0-2 (SEQ ID NO: 122)) |
| GH 93 | Activity toward linear α-1,5-L-arabinan (EC:3.2.1-), Abnx (Penicillium chrysogenum), Arb93A (Fusarium graminearum) | |

| GH 94 | Phosphorylases, activity toward β-glycosidic bonds, cellobiose (Glc-β1,4-Glc) phosphorylase (EC 2.4.1.20), cellodextrin ((Glc-β1,4-)n-1Glc; n ≥ 3) phosphorylase (EC 2.4.1.49), and N,N′-diacetyl chitobiose (GlcNAc-β1,4-GlcNAc) phosphorylase, Clostridium thermocellum | Eubacterium |
|---|---|---|
| GH 95 | 1,2-α-L-fucosidases (EC 3.2.1.63), activity toward α-Fuc-1,2-Gal linkages, and 1,2-α-L-galactosidases, activity toward L-galactoside linkages in arabinoxylans, Bifidobacterium bifidum (BbAfcA) | Bacteroides |
| GH 96 | Not annotated | |
| GH 97 | α-glucosidase (EC 3.2.1.20) and α-galactosidase (EC 3.2.1.22), activity toward α-linked D-glycosides, Bacteroides thetaiotaomicron, activity toward α-1,6-, α-1,3- and α-1,2-, as well as α-1,4-linkages | Bacteroides (Bacteroides GH97.0 (SEQ ID NO: 103)) |
| GH 98 | endo-β-galactosidases, Sp3GH98 and Sp4GH98 (S. pneumoniae) | |
| GH 99 | Endo-α-mannosidase activity toward glucose-substituted mannose, endo-α-1,2-mannanase activity toward αMan-1,3-αMan-1,2-αMan-1,2-αMan, Shewanella amazonensis, Bacteroides thetaiotaomicron and Bacteroides xylanisolvens activity toward Glc1/3Man9/7GlcNAc2 structures, Glc3Man9GlcNAc2 structure, Manα1-3Manα1-2Manα1-2Manα-OMe | |
| GH 100 | Not annotated | |
| GH 101 | Activity toward disaccharide Gal-beta-1,3-GalNAc-alpha-R, Clostridium perfringens, Streptococcus pnuemoniae, SpGH101 | Enterococcus |
| GH 102 | lytic transglycosylases (peptidoglycan lyases), bacterial family 2, membrane-bound lytic transglycosylase A (MltA) (E. coli), activity toward β-1,4-linkage between N-acetylmuramoyl and N-acetylglucosaminyl residues in peptidoglycan | |
| GH 103 | lytic transglycosylases (peptidoglycan lyases), bacterial family 3, membrane-bound lytic transglycosylase B (MltB) (E. coli), activity toward β-1,4-linkage between N-acetylmuramoyl and N-acetylglucosaminyl residues in peptidoglycan | Escherichia |
| GH 104 | lytic transglycosylases (peptidoglycan lyases), bacterial family 4, lambda phage, activity toward β-1,4-linkage between N-acetylmuramoyl and N-acetylglucosaminyl residues in peptidoglycan | Escherichia |
| GH 105 | Not annotated | Bacteroides |
| GH 106 | Not annotated | |
| GH 107 | Not annotated | |
| GH 108 | Not annotated | Bacteroides |
| GH 109 | α-N-acetylgalactosaminidase (Elizabethkingia meningosepticum) | |

| GH 110 | Activity toward Galα1–3(Fucα1–2)Gal, branched, removal of terminal α-galactose, α-1,3-linked galactose, B. fragilis NCTC 9343 (BfGal110A) | Bacteroides (Bacteroides GH110.0 (SEQ ID NO: 116)) |
|---|---|---|
| GH 111 | Not annotated | |
| GH 112 | phosphorylases; beta-galactoside phosphorylase, β-1,3-D-galactosyl-D-hexososamine phosphorylase (EC 2.4.1.211) and β-1,4-D-galactosyl-L-rhamnose phosphorylase (EC 2.4.1.-), galacto-N-biose phosphorylase, (GNBP), lacto-N-biose I phosphorylase (LNBP), and galacto-N-biose/lacto-N-biose I phosphorylase (GLNBP), with activity toward galacto-N-biose (GNB, Gal-β1,3-GalNAc) and lacto-N-biose I (LNB, Gal-β1,3-GlcNAc), β-1,3-D-galactosyl-D-hexososamine phosphorylase from Bifidobacterium bifidum, Bifidobacterium longum | Bifidobacterium |
| GH 113 | intracellular AaManA (Alicyclobacillus acidocaldarius Tc-12-31), activity toward β-1,4-mannosidic linkages, konjac glucomannan, and galactomannan from locust bean gum, crystalline ivory nut mannan (an unsubstituted β-1,4-mannan) and guar gum (a more highly-substituted galactomannan), endo-type cleavage | |
| GH 114 | endo-α-1,4-polygalactosaminidase (Pseudomonas sp. 881), activity toward α-1,4-polygalactosamine (galactosaminoglycan), α-1,4-linked galactosamine residues, endo-acting manner, Streptomyces griseus | |
| GH 115 | α-glucuronidase, with activity toward 4-O-methyl D-glucuronic acid sidechains from native xylan polysaccharides (EC 3.2.1.131), remove glucuronic acid from both terminal and internal regions of xylooligosaccharides and xylans, Thermoascus aurantiacus, Schizophyllum commune, Pichia stipitis (4-O-methyl)-α-glucuronidase, Streptomyces pristinaespiralis | Bacteroides |
| GH 116 | β-glycosidase (Sulfolobus solfataricus), mammalian non-lysosomal bile acid β-glucosidase GBA2 (EC 3.2.1.45, glucosylceramidase), β-glucosidase (EC 3.2.1.21) and β-xylosidase (EC 3.2.1.37), β-glycosidase from S. solfataricus (SSO1353), β-N-acetylglucosaminidase from S. solfataricus (SSO3039), activity toward gluco- and xylosides β-bound to hydrophobic groups, β-glucosides, glucosylceramides, N-acetyl-glucosaminides, and xylosides, | |
| | subfamily 1 contains GBA2 glucosylceramidase, subfamily 2 includes SSO3039, and subfamily 3 contains SSO1353 | |
| GH 117 | α-1,3-L-(3,6-anhydro)-galactosidase, Zg3597 (Clade C) | |
| GH 118 | Not annotated | |
| GH 119 | Not annotated | |

| GH 120 | β-xylosidase, XylC (Thermoanaerobacterium saccharolyticum), XylB (Bifidobacterium adolescentis), activity toward xylobiose and xylotriose through xylohexaose, exo-xylosidase assorted aryl β-xylosides, weak/no activity toward p-nitrophenyl-α-L-arabinofuranoside | Bifidobacterium |
|---|---|---|
| GH 121 | β-L-arabinobiosidases, HypBA2 (Bifidobacterium longum JCM 1217), activity toward unmodified Arafβ1-2Arafβ1-2Arafβ-hydroxyproline (Ara3-Hyp), but not Arafα1-3Arafβ1-2Arafβ1-2Arafβ-Hyp (Ara4-Hyp) or Arafβ1-2Arafβ-Hyp (Ara2-Hyp), hydroxyproline-rich glycoproteins (HRGPs) such as carrot extensin and potato lectin | |
| GH 122 | Not annotated | |
| GH 123 | N-acetyl-β-galactosaminidases (EC 3.2.1.53), with activity toward glycosphingolipids, hydrolyze non-reducing terminal β-GalNAc linkage, but not β-GlcNAc linkages, distinguished from β-hexosaminidases (EC 3.2.1.52) and N-acetyl-β-glucosaminidases (EC 3.2.1.52), NgaP N-acetyl-β-galactosaminidase (Paenibacillus sp.) with activity toward pNP-β-GalNAc but not pNP-β-GlcNAc, pNP-β-Gal, pNP-α-GalNAc or other pNP-glycosides, CpNga123 from Clostridium perfringens (CpNga123), Bacteroides vulgatus BvGH123 | Bacteroides |
| GH 124 | endo-β1,4-glucanase, CtCel124A (Clostridium thermocellum) | |
| GH 125 | α-mannosidases, SpGH125 (Streptococcus pneumoniae), CpGH125 (Clostridium perfringens), activity toward α-1,6-linked non-reducing terminal mannose residues | Bacteroides |
| GH 126 | Not annotated | Lactobacillus |
| GH 127 | β-L-arabinofuranosidase, HypBA1 (Bifidobacterium longum JCM 1217), previously known as members of the Pfam DUF1680 family | Bacteroides |
| GH 128 | β-1,3-glucanases, activity toward β-1,3 linkages in various β-glucans, GLU1 (EC 3.2.1.39) (L. edodes fruiting bodies (shiitake mushroom)) does not degrade β-1,3-linkages within β-1,3-1,4-glucans such as barley glucan | |
| GH 129 | α-N-acetylgalactosaminidase, exo/endo-α-N-acetylgalactosaminidase, (NagBb) (Bifidobacterium bifidum JCM 1254), mucin degradation, acts more rapidly on GalNAcα1-pNP than Galβ1-3GalNAcα1-pNP, B. longum subsp. longum, B. longum subsp. infants and B. breve, different from exo-α-N-acetylgalactosaminidases (EC 3.2.1.49) | |

| GH 130 | Phosphorylases, activity toward β-mannosidic linkages at the non-reducing end, 4-O-β-D-mannosyl-D-glucose phosphorylase activity (EC 2.4.1.281), BfMGP derived from the gene BF0772 (Bacteroides fragilis), activity toward β-1,4-D-mannosyl-N-acetyl-D-glucosamine linkages in the core of N-glycans, β-1,4-mannooligosaccharide phosphorylase (EC 2.4.1.319) (Ruminococcus albus), 1,4-β-mannosyl-N-acetylglucosamine phosphorylase (EC 2.4.1.320), 1,2-β-oligomannan phosphorylase (Thermoanaerobacter sp. X-514), and β-1,2-mannnobiose phosphorylase (Thermoanaerobacter sp. X-514) | Bacteroides |
|---|---|---|
| GH 131 | β-glucanase, exo-acting, activity toward β-(1,3)- and β-(1,6)-linked glucan substrates, endo-acting activity toward β-(1,4)-linked glucan substrates, can contain cellulose-binding modules from family CBM1, gene Pa_3_10940 (Podospora anserine) expresses broad specificity β-glucanase with exo-β-1,3/1,6- and endo-β-1,4-glucanase activity | |
| GH 132 | Not annotated | |
| GH 133 | Not annotated | Bacteroides |
| GH 134 | β-1,4-mannanases, Man134A (Aspergillus nidulans), weak activity on galactomannan but robust activity on glucomannan, β-1,4-linked mannopentaose and hexaose, SsGH134 (Streptomyces sp. NRRL B-24484), activity toward unsubstituted linear β-mannans over gluco- and galactomannans, activity on β-1,4-linked mannotetraose, pentaose and hexaose | |
| GH 135 | α-galactosidase, α-galactosaminase, N-acetyl-α-galactosaminidase, activity toward galactosaminogalactan (GAG), Aspergillus clavatus | |
| | | |
| Glycosyl Transferase Family (CAZy) | **Family members show glycosyltransferase activities of:** | |

| GT1 | UDP-glucuronosyltransferase (EC 2.4.1.17); zeatin O-β-xylosyltransferase (EC 2.4.2.40); 2-hydroxyacylsphingosine 1-β-galactosyltransferase (EC 2.4.1.45); N-acylsphingosine galactosyltransferase (EC 2.4.1.47); flavonol 3-O-glucosyltransferase (EC 2.4.1.91); anthocyanidin 3-O-glucosyltransferase (EC 2.4.1.115); sinapate 1-glucosyltransferase (EC 2.4.1.120); indole-3-acetate β-glucosyltransferase (EC 2.4.1.121); flavonol L-rhamnosyltransferase (EC 2.4.1.159); sterol glucosyltransferase (EC 2.4.1.173); UDP-Glc: 4-hydroxybenzoate 4-O-β-glucosyltransferase (EC 2.4.1.194); zeatin O-β-glucosyltransferase (EC 2.4.1.203); limonoid glucosyltransferase (EC 2.4.1.210); UDP-GlcA: baicalein 7-O-β-glucuronosyltransferase (EC 2.4.1.253); UDP-Glc: chalcone 4?-O-β-glucosyltransferase (EC 2.4.1.286); ecdysteroid UDP-glucosyltransferase (EC 2.4.1.-); salicylic acid β-glucosyltransferase (EC 2.4.1.-); anthocyanin 3-O-galactosyltransferase (EC 2.4.1.-); anthocyanin 5-O-glucosyltransferase (EC 2.4.1.-); dTDP-β-2-deoxy-L-fucose: α-L-2-deoxyfucosyltransferase (EC 2.4.1.-); UDP-β-L-rhamnose: α-L-rhamnosyltransferase (EC 2.4.1.-); zeaxanthin glucosyltransferase (EC 2.4.1.-) | Bacillus |
| GT2 | cellulose synthase (EC 2.4.1.12); chitin synthase (EC 2.4.1.16); dolichyl-phosphate β-D-mannosyltransferase (EC 2.4.1.83); dolichyl-phosphate β-glucosyltransferase (EC 2.4.1.117); N-acetylglucosaminyltransferase (EC 2.4.1.-); N-acetylgalactosaminyltransferase (EC 2.4.1.-); hyaluronan synthase (EC 2.4.1.212); chitin oligosaccharide synthase (EC 2.4.1.-); β-1,3-glucan synthase (EC 2.4.1.34); β-1,4-mannan synthase (EC 2.4.1.-); β-mannosylphosphodecaprenol-mannooligosaccharide α-1,6-mannosyltransferase (EC 2.4.1.199); UDP-Galf: rhamnopyranosyl-N-acetylglucosaminyl-PP-decaprenol β-1,4/1,5-galactofuranosyltransferase (EC 2.4.1.287); UDP-Galf: galactofuranosyl-galactofuranosyl-rhamnosyl-N-acetylglucosaminyl-PP-decaprenol β-1,5/1,6-galactofuranosyltransferase (EC 2.4.1.288); dTDP-L-Rha: N-acetylglucosaminyl-PP-decaprenol α-1,3-L-rhamnosyltransferase (EC 2.4.1.289) | Bacteroides; Enterococcus |
| GT3 | glycogen synthase (EC 2.4.1.11). | |

| GT4 | sucrose synthase (EC 2.4.1.13); sucrose-phosphate synthase (EC 2.4.1.14); α-glucosyltransferase (EC 2.4.1.52); lipopolysaccharide N-acetylglucosaminyltransferase (EC 2.4.1.56); phosphatidylinositol α-mannosyltransferase (EC 2.4.1.57); GDP-Man: Man1GlcNAc2-PP-dolichol α-1,3-mannosyltransferase (EC 2.4.1.132); GDP-Man: Man3GlcNAc2-PP-dolichol/Man4GlcNAc2-PP-dolichol α-1,2-mannosyltransferase (EC 2.4.1.131); digalactosyldiacylglycerol synthase (EC 2.4.1.141); 1,2-diacylglycerol 3-glucosyltransferase (EC 2.4.1.157); diglucosyl diacylglycerol synthase (EC 2.4.1.208); trehalose phosphorylase (EC 2.4.1.231); NDP-Glc: α-glucose α-glucosyltransferase / α,α-trehalose synthase (EC 2.4.1.245); GDP-Man: Man2GlcNAc2-PP-dolichol α-1,6-mannosyltransferase (EC 2.4.1.257); UDP-GlcNAc: 2-deoxystreptamine α-N-acetylglucosaminyltransferase (EC 2.4.1.283); UDP-GlcNAc: ribostamycin α-N-acetylglucosaminyltransferase (EC 2.4.1.285); UDP-Gal α-galactosyltransferase (EC 2.4.1.-); UDP-Xyl α-xylosyltransferase (EC 2.4.2.-); UDP-GlcA α-glucuronyltransferase (EC 2.4.1.-); UDP-Glc α-glucosyltransferase (EC 2.4.1.-); UDP-GalNAc: GalNAc-PP-Und α-1,3-N-acetylgalactosaminyltransferase (EC 2.4.1.306); UDP-GalNAc: N,N'-diacetylbacillosaminyl-PP-Und α-1,3-N-acetylgalactosaminyltransferase (EC 2.4.1.290); ADP-dependent α-maltose-1-phosphate synthase (2.4.1.-) | Bacteroides |
| GT5 | UDP-Glc: glycogen glucosyltransferase (EC 2.4.1.11); ADP-Glc: starch glucosyltransferase (EC 2.4.1.21); NDP-Glc: starch glucosyltransferase (EC 2.4.1.242); UDP-Glc: α-1,3-glucan synthase (EC 2.4.1.183) UDP-Glc: α-1,4-glucan synthase (EC 2.4.1.-) | Bacteroides |
| GT6 | α-1,3-galactosyltransferase (EC 2.4.1.87); α-1,3 N-acetylgalactosaminyltransferase (EC 2.4.1.40); α-galactosyltransferase (EC 2.4.1.37); globoside α-N-acetylgalactosaminyltransferase (EC 2.4.1.88). | |
| GT7 | lactose synthase (EC 2.4.1.22); β-N-acetylglucosaminyl-glycopeptide β-1,4-galactosyltransferase (EC 2.4.1.38); N-acetyllactosamine synthase (EC 2.4.1.90); xylosylprotein β-4-galactosyltransferase (EC 2.4.1.133); UDP-Gal: neolactotriaosylceramide β-1,4-galactosyltransferase (EC 2.4.1.275); β-1,4-N-acetylglucosaminyltransferase (EC 2.4.1.-) | |
| GT8 | lipopolysaccharide α-1,3-galactosyltransferase (EC 2.4.1.44); UDP-Glc: (glucosyl)lipopolysaccharide α-1,2-glucosyltransferase (EC 2.4.1.-); lipopolysaccharide glucosyltransferase 1 (EC 2.4.1.58); glycogenin glucosyltransferase (EC 2.4.1.186); inositol 1-α-galactosyltransferase (galactinol synthase) (EC 2.4.1.123); homogalacturonan α-1,4-galacturonosyltransferase (EC 2.4.1.43); UDP-GlcA: xylan α-glucuronyltransferase (EC 2.4.1.-) | Helicobacter |
| GT9 | lipopolysaccharide N-acetylglucosaminyltransferase (EC 2.4.1.56); heptosyltransferase (EC 2.4.-.-). | Escherichia |
| GT10 | galactoside α-1,3/1,4-L-fucosyltransferase (EC 2.4.1.65); galactoside α-1,3-L-fucosyltransferase (EC 2.4.1.152); glycoprotein α-1,3-L-fucosyltransferase (EC 2.4.1.214) | |

| GT11 | GDP-L-Fuc: galactoside α-1,2-L-fucosyltransferase (EC 2.4.1.69); GDP-L-Fuc: β-LacNac α-1,3-L-fucosyltransferase (EC 2.4.1.-) | Bacteroides |
|---|---|---|
| GT12 | [N-acetylneuraminyl]-galactosylglucosylceramide N-acetylgalactosaminyltransferase (EC 2.4.1.92). | |
| GT13 | α-1,3-mannosyl-glycoprotein β-1,2-N-acetylglucosaminyltransferase (EC 2.4.1.101) | |
| GT14 | β-1,3-galactosyl-O-glycosyl-glycoprotein β-1,6-N-acetylglucosaminyltransferase (EC 2.4.1.102); N-acetyllactosaminide β-1,6-N-acetylglucosaminyltransferase (EC 2.4.1.150); protein O-β-xylosyltransferase (EC 2.4.2.26); UDP-GlcA:arabinogalactan β-glucuronosyltransferase (EC 2.4.1.-) | Bacteroides |
| GT15 | glycolipid 2-α-mannosyltransferase (EC 2.4.1.131); GDP-Man: α-1,2-mannosyltransferase (EC 2.4.1.-). | |
| GT16 | α-1,6-mannosyl-glycoprotein β-1,2-N-acetylglucosaminyltransferase (EC 2.4.1.143). | |
| GT17 | β-1,4-mannosyl-glycoprotein β-1,4-N-acetylglucosaminyltransferase (EC 2.4.1.144). | |
| GT18 | α-1,3(6)-mannosylglycoprotein β-1,6-N-acetyl-glucosaminyltransferase (EC 2.4.1.155). | |
| GT19 | lipid-A-disaccharide synthase (EC 2.4.1.182). | Bacteroides |
| GT20 | α,α-trehalose-phosphate synthase [UDP-forming] (EC 2.4.1.15); Glucosylglycerol-phosphate synthase (EC 2.4.1.213); trehalose-6-P phosphatase (EC 3.1.3.12); [retaining] GDP-valeniol: validamine 7-phosphate valeniolyltransferase (EC 2.-.-.-) | Bacteroides |
| GT21 | UDP-Glc: ceramide β-glucosyltransferase (EC 2.4.1.80). | |
| GT22 | Dol-P-Man: Man6GlcNAc2-PP-Dol α-1,2-mannosyltransferase (EC 2.4.1.259); Dol-P-Man: Man8GlcNAc2-PP-Dol α-1,2-mannosyltransferase (EC 2.4.1.261); Dol-P-Man: Man2-GlcNAc-phosphatidylinositol α-1,2-mannosyltransferase (EC 2.4.1.-); Dol-P-Man: Man3-GlcNAc-phosphatidylinositol α-1,2-mannosyltransferase (EC 2.4.1.-) | |
| GT23 | N-acetyl-β-D-glucosaminide α-1,6-L-fucosyltransferase (EC 2.4.1.68); chitin-oligosaccharide α-1,6-L-fucosyltransferase (EC 2.4.1.-) | |
| GT24 | UDP-Glc: glycoprotein α-glucosyltransferase (EC 2.4.1.-). | |
| GT25 | lipopolysaccharide β-1,4-galactosyltransferase (EC 2.4.1.-); β-1,3-glucosyltransferase (EC 2.4.1.-); β-1,2-glucosyltransferase (EC 2.4.1.-); β-1,2-galactosyltransferase (EC 2.4.1.-); LPS β-1,4-galactosyltransferase (EC 2.4.1.-); occidiofungin β-xylosyltransferase (EC 2.4.2.-); UDP-Gal:procollagen β-galactosyltransferase (EC 2.4.1.50) | Helicobacter |
| GT26 | UDP-ManNAcA: β-N-acetyl mannosaminuronyltransferase (EC 2.4.1.-); UDP-ManNAc: β-N-acetyl-mannosaminyltransferase (EC 2.4.1.-); UDP-Glc: β-1,4-glucosyltransferase (EC 2.4.1.-); β-1,4-galactosyltransferase (EC 2.4.1.-) | Bacteroides |
| GT27 | polypeptide α-N-acetylgalactosaminyltransferase (EC 2.4.1.41) | |

| GT28 | 1,2-diacylglycerol 3-β-galactosyltransferase (EC 2.4.1.46); 1,2-diacylglycerol 3-β-glucosyltransferase (EC 2.4.1.157); UDP-GlcNAc: Und-PP-MurAc-pentapeptide β-N-acetylglucosaminyltransferase (EC 2.4.1.227); digalactosyldiacylglycerol synthase (EC 2.4.1.241) | Bacillus; Bacteroides; Enterococcus |
|---|---|---|
| GT29 | sialyltransferase (EC 2.4.99.-); β-galactoside α-2,6-sialyltransferase (EC 2.4.99.1); α-N-acetylgalactosaminide α-2,6-sialyltransferase (EC 2.4.99.3); β-galactoside α-2,3-sialyltransferase (EC 2.4.99.4); N-acetyllactosaminide α-2,3-sialyltransferase (EC 2.4.99.6); (α-N-acetyl-neuraminyl-2,3-β-galactosyl-1,3)-N-acetylgalactosaminide α-2,6-sialyltransferase (EC 2.4.99.7); α-N-acetyl-neuraminide α-2,8-sialyltransferase (EC 2.4.99.8); lactosylceramide α-2,3-sialyltransferase (EC 2.4.99.9) | |
| GT30 | CMP-β-KDO: α-3-deoxy-D-manno-octulosonic-acid (KDO) transferase (EC 2.4.99.-). | Bacteroides |
| GT31 | N-acetyllactosaminide β-1,3-N-acetylglucosaminyltransferase (EC 2.4.1.149); Glycoprotein-N-acetylgalactosamine 3-β-galactosyltransferase (EC 2.4.1.122); fucose-specific β-1,3-N-acetylglucosaminyltransferase (EC 2.4.1.-); globotriosylceramide β-1,3-GalNAc transferase (EC 2.4.1.79); chondroitin synthase (β-1,3-GlcUA and β-1,4-GalNAc transferase (EC 2.4.1.175); chondroitin β-1,3-glucuronyltransferase (EC 2.4.1.226); chondroitin β-1,4-N-acetylgalactosaminyltransferase (EC 2.4.1.-); UDP-Gal: β-galactosylxylosylprotein β-1,3-galactosyltransferase (EC 2.4.1.134); UDP-GlcNAc: O-fucosylpeptide β-1,3-N-acetylglucosaminyltransferase (EC 2.4.1.222) | |
| GT32 | α-1,6-mannosyltransferase (EC 2.4.1.-); α-1,4-N-acetylglucosaminyltransferase (EC 2.4.1.-); α-1,4-N-acetylgalactosaminyltransferase (EC 2.4.1.-); GDP-Man: inositol-phosphorylceramide transferase (EC 2.4.1.-); UDP-Gal: β-galactoside α-1,4-galactosyltransferase (EC 2.4.1.-); UDP-Gal: lactose/N-acetyl-lactosamine α-1,4-galactosyltransferase (EC 2.4.1.-) | Bacteroides |
| GT33 | GDP-Man: chitobiosyldiphosphodolichol β-mannosyltransferase (EC 2.4.1.142). | |
| GT34 | UDP-Gal: galactomannan α-1,6-galactosyltransferase (EC 2.4.1.-); UDP-Xyl: xyloglucan α-1,6-xylosyltransferase (EC 2.4.2.39); α-1,2-galactosyltransferase (EC 2.4.1.-) | |
| GT35 | glycogen or starch phosphorylase (EC 2.4.1.1). | Bacteroides; Escherichia; Citrobacter |
| GT36 | Family deleted | |
| GT37 | galactoside 2-L-fucosyltransferase (EC 2.4.1.69) | |
| GT38 | polysialyltransferase (EC 2.4.-.-) | |
| GT39 | Dol-P-Man: protein α-mannosyltransferase (EC 2.4.1.109) | |
| GT40 | β-1,3-galactofuranosyltransferases (EC 2.4.1.-) | |
| GT41 | UDP-GlcNAc: peptide β-N-acetylglucosaminyltransferase (EC 2.4.1.255); UDP-Glc: peptide N-β-glucosyltransferase (EC 2.4.1.-) | |
| GT42 | CMP-NeuAc α-2,3-sialyltransferase (EC 2.4.99.-) | |
| GT43 | β-glucuronyltransferase (EC 2.4.1.135); UDP-Xyl: xylan β-1,4-xylosyltransferase (EC 2.4.2.-) | |

| GT44 | UDP-Glc: α-glucosyltransferase (EC 2.4.1.-); UDP-GlcNAc: α-N-acetylglucosaminyltransferase (EC 2.4.1.-). | |
|------|------|------|
| GT45 | α-N-acteylglucosaminyltransferase (EC 2.4.1.-) | |
| GT46 | Deleted family | |
| GT47 | heparan β-glucuronyltransferase (EC 2.4.1.225); xyloglucan β-galactosyltransferase (EC 2.4.1.-); heparan synthase (EC 2.4.1.-); arabinan α-L-arabinosyltransferase (EC 2.4.2.-). | |
| GT48 | 1,3-β-glucan synthase (EC 2.4.1.34) | |
| GT49 | β-1,3-N-acetylglucosaminyltransferase (EC 2.4.1.-). | |
| GT50 | Dol-P-Man α-1,4-mannosyltransferase (EC 2.4.1.-) | |
| GT51 | murein polymerase (EC 2.4.1.129). | Bacteroides |
| GT52 | α-2,3-sialyltransferase (EC 2.4.99.4); α-glucosyltransferase (EC 2.4.1.-) | |
| GT53 | UDP-L-Ara: α-L-arabinosyltransferase (EC 2.4.2.-) | |
| GT54 | UDP-GlcNAc: α-1,3-D-mannoside β-1,4-N-acetylglucosaminyltransferase (EC 2.4.1.145) | |
| GT55 | GDP-Man: mannosyl-3-phosphoglycerate synthase (EC 2.4.1.217) | |
| GT56 | TDP-Fuc4NAc: lipid II Fuc4NAc transferase (EC 2.4.1.-) | Escherichia |
| GT57 | Dol-P-Glc: α-1,3-glucosyltransferase (EC 2.4.1.-) | |
| GT58 | Dol-P-Man: Man5GlcNAc2-PP-Dol α-1,3-mannosyltransferase (EC 2.4.1.258) | |
| GT59 | Dol-P-Glc: Glc2Man9GlcNAc2-PP-Dol α-1,2-glucosyltransferase (EC 2.4.1.256) | |
| GT60 | UDP-GlcNAc: polypeptide α-N-acetylglucosaminyltransferase (EC 2.4.1.-); UDP-GlcNAc: hydroxyproline polypeptide α-N-acetylglucosaminyltransferase (EC 2.4.1.-) | |
| GT61 | β-1,2-xylosyltransferase (EC 2.4.2.38) ; protein O-β-N-acetylglucosaminyltransferase (EC 2.4.1.94) ; xylan α-1,3-arabinofuranosyltransferase (EC 2.4.2.-) ; | |
| GT62 | α-1,2-mannosyltransferase (EC 2.4.1.-); α-1,6-mannosyltransferase (EC 2.4.1.-) | |
| GT63 | UDP-Glc: DNA β-glucosyltransferase (EC 2.4.1.27) | |
| GT64 | UDP-GlcNAc: heparan α-N-acetylhexosaminyltransferase (EC 2.4.1.224) | |
| GT65 | GDP-Fuc: protein O-α-fucosyltransferase (EC 2.4.1.-) | |
| GT66 | dolichyl-diphosphooligosaccharide—protein glycotransferase (EC 2.4.99.18); undecaprenyl-diphosphooligosaccharide—protein glycotransferase (EC 2.4.99.19) | |
| GT67 | UDP-Gal: phosphoglycan β-1,3-galactosyltransferase 1 (SCG1) (EC 2.4.1.-); UDP-GlcNAc β-1,2-N-acetylglucosaminyltransferase (EC 2.4.1.-) | |
| GT68 | GDP-Fuc: protein O-α-fucosyltransferase (EC 2.4.1.-) | |
| GT69 | GDP-Man: α-1,3-mannosyltransferase (EC 2.4.1.-) | |
| GT70 | UDP-GlcA: β-glucuronosyltransferase (EC 2.4.1.17) | |
| GT71 | α-mannosyltransferase (EC 2.4.1.-) | |
| GT72 | UDP-Glc: DNA α-glucosyltransferase (EC 2.4.1.26) | |

| GT73 | CMP-β-KDO: α-3-deoxy-D-manno-octulosonic-acid (KDO) transferase (EC 2.4.99.-). | Escherichia |
|------|------|------|
| GT74 | α-1,2-L-fucosyltransferase (EC 2.4.1.69) | |
| GT75 | UDP-Glc: self-glucosylating β-glucosyltransferase (EC 2.4.1.-); UDP-L-arabinopyranose mutase (EC 5.4.99.-) | |
| GT76 | Dol-P-Man: α-1,6-mannosyltransferase (EC 2.4.1.-) | |
| GT77 | α-xylosyltransferase (EC 2.4.2.39); α-1,3-galactosyltransferase (EC 2.4.1.37); arabinosyltransferase (EC 2.4.2.-); arabinosyltransferase (EC 2.4.2.-) | |
| GT78 | GDP-Man: α-mannosyltransferase (mannosylglycerate synthase) (EC 2.4.1.-) | |
| GT79 | GDP-D-Ara: phosphoglycan α-1,2-D-arabinopyranosyltransferase 1 (EC 2.4.2.-) | |
| GT80 | β-galactoside α-2,6-sialyltransferase (EC 2.4.99.1); β-galactoside α-2,3-sialyltransferase (EC 2.4.99.4) | |
| GT81 | NDP-Glc: glucosyl-3-phosphoglycerate synthase (EC 2.4.1.-); NDP-Man: mannosyl-3-phosphoglycerate synthase (EC 2.4.1.-); ADP-Glc: glucosyl-2-glycerate synthase (EC 2.4.1.-) | |
| GT82 | UDP-GalNAc: β-1,4-N-acetylgalactosaminyltransferase (EC 2.4.1.-) | |
| GT83 | undecaprenyl phosphate-α-L-Ara4N: 4-amino-4-deoxy-β-L-arabinosyltransferase (EC 2.4.2.43); dodecaprenyl phosphate-β-galacturonic acid: lipopolysaccharide core α-galacturonosyl transferase (EC 2.4.1.-) | Bacteroides |
| GT84 | cyclic β-1,2-glucan synthase (EC 2.4.1.-); | |
| GT85 | β-D-arabinofuranosyl monophosphoryldecaprenol: galactan α-D-arabinofuranosyltransferase (EC 2.4.2.-) | |
| GT86 | Deleted family | |
| GT87 | polyprenol-P-Man: α-1,2-mannosyltransferase (EC 2.4.1.-) | |
| GT88 | UDP-Glc: α-glucosyltransferase (EC 2.4.1.-) | |
| GT89 | β-D-arabinofuranosyl-1-monophosphoryldecaprenol : arabinan β-1,2-arabinofuranosyltransferase (EC 2.4.2.-) | |
| GT90 | UDP-Xyl: (mannosyl) glucuronoxylomannan/galactoxylomannan β-1,2-xylosyltransferase (EC 2.4.2.-); UDP-Glc: protein O-β-glucosyltransferase (EC 2.4.1.-); UDP-Xyl: protein O-β-xylosyltransferase (EC 2.4.2.-) | |
| GT91 | β-1,2-mannosyltransferase (EC 2.4.1.-) | |
| GT92 | UDP-Gal: N-glycan core α-1,6-fucoside β-1,4-galactosyltransferase (EC 2.4.1.-); UDP-Gal: β-galactoside β-1,4-galactosyltransferase (EC 2.4.1.-) | |
| GT93 | UDP-GluA : α-glucuronyltransferase (EC 2.4.1.-) involved in GAG polymerization | |
| GT94 | GDP-Man: GlcA-β-1,2-Man-α-1,3-Glc-β-1,4-Glc-α-1-PP-undecaprenol β-1,4-mannosyltransferase (2.4.1.251) | |
| GT95 | UDP-β-L-Araf:hydroxyproline β-L-arabinofuranosyltransferase (EC 2.4.2.-); | |
| GT96 | UDP-Gal: peptidyl serine α-galactosyltransferase (EC 2.4.1.-) | |

| GT97 | CMP-Neu5Ac:α-galactoside α-2,6-sialyltransferase (EC 2.4.99.-); CMP-Neu5Ac:α-glucoside α-2,6-sialyltransferase (EC 2.4.99.-); | |
|---|---|---|
| GT98 | Dol-P-Man : protein [tryptophan] α-C-mannosyltransferase (EC 2.4.1.-) | |
| GT99 | CMP-β-KDO 3-deoxy-β-D-manno-oct-2-ulosonic acid transferase (EC 2.4.99.-) | |
| GT100 | α-sialyltransferase (EC 2.4.99.-) | |
| GT101 | glucosyltransferase (EC 2.4.1.-) | |

**Table 5: Metabolites and associated indications**

| Metabolite | Indication |
|---|---|
| Short chain fatty acids (SCFA) | acute pouchitis, allergic diseases, AIDS, atherosclerosis, asthma, atopic dermatitis, autism spectrum disorder, chronic functional constipation, celiac disease, chronic atrophic gastritis, chronic pouchitis, Clostridium difficile-associated disease (CDAD), celiac disease, colorectal adenoma, colorectal cancer, Crohn's disease, cystic fibrosis, depression, diabetes (Type I), diabetes (Type II), diarrhea, eczema, enterostomy, familial mediterranean fever, food hypersensitivity, graft-versus-host disease (GvHD), hepatic encephalopathy, hypertension, inflammatory bowel disease, irritable bowel disease, irritable bowel disease-constipation (IBS-C), lung cancer, microscopic colitis, multiple sclerosis, non-alcoholic fatty liver disease (NAFLD), non-alcoholic steatohepatitis (NASH), obesity-related asthma, Parkinson's disease (PD), radiation-induced acute intestinal symptoms, Shigellosis, short bowel syndrome, spinal cord injury associated bowel dysfunction, systemic inflammatory response syndrome, systemic lupus erythematosus, ulcerative colitis, drug toxicity, diarrhea, propionic acidemia |
| Trimethylamine (TMA) / Trimethylamine N-oxide (TMAO) | atherosclerosis, cardiovascular disease, cardiovascular risk in HIV, carotid atherosclerosis, chronic heart disease, chronic heart failure, chronic kidney disease, chronic vascular disease, colorectal cancer, coronary heart disease, coronary artery disease (CAD), diabetes (Type II), end stage renal disease, HIV, inflammatory bowel disease, ischemic attack, metabolic syndrome, non-alcoholic fatty liver disease (NAFLD), obesity, radiation-induced acute intestinal symptoms (RIAISs), stroke |
| Ammonia | chronic kidney disease, Helicobacter pylori infection, hepatic encephalopathy, liver cirrhosis with minimal hepatic encephalopathy (MHE) |
| Bile acid | alcoholic liver cirrhosis, atherosclerosis, chronic pouchitis, cirrhosis, colorectal adenoma, colorectal cancer, colorectal cancer (postcholecystectony pateints), coronary artery disease, Crohn's disease, cystic fibrosis, inflammatory bowel disease, diabetes (Type II), intestinal failure-associated liver disease, irritable bowel disease, irritable bowel disease-constipation (IBS-C), malabsorption syndrome, non-alcoholic fatty liver disease (NAFLD), non-alcoholic steatohepatitis (NASH), obesity, obesity-related asthma, postcholecystectomy, primary biliary cirrhosis, primary sclerosing cholangitis (PSC), progressive familial intrahepatic cholestasis, reflux esophagitis, short bowel syndrome, Steven Johnson syndrome, ulcerative colitis, uncomplicated diverticular disease |
| Lipopolysaccharide | allergic diseases, atherosclerosis, autism spectrum disorder, autoimmune hepatitis, chronic fatigue syndroms (CFS), chronic kidney diseases, chronic vascular diseases, common variable immunodeficiency (CVID), Crohn's disease, depression, diabetes (Type II), hepatic encephalopathy, hepatitis B, hepatitis C, HIV, HIV-elite controllers, intestinal failure-associated liver diseases, irritable bowel disease, metabolic syndrome, neonatal necrotizing enterocolitis (NEC), obesity, Parkinson's disease (PD), ulcerative colitis |
| Indole | Chronic kidney disease, Hartnup disease, phenylketonuria, hepatic encephalopathy |
| p-cresol | Chronic kidney disease |

## Table 19.

| 1 | 2 | 3 | 4 | 5 | 6 |
|---|---|---|---|---|---|
| Bacterial taxa (spore-former) | Public DB # | Xylose-containing Glycan | Ara-containing Glycan | Glu-containing Glycan | Gal-containing Glycan |
| Acetanaerobacterium elongatum | NR_042930 | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising an arabinose glycan unit, optionally wherein the glycan preparation comprises any amount of | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a glucose glycan unit, optionally wherein the glycan preparation comprises any amount of glucose between 1% and 100%, further optionally | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a galactose |
| Acetivibrio cellulolyticus | NR_025917 | | | | |
| Acetivibrio ethanolgignens | FR749897 | | | | |
| Alkaliphilus metalliredigenes | AY137848 | | | | |
| Anaerofustis stercorihominis | ABIL02000005 | | | | |
| Anaerosporobacter mobilis | NR_042953 | | | | |
| Anaerostipes caccae | ABAX03000023 | | | | |
| Anaerostipes sp. 3_2_56FAA | ACWB01000002 | | | | |
| Anaerotruncus colihominis | ABGD02000021 | | | | |
| Bacillus aerophilus | NR_042339 | | | | |
| Bacillus aestuarii | GQ980243 | | | | |
| Bacillus alcalophilus | X76436 | | | | |
| Bacillus amyloliquefaciens | NR_075005 | | | | |
| Bacteroides galacturonicus | DQ497994 | | | | |
| Bacteroides pectinophilus | ABVQ01000036 | | | | |
| Blautia coccoides | AB571656 | | | | |
| Blautia glucerasea | AB588023 | | | | |
| Blautia glucerasei | AB439724 | | | | |
| Blautia hansenii | ABYU02000037 | | | | |
| Blautia hydrogenotrophica | ACBZ01000217 | | | | |
| Blautia luti | AB691576 | | | | |
| Blautia producta | AB600998 | | | | |
| Blautia schinkii | NR_026312 | | | | |
| Blautia sp. M25 | HM626178 | | | | |
| Blautia stercoris | HM626177 | | | | |
| Blautia wexlerae | EF036467 | | | | |
| Brevibacillus laterosporus | NR_037005 | | | | |
| Bryantella formatexigens | ACCL02000018 | | | | |
| Bulleidia extructa | ADFR01000011 | | | | |
| Butyricicoccus pullicaecorum | HH793440 | | | | |
| Butyrivibrio crossotus | ABWN01000012 | | | | |
| Catenibacterium mitsuokai | AB030224 | | | | |
| Chlamydiales bacterium NS11 | JN606074 | | | | |
| Clostridiaceae bacterium JC13 | JF824807 | | | | |
| Clostridiales bacterium 1_7_47FAA | ABQR01000074 | | | | |
| Clostridiales bacterium SY8519 | AB477431 | | | | |
| Clostridiales sp. SM4_1 | FP929060 | | | | |
| Clostridiales sp. SS3_4 | AY305316 | | | | |
| Clostridiales sp. SSC_2 | FP929061 | | | | |
| Clostridium acetobutylicum | NR_074511 | | | | |
| Clostridium aerotolerans | X76163 | | | | |

| | | | | | |
|---|---|---|---|---|---|
| Clostridium aldenense | NR_043680 | sweetn ess, etc.) describ ed herein) compri sing glycan s compri sing a xylose glycan unit, option ally wherei n the glycan prepar ation compri ses any amoun t of xylose betwee n 1% and 100%, further option ally wherei n the glycan prepar ation compri ses a second , third, fourth or fifth glycan unit (option ally, indepe ndentl y selecte | arabinose between 1% and 100%, further optionally wherein the glycan preparation comprises a second, third, fourth or fifth glycan unit (optionally, independen tly selected from xylose, glucose, galactose, mannose, rhamnose, fructose, or fucose), further optionally, wherein the glycan preparation is one of: ara100, ara50gal50, ara50xyl50, ara60xyl40, ara80xyl20, gal20ara80, Gal25Man 25Xyl25Ar a25, gal33man3 3ara33, Gal33Xyl3 3Ara33, gal40ara60, gal60ara40, gal80ara20, glu20ara80, Glu20Gal2 0Man20Xy l20Ara20, Glu25Gal2 | wherein the glycan preparation comprises a second, third, fourth or fifth glycan unit (optionally, independentl y selected from xylose, arabinose, galactose, mannose, rhamnose, fructose, or fucose), further optionally, wherein the glycan preparation is one of: gal50glu25fr u25, gal57glu43, gal57glu43, glu100, Glu10Gal10 Man80, Glu10Gal45 Man45, Glu10Gal80 Man10, glu20ara80, Glu20Gal20 Man20Xyl2 0Ara20, Glu20Gal20 Man60, Glu20Gal40 Man40, Glu20Gal60 Man20, glu20gal80, glu20xyl80, Glu25Gal25 Man25Ara2 5, Glu25Gal25 Man25Xyl2 5, | glycan unit, optionall y wherein the glycan preparati on comprise s any amount of galactose between 1% and 100%, further optionall y wherein the glycan preparati on comprise s a second, third, fourth or fifth glycan unit (optionall y, independ ently selected from xylose, arabinose , glucose, mannose, rhamnose , fructose, or fucose), further optionall y, wherein the |
| Clostridium aldrichii | NR_026099 | | | | |
| Clostridium algidicarnis | NR_041746 | | | | |
| Clostridium algidixylanolyticum | NR_028726 | | | | |
| Clostridium aminovalericum | NR_029245 | | | | |
| Clostridium amygdalinum | AY353957 | | | | |
| Clostridium argentinense | NR_029232 | | | | |
| Clostridium asparagiforme | ACCJ01000522 | | | | |
| Clostridium baratii | NR_029229 | | | | |
| Clostridium bartlettii | ABEZ02000012 | | | | |
| Clostridium beijerinckii | NR_074434 | | | | |
| Clostridium bifermentans | X73437 | | | | |
| Clostridium bolteae | ABCC02000039 | | | | |
| Clostridium butyricum | ABDT01000017 | | | | |
| Clostridium cadaveris | AB542932 | | | | |
| Clostridium carboxidivorans | FR733710 | | | | |
| Clostridium carnis | NR_044716 | | | | |
| Clostridium celatum | X77844 | | | | |
| Clostridium celerecrescens | JQ246092 | | | | |
| Clostridium cellulosi | NR_044624 | | | | |
| Clostridium chauvoei | EU106372 | | | | |
| Clostridium citroniae | ADLJ01000059 | | | | |
| Clostridium clariflavum | NR_041235 | | | | |
| Clostridium clostridiiformes | M59089 | | | | |
| Clostridium clostridioforme | NR_044715 | | | | |
| Clostridium coccoides | EF025906 | | | | |
| Clostridium cochlearium | NR_044717 | | | | |
| Clostridium cocleatum | NR_026495 | | | | |
| Clostridium colicanis | FJ957863 | | | | |
| Clostridium colinum | NR_026151 | | | | |
| Clostridium disporicum | NR_026491 | | | | |
| Clostridium estertheticum | NR_042153 | | | | |
| Clostridium fallax | NR_044714 | | | | |
| Clostridium favososporum | X76749 | | | | |
| Clostridium felsineum | AF270502 | | | | |
| Clostridium frigidicarnis | NR_024919 | | | | |
| Clostridium gasigenes | NR_024945 | | | | |
| Clostridium ghonii | AB542933 | | | | |
| Clostridium glycolicum | FJ384385 | | | | |
| Clostridium glycyrrhizinilyticum | AB233029 | | | | |
| Clostridium haemolyticum | NR_024749 | | | | |
| Clostridium hathewayi | AY552788 | | | | |
| Clostridium hiranonis | AB023970 | | | | |
| Clostridium histolyticum | HF558362 | | | | |
| Clostridium hylemonae | AB023973 | | | | |
| Clostridium indolis | AF028351 | | | | |
| Clostridium innocuum | M23732 | | | | |
| Clostridium irregulare | NR_029249 | | | | |
| Clostridium isatidis | NR_026347 | | | | |
| Clostridium kluyveri | NR_074165 | selecte | Glu25Gal2 | 5, | the |
| Clostridium lactatifermentans | NR_025651 | | | | |

| | | | | | |
|---|---|---|---|---|---|
| Clostridium lavalense | EF564277 | d from arabinose, glucose, galactose, mannose, rhamnose, fructose, or fucose), further optionally, wherein the glycan preparation is one of: ara50xyl50, ara60xyl40, ara80xyl20, gal20xyl80, Gal25Man25Xyl25Ara25, gal33man33xyl33, Gal33Xyl33Ara33, gal40xyl60, gal60xyl40, gal75xyl25, gal80xyl20, Glu20Gal20Man20Xyl20 | 5Man25Ara25, Glu25Gal25Xyl25Ara25, Glu25Man25Xyl25Ara25, glu33gal33ara33, Glu33Man33Ara33, Glu33Xyl33Ara33, glu40ara60, glu60ara40, glu80ara20, man20ara80, Man33Xyl33Ara33, man40ara60, man60ara40, man80ara20, xyl60ara40, xyl75ara25, or xyl80ara20. | Glu25Gal25Xyl25Ara25, Glu25Man25Xyl25Ara25, Glu30Gal30Man40, Glu30Gal40Man30, glu33gal33ara33, glu33gal33fuc33, glu33gal33man33, glu33gal33xyl33, Glu33Man33Ara33, Glu33Man33Xyl33, Glu33Xyl33Ara33, glu40ara60, Glu40Gal20Man40, Glu40Gal30Man30, Glu40Gal40Man20, glu40gal60, glu40xyl60, Glu45Gal10Man45, Glu45Gal45Man10, glu50gal50, Glu5Gal5Man90, Glu5Gal90Man5, glu60ara40, Glu60Gal20Man20, glu60gal40, glu60man40, glu60xyl40, glu66fru33, glu80ara20, Glu80Gal10Man10, glu80gal20, | glycan preparation is one of: ara50gal50, gal100, gal20ara80, gal20xyl80, Gal25Man25Xyl25Ara25, gal33man33ara33, gal33man33xyl33, Gal33Xyl33Ara33, gal40ara60, gal40man60, gal40xyl60, gal50glu25fru25, gal57fru43, gal57glu43, gal60ara40, gal60man40, gal60xyl40, gal75xyl25, gal80ara20, gal80man20, gal80xyl20, Glu10Gal10Man80, Glu10Gal45Man45, Glu10Gal80Man10 |
| Clostridium leptum | AJ305238 | | | | |
| Clostridium limosum | FR870444 | | | | |
| Clostridium magnum | X77835 | | | | |
| Clostridium malenominatum | FR749893 | | | | |
| Clostridium mayombei | FR733682 | | | | |
| Clostridium methylpentosum | ACEC01000059 | | | | |
| Clostridium nexile | X73443 | | | | |
| Clostridium novyi | NR_074343 | | | | |
| Clostridium orbiscindens | Y18187 | | | | |
| Clostridium oroticum | FR749922 | | | | |
| Clostridium paraputrificum | AB536771 | | | | |
| Clostridium phytofermentans | NR_074652 | | | | |
| Clostridium piliforme | D14639 | | | | |
| Clostridium putrefaciens | NR_024995 | | | | |
| Clostridium quinii | NR_026149 | | | | |
| Clostridium ramosum | M23731 | | | | |
| Clostridium rectum | NR_029271 | | | | |
| Clostridium saccharogumia | DQ100445 | | | | |
| Clostridium saccharolyticum | CP002109 | | | | |
| Clostridium sardiniense | NR_041006 | | | | |
| Clostridium sartagoforme | NR_026490 | | | | |
| Clostridium scindens | AF262238 | | | | |
| Clostridium septicum | NR_026020 | | | | |
| Clostridium sordellii | AB448946 | | | | |
| Clostridium sp. 7_2_43FAA | ACDK01000101 | | | | |
| Clostridium sp. D5 | ADBG01000142 | | | | |
| Clostridium sp. HGF2 | AENW01000022 | | | | |
| Clostridium sp. HPB_46 | AY862516 | | | | |
| Clostridium sp. JC122 | CAEV01000127 | | | | |
| Clostridium sp. L2_50 | AAYW02000018 | | | | |
| Clostridium sp. LMG 16094 | X95274 | | | | |
| Clostridium sp. M62_1 | ACFX02000046 | | | | |
| Clostridium sp. MLG055 | AF304435 | | | | |
| Clostridium sp. MT4 E | FJ159523 | | | | |
| Clostridium sp. NMBHI_1 | JN093130 | | | | |
| Clostridium sp. NML 04A032 | EU815224 | | | | |
| Clostridium sp. SS2_1 | ABGC03000041 | | | | |
| Clostridium sp. SY8519 | AP012212 | | | | |
| Clostridium sp. TM_40 | AB249652 | | | | |
| Clostridium sp. YIT 12069 | AB491207 | | | | |
| Clostridium sp. YIT 12070 | AB491208 | | | | |
| Clostridium sphenoides | X73449 | | | | |
| Clostridium spiroforme | X73441 | | | | |
| Clostridium sporogenes | ABKW02000003 | | | | |
| Clostridium sporosphaeroides | NR_044835 | | | | |
| Clostridium stercorarium | NR_025100 | | | | |
| Clostridium sticklandii | L04167 | | | | |
| Clostridium straminisolvens | NR_024829 | | | | |
| Clostridium subterminale | NR_041795 | | | | |
| Clostridium sulfidigenes | NR_044161 | | | | |

| | | | | | |
|---|---|---|---|---|---|
| Clostridium symbiosum | ADLQ01000114 | Ara20, glu20xyl80, Glu25Gal25Man25Xyl25, Glu25Gal25Xyl25Ara25, Glu25Man25Xyl25Ara25, glu33gal33xyl33, Glu33Man33Xyl33, Glu33Xyl33Ara33, glu40xyl60, glu60xyl40, glu80xyl20, man20xyl80, Man33Xyl33Ara33, man40xyl60, man60xyl40, man80xyl20, xyl100 , xyl33glu33gal33, xyl60ara40, xyl75ara25, xyl75gal25, xyl75glu12ga | | glu80man20 , glu80man20 , glu80xyl20, Glu90Gal5Man5, man52glu29gal19, man60glu40 , man62glu38 , man80glu20 , xyl33glu33gal33, or xyl75glu12gal12. | , Glu20Gal20Man20Xyl20Ara20, Glu20Gal20Man60 , Glu20Gal40Man40 , Glu20Gal60Man20 , glu20gal80, Glu25Gal25Man25Ara25, Glu25Gal25Man25Xyl25, Glu25Gal25Xyl25Ara25, Glu30Gal30Man40 , Glu30Gal40Man30 , glu33gal33ara33, glu33gal33fuc33, glu33gal33man33 , glu33gal33xyl33, Glu40Gal20Man40 , Glu40Gal30Man30 , Glu40Gal40Man20 , glu40gal60, Glu45Gal10Man45 , |
| Clostridium tertium | Y18174 | | | | |
| Clostridium tetani | NC_004557 | | | | |
| Clostridium thermocellum | NR_074629 | | | | |
| Clostridium tyrobutyricum | NR_044718 | | | | |
| Clostridium viride | NR_026204 | | | | |
| Clostridium xylanolyticum | NR_037068 | | | | |
| Collinsella aerofaciens | AAVN02000007 | | | | |
| Coprobacillus cateniformis | AB030218 | | | | |
| Coprobacillus sp. 29_1 | ADKX01000057 | | | | |
| Coprobacillus sp. D7 | ACDT01000199 | | | | |
| Coprococcus catus | EU266552 | | | | |
| Coprococcus comes | ABVR01000038 | | | | |
| Coprococcus eutactus | EF031543 | | | | |
| Coprococcus sp. ART55_1 | AY350746 | | | | |
| Deferribacteres sp. oral clone JV006 | AY349371 | | | | |
| Desulfitobacterium frappieri | AJ276701 | | | | |
| Desulfitobacterium hafniense | NR_074996 | | | | |
| Desulfotomaculum nigrificans | NR_044832 | | | | |
| Dorea formicigenerans | AAXA02000006 | | | | |
| Dorea longicatena | AJ132842 | | | | |
| Eggerthella lenta | AF292375 | | | | |
| Erysipelotrichaceae bacterium 5 2 54FAA | ACZW01000054 | | | | |
| Ethanoligenens harbinense | AY675965 | | | | |
| Eubacterium barkeri | NR_044661 | | | | |
| Eubacterium biforme | ABYT01000002 | | | | |
| Eubacterium brachy | U13038 | | | | |
| Eubacterium budayi | NR_024682 | | | | |
| Eubacterium callanderi | NR_026330 | | | | |
| Eubacterium cellulosolvens | AY178842 | | | | |
| Eubacterium contortum | FR749946 | | | | |
| Eubacterium coprostanoligenes | HM037995 | | | | |
| Eubacterium cylindroides | FP929041 | | | | |
| Eubacterium desmolans | NR_044644 | | | | |
| Eubacterium dolichum | L34682 | | | | |
| Eubacterium eligens | CP001104 | | | | |
| Eubacterium fissicatena | FR749935 | | | | |
| Eubacterium hadrum | FR749933 | | | | |
| Eubacterium hallii | L34621 | | | | |
| Eubacterium infirmum | U13039 | | | | |
| Eubacterium limosum | CP002273 | | | | |
| Eubacterium moniliforme | HF558373 | | | | |
| Eubacterium multiforme | NR_024683 | | | | |
| Eubacterium nitritogenes | NR_024684 | | | | |
| Eubacterium nodatum | U13041 | | | | |
| Eubacterium ramulus | AJ011522 | | | | |
| Eubacterium rectale | FP929042 | | | | |
| Eubacterium ruminantium | NR_024661 | | | | |
| Eubacterium saburreum | AB525414 | | | | |
| Eubacterium saphenum | NR_026031 | | | | |
| Eubacterium siraeum | ABCA03000054 | | | | |

| | | | | | |
|---|---|---|---|---|---|
| Eubacterium sp. 3_1_31 | ACTL01000045 | 112, or xyl80ara20. | | | Glu45Gal45Man10, glu50gal50, Glu5Gal5Man90, Glu5Gal90Man5, Glu60Gal20Man20, glu60gal40, Glu80Gal10Man10, glu80gal20, Glu90Gal5Man5, man52glu29gal19, Man66gal33, Man75gal25, Man80gal20, xyl33glu33gal33, xyl75gal25, or xyl75glu12gal12. |
| Eubacterium sp. AS15b | HQ616364 | | | | |
| Eubacterium sp. OBRC9 | HQ616354 | | | | |
| Eubacterium sp. oral clone GI038 | AY349374 | | | | |
| Eubacterium sp. oral clone IR009 | AY349376 | | | | |
| Eubacterium sp. oral clone JH012 | AY349373 | | | | |
| Eubacterium sp. oral clone JI012 | AY349379 | | | | |
| Eubacterium sp. oral clone JN088 | AY349377 | | | | |
| Eubacterium sp. oral clone JS001 | AY349378 | | | | |
| Eubacterium sp. oral clone OH3A | AY947497 | | | | |
| Eubacterium sp. WAL 14571 | FJ687606 | | | | |
| Eubacterium tenue | M59118 | | | | |
| Eubacterium tortuosum | NR_044648 | | | | |
| Eubacterium ventriosum | L34421 | | | | |
| Eubacterium xylanophilum | L34628 | | | | |
| Eubacterium yurii | AEES01000073 | | | | |
| Faecalibacterium prausnitzii | ACOP02000011 | | | | |
| Filifactor alocis | CP002390 | | | | |
| Filifactor villosus | NR_041928 | | | | |
| Flavonifractor plautii | AY724678 | | | | |
| Flexistipes sinusarabici | NR_074881 | | | | |
| Fulvimonas sp. NML 060897 | EF589680 | | | | |
| Fusobacterium nucleatum | ADVK01000034 | | | | |
| Gemmiger formicilis | GU562446 | | | | |
| Geobacillus kaustophilus | NR_074989 | | | | |
| Geobacillus stearothermophilus | NR_040794 | | | | |
| Geobacillus thermodenitrificans | NR_074976 | | | | |
| Geobacillus thermoglucosidasius | NR_043022 | | | | |
| Glocobacter violaccus | NR_074282 | | | | |
| Holdemania filiformis | Y11466 | | | | |
| Hydrogenoanaerobacterium saccharovorans | NR_044425 | | | | |
| Kocuria palustris | EU333884 | | | | |
| Lachnobacterium bovis | GU324407 | | | | |
| Lachnospira multipara | FR733699 | | | | |
| Lachnospira pectinoschiza | L14675 | | | | |
| Lachnospiraceae bacterium 1_1_57FAA | ACTM01000065 | | | | |
| Lachnospiraceae bacterium 1_4_56FAA | ACTN01000028 | | | | |
| Lachnospiraceae bacterium 2_1_46FAA | ADLB01000035 | | | | |
| Lachnospiraceae bacterium 2_1_58FAA | ACTO01000052 | | | | |
| Lachnospiraceae bacterium 3_1_57FAA_CT1 | ACTP01000124 | | | | |
| Lachnospiraceae bacterium 4_1_37FAA | ADCR01000030 | | | | |
| Lachnospiraceae bacterium 5_1_57FAA | ACTR01000020 | | | | |
| Lachnospiraceae bacterium 5_1_63FAA | ACTS01000081 | | | | |
| Lachnospiraceae bacterium 6_1_63FAA | ACTV01000014 | | | | |
| Lachnospiraceae bacterium 8_1_57FAA | ACWQ01000079 | | | | |
| Lachnospiraceae bacterium 9_1_43BFAA | ACTX01000023 | | | | |
| Lachnospiraceae bacterium A4 | DQ789118 | | | | |
| Lachnospiraceae bacterium DJF VP30 | EU728771 | | | | |
| Lachnospiraceae bacterium ICM62 | HQ616401 | | | | |

| | | | | | |
|---|---|---|---|---|---|
| Lachnospiraceae bacterium MSX33 | HQ616384 | | | | |
| Lachnospiraceae bacterium oral taxon 107 | ADDS01000069 | | | | |
| Lachnospiraceae bacterium oral taxon F15 | HM099641 | | | | |
| Lachnospiraceae genomosp. C1 | AY278618 | | | | |
| Lactobacillus rogosae | GU269544 | | | | |
| Lactonifactor longoviformis | DQ100449 | | | | |
| Lutispora thermophila | NR_041236 | | | | |
| Mollicutes bacterium pACH93 | AY297808 | | | | |
| Moorella thermoacetica | NR_075001 | | | | |
| Oscillibacter sp. G2 | HM626173 | | | | |
| Oscillibacter valericigenes | NR_074793 | | | | |
| Oscillospira guilliermondii | AB040495 | | | | |
| Paenibacillus lautus | NR_040882 | | | | |
| Paenibacillus polymyxa | NR_037006 | | | | |
| Paenibacillus sp. HGF5 | AEXS01000095 | | | | |
| Paenibacillus sp. HGF7 | AFDH01000147 | | | | |
| Papillibacter cinnamivorans | NR_025025 | | | | |
| Phascolarctobacterium faecium | | | | | |
| Pseudoflavonifractor capillosus | AY136666 | | | | |
| Robinsoniella peoriensis | AF445258 | | | | |
| Roseburia cecicola | GU233441 | | | | |
| Roseburia faecalis | AY804149 | | | | |
| Roseburia faecis | AY305310 | | | | |
| Roseburia hominis | AJ270482 | | | | |
| Roseburia intestinalis | FP929050 | | | | |
| Roseburia inulinivorans | AJ270473 | | | | |
| Ruminococcaceae bacterium D16 | ADDX01000083 | | | | |
| Ruminococcus albus | AY445600 | | | | |
| Ruminococcus bromii | EU266549 | | | | |
| Ruminococcus callidus | NR_029160 | | | | |
| Ruminococcus champanellensis | FP929052 | | | | |
| Ruminococcus flavefaciens | NR_025931 | | | | |
| Ruminococcus gnavus | X94967 | | | | |
| Ruminococcus hansenii | M59114 | | | | |
| Ruminococcus lactaris | ABOU02000049 | | | | |
| Ruminococcus obeum | AY169419 | | | | |
| Ruminococcus sp. 18P13 | AJ515913 | | | | |
| Ruminococcus sp. 5_1_39BFAA | ACII01000172 | | | | |
| Ruminococcus sp. 9SE51 | FM954974 | | | | |
| Ruminococcus sp. ID8 | AY960564 | | | | |
| Ruminococcus sp. K_1 | AB222208 | | | | |
| Ruminococcus torques | AAVP02000002 | | | | |
| Sarcina ventriculi | NR_026146 | | | | |
| Solobacterium moorei | AECQ01000039 | | | | |
| Sporobacter termitidis | NR_044972 | | | | |
| Sporolactobacillus inulinus | NR_040962 | | | | |
| Streptomyces albus | AJ697941 | | | | |
| Subdoligranulum variabile | AJ518869 | | | | |
| Sutterella parvirubra | AB300989 | | | | |
| Syntrophococcus sucromutans | NR_036869 | | | | |

| | | | |
|---|---|---|---|---|---|---|
| Thermoanaerobacter pseudethanolicus | CP000924 | | | | |
| Thermobifida fusca | NC_007333 | | | | |
| Turicibacter sanguinis | AF349724 | | | | |

## Table 19 (continued)

| 1 | 2 | 7 | 8 | 9 | 10 |
|---|---|---|---|---|---|
| Bacterial taxa (spore-former) | Public DB # | Man-containing Glycan | Rha-containing Glycan | Fru-containing Glycan | Fuc-containing Glycan |
| Acetanaerobacterium elongatum | NR_042930 | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a mannose glycan unit, optionally wherein the glycan preparation comprises any amount | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprisi | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprisi | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a fucose glycan unit, |
| Acetivibrio cellulolyticus | NR_025917 | | | | |
| Acetivibrio ethanolgignens | FR749897 | | | | |
| Alkaliphilus metalliredigenes | AY137848 | | | | |
| Anaerofustis stercorihominis | ABIL02000005 | | | | |
| Anaerosporobacter mobilis | NR_042953 | | | | |
| Anaerostipes caccae | ABAX03000023 | | | | |
| Anaerostipes sp. 3_2_56FAA | ACWB01000002 | | | | |
| Anaerotruncus colihominis | ABGD02000021 | | | | |
| Bacillus acrophilus | NR_042339 | | | | |
| Bacillus aestuarii | GQ980243 | | | | |
| Bacillus alcalophilus | X76436 | | | | |
| Bacillus amyloliquefaciens | NR_075005 | | | | |
| Bacteroides galacturonicus | DQ497994 | | | | |
| Bacteroides pectinophilus | ABVQ01000036 | | | | |
| Blautia coccoides | AB571656 | | | | |
| Blautia glucerasea | AB588023 | | | | |
| Blautia glucerasei | AB439724 | | | | |
| Blautia hansenii | ABYU02000037 | | | | |
| Blautia hydrogenotrophica | ACBZ01000217 | | | | |
| Blautia luti | AB691576 | | | | |
| Blautia producta | AB600998 | | | | |
| Blautia schinkii | NR_026312 | | | | |
| Blautia sp. M25 | HM626178 | | | | |
| Blautia stercoris | HM626177 | | | | |
| Blautia wexlerae | EF036467 | | | | |
| Brevibacillus laterosporus | NR_037005 | | | | |
| Bryantella formatexigens | ACCL02000018 | | | | |
| Bulleidia extructa | ADFR01000011 | | | | |
| Butyricicoccus pullicaecorum | HH793440 | | | | |
| Butyrivibrio crossotus | ABWN01000012 | | | | |

| | | | | | |
|---|---|---|---|---|---|
| Catenibacterium mitsuokai | AB030224 | of mannose between 1% and 100%, further optionally wherein the glycan preparation comprises a second, third, fourth or fifth glycan unit (optionally, independently selected from xylose, arabinose, glucose, galactose, rhamnose, fructose, or fucose), further optionally, wherein the glycan preparation is one of: Gal25Man25Xyl25Ara25, gal33man33ara33, gal33man33xyl33, gal40man60, gal60man40, gal80man20, Glu10Gal10Man80, Glu10Gal45Man45, Glu10Gal80Man10, Glu20Gal20Man20Xy | ng a rhamnose glycan unit, optionally wherein the glycan preparation comprises any amount of rhamnose between 1% and 100%, further optionally wherein the glycan preparation comprises a second, third, fourth or fifth glycan unit (optionally, independently selected from xylose, arabinose, glucose, galactose, mannose, fructose, or fucose), further optionall | ng a fructose glycan unit, optionally wherein the glycan preparation comprises any amount of fructose between 1% and 100%, further optionally wherein the glycan preparation comprises a second, third, fourth or fifth glycan unit (optionally, independently selected from xylose, arabinose, glucose, galactose, mannose, rhamnose, or fucose), further optionally, | optionally wherein the glycan preparation comprises any amount of fucose between 1% and 100%, further optionally wherein the glycan preparation comprises a second, third, fourth or fifth glycan unit (optionally, independently selected from xylose, arabinose, glucose, galactose, mannose, rhamnose, or fructose), further optionally, wherein the glycan preparation is one of: glu33gal33fuc33. |
| Chlamydiales bacterium NS11 | JN606074 | | | | |
| Clostridiaceae bacterium JC13 | JF824807 | | | | |
| Clostridiales bacterium 1_7_47FAA | ABQR01000074 | | | | |
| Clostridiales bacterium SY8519 | AB477431 | | | | |
| Clostridiales sp. SM4_1 | FP929060 | | | | |
| Clostridiales sp. SS3_4 | AY305316 | | | | |
| Clostridiales sp. SSC_2 | FP929061 | | | | |
| Clostridium acetobutylicum | NR_074511 | | | | |
| Clostridium aerotolerans | X76163 | | | | |
| Clostridium aldenense | NR_043680 | | | | |
| Clostridium aldrichii | NR_026099 | | | | |
| Clostridium algidicarnis | NR_041746 | | | | |
| Clostridium algidixylanolyticum | NR_028726 | | | | |
| Clostridium aminovalericum | NR_029245 | | | | |
| Clostridium amygdalinum | AY353957 | | | | |
| Clostridium argentinense | NR_029232 | | | | |
| Clostridium asparagiforme | ACCJ01000522 | | | | |
| Clostridium baratii | NR_029229 | | | | |
| Clostridium bartlettii | ABEZ02000012 | | | | |
| Clostridium beijerinckii | NR_074434 | | | | |
| Clostridium bifermentans | X73437 | | | | |
| Clostridium bolteae | ABCC02000039 | | | | |
| Clostridium butyricum | ABDT01000017 | | | | |
| Clostridium cadaveris | AB542932 | | | | |
| Clostridium carboxidivorans | FR733710 | | | | |
| Clostridium carnis | NR_044716 | | | | |
| Clostridium celatum | X77844 | | | | |
| Clostridium celerecrescens | JQ246092 | | | | |
| Clostridium cellulosi | NR_044624 | | | | |
| Clostridium chauvoei | EU106372 | | | | |
| Clostridium citroniae | ADLJ01000059 | | | | |
| Clostridium clariflavum | NR_041235 | | | | |
| Clostridium clostridiiformes | M59089 | | | | |
| Clostridium clostridioforme | NR_044715 | | | | |
| Clostridium coccoides | EF025906 | | | | |
| Clostridium cochlearium | NR_044717 | | | | |
| Clostridium cocleatum | NR_026495 | | | | |
| Clostridium colicanis | FJ957863 | | | | |
| Clostridium colinum | NR_026151 | | | | |

| | | | | | |
|---|---|---|---|---|---|
| Clostridium disporicum | NR_026491 | 120Ara20, Glu20Gal20Man60, Glu20Gal40Man40, Glu20Gal60Man20, Glu25Gal25Man25Ara25, Glu25Gal25Man25Xyl25, Glu25Man25Xyl25Ara25, Glu30Gal30Man40, Glu30Gal40Man30, glu33gal33man33, Glu33Man33Ara33, Glu33Man33Xyl33, Glu40Gal20Man40, Glu40Gal30Man30, Glu40Gal40Man20, Glu45Gal10Man45, Glu45Gal45Man10, Glu5Gal5Man90, Glu5Gal90Man5, Glu60Gal20Man20, glu60man40, Glu80Gal10Man10, glu80man20, glu80man20, Glu90Gal5Man5, man100, | y, wherein the glycan preparation is rha100. | wherein the glycan preparation is one of: fru100, gal50glu25fru25, gal57fru43, or glu66fru33. | |
| Clostridium estertheticum | NR_042153 | | | | |
| Clostridium fallax | NR_044714 | | | | |
| Clostridium favososporum | X76749 | | | | |
| Clostridium felsineum | AF270502 | | | | |
| Clostridium frigidicarnis | NR_024919 | | | | |
| Clostridium gasigenes | NR_024945 | | | | |
| Clostridium ghonii | AB542933 | | | | |
| Clostridium glycolicum | FJ384385 | | | | |
| Clostridium glycyrrhizinilyticum | AB233029 | | | | |
| Clostridium haemolyticum | NR_024749 | | | | |
| Clostridium hathewayi | AY552788 | | | | |
| Clostridium hiranonis | AB023970 | | | | |
| Clostridium histolyticum | HF558362 | | | | |
| Clostridium hylemonae | AB023973 | | | | |
| Clostridium indolis | AF028351 | | | | |
| Clostridium innocuum | M23732 | | | | |
| Clostridium irregulare | NR_029249 | | | | |
| Clostridium isatidis | NR_026347 | | | | |
| Clostridium kluyveri | NR_074165 | | | | |
| Clostridium lactatifermentans | NR_025651 | | | | |
| Clostridium lavalense | EF564277 | | | | |
| Clostridium leptum | AJ305238 | | | | |
| Clostridium limosum | FR870444 | | | | |
| Clostridium magnum | X77835 | | | | |
| Clostridium malenominatum | FR749893 | | | | |
| Clostridium mayombei | FR733682 | | | | |
| Clostridium methylpentosum | ACEC01000059 | | | | |
| Clostridium nexile | X73443 | | | | |
| Clostridium novyi | NR_074343 | | | | |
| Clostridium orbiscindens | Y18187 | | | | |
| Clostridium oroticum | FR749922 | | | | |
| Clostridium paraputrificum | AB536771 | | | | |
| Clostridium phytofermentans | NR_074652 | | | | |
| Clostridium piliforme | D14639 | | | | |
| Clostridium putrefaciens | NR_024995 | | | | |
| Clostridium quinii | NR_026149 | | | | |
| Clostridium ramosum | M23731 | | | | |
| Clostridium rectum | NR_029271 | | | | |
| Clostridium saccharogumia | DQ100445 | | | | |

| | | |
|---|---|---|
| Clostridium saccharolyticum | CP002109 | man20ara80, man20xyl80, Man33Xyl33Ara33, man40ara60, man40xyl60, man52glu29gal19, man60ara40, man60glu40, man60xyl40, man62glu38, Man66gal33, Man75gal25, man80ara20, Man80gal20, man80glu20, or man80xyl20. |
| Clostridium sardiniense | NR_041006 | |
| Clostridium sartagoforme | NR_026490 | |
| Clostridium scindens | AF262238 | |
| Clostridium septicum | NR_026020 | |
| Clostridium sordellii | AB448946 | |
| Clostridium sp. 7_2_43FAA | ACDK01000101 | |
| Clostridium sp. D5 | ADBG01000142 | |
| Clostridium sp. HGF2 | AENW01000022 | |
| Clostridium sp. HPB_46 | AY862516 | |
| Clostridium sp. JC122 | CAEV01000127 | |
| Clostridium sp. L2_50 | AAYW02000018 | |
| Clostridium sp. LMG 16094 | X95274 | |
| Clostridium sp. M62_1 | ACFX02000046 | |
| Clostridium sp. MLG055 | AF304435 | |
| Clostridium sp. MT4 E | FJ159523 | |
| Clostridium sp. NMBHI_1 | JN093130 | |
| Clostridium sp. NML 04A032 | EU815224 | |
| Clostridium sp. SS2_1 | ABGC03000041 | |
| Clostridium sp. SY8519 | AP012212 | |
| Clostridium sp. TM_40 | AB249652 | |
| Clostridium sp. YIT 12069 | AB491207 | |
| Clostridium sp. YIT 12070 | AB491208 | |
| Clostridium sphenoides | X73449 | |
| Clostridium spiroforme | X73441 | |
| Clostridium sporogenes | ABKW02000003 | |
| Clostridium sporosphaeroides | NR_044835 | |
| Clostridium stercorarium | NR_025100 | |
| Clostridium sticklandii | L04167 | |
| Clostridium straminisolvens | NR_024829 | |
| Clostridium subterminale | NR_041795 | |
| Clostridium sulfidigenes | NR_044161 | |
| Clostridium symbiosum | ADLQ01000114 | |
| Clostridium tertium | Y18174 | |
| Clostridium tetani | NC_004557 | |
| Clostridium thermocellum | NR_074629 | |
| Clostridium tyrobutyricum | NR_044718 | |
| Clostridium viride | NR_026204 | |
| Clostridium xylanolyticum | NR_037068 | |
| Collinsella aerofaciens | AAVN02000007 | |

| | | | | | |
|---|---|---|---|---|---|
| Coprobacillus cateniformis | AB030218 | | | | |
| Coprobacillus sp. 29_1 | ADKX01000057 | | | | |
| Coprobacillus sp. D7 | ACDT01000199 | | | | |
| Coprococcus catus | EU266552 | | | | |
| Coprococcus comes | ABVR01000038 | | | | |
| Coprococcus eutactus | EF031543 | | | | |
| Coprococcus sp. ART55_1 | AY350746 | | | | |
| Deferribacteres sp. oral clone JV006 | AY349371 | | | | |
| Desulfitobacterium frappieri | AJ276701 | | | | |
| Desulfitobacterium hafniense | NR_074996 | | | | |
| Desulfotomaculum nigrificans | NR_044832 | | | | |
| Dorea formicigenerans | AAXA02000006 | | | | |
| Dorea longicatena | AJ132842 | | | | |
| Eggerthella lenta | AF292375 | | | | |
| Erysipelotrichaceae bacterium 5 2 54FAA | ACZW01000054 | | | | |
| Ethanoligenens harbinense | AY675965 | | | | |
| Eubacterium barkeri | NR_044661 | | | | |
| Eubacterium biforme | ABYT01000002 | | | | |
| Eubacterium brachy | U13038 | | | | |
| Eubacterium budayi | NR_024682 | | | | |
| Eubacterium callanderi | NR_026330 | | | | |
| Eubacterium cellulosolvens | AY178842 | | | | |
| Eubacterium contortum | FR749946 | | | | |
| Eubacterium coprostanoligenes | HM037995 | | | | |
| Eubacterium cylindroides | FP929041 | | | | |
| Eubacterium desmolans | NR_044644 | | | | |
| Eubacterium dolichum | L34682 | | | | |
| Eubacterium eligens | CP001104 | | | | |
| Eubacterium fissicatena | FR749935 | | | | |
| Eubacterium hadrum | FR749933 | | | | |
| Eubacterium hallii | L34621 | | | | |
| Eubacterium infirmum | U13039 | | | | |
| Eubacterium limosum | CP002273 | | | | |
| Eubacterium moniliforme | HF558373 | | | | |
| Eubacterium multiforme | NR_024683 | | | | |
| Eubacterium nitritogenes | NR_024684 | | | | |
| Eubacterium nodatum | U13041 | | | | |
| Eubacterium ramulus | AJ011522 | | | | |
| Eubacterium rectale | FP929042 | | | | |
| Eubacterium ruminantium | NR_024661 | | | | |

| | |
|---|---|
| Eubacterium saburreum | AB525414 |
| Eubacterium saphenum | NR_026031 |
| Eubacterium siraeum | ABCA03000054 |
| Eubacterium sp. 3_1_31 | ACTL01000045 |
| Eubacterium sp. AS15b | HQ616364 |
| Eubacterium sp. OBRC9 | HQ616354 |
| Eubacterium sp. oral clone GI038 | AY349374 |
| Eubacterium sp. oral clone IR009 | AY349376 |
| Eubacterium sp. oral clone JH012 | AY349373 |
| Eubacterium sp. oral clone JI012 | AY349379 |
| Eubacterium sp. oral clone JN088 | AY349377 |
| Eubacterium sp. oral clone JS001 | AY349378 |
| Eubacterium sp. oral clone OH3A | AY947497 |
| Eubacterium sp. WAL 14571 | FJ687606 |
| Eubacterium tenue | M59118 |
| Eubacterium tortuosum | NR_044648 |
| Eubactcrium ventriosum | L34421 |
| Eubacterium xylanophilum | L34628 |
| Eubacterium yurii | AEES01000073 |
| Faecalibacterium prausnitzii | ACOP02000011 |
| Filifactor alocis | CP002390 |
| Filifactor villosus | NR_041928 |
| Flavonifractor plautii | AY724678 |
| Flexistipes sinusarabici | NR_074881 |
| Fulvimonas sp. NML 060897 | EF589680 |
| Fusobacterium nucleatum | ADVK01000034 |
| Gemmiger formicilis | GU562446 |
| Geobacillus kaustophilus | NR_074989 |
| Gcobacillus stcarothcrmophilus | NR_040794 |
| Geobacillus thermodenitrificans | NR_074976 |
| Geobacillus thermoglucosidasius | NR_043022 |
| Gloeobacter violaceus | NR_074282 |
| Holdemania filiformis | Y11466 |
| Hydrogenoanaerobacterium saccharovorans | NR_044425 |
| Kocuria palustris | EU333884 |
| Lachnobacterium bovis | GU324407 |
| Lachnospira multipara | FR733699 |
| Lachnospira pectinoschiza | L14675 |
| Lachnospiraceae bacterium 1_1_57FAA | ACTM01000065 |

| | | | | | |
|---|---|---|---|---|---|
| Lachnospiraceae bacterium 1_4_56FAA | ACTN01000028 | | | | |
| Lachnospiraceae bacterium 2_1_46FAA | ADLB01000035 | | | | |
| Lachnospiraceae bacterium 2_1_58FAA | ACTO01000052 | | | | |
| Lachnospiraceae bacterium 3_1_57FAA_CT1 | ACTP01000124 | | | | |
| Lachnospiraceae bacterium 4_1_37FAA | ADCR01000030 | | | | |
| Lachnospiraceae bacterium 5_1_57FAA | ACTR01000020 | | | | |
| Lachnospiraceae bacterium 5_1_63FAA | ACTS01000081 | | | | |
| Lachnospiraceae bacterium 6_1_63FAA | ACTV01000014 | | | | |
| Lachnospiraceae bacterium 8_1_57FAA | ACWQ01000079 | | | | |
| Lachnospiraceae bacterium 9_1_43BFAA | ACTX01000023 | | | | |
| Lachnospiraceae bacterium A4 | DQ789118 | | | | |
| Lachnospiraceae bacterium DJF VP30 | EU728771 | | | | |
| Lachnospiraceae bacterium ICM62 | HQ616401 | | | | |
| Lachnospiraceae bacterium MSX33 | HQ616384 | | | | |
| Lachnospiraceae bacterium oral taxon 107 | ADDS01000069 | | | | |
| Lachnospiraceae bacterium oral taxon F15 | HM099641 | | | | |
| Lachnospiraceae genomosp. C1 | AY278618 | | | | |
| Lactobacillus rogosae | GU269544 | | | | |
| Lactonifactor longoviformis | DQ100449 | | | | |
| Lutispora thermophila | NR_041236 | | | | |
| Mollicutes bacterium pACH93 | AY297808 | | | | |
| Moorella thermoacetica | NR_075001 | | | | |
| Oscillibacter sp. G2 | HM626173 | | | | |
| Oscillibacter valericigenes | NR_074793 | | | | |
| Oscillospira guilliermondii | AB040495 | | | | |
| Paenibacillus lautus | NR_040882 | | | | |
| Paenibacillus polymyxa | NR_037006 | | | | |
| Paenibacillus sp. HGF5 | AEXS01000095 | | | | |
| Paenibacillus sp. HGF7 | AFDH01000147 | | | | |
| Papillibacter cinnamivorans | NR_025025 | | | | |
| Phascolarctobacterium faecium | | | | | |
| Pseudoflavonifractor capillosus | AY136666 | | | | |
| Robinsoniella peoriensis | AF445258 | | | | |
| Roseburia cecicola | GU233441 | | | | |

| | |
|---|---|
| Roseburia faecalis | AY804149 |
| Roseburia faecis | AY305310 |
| Roseburia hominis | AJ270482 |
| Roseburia intestinalis | FP929050 |
| Roseburia inulinivorans | AJ270473 |
| Ruminococcaceae bacterium D16 | ADDX01000083 |
| Ruminococcus albus | AY445600 |
| Ruminococcus bromii | EU266549 |
| Ruminococcus callidus | NR_029160 |
| Ruminococcus champanellensis | FP929052 |
| Ruminococcus flavefaciens | NR_025931 |
| Ruminococcus gnavus | X94967 |
| Ruminococcus hansenii | M59114 |
| Ruminococcus lactaris | ABOU02000049 |
| Ruminococcus obeum | AY169419 |
| Ruminococcus sp. 18P13 | AJ515913 |
| Ruminococcus sp. 5_1_39BFAA | ACII01000172 |
| Ruminococcus sp. 9SE51 | FM954974 |
| Ruminococcus sp. ID8 | AY960564 |
| Ruminococcus sp. K_1 | AB222208 |
| Ruminococcus torques | AAVP02000002 |
| Sarcina ventriculi | NR_026146 |
| Solobacterium moorei | AECQ01000039 |
| Sporobacter termitidis | NR_044972 |
| Sporolactobacillus inulinus | NR_040962 |
| Streptomyces albus | AJ697941 |
| Subdoligranulum variabile | AJ518869 |
| Sutterella parvirubra | AB300989 |
| Syntrophococcus sucromutans | NR_036869 |
| Thermoanaerobacter pseudethanolicus | CP000924 |
| Thermobifida fusca | NC_007333 |
| Turicibacter sanguinis | AF349724 |

## Table 20.

| 1 | 2 | 3 | 4 | 5 |
|---|---|---|---|---|
| **Bacterial taxa (spore-former)** | **Xylose-containing Glycan** | **Ara-containing Glycan** | **Glu-containing Glycan** | **Gal-containing Glycan** |
| Acetivibrio | a glycan preparation (as described herein, e.g., having any DP, DB, alpha/beta-glycosidic bond | a glycan preparation (as described herein, e.g., having any DP, DB, alpha/beta-glycosidic bond ratio, | a glycan preparation (as described herein, e.g., having any DP, DB, alpha/beta-glycosidic bond ratio, | a glycan preparation (as described herein, e.g., having any DP, DB, |
| Bacillus | | | | |
| Bacteroides | | | | |
| Blautia | | | | |

| | | | | |
|---|---|---|---|---|
| Clostridiales | ratio, number of glycosidic bonds, bond regiochemistry and bond stereochemistry, and other characteristics (e.g., solubility, fermentability, viscosity, sweetness, etc.) described herein) comprising glycans comprising a xylose glycan unit, optionally wherein the glycan preparation comprises any amount of xylose between 1% and 100%, further optionally wherein the glycan preparation comprises a second, third, fourth or fifth glycan unit (optionally, independently selected from arabinose, glucose, galactose, mannose, rhamnose, fructose, or fucose), further optionally, wherein the glycan preparation is one of: ara50xyl50, ara60xyl40, ara80xyl20, gal20xyl80, Gal25Man25Xyl25Ara25, gal33man33xyl33, Gal33Xyl33Ara33, gal40xyl60, gal60xyl40, gal75xyl25, gal80xyl20, Glu20Gal20Man20Xyl20Ara20, glu20xyl80, Glu25Gal25Man25Xyl25, Glu25Gal25Xyl25A | number of glycosidic bonds, bond regiochemistry and bond stereochemistry, and other characteristics (e.g., solubility, fermentability, viscosity, sweetness, etc.) described herein) comprising glycans comprising an arabinose glycan unit, optionally wherein the glycan preparation comprises any amount of arabinose between 1% and 100%, further optionally wherein the glycan preparation comprises a second, third, fourth or fifth glycan unit (optionally, independently selected from xylose, glucose, galactose, mannose, rhamnose, fructose, or fucose), further optionally, wherein the glycan preparation is one of: ara100, ara50gal50, ara50xyl50, ara60xyl40, ara80xyl20, gal20ara80, Gal25Man25Xyl25Ara25, gal33man33ara33, Gal33Xyl33Ara33, gal40ara60, gal60ara40, gal80ara20, glu20ara80, Glu20Gal20Man20Xyl20Ara20, Glu25Gal25Man25Ara25, Glu25Gal25Xyl25Ara25, Glu25Man25Xyl25Ara25, glu33gal33ara33, Glu33Man33Ara33, | number of glycosidic bonds, bond regiochemistry and bond stereochemistry, and other characteristics (e.g., solubility, fermentability, viscosity, sweetness, etc.) described herein) comprising glycans comprising a glucose glycan unit, optionally wherein the glycan preparation comprises any amount of glucose between 1% and 100%, further optionally wherein the glycan preparation comprises a second, third, fourth or fifth glycan unit (optionally, independently selected from xylose, arabinose, galactose, mannose, rhamnose, fructose, or fucose), further optionally, wherein the glycan preparation is one of: gal50glu25fru25, gal57glu43, gal57glu43, glu100, Glu10Gal10Man80, Glu10Gal45Man45, Glu10Gal80Man10, glu20ara80, Glu20Gal20Man20Xyl20Ara20, Glu20Gal20Man60, Glu20Gal40Man40, Glu20Gal60Man20, glu20gal80, glu20xyl80, Glu25Gal25Man25Ara25, Glu25Gal25Man25Xyl25, Glu25Gal25Xyl25Ara25, Glu25Man25Xyl25Ara25, | alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a galactose glycan unit, optionally wherein the glycan preparation comprises any amount of galactose between 1% and 100%, further optionally wherein the glycan preparation comprises a second, third, fourth or fifth glycan unit (optionally, independently selected from xylose, arabinose, glucose, mannose, rhamnose, fructose, or fucose), further optionally, wherein the glycan preparation is one of: ara50gal50, gal100, gal20ara80, gal20xyl80, Gal25Man25Xyl25Ara25, gal33man33ara33, gal33man33xyl33, Gal33Xyl33Ara33 |
| Clostridium | | | | |
| Coprobacillus | | | | |
| Coprococcus | | | | |
| Eubacterium | | | | |
| Geobacillus | | | | |
| Lachnospiraceae | | | | |
| Paenibacillus | | | | |
| Roseburia | | | | |
| Ruminococcus | | | | |

| | | | |
|---|---|---|---|
| ra25, Glu25Man25Xyl25Ara25, glu33gal33xyl33, Glu33Man33Xyl33, Glu33Xyl33Ara33, glu40xyl60, glu60xyl140, glu80xyl120, man20xyl80, Man33Xyl33Ara33, man40xyl60, man60xyl40, man80xyl120, xyl100, xyl33glu33gal33, xyl60ara40, xyl75ara25, xyl75gal25, xyl75glu12gal12, or xyl80ara20. | Glu33Xyl133Ara33, glu40ara60, glu60ara40, glu80ara20, man20ara80, Man33Xyl33Ara33, man40ara60, man60ara40, man80ara20, xyl60ara40, xyl75ara25, or xyl180ara20. | Glu30Gal30Man40, Glu30Gal40Man30, glu33gal33ara33, glu33gal33fuc33, glu33gal33man33, glu33gal33xyl33, Glu33Man33Ara33, Glu33Man33Xyl33, Glu33Xyl33Ara33, glu40ara60, Glu40Gal20Man40, Glu40Gal30Man30, Glu40Gal40Man20, glu40gal60, glu40xyl60, Glu45Gal10Man45, Glu45Gal45Man10, glu50gal50, Glu5Gal5Man90, Glu5Gal90Man5, glu60ara40, Glu60Gal20Man20, glu60gal40, glu60man40, glu60xyl140, glu66fru33, glu80ara20, Glu80Gal10Man10, glu80gal20, glu80man20, glu80man20, glu80xyl120, Glu90Gal5Man5, man52glu29gal19, man60glu40, man62glu38, man80glu20, xyl133glu33gal33, or xyl75glu12gal12. | , gal40ara60, gal40man60, gal40xyl60, gal50glu25fru25, gal57fru43, gal57glu43, gal60ara40, gal60man40, gal60xyl140, gal75xyl125, gal80ara20, gal80man20, gal80xyl120, Glu10Gal10Man80, Glu10Gal45Man45, Glu10Gal80Man10, Glu20Gal20Man20Xyl20Ara20, Glu20Gal20Man60, Glu20Gal40Man40, Glu20Gal60Man20, glu20gal80, Glu25Gal25Man25Ara25, Glu25Gal25Man25Xyl25, Glu25Gal25Xyl25Ara25, Glu30Gal30Man40, Glu30Gal40Man30, glu33gal33ara33, glu33gal33fuc33, glu33gal33man33, glu33gal33xyl133, Glu40Gal20Man40, Glu40Gal30Man30, Glu40Gal40Man20, glu40gal60, Glu45Gal10Man45, Glu45Gal45Man10, glu50gal50, Glu5Gal5Man90, |

| | | | | Glu5Gal90Man5, Glu60Gal20Man20, glu60gal40, Glu80Gal10Man10, glu80gal20, Glu90Gal5Man5, man52glu29gal19, Man66gal33, Man75gal25, Man80gal20, xyl33glu33gal33, xyl75gal25, or xyl75glu12gal12. |
|---|---|---|---|---|
| | | | | |

## Table 20 (continued)

| 1 | 6 | 7 | 8 | 9 |
|---|---|---|---|---|
| **Bacterial taxa (spore-former)** | **Man-containing Glycan** | **Rha-containing Glycan** | **Fru-containing Glycan** | **Fuc-containing Glycan** |
| Acetivibrio | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a mannose glycan unit, optionally wherein the glycan preparation comprises any amount of mannose between 1% and 100%, further optionally wherein the glycan preparation | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a rhamnose glycan unit, optionally wherein the glycan preparation comprises any amount of rhamnose between 1% and 100%, further optionally wherein the glycan preparation comprises a second, third, fourth or fifth glycan unit (optionally, independently selected from xylose, arabinose, glucose, galactose, mannose, | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a fructose glycan unit, optionally wherein the glycan preparation comprises any amount of fructose between 1% and 100%, further optionally wherein the glycan preparation comprises a second, third, fourth or fifth glycan unit (optionally, independently selected from xylose, arabinose, glucose, galactose, mannose, | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a fucose glycan unit, optionally wherein the glycan preparation comprises any amount of fucose between 1% and 100%, further optionally wherein the glycan |
| Bacillus | | | | |
| Bacteroides | | | | |
| Blautia | | | | |
| Clostridiales | | | | |
| Clostridium | | | | |
| Coprobacillus | | | | |
| Coprococcus | | | | |
| Eubacterium | | | | |
| Geobacillus | | | | |
| Lachnospiraceae | | | | |
| Paenibacillus | | | | |
| Roseburia | | | | |
| Ruminococcus | | | | |

| | | | | |
|---|---|---|---|---|
| comprises a second, third, fourth or fifth glycan unit (optionally, independently selected from xylose, arabinose, glucose, galactose, rhamnose, fructose, or fucose), further optionally, wherein the glycan preparation is one of: Gal25Man25Xyl25Ara25, gal33man33ara33, gal33man33xyl33, gal40man60, gal60man40, gal80man20, Glu10Gal10Man80, Glu10Gal45Man45, Glu10Gal80Man10, Glu20Gal20Man20Xyl20Ara20, Glu20Gal20Man60, Glu20Gal40Man40, Glu20Gal60Man20, Glu25Gal25Man25Ara25, Glu25Gal25Man25Xyl25, Glu25Man25Xyl25Ara25, Glu30Gal30Man40, Glu30Gal40Man30, glu33gal33man33, Glu33Man33Ara33, Glu33Man33Xyl33, Glu40Gal20Man40, Glu40Gal30Man30 | fructose, or fucose), further optionally, wherein the glycan preparation is rha100. | rhamnose, or fucose), further optionally, wherein the glycan preparation is one of: fru100, gal50glu25fru25, gal57fru43, or glu66fru33. | preparation comprises a second, third, fourth or fifth glycan unit (optionally, independently selected from xylose, arabinose, glucose, galactose, mannose, rhamnose, or fructose), further optionally, wherein the glycan preparation is one of: glu33gal33fuc33. | |

| | | | | |
|---|---|---|---|---|
| , Glu40Gal40Man20 , Glu45Gal10Man45 , Glu45Gal45Man10 , Glu5Gal5Man90, Glu5Gal90Man5, Glu60Gal20Man20 , glu60man40, Glu80Gal10Man10 , glu80man20, glu80man20, Glu90Gal5Man5, man100, man20ara80, man20xyl80, Man33Xyl33Ara33 , man40ara60, man40xyl60, man52glu29gal19, man60ara40, man60glu40, man60xyl40, man62glu38, Man66gal33, Man75gal25, man80ara20, Man80gal20, man80glu20, or man80xyl20. | | | | |

## Table 21.

| 1 | 2 | 3 | 4 | 5 |
|---|---|---|---|---|
| **Bacterial taxa (spore-former)** | **Xylose-containing Glycan** | **Ara-containing Glycan** | **Glu-containing Glycan** | **Gal-containing Glycan** |
| Blautia | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, |
| Clostridiales | | | | |
| Clostridium | | | | |
| Eubacterium | | | | |
| Lachnospiraceae | | | | |
| Roseburia | | | | |
| Ruminococcus | | | | |

| | | | |
|---|---|---|---|
| xylose glycan unit, optionally wherein the glycan preparation comprises any amount of xylose between 1% and 100%, further optionally wherein the glycan preparation comprises a second, third, fourth or fifth glycan unit (optionally, independently selected from arabinose, glucose, galactose, mannose, rhamnose, fructose, or fucose), further optionally, wherein the glycan preparation is one of: ara50xyl50, ara60xyl40, ara80xyl20, gal20xyl80, Gal25Man25Xyl25Ara25, gal33man33xyl33, Gal33Xyl33Ara33, gal40xyl60, gal60xyl40, gal75xyl25, gal80xyl20, Glu20Gal20Man20Xyl20Ara20, glu20xyl80, Glu25Gal25Man25Xyl25, Glu25Gal25Xyl25Ara25, Glu25Man25Xyl25Ara25, glu33gal33xyl33, Glu33Man33Xyl33, Glu33Xyl33Ara33, glu40xyl60, glu60xyl40, glu80xyl20, man20xyl80, | an arabinose glycan unit, optionally wherein the glycan preparation comprises any amount of arabinose between 1% and 100%, further optionally wherein the glycan preparation comprises a second, third, fourth or fifth glycan unit (optionally, independently selected from xylose, glucose, galactose, mannose, rhamnose, fructose, or fucose), further optionally, wherein the glycan preparation is one of: ara100, ara50gal50, ara50xyl50, ara60xyl40, ara80xyl20, gal20ara80, Gal25Man25Xyl25Ara25, gal33man33ara33, Gal33Xyl33Ara33, gal40ara60, gal60ara40, gal80ara20, glu20ara80, Glu20Gal20Man20Xyl20Ara20, Glu25Gal25Man25Ara25, Glu25Gal25Xyl25Ara25, Glu25Man25Xyl25Ara25, glu33gal33ara33, Glu33Man33Ara33, Glu33Xyl33Ara33, glu40ara60, glu60ara40, glu80ara20, | viscosity, sweetness, etc.) described herein) comprising glycans comprising a glucose glycan unit, optionally wherein the glycan preparation comprises any amount of glucose between 1% and 100%, further optionally wherein the glycan preparation comprises a second, third, fourth or fifth glycan unit (optionally, independently selected from xylose, arabinose, galactose, mannose, rhamnose, fructose, or fucose), further optionally, wherein the glycan preparation is one of: gal50glu25fru25, gal57glu43, gal57glu43, glu100, Glu10Gal10Man80, Glu10Gal45Man45, Glu10Gal80Man10, glu20ara80, Glu20Gal20Man20Xyl20Ara20, Glu20Gal20Man60, Glu20Gal40Man40, Glu20Gal60Man20, glu20gal80, | viscosity, sweetness, etc.) described herein) comprising glycans comprising a galactose glycan unit, optionally wherein the glycan preparation comprises any amount of galactose between 1% and 100%, further optionally wherein the glycan preparation comprises a second, third, fourth or fifth glycan unit (optionally, independently selected from xylose, arabinose, glucose, mannose, rhamnose, fructose, or fucose), further optionally, wherein the glycan preparation is one of: ara50gal50, gal100, gal20ara80, gal20xyl80, Gal25Man25Xyl25Ara25, gal33man33ara33, gal33man33xyl33, Gal33Xyl33Ara33, gal40ara60, gal40man60, gal40xyl60, gal50glu25fru25, gal57fru43, gal57glu43, gal60ara40, gal60man40, gal60xyl40, gal75xyl25, |

| | | | |
|---|---|---|---|
| Man33Xyl33Ara33, man40xyl60, man60xyl40, man80xyl20, xyl100, xyl33glu33gal33, xyl60ara40, xyl75ara25, xyl75gal25, xyl75glu12gal12, or xyl80ara20. | man20ara80, Man33Xyl33Ara33, man40ara60, man60ara40, man80ara20, xyl60ara40, xyl75ara25, or xyl80ara20. | glu20xyl80, Glu25Gal25Man25Ara25, Glu25Gal25Man25Xyl25, Glu25Gal25Xyl25Ara25, Glu25Man25Xyl25Ara25, Glu30Gal30Man40, Glu30Gal40Man30, glu33gal33ara33, glu33gal33fuc33, glu33gal33man33, glu33gal33xyl33, Glu33Man33Ara33, Glu33Man33Xyl33, Glu33Xyl33Ara33, glu40ara60, Glu40Gal20Man40, Glu40Gal30Man30, Glu40Gal40Man20, glu40gal60, glu40xyl60, Glu45Gal10Man45, Glu45Gal45Man10, glu50gal50, Glu5Gal5Man90, Glu5Gal90Man5, glu60ara40, Glu60Gal20Man20, glu60gal40, glu60man40, glu60xyl40, glu66fru33, glu80ara20, Glu80Gal10Man10, glu80gal20, glu80man20, glu80man20, glu80xyl20, Glu90Gal5Man5, man52glu29gal19, man60glu40, man62glu38, man80glu20, | gal80ara20, gal80man20, gal80xyl20, Glu10Gal10Man80, Glu10Gal45Man45, Glu10Gal80Man10, Glu20Gal20Man20Xyl20Ara20, Glu20Gal20Man60, Glu20Gal40Man40, Glu20Gal60Man20, glu20gal80, Glu25Gal25Man25Ara25, Glu25Gal25Man25Xyl25, Glu25Gal25Xyl25Ara25, Glu30Gal30Man40, Glu30Gal40Man30, glu33gal33ara33, glu33gal33fuc33, glu33gal33man33, glu33gal33xyl33, Glu40Gal20Man40, Glu40Gal30Man30, Glu40Gal40Man20, glu40gal60, Glu45Gal10Man45, Glu45Gal45Man10, glu50gal50, Glu5Gal5Man90, Glu5Gal90Man5, Glu60Gal20Man20, glu60gal40, Glu80Gal10Man10, glu80gal20, Glu90Gal5Man5, man52glu29gal19, Man66gal33, Man75gal25, Man80gal20, xyl33glu33gal33, xyl75gal25, or |

|  |  |  | xyl33glu33gal33, or xyl75glu12gal12. | xyl75glu12gal12. |
|---|---|---|---|---|

## Table 21 (continued)

| 1 | 6 | 7 | 8 | 9 |
|---|---|---|---|---|
| **Bacterial taxa (spore-former)** | **Man-containing Glycan** | **Rha-containing Glycan** | **Fru-containing Glycan** | **Fuc-containing Glycan** |
| Blautia<br>Clostridiales<br>Clostridium<br>Eubacterium<br>Lachnospiraceae<br>Roseburia<br>Ruminococcus | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a mannose glycan unit, optionally wherein the glycan preparation comprises any amount of mannose between 1% and 100%, further optionally wherein the glycan preparation comprises a second, third, fourth or fifth glycan unit (optionally, independently selected from xylose, arabinose, glucose, galactose, rhamnose, fructose, or fucose), further optionally, wherein the glycan preparation is one of: Gal25Man25Xyl25Ara25, gal33man33ara33, gal33man33xyl33, gal40man60, gal60man40, gal80man20, Glu10Gal10Man80, Glu10Gal45Man45, Glu10Gal80Man10, Glu20Gal20Man20Xyl20Ara20, Glu20Gal20Man60, Glu20Gal40Man40, | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a rhamnose glycan unit, optionally wherein the glycan preparation comprises any amount of rhamnose between 1% and 100%, further optionally wherein the glycan preparation comprises a second, third, fourth or fifth glycan unit (optionally, independently selected from xylose, arabinose, glucose, galactose, mannose, fructose, or fucose), further optionally, wherein the glycan preparation is rha100. | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a fructose glycan unit, optionally wherein the glycan preparation comprises any amount of fructose between 1% and 100%, further optionally wherein the glycan preparation comprises a second, third, fourth or fifth glycan unit (optionally, independently selected from xylose, arabinose, | a glycan preparation (as described herein, e.g., having any DP, DB, alpha/beta-glycosidic bond ratio, number of glycosidic bonds, bond regiochemistry and bond stereochemistry, and other characteristics (e.g., solubility, fermentability, viscosity, sweetness, etc.) described herein) comprising glycans comprising a fucose glycan unit, optionally wherein the glycan preparation comprises any amount of fucose between 1% and 100%, further optionally wherein the glycan preparation comprises a second, third, fourth or fifth glycan unit (optionally, independently selected from xylose, arabinose, glucose, galactose, mannose, |

113

| | | | | |
|---|---|---|---|---|
| | Glu20Gal60Man20, Glu25Gal25Man25Ara25, Glu25Gal25Man25Xyl25, Glu25Man25Xyl25Ara25, Glu30Gal30Man40, Glu30Gal40Man30, glu33gal33man33, Glu33Man33Ara33, Glu33Man33Xyl33, Glu40Gal20Man40, Glu40Gal30Man30, Glu40Gal40Man20, Glu45Gal10Man45, Glu45Gal45Man10, Glu5Gal5Man90, Glu5Gal90Man5, Glu60Gal20Man20, glu60man40, Glu80Gal10Man10, glu80man20, glu80man20, Glu90Gal5Man5, man100, man20ara80, man20xyl80, Man33Xyl33Ara33, man40ara60, man40xyl60, man52glu29gal19, man60ara40, man60glu40, man60xyl40, man62glu38, Man66gal33, Man75gal25, man80ara20, Man80gal20, man80glu20, or man80xyl20. | | glucose, galactose, mannose, rhamnose, or fucose), further optionally, wherein the glycan preparation is one of: fru100, gal50glu25fru25, gal57fru43, or glu66fru33. | rhamnose, or fructose), further optionally, wherein the glycan preparation is one of: glu33gal33fuc33. |

Table 22: Summary of exemplary glycosidase enzymes and glycosidase enzyme molecules

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Bacteroides. GH43.19-1 (SEQ ID NO: 120) | Cluster_ 119 | BSIG_364 6 | None | 323 | 972 | Bacteroides_sp._ 1_1_6 | NA | 3.2.1.5 5 | Non-reducing end alpha-L-arabinofuranosida se | Arabinose |
| Bacteroides. GH43.0 (SEQ ID NO: 119) | Cluster_ 114 | BSIG_155 4 | None | 376 | 1131 | Bacteroides_sp._ 1_1_6 | NA | 3.2.1.5 5 | Non-reducing end alpha-L-arabinofuranosida se | Arabinose |
| Bifidobacterium.GH8.0-1 (SEQ ID NO: 41) | Cluster_ 109 | BIFPSEU DO_02650 | None | 379 | 1140 | Bifidobacterium_ pseudocatenulatum_ DSM_ 20438_=_JCM_ 1200_=_LMG_10505 | DSM.204 38 | 3.2.1.1 56 | Oligosaccharide reducing-end xylanase | Xylose |
| Bacteroides. GH8.0-3 (SEQ ID NO: 30) | Cluster_ 104 | BACINT_ 00927 | None | 419 | 1260 | Bacteroides_ intestinalis_DSM_ 17393 | DSM.179 39 | 3.2.1.1 56 | Oligosaccharide reducing-end xylanase | Xylose |
| Ruminococc us.GH1.0 (SEQ ID NO: 31) | Cluster_ 101 | RUM_101 20 | None | 444 | 1335 | Ruminococcus_ champanellensis_ 18P13_=_J CM_17042 | DSM.188 48 | 3.2.1.2 1 | Beta-glucosidase | Glucose |
| Bacteroides. GH51.0-1 (SEQ ID NO: 17) | Cluster_ 93 | BACOVA _01708 | None | 514 | 1545 | Bacteroides_ovatus_ ATCC_8483 | ATCC.84 83 | 3.2.1.5 5 | Non-reducing end alpha-L-arabinofuranosida se | Arabinose |

| GeneID | Cluster | Representative_Protein | Binding Domains | Protein_Length | Nucleotide_Length | Strains | StrainID | EC | Annotation | Monomer |
|---|---|---|---|---|---|---|---|---|---|---|
| Bifidobacter ium.GH51.0-10 (SEQ ID NO: 9) | Cluster_91 | BLIG_005 51 | None | 515 | 1548 | Bifidobacterium_ longum_subsp._ infantis_C CUG_ 52486; Bifidobacterium_ longum_subsp._ longum_A TCC_ 55813; Bifidobacterium_ longum_subsp._ longum_44 B | ATCC.55 813 | 3.2.1.5 5 | Non-reducing end alpha-L-arabinofuranosida se | Arabinose |
| Bifidobacter ium.GH51.0-3 (SEQ ID NO: 123) | Cluster_80 | BLLJ_044 5 | None | 566 | 1701 | Bifidobacterium_ longum_subsp._ longum_JC M_1217 | NA | 3.2.1.5 5 | Non-reducing end alpha-L-arabinofuranosida se | Arabinose |
| Bacteroides. GH110.0 (SEQ ID NO: 116) | Cluster_77 | BSIG_151 0 | None | 568 | 1707 | Bacteroides_sp._ 1_1_6 | NA | 3.2.1.2 2 | Alpha-galactosidase | Galactose |
| Bacteroides. GH43.12-8 (SEQ ID NO: 16) | Cluster_73 | BACOVA _03421 | None | 575 | 1728 | Bacteroides_ovatus_ ATCC_8483 | ATCC.84 83 | 3.2.1.5 5 | Non-reducing end alpha-L-arabinofuranosida se | Arabinose |
| Bacteroides. GH43.12-1 (SEQ ID NO: 15) | Cluster_65 | BACOVA _03425 | None | 600 | 1803 | Bacteroides_ovatus_ ATCC_8483 | ATCC.84 83 | 3.2.1.5 5 | Non-reducing end alpha-L-arabinofuranosida se | Arabinose |
| Ruminococc us.GH26.0-2 (SEQ ID NO: 33) | Cluster_64 | RUM_212 70 | CBM35 | 616 | 1851 | Ruminococcus_ champanellensis_ 18P13_=_J CM_17042 | DSM.188 48 | 3.2.1.7 8 | Mannan endo-1,4-beta-mannosidase | Mannose |
| Ruminococc us.GH5.8 (SEQ ID NO: 37) | Cluster_60 | RUM_216 50 | CBM23; CBM23 | 644 | 1935 | Ruminococcus_ champanellensis_ 18P13_=_J CM_17042 | DSM.188 48 | 3.2.1.7 8 | Mannan endo-1,4-beta-mannosidase | Mannose |

| GeneID | Cluster | Representative_Protein | Binding Domains | Protein_Length | Nucleotide_Length | Strains | StrainID | EC | Annotation | Monomer |
|---|---|---|---|---|---|---|---|---|---|---|
| Ruminococc us.GH31.0 (SEQ ID NO: 4) | Cluster_58 | RUMOBE_03919 | None | 663 | 1992 | Ruminococcus_obeum_ATCC_29174 | ATCC.29174 | 3.2.1.2 0 | Alpha-glucosidase | Glucose |
| Bifidobacter ium.GH42.0-2 (SEQ ID NO: 38) | Cluster_52 | BLIJ_2092 | None | 691 | 2076 | Bifidobacterium_longum_subsp._infantis_A TCC_15697_=_JCM_1222_=_DSM_20088 | DSM.20088 | 3.2.1.2 3 | Beta-galactosidase | Galactose |
| Ruminococcus.GH26.0-I (SEQ ID NO: 32) | Cluster_50 | RUM_152 70 | CBM35 | 716 | 2151 | Ruminococcus_champanellensis_18P13_=_J CM_17042 | DSM.18848 | 3.2.1.7 8 | Mannan endo-1,4-beta-mannosidase | Mannose |
| Bacteroides. GH31.0-13 (SEQ ID NO: 118) | Cluster_49 | HMPREF9007_03836 | None | 717 | 2154 | Bacteroides_sp._1_1_14 | NA | 3.2.1.2 0 | Alpha-glucosidase | Glucose |
| Ruminococc us.GH43.37 (SEQ ID NO: 35) | Cluster_47 | RUM_093 20 | CBM61 | 724 | 2175 | Ruminococcus_champanellensis_18P13_=_J CM_17042 | DSM.18848 | 3.2.1.9 9 | Arabinan endo-1,5-alpha-L-arabinosidase | Arabinose |
| Lactobacillu s.GH36.0-2 (SEQ ID NO: 6) | Cluster_46 | HMPREFO492_1819 | None | 732 | 2199 | Lactobacillus_acidophilus_ATCC_4796 | ATCC.4796 | 3.2.1.2 2 | Alpha-galactosidase | Galactose |
| Lactobacillu s.GH36.0-1 (SEQ ID NO:1) | Cluster_45 | HMPREF0531_12742 | None | 738 | 2217 | Lactobacillus_plantarum_subsp._plantarum_ ATCC_14917_=_JCM_1149_= | ATCC.14917 | 3.2.1.2 2 | Alpha-galactosidase | Galactose |
| Ruminococc us.GH43.16 (SEQ ID NO: 34) | Cluster_44 | RUM_140 20 | CBM6 | 742 | 2229 | Ruminococcus_champanellensis_18P13_=_J CM_17042 | DSM.18848 | 3.2.1.5 5 | Non-reducing end alpha-L-arabinofuranosida se | Arabinose |
| Ruminococc us.GH43.4 (SEQ ID NO: 36) | Cluster_38 | RUM_092 80 | CBM13 | 751 | 2256 | Ruminococcus_champanellensis_18P13_=_J CM_17042 | DSM.18848 | 3.2.1.9 9 | Arabinan endo-1,5-alpha-L-arabinosidase | Arabinose |

| GeneID | Cluster | Representative_Protein | Binding Domains | Protein_Length | Nucleotide_Length | Strains | StrainID | EC | Annotation | Monomer |
|---|---|---|---|---|---|---|---|---|---|---|
| Bacteroides. GH3.0-6 (SEQ ID NO: 117) | Cluster_33 | HMPREF1007_00160 | None | 774 | 2325 | Bacteroides_sp._4_1_36 | NA | 3.2.1.2 1 | Beta-glucosidase | Glucose |
| Bacteroides. GH92.0-2 (SEQ ID NO: 122) | Cluster_30 | BSIG_270 6 | None | 779 | 2340 | Bacteroides_sp._1_1_6 | NA | 3.2.1.2 4 | Alpha-mannosidase | Mannose |
| Bacteroides. GH3.0-1 (SEQ ID NO: 12) | Cluster_26 | BACOVA_02659 | None | 786 | 2361 | Bacteroides_ovatus_ATCC_8483 | ATCC.8483 | 3.2.1.2 1 | Beta-glucosidase | Glucose |
| Bacteroides. GH31.0-7 (SEQ ID NO: 13) | Cluster_23 | BACOVA_03422 | None | 814 | 2445 | Bacteroides_ovatus_ATCC_8483 | ATCC.8483 | 3.2.1.1 77 | Alpha-D-ayloside xylohydrolase | Xylose |
| Bacteroides. GH2.0-1 (SEQ ID NO: 11) | Cluster_17 | BACOVA_02645 | None | 851 | 2556 | Bacteroides_ovatus_ATCC_8483 | ATCC.8483 | 3.2.1.2 3 | Beta-galactosidase | Galactose |
| Bacteroides. GH92.0-1 (SEQ ID NO: 121) | Cluster_16 | 8 | None | BSIG_169 897 | 2694 | Bacteroides_sp._1_1_6 | NA | 3.2.1.2 4 | Alpha-mannosidase | Mannose |
| Bacteroides. GH31.0-1 (SEQ ID NO: 14) | Cluster_10 | BACOVA_02646 | None | 954 | 2865 | Bacteroides_ovatus_ATCC_8483 | ATCC.8483 | 3.2.1.1 77 | Alpha-D-ayloside xylohydrolase | Xylose |
| Bifidobacterium.GH2.0-5 (SEQ ID NO: 8) | Cluster_8 | BLIF_0659 | None | 1023 | 3072 | Bifidobacterium_longum_subsp._infantis_15 7F; Bifidobacterium_longum_subsp._infantis_C CUG 52486; | ATCC.55 813 | 3.2.1.2 3 | Beta-galactosidase | Galactose |
| | | | | | | Bifidobacterium_longum_subsp._longum_A TCC_55813 | | | | |
| Roseburia.G H13.41-2 (SEQ ID NO: 24) | Cluster_3 | ROSEINA 2194_0333 4 | CBM41 | 1314 | 3945 | Roseburia_inulinivorans_DSM_16841 | DSM.168 41 | 3.2.1.1 | Alpha-amylase | Glucose |
| Butyrivibrio. GH13.28 (SEQ ID NO: 124) | Cluster_2 | CIY_1220 0 | CBM26 | 1333 | 4002 | Butyrivibrio_fibrisolvens_16/4 | NA | 3.2.1.1 | Alpha-amylase | Glucose |

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Eubacterium .GH13.41 (SEQ ID NO: 28) | Cluster_ 1 | EUR_2110 0 | CBM26 | 1364 | 4095 | Eubacterium_rectale_ DSM_17629 | DSM.176 29 | 3.2.1.1 | Alpha-amylase | Glucose |
| Bacteroides. GH31.0-10 (SEQ ID NO: 111) | Cluster_ 39 | BSIG_016 3 | None | 748 | 2247 | Bacteroides_sp._ 1_1_6 | NA | 3.2.1.2 0 | Alpha-glucosidase | Glucose |
| Bacteroides. GH31.0-11 (SEQ ID NO: 112) | Cluster_ 40 | HMPREF9 007_01268 | None | 748 | 2247 | Bacteroides_sp._ 1_1_14 | NA | 3.2.1.2 0 | Alpha-glucosidase | Glucose |
| Bacteroides. GH3.0-7 (SEQ ID NO: 110) | Cluster_ 34 | HMPREF0 969_01391 | None | 774 | 2325 | Bacteroides_sp._D20 | NA | 3.2.1.2 1 | Beta-glucosidase | Glucose |
| Bacteroides. GH92.0-3 (SEQ ID NO: 113) | Cluster_ 31 | HMPREF9 007_01545 | None | 779 | 2340 | Bacteroides_sp._ 1_1_14 | NA | 3.2.1.2 4 | Alpha-mannosidase | Mannose |
| Bifidobacterium.GH2.0-4 (SEQ ID NO: 114) | 7 | BIL_12070 | None | Cluster_ 1023 | 3072 | Bifidobacterium_ longum_subsp._ longum_F8 | NA | 3.2.1.2 3 | Beta-galactosidase | Galactose |
| Bifidobacterium.GH2.0-6 (SEQ ID NO: 115) | Cluster_ 9 | HMPREF1 312_0994 | None | 1023 | 3072 | Bifidobacterium_ longum_subsp._ longum_44 B | NA | 3.2.1.2 3 | Beta-galactosidase | Galactose |
| Citrobacter. GH4.0-1 (SEQ ID NO: 109) | Cluster_ 100 | CSAG_044 86 | None | 450 | 1353 | Citrobacter_sp._30_2 | NA | 3.2.1.2 2 | Alpha-galactosidase | Galactose |
| Bacteroides. GH43.10-2 (SEQ ID NO: 102) | Cluster_ 95 | 8 | None | BSIG_179 514 | 1545 | Bacteroides_sp._ 1_1_6 | NA | 3.2.1.5 5 | Non-reducing end alpha-L-arabinofuranosida se | Arabinose |
| Bifidobacter ium.GH51.0 -11 (SEQ ID NO: 108) | Cluster_ 92 | HMPREF1 315_1254 | None | 515 | 1548 | Bifidobacterium_ longum_subsp._ longum_2-2B | NA | 3.2.1.5 5 | Non-reducing end alpha-L-arabinofuranosida se | Arabinose |

| GeneID | Cluster | Representative_Protein | Binding Domains | Protein _Length | Nucleotide_Length | Strains | StrainID | EC | Annotation | Monomer |
|---|---|---|---|---|---|---|---|---|---|---|
| Bifidobacter ium.GH51.0-9 (SEQ ID NO: 107) | Cluster_90 | HMPREF0 177 01569 | None | 515 | 1548 | Bifidobacterium_sp._12_1_47BFAA | NA | 3.2.1.5 5 | Non-reducing end alpha-L-arabinofuranosidase | Arabinose |
| Bifidobacter ium.GH51.0-8 (SEQ ID NO: 27) | Cluster_88 | BIFCAT_0 0349 | None | 518 | 1557 | Bifidobacterium_catenulatum_DSM_16992_=_JCM_1194_=_LMG_11043 | DSM.169 92 | 3.2.1.5 5 | Non-reducing end alpha-L-arabinofuranosidase | Arabinose |
| Bifidobacter ium.GH51.0-6 (SEQ ID NO: 106) | Cluster_83 | BLIG_001 59 | None | 566 | 1701 | Bifidobacterium_longum_subsp._infantis_C CUG 52486 | NA | 3.2.1.5 5 | Non-reducing end alpha-L-arabinofuranosidase | Arabinose |
| Bifidobacter ium.GH13.30 (SEQ ID NO: 104) | Cluster_68 | BIFADO_00731 | None | 590 | 1773 | Bifidobacterium_adolescentis_L2-32 | NA | 3.2.1.2 0 | Alpha-glucosidase | Glucose |
| Bacteroides. GH97.0 (SEQ ID NO: 103) | Cluster_59 | BSIG_522 9 | None | 662 | 1989 | Bacteroides_sp._1_1_6 | NA | 3.2.1.2 2 | Alpha-galactosidase | Galactose |
| Bifidobacterium.GH2.0-3 (SEQ ID NO: 105) | Cluster_6 | HMPREF0 177_00324 | None | 1023 | 3072 | Bifidobacterium_sp._12_1_47BFAA | NA | 3.2.1.2 3 | Beta-galactosidase | Galactose |
| Bacteroides. GH43.19-2 (SEQ ID NO: 101) | Cluster_120 | HMPREF9 007_03519 | None | 323 | 972 | Bacteroides_sp._1_1_14 | NA | 3.2.1.5 5 | Non-reducing end alpha-L-arabinofuranosidase | Arabinose |
| Bacteroides. GH76.0-4 (SEQ ID NO: 10) | Cluster_112 | BACOVA_03627 | None | 376 | 1131 | Bacteroides_ovatus_ATCC_8483 | ATCC.84 83 | 3.2.1.1 01 | Mannan endo-1,6-alpha-mannosidase | Mannose |
| Bacteroides. GH31.0-12 (SEQ ID NO: 100) | Cluster_48 | BSIG_137 5 | None | 717 | 2154 | Bacteroides_sp._1_1_6 | NA | 3.2.1.2 0 | Alpha-glucosidase | Glucose |
| Bacteroides. GH3.0-8 (SEQ ID NO: 99) | Cluster_35 | HMPREF1 018_04051 | None | 764 | 2295 | Bacteroides_sp._2_1_56FAA | NA | 3.2.1.2 1 | Beta-glucosidase | Glucose |

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Citrobacter. GH4.0-2 (SEQ ID NO: 98) | Cluster_ 99 | HMPREF9 428_04500 | None | 450 | 1353 | Citrobacter_freundii_ 4_7_47CFAA | NA | 3.2.1.2 2 | Alpha-galactosidase | Galactose |
| Bifidobacter ium.GH51.0 -7 (SEQ ID NO: 97) | Cluster_ 84 | HMPREF1 312_0160 | None | 566 | 1701 | Bifidobacterium_ longum_subsp._ longum_44 B, Bifidobacterium_ longum_subsp._ longum_2-2B | NA | 3.2.1.5 5 | Non-reducing end alpha-L-arabinofuranosida se | Arabinose |
| Bifidobacter ium.GH42.0 -1 (SEQ ID NO: 39) | Cluster_ 51 | BIFBRE _0 3324 | None | 710 | 2133 | Bifidobacterium_ breve_DSM_ 20213_=_J C M_1192 | DSM.202 13 | 3.2.1.2 3 | Beta-galactosidase | Galactose |
| Bifidobacterium.GH8.0-2 (SEQ ID NO: 96) | Cluster_ 110 | BIFADO_ 00546 | None | 379 | 1140 | Bifidobacterium_ adolescentis_L2-32 | NA | 3.2.1.1 56 | Oligosaccharide reducing-end xylanase | Xylose |
| Bifidobacter ium.GH51.0 -1 (SEQ ID NO: 7) | Cluster_ 102 | HMPREF0 175_0767 | None | 420 | 1263 | Bifidobacterium_ longum_subsp._ longum_A TCC_55813 | ATCC.55 813 | 3.2.1.5 5 | Non-reducing end alpha-L-arabinofuranosida se | Arabinose |
| Bifidobacter ium.GH51.0 -2 (SEQ ID NO: 95) | Cluster_ 79 | HMPREF0 177_00949 | None | 566 | 1701 | Bifidobacterium_sp._ 12_1_47BFAA | NA | 3.2.1.5 5 | Non-reducing end alpha-L-arabinofuranosida se | Arabinose |
| Bifidobacter ium.GH2.0-2 (SEQ ID NO: 94) | Cluster_ 4 | BBNG_00 071 | None | 1291 | 3876 | Bifidobacterium_ bifidum_NCIMB_ 41171 | NA | 3.2.1.2 3 | Beta-galactosidase | Galactose |
| Bacteroides. GH51.0-3 (SEQ ID NO: 91) | Cluster_ 96 | CW1_4775 | None | 492 | 1477 | Bacteroides_ xylanisolvens_SD_ CC_2a | NA | 3.2.1.5 5 | Non-reducing end alpha-L-arabinofuranosida se | Arabinose |

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Bacteroides. GH43.4 (SEQ ID NO: 90) | Cluster_ 62 | HMPREF9 007_01284 | None | 625 | 1878 | Bacteroides_sp._ 1_1_14 | NA | 3.2.1.9 9 | Arabinan endo-1,5-alpha-L-arabinosidase | Arabinose |
| Lactobacillu s.GH2.0 (SEQ ID NO: 2) | Cluster_ 61 | HMPREF0 511_0110 | None | 636 | 1911 | Lactobacillus_ fermentum_ATCC_ 14931 | ATCC.14 931 | 3.2.1.2 3 | Beta-galactosidase | Galactose |
| Klebsiella.G H42.0-1 (SEQ ID NO: 92) | Cluster_ 53 | HMPREF1 307_04157 | None | 685 | 2058 | Klebsiella_ pneumoniae_subsp._ pneumoniae _WGLW3 | NA | 3.2.1.2 3 | Beta-galactosidase | Galactose |
| Klebsiella.G H42.0-5 (SEQ ID NO: 93) | Cluster_ 57 | HMPREF1 308_00556 | None | 685 | 2058 | Klebsiella_ pneumoniae_subsp._ pneumoniae _WGLW5 | NA | 3.2.1.2 3 | Beta-galactosidase | Galactose |
| Bifidobacter ium.GH51.0 -5 (SEQ ID NO: 89) | Cluster_ 82 | BLIF_0462 | None | 566 | 1701 | Bifidobacterium_ longum_subsp._ infantis_15 7F | NA | 3.2.1.5 5 | Non-reducing end alpha-L-arabinofuranosida se | Arabinose |
| Bifidobacterium.GH8.0-3 (SEQ ID NO: 26) | Cluster_ 111 | BIFCAT_0 1564 | None | 379 | 1140 | Bifidobacterium_ catenulatum_DSM_ 16992_ =_JCM_ 1194_=_LMG_11043 | DSM.169 92 | 3.2.1.1 56 | Oligosaccharide reducing-end xylanase | Xylose |
| Bacteroides. GH76.0-1 (SEQ ID NO: 85) | Cluster_ 106 | CW1_1391 | None | 385 | 1158 | Bacteroides_ aylanisolvens SD CC 2a; Bacteroides_ xylanisolvens_SD_ CC_lb; Bacteroides_sp._D1; Bacteroides_sp._ 2_1_22; Bacteroides sp. 2_2_4 | NA | 3.2.1.1 01 | Mannan endo-1,6-alpha-mannosidase | Mannose |

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Bacteroides. GH76.0-2 (SEQ ID NO: 86) | Cluster_ 107 | HMPREF9 010_04159 | None | 385 | 1158 | Bacteroides_sp._ 3_1_23 | NA | 3.2.1.1 01 | Mannan endo-1,6-alpha-mannosidase | Mannose |
| Bifidobacter ium.GH43.1 0-2 (SEQ ID NO: 25) | Cluster_ 86 | BIFCAT_0 1563 | None | 529 | 1590 | Bifidobacterium_ catenulatum_DSM_ 16992_ =_JCM_ 1194_=_LMG_11043 | DSM.169 92 | 3.2.1.5 5 | Non-reducing end alpha-L-arabinofuranosida se | Arabinose |
| Bacteroides. GH92.0-4 (SEQ ID NO: 87) | Cluster_ 36 | HMPREF9 007_02477 | None | 756 | 2271 | Bacteroides_sp._ 1_1_14 | NA | 3.2.1.2 4 | Alpha-mannosidase | Mannose |
| Bifidobacterium.GH2.0-1 (SEQ ID NO: 88) | Cluster_ 0 | BBNG_00 396 | CBM32 | 1891 | 5676 | Bifidobacterium_ bifidum_NCIMB_ 41171 | NA | 3.2.1.2 3 | Beta-galactosidase | Galactose |
| Bifidobacter ium.GH43.1 0-1 (SEQ ID NO: 40) | Cluster_ 85 | BIFPSEU DO_02649 | None | 529 | 1590 | Bifidobacterium_ pseudocatenulatum_ DSM_ 20438_=_JCM_ 1200_=_LMG_10505 | DSM.204 38 | 3.2.1.5 5 | Non-reducing end alpha-L-arabinofuranosida se | Arabinose |
| Bifidobacter ium.GH51.0 -4 (SEQ ID NO: 82) | Cluster_ 81 | BIL_14000 | None | 566 | 1701 | Bifidobacterium_ longum_subsp._ longum_F8 | NA | 3.2.1.5 5 | Non-reducing end alpha-L-arabinofuranosida se | Arabinose |
| Bacteroides. GH43.12-11 (SEQ ID NO: 80) | Cluster_ 76 | CUY_0318 | None | 575 | 1728 | Bacteroides_ovatus_ SD_CMC_3f | NA | 3.2.1.5 5 | Non-reducing end alpha-L-arabinofuranosida se | Arabinose |
| Klebsiella.G H42.0-3 (SEQ ID NO: 83) | Cluster_ 55 | HMPREF1 024_01211 | None | 685 | 2058 | Klebsiella_sp._ 4_1_44FAA | NA | 3.2.1.2 3 | Beta-galactosidase | Galactose |
| Klebsiella.G H42.0-4 (SEQ ID NO: 84) | Cluster_ 56 | HMPREF9 538_04862 | None | 685 | 2058 | Klebsiella_sp._MS_ 92-3 | NA | 3.2.1.2 3 | Beta-galactosidase | Galactose |
| Bacteroides. GH92.0-6 (SEQ ID NO: 81) | Cluster_ 41 | BSIG_058 1 | None | 747 | 2244 | Bacteroides_sp._ 1_1_6 | NA | 3.2.1.2 4 | Alpha-mannosidase | Mannose |

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Bacteroides. GH2.0-2 (SEQ ID NO: 79) | Cluster_ 18 | HMPREF9 010_00347 | None | 851 | 2556 | Bacteroides_sp._ 3_1_23 | NA | 3.2.1.2 3 | Beta-galactosidase | Galactose |
| Bacteroides. GH76.0-3 (SEQ ID NO: 78) | Cluster_ 108 | CUY_0575 | None | 385 | 1158 | Bacteroides_ovatus_ 3_8_47FAA; Bacteroides_ovatus_ SD_CMC_3f | NA | 3.2.1.1 01 | Mannan endo-1,6-alpha-mannosidase | Mannose |
| Bacteroides. GH43.12-10 (SEQ ID NO: 77) | Cluster_ 75 | HMPREF9 010_03959 | None | 575 | 1728 | Bacteroides_sp._ 3_1_23 | NA | 3.2.1.5 5 | Non-reducing end alpha-L-arabinofuranosida se | Arabinose |
| Bacteroides. GH76.0-5 (SEQ ID NO: 76) | Cluster_ 113 | BSGG_178 0 | None | 376 | 1131 | Bacteroides_sp._D2 | NA | 3.2.1.1 01 | Mannan endo-1,6-alpha-mannosidase | Mannose |
| Bacteroides. GH31.0-6 (SEQ ID NO: 75) | Cluster_ 22 | HMPREF0 106_02097 | None | 814 | 2445 | Bacteroides_sp._D22 | NA | 3.2.1.1 77 | Alpha-D-xyloside xylohydrolase | Xylose |
| Bacteroides. GH43.12-7 (SEQ ID NO: 73) | Cluster_ 72 | CW1_1655 | None | 575 | 1728 | Bacteroides_ aylanisolvens SD_CC_ 2a; Bacteroides_ xylanisolvens_SD_ CC_1b; Bacteroides_ sp._D1; Bacteroides sp. 2_1_22 | NA | 3.2.1.5 5 | Non-reducing end alpha-L-arabinofuranosida se | Arabinose |
| Bacteroides. GH43.12-9 (SEQ ID NO: 74) | Cluster_ 74 | HMPREF1 017_02810 | None | 575 | 1728 | Bacteroides_ovatus_ 3_8_47FAA | NA | 3.2.1.5 5 | Non-reducing end alpha-L-arabinofuranosida se | Arabinose |
| Bacteroides. GH43.12-12 (SEQ ID NO: 71) | Cluster_ 78 | CUY_0324 | None | 568 | 1707 | Bacteroides_ovatus_ SD_CMC_3f | NA | 3.2.1.5 5 | Non-reducing end alpha-L-arabinofuranosida se | Arabinose |

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Lactobacillu s.GH42.0 (SEQ ID NO: 5) | Cluster_ 63 | HMPREF0 492_1842 | None | 621 | 1866 | Lactobacillus_ acidophilus_ATCC_ 4796 | ATCC.47 96 | 3.2.1.2 3 | Beta-galactosidase | Galactose |
| Bacteroides. GH3.0-5 (SEQ ID NO: 18) | Cluster_ 32 | BACUNI_ 00919 | None | 775 | 2328 | Bacteroides_ uniformis_ATCC_8492 | ATCC.84 92 | 3.2.1.2 1 | Beta-glucosidase | Glucose |
| Bacteroides. GH31.0-5 (SEQ ID NO: 69) | Cluster_ 21 | CW1_1654 | None | 814 | 2445 | Bacteroides_ aylanisolvens_SD_ CC_2a; Bacteroides_ xylanisolvens_SD_ CC_1b; Bacteroides_ sp._D1; Bacteroides sp. 2_1_22 | NA | 3.2.1.1 77 | Alpha-D-xyloside xylohydrolase | Xylose |
| Bacteroides. GH31.0-8 (SEQ ID NO: 70) | Cluster_ 24 | HMPREF0 127_02636 | None | 814 | 2445 | Bacteroides_sp._ 1_1_30 | NA | 3.2.1.1 77 | Alpha-D-xyloside xylohydrolase | Xylose |
| Lachnospira ceae.GH36.0 -1 (SEQ ID NO: 72) | Cluster_ 15 | HMPREF0 992_01719 | None | 935 | 2808 | Lachnospiraceae_ bacterium_6_1_63FAA | NA | 3.2.1.2 2 | Alpha- galactosidase | Galactose |
| Bacteroides. GH43.12-4 (SEQ ID NO: 67) | Cluster_ 69 | HMPREF0 127_02639 | None | 577 | 1734 | Bacteroides_sp._ 1_1_30 | NA | 3.2.1.5 5 | Non-reducing end alpha-L- arabinofuranosida se | Arabinose |
| Bacteroides. GH31.0-9 (SEQ ID NO: 66) | Cluster_ 25 | HMPREF1 017_02809 | None | 814 | 2445 | Bacteroides_ovatus_ 3_8_47FAA | NA | 3.2.1.1 77 | Alpha-D-xyloside xylohydrolase | Xylose |
| Bifidobacter ium.GH13.2 8 (SEQ ID NO: 68) | Cluster_ 5 | BIFADO_ 01864 | CBM13; CBM26; CBM25 | 1269 | 3810 | Bifidobacterium_ adolescentis_L2-32 | NA | 3.2.1.1 | Alpha-amylase | Glucose |
| Bacteroides. GH51.0-2 (SEQ ID NO: 65) | Cluster_ 94 | HMPREF1 017_04729 | None | 514 | 1545 | Bacteroides_ovatus_ 3_8_47FAA | NA | 3.2.1.5 5 | Non-reducing end alpha-L- arabinofuranosida se | Arabinose |

EP 4 295 840 A2

| GeneID | Cluster | Represent ative_Prot ein | Binding Domains | Protein _Lengt h | Nucleoti de_Leng th | Strains | StrainID | EC | Annotation | Monomer |
|---|---|---|---|---|---|---|---|---|---|---|
| Bacteroides. GH3.0-3 (SEQ ID NO: 64) | Cluster_ 28 | HMPREF9 010_00355 | None | 786 | 2361 | Bacteroides_sp._ 3_1_23 | NA | 3.2.1.2 1 | Beta-glucosidase | Glucose |
| Escherichia. GH42.0 (SEQ ID NO: 63) | Cluster_ 117 | HMPREF9 541_02123 | None | 341 | 1027 | Escherichia_coli_MS_ 116-1 | NA | 3.2.1.2 3 | Beta-galactosidase | Galactose |
| Streptococcu s.GH13.28-2 (SEQ ID NO: 21) | Cluster_ 123 | HMPREF0 819_0981 | None | 82 | 249 | Streptococcus_ equinus_ATCC_9812 | ATCC.98 12 | 3.2.1.1 | Alpha-amylase | Glucose |
| Bacteroides. GH43.12-5 (SEQ ID NO: 61) | Cluster_ 70 | HMPREF1 017_02805 | None | 577 | 1734 | Bacteroides_ovatus_ 3_8_47FAA | NA | 3.2.1.5 5 | Non-reducing end alpha-L-arabinofuranosida se | Arabinose |
| Bacteroides. GH43.12-6 (SEQ ID NO: 62) | Cluster_ 71 | BSCG_037 59 | None | 577 | 1734 | Bacteroides_sp._ 2_2_4 | NA | 3.2.1.5 5 | Non-reducing end alpha-L-arabinofuranosida se | Arabinose |
| Bacteroides. GH43.12-2 (SEQ ID NO: 60) | Cluster_ 66 | HMPREF0 102_00215 | None | 595 | 1788 | Bacteroides_ aylanisolvens SD_CC_ 2a; Bacteroides_ xylanisolvens_SD_ CC_1b; Bacteroides_ sp._D1; Bacteroides sp. 2_1_22 | NA | 3.2.1.5 5 | Non-reducing end alpha-L-arabinofuranosida se | Arabinose |
| Bacteroides. GH76.0-7 (SEQ ID NO: 59) | Cluster_ 89 | HMPREF9 007_03654 | None | 516 | 1551 | Bacteroides_sp._ 1_1_14 | NA | 3.2.1.1 01 | Mannan endo-1,6-alpha-mannosidase | Mannose |
| Bacteroides. GH76.0-6 (SEQ ID NO: 58) | Cluster_ 87 | BSIG_378 5 | None | 525 | 1578 | Bacteroides_sp._ 1_1_6 | NA | 3.2.1.1 01 | Mannan endo-1,6-alpha-mannosidase | Mannose |
| Lachnospira ceae.GH36.0 -2 (SEQ ID NO: 57) | Cluster_ 42 | HMPREF0 991_02357 | None | 743 | 2232 | Lachnospiraceae_ bacterium_2_1_58FAA | NA | 3.2.1.2 2 | Alpha-galactosidase | Galactose |

EP 4 295 840 A2

| GeneID | Cluster | Representative_Protein | Binding Domains | Protein_Length | Nucleotide_Length | Strains | StrainID | EC | Annotation | Monomer |
|---|---|---|---|---|---|---|---|---|---|---|
| Bacteroides. GH3.0-2 (SEQ ID NO: 56) | Cluster_27 | HMPREF1017_00258 | None | 786 | 2361 | Bacteroides_ovatus_3_8_47FAA | NA | 3.2.1.2 1 | Beta-glucosidase | Glucose |
| Bifidobacter ium.GH26.0-2 (SEQ ID NO: 55) | Cluster_98 | BIFADO_02125 | CBM23 | 469 | 1410 | Bifidobacterium_adolescentis_L2-32 | NA | 3.2.1.7 8 | Mannan endo-1,4-beta-mannosidase | Mannose |
| Bacteroides. GH43.12-3 (SEQ ID NO: 54) | Cluster_67 | HMPREF9010_03964 | None | 595 | 1788 | Bacteroides_sp._3_1_23 | NA | 3.2.1.5 5 | Non-reducing end alpha-L-arabinofuranosidase | Arabinose |
| Bacteroides. GH8.0-2 (SEQ ID NO: 22) | Cluster_103 | BACCELL_02261 | None | 419 | 1260 | Bacteroides_cellulosilyticus_DSM_14838 | DSM.14838 | 3.2.1.1 56 | Oligosaccharide reducing-end xylanase | Xylose |
| Ruminococc us.GH36.0 (SEQ ID NO: 3) | Cluster_43 | RUMGNA_03611 | None | 743 | 2232 | Ruminococcus_gnavus_ATCC_29149 | ATCC.29149 | 3.2.1.2 2 | Alpha-galactosidase | Galactose |
| Bacteroides. GH31.0-2 (SEQ ID NO: 53) | Cluster_12 | HMPREF1017_00249 | None | 952 | 2859 | Bacteroides_ovatus_3_8_47FAA | NA | 3.2.1.1 77 | Alpha-D-xyloside xylohydrolase | Xylose |
| Paenibacillu s.GH5.8 (SEQ ID NO: 52) | Cluster_118 | HMPREF9412_0760 | None | 326 | 981 | Paenibacillus_sp._HGF5 | NA | 3.2.1.7 8 | Mannan endo-1,4-beta-mannosidase | Mannose |
| Bifidobacter ium.GH26.0-1 (SEQ ID NO: 51) | Cluster_121 | BIFADO_02124 | None | 283 | 852 | Bifidobacterium_adolescentis_L2-32 | NA | 3.2.1.7 8 | Mannan endo-1,4-beta-mannosidase | Mannose |
| Bacteroides. GH31.0-3 (SEQ ID NO: 50) | Cluster_11 | HMPREF9010_00348 | None | 954 | 2865 | Bacteroides_sp._3_1_23 | NA | 3.2.1.1 77 | Alpha-D-xyloside xylohydrolase | Xylose |
| Blautia.GH3 6.0 (SEQ ID NO: 42) | Cluster_14 | BLAHAN_04451 | None | 935 | 2808 | Blautia_hansenii_DSM_20583 | DSM.20583 | 3.2.1.2 2 | Alpha-galactosidase | Galactose |
| Streptococcu s.GH13.5 (SEQ ID NO: 20) | Cluster_97 | HMPREF0819_0402 | None | 485 | 1458 | Streptococcus_equinus_ATCC_9812 | ATCC.9812 | 3.2.1.1 | Alpha-amylase | Glucose |
| Klebsiella.G H42.0-2 (SEQ ID NO: 49) | Cluster_54 | HMPREF0485_01912 | None | 685 | 2058 | Klebsiella_sp._1_1_55 | NA | 3.2.1.2 3 | Beta-galactosidase | Galactose |

127

EP 4 295 840 A2

(continued)

| GeneID | Cluster | Representative_Protein | Binding Domains | Protein_Length | Nucleotide_Length | Strains | StrainID | EC | Annotation | Monomer |
|---|---|---|---|---|---|---|---|---|---|---|
| Streptococcus.GH13.28-1 (SEQ ID NO: 19) | Cluster_122 | HMPREF0819_0979 | CBM26 | 111 | 336 | Streptococcus_equinus_ATCC_9812 | ATCC.9812 | 3.2.1.1 | Alpha-amylase | Glucose |
| Bacteroides. GH3.0-4 (SEQ ID NO: 48) | Cluster_29 | BSGG_2666 | None | 785 | 2358 | Bacteroides_sp._D2 | NA | 3.2.1.21 | Beta-glucosidase | Glucose |
| Bacteroides. GH31.0-4 (SEQ ID NO: 47) | Cluster_13 | BSGG_2676 | None | 952 | 2859 | Bacteroides_sp._D2 | NA | 3.2.1.177 | Alpha-D-xyloside xylohydrolase | Xylose |
| Roseburia.G H13.41-1 (SEQ ID NO: 23) | Cluster_116 | ROSEINA2194_03333 | CBM26 | 349 | 1050 | Roseburia_inulinivorans_DSM_16841 | DSM.16841 | 3.2.1.1 | Alpha-amylase | Glucose |
| Bacteroides. GH43.10-1 (SEQ ID NO: 46) | Cluster_115 | CW1_1658 | None | 358 | 1077 | Bacteroides_xylanisolvens_SD_CC_2a | NA | 3.2.1.55 | Non-reducing end alpha-L-arabinofuranosidase | Arabinose |
| Bacteroides. GH8.0 (SEQ ID NO: 45) | Cluster_105 | HMPREF9447_02675 | None | 419 | 1260 | Bacteroides_oleiciplenus_YIT_12058 | NA | 3.2.1.156 | Oligosaccharide reducing-end xylanase | Xylose |
| Bacteroides. GH2.0-4 (SEQ ID NO: 44) | Cluster_20 | BSGG_2677 | None | 840 | 2523 | Bacteroides_sp._D2 | NA | 3.2.1.23 | Beta-galactosidase | Galactose |
| Bacteroides. GH92.0-5 (SEQ ID NO: 29) | Cluster_37 | BACFIN_06815 | None | 754 | 2265 | Bacteroides_finegoldii_DSM_17565 | DSM.17939 | 3.2.1.24 | Alpha-mannosidase | Mannose |
| Bacteroides. GH2.0-3 (SEQ ID NO: 43) | Cluster_19 | HMPREF1017_00248 | None | 840 | 2523 | Bacteroides_ovatus_3_8_47FAA | NA | 3.2.1.23 | Beta-galactosidase | Galactose |

**NUMBERED EMBODIMENTS**

1. A method of treating a subject having a disease or disorder associated with an unwanted level of a metabolite (e.g., a short chain fatty acid (SCFA) (e.g., propionate or butyrate), ammonia, trimethylamine (TMA), trimethylamine N-oxide (TMAO), a uremic solute (e.g., p-cresol or indole), lipopolysaccharide (LPS), or a bile acid (e.g., a secondary bile acid)), comprising:

optionally, selecting a glycan polymer preparation on the basis that it modulates the production or level of the metabolite, and
administering an amount of the glycan polymer preparation effective to result in a modulation of the level of the metabolite, thereby treating the disease or disorder.

2. A method of treating a subject having a disease or disorder associated with an unwanted level of a metabolite (e.g., a short chain fatty acid (SCFA) (e.g., propionate or butyrate), ammonia, trimethylamine (TMA), trimethylamine N-oxide (TMAO), a uremic solute (e.g., p-cresol or indole), lipopolysaccharide (LPS), or a bile acid (e.g., a secondary bile acid)), comprising:

optionally, acquiring knowledge that a glycan polymer preparation modulates the production or level of the metabolite, and
administering an amount of the glycan polymer preparation effective to result in a modulation of the level of the metabolite, thereby treating the disease or disorder.

3. The method of either of paragraphs 1 or 2, wherein responsive to the basis or knowledge that the glycan polymer preparation modulates the production or level of the metabolite, administering the glycan polymer preparation.

3. The method of any of paragraphs 1-3, wherein the glycan polymers, or at least 20, 30, 40, 50, 60, 70, 80, 90, 95, or 99 % (by weight or number) of the glycan polymers, of the glycan polymer preparation have one or more (e.g. two, three, four, five, or six) of the properties listed in Table 1, optionally selected from:

a. glycan polymers compring a glucose, mannose, or galactose subunit, or a combination thereof and at least one alpha-glycosidic bond,
b. glycan polymers comprising a glucose, mannose, or galactose subunit, or a combination thereof and at least one beta-glycosidic bond,
c. glycan polymers comprising a xylose, arabinose, fucose or rhamnose subunit, or a combination thereof and at least one alpha-glycosidic bond,
d. glycan polymers comprising a xylose, arabinose, fucose or rhamnose subunit, or a combination thereof and at least one beta-glycosidic bond,
e. glycan polymers comprising a glucose or galactose subunit, or a combination thereof and at least one alpha-glycosidic bond, or
f. glycan polymers comprising a glucose or galactose subunit, or a combination thereof and at least one beta-glycosidic bond.

4. The method of any of paragraphs 1-3, wherein the glycan polymers and/or glycan polymer preparation comprise one, two, three, or more, e.g., all, of the following features:

i. the glycan polymers comprise glucose and at least one alpha-glycosidic bond, optionally, wherein the alpha-glycosidic bond is alpha-1,3 glycosidic bond, alpha-1,4 glycosidic bond, or a combination thereof, and further optionally, wherein the mean degree of polymerization (DP) of the preparation is between DP2-4, DP2-6, DP3-10, or between DP3-15;
ii. the glycan polymer preparation further comprises glycan polymers comprising at least one beta-glycosidic bond, optionally wherein the beta-glycosidic bond is beta-1,3 glycosidic bond, beta-1,4 glycosidic bond or a combination thereof;
iii. the glycan polymer preparation further comprises glycan polymers comprising galactose (e.g., a glu-gal preparation);
iv. the glycan polymer preparation further comprises glycan polymers comprising mannose (e.g., a glu-man preparation); and

v. the glycan polymer preparation further comprises glycan polymers comprising galactose and mannose (e.g., a glu-gal-man preparation).

5. The method of any of paragraphs 1-3, wherein the glycan polymers and/or glycan polymer preparation comprise one, two, three, or more, e.g., all, of the following features:

i. the glycan polymers comprise glucose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;
ii. the glycan polymer preparation further comprises glycan polymers comprising at least one alpha-glycosidic bond, optionally, wherein the alpha-glycosidic bond is alpha-1,3 glycosidic bond, alpha-1,4 glycosidic bond or a combination thereof;
iii. the glycan polymer preparation further comprises glycan polymers comprising galactose (e.g., a glu-gal preparation);
iv. the glycan polymer preparation further comprises glycan polymers comprising mannose (e.g., a glu-man preparation); and
v. the glycan polymer preparation further comprises glycan polymers comprising galactose and mannose (e.g., a glu-gal-man preparation).

6. The method of any of paragraphs 1-3, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;
ii. the glycan polymer preparation further comprises glycan polymers comprising at least one beta-glycosidic bond, optionally, wherein the beta-glycosidic bond is beta-1,3 glycosidic bond, beta-1,4 glycosidic bond or a combination thereof;
iii. the glycan polymer preparation further comprises glycan polymers comprising glucose (e.g., a gal-glu preparation);
iv. the glycan polymer preparation further comprises glycan polymers comprising mannose (e.g., a gal-man preparation); and
v. the glycan polymer preparation further comprises glycan polymers comprising glucose and mannose (e.g., a gal-man-glu preparation).

7. The method of any of paragraphs 1-3, wherein the glycan polymers and/or glycan polymer preparation comprise one, two, three, or more, e.g., all, of the following features:

i. the glycan polymers comprise galactose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;
ii. the glycan polymer preparation further comprises glycan polymers comprising at least one alpha-glycosidic bond, optionally wherein the alpha-glycosidic bond is alpha-1,3 glycosidic bond, alpha-1,4 glycosidic bond or a combination thereof;
iii. the glycan polymer preparation further comprises glycan polymers comprising glucose (e.g., a gal-glu preparation);
iv. the glycan polymer preparation further comprises glycan polymers comprising mannose (e.g., a gal-man preparation); and
v. the glycan polymer preparation further comprises glycan polymers comprising glucose and mannose (e.g., a gal-glu-man preparation).

8. The method of any of paragraphs 1-3, wherein the glycan polymers and/or glycan polymer preparation comprise one, two, three, or more, e.g., all, of the following features:

i. the glycan polymers comprise mannose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond, optionally, wherein the beta-glycosidic bond is beta-1,3 glycosidic bond, beta-1,4 glycosidic bond or a combination thereof;

iii. the glycan polymer preparation further comprises glycan polymers comprising galactose (e.g., a man-gal preparation);

iv. the glycan polymer preparation further comprises glycan polymers comprising glucose (e.g., a man-glu preparation); and

v. the glycan polymer preparation further comprises glycan polymers comprising galactose and glucose (e.g., a man-gal-glu preparation).

9. The method of any of paragraphs 1-3, wherein the glycan polymers and/or glycan polymer preparation comprise one, two, three, or more, e.g., all, of the following features:

i. the glycan polymers comprise mannose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one alpha-glycosidic bond, optionally wherein the alpha-glycosidic bond is alpha-1,3 glycosidic bond, alpha-1,4 glycosidic bond or a combination thereof;

iii. the glycan polymer preparation further comprises glycan polymers comprising galactose (e.g., a man-gal preparation);

iv. the glycan polymer preparation further comprises glycan polymers comprising glucose (e.g., a man-glu preparation); and

v. the glycan polymer preparation further comprises glycan polymers comprising galactose and glucose (e.g., a man-gal-glu preparation).

10. The method of any of paragraphs 1-3, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation further comprises glycan polymers comprising fucose (e.g., a gal-fuc preparation);

v. the glycan polymer preparation further comprises glycan polymers comprising mannose (e.g., a gal-man preparation); and

vi. the glycan polymer preparation further comprises glycan polymers comprising fucose and mannose (e.g., a gal-fuc-man preparation).

11. The method of any of paragraphs 1-3, wherein the glycan polymers and/or glycan polymer preparation comprise one, two, three, or more, e.g., all, of the following features:

i. the glycan polymers comprise galactose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation further comprises glycan polymers comprising fucose (e.g., a gal-fuc preparation);

v. the glycan polymer preparation further comprises glycan polymers comprising mannose (e.g., a gal-man preparation); and

vi. the glycan polymer preparation further comprises glycan polymers comprising fucose and mannose (e.g., a gal-fuc-man preparation).

12. The method of any of paragraphs 1-3, wherein the glycan polymers and/or glycan polymer preparation comprise one, two, three, or more, e.g., all, of the following features:

i. the glycan polymers comprise fucose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation further comprises glycan polymers comprising galactose (e.g., a fuc-gal preparation);

v. the glycan polymer preparation further comprises glycan polymers comprising mannose (e.g., a fuc-man preparation); and

vi. the glycan polymer preparation further comprises glycan polymers comprising galactose and mannose (e.g., a fuc-gal-man preparation).

13. The method of any of paragraphs 1-3, wherein the glycan polymers and/or glycan polymer preparation comprise one, two, three, or more, e.g., all, of the following features:

i. the glycan polymers comprise fucose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-1;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation further comprises glycan polymers comprising galactose (e.g., a fuc-gal preparation);

v. the glycan polymer preparation further comprises glycan polymers comprising mannose (e.g., a fuc-man preparation); and

vi. the glycan polymer preparation further comprises glycan polymers comprising galactose and mannose (e.g., a fuc-gal-man preparation).

14. The method of any of paragraphs 1-3, wherein the glycan polymers and/or glycan polymer preparation comprise one, two, three, or more, e.g., all, of the following features:

i. the glycan polymers comprise mannose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation further comprises glycan polymers comprising fucose (e.g., a man-fuc preparation);

v. the glycan polymer preparation further comprises glycan polymers comprising galactose (e.g., a man-gal preparation); and

vi. the glycan polymer preparation further comprises glycan polymers comprising galactose and fucose (e.g., a man-gal-fuc preparation).

15. The method of any of paragraphs 1-3, wherein the glycan polymers and/or glycan polymer preparation comprise one, two, three, or more, e.g., all, of the following features:

i. the glycan polymers comprise mannose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation further comprises glycan polymers comprising fucose (e.g., a man-fuc preparation);

v. the glycan polymer preparation further comprises glycan polymers comprising galactose (e.g., a man-gal preparation); and

vi. the glycan polymer preparation further comprises glycan polymers comprising galactose and fucose (e.g., a man-gal-fuc preparation).

16. The method of any of paragraphs 1-3, wherein the glycan polymers and/or glycan polymer preparation comprise one, two, three, or more, e.g., all, of the following features:

i. the glycan polymers comprise one of, two of, or three of glucose, xylose and arabinose, and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation comprises glycan polymers comprising glucose;

v. the glycan polymer preparation comprises glycan polymers comprising xylose; and

vi. the glycan polymer preparation comprises glycan polymers comprising arabinose.

17. The method of any of paragraphs 1-3, wherein the glycan polymers and/or glycan polymer preparation comprise one, two, three, or more, e.g., all, of the following features:

i. the glycan polymers comprise one of, two of, or three of glucose, xylose and arabinose, and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation comprises glycan polymers comprising glucose;

v. the glycan polymer preparation comprises glycan polymers comprising xylose; and

vi. the glycan polymer preparation comprises glycan polymers comprising arabinose.

18. The method of any of paragraphs 1-3, wherein the glycan polymers and/or glycan polymer preparation comprise one, two, three, or more, e.g., all, of the following features:

i. the glycan polymers comprise glucose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising galactose (e.g., a glu-gal preparation);

v. the glycan polymer preparation further comprises glycan polymers comprising arabinose (e.g., a glu-ara preparation);

vi. the glycan polymer preparation further comprises glycan polymers comprising xylose (e.g., a glu-xyl preparation); and

vii. the glycan polymer preparation further comprises glycan polymers comprising two or three of galactose, arabinose, and xylose.

19. The method of any of paragraphs 1-3, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising glucose (e.g., a gal-glu preparation);

v. the glycan polymer preparation further comprises glycan polymers comprising arabinose (e.g., a gal-ara preparation);

vi. the glycan polymer preparation further comprises glycan polymers comprising xylose (e.g., a gal-xyl preparation); and

vii. the glycan polymer preparation further comprises glycan polymers comprising two or three of glucose, arabinose, and xylose.

20. The method of any of paragraphs 1-3, wherein the glycan polymers and/or glycan polymer preparation comprise one, two, three, or more, e.g., all, of the following features:

i. the glycan polymers comprise one of or two of xylose and arabinose, and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation comprises glycan polymers comprising xylose; and

v. the glycan polymer preparation comprises glycan polymers comprising arabinose.

21. The method of any of paragraphs 1-3, wherein the glycan polymers and/or glycan polymer preparation comprise one, two, three, or more, e.g., all, of the following features:

i. the glycan polymers comprise arabinose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond;

iii. the glycan polymer preparation further comprises glycan polymers comprising galactose (e.g., an ara-gal preparation);

iv. the glycan polymer preparation further comprises glycan polymers comprising xylose (e.g., an ara-xyl preparation); and

v. the glycan polymer preparation further comprises glycan polymers comprising galactose and xylose (e.g., an ara-gal-xyl preparation).

22. The method of any of paragraphs 1-3, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond;

iii. the glycan polymer preparation further comprises glycan polymers comprising arabinose (e.g., a gal-ara preparation);

iv. the glycan polymer preparation further comprises glycan polymers comprising xylose (e.g., a gal-xyl preparation); and

v. the glycan polymer preparation further comprises glycan polymers comprising arabinose and xylose (e.g., a gal-ara-xyl preparation).

23. The method of any of paragraphs 1-3, wherein the glycan polymers and/or glycan polymer preparation comprise one, two, three, or more, e.g., all, of the following features:

i. the glycan polymers comprise xylose and at least one alpha-glycosidic bond, optionally, wherein the alpha-glycosidic bond is alpha-1,3 glycosidic bond, further optionally, wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond;

iii. the glycan polymer preparation further comprises glycan polymers comprising galactose (e.g., a xyl-gal preparation);

iv. the glycan polymer preparation further comprises glycan polymers comprising arabinose (e.g., a xyl-ara preparation); and

v. the glycan polymer preparation further comprises glycan polymers comprising galactose and arabinose (e.g., a xyl-ara-gal preparation).

24. The method of any of paragraphs 1-3, wherein the glycan polymers and/or glycan polymer preparation comprise one, two, or more, e.g., all, of the following features:

i. the glycan polymers comprise glucose and at least one alpha-glycosidic bond, optionally, wherein the alpha-glycosidic bond is alpha-1,3 glycosidic bond, further optionally, wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond; and

iii. the glycan polymer preparation further comprises glycan polymers comprising one of, two of, or three of arabinose, galactose or xylose.

25. The method of any of paragraphs 1-3, wherein the glycan polymers and/or glycan polymer preparation comprise one, two, three, or more, e.g., all, of the following features:

i. the glycan polymers comprise glucose and at least one alpha-glycosidic bond, optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,3 glycosidic bond, alpha-1,4 glycosidic bond, alpha-1,6 glycosidic bond, or a combination thereof;

iii. the glycan polymer preparation further comprises glycan polymers comprising at least one beta-glycosidic bond; and

iv. the glycan polymer preparation further comprises glycan polymers comprising one of, two of, three of, or four of galactose, mannose, arabinose, or sialic acid.

26. The method of any of paragraphs 1-3, wherein the glycan polymers and/or glycan polymer preparation comprise one, two, three, or more, e.g., all, of the following features:

i. the glycan polymers comprise glucose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;
ii. the glycan polymer preparation further comprises glycan polymers comprising at least one beta-glycosidic bond;
iii. the glycan polymer preparation further comprises glycan polymers comprising xylose (e.g., a glu-xyl preparation); and
iv. the glycan polymer preparation further comprises glycan polymers comprising one of, two of, or three of mannose, arabinose, or galactose.

27. The method of any of paragraphs 1-3, wherein the glycan polymers and/or glycan polymer preparation comprise one, two, three, or more, e.g., all, of the following features:

i. the glycan polymers comprise glucose and at least one beta-glycosidic bond, optionally wherein the mean degree of polymerization (DP) of the preparation is between DP2-4, DP2-6, DP3-10 or between DP3-15;
ii. the glycan polymer preparation further comprises glycan polymers comprising at least one alpha-glycosidic bond, optionally wherein the alpha-glycosidic bond is alpha-1,3 glycosidic bond;
iii. the glycan polymer preparation further comprises glycan polymers comprising at least one beta-glycosidic bond;
iv. the glycan polymer preparation further comprises glycan polymers comprising xylose (e.g., a glu-xyl preparation); and
v. the glycan polymer preparation further comprises glycan polymers comprising one of, two of, or three of mannose, arabinose, or galactose.

28. The method of any of paragraphs 1-3, wherein the glycan polymers and/or glycan polymer preparation comprise one, two, three, or more, e.g., all, of the following features:

i. the glycan polymers comprise xylose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;
ii. the glycan polymer preparation further comprises glycan polymers comprising at least one beta-glycosidic bond;
iii. the glycan polymer preparation further comprises glycan polymers comprising glucose (e.g., a xyl-glu preparation); and
iv. the glycan polymer preparation further comprises glycan polymers comprising one of, two of, or three of mannose, arabinose, or galactose.

29. The method of any of paragraphs 1-3, wherein the glycan polymers and/or glycan polymer preparation comprise one, two, three, or more, e.g., all, of the following features:

i. the glycan polymers comprise xylose and at least one beta-glycosidic bond, further optionally wherein the mean degree of polymerization (DP) of the preparation is between DP2-4, DP2-6, DP3-10 or between DP3-15;
ii. the glycan polymer preparation further comprises glycan polymers comprising at least one alpha-glycosidic bond, optionally wherein the alpha-glycosidic bond is alpha-1,3 glycosidic bond;
iii. the glycan polymer preparation further comprises glycan polymers comprising at least one beta-glycosidic bond;
iv. the glycan polymer preparation further comprises glycan polymers comprising glucose (e.g., a xyl-glu preparation); and
v. the glycan polymer preparation further comprises glycan polymers comprising one of, two of, or three of mannose, arabinose, or galactose.

30. The method of any of paragraphs 1-3, wherein the glycan polymers and/or glycan polymer preparation comprise one, two, three, or more, e.g., all, of the following features:

i. the glycan polymers comprise glucose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising xylose (e.g., a glu-xyl preparation);

v. the glycan polymer preparation further comprises glycan polymers comprising arabinose (e.g., a glu-ara preparation);

vi. the glycan polymer preparation further comprises glycan polymers comprising galactose (e.g., a glu-gal preparation); and

vii. the glycan polymer preparation further comprises glycan polymers comprising one of, two of, or three of xylose, arabinose, or galactose.

31. The method of any of paragraphs 1-3, wherein the glycan polymers and/or glycan polymer preparation comprise one, two, three, or more, e.g., all, of the following features:

i. the glycan polymers comprise xylose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising glucose (e.g., a xyl-glu preparation);

v. the glycan polymer preparation further comprises glycan polymers comprising arabinose (e.g., a xyl-ara preparation);

vi. the glycan polymer preparation further comprises glycan polymers comprising galactose (e.g., a xyl-gal preparation); and

vii. the glycan polymer preparation further comprises glycan polymers comprising one of, two of, or three of glucose, arabinose, or galactose.

32. The method of any of paragraphs 1-3, wherein the glycan polymers and/or glycan polymer preparation comprise one, two, three, or more, e.g., all, of the following features:

i. the glycan polymers comprise arabinose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising xylose (e.g., a ara-xyl preparation);

v. the glycan polymer preparation further comprises glycan polymers comprising glucose (e.g., a ara-glu preparation);

vi. the glycan polymer preparation further comprises glycan polymers comprising galactose (e.g., a ara-gal preparation); and

vii. the glycan polymer preparation further comprises glycan polymers comprising one of, two of, or three of xylose, glucose, or galactose.

33. The method of any of paragraphs 1-3, wherein the glycan polymers and/or glycan polymer preparation comprise one, two, three, or more, e.g., all, of the following features:

i. glycan polymers comprise galactose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising xylose (e.g., a gal-xyl preparation);

v. the glycan polymer preparation further comprises glycan polymers comprising arabinose (e.g., a gal-ara preparation);

vi. the glycan polymer preparation further comprises glycan polymers comprising glucose (e.g., a gal-glu preparation); and

vii. the glycan polymer preparation further comprises glycan polymers comprising one of, two of, or three of xylose, arabinose, or glucose.

34. The method of any of paragraphs 1-33, wherein the glycan polymers, or at least 20, 30, 40, 50, 60, 70, 80, 90, 95, or 99 % (by weight or number) of the glycan polymers, of the glycan polymers of the glycan polymer preparation is a substrate for a glycosidase enzyme.

35. The method of paragraph 34, wherein the glycosidase enzyme is present in a human gut microbe.

36. The method of paragraph 35, wherein the human gut microbe is a member of glycotaxa class 1, the *but* and/or *buk* gene-containing bacterial taxa.

37. The method of paragraph 35, wherein the human gut microbe is a member of glycotaxa class 2, *cutC* gene-negative bacterial taxa.

38. The method of paragraph 35, wherein the human gut microbe is a member of glycotaxa class 3, *urease* gene-negative bacterial taxa.

39. The method of paragraph 35, wherein the human gut microbe is a member of glycotaxa class 4, bacterial taxa that do not comprise one or more (e.g., not comprising one, two, three, four, or more (e.g., all)) propionate production associated enzymes chosen from propionate kinase, propionate CoA-transferase, propionate-CoA ligase, propionyl-CoA carboxylase, methylmalonyl-CoA carboxytransferase, (S)-methylmalonyl-CoA decarboxylase, methylmalonate-semialdehyde dehydrogenase, and propanal dehydrogenase (e.g., chosen from the enzymes corresponding to Enzyme Commission (EC) numbers 6.4.1.3, 2.1.3.1, 4.1.1.41, 1.2.1.27, 2.3.3.5, 1.2.1.87, 1.3.1.95, 1.3.8.7, 2.3.1.54, 2.3.1.168, 2.3.1.8, and 2.3.1.222)).

40. The method of paragraph 35, wherein the human gut microbe is a member of glycotaxa class 5, bacterial taxa that comprise one or more (e.g., comprising one, two, three, four, or more (e.g., all)) bile acid production (e.g., secondary bile acid production) associated enzymes chosen from 7alpha-hydroxysteroid dehydrogenase, 12alpha-hydroxysteroid dehydrogenase, 7beta-hydroxysteroid dehydrogenase (NADP+), 2beta-hydroxysteroid dehydrogenase, 3beta-hydroxycholanate 3-dehydrogenase (NAD+), 3alpha-hydroxycholanate dehydrogenase (NADP+), 3beta-hydroxycholanate 3-dehydrogenase (NADP+), 3alpha-hydroxy bile acid-CoA-ester 3-dehydrogenase, 3alpha-hydroxycholanate dehydrogenase (NAD+), bile acid CoA-transferase, bile-acid 7alpha-dehydratase, and bile acid CoA ligase (e.g., chosen from the enzymes corresponding to Enzyme Commission (EC) numbers 1.1.1.159, 1.1.1.176, 1.1.1.201, .1.1.238, 1.1.1.391, 1.1.392, 1.1.393, 1.1.395, 1.1.1.52, 2.8.3.25, 4.2.1.106, and 6.2.1.7).

41. The method of paragraph 35, wherein the human gut microbe is a member of glycotaxa class 6, bacterial taxa that do not comprise one or more (e.g., not comprising one, two, three, four, or more (e.g., all)) indole production associated enzymes chosen from tryptophanase (e.g., the enzymes corresponding to Enzyme Commission (EC) number 4.1.99.1).

42. The method of paragraph 35, wherein the human gut microbe is a member of glycotaxa class 7, bacterial taxa that do not comprise one or more (e.g., not comprising one or both) p-cresol production associated enzymes chosen from 4-hydroxyphenylacetate decarboxylase and aldehyde ferredoxin oxidoreductase (e.g., chosen from the enzymes corresponding to Enzyme Commission (EC) numbers 4.1.1.83, 2.6.1.-, 4.1.1.-, and 1.2.7.5).

43. The method of paragraphs 34, 35, or 36, wherein the glycan polymer is a substrate for a glycosidase enzyme selected from one or more of, e.g., two, three, four, or more of, GT5, GH94, GH13 subfamily 9, GH13 subfamily 39, GH13 subfamily 36, GH113, or GH112 CAZy family.

44. The method of paragraphs 34, 35, or 36, wherein the glycan polymer is a substrate for a glycosidase enzyme selected from one or more of, e.g., two, three, four, or more of, GT2, GT4, GT5, GT35, GT51, GH1, GH2, GH3, GH4, GH13, GH13 subfamily 9, GH13 subfamily 31, GH18, GH23, GH25, GH28, GH31, GH32, GH36, GH51, GH73, GH77, or GH94 CAZy family.

45. The method of paragraphs 34, 35, or 37, wherein the glycan polymer is a substrate for a glycosidase enzyme selected from one or more of, e.g., two, three, four, or more of, GT11, GT10, GH92, GH51, GH35, GH29, GH28, GH20, GH130, GH13 subfamily 8, or GH13 subfamily 14 CAZy family.

46. The method of paragraphs 34, 35, or 37, wherein the glycan polymer is a substrate for a glycosidase enzyme selected from one or more of, e.g., two, three, four, or more of, GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, or GH77 CAZy family.

47. The method of paragraphs 34, 35, or 38, wherein the glycan polymer is a substrate for a glycosidase enzyme selected from one or more of, e.g., two, three, four, or more of, GT3, GH97, GH43 subfamily 24, GH27, GH133, GH13 subfamily 8, or GH13 CAZy family.

48. The method of paragraphs 34, 35, or 38, wherein the glycan polymer is a substrate for a glycosidase enzyme selected from one or more of, e.g., two, three, four, or more of, GT2, GT4, GH2, GH23, GH3, GT51, GH1, GT8, GH92, GT9, GH73, GH31, GH20, GH28, GT35, GT28, GH18, GH13, GH97, GH25, GH36, GH4, GH105, GH32, GH78, GH29, GH0, GT25, GH51, GH77, GH88, or GH24 CAZy family.

49. The method of paragraphs 34, 35, or 39, wherein the glycan polymer is a substrate for a glycosidase enzyme selected from one or more of, e.g., two, three, four, or more of, GH13 subfamily 3, GH13 subfamily 30, GH30 subfamily 2, GH30 subfamily 5, GH43 subfamily 22, GH43 subfamily 8, or GH84 CAZy family.

50. The method of paragraphs 34, 35, or 39, wherein the glycan polymer is a substrate for a glycosidase enzyme selected from one or more of, e.g., two, three, four, or more of, GH3, GH106, GH105, GH2, GH20, GH28, GH76, GH97, or GH92 CAZy family.

51. The method of paragraphs 34, 35, or 40, wherein the glycan polymer is a substrate for a glycosidase enzyme selected from one or more of, e.g., two, three, four, or more of, GH13 subfamily 19, GH13 subfamily 21, GH23, GH33, GH37 or GH104 CAZy family.

52. The method of paragraphs 34, 35, or 40, wherein the glycan polymer is a substrate for a glycosidase enzyme selected from one or more of, e.g., two, three, four, or more of, GH23, GH24, or GH33 CAZy family.

53. The method of paragraphs 34, 35, or 41, wherein the glycan polymer is a substrate for a glycosidase enzyme selected from one or more of, e.g., two, three, four, or more of, GH13 subfamily 20, GH13 subfamily 31, GH13 subfamily 39, GH39, GH43 subfamily 11, GH5 subfamily 44, or GH94 CAZy family.

54. The method of paragraphs 34, 35, or 41, wherein the glycan polymer is a substrate for a glycosidase enzyme selected from one or more of, e.g., two, three, four, or more of, GH2, GH31, GH23, GH13, or GH24 CAZy family.

55. The method of paragraphs 34, 35, or 42, wherein the glycan polymer is a substrate for a glycosidase enzyme selected from one or more of, e.g., two, three, four, or more of, GH13 subfamily 3, GH13 subfamily 30, GH121, GH15, GH43 subfamily 27, GH43 subfamily 34, or GH43 subfamily 8 CAZy family.

56. The method of paragraphs 34, 35, or 42, wherein the glycan polymer is a substrate for a glycosidase enzyme selected from one or more of, e.g., two, three, four, or more of, GH92, GH97, GH76, GH28, GH20, GH105, GH2, GH50, GH3, or GH106 CAZy family.

57. The method of paragraph 1, wherein selecting a glycan polymer comprises selecting on the basis that it has the substrate specificity of any one of paragraphs 43, 44, 45, 46, 47, 48, 49, 50, 51, 52, 53, 54, 55, or 56.

58. The method of any one of paragraphs 1-57, wherein the metabolite is one of: a short chain fatty acid (SCFA) (e.g., butyrate and/or propionate), ammonia, trimethylamine (TMA), trimethylamine N-oxide (TMAO), a uremic solute (e.g., p-cresol or indole), or a bile acid (e.g., a secondary bile acid).

59. The method of paragraph 58, wherein the metabolite is a short-chain fatty acid (SCFA).

60. The method of paragraph 59, wherein the SCFA is acetate, butyrate, and/or propionate.

61. The method of any one of paragraphs 58, wherein the metabolite is TMA and/or TMAO.

62. The method of any one of paragraphs 58, wherein the metabolite is ammonia.

63. The method of any one of paragraphs 58, wherein the metabolite is a bile acid.

64. The method of any one of paragraphs 58, wherein the metabolite is a uremic solute, e.g., p-cresol.

65. The method of any one of paragraphs 58, wherein the metabolite is a uremic solute, e.g., indole.

66. The method of either of paragraphs 59 or 60, wherein the disease or disorder is diarrhea (e.g., drug toxicity-induced diarrhea, e.g., induced by treatment regimen comprising administering a tyrosine kinase inhibitor or a chemotherapeutic agent (e.g., a FOLFIRI regimen); or radiation-induced diarrhea and radiation-induced acute intestinal symptoms), optionally, wherein the SCFA is butyrate, and further optionally wherein the level of butyrate is increased (e.g., relative to a subject undergoing the same treatment but not having been administered a glycan polymer preparation or relative to the level in a subject prior to administration of the glycan polymer preparation).

67. The method of either of paragraphs 59 or 60, wherein the disease or disorder is selected from Crohn's disease, inflammatory bowel disease, irritable bowel disease, irritable bowel disease-constipation (IBS-C), or ulcerative colitis, and optionally wherein the SCFA is butyrate.

68. The method of either of paragraphs 59 or 60, wherein the disease or disorder is selected from non-alcoholic fatty liver disease (NAFLD) or non-alcoholic steatohepatitis (NASH), optionally wherein the SCFA is butyrate.

69. The method of either of paragraphs 59 or 60, wherein the disease or disorder is hepatic encephalopathy and, optionally, wherein the SCFA is butyrate.

70. The method of paragraph 61, wherein the disease or disorder is timethylaminuria (e.g., secondary trimethylaminuria).

71. The method of paragraph 61, wherein the disease or disorder is a chronic disease (e.g., chronic kidney disease or end stage renal disease).

72. The method of paragraph 61, wherein the disease or disorder is a chronic disease (e.g., chronic heart disease, chronic heart failure, chronic vascular disease).

73. The method of paragraph 61, wherein the disease or disorder is one of non-alcoholic fatty liver disease (NAFLD) or non-alcoholic steatohepatitis (NASH).

74. The method of paragraph 62, wherein the disease or disorder is chronic kidney disease.

75. The method of paragraph 62, wherein the disease or disorder is liver cirrhosis, optionally with minimal hepatic encephalopathy (MHE).

76. The method of paragraph 62, wherein the disease or disorder is hepatic encephalopathy.

77. The method of paragraph 62, wherein the disease or disorder is a urea cycle disorder.

78. The method of either of paragraphs 59 or 60, wherein the disease or disorder is propionic acidemia.

79. The method of paragraph 63, wherein the disease or disorder is selected from cirrhosis, alcoholic liver cirrhosis, primary biliary cirrhosis, or intestinal failure-associated liver disease.

80. The method of paragraph 63, wherein the disease or disorder is selected from Crohn's disease, inflammatory bowel disease, irritable bowel disease, irritable bowel disease-constipation (IBS-C), or ulcerative colitis.

81. The method of paragraph 63, wherein the disease or disorder is selected from non-alcoholic fatty liver disease (NAFLD) or non-alcoholic steatohepatitis (NASH).

82. The method of paragraph 65, wherein the disease or disorder is chronic kidney disease.

83. The method of paragraph 65, wherein the disease or disorder is hepatic encephalopathy.

84. The method of paragraph 65, wherein the disease or disorder is hepatic phenylketonuria.

85. The method of paragraph 64, wherein the disease or disorder is chronic kidney disease.

86. The method of paragraph 64, wherein the disease or disorder is hepatic encephalopathy.

87. The method of any one of paragraphs 66-86, wherein the metabolite level is increased in the subject or in a suitable sample from the subject having the disease or disorder, e.g., increased as compared to a reference, e.g., a predetermined reference value, the level in the subject prior to treatment, or a healthy control.

88. The method of any one of paragraphs 66-86, wherein the metabolite level is decreased in the subject or a suitable sample from the subject having the disease or disorder, e.g., decreased as compared to a reference, e.g., a predetermined reference value, the level in the subject prior to treatment, or a healthy control.

89. The method of any one of paragraphs 1-88 further comprising evaluating the level of the metabolite, or a symptom of an unwanted level of the metabolite, e.g., by acquiring a level of the metabolite, optionally prior to treating the subject (e.g., as a baseline), during the treatment (e.g., to monitor treatment success), and/or post-treatment (e.g., to assess recurrence of the disease or disorder).

90. The method of any of paragraphs 4-9, 36, 43, 44, 59, 60, 66-69, or 87, wherein the level (e.g., systemic level, e.g. blood or fecal levels) of butyrate is increased (e.g., the rate or level of butyrate production, e.g., by gastrointestinal microbes, is increased), e.g., relative to a subject not treated with the glycan polymer preparation.

91. The method of any of paragraphs 10-17, 36, 43, 44, 59, 60, 70, or 88, wherein the level (e.g., systemic level, e.g. blood or fecal levels) of TMA is decreased (e.g., the rate or level of conversion of choline to TMA, e.g., by gastrointestinal microbes, is reduced), e.g., relative to a subject not treated with the glycan polymer preparation.

92. The method of any of paragraphs 18-20, 37, 45, 46, 61, 70-73, or 88, wherein the level (e.g., systemic level, e.g. blood or fecal levels) of ammonia is decreased (e.g., the rate or level of conversion of urea to ammonia, e.g., by gastrointestinal microbes, is reduced), e.g., relative to a subject not treated with the glycan polymer preparation.

93. The method of any of paragraphs 21-24, 39, 49, 50, 59, 60, 78, or 88, wherein the level (e.g., systemic level, e.g. blood or fecal levels) of propionic acid is decreased (e.g., the rate or level of propionic acid production, e.g., by gastrointestinal microbes, is reduced), e.g., relative to a subject not treated with the glycan polymer preparation.

94. The method of any of paragraphs 25, 40, 51, 52, 63, 79-81, or 87, wherein the level (e.g., systemic level, e.g., gut or fecal levels) of secondary bile acid is increased (e.g., the rate or level of conversion of bile acids to secondary bile acids, e.g., by gastrointestinal microbes, is increased), e.g., relative to a subject not treated with the glycan polymer preparation.

95. The method of any of paragraphs 26-29, 41, 53, 54, 65, 82-84, or 88, wherein the level (e.g., systemic level, e.g., fecal level) of indole is decreased (e.g., the rate or level of indole production, e.g., by gastrointestinal microbes, is decreased), e.g., relative to a subject not treated with the glycan polymer preparation.

96. The method of any of paragraphs 30-33, 42, 55, 56, 64, 85, 86, or 88, wherein the level (e.g., systemic level) of p-cresol is decreased (e.g., the rate or level of tyrosine conversion to p-cresol, e.g., by gastrointestinal microbes, is decreased), e.g., relative to a subject not treated with the glycan polymer preparation.

97. The method of any one of paragraphs 1-96, further comprising selecting a subject for treatment on the basis of or responsive to acquiring knowledge of any one or more of:

a) the subject having an unwanted level of a metabolite (e.g., an unwanted level of a metabolite of any of paragraphs 58-65),
b) the subject having a disease or disorder (e.g. a disease or disorder of any one of paragraphs 66-86),
c) the subject having a dysbiosis of the gut microbiota (e.g. miscalibrated levels/relative abundance of, e.g., class 1, class 2, class 3, class 4, class 5, class 6, or class 7 bacterial taxa of any of paragraphs 36-42),
d) the subject having responded to a prior treatment with a glycan polymer (e.g. a glycan polymer of any of paragraphs 3-33),
e) the subject having undergone a therapy or other environment that results in a dysbiosis, e.g., antibiotic treatment, or gastric surgery prior to treating,

optionally comprising acquiring a suitable value to determine the selection criteria.

98. The method of paragraph 97, wherein the subject is selected for treatment on the basis of or responsive to acquiring knowledge of any two or more of (a) through (e).

99. The method of paragraph 97, wherein the subject is selected for treatment on the basis of or responsive to acquiring knowledge of any three or more of (a) through (e).

100. The method of paragraph 97, wherein the subject is selected for treatment on the basis of or responsive to acquiring knowledge of any four or more of (a) through (e).

101. The method of paragraph 97, wherein the subject is selected for treatment on the basis of or responsive to acquiring knowledge of all of (a) through (e).

102. The method of any of paragraphs 97-101, wherein a suitable value may be acquired by analyzing a suitable biological sample from the subject.

103. The method of paragraph 102, wherein the sample is blood, feces, urine, saliva, or an organ tissue sample.

104. The method of any one of paragraphs 1-103, wherein the unwanted level of the metabolite is modulated, e.g., decreased, (e.g. in the subject or in a suitable sample taken from the treated subject) by 3%, 5%, 10%, 15%, 20%, 25%, 30%, 35%, 40%, or 50% after a treatment period (e.g. when compared to a reference, e.g., a predetermined reference value, the level in the subject prior to treatment, or a healthy control).

105. The method of any one of paragraphs 1-104, wherein the unwanted level of the metabolite is increased (e.g. in a suitable sample taken from the treated subject) by 3%, 5%, 10%, 15%, 20%, 25%, 30%, 35%, 40%, or 50% after a treatment period (e.g. when compared to a reference, e.g., a predetermined reference value, the level in the subject prior to treatment, or a healthy control).

106. The method of any one of paragraphs 1-105, wherein the treating further comprises administering a second therapeutic agent (e.g. a therapeutic agent other than the glycan polymer for treating the disease or disorder and/or for modulating the level of the metabolite).

107. The method of any one of paragraphs 1-106, wherein the treating further comprises administering a preparation of a gut microbe (e.g., a human gut microbe).

108. The method of paragraph 107, wherein the gut microbe (e.g., a human gut microbe) is:

i. a class 1 (e.g., *but* and/or *buk* gene-containing bacterial taxa),
ii. a class 2 (e.g., *cutC* gene-negative bacterial taxa),
iii. a class 3 (e.g., *urease* gene-negative bacterial taxa),
iv. a class 4 (e.g., bacterial taxa lacking one or more propionate production associated enzymes chosen from propionate kinase, propionate CoA-transferase, propionate-CoA ligase, propionyl-CoA carboxylase, methylmalonyl-CoA carboxytransferase, (S)-methylmalonyl-CoA decarboxylase, methylmalonate-semialdehyde dehydrogenase, and propanal dehydrogenase (e.g., chosen from the enzymes corresponding to Enzyme Commission (EC) numbers 6.4.1.3, 2.1.3.1, 4.1.1.41, 1.2.1.27, 2.3.3.5, 1.2.1.87, 1.3.1.95, 1.3.8.7, 2.3.1.54, 2.3.1.168, 2.3.1.8, and 2.3.1.222)),

v. a class 5 (e.g., bacterial taxa comprising one or more bile acid production associated enzymes chosen from 7alpha-hydroxysteroid dehydrogenase, 12alpha-hydroxysteroid dehydrogenase, 7beta-hydroxysteroid dehydrogenase (NADP+), 2beta-hydroxysteroid dehydrogenase, 3beta-hydroxycholanate 3-dehydrogenase (NAD+), 3alpha-hydroxycholanate dehydrogenase (NADP+), 3beta-hydroxycholanate 3-dehydrogenase (NADP+), 3alpha-hydroxy bile acid-CoA-ester 3-dehydrogenase, 3alpha-hydroxycholanate dehydrogenase (NAD+), bile acid CoA-transferase, bile-acid 7alpha-dehydratase, and bile acid CoA ligase (e.g., chosen from the enzymes corresponding to Enzyme Commission (EC) numbers 1.1.1.159, 1.1.1.176, 1.1.1.201, .1.1.238, 1.1.1.391, 1.1.392, 1.1.393, 1.1.395, 1.1.1.52, 2.8.3.25, 4.2.1.106, and 6.2.1.7)),

vi. a class 6 (e.g., bacterial taxa lacking one or more indole production associated enzymes chosen from tryptophanase (e.g., the enzymes corresponding to Enzyme Commission (EC) number 4.1.99.1)), or

vii. a class 7 (e.g., bacterial taxa lacking one or more p-cresol production associated enzymes chosen from 4-hydroxyphenylacetate decarboxylase and aldehyde ferredoxin oxidoreductase (e.g., chosen from the enzymes corresponding to Enzyme Commission (EC) numbers 4.1.1.83, 2.6.1.-, 4.1.1.-, and 1.2.7.5))

bacterial taxa.

109. The method of paragraph 108, wherein the gut microbe is selected on the basis of its association with the metabolite (e.g., on the basis of its positive, negative, or lack of correlation with the metabolite).

110. The method of paragraph 109, wherein the selection of the gut microbe comprises choosing a gut microbe from Table 3 based on the gut microbe's association with the metabolite (e.g., on the basis of its positive, negative, or lack of correlation with the metabolite).

111. The method of any of paragraphs 107-110, wherein the glycan polymer is a substrate of the gut microbe (e.g., a human gut microbe).

112. The method of any one of paragraphs 1-111, wherein the glycan polymer is a substrate of a gut microbial glycosidase enzyme and promotes the growth of the gut microbe.

113. The method of any one of paragraphs 1-112, wherein the glycan preparation is administered daily.

114. The method of any one of paragraphs 1-113, wherein the glycan preparation is administered for a single treatment period.

115. The method of any of paragraphs 1-113, wherein the glycan preparation is administered for more than one treatment period, e.g., wherein an inter-treatment period is longer than one or both of the adjacent treatment periods or wherein an inter-treatment period is shorter than one or both of the adjacent treatment periods.

116. The method of any of paragraphs 1-115, wherein the glycan polymer is a substrate for a microbial constituent of the colon or intestine.

117. The method of any of paragraphs 1-116, wherein the glycan polymer preparation is administered orally or rectally.

118. A method of modulating the production or level of a product (e.g., a short chain fatty acid (SCFA), ammonia, trimethylamine (TMA), trimethylamine N-oxide (TMAO), a uremic solute, or a bile acid) in the body (e.g., the gut (colon, intestine), blood, urine, an organ (e.g. liver, kidney), the brain) of a subject comprising: administering (e.g. orally or rectally) an effective amount of a glycan polymer preparation to the subject sufficient to modulate the production or level of a product, optionally, wherein the glycan polymer is a substrate for a microbial constituent of the colon or intestine.

119. The method of paragraph 118, wherein the microbial constituent:

a) produces the product, e.g., thereby increasing the level or production of the product,

b) produces a pre-cursor or alternate product that is converted to the product by a producer taxa, e.g., thereby increasing the level or production of the product,

c) does not produce the product but competes with or antagonizes a producer taxa of the product (e.g. competes for space and/or nutrients or produces anti-microbial substances toxic for the producing taxa), e.g. thereby reducing the relative abundance of the producer taxa and decreasing the level or production of the product.

120. The method of paragraph 119, wherein the microbial constituent is selected from a constituent from Table 2.

121. The method of paragraph 119, wherein the microbial constituent is selected from a strain from Table 3.

122. The method of paragraph 119, wherein the microbial constituent is selected from a constituent comprising a glycosidase enzyme from a glycosidase family of Table 4.

123. The method of paragraph 119, wherein the microbial constituent is selected from a constituent comprising a glycosidase enzyme from a glycosidase family recited in any of paragraphs 43-55.

124. The method of either of paragraphs 119 or 121, wherein the product is selected from a metabolite of Table 3.

125. The method of paragraph 119, wherein the product is SCFA, and the subject has a condition selected from the SCFA row of Table 5.

126. The method of paragraph 119, wherein the product is ammonia, and the subject has a condition selected from the ammonia row of Table 5.

127. The method of paragraph 119, wherein the product is TMA, and the subject has a condition selected from the TMA row of Table 5.

128. The method of paragraph 119, wherein the product is bile acid, and the subject has a condition selected from the bile acid row of Table 5.

129. The method of paragraph 119, wherein the product is a uremic solute (e.g., p-cresol or indole), and the subject has a condition selected from the p-cresol or indole row of Table 5.

130. The method of paragraphs 118 or 119, further comprising acquiring the identity of a microbe (e.g. a bacterial taxa) that modulates, e.g., produces, the product.

131. The method of any one of paragraphs 118-130, further comprising selecting the glycan preparation on the basis of its ability to modulate the microbial constituent.

132. The method of any one of paragraphs 118-130, wherein the glycan preparation is a substrate of a glycosidase enzyme of the microbial constituent, e.g., wherein the microbial constitudent and the product are from the same row of Table 3.

133. The method of any of paragraphs 1-132, wherein the subject is a human, e.g., a human patient.

134. A glycan polymer preparation, e.g., described herein, for use in a method described in any of paragraphs 1-133.

135. A method of selecting a glycan polymer preparation for use as a substrate for a glycosidase enzyme (e.g. CAZy family) of a preselected human gut microbe (e.g. selected because of its glycosidase profile), comprising:

a) acquiring a value for the glycosidase (e.g. CAZy family) profile of a microbe,
b) identifying, designing, or selecting a glycan polymer capable of being a substrate of the microbe on the basis of the glycosidase (e.g. CAZy family) profile,
c) optionally,

i. assembling a panel of human gut microbes (e.g. single strains, designed communities of strains, or ex vivo communities, e.g. from fecal samples, which include the microbe of interest)
ii. contacting the panel of microbes with a test glycan preparation,

iii. assessing the growth of the human gut microbe (of interest)

d) selecting the glycan polymer preparation.

136. The method of paragraph 135, wherein (a) comprises finding the value for the glycosidase (e.g., CAZy family) profile in Table 4.

137. The method of paragraph 135, wherein (b) comprises identifying, designing, or selecting a glycan polymer found in Table 4.

138. The method of paragraph 135, wherein (a) comprises finding the value for the glycosidase (e.g., CAZy family) profile in Table 4, and wherein (b) comprises identifying, designing, or selecting a glycan polymer found in Table 4 that is in the same row, e.g., is a substrate of, a glycosidase of the glycosidase profile (e.g., CAZy family) of (a).

139. A glycan preparation made or selected by the method of any of paragraphs 135-138.

140. A glycan polymer preparation comprising glycan polymers, e.g., wherein the preparation comprises at least .5, 1, 2, 5, 10, 50, or 100 kg, and, e.g., is at least 20, 30, 40, 50, 60, 70, 80, 90, 95 or 99 % pure, comprising:

    i) a glucose, mannose, or galactose subunit, or a combination thereof and at least one alpha-glycosidic bond, or
    ii) a glucose, mannose, or galactose subunit, or a combination thereof and at least one beta-glycosidic bond, and

which are a substrate of one or more, e.g., two, three, four, or more, human gut microbe glycosidase enzymes selected from:

    i) GT5, GH94, GH13 subfamily 9, GH13 subfamily 39, GH13 subfamily 36, GH113 or GH112 CAZy family,
    ii) GT2, GT4, GT5, GT35, GT51, GH1, GH2, GH3, GH4, GH13, GH13 subfamily 9, GH13 subfamily 31, GH18, GH23, GH25, GH28, GH31, GH32, GH36, GH51, GH73, GH77, or GH94 CAZy family,
    iii) GT11, GT10, GH92, GH51, GH35, GH29, GH28, GH20, GH130, GH13 subfamily 8, or GH13 subfamily 14 CAZy family, or
    iv) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, or GH77 CAZy family.

141. A glycan polymer preparation, e.g., wherein the preparation comprises at least about 0.5, 1, 2, 5, 10, 50, or 100 kg, and, e.g., is at least 20, 30, 40, 50, 60, 70, 80, 90, 95 or 99 % pure, comprising glycan polymers comprising:

    i) a xylose, arabinose, fucose or rhamnose subunit, or a combination thereof and at least one alpha-glycosidic bond, or
    ii) a xylose, arabinose, fucose or rhamnose subunit, or a combination thereof and at least one beta-glycosidic bond, and

which are a substrate of one or more, e.g., two, three, four, or more, human gut microbe glycosidase enzymes selected from:

    i) GT11, GT10, GH92, GH51, GH35, GH29, GH28, GH20, GH130, GH13 subfamily 8, or GH13 subfamily 14 CAZy family, or
    ii) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, or GH77 CAZy family.

142. A glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

    i) a glucose or galactose subunit, or a combination thereof and at least one alpha-glycosidic bond, or
    ii) a glucose or galactose subunit, or a combination thereof and at least one beta-glycosidic bond, and

which are a substrate of one or more, e.g., two, three, four, or more, human gut microbe glycosidase enzymes selected from:

i) GT3, GH97, GH43 subfamily 24, GH27, GH133, GH13 subfamily 8, GH13 CAZy family, or
ii) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, GH24 CAZy family.

143. A glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

an arabinose, galactose, xylose, or glucose subunit, or a combination thereof and at least one alpha-glycosidic bond, and
which are a substrate of one or more, e.g., two, three, four, or more, human gut microbe glycosidase enzymes selected from:

i) GH13 subfamily 3, GH13 subfamily 30, GH30 subfamily 2, GH30 subfamily 5, GH43 subfamily 22, GH43 subfamily 8, or GH84 CAZy family, or
ii) GH3, GH106, GH105, GH2, GH20, GH28, GH76, GH97, or GH92 CAZy family.

144. A glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

a glucose and at least one alpha-glycosidic bond, and
which are a substrate of one or more, e.g., two, three, four, or more, human gut microbe glycosidase enzymes selected from:

i) GH13 subfamily 19, GH13 subfamily 21, GH23, GH33, GH37 or GH104 CAZy family, or
ii) GH23, GH24, or GH33 CAZy family.

145. A glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

i) a glucose or xylose subunit, or a combination thereof and at least one alpha-glycosidic bond, or
ii) a glucose or xylose subunit, or a combination thereof and at least one beta-glycosidic bond, and

which are a substrate of one or more, e.g., two, three, four, or more, human gut microbe glycosidase enzymes selected from:

i) GH13 subfamily 20, GH13 subfamily 31, GH13 subfamily 39, GH39, GH43 subfamily 11, GH5 subfamily 44, or GH94 CAZy family, or
ii) GH2, GH31, GH23, GH13, or GH24 CAZy family.

146. A glycan polymer preparation, e.g., wherein the preparation comprises at least 0.5, 1, 2, 5, 10, 50, or 100 kg, and, e.g., is at least 20, 30, 40, 50, 60, 70, 80, 90, 95 or 99 % pure, comprising glycan polymers comprising:

a glucose, xylose, arabinose, or galactose subunit, or a combination thereof and at least one alpha-glycosidic bond, and
which are a substrate of one or more, e.g., two, three, four, or more, human gut microbe glycosidase enzymes selected from:

i) GH13 subfamily 3, GH13 subfamily 30, GH121, GH15, GH43 subfamily 27, GH43 subfamily 34, or GH43 subfamily 8 CAZy family, or
ii) GH92, GH97, GH76, GH28, GH20, GH105, GH2, GH50, GH3, or GH106 CAZy family.

147. The glycan preparation of any one of paragraphs 140-146, formulated as a pharmaceutical composition, a medical food, a dietary supplement, a food ingredient, or a therapeutic nutrition product, e.g., wherein formulating comprises dividing the preparation into a plurality of dosage forms or portions.

148. The glycan preparation of any one of paragraphs 140-147, formulated for oral administration as a liquid.

149. The glycan preparation of paragraph 148, wherein the liquid is a beverage, a syrup, an aqueous solution, or an aqueous suspension.

150. The glycan preparation of any one of paragraphs 140-147, formulated for oral administration as a solid.

151. The glycan preparation of paragraph 150, wherein the solid is a tablet, a pill, a capsule, a lozenge, a candy, or a powder.

152. The glycan preparation of paragraph 150, wherein the solid is a solid food product.

153. The glycan preparation of paragraph 151, wherein the powder is formulated for reconstitution in an aqueous solution prior to oral administration.

154. The glycan preparation of any one of paragraphs 140-147, formulated for rectal administration as a solid or liquid.

155. The glycan preparation of paragraph 154, formulated as an enema or suppository.

156. The glycan preparation of any one of paragraphs 140-155, formulated as a delayed release or time controlled system.

157. The glycan preparation of any one of paragraphs 140-156, further comprising a pharmaceutically acceptable carrier or excipient.

158. The glycan preparation of any one of paragraphs 140-156, further comprising a food acceptable carrier or excipient.

159. The glycan preparation of any one of paragraphs 140-158, further comprising a second therapeutic agent.

160. The glycan preparation of any one of paragraphs 140-159, further comprising a preparation of a gut microbe (e.g., a human gut microbe).

161. The glycan preparation of paragraph 160, wherein the glycan polymer is a substrate of the gut microbe.

162. The glycan preparation of paragraph 161, wherein the glycan polymer is a substrate of a gut microbial glycosidase enzyme and promotes the growth of the gut microbe.

163. A unit dosage from comprising the glycan preparation of any one of paragraphs 140-162.

164. The unit dosage form of paragraph 163, formulated for enteral administration, nasal, oral or rectal administration, or for tube feeding.

165. The unit dosage form of paragraphs 163 or 164, wherein the unit-dosage form, e.g., the glycan polymer preparation component of the unit-dosage form, has a caloric value of about 0.01 kcal to about 1 kcal, 0.1 kcal to 5 kcal, 0.01 kcal to 10 kcal, or 0.1 kcal to 10 kcal.

166. The unit dosage form of any one of paragraphs 163-165, formulated for timed and/or targeted release in the colon or large intestine.

167. A pharmaceutical composition comprising the glycan preparation of any one of paragraphs 140-162.

168. A set of pharmaceutical compositions, each comprising the glycan polymer preparation, or a portion thereof, of any one of paragraphs 140-162, wherein collectively, the set comprises at least 0.1, 0.5, 1, 2, 5, 10, or 100 kilograms of the preparation.

169. A medical food comprising the glycan preparation of any one of paragraphs 140-162.

170. A set of medical food portions, each comprising the glycan polymer preparation, or a portion thereof, of any one of paragraphs 140-162, wherein collectively, the set comprises at least 0.1, 0.5, 1, 2, 5, 10, or 100 kilograms of the preparation.

171. A dietary supplement comprising the glycan preparation of any one of paragraphs 140-162.

172. A set of dietary supplement portions, each comprising the glycan polymer preparation, or a portion thereof, of any one of paragraphs 140-162, wherein collectively, the set comprises at least 0.1, 0.5, 1, 2, 5, 10, or 100 kilograms of the preparation.

173. A food ingredient comprising the glycan preparation of any one of paragraphs 140-162.

174. A set of food ingredient portions, each comprising the glycan polymer preparation, or a portion thereof, of any one of paragraphs 140-162, wherein collectively, the set comprises at least 0.1, 0.5, 1, 2, 5, 10, or 100 kilograms of the preparation.

175. A method of making a co-preparation comprising:

   providing a preparation of a human gut microbe,
   providing the glycan polymer preparation of any one of paragraphs 140-162,

wherein the glycan polymer is a substrate of the human gut microbe, and
combining the human gut microbe comprising with the glycan polymer.

176. The method of paragraph 175, wherein the human gut microbe is selected from a microbe listed in Table 2.

177. The method of paragraph 175, wherein the human gut microbe is selected from a microbe listed in Table 3.

178. The method of any one of paragraphs 175-177, further comprising identifying the CAZy family profile of the human gut microbe and selecting a glycan polymer preparation that is a substrate based on the identified CAZy family profile of the human gut microbe.

179. The method of any one of paragraphs 175-178, further comprising formulating the co-preparation for oral, nasal or rectal delivery or tube feeding.

180. The method of any one of paragraphs 175-179, further comprising formulating the co-preparation as a timed-release formulation.

181. The method of paragraph 180, wherein release of the preparation occurs in the colon or large intestine.

182. The method of any one of paragraphs 175-181, wherein greater than about 50%, 60%, 70%, 80%, 90%, 95% or greater than 98% of the microbes of the preparation are viable after stomach transit (e.g. when reaching the colon or large intestine).

183. The method of any one of paragraphs 175-182, wherein greater than about 1%, 5%, 10%, 15%, 20%, 25%, 30%, 40%, 50%, 60% or greater than 75% of the microbes of the preparation engraft after release in the colon or large intestine.

184. The method of any one of paragraphs 175-183, wherein the glycan polymer preparation is made by glycosidase-directed synthesis selecting one or more glycosidase from the identified CAZy family profile for the synthesis of the glycan polymers.

185. The method of any one of paragraphs 175-183, wherein the glycan polymer preparation is synthesized and designed on the basis of the identified CAZy family profile using a non-enzymatic, polymeric catalyst.

186. The method of any one of paragraphs 175-185, further comprising formulating the co-preparation into a pharmaceutical composition.

187. A synbiotic co-preparation comprising a preparation of a human gut microbe and a preparation of a glycan polymer of any one of paragraphs 140-162.

188. The synbiotic co-preparation of paragraph 187, further comprising a pharmaceutically acceptable excipient or carrier.

189. The synbiotic co-preparation of paragraphs 187 or 188, formulated as a unit dosage form for nasal, oral, gastric or rectal delivery.

190. The synbiotic co-preparation of any one of paragraphs 187-189, formulated to protect the human gut microbes of the preparation from stomach acid inactivation.

191. A method of engrafting a human gut microbe in the colon or large intestine of a human subject in need thereof, comprising: administering a synbiotic co-preparation of any one of paragraphs 187-190 to the subject in an amount and for a time effective to engraft the human gut microbe.

192. The method of paragraph 191, wherein the human subject has a dysbiosis of the microbiota of the gut, and e.g., has undergone a treatment or exposure that causes such dysbiosis, and e.g., the human subject has been identified as having undergone the treatment or exposure.

193. The method of paragraphs 191 or 192, wherein the human subject has undergone antibiotic treatment.

194. The method of paragraphs 191 or 192, wherein the human subject has not undergone antibiotic treatment.

195. The method of any one of paragraphs 191-194, wherein the microbiota of the gut (e.g. colon or large intestine) is stable (e.g. in the absence of significant changes in relative abundance of taxa).

196. The method of any one of paragraphs 191-194, wherein the microbiota of the gut (e.g. colon or large intestine) is instable (e.g. in the presence of significant changes in relative abundance of taxa).

197. The method of any one of paragraphs 191-196, wherein the extent of engraftment is determined through analysis, e.g., by 16S, quantitative culture, or qPCR, before and after administering the synbiotic co-preparation.

198. The method of any one of paragraphs 191-197, wherein the extent of engraftment is determined through comparison of the number of organisms administered to the subject in the synbiotic co-preparation with the number of organisms recoverable from the gut of the subject, e.g., through quantitative culture or qPCR.

199. The method of any one of paragraphs 191-198, wherein the human subject has a disease or disorder listed in Table 5, e.g., acute pouchitis, allergic diseases, AIDS, atherosclerosis, asthma, atopic dermatitis, autism spectrum disorder, chronic functional constipation, celiac disease, chronic atrophic gastritis, chronic pouchitis, Clostridium difficile-associated disease (CDAD), celiac disease, colorectal adenoma, colorectal cancer, Crohn's disease, cystic fibrosis, depression, diabetes (Type I), diabetes (Type II), diarrhea, eczema, enterostomy, familial mediterranean fever, food hypersensitivity, graft-versus-host disease (GvHD), hepatic encephalopathy, hypertension, inflammatory bowel disease, irritable bowel disease, irritable bowel disease-constipation (IBS-C), lung cancer, microscopic colitis, multiple sclerosis, non-alcoholic fatty liver disease (NAFLD), non-alcoholic steatohepatitis (NASH), obesity-related asthma, Parkinson's disease (PD), radiation-induced acute intestinal symptoms, Shigellosis, short bowel syndrome, spinal cord injury associated bowel dysfunction, systemic inflammatory response syndrome, systemic lupus erythematosus, or ulcerative colitis.

200. The method of any one of paragraphs 191-198, wherein the human subject has a disease or disorder listed in Table 5, e.g., atherosclerosis, cardiovascular disease, cardiovascular risk in HIV, carotid atherosclerosis, chronic heart disease, chronic heart failure, chronic kidney disease, chronic vascular disease, colorectal cancer, coronary heart disease, coronary artery disease (CAD), diabetes (Type II), end stage renal disease, HIV, inflammatory bowel disease, ischemic attack, metabolic syndrome, non-alcoholic fatty liver disease (NAFLD), obesity, radiation-induced acute intestinal symptoms (RIAISs), or stroke.

201. The method of any one of paragraphs 191-198, wherein the human subject has a disease or disorder listed in Table 5, e.g., chronic kidney disease, Helicobacter pylori infection, hepatic encephalopathy, or liver cirrhosis with minimal hepatic encephalopathy (MHE).

202. A method of treating a subject having a dysbiosis, comprising:
administering a composition comprising a glycan polymer preparation described herein and a preparation of a microbe in an amount effective to treat the dysbiosis.

203. The method of paragraph 202, wherein the microbe is a spore-forming microbe.

204. The method of paragraph 202 or 203, wherein the glycan polymer preparation comprises: xylose, arabinose, glucose, galactose or a combination thereof.

205. The method of any one of paragraphs 202-204, wherein the glycan polymers, or at least 20, 30, 40, 50, 60, 70, 80, 90, 95, or 99 % (by weight or number) of the glycan polymers, of the glycan polymer preparation have one or more (e.g. two, three, four, five, or six) of the properties listed in Table 1, optionally selected from:

a. glycan polymers comprising a xylose or arabinose subunit, or a combination thereof and at least one alpha-glycosidic bond,
b. glycan polymers comprising a xylose or arabinose subunit, or a combination thereof and at least one beta-glycosidic bond,
c. glycan polymers comprising a galactose, xylose, or arabinose subunit, or a combination thereof and at least one alpha-glycosidic bond,
d. glycan polymers comprising a galactose, xylose, or arabinose subunit, or a combination thereof and at least one beta-glycosidic bond,
e. glycan polymers comprising a glucose, xylose, or arabinose subunit, or a combination thereof and at least one alpha-glycosidic bond,
f. glycan polymers comprising a glucose, xylose, or arabinose subunit, or a combination thereof and at least one beta-glycosidic bond,
g. glycan polymers comprising a xylose, arabinose, glucose or galactose subunit, or a combination thereof and at least one alpha-glycosidic bond,
h. glycan polymers comprising a xylose, arabinose, glucose or galactose subunit, or a combination thereof and at least one beta-glycosidic bond, or a combination thereof and at least one beta-glycosidic bond.

206. The method of any one of paragraphs 202-205, wherein the glycan polymers, or at least 20, 30, 40, 50, 60, 70, 80, 90, 95, or 99 % (by weight or number) of the glycan polymers, of the glycan polymers of the glycan polymer preparation is a substrate for a glycosidase enzyme.

207. The method of any one of paragraphs 202-206, wherein the glycosidase enzyme is present in a spore-forming human gut microbe.

208. The method of any one of paragraphs 202-207, wherein the glycan polymer is a substrate for a glycosidase enzyme of one of GT5, GT35, GT3, GH97, GH95, GH92, GH89, GH88, GH78, GH77, GH57, GH51, GH43 subfamily 34, GH43 subfamily 24, GH43 subfamily 10, GH42, GH36, GH35, GH33, GH32, GH31, GH3, GH29, GH28, GH27, GH24, GH20, GH2, GH16, GH133, GH130, GH13 subfamily 8, GH13 subfamily 38, GH13 subfamily 14, GH13, GH123, GH115, GH109, or GH105 CAZy family.

209. The method of any one of paragraphs 202-208, wherein the microbe is any one of those of Table 19, column 1.

210. The method of any one of paragraphs 202-208, wherein the microbe is any one of those of Table 20, column 1.

211. The method of any one of paragraphs 202-208, wherein the microbe is any one of those of Table 21, column 1.

212. The method of any one of paragraphs 202-208, wherein the microbe is any one of those of Table 19, column 1 and the glycan preparation is any one of Table 19, column 3, Table 19, column 4, Table 19, column 5, Table 19, column 6, Table 19, column 7, Table 19, column 8, Table 19, column 9, or Table 19, column 10.

213. The method of any one of paragraphs 202-208, wherein the microbe is any one of those of Table 20, column 1 and the glycan preparation is any one of Table 20, column 2, Table 20, column 3, Table 20, column 4, Table 20, column 5, Table 20, column 6, Table 20, column 7, Table 20, column 8, or Table 20, column 9.

214. The method of any one of paragraphs 202-208, wherein the microbe is any one of those of Table 21, column 1 and the glycan preparation is any one of Table 21, column 2, Table 21, column 3, Table 21, column 4, Table 21, column 5, Table 21, column 6, Table 21, column 7, Table 21, column 8, or Table 21, column 9.

215. A glycan polymer preparation described herein comprising glycan polymers which are a substrate of a human gut microbe glycosidase enzyme of a spore-forming microbe (e.g. spore-forming bacterial taxa)

216. A glycan polymer preparation, optionally, e.g., wherein the preparation comprises at least about 0.5, 1, 2, 5, 10, 50, or 100 kg, and/or, further optionally, e.g., is at least 20, 30, 40, 50, 60, 70, 80, 90, 95 or 99 % pure, comprising glycan polymers comprising:

a. a xylose or arabinose subunit, or a combination thereof and at least one alpha-glycosidic bond,
b. a xylose or arabinose subunit, or a combination thereof and at least one beta-glycosidic bond,
c. a galactose, xylose, or arabinose subunit, or a combination thereof and at least one alpha-glycosidic bond,
d. a galactose, xylose, or arabinose subunit, or a combination thereof and at least one beta-glycosidic bond,
e. a glucose, xylose, or arabinose subunit, or a combination thereof and at least one alpha-glycosidic bond,
f. a glucose, xylose, or arabinose subunit, or a combination thereof and at least one beta-glycosidic bond,
g. a xylose, arabinose, glucose or galactose subunit, or a combination thereof and at least one alpha-glycosidic bond,
h. a xylose, arabinose, glucose or galactose subunit, or a combination thereof and at least one beta-glycosidic bond, or a combination thereof and at least one beta-glycosidic bond, and

which are a substrate of a human gut microbe glycosidase enzyme of one of: GT5, GT35, GT3, GH97, GH95, GH92, GH89, GH88, GH78, GH77, GH57, GH51, GH43 subfamily 34, GH43 subfamily 24, GH43 subfamily 10, GH42, GH36, GH35, GH33, GH32, GH31, GH3, GH29, GH28, GH27, GH24, GH20, GH2, GH16, GH133, GH130, GH13 subfamily 8, GH13 subfamily 38, GH13 subfamily 14, GH13, GH123, GH115, GH109, or GH105 CAZy family.

217. The glycan polymer preparation of paragraph 215 or 216, wherein the microbe is any one of those of Table 19, column 1.

218. The glycan polymer preparation of paragraph 215 or 216, wherein the microbe is any one of those of Table 20, column 1.

219. The glycan polymer preparation of paragraph 215 or 216, wherein the microbe is any one of those of Table 21, column 1.

220. The glycan polymer preparation of any one of paragraphs 215-219, wherein the microbe is any one of those of Table 19, column 1 and the glycan preparation is any one of Table 19, column 3, Table 19, column 4, Table 19, column 5, Table 19, column 6, Table 19, column 7, Table 19, column 8, Table 19, column 9, or Table 19, column 10.

221. The glycan polymer preparation of any one of paragraphs 215-219, wherein the microbe is any one of those of Table 20, column 1 and the glycan preparation is any one of Table 20, column 2, Table 20, column 3, Table 20, column 4, Table 20, column 5, Table 20, column 6, Table 20, column 7, Table 20, column 8, or Table 20, column 9.

222. The glycan polymer preparation of any one of paragraphs 215-219, wherein the microbe is any one of those of Table 21, column 1 and the glycan preparation is any one of Table 21, column 2, Table 21, column 3, Table 21, column 4, Table 21, column 5, Table 21, column 6, Table 21, column 7, Table 21, column 8, or Table 21, column 9.

223. A method of making a co-preparation comprising:

providing a preparation of a spore-forming microbe (e.g. a spore-forming human gut microbe),
providing the glycan polymer preparation (described herein),

wherein the glycan polymer is a substrate of the spore-forming microbe, and
combining the preparation of the spore-forming microbe with the glycan polymer preparation.

224. The method of paragraph 223, wherein the glycan polymers comprise one of:

a. a xylose or arabinose subunit, or a combination thereof and at least one alpha-glycosidic bond,
b. a xylose or arabinose subunit, or a combination thereof and at least one beta-glycosidic bond,
c. a galactose, xylose, or arabinose subunit, or a combination thereof and at least one alpha-glycosidic bond,
d. a galactose, xylose, or arabinose subunit, or a combination thereof and at least one beta-glycosidic bond,
e. a glucose, xylose, or arabinose subunit, or a combination thereof and at least one alpha-glycosidic bond,
f. a glucose, xylose, or arabinose subunit, or a combination thereof and at least one beta-glycosidic bond,
g. a xylose, arabinose, glucose or galactose subunit, or a combination thereof and at least one alpha-glycosidic bond, or
h. a xylose, arabinose, glucose or galactose subunit, or a combination thereof and at least one beta-glycosidic bond, or a combination thereof and at least one beta-glycosidic bond.

225. The method of paragraph 223 or 224, wherein the glycan polymer is a substrate for a glycosidase enzyme of one of GT5, GT35, GT3, GH97, GH95, GH92, GH89, GH88, GH78, GH77, GH57, GH51, GH43 subfamily 34, GH43 subfamily 24, GH43 subfamily 10, GH42, GH36, GH35, GH33, GH32, GH31, GH3, GH29, GH28, GH27, GH24, GH20, GH2, GH16, GH133, GH130, GH13 subfamily 8, GH13 subfamily 38, GH13 subfamily 14, GH13, GH123, GH115, GH109, or GH105 CAZy family.

226. The method of any one of paragraphs 223-225, wherein the microbe is any one of those of Table 19, column 1.

227. The method of any one of paragraphs 223-225, wherein the microbe is any one of those of Table 20, column 1.

228. The method of any one of paragraphs 223-225, wherein the microbe is any one of those of Table 21, column 1.

229. The method of any one of paragraphs 223-228, wherein the microbe is any one of those of Table 19, column 1 and the glycan preparation is any one of Table 19, column 3, Table 19, column 4, Table 19, column 5, Table 19, column 6, Table 19, column 7, Table 19, column 8, Table 19, column 9, or Table 19, column 10.

230. The method of any one of paragraphs 223-228, wherein the microbe is any one of those of Table 20, column 1 and the glycan preparation is any one of Table 20, column 2, Table 20, column 3, Table 20, column 4, Table 20, column 5, Table 20, column 6, Table 20, column 7, Table 20, column 8, or Table 20, column 9.

231. The method of any one of paragraphs 223-228, wherein the microbe is any one of those of Table 21, column 1 and the glycan preparation is any one of Table 21, column 2, Table 21, column 3, Table 21, column 4, Table 21, column 5, Table 21, column 6, Table 21, column 7, Table 21, column 8, or Table 21, column 9.

232. The method of any one of paragraphs 223-231, further comprising formulating the co-preparation for oral, nasal or rectal delivery or tube feeding.

233. The method of any one of paragraphs 223-232, further comprising formulating the co-preparation as a timed-release formulation.

234. The method of paragraph 233, wherein release of the preparation occurs in the colon or large intestine.

235. The method of any one of paragraphs 223-234, wherein greater than about 50%, 60%, 70%, 80%, 90%, 95% or greater than 98% of the microbes of the preparation are viable after stomach transit (e.g. when reaching the colon or large intestine).

236. The method of any one of paragraphs 223-235, wherein greater than about 1%, 5%, 10%, 15%, 20%, 25%, 30%, 40%, 50%, 60% or greater than 75% of the microbes of the preparation engraft after release in the colon or large intestine.

237. The method of any one of paragraphs 223-236, wherein the glycan polymer preparation is made by glycosidase-directed synthesis selecting one or more glycosidase from the identified CAZy family profile for the synthesis of the glycan polymers.

238. The method of any one of paragraphs 223-237, wherein the glycan polymer preparation is synthesized and designed on the basis of the identified CAZy family profile using a non-enzymatic, polymeric catalyst.

239. The method of any one of paragraphs 223-238, further comprising formulating the co-preparation into a pharmaceutical composition.

240. A synbiotic co-preparation comprising a preparation of a human gut microbe and a preparation of a glycan polymer of any one of paragraphs 223-239.

241. The synbiotic co-preparation of paragraph 240, further comprising a pharmaceutically acceptable excipient or carrier.

242. The synbiotic co-preparation of paragraphs 240 or 241, formulated as a unit dosage form for nasal, oral, gastric or rectal delivery.

243. The synbiotic co-preparation of any one of paragraphs 240-242, formulated to protect the human gut microbes of the preparation from stomach acid inactivation.

244. A method of engrafting a human gut microbe in the colon or large intestine of a human subject in need thereof, comprising: administering a synbiotic co-preparation of any one of paragraphs 240-243 to the subject in an amount and for a time effective to engraft the human gut microbe.

245. The method of paragraph 244, wherein the human subject has a dysbiosis of the microbiota of the gut, and e.g., has undergone a treatment (e.g. antimicrobial treatment, cancer treatment, etc.) or exposure (e.g. exposure to a pathogen, such as a bacterial pathogen, e.g., C. diff.) that causes such dysbiosis, and optionally, e.g., the human subject has been identified as having undergone the treatment or exposure.

246. The method of paragraphs 244 or 245, wherein the human subject has undergone antibiotic treatment.

247. The method of paragraphs 244 or 245, wherein the human subject has not undergone antibiotic treatment.

248. The method of any one of paragraphs 244-247, wherein the microbiota of the gut (e.g. colon or large intestine) is stable (e.g. in the absence of significant changes in relative abundance of taxa).

249. The method of any one of paragraphs 244-247, wherein the microbiota of the gut (e.g. colon or large intestine) is instable (e.g. in the presence of significant changes in relative abundance of taxa).

250. The method of any one of paragraphs 244-249, wherein the extent of engraftment is determined through analysis, e.g., by 16S, quantitative culture, or qPCR, before and after administering the synbiotic co-preparation.

251. The method of any one of paragraphs 244-250, wherein the extent of engraftment is determined through comparison of the number of organisms administered to the subject in the synbiotic co-preparation with the number of organisms recoverable from the gut of the subject, e.g., through quantitative culture or qPCR.

252. A method of any embodiment described herein.

253. A composition of any embodiment described herein.

254. A method of making a preparation of a glycan polymer, e.g., a glycan polymer that is a substrate for a glycosidase enzyme present in a human gut microbe, comprising:

    providing a plurality of glycan subunits, e.g., a sugar monomer or a sugar dimer, suitable for the production of the glycan polymer; and

    contacting the glycan subunits of the plurality with a glycosidase enzyme molecule, e.g. derived from a human gut microbe, under conditions that result in the incorporation, e.g., by a condensation reaction, of the glycan subunits into a glycan polymer,

thereby making a glycan polymer preparation that is a substrate for a human gut microbe, optionally wherein:

i) the glycan polymer preparation comprises at least about 0.25, 0.5, 1, 5, 10, 20, 50, 100, 200, 300, 400 or 500 kilograms of glycan polymer, and/or

ii) the glycan polymer preparation is produced at a yield of at least about 15%, 30%, 45%, 60%, or of about 75% (as determined on a weight/weight basis as a percentage of input glycan subunits).

255. The method of paragraph 254, wherein the human gut microbe from which the glycosidase enzyme molecule is derived is of the same taxa, e.g., phyla, order, family, genus or species as the human gut microbe for which the glycan polymer is a substrate.

256. The method of paragraph 254, wherein the human gut microbe from which the glycosidase enzyme molecule is derived is of a first taxa, e.g., phyla, order, family, genus or species and the human gut microbe for which the glycan polymer is a substrate is of a second taxa, e.g., phyla, order, family, genus or species.

257. The method of any of paragraphs 254-256, further comprising formulating the glycan polymer preparation into a pharmaceutical composition, a medical food, a dietary supplement, a food ingredient, or a therapeutic nutrition product.

258. The method of any of paragraphs 254-257, further comprising dividing the preparation into a plurality of portions, e.g., unit dosages or formulations, e.g. for enteral administration, such as oral or rectal, or for tube feeding, such as nasal, oral or gastric tube feeding, e.g., dividing the preparation into at least 10, 100, or 1,000 portions.

259. The method of paragraph 258, wherein the plurality of portions differ by weight by no more than 0.5% 1%, 2%, 5%, 10%, or 20% in terms of the amount of glycan polymers present in the portions.

260. The method of any one of paragraphs 254-259 comprising combining the preparation with an excipient or carrier.

261. The method of paragraph 260, wherein the excipient or carrier is a pharmaceutically acceptable excipient or carrier.

262. The method of paragraph 260, wherein the excipient or carrier is food stuff.

263. The method of any one of paragraphs 254-262, wherein the glycosidase enzyme and the glycosidase enzyme molecule are independently selected from Tables 4 (column 2), 23 (column A), 24 (column A), or 22 (column 1).

264. The method of any one of paragraphs 254-263, wherein the amino acid sequence encoding the glycosidase enzyme shares at least 95%, 97%, or 99% sequence identity with an amino acid encoded by any one of SEQ ID Nos 1-124.

265. The method of any one of paragraphs 254-264, wherein the amino acid sequence encoding the glycosidase enzyme shares at least 95%, 97%, or 99% sequence identity with an amino acid encoded by any one of SEQ ID Nos 12, 18, 31, 38, 39, 48, 56, 57, 64, 68, 72, 83, 84, 92, 93, 99, 104, 110, and 117 of Tables 23 or 24.

266. The method of any one of paragraphs 254-265, wherein the amino acid sequence encoding the glycosidase enzyme molecule shares at least 95%, 97%, or 99% sequence identity with an amino acid encoded by any one of SEQ ID Nos 1-124.

267. The method of any one of paragraphs 254-266, wherein the amino acid sequence encoding the glycosidase enzyme molecule shares at least 95%, 97%, or 99% sequence identity with an amino acid encoded by any one of SEQ ID Nos 12, 18, 31, 38, 39, 48, 56, 57, 64, 68, 72, 83, 84, 92, 93, 99, 104, 110, and 117 of Tables 23 or 24.

268. The method of any one of paragraphs 262 to 267, wherein the glycosidase enzyme and/or the glycosidase enzyme molecule is other than from Bifidobacterium.

269. The method of any one of paragraphs 262 to 267, wherein the glycosidase enzyme and/or the glycosidase enzyme molecule is other than from Lactobacillus.

270. The method of any one of paragraphs 254-269, wherein the glycosidase enzyme and the glycosidase enzyme molecule are of the same human gut microbial origin.

271. The method of paragraph 270, wherein the glycosidase enzyme and the glycosidase enzyme molecule are selected from Tables 4 (column 2), 23 (column A), 24 (column A), or 22 (column 1).

272. The method of any one of paragraphs 254-271, wherein the amino acid sequences of the glycosidase enzyme and the glycosidase enzyme molecule share at least 95%, 97%, or 99% sequence identity.

273. The method of paragraph 272, wherein the nucleic acid sequence encoding the amino acid sequence is one of SEQ ID Nos 1-124.

274. The method of paragraph 272, wherein the nucleic acid sequence encoding the amino acid sequence is one of SEQ ID Nos 12, 18, 31, 38, 39, 48, 56, 57, 64, 68, 72, 83, 84, 92, 93, 99, 104, 110, and 117 of Tables 23 or 24.

275. The method of paragraph 272, wherein the glycosidase enzyme and the glycosidase enzyme molecule are selected from Tables 4 (column 2), 23 (column A), 24 (column A), or 22 (column 1).

276. The method of any one of paragraphs 272 to 275, wherein the glycosidase enzyme and/or the glycosidase enzyme molecule is other than from Bifidobacterium.

277. The method of any one of paragraphs 272 to 275, wherein the glycosidase enzyme and/or the glycosidase enzyme molecule is other than from Lactobacillus.

278. The method of any one of paragraphs 254-277, wherein both the glycosidase enzyme and the glycosidase enzyme molecule are of the same CAZy family (e.g. of the same GH family (e.g., one or more of GH1 to GH135) and/or GT family (e.g., one or more of GT1 to GT101), e.g., those listed in Tables 4 (column 1), 23 (column C), 24 (column C), or 22 (column 1).

279. The method of any one of paragraphs 254-278, comprising acquiring the identity (e.g. taxonomic, 16s) of the human gut microbe and optionally its glycosidase profile (e.g. CAZy family profile).

280. The method of any one of paragraphs 254-279, wherein the human gut microbe is selected from a microbial taxa of a phylum (column 1), class (column 2) or genus (column 3) listed in Table 2.

281. The method of any one of paragraphs 254-279, wherein the human gut microbe is selected from a microbial taxa of a strain (column 1) or phylum (column 2) listed in Table 3.

282. The method of any one of paragraphs 254-279, wherein the human gut microbe is selected from a microbial taxa of a genus listed in Table 4, column 3.

283. The method of any one of paragraphs 254-279, wherein the human gut microbe is selected from a microbe listed in Table 22, column 1.

284. The method of any one of paragraphs 254-279, wherein the human gut microbe is selected from a microbial taxa (spore-former) listed in Table 19, columns 1 and 2.

285. The method of any one of paragraphs 254-279, wherein the human gut microbe is selected from a microbial taxa (spore-former) listed in Table 20, column 1.

286. The method of any one of paragraphs 254-279, wherein the human gut microbe is selected from a microbe (spore-former) listed in Table 21, column 1.

287. The method of any one of paragraphs 254-286, wherein the human gut microbe is other than a Bifidobacterium.

288. The method of any one of paragraphs 254-287, wherein the human gut microbe is other than a Lactobacillus.

289. The method of paragraph 279, comprising, responsive to the identity of the human gut microbe and/or its glycosidase gene profile, selecting either or both of a glycosidase enzyme molecule and a glycan subunit.

290. The method of any one of paragraphs 254-289, wherein the glycosidase enzyme molecule (e.g. an isolated glycosidase enzyme molecule or a cell extract comprising a glycosidase enzyme molecule) is disposed on, e.g., coupled, covalently or noncovalently, to, a binding substrate (e.g., a solid surface such as that of a solid particle, or a matrix material, such as high MW carbon containing molecules, e.g. agarose, cellulose).

291. The method of paragraph 290, wherein the binding substrate is other than a bacterial cell.

292. The method of any one of paragraphs 254-291, wherein contacting comprises a cell-free process.

293. The method of any one of paragraphs 254-292, wherein the human gut microbe is a bacterium.

294. The method of any one of paragraphs 254-293, further comprising acquiring a value for a parameter related to the preparation, e.g., a physical parameter, e.g., molecular weight, e.g., average molecular weight or molecular weight distribution, glycan subunit composition, or purity or a parameter related to a biological property, e.g., the ability to modulate growth of the human gut microbe, the ability to modulate a microbial metabolite produced by a microbe, e.g., in an ex vivo assay, or the ability to modulate a biomarker, e.g., an inflammatory or immune biomarker, a toxic or waste compound, a bacterial compound) e.g., in a human subject.

295. The method of paragraph 294, comprising performing an assay to acquire the value.

296. The method of paragraph 294, comprising acquiring the value from another party.

297. The method of any of paragraphs 294-296, wherein the value is compared with a reference value to evaluate the glycan preparation, e.g., for suitability for use, e.g., therapeutic use.

298. The method of any one of paragraphs 254-297, wherein the glycosidase enzyme is encoded by a nucleic acid sequence selected from one or more of SEQ ID NOs: 1-124.

299. The method of any one of paragraphs 254-298, wherein the glycosidase enzyme is encoded by a nucleic acid sequence selected from one or more of SEQ ID Nos 12, 18, 31, 38, 39, 48, 56, 57, 64, 68, 72, 83, 84, 92, 93, 99, 104, 110, and 117.

300. The method of any one of paragraphs 254-299, wherein the glycosidase enzyme molecule is encoded by a nucleic acid sequence that is at least 80, 85, 90, 91, 92, 93, 94, 95, 96, 97, 98, 99, or 100% identical to a nucleic acid sequence selected from one or more of SEQ ID NOs: 1-124.

301. The method of any one of paragraphs 254-300, wherein the glycosidase enzyme molecule is encoded by a nucleic acid sequence that is at least 80, 85, 90, 91, 92, 93, 94, 95, 96, 97, 98, 99, or 100% identical to a nucleic acid sequence selected from one or more of SEQ ID Nos 12, 18, 31, 38, 39, 48, 56, 57, 64, 68, 72, 83, 84, 92, 93, 99, 104, 110, and 117.

302. The method of any one of paragraphs 254-301, wherein the glycosidase enzyme and/or the glycosidase enzyme molecule is derived from a human gut bacterium other than Bifidobacterium.

303. The method of any one of paragraphs 254-302, wherein the glycosidase enzyme and/or the glycosidase enzyme molecule is derived from a human gut bacterium other than Lactobacillus.

304. The method of any one of paragraphs 254-303, wherein the glycosidase enzyme and/or the glycosidase enzyme molecule is other than alpha-galactosidase.

305. The method of any one of paragraphs 254-304, wherein the glycosidase enzyme and/or the glycosidase enzyme molecule is other than beta-galactosidase.

306. The method of any one of paragraphs 254-305, wherein the glycosidase enzyme and/or the glycosidase enzyme molecule is other than: i) alpha-galactosidase; ii) beta-galactosidase, iii) alpha-glucosidase iv) beta-glucosidase, v) alpha-xylosidase, vi) beta-xylosidase, vii) alpha-mannosidase, viii) beta-mannosidase, ix) alpha-fructofuranosidase, and/or x) beta-fructofuranosidase, or other than any combination (e.g., any two of, three of, four of, five of, six of, seven of, or eight of) i), ii), iii), iv), v), vi), vii), viii), ix), and x).

307. The method of any one of paragraphs 254-306, wherein a glycan subunit is a sugar monomer selected from: glucose, galactose, mannose, fructose, fucose, rhamnose, xylose, and arabinose.

308. The method of any one of paragraphs 254-307, wherein a glycan unit is a sugar dimer selected from sucrose, maltose, gentibiose, lactulose, lactose, raffinose, melibiose, xylobiose, arabinobiose, fructobiose, turanose, cellobiose, mannobiose, galactobiose, sophorose, laminaribiose, and chitobiose.

309. The method of any one of paragraphs 254-308, wherein a glycan unit is a sugar dimer selected from sucrose, isomaltose, maltose, melezitose, gentibiose, cellobiose, melibiose, raffinose, lactose, lactulose, and palatinose (e.g., those listed in Tables 23, column E and 24, column E).

310. The method of any one of paragraphs 254-309, wherein a glycan unit is a sugar dimer other than lactose.

311. The method of any one of paragraphs 254-310, wherein a glycan unit is a sugar dimer other than lactulose.

312. The method of any one of paragraphs 254-311, wherein the conditions that result in the incorporation of a glycan subunit into a glycan polymer are suitable for a condensation reaction to incorporate a monomer into the glycan polymer.

313. The method of any one of paragraphs 254-312, wherein the conditions that result in the incorporation of a glycan subunit into a glycan polymer are suitable for a transglycosylation reaction (e.g., transgalactosylation, transglucosylation, transfructosylation) involving incorporation of a monomer into the glycan polymer from a dimer starting material.

314. The method of any one of paragraphs 254-313, wherein the conditions that result in the incorporation of a glycan subunit into a glycan polymer are suitable for a hydrolysis reaction.

315. The method of any one of paragraphs 254-314, wherein the average degree of polymerization (DP) of the glycan preparation is at least about DP2, at least about DP3, at least about DP4, or at least DP5.

316. The method of any one of paragraphs 254-315, wherein the average degree of polymerization (DP) of the glycan preparation is between about DP2 and DP4, DP2 and DP5, DP2 and DP6, DP3 and DP5 or DP3 and DP6.

317. The method of any one of paragraphs 254-316, wherein the average degree of polymerization (DP) of the glycan preparation is between about DP2 and DP8, between about DP2 and DP10, between about DP3 and DP8, or between about DP3 and DP10.

318. The method of any one of paragraphs 254-317, wherein at least 50%, 60%, 70%, 80%, 90%, 95%, or at least 99% of the glycan polymers of the preparation have a DP of 2 or greater.

319. The method of any one of paragraphs 254-318, wherein at least 50%, 60%, 70%, 80%, 90% or at least 95% of the glycan polymers of the preparation have a DP of 3 or greater.

320. The method of any one of paragraphs 254-319, wherein at least 50%, 60%, 70%, 80%, 90% or at least 95% of the glycan polymers of the preparation have a DP of between about DP2-4, DP2-5, DP2-6, DP2-8, DP2-10, DP3-5, DP3-6, DP3-8, or of between about DP3-10.

321. The method of any one of paragraphs 254-320, wherein the glycan polymers of the preparation have a degree of branching (DB) of 0.

322. The method of any one of paragraphs 254-321, wherein at least 50%, 60%, 70%, 80%, 90% or at least 95% of the glycan polymers of the preparation are branched.

323. The method of any one of paragraphs 254-322, wherein no more than 1%, 5%, 10%, 20%, 30%, 40% or no more than 50% of the glycan polymers of the preparation are branched.

324. The method of paragraph 322 or 323, wherein the branched glycan polymers of the preparation comprise one or more (e.g., one, two, three, four, or five) branching points.

325. The method of any one of paragraphs 254-324, wherein the glycan polymers of the preparation comprise alpha-glycosidic bonds, e.g. at least about 90%, 95%, 98%, 99%, or 100% of the glycosidic bonds of the glycan polymers of the preparation are alpha-glycosidic bonds.

326. The method of any one of paragraphs 254-325, wherein the glycan polymers of the preparation comprise beta-glycosidic bonds, e.g. at least about 90%, 95%, 98%, 99%, or 100% of the glycosidic bonds of the glycan polymers of the preparation are beta-glycosidic bonds.

327. The method of any one of paragraphs 254-326, wherein the glycan polymers of the preparation comprise alpha- and beta-glycosidic bonds.

328. The method of paragraph 327, wherein the alpha- to beta-glycosidic bond ratio is 1:1, 1:2, 1:3, 1:4 or 1:5.

329. The method of paragraph 327, wherein the beta- to alpha-glycosidic bond ratio is 1:1, 1:2, 1:3, 1:4 or 1:5.

330. The method of paragraph 327, wherein the beta- to alpha-glycosidic bond ratio is 1:4.

331. The method of any one of paragraphs 254-330, wherein the alpha- to beta-glycosidic bond ratio of the glycan polymers of the preparation is 0 or between about 0.1:1 to 1:5, 1:1 to 1:5 or 1:1 to 1:4.

332. The method of any one of paragraphs 254-331, wherein the beta- to alpha-glycosidic bond ratio of the glycan polymers of the preparation is 0 or between about 0.1:1 to 1:5, 1:1 to 1:5 or 1:1 to 1:4.

333. The method of any one of paragraphs 254-332, wherein the glycan polymers comprise one or more glycan unit of: glucose, galactose, mannose, fructose, fucose, rhamnose, xylose, and/or arabinose.

334. The method of any one of paragraphs 254-333, wherein the glycan polymers comprise one or more glycosidic bonds selected from: 1,2 glycosidic bond, a 1,3 glycosidic bond, a 1,4 glycosidic bond, a 1,5 glycosidic bond or a 1,6 glycosidic bond.

335. The method of paragraph 334, wherein the glycan polymer preparation comprises at least 20%, 30%, 40%, 50% or at least 60% (mol%) 1,4 glycosidic bonds.

336. The method of paragraph 334, wherein the glycan polymer preparation comprises at least 80%, 90%, at least 95%, or 100% (mol%) 1,4 glycosidic bonds.

337. The method of paragraph 334, wherein the glycan polymer preparation comprises at least 20%, 30%, 40%, 50% or at least 60% (mol%) 1,6 glycosidic bonds.

338. The method of paragraph 334, wherein the glycan polymer preparation comprises at least 80%, 90%, at least 95%, or 100% (mol%) 1,6 glycosidic bonds.

339. The method of paragraph 334, wherein the glycan polymer preparation comprises no more than 10%, 5%, no more than 1% or 0% 1,2 glycosidic bonds.

340. The method of paragraph 334, wherein the glycan polymer preparation comprises no more than 10%, 5%, no more than 1% or 0% 1,3 glycosidic bonds.

341. The method of paragraph 334, wherein the glycan polymer preparation comprises no more than 10%, 5%, no more than 1% or 0% 1,4 glycosidic bonds.

342. The method of paragraph 334, wherein the glycan polymer preparation comprises no more than 10%, 5%, no more than 1% or 0% 1,6 glycosidic bonds.

343. The method of any one of paragraphs 254-342, wherein the glycan polymers is other than galactooligosaccharide (GOS).

344. The method of paragraph 333, wherein the glycan polymer is other than a galactose homopolymer.

345. The method of paragraph 333, wherein the glycan polymer preparation is less than 99%, 95%, 90%, 80%, 70%, 60%, 50% galactose homopolymer.

346. The method of paragraph 333, wherein the first and second most abundant glycan polymer in the preparation are other than i) a galactose homopolymer and/or ii) a galactose polymer with a terminal glycose.

347. The method of any one of paragraphs 254-346, wherein the glycan polymer is other than: i) fructooligosaccharide (FOS), ii) galactooligosaccharide (GOS), iii) xylooligosacchaaride (XOS), iv) isomaltooligosaccharide (IMOS), and v) glucooligosaccharide (GLOS), or any combination (one of, two of, three of or four of, or all of) i), ii), iii), iv) and v).

348. The method of any one of paragraphs 254-347, wherein the glycan polymer is other than: i) lactosucrose, ii) lactulosucrose, iii) 2-alpha-glucosyl-lactose, iv) gentiooligosaccharide, v) pectic-oligosaccharide, and vi) maltosyl-fructoside, or any combination (one of, two of, three of or four of, five of, or all of) i), ii), iii), iv), v), and vi).

349. The method of any one of paragraphs 254-348, wherein the plurality of glycan subunits comprise a first and a second glycan subunit, wherein the first and second glycan subunits have different structures.

350. The method of any one of paragraphs 254-349, wherein the plurality of glycan subunits comprise a first and a second glycan subunit, wherein the first and second glycan subunits have the same structure.

351. The method of any one of paragraphs 254-350, wherein the glycan polymer comprises a glucose, mannose, or galactose subunit, or a combination thereof and at least one alpha-glycosidic bond.

352. The method of any one of paragraphs 254-351, wherein the glycan polymer comprises a glucose, mannose, or galactose subunit, or a combination thereof and at least one beta-glycosidic bond.

353. The method of any one of paragraphs 254-352, wherein the glycan polymer comprises a xylose, arabinose, fucose or rhamnose subunit, or a combination thereof and at least one alpha-glycosidic bond.

354. The method of any one of paragraphs 254-353, wherein the glycan polymer comprises a xylose, arabinose, fucose or rhamnose subunit, or a combination thereof and at least one beta-glycosidic bond.

355. The method of any one of paragraphs 254-354, wherein the glycan polymer comprises a glucose or galactose subunit, or a combination thereof and at least one alpha-glycosidic bond.

356. The method of any one of paragraphs 254-355, wherein the glycan polymer comprises a glucose or galactose subunit, or a combination thereof and at least one beta-glycosidic bond.

357. The method of any of paragraphs 254-306, wherein the glycan polymers and/or glycan polymer preparation comprise one, two, three, or more, e.g., all, of the following features:

i. the glycan polymers comprise glucose and at least one alpha-glycosidic bond, optionally, wherein the alpha-glycosidic bond is alpha-1,3 glycosidic bond, alpha-1,4 glycosidic bond, or a combination thereof, and further optionally, wherein the mean degree of polymerization (DP) of the preparation is between DP2-4, DP2-6, DP3-10, or between DP3-15;
ii. the glycan polymer preparation further comprises glycan polymers comprising at least one beta-glycosidic bond, optionally wherein the beta-glycosidic bond is beta-1,3 glycosidic bond, beta-1,4 glycosidic bond or a combination thereof;
iii. the glycan polymer preparation further comprises glycan polymers comprising galactose (e.g., a glu-gal preparation);
iv. the glycan polymer preparation further comprises glycan polymers comprising mannose (e.g., a glu-man preparation); and
v. the glycan polymer preparation further comprises glycan polymers comprising galactose and mannose (e.g., a glu-gal-man preparation).

358. The method of any of paragraphs 254-306, wherein the glycan polymers and/or glycan polymer preparation comprise one, two, three, or more, e.g., all, of the following features:

i. the glycan polymers comprise glucose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one alpha-glycosidic bond, optionally, wherein the alpha-glycosidic bond is alpha-1,3 glycosidic bond, alpha-1,4 glycosidic bond or a combination thereof;

iii. the glycan polymer preparation further comprises glycan polymers comprising galactose (e.g., a glu-gal preparation);

iv. the glycan polymer preparation further comprises glycan polymers comprising mannose (e.g., a glu-man preparation); and

v. the glycan polymer preparation further comprises glycan polymers comprising galactose and mannose (e.g., a glu-gal-man preparation).

359. The method of any of paragraphs 254-306, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond, optionally, wherein the beta-glycosidic bond is beta-1,3 glycosidic bond, beta-1,4 glycosidic bond or a combination thereof;

iii. the glycan polymer preparation further comprises glycan polymers comprising glucose (e.g., a gal-glu preparation);

iv. the glycan polymer preparation further comprises glycan polymers comprising mannose (e.g., a gal-man preparation); and

v. the glycan polymer preparation further comprises glycan polymers comprising glucose and mannose (e.g., a gal-man-glu preparation).

360 The method of any of paragraphs 254-306, wherein the glycan polymers and/or glycan polymer preparation comprise one, two, three, or more, e.g., all, of the following features:

i. the glycan polymers comprise galactose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one alpha-glycosidic bond, optionally wherein the alpha-glycosidic bond is alpha-1,3 glycosidic bond, alpha-1,4 glycosidic bond or a combination thereof;

iii. the glycan polymer preparation further comprises glycan polymers comprising glucose (e.g., a gal-glu preparation);

iv. the glycan polymer preparation further comprises glycan polymers comprising mannose (e.g., a gal-man preparation); and

v. the glycan polymer preparation further comprises glycan polymers comprising glucose and mannose (e.g., a gal-glu-man preparation).

361. The method of any of paragraphs 254-306, wherein the glycan polymers and/or glycan polymer preparation comprise one, two, three, or more, e.g., all, of the following features:

i. the glycan polymers comprise mannose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond, optionally, wherein the beta-glycosidic bond is beta-1,3 glycosidic bond, beta-1,4 glycosidic bond or a combination thereof;

iii. the glycan polymer preparation further comprises glycan polymers comprising galactose (e.g., a man-gal preparation);

iv. the glycan polymer preparation further comprises glycan polymers comprising glucose (e.g., a man-glu preparation); and

v. the glycan polymer preparation further comprises glycan polymers comprising galactose and glucose (e.g., a man-gal-glu preparation).

362. The method of any of paragraphs 254-306, wherein the glycan polymers and/or glycan polymer preparation comprise one, two, three, or more, e.g., all, of the following features:

i. the glycan polymers comprise mannose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising at least one alpha-glycosidic bond, optionally wherein the alpha-glycosidic bond is alpha-1,3 glycosidic bond, alpha-1,4 glycosidic bond or a combination thereof;

iii. the glycan polymer preparation further comprises glycan polymers comprising galactose (e.g., a man-gal preparation);

iv. the glycan polymer preparation further comprises glycan polymers comprising glucose (e.g., a man-glu preparation); and

v. the glycan polymer preparation further comprises glycan polymers comprising galactose and glucose (e.g., a man-gal-glu preparation).

363. The method of any of paragraphs 254-306, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation further comprises glycan polymers comprising fucose (e.g., a gal-fuc preparation);

v. the glycan polymer preparation further comprises glycan polymers comprising mannose (e.g., a gal-man preparation); and

vi. the glycan polymer preparation further comprises glycan polymers comprising fucose and mannose (e.g., a gal-fuc-man preparation).

364. The method of any of paragraphs 254-306, wherein the glycan polymers and/or glycan polymer preparation comprise one, two, three, or more, e.g., all, of the following features:

i. the glycan polymers comprise galactose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation further comprises glycan polymers comprising fucose (e.g., a gal-fuc preparation);

v. the glycan polymer preparation further comprises glycan polymers comprising mannose (e.g., a gal-man preparation); and

vi. the glycan polymer preparation further comprises glycan polymers comprising fucose and mannose (e.g., a gal-fuc-man preparation).

365. The method of any of paragraphs 254-306, wherein the glycan polymers and/or glycan polymer preparation comprise one, two, three, or more, e.g., all, of the following features:

i. the glycan polymers comprise fucose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation further comprises glycan polymers comprising galactose (e.g., a fuc-gal preparation);

v. the glycan polymer preparation further comprises glycan polymers comprising mannose (e.g., a fuc-man preparation); and

vi. the glycan polymer preparation further comprises glycan polymers comprising galactose and mannose (e.g., a fuc-gal-man preparation).

366. The method of any of paragraphs 254-306, wherein the glycan polymers and/or glycan polymer preparation comprise one, two, three, or more, e.g., all, of the following features:

i. the glycan polymers comprise fucose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-1;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation further comprises glycan polymers comprising galactose (e.g., a fuc-gal preparation);

v. the glycan polymer preparation further comprises glycan polymers comprising mannose (e.g., a fuc-man preparation); and

vi. the glycan polymer preparation further comprises glycan polymers comprising galactose and mannose (e.g., a fuc-gal-man preparation).

367. The method of any of paragraphs 254-306, wherein the glycan polymers and/or glycan polymer preparation comprise one, two, three, or more, e.g., all, of the following features:

i. the glycan polymers comprise mannose and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation further comprises glycan polymers comprising fucose (e.g., a man-fuc preparation);

v. the glycan polymer preparation further comprises glycan polymers comprising galactose (e.g., a man-gal preparation); and

vi. the glycan polymer preparation further comprises glycan polymers comprising galactose and fucose (e.g., a man-gal-fuc preparation).

368. The method of any of paragraphs 254-306, wherein the glycan polymers and/or glycan polymer preparation comprise one, two, three, or more, e.g., all, of the following features:

i. the glycan polymers comprise mannose and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation further comprises glycan polymers comprising fucose (e.g., a man-fuc preparation);

v. the glycan polymer preparation further comprises glycan polymers comprising galactose (e.g., a man-gal preparation); and

vi. the glycan polymer preparation further comprises glycan polymers comprising galactose and fucose (e.g., a man-gal-fuc preparation).

369. The method of any of paragraphs 254-306, wherein the glycan polymers and/or glycan polymer preparation comprise one, two, three, or more, e.g., all, of the following features:

i. the glycan polymers comprise one of, two of, or three of glucose, xylose and arabinose, and at least one alpha-glycosidic bond, optionally wherein the alpha-glycosidic bond is alpha-1,3 glycosidic bond, alpha-1,4 glycosidic bond, or a combination thereof, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,6 glycosidic bond, or a combination thereof;

iii. the glycan polymer preparation further comprises glycan polymers comprising at least one beta-glycosidic bond, optionally wherein the beta-glycosidic bond is beta-1,3 glycosidic bond, beta-1,4 glycosidic bond, beta-1,6 glycosidic bond or a combination thereof;

iv. the glycan polymer preparation comprises glycan polymers comprising glucose;

v. the glycan polymer preparation comprises glycan polymers comprising xylose; and

vi. the glycan polymer preparation comprises glycan polymers comprising arabinose.

370. The method of any of paragraphs 254-306, wherein the glycan polymers and/or glycan polymer preparation comprise one, two, three, or more, e.g., all, of the following features:

i. the glycan polymers comprise one of, two of, or three of glucose, xylose and arabinose, and at least one beta-glycosidic bond, optionally wherein the beta-glycosidic bond is beta-1,3 glycosidic bond, beta-1,4 glycosidic bond or a combination thereof, further optionally wherein the mean degree of polymerization (DP) of the preparation is between DP2-4, DP2-6, DP3-10 or between DP3-15;

ii. the glycan polymer preparation further comprises glycan polymers comprising beta-1,6 glycosidic bond;

iii. the glycan polymer preparation further comprises glycan polymers comprising at least one alpha-glycosidic bond, optionally wherein the alpha-glycosidic bond is alpha-1,2 glycosidic bond, alpha-1,3 glycosidic bond, alpha-1,4 glycosidic bond, alpha-1,6 glycosidic bond or a combination thereof;

iv. the glycan polymer preparation comprises glycan polymers comprising glucose;

v. the glycan polymer preparation comprises glycan polymers comprising xylose; and

vi. the glycan polymer preparation comprises glycan polymers comprising arabinose.

371. The method of any of paragraphs 254-306, wherein the glycan polymers and/or glycan polymer preparation comprise one, two, three, or more, e.g., all, of the following features:

i. the glycan polymers comprise glucose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising galactose (e.g., a glu-gal preparation);

v. the glycan polymer preparation further comprises glycan polymers comprising arabinose (e.g., a glu-ara preparation);

vi. the glycan polymer preparation further comprises glycan polymers comprising xylose (e.g., a glu-xyl preparation); and

vii. the glycan polymer preparation further comprises glycan polymers comprising two or three of galactose, arabinose, and xylose.

372. The method of any of paragraphs 254-306, wherein the glycan polymers and/or glycan polymer preparation comprise one, two, three, or more, e.g., all, of the following features:

i. the glycan polymers comprise galactose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising glucose (e.g., a gal-glu preparation);

v. the glycan polymer preparation further comprises glycan polymers comprising arabinose (e.g., a gal-ara preparation);

vi. the glycan polymer preparation further comprises glycan polymers comprising xylose (e.g., a gal-xyl preparation); and

vii. the glycan polymer preparation further comprises glycan polymers comprising two or three of glucose, arabinose, and xylose.

373. The method of any of paragraphs 254-306, wherein the glycan polymers and/or glycan polymer preparation comprise one, two, three, or more, e.g., all, of the following features:

i. the glycan polymers comprise one of or two of xylose and arabinose, and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation comprises glycan polymers comprising xylose; and

v. the glycan polymer preparation comprises glycan polymers comprising arabinose.

374. The method of any of paragraphs 254-306, wherein the glycan polymers and/or glycan polymer preparation comprise one, two, three, or more, e.g., all, of the following features:

i. the glycan polymers comprise arabinose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond;

iii. the glycan polymer preparation further comprises glycan polymers comprising galactose (e.g., an ara-gal preparation);

iv. the glycan polymer preparation further comprises glycan polymers comprising xylose (e.g., an ara-xyl preparation); and

v. the glycan polymer preparation further comprises glycan polymers comprising galactose and xylose (e.g., an ara-gal-xyl preparation).

375. The method of any of paragraphs 254-306, wherein the glycan polymers and/or glycan polymer preparation comprise one, two, three, or more, e.g., all, of the following features:

> i. the glycan polymers comprise galactose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;
>
> ii. the glycan polymer preparation further comprises glycan polymers comprising at least one beta-glycosidic bond;
>
> iii. the glycan polymer preparation further comprises glycan polymers comprising arabinose (e.g., a gal-ara preparation);
>
> iv. the glycan polymer preparation further comprises glycan polymers comprising xylose (e.g., a gal-xyl preparation); and
>
> v. the glycan polymer preparation further comprises glycan polymers comprising arabinose and xylose (e.g., a gal-ara-xyl preparation).

376. The method of any of paragraphs 254-306, wherein the glycan polymers and/or glycan polymer preparation comprise one, two, three, or more, e.g., all, of the following features:

> i. the glycan polymers comprise xylose and at least one alpha-glycosidic bond, optionally, wherein the alpha-glycosidic bond is alpha-1,3 glycosidic bond, further optionally, wherein the mean degree of polymerization (DP) of the preparation is between DP2-4, DP2-6, DP3-10 or between DP3-15;
>
> ii. the glycan polymer preparation further comprises glycan polymers comprising at least one beta-glycosidic bond;
>
> iii. the glycan polymer preparation further comprises glycan polymers comprising galactose (e.g., a xyl-gal preparation);
>
> iv. the glycan polymer preparation further comprises glycan polymers comprising arabinose (e.g., a xyl-ara preparation); and
>
> v. the glycan polymer preparation further comprises glycan polymers comprising galactose and arabinose (e.g., a xyl-ara-gal preparation).

377. The method of any of paragraphs 254-306, wherein the glycan polymers and/or glycan polymer preparation comprise one, two, or more, e.g., all, of the following features:

> i. the glycan polymers comprise glucose and at least one alpha-glycosidic bond, optionally, wherein the alpha-glycosidic bond is alpha-1,3 glycosidic bond, further optionally, wherein the mean degree of polymerization (DP) of the preparation is between DP2-4, DP2-6, DP3-10 or between DP3-15;
>
> ii. the glycan polymer preparation further comprises glycan polymers comprising at least one beta-glycosidic bond; and
>
> iii. the glycan polymer preparation further comprises glycan polymers comprising one of, two of, or three of arabinose, galactose or xylose.

378. The method of any of paragraphs 254-306, wherein the glycan polymers and/or glycan polymer preparation comprise one, two, three, or more, e.g., all, of the following features:

> i. the glycan polymers comprise glucose and at least one alpha-glycosidic bond, optionally wherein the mean degree of polymerization (DP) of the preparation is between DP2-4, DP2-6, DP3-10 or between DP3-15;
>
> ii. the glycan polymer preparation further comprises glycan polymers comprising alpha-1,2 glycosidic bond, alpha-1,3 glycosidic bond, alpha-1,4 glycosidic bond, alpha-1,6 glycosidic bond, or a combination thereof;
>
> iii. the glycan polymer preparation further comprises glycan polymers comprising at least one beta-glycosidic bond; and
>
> iv. the glycan polymer preparation further comprises glycan polymers comprising one of, two of, three of, or four of galactose, mannose, arabinose, or sialic acid.

379. The method of any of paragraphs 254-306, wherein the glycan polymers and/or glycan polymer preparation comprise one, two, three, or more, e.g., all, of the following features:

> i. the glycan polymers comprise glucose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond;

iii. the glycan polymer preparation further comprises glycan polymers comprising xylose (e.g., a glu-xyl preparation); and

iv. the glycan polymer preparation further comprises glycan polymers comprising one of, two of, or three of mannose, arabinose, or galactose.

380. The method of any of paragraphs 254-306, wherein the glycan polymers and/or glycan polymer preparation comprise one, two, three, or more, e.g., all, of the following features:

i. the glycan polymers comprise glucose and at least one beta-glycosidic bond, optionally wherein the mean degree of polymerization (DP) of the preparation is between DP2-4, DP2-6, DP3-10 or between DP3-15;

ii. the glycan polymer preparation further comprises glycan polymers comprising at least one alpha-glycosidic bond, optionally wherein the alpha-glycosidic bond is alpha-1,3 glycosidic bond;

iii. the glycan polymer preparation further comprises glycan polymers comprising at least one beta-glycosidic bond;

iv. the glycan polymer preparation further comprises glycan polymers comprising xylose (e.g., a glu-xyl preparation); and

v. the glycan polymer preparation further comprises glycan polymers comprising one of, two of, or three of mannose, arabinose, or galactose.

381. The method of any of paragraphs 254-306, wherein the glycan polymers and/or glycan polymer preparation comprise one, two, three, or more, e.g., all, of the following features:

i. the glycan polymers comprise xylose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising at least one beta-glycosidic bond;

iii. the glycan polymer preparation further comprises glycan polymers comprising glucose (e.g., a xyl-glu preparation); and

iv. the glycan polymer preparation further comprises glycan polymers comprising one of, two of, or three of mannose, arabinose, or galactose.

382. The method of any of paragraphs 254-306, wherein the glycan polymers and/or glycan polymer preparation comprise one, two, three, or more, e.g., all, of the following features:

i. the glycan polymers comprise xylose and at least one beta-glycosidic bond, further optionally wherein the mean degree of polymerization (DP) of the preparation is between DP2-4, DP2-6, DP3-10 or between DP3-15;

ii. the glycan polymer preparation further comprises glycan polymers comprising at least one alpha-glycosidic bond, optionally wherein the alpha-glycosidic bond is alpha-1,3 glycosidic bond;

iii. the glycan polymer preparation further comprises glycan polymers comprising at least one beta-glycosidic bond;

iv. the glycan polymer preparation further comprises glycan polymers comprising glucose (e.g., a xyl-glu preparation); and

v. the glycan polymer preparation further comprises glycan polymers comprising one of, two of, or three of mannose, arabinose, or galactose.

383. The method of any of paragraphs 254-306, wherein the glycan polymers and/or glycan polymer preparation comprise one, two, three, or more, e.g., all, of the following features:

i. the glycan polymers comprise glucose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising xylose (e.g., a glu-xyl preparation);

v. the glycan polymer preparation further comprises glycan polymers comprising arabinose (e.g., a glu-ara preparation);

vi. the glycan polymer preparation further comprises glycan polymers comprising galactose (e.g., a glu-gal preparation); and

vii. the glycan polymer preparation further comprises glycan polymers comprising one of, two of, or three of xylose, arabinose, or galactose.

384. The method of any of paragraphs 254-306, wherein the glycan polymers and/or glycan polymer preparation comprise one, two, three, or more, e.g., all, of the following features:

i. the glycan polymers comprise xylose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising glucose (e.g., a xyl-glu preparation);

v. the glycan polymer preparation further comprises glycan polymers comprising arabinose (e.g., a xyl-ara preparation);

vi. the glycan polymer preparation further comprises glycan polymers comprising galactose (e.g., a xyl-gal preparation); and

vii. the glycan polymer preparation further comprises glycan polymers comprising one of, two of, or three of glucose, arabinose, or galactose.

385. The method of any of paragraphs 254-306, wherein the glycan polymers and/or glycan polymer preparation comprise one, two, three, or more, e.g., all, of the following features:

i. the glycan polymers comprise arabinose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising xylose (e.g., a ara-xyl preparation);

v. the glycan polymer preparation further comprises glycan polymers comprising glucose (e.g., a ara-glu preparation);

vi. the glycan polymer preparation further comprises glycan polymers comprising galactose (e.g., a ara-gal preparation); and

vii. the glycan polymer preparation further comprises glycan polymers comprising one of, two of, or three of xylose, glucose, or galactose.

386. The method of any of paragraphs 254-306, wherein the glycan polymers and/or glycan polymer preparation comprise one, two, three, or more, e.g., all, of the following features:

i. glycan polymers comprise galactose and at least one alpha-glycosidic bond, optionally wherein the alpha-glycosidic bond is alpha-1,3 glycosidic bond, further optionally wherein the mean degree of polymerization (DP) of the preparation is between DP2-4, DP2-6, DP3-10 or between DP3-15;

ii. the glycan polymer preparation further comprises glycan polymers comprising alpha-1,2 glycosidic bond, alpha-1,4 glycosidic bond, alpha-1,6 glycosidic bond, or a combination thereof;

iii. the glycan polymer preparation further comprises glycan polymers comprising at least one beta-glycosidic bond;

iv. the glycan polymer preparation further comprises glycan polymers comprising xylose (e.g., a gal-xyl preparation);

v. the glycan polymer preparation further comprises glycan polymers comprising arabinose (e.g., a gal-ara preparation);

vi. the glycan polymer preparation further comprises glycan polymers comprising glucose (e.g., a gal-glu preparation); and

vii. the glycan polymer preparation further comprises glycan polymers comprising one of, two of, or three of xylose, arabinose, or glucose.

387. The method of any of paragraphs 351, 352, or 357-362 wherein the glycan polymer is a substrate for a human gut microbe glycosidase enzyme selected from one or more of, e.g., two, three, four, or more of, GT5, GH94, GH13 subfamily 9, GH13 subfamily 39, GH13 subfamily 36, GH113 or GH112 CAZy family.

388. The method of any of paragraphs 351, 352, or 357-362, wherein the glycan polymer is a substrate for a human gut microbe glycosidase enzyme selected from one or more of, e.g., two, three, four, or more of, GT2, GT4, GT5, GT35, GT51, GH1, GH2, GH3, GH4, GH13, GH13 subfamily 9, GH13 subfamily 31, GH18, GH23, GH25, GH28, GH31, GH32, GH36, GH51, GH73, GH77, or GH94 CAZy family.

389. The method of any one of paragraphs 353, 354, or 363-370, wherein the glycan polymer is a substrate for a human gut microbe glycosidase enzyme selected from one or more of, e.g., two, three, four, or more of, GT11, GT10, GH92, GH51, GH35, GH29, GH28, GH20, GH130, GH13 subfamily 8, or GH13 subfamily 14 CAZy family.

390. The method of any one of paragraphs 353, 354, or 363-370, wherein the glycan polymer is a substrate for a human gut microbe glycosidase enzyme selected from one or more of, e.g., two, three, four, or more of, GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, or GH77 CAZy family.

391. The method of any of paragraphs 355, 356, or 371-373, wherein the glycan polymer is a substrate for a human gut microbe glycosidase enzyme selected from one or more of, e.g., two, three, four, or more of, GT3, GH97, GH43 subfamily 24, GH27, GH133, GH13 subfamily 8, or GH13 CAZy family.

392. The method of any of paragraphs 355, 356, or 371-373, wherein the glycan polymer is a substrate for a human gut microbe glycosidase enzyme selected from one or more of, e.g., two, three, four, or more of, GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, or GH24 CAZy family.

393. The method of any of paragraphs 349, 350, or 374-377, wherein the glycan polymer is a substrate for a human gut microbe glycosidase enzyme selected from one or more of, e.g., two, three, four, or more of, GH13 subfamily 3, GH13 subfamily 30, GH30 subfamily 2, GH30 subfamily 5, GH43 subfamily 22, GH43 subfamily 8, or GH84 CAZy family.

394. The method of any of paragraphs 349, 350, or 374-377, wherein the glycan polymer is a substrate for a glycosidase enzyme selected from one or more of, e.g., two, three, four, or more of, GH3, GH106, GH105, GH2, GH20, GH28, GH76, GH97, or GH92 CAZy family.

395. The method of any of paragraphs 349, 350, or 378, wherein the glycan polymer is a substrate for a human gut microbe glycosidase enzyme selected from one or more of, e.g., two, three, four, or more of, GH13 subfamily 19, GH13 subfamily 21, GH23, GH33, GH37 or GH104 CAZy family.

396. The method of any of paragraphs 349, 350, or 378, wherein the glycan polymer is a substrate for a human gut microbe glycosidase enzyme selected from one or more of, e.g., two, three, four, or more of, GH23, GH24, or GH33 CAZy family.

397. The method of any of paragraphs 349, 350, or 379-382, wherein the glycan polymer is a substrate for a human gut microbe glycosidase enzyme selected from one or more of, e.g., two, three, four, or more of, GH13 subfamily 20, GH13 subfamily 31, GH13 subfamily 39, GH39, GH43 subfamily 11, GH5 subfamily 44, or GH94 CAZy family.

398. The method of any of paragraphs 349, 350, or 379-382, wherein the glycan polymer is a substrate for a human gut microbe glycosidase enzyme selected from one or more of, e.g., two, three, four, or more of, GH2, GH31, GH23, GH13, or GH24 CAZy family.

399. The method of any of paragraphs 349, 350, or 383-386, wherein the glycan polymer is a substrate for a human gut microbe glycosidase enzyme selected from one or more of, e.g., two, three, four, or more of, GH13 subfamily 3, GH13 subfamily 30, GH121, GH15, GH43 subfamily 27, GH43 subfamily 34, or GH43 subfamily 8 CAZy family.

400. The method of any of paragraphs 349, 350, or 383-386, wherein the glycan polymer is a substrate for a human gut microbe glycosidase enzyme selected from one or more of, e.g., two, three, four, or more of, GH92, GH97, GH76, GH28, GH20, GH105, GH2, GH50, GH3, or GH106 CAZy family.

401. A method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT5, GH94, GH13 subfamily 9, GH13 subfamily 39, GH13 subfamily 36, GH113 or GH112 CAZy family;

under conditions that result in making a glycan polymer preparation, wherein a glycan polymer of the preparation is a substrate for a human gut microbe comprising a glycosidase enzyme of a GT5, GH94, GH13 subfamily 9, GH13 subfamily 39, GH13 subfamily 36, GH113 or GH112 CAZy family.

402. A method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT2, GT4, GT5, GT35, GT51, GH1, GH2, GH3, GH4, GH13.0, GH13.9, GH13.31, GH18, GH23, GH25, GH28, GH31, GH32, GH36, GH51, GH73, GH77, or GH94 CAZy family,

under conditions that result in making a glycan polymer preparation, wherein a glycan polymer of the preparation is a substrate for a human gut microbe comprising a glycosidase enzyme of a GT2, GT4, GT5, GT35, GT51, GH1, GH2, GH3, GH4, GH13.0, GH13.9, GH13.31, GH18, GH23, GH25, GH28, GH31, GH32, GH36, GH51, GH73, GH77, GH94 CAZy family.

403. A method of making a glycan polymer preparation, comprising:

providing a plurality of xylose, arabinose, galactose and/or glucose containing glycan subunits (e.g., monomers or dimers);

contacting the plurality of glycan subunits with a glycosidase enzyme selected from one of GH13 subfamily 3, GH13 subfamily 30, GH30 subfamily 2, GH30 subfamily 5, GH43 subfamily 22, GH43 subfamily 8, or GH84 CAZy family,

under conditions that result in making a glycan polymer preparation, wherein a glycan polymer of the preparation is a substrate for a human gut microbe comprising a glycosidase enzyme of a GH13 subfamily 3, GH13 subfamily 30, GH30 subfamily 2, GH30 subfamily 5, GH43 subfamily 22, GH43 subfamily 8, or GH84 CAZy family.

404. A method of making a glycan polymer preparation, comprising:

providing a plurality of xylose, arabinose, galactose and/or glucose containing glycan subunits (e.g., monomers or dimers);

contacting the plurality of glycan subunits with a glycosidase enzyme selected from one of GH3, GH106, GH105, GH2, GH20, GH28, GH76, GH97, or GH92 CAZy family,

under conditions that result in making a glycan polymer preparation, wherein a glycan polymer of the preparation is a substrate for a human gut microbe comprising a glycosidase enzyme of a GH3, GH106, GH105, GH2, GH20, GH28, GH76, GH97, or GH92 CAZy family.

405. A method of making a glycan polymer preparation, comprising:

providing a plurality of glucose and/or sialic acid containing glycan subunits (e.g., monomers or dimers);

contacting the plurality of glycan subunits with a glycosidase enzyme selected from one of GH13 subfamily 19, GH13 subfamily 21, GH23, GH33, GH37 or GH104 CAZy family,

under conditions that result in making a glycan polymer preparation, wherein a glycan polymer of the preparation is a substrate for a human gut microbe comprising a glycosidase enzyme of a GH13 subfamily 19, GH13 subfamily 21, GH23, GH33, GH37 or GH104 CAZy family.

406. A method of making a glycan polymer preparation, comprising:

providing a plurality of glucose and/or sialic acid containing glycan subunits (e.g., monomers or dimers);

contacting the plurality of glycan subunits with a glycosidase enzyme selected from one of GH23, GH24, or GH33 CAZy family,

under conditions that result in making a glycan polymer preparation, wherein a glycan polymer of the preparation is a substrate for a human gut microbe comprising a glycosidase enzyme of a GH23, GH24, or GH33 CAZy family.

407. A method of making a glycan polymer preparation, comprising:

providing a plurality of glucose, xylose, mannose, arabinose, and/or galactose containing glycan subunits (e.g., monomers or dimers);

contacting the plurality of glycan subunits with a glycosidase enzyme selected from one of GH13 subfamily 20, GH13 subfamily 31, GH13 subfamily 39, GH39, GH43 subfamily 11, GH5 subfamily 44, or GH94 CAZy family,

under conditions that result in making a glycan polymer preparation, wherein a glycan polymer of the preparation is a substrate for a human gut microbe comprising a glycosidase enzyme of a GH13 subfamily 20, GH13 subfamily 31, GH13 subfamily 39, GH39, GH43 subfamily 11, GH5 subfamily 44, or GH94 CAZy family.

408. A method of making a glycan polymer preparation, comprising:

providing a plurality of glucose, xylose, mannose, arabinose, and/or galactose containing glycan subunits (e.g., monomers or dimers);

contacting the plurality of glycan subunits with a glycosidase enzyme selected from one of GH2, GH31, GH23, GH13, or GH24 CAZy family,

under conditions that result in making a glycan polymer preparation, wherein a glycan polymer of the preparation is a substrate for a human gut microbe comprising a glycosidase enzyme of a GH2, GH31, GH23, GH13, or GH24 CAZy family.

409. A method of making a glycan polymer preparation, comprising:

providing a plurality of glucose, xylose, arabinose, and/or galactose containing glycan subunits (e.g., monomers or dimers);

contacting the plurality of glycan subunits with a glycosidase enzyme selected from one of GH13 subfamily 3, GH13 subfamily 30, GH121, GH15, GH43 subfamily 27, GH43 subfamily 34, or GH43 subfamily 8 CAZy family,

under conditions that result in making a glycan polymer preparation, wherein a glycan polymer of the preparation is a substrate for a human gut microbe comprising a glycosidase enzyme of a GH13 subfamily 3, GH13 subfamily 30, GH121, GH15, GH43 subfamily 27, GH43 subfamily 34, or GH43 subfamily 8 CAZy family.

410. A method of making a glycan polymer preparation, comprising:

providing a plurality of glucose, xylose, arabinose, and/or galactose containing glycan subunits (e.g., monomers or dimers);

contacting the plurality of glycan subunits with a glycosidase enzyme selected from one of GH92, GH97, GH76, GH28, GH20, GH105, GH2, GH50, GH3, or GH106 CAZy family,

under conditions that result in making a glycan polymer preparation, wherein a glycan polymer of the preparation is a substrate for a human gut microbe comprising a glycosidase enzyme of a GH92, GH97, GH76, GH28, GH20, GH105, GH2, GH50, GH3, or GH106 CAZy family.

411. A method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT11, GT10, GH92, GH51, GH35, GH29, GH28, GH20, GH130, GH13 subfamily 8, GH13 subfamily 14 CAZy family

under conditions that result in making a glycan polymer preparation, wherein a glycan polymer of the preparation is a substrate for a human gut microbe comprising a glycosidase enzyme of a GT11, GT10, GH92, GH51, GH35, GH29, GH28, GH20, GH130, GH13 subfamily 8, GH13 subfamily 14 CAZy family.

412. A method of making a glycan polymer preparation, comprising:

providing a plurality of glucose, mannose, and/or galactose containing glycan subunits (e.g., monomers or dimers);

contacting the plurality of glycan subunits with a glycosidase enzyme selected from one of GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family,

under conditions that result in making a glycan polymer preparation, wherein a glycan polymer of the preparation is a substrate for a human gut microbe comprising a glycosidase enzyme of a GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family.

413. A method of making a glycan polymer preparation, comprising:

providing a plurality of xylose, arabinose, fucose and/or rhamnose containing glycan subunits (e.g., monomers or dimers);

contacting the plurality of glycan subunits with a glycosidase enzyme selected from one of GT11, GT10, GH92, GH51, GH35, GH29, GH28, GH20, GH130, GH13 subfamily 8, GH13 subfamily 14 CAZy family

under conditions that result in making a glycan polymer preparation, wherein a glycan polymer of the preparation is a substrate for a human gut microbe comprising a glycosidase enzyme of a GT11, GT10, GH92, GH51, GH35, GH29, GH28, GH20, GH130, GH13 subfamily 8, GH13 subfamily 14 CAZy family.

414. A method of making a glycan polymer preparation, comprising:

providing a plurality of glycan subunits of a substrate of column E of Table 23, e.g., monomers or dimers; contacting the plurality of glycan subunits of a substrate with a glycosidase enzyme of column A of the same row as the substrate;

under conditions that result in making a glycan polymer preparation, e.g., conditions of columns F, G, H, I, J, K, and/or L of the same row as the substrate and glycosidase enzyme.

415. The method of paragraph 414, wherein the glycan polymer preparation has a mean DP of between about 2 and 4 or between about 2 and 5.

416. The method of either of paragraphs 414 or 415, wherein the glycan polymer preparation comprises at least 20%, 30%, 40%, 50% or at least 60% (mol%) 1,4 glycosidic bonds.

417. The method of either of paragraphs 414 or 415, wherein the glycan polymer preparation comprises at least 80%, 90%, at least 95%, or 100% (mol%) 1,4 glycosidic bonds.

418. The method of either of paragraphs 414 or 415, wherein the glycan polymer preparation comprises at least 20%, 30%, 40%, 50% or at least 60% (mol%) 1,6 glycosidic bonds.

419. The method of either of paragraphs 414 or 415, wherein the glycan polymer preparation comprises at least 80%, 90%, at least 95%, or 100% (mol%) 1,6 glycosidic bonds.

420. The method of either of paragraphs 414 or 415, wherein the glycan polymer preparation comprises no more than 10%, 5%, no more than 1% or 0% 1,2 glycosidic bonds.

421. The method of either of paragraphs 414 or 415, wherein the glycan polymer preparation comprises no more than 10%, 5%, no more than 1% or 0% 1,3 glycosidic bonds.

422. The method of either of paragraphs 414 or 415, wherein the glycan polymer preparation comprises no more than 10%, 5%, no more than 1% or 0% 1,4 glycosidic bonds.

423. The method of either of paragraphs 414 or 415, wherein the glycan polymer preparation comprises no more than 10%, 5%, no more than 1% or 0% 1,6 glycosidic bonds.

424. The method of either of paragraphs 414 or 415, wherein the glycosidic bond distribution (mol%) is one of:

a) alpha-1,2 less than 10%, alpha 1,3 less than 10%, alpha 1,4 at least 30%, alpha 1,6 at least 30%, beta 1,2 less than 5%, beta 1,3 less than 5%, beta 1,4/1,6 less than 5%,
b) alpha-1,2 less than 5%, alpha 1,3 less than 5%, alpha 1,4 at least 5%, alpha 1,6 less than 5%, beta 1,2 at least 1%, beta 1,3 at least 1%, beta 1,4/1,6 at least 85%,
c) alpha-1,2 less than 5%, alpha 1,3 less than 5%, alpha 1,4 less than 5%, alpha 1,6 at least 85%, beta 1,2 less than 5%, beta 1,3 less than 5%, beta 1,4/1,6 less than 5%,
d) alpha-1,2 less than 10%, alpha 1,3 less than 5%, alpha 1,4 at least 15%, alpha 1,6 at least 50%, beta 1,2 less than 5%, beta 1,3 less than 5%, beta 1,4/1,6 less than 5%,
e) alpha-1,2 less than 5%, alpha 1,3 less than 5%, alpha 1,4 less than 15%, alpha 1,6 at least 85%, beta 1,2 less than 5%, beta 1,3 less than 5%, beta 1,4/1,6 less than 5%,
f) alpha-1,2 less than 5%, alpha 1,3 less than 5%, alpha 1,4 less than 5%, alpha 1,6 less than 5%, beta 1,2 less than 5%, beta 1,3 less than 5%, beta 1,4/1,6 at least 85%,
g) alpha-1,2 less than 5%, alpha 1,3 less than 5%, alpha 1,4 at least 50%, alpha 1,6 at least 5%, beta 1,2 less than 10%, beta 1,3 less than 5%, beta 1,4/1,6 at least 10%.

425. A method of making a glycan polymer preparation, comprising:

providing a plurality of xylose, arabinose, fucose and/or rhamnose containing glycan subunits (e.g., monomers or dimers);

contacting the plurality of glycan subunits with a glycosidase enzyme selected from one of GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family,

under conditions that result in making a glycan polymer preparation, wherein a glycan polymer of the preparation is a substrate for a human gut microbe comprising a glycosidase enzyme of a GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77 CAZy family.

426. A method of making a glycan polymer preparation, comprising:

providing a plurality of glucose and/or galactose containing glycan subunits (e.g., monomers or dimers);

contacting the plurality of glycan subunits with a glycosidase enzyme selected from one of GT3, GH97, GH43 subfamily 24, GH27, GH133, GH13 subfamily 8, GH13 CAZy family,

under conditions that result in making a glycan polymer preparation, wherein a glycan polymer of the preparation is a substrate for a human gut microbe comprising a glycosidase enzyme of a GT3, GH97, GH43 subfamily 24, GH27, GH133, GH13 subfamily 8, GH13 CAZy family.

427. A method of making a glycan polymer preparation, comprising:

providing a plurality of glucose and/or galactose containing glycan subunits (e.g., monomers or dimers);

contacting the plurality of glycan subunits with a glycosidase enzyme selected from one of GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, GH24 CAZy family,

under conditions that result in making a glycan polymer preparation, wherein a glycan polymer of the preparation is a substrate for a human gut microbe comprising a glycosidase enzyme of a GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, GH77, GT2, GT4, GH2, GH23, GH3, GT51, GH1, GT8, GH92, GT9, GH73, GH31, GH20, Gh28, GT35, GT28, GH18, GH13, GH97, GH25, GH36, GH4, GH105, GH32, GH78, GH29, GH0, GT25, GH51, GH77, GH88, GH24 CAZy family.

428. The method of any one of paragraphs 401-413 or 425-427, wherein the glycosidase enzyme or the glycosidase enzyme molecule is other than one or more of: GH1, GH2, GH3, GH35, GH42, and GH50.

429. The method of any one of paragraphs 401-413 or 425-427, wherein the glycosidase enzyme or the glycosidase enzyme molecule is other than one or more of: GH32, GH68, GH100.

430. The method of any one of paragraphs 401-413 or 425-427, wherein the glycosidase enzyme or the glycosidase enzyme molecule is other than one or more of: GH1, GH2, GH3, GH4, GH5, GH8, GH9, GH10, GH11, GH12, GH13, GH14, GH16, GH26, GH28, GH30, GH31, GH32, GH35, GH42, GH43, GH44, GH50, GH51, GH57, GH62, GH63, GH68, GH70, GH97, GH100, GH116, GH119, GH122

431. A glycan polymer preparation made by, producible by, or makeable by, a method disclosed herein, e.g., by the method of any of paragraphs 254-430.

432. A glycan polymer preparation selected by, or selectable by, a method disclosed herein, e.g., by the method of any of paragraphs 254-430.

433. The glycan polymer preparation of paragraph 431, formulated as a pharmaceutical composition, a medical food, a dietary supplement, a food ingredient, or a therapeutic nutrition product.

434. The glycan polymer preparation of paragraph 431 further comprising an excipient or carrier.

435. A unit dosage from comprising the glycan preparation of any one of paragraphs 431-434.

436. The unit dosage form of paragraph 435 formulated for enteral administration, oral, oral or rectal administration, or for tube feeding.

437. The unit dosage form of either of paragraphs 435 or 436 formulated as a powder or syrup.

438. The unit dosage form of any one of paragraphs 435-437 formulated for timed and/or targeted release in the colon or large intestine.

439. A pharmaceutical composition comprising the glycan polymer preparation of any one of paragraphs 431-434.

440. A medical food comprising the glycan polymer preparation of any one of paragraphs 431-434.

441. A dietary supplement comprising the glycan polymer preparation of any one of paragraphs 431-434.

442. A food ingredient comprising the glycan polymer preparation of any one of paragraphs 431-434.

443. A therapeutic nutrition product comprising the glycan polymer preparation of any one of paragraphs 431-434.

444. A reaction mixture, described herein, e.g., generated by any one of the methods of paragraphs 254-430, comprising:

> a plurality of glycan subunits, e.g., a sugar monomer or a sugar dimer, suitable for the production of the glycan polymer; and

> a glycosidase enzyme molecule (e.g., Tables 4 (column 2), 23 (column A), 24 (column A), or 22 (column 1); or one or more glycosidase enzymes associated with glycotaxa class 1, class 2, class3, class 4, class 5, class 6, or class 7),

in amounts suitable to produce a glycan polymer preparation comprising at least 0.25, 0.5, 1, 5, 10, 20, 50, 100, 200, 300, 400 or 500 kilograms of glycan polymer and/or under conditions suitable to obtain a yield of at least about 15%, 30%, 45%, 60%, or of about 75% (as determined on a weight/weight basis as a % of input glycan subunits).

445. The reaction mixture of paragraph 444, suitable for practice of a method described herein, e.g., the method of any of paragraphs 254-430.

446. A method of making a pharmaceutical composition, a medical food, a dietary supplement, a food ingredient, or a therapeutic nutrition product, comprising formulating the preparation of paragraph 431 into a pharmaceutical composition, a medical food, a dietary supplement, a food ingredient, or a therapeutic nutrition product.

447. The method of paragraph 446, comprising dividing the preparation into a plurality of portions, e.g., unit dosages or formulations, e.g., at least 10, 100 or at least 1,000 portions.

448. The method of paragraph 446, comprising combining the preparation with an excipient.

449. A glycan polymer preparation, or a portion thereof, of paragraph 431.

450. A fraction, e.g., a molecular weight fraction, of the glycan polymer preparation of paragraph 431.

451. The molecular weight fraction of paragraph 450, wherein the fraction comprises an average DP which differs from that of the glycan preparation, e.g., an average DP of about 3, 4, or 5.

452. A method of making, evaluating, selecting, classifying, or providing a preparation of a glycan polymer made or makeable by a method of any of paragraphs 254-430comprising

> acquiring a candidate preparation;

> acquiring, e.g., by performing an assay, a value for a parameter related to the preparation, e.g., a physical parameter, e.g., molecular weight, e.g., average molecular weight or molecular weight distribution, glycan subunit composition, or purity or a parameter related to a biological property, e.g., the ability to modulate growth of the human gut microbe, the ability to modulate a microbial metabolite produced by a microbe, e.g., in an ex vivo assay, or the ability to modulate a biomarker, e.g., an inflammatory or immune biomarker, a toxic or waste compound, a bacterial compound) e.g., in a human subject; and

comparing the value with a reference value;

thereby making, evaluating, selecting, classifying, or providing a preparation of a glycan polymer.

453. The method of paragraph 452, comprising performing an assay to acquire the value.

454. The method of paragraph 452, comprising acquiring the value from another party.

455. The method of any of paragraphs 452-454, wherein the value is compared with a reference value to evaluate the candidate, e.g., for suitability for use, e.g., as a preparation of a glycan polymer, or for formulation into a product or dosage form, e.g., a product or dosage form described herein.

456. A method of making a pharmaceutical composition that modulates a target human gut microbe, comprising

providing a plurality of glycan subunits;
contacting the glycan subunits of the plurality with a glycosidase enzyme composition having a glycosidase activity present in the target gut microbe, under conditions that result in the incorporation of the glycan subunits into a glycan polymer,
optionally purifying the glycan polymer, and
formulating the glycan polymer as a pharmaceutical composition for administration to the gut and modulation of the gut microbe, thereby making a pharmaceutical composition that modulates the target human gut microbe.

457. A purified preparation of glycosidase enzyme molecules comprising a glycosidase enzyme encoded by a nucleic acid sequence that is at least 80, 85, 90, 91, 92, 93, 94, 95, 96, 97, 98, 99, or 100% identical to a nucleic acid sequence selected from one or more of SEQ ID NOs: 1-124,
wherein the glycosidase enzyme is present in a human gut microbe.

458. A vector comprising a nucleic acid sequence that is at least 80, 85, 90, 91, 92, 93, 94, 95, 96, 97, 98, 99, or 100% identical to a nucleic acid sequence selected from one or more of SEQ ID NOs: 1-124, wherein the nucleic acid encodes a glycosidase enzyme present in a human gut microbe, and wherein the vector is capable of being used to express the glycosidase enzyme.

459. A reaction mixture comprising:

a glycosidase enzyme encoded by a nucleic acid sequence selected from one or more of SEQ ID NOs: 1-124, and a substrate, e.g., glycan subunits, e.g., monomers or dimers, of the glycosidase enzyme,
wherein the substrate is present in a sufficient amount to form, e.g., by condensation, a glycan polymer.

**EXAMPLES**

The invention is further illustrated by the following examples. The examples are provided for illustrative purposes only, and are not to be construed as limiting the scope or content of the invention in any way. The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); Green & Sambrook et al., Molecular Cloning: A Laboratory Manual, 4th Edition (Cold Spring Harbor Laboratory Press, 2012); Colowick & Kaplan, Methods In Enzymology (Academic Press); Remington: The Science and Practice of Pharmacy, 22nd Edition (Pharmaceutical Press, 2012); Sundberg & Carey, Advanced Organic Chemistry: Parts A and B, 5th Edition (Springer, 2007).

**Example 1. Method for producing glycan polymers using a purified glycosidase enzyme**

Glycans may be generated by reverse hydrolysis of glycosidic bonds catalyzed by one or more partially or fully purified hydrolase or transferase enzymes, described herein, e.g., Tables 4, 22, 23 and 24 and, e.g., encoded by SEQ ID Nos 1-124. The enzyme(s) selected may be any enzyme known to hydrolyze glycosidic bonds including glycosylhydrolases, glycosyltransferases, and polysaccharide lyases including those that use unactivated sugar donors or activated sugar donors including but not limited to sugar nucleotides and phosphoryl sugars. Ideally, the selected enzyme is isolated from or related to a glycosyl hydrolase from a glycotaxa (e.g., class 1, class 2, class 3, class 4, class 5, class 6, or class 7) with a positive effect on the structure or health of the host microbiome (e.g., the modulation of a metabolite, e.g., SCFA (e.g., butyrate, propionate), TMA/TMAO, ammonia, uremic solute (e.g., indole, p-cresol), LPS, or a bile acid (e.g., a secondary bile acid) .

The glycosyl donor may consist of an unactivated monomeric glycoside (e.g. glucose, galactose, glucuronic acid, etc.), an activated monomeric glycoside (e.g. phosphoglucose, lactose, maltobiose, UDP-glucose, 1-fluoroglucose, trichloroacetimidated glucose, *para*nitrophenylglucose, hexeneuronic acid), or oligomeric or polymeric glycoside (e.g. maltose, galactooligosaccharides).

The glycosyl acceptor may consist of any substrate compatible with glycosyl hydrolases or transferases including monomeric sugars (e.g. glucose, galactose, glucuronic acid), oligomers (e.g. lactose, maltobiose, galactooligosaccharides), or non-sugar substrates reported to be compatible with other glycosides (e.g. sorbitol, glycerol). In some embodiments, the glycosyl acceptor and the glycosyl donor may be the same material, e.g. maltobiose may be both a donor and an acceptor.

The enzyme of interest may be dissolved or suspended to a concentration of 1-50 U/mL in a biocompatible solvent including water; mixtures of water and a miscible solvent such as acetone, ethanol, isopropanol, polyethylene glycol, t-butanol or other reported solvents; or a pure organic solvent. Glycosyl donor and acceptor are added to the media in a concentration between 5% and 50% by weight. The pH of the media is adjusted to a range between 2.5 and 8.0 using a biocompatible buffer, e.g. sodium acetate buffer, citric acid/disodium hydrogen phosphate buffer, phosphate buffer, etc. The reaction is then allowed to stir gently for 2-48 hr at a temperature compatible with the selected glycosidase enzyme molecule, typically 35 °C to 90 °C.

Conditions for synthesis using a glycosidase enzyme molecule that is isolated with minimal structural changes from its host or expressed from a recombinant system may more closely reflect native physiology, i.e. pH between 5 and 7.5 and a temperature between 35 and 60 °C. Ideally, conditions for synthesis using a glycosidase enzyme molecule that has been modified to improve its stability may deviate further from physiological conditions, e.g., pH between 3.5 and 8 and a temperature between 35 and 70 °C. Changes in protein stability may be used to increase desirable properties including the synthetic yield, conversion rate, recyclability, or protein production yield.

Ideally, conditions for synthesis using a glycosidase enzyme molecule that has been highly modified or isolated from extremophile bacteria evolved to withstand extreme conditions of temperature or pH may deviate broadly from physiological conditions, e.g., pH between 2.0 and 8 and a temperature up to 90 °C.

In one embodiment, the selected substrate (Table 23, column E) was placed in a 20-ml scintillation vial. McIlvaine buffer of specified pH (Table 23, column J) was added to the vial and the powder dissolved by a brief heating with a heat gun. After the buffered substrate solution was cooled to room temperature, the selected enzyme was added (Table 23, column H), the solution was swirled gently to mix, and the capped vial was placed in a water bath at the specified temperature (Table 23, column K), unstirred. Once the specified time elapsed (Table 23, column L), the vial was heated in a boiling water bath for 20 min to inactivate the enzyme. The samples were then transferred into 50 ml conical centrifuge tubes and diluted down to ~200 mg/ml. The diluted products were then purified by the methods described in Example 18. Isolated materials were characterized as described in Examples 11-15 and data are presented in Table 23 (SEC) and Table 24 (HSQC-NMR).

**Table 23. Conditions for glycan synthesis using an enzyme catalyst.**

| | A Enzyme Annotation | B EC# | C Family | D source | E substrate | F Substrate Mass (g) | G buffer vol. (ml) | H Enzyme Mass (mg) | I total vol. (ml) | J reaction pH (0.2M disodium phosphate/0.1M citric acid) | K T (°C) | L time (h) | M DP3+ conversion | N Mn | O Mw | P PDI | Q Avg DP |
|---|---|---|---|---|---|---|---|---|---|---|---|---|---|---|---|---|---|
| 2 | oligo-alpha-(1-4,6)-glucosidase, recombinant | 3.2.1.10 | GH13 | Bacillus sp. (potentially Human) | sucrose | 1.3 | 1.2 | 1.8 | 1.3 | 7 | 40 | 24 | 30% | 395 | 409 | 1.0 | 2.4 |
| 3 | oligo-alpha-(1-4,6)-glucosidase, recombinant | 3.2.1.10 | GH13 | Bacillus sp. (potentially Human) | isomaltose | 1 | 0.9 | 1.4 | 1 | 7 | 40 | 24 | 40% | 513 | 524 | 1.0 | 3.1 |
| 4 | Transglucosidase | 3.2.1.20 | GH31 | Aspergillus niger | maltose | 1.0 | 1.0 | 0.3 | 1.0 | 4.5 | 50 | 4 | 40% | 533 | 563 | 1.1 | 3.4 |
| 5 | Transglucosidase | 3.2.1.20 | GH31 | Aspergillus niger | melezitose | 2.0 | 2.0 | 0.6 | 2.0 | 4.5 | 50 | 8 | 20% | 507 | 524 | 1.0 | 3.1 |
| 6 | Transglucosidase | 3.2.1.20 | GH31 | Aspergillus niger | isomaltose | 1 | 1.0 | 0.3 | 1 | 4.5 | 50 | 4 | 40% | 518 | 532 | 1.0 | 32 |
| 7 | beta-glucosidase, recombinant | 3.2.1.21 | GH1 | Agrobacterium sp. | gentiobiose | 2.5 | 2.2 | 0.8 | 2.5 | 6.5 | 50 | 24 | 16% | | | | |
| 8 | beta-glucosidase, recombinant | 3.2.1.21 | GH3 | Phaerochaete chrysosporium | cellobiose | 2.7 | 13 | 1.3 | 13 | 4.5 | 60 | 1 | 15% | 467 | 487 | 1.0 | 2.9 |
| 9 | beta-glucosidase, recombinant | 3.2.1.21 | GH3 | Phaerochaete chrysosporium | gentiobiose | 2 | 2.0 | 0.4 | 2 | 4.5 | 60 | 4 | 20% | | | | |
| 10 | beta-glucosidase | 3.2.1.21 | GH3 | Aspergillus niger | cellobiose | 1.3 | 6.5 | 0.1 | 6.7 | 4 | 50 | 1.5 | 30% | 477 | 490 | 1.0 | 2.9 |
| 11 | beta-glucosidase | 3.2.1.21 | GH3 | Aspergillus niger | gentiobiose | 2 | 1.5 | 0.3 | 2 | 4 | 50 | 1.5 | 20% | | | | |

| | A | B | C | D | E | F | G | H | I | J | K | L | M | N | O | P | Q |
|---|---|---|---|---|---|---|---|---|---|---|---|---|---|---|---|---|---|
| 12 | alpha-galac-tosidase | 3.2.1.22 | GH27 | Cyamopsis tetragonoloba | melibiose | 3.3 | 3.1 | 2.4 | 3.3 | 4.5 | 40 | 24 | 12% | 478 | 551 | 12 | 3.3 |
| 13 | alpha-galactosidase | 3.2.1.22 | GH27 | Cyamopsis tetragonoloba | raffinose | 3.2 | 2.9 | 2.9 | 3.2 | 4.5 | 40 | 8 | 13% | 550 | 578 | 1.1 | 3.5 |
| 14 | alpha-galac-tosidase, re-combinant | 3.2.1.22 | GH27 | Penicillium simplicissimu m | melibiose | 3.3 | 3.2 | 0.8 | 3.3 | 4 | 40 | 24 | 12% | 430 | 450 | 1.0 | 2.7 |
| 15 | alpha-galac-tosidase, re-combinant | 3.2.1.22 | GH27 | Penicillium simplicissimu m | raffinose | 4.4 | 4.3 | 1.1 | 4.4 | 4 | 40 | 8 | 9% | | | | |
| 16 | alpha-galac-tosidase, re-combinant | 3.2.1.22 | GH36 | Lachnospirace ae_bacterium_ 6 1 63FAA | melibiose | 2.7 | 2.6 | 0.6 | 2.7 | 5.6 | 40 | 24 | 15% | 500 | 535 | 1.1 | 32 |
| | | | | (Human) SEQ ID NOS: 57, 72 | | | | | | | | | | | | | |
| 17 | alpha-galac-tosidase, re-combinant | 3.2.1.22 | GH36 | Lachnospirace ae_bacterium_ 6_1_63FAA (Human) SEQ ID NOS: 57, 72 | raffinose | 4.0 | 3.9 | 0.9 | 4.0 | 5.6 | 40 | 6 | 10% | 520 | 537 | 1.0 | 3.2 |
| 18 | lpha-galac-tosidase, re-combinant | 3.2.1.22 | GH36 | Lachnospirace ae_bacterium_ 2_1_58FAA (Human) SEQ ID NOS: 57, 72 | melibiose | 6.7 | 6.5 | 1.6 | 6.7 | 5.6 | 40 | 8 | 6% | 504 | 530 | 1.1 | 3.2 |

| | A | B | C | D | E | F | G | H | I | J | K | L | M | N | O | P | Q |
|---|---|---|---|---|---|---|---|---|---|---|---|---|---|---|---|---|---|
| 19 | a alpha-ga-lactosidase, recombinant | 3.2.1.23 | GH42 | Bifidobacterium_longum_subsp._ infantis_ ATCC_15697 _= _JCM 122 2 = DSM 20 088 (Human) SEQ ID NOS: 38, 39 | lactose | 2.7 | 6.6 | 0.4 | 6.7 | 5.6 | 40 | 6 | 15% | 508 | 522 | 1.0 | 3.1 |
| 20 | alpha-galac-tosidase, re-combinant | 3.2.1.23 | GH42 | Bifidobacterium_longum_subsp._infantis _ ATCC_15697 _=_JCM_122 2_=_DSM_20 088 (Human) SEQ ID NOS: 38, 39 | lactulose | 1.3 | 1.3 | 0.1 | 1.3 | 5.6 | 40 | 6 | 30% | 508 | 520 | 1.0 | 3.1 |
| 21 | alpha-galac-tosidase, re-combinant | 3.2.1.23 | GH42 | Klebsiella sp. 4_1_44FAA (Human) SEQ ID NOS: 49, 83, 84, 92, 93 | lactulose | 5.0 | 4.5 | 3.0 | 5.0 | 5.6 | 40 | 24 | 8% | 570 | 598 | 1.0 | 3.6 |
| 22 | alpha-glu-cosidase, re-combinant | 3.2.1.21 | GH1 | Ruminococcus _champanellensis_18P13_= JCM 17042 | gentiobiose | 4 | 3.8 | 3.0 | 4 | 5.6 | 40 | 6 | 10% | | | | |
| | | | | (Human) SEQ ID NO: 31 | | | | | | | | | | | | | |
| 23 | beta-glucosi-dase, recom-binant | 3.2.1.21 | GH3 | Bacteroides_sp._D20 (Human) SEQ ID NOS: 12, | gentiobiose | 4 | 3.6 | 1.8 | 4 | 5.6 | 40 | 24 | 10% | | | | |

| | A | B | C | D | E | F | G | H | I | J | K | L | M | N | O | P | Q |
|---|---|---|---|---|---|---|---|---|---|---|---|---|---|---|---|---|---|
| | | | | 18,48, 56, 64, 99, 110, 117 | | | | | | | | | | | | | |
| 24 | alpha-glu-cosidase, re-combinant | 3.2.1.20 | GH13 | Bifidobacterium_adolescentis_L2-32 (Human) SEQ ID NOS: 68, 104 i | maltose | 5.7 | 5.7 | 0.6 | 5.7 | 5.6 | 40 | 24 | 7% | 456 | 474 | 1.0 | 2.8 |
| 25 | alpha-glu-cosidase, re-combinant | 3.2.1.20 | GH13 | Bifidobacterium_adolescentis_L2-32 (Human) SEQ ID NOS: 68, 104 | sucrose | 4.0 | 3.8 | 2.0 | 4.0 | 5.6 | 40 | 24 | 10% | 426 | 440 | 1.0 | 2.6 |
| 26 | alpha-glu-cosidase, re-combinant | 3.2.1.20 | GH13 | Bifidobacterium_adolescentis_L2-32 (Human) SEQ ID NOS: 68, 104 | palatinose | 2.7 | 2.6 | 0.3 | 2.7 | 5.6 | 40 | 24 | 15% | 423 | 468 | 1.1 | 2.8 |
| 27 | alpha-glucosidase | 3.2.1.20 | GH13 | Bacillus stearothermop hilus | maltose | 10 | 10 | 1 | 10 | 6.5 | 55 | 5 | 40% | 399 | 416 | 1.0 | 2.5 |
| 28 | alpha-glucosidase | 3.2.1.20 | GH13 | Bacillus stearothermop hilus | sucrose | 10 | 10 | 1 | 10 | 6.5 | 55 | 5 | 22% | 313 | 322 | 1.0 | 1.9 |
| 29 | alpha-galactosidase | 3.2.1.22 | GH36 | Aspergillus niger | melibiose | 4.7 | 4.7 | 0.1 | 4.8 | 5.0 | 60 | 5.8 | 25% | 374 | 390 | 1.0 | 23 |
| 30 | alpha-galactosidase | 3.2.1.22 | GH36 | Aspergillus niger | raffinose | 10 | 10 | 0.2 | 10 | 5.0 | 60 | 5 | 24% | 377 | 406 | 1.1 | 2.4 |
| 31 | beta-galacto sidase | 3.2.1.23 | GH35 | Aspergillus niger | lactose | 4.0 | 10 | 0.4 | 10 | 5.0 | 60 | 4 | 30% | 399 | 416 | 1.0 | 2.5 |
| 32 | beta-galacto sidase | 3.2.1.23 | GH35 | Aspergillus niger | lactulose | 10 | 10 | 0.4 | 10 | 5.0 | 60 | 3.5 | 37% | 313 | 322 | 1.0 | 1.9 |

**Key**: For a given cell in columns F-Q of Table 23, values provided are +/- 10, 20, 30, 40, or 50% of the cell value, greater than or equal to the cell value, or less than or equal to the cell value.

**Table 24. Bond distribution of glycans synthesized by enzymes by HSQC-NMR**

| | A | B | C | D | E | F | G | H | I | J | K | L |
|---|---|---|---|---|---|---|---|---|---|---|---|---|
| 1 | Enzyme Annotation | EC# | Family | source | substrate | alpha-1,2 | alpha - 1,3 | alpha-1,4 | alpha - 1,6 | beta-1,2 | beta-1,3 | beta - 1,4/1,6 |
| 2 | alpha-glucosidase, recombinant | 3.2.1.20 | GH13 | Bifidobacterium_adolescentis_ L2-32 (Human) SEQ ID NOS: 68, 104 | maltose | 73% | 2.6% | 50.5% | 38.6% | 0.0% | 0.0% | 0.9% |
| 3 | alpha-glucosidase, recombinant | 3.2.1.21 | GH1 | Ruminococcus_champanellensis_ 18P13_=_J CM_17042 (Human) SEQ ID NO: 31 | gentiobiose | 0.0% | 0.0% | 0.0% | 0.0% | 2.7% | 1.5% | 95.8% |
| 4 | oligo-alpha-(1-4,6)-g lucosidase, recombinant | 3.2.1.10 | GH13 | Bacillus sp. (potentially Human) | isomaltose | 0.0% | 0.0% | 0.0% | 100.0% | 0.0% | 0.0% | 0.0% |
| 5 | Transglucosid ase | 3.2.1.20 | GH31 | Aspergillus niger | maltose | 4.5% | 0.0% | 23.5% | 68.4% | 1.7% | 0.0% | 1.8% |
| 6 | Transglucosid ase | 3.2.1.20 | GH31 | Aspergillus niger | isomaltose | 1.1% | 0.0% | 5.6% | 93.1% | 0.2% | 0.0% | 0.0% |
| 7 | beta-glucosidase, recombinant | 3.2.1.21 | GH3 | Phaerochaete chrysosporium | gentiobiose | 0.0% | 0.0% | 0.0% | 0.0% | 1.9% | 0.0% | 98.1% |
| 8 | beta-glucosidase, recombinant | 3.2.1.21 | GH3 | Phaerochaete chrysosporium | cellobiose | 0.0% | 0.0% | 0.0% | 0.0% | 3.7% | 2.0% | 94.3% |
| 9 | beta-glucosidase | 3.2.1.21 | GH3 | Aspergillus niger | gentiobiose | 0.0% | 0.0% | 0.0% | 0.0% | 0.2% | 0.0% | 99.8% |
| 10 | beta-glucosidase | 3.2.1.21 | GH3 | Aspergillus niger | cellobiose | 0.0% | 0.0% | 0.0% | 0.0% | 0.4% | 2.0% | 97.5% |
| 11 | alpha-glucosidase | 3.2.1.20 | GH13 | Bacillus stearothermophilus | maltose | 0.0% | 3.0% | 66.2% | 9.0% | 5.1% | 0.1% | 16.6% |

**Key**: For a given cell in columns F-L of Table 24, values provided are +/- 10, 20, 30, 40, or 50% of the cell value, greater than or equal to the cell value, or less than or equal to the cell value.

**Example 2. Generation of beta-galactooligosaccharides via beta-galactosidase and lactose**

To a 0.05 M, pH 5 sodium acetate buffer at 60 °C was added lactose to a concentration of 400 mg/mL. The reaction was agitated gently until the substrates were dissolved. A stock solution of beta-galactosidase (Megazyme, catalog# E-BGLAN) was then added to the reaction to a final concentration of 10 U/ml. The reaction was maintained at 60 °C for 26 hr in a covered water bath. When the reaction was deemed complete it was heated to 100 °C for 10 min to inactivate the enzyme, then diluted with water to a 2% total carbohydrate concentration (Table 23, line 31). The products were then characterized, isolated, and purified as described in Examples 11-18 and shown in FIG. 5. The isolated oligosaccharide has a molecular weight >20% DP≥3, a glycosidic stereochemistry >50% beta-glycosides, a regiochemistry >10% 1,4-glycosidic bonds, and a constituency >50% D-galactose.

**Example 3. Generation of alpha-galactooligosaccharides via alpha-galactosidase and melibiose**

To a 0.05 M, pH 5 sodium acetate buffer at 60 °C was added melibiose to a concentration of 1000 mg/mL. The reaction was agitated gently until the substrates were dissolved. A stock solution of alpha-galactosidase (Megazyme, catalog# E-AGLAN) was then added to the reaction to a final concentration of 10 U/mL. The reaction was maintained at 60 °C for 22 hr in a covered water bath. When the reaction was deemed complete it was heated to 100 °C for 10 min to inactivate the enzyme, then diluted with water to a 2% total carbohydrate concentration (Table 23, line 29). The products were then characterized, isolated, and purified as described in Examples 11-18 (FIG. 8). The isolated oligosaccharide has a molecular weight >20% DP≥3, a glycosidic stereochemistry >50% alpha-glycosides, a regiochemistry >10% 1,4-glycosidic bonds, and a constituency >50% D-galactose.

**Example 4. Generation of beta-glucooligosaccharides via beta-glucosidase and cellobiose**

To a 0.05 M, pH 5 sodium acetate buffer at 60 °C was added cellobiose to a concentration of 200 mg/mL. The reaction was agitated gently until the substrates were dissolved. A stock solution of beta-glucosidase (Megazyme, catalog# E-BGOSPC) was then added to the reaction to a final concentration of 10 U/mL. The reaction was maintained at 60 °C for 26 hr in a covered water bath. When the reaction was deemed complete it was heated to 100 °C for 10 min to inactivate the enzyme, then diluted with water to a 2% total carbohydrate concentration (Table 23, line 10). The products were then characterized, isolated, and purified as described in Examples 11-18 (FIG. 6). The isolated oligosaccharide has a molecular weight >10% DP≥3, a glycosidic stereochemistry >50% beta-glycosides, a regiochemistry >10% 1,4-glycosidic bonds, and a constituency >90% D-glucose (Table 24, line 10).

**Example 5. Generation of alpha-glucooligosaccharides via alpha-glucosidase and maltose**

To a 0.05 M, pH 6.5 sodium maleate buffer at 55 °C was added maltose to a concentration of 1000 mg/mL. The reaction was agitated gently until the substrates were dissolved. A stock solution of alpha-glucosidase (Megazyme, catalog# E-TSAGL) was then added to the reaction to a final concentration of 10 U/mL. The reaction was maintained at 55 °C for 24 hr in a covered water bath. When the reaction was deemed complete it was heated to 100 °C for 10 min to inactivate the enzyme, then diluted with water to a 2% total carbohydrate concentration (Table 23, line 27). The products were then characterized, isolated, and purified as described in Examples 11-18 (FIG. 7A, B). The isolated oligosaccharide has a molecular weight >15% DP≥3, a glycosidic stereochemistry >50% alpha-glycosides, a regiochemistry >10% 1,4-glycosidic bonds, and a constituency >90% D-glucose (Table 24, line 11).

**Example 6. Selective generation of oligosaccharides by treatment of a mixtures of substrates with a selective glycosidase**

Oligosaccharides with mixed compositions (including mixtures of stereochemistries, regiochemistries, and monomeric composition) are generated by treating a mixture of substrates with a glycosidase that is selective for one or more of the substrates under the conditions described in Example 1. In this example, the substrates susceptible to the glycosidase serves as the glycosidic bond donor and the substrates that are not susceptible to the glycosidase only serves as the glycosidic bond acceptor. By mixing different combinations of substrates with differently selective glycosidases, distinct combinations of oligosaccharides may be generated (FIG. 12).

In one embodiment, to a 0.05 M, pH 5 sodium acetate buffer at 60 °C was added a 1:2 w/w mixture of cellobiose and lactose to a final concentration of 600 mg/mL. The reaction was agitated gently until the substrates were dissolved. A stock solution of beta-glucosidase (Megazyme, catalog# E-BGOSPC) was then added to the reaction to a final concentration of 10 U/mL. The reaction was maintained at 60 °C for 26 hr in a covered water bath. When the reaction was deemed complete it was heated to 100 °C for 10 min to inactivate the enzyme, then diluted with water to a 2% total carbohydrate concentration. The products were then characterized, isolated, and purified as described in Examples 11-18 (FIG. 14). The isolated oligosaccharide has a molecular weight >10% DP≥3, a glycosidic stereochemistry >50% beta-glycosides, a regiochemistry >10% 1,4-glycosidic bonds, and a constituency >50% D-glucose and <50% D-galactose.

In a second embodiment, to a 0.05 M, pH 5 sodium acetate buffer at 60 °C was added a 1:2 w/w mixture of cellobiose and lactose to a final concentration of 600 mg/mL. The reaction was agitated gently until the substrates were dissolved. A stock solution of beta-galactosidase (Megazyme, catalog# E-BGLAN) was then added to the reaction to a final concentration of 10 U/ml. The reaction was maintained at 60 °C for 26 hr in a covered water bath. When the reaction was deemed complete it was heated to 100 °C for 10 min to inactivate the enzyme, then diluted with water to a 2% total carbohydrate concentration. The products were then characterized, isolated, and purified as described in Examples 11-18 (FIG. 13). The isolated oligosaccharide has a molecular weight >20% DP≥3, a glycosidic stereochemistry >50% beta-glycosides, a regiochemistry >10% 1,4-glycosidic bonds, and a constituency <50% D-glucose and >50% D-galactose.

In other embodiments, the selected substrates mixture may differ by constituent monomer (e.g. maltose and melibiose or L-arabinobiose and D-arabinobiose), by glycosidic stereochemistry (e.g. maltose and cellobiose), by regiochemistry (e.g. lactose and allo-lactose), by activation strategy (e.g. 1-(para-nitrophenyl)-D-glucose and lactose), or by any combination of these or other factors that differentiate the subtrates of glycosidic enzymes. The selected glycosidases may differ by selectivity for substrate (e.g. glucosidase vs. galactosidase), glycosidic stereochemistry (e.g. alpha-glucosidase vs. beta-glucosidase), regiochemistry (e.g. 1,6-glucosidase vs. 1,3-glucosidase), chain position (e.g. endo-glycosidase vs. exo-glycosidase; reducing end selective vs. non-reducing end selective), or any combination of these or other factors that differentiate glycosidic enzymes.

## Example 7. Generation of oligosaccharides from monomeric sugars using a mixed organic/aqueous solvent system

The glycans described in Example 1 may be generated from monomeric sugars via a thermodynamic reverse hydrolysis event. Sugar hydrolases establish a thermodynamic equilibrium in which monomeric sugars are in constant exchange with higher-order oligosaccharides with the dominant direction of the reaction dictated by the water activity of the environment. The high concentration of water in relation to substrate found under physiological conditions causes these enzymes to be primarily hydrolytic in action. Under certain laboratory conditions, the dominant direction of the reaction can be altered to favor the formation of glycosidic bonds allowing the formation of oligosaccharides from monomeric constituents. Glucose was dissolved into 0.1M, pH 5.2 sodium acetate buffer at a 10% (w/w) concentration. After the substrates were dissolved, four volumes of one of the following organic solvents was added to the aqueous portion: tert-butanol, diethylene glycol dimethyl ether (DEGD), tetraethylene glycol dimethyl ether (TEGD), or trimethyl phosphate (TMP). Beta-glucosidase stock solution was added to the reaction to a concentration of 10 U/ml. The reaction was heated to 50 °C in a covered water bath and monitored over a 10-day period. After the reaction was deemed complete, the reaction was heated at 100 °C for 10 min to inactivate the enzymes. The reaction was then centrifuged on a benchtop centrifuge to collect the precipitated glycans and the supernatant was removed by decanting. The products were then characterized and purified as described in Examples 11-18. A fluorophore-assisted carbohydrate electrophoresis experiment demonstrating the presence of DP>1 glycans is shown in FIG. 9.

## Example 8. Shifting product distributions by the addition of reaction modifiers.

Other modifiers may be added to the reaction to control the reaction rate, total material yield, conversion efficiency, or structural distribution of the resulting glycan. In one embodiment, monomeric D-galactose was added to the reaction described in Example 12. Comparison of the product profile over 24 hours showed that the galactose both slowed the rate of oligosaccharide formation as well as shifted the product distribution towards a higher proportion of oligosaccharides (FIG. 10).

To a 0.05 M, pH 5 sodium acetate buffer at 60 °C was added lactose to a concentration of 400 mg/mL. The lactose was agitated gently until dissolved. D-galactose was then added to the reaction to a final concentration of 115 mg/mL and agitated gently until dissolved. A stock solution of beta-galactosidase (Megazyme, catalog# E-BGLAN) was then added to the reaction to a final concentration of 10 U/ml. The reaction was maintained at 60 °C for 24 hr in a covered water bath. When the reaction was deemed complete it was heated to 100 °C for 10 min to inactivate the enzyme, then diluted with water to a 2% total carbohydrate concentration. The products were then characterized, isolated, and purified as described in Examples 6-8.

**Example 9. Method for producing glycans via reverse hydrolysis by bacterial culture isolate**

The glycans described, e.g., in Example 1 may be generated by reverse hydrolysis of glycosidic bonds catalyzed by an isolated bacterial preparation including one or more from the categories of viable whole cells, non-viable whole cells, partial cells or cell components, cell culture supernatants, or other portions of bacterial media that contain glycoside hydrolases. In this experiment, a bacterial strain, combination of strains, or full bacterial community is first grown in suitable media under suitable conditions. The culture is then harvested using standard techniques and the isolated culture is then subjected to the reverse-hydrolysis conditions described in Example 1 to generate a glycan that is tailored to that strain, combination, or community.

The strain, combination, or community of strains may be cultured using any of the well-known means described, e.g., in Martens, E. C.; et al. Cell Host & Microbe, 2008, 4, 447.; Romano, K. A.; et al. mBio, 2015, 6, e02481-14.; Atlas, R. M., Handbook of Microbiological Media, 4th Ed., 2010.; or other published literature selecting culturing conditions optimized for the strain or strains of interest. Once the culture has reached a suitable density, the culture is harvested from the media by any suitable method including filtration, centrifugation, or lyophilization.

If desired, the isolated cells are lysed after collection by bead beating, lyophilization, or other technique to yield a mixture of non-viable cell matter containing natively expressed, endocellular or membrane-bound glycosyl hydrolases.

If desired, the culture supernatant is isolated and lyophilized independent of the cell matter to yield a mixture of natively expressed exo-cellular glycosyl hydrolases.

If desired, the combined cells and culture supernatant are lysed and lyophilized together to yield a mixture of all expressed glycosyl hydrolases.

The glycosyl hydrolases isolated as described are then used to generate glycans, e.g., as described in Example 1 using the isolated glycosyl hydrolases in place of the purified enzymes. The conditions described in Example 1 may be further modified by the addition of cellpermeabilizing agents including DMSO or toluene to increase the accessibility of the expressed enzymes. The activity of the isolated solids may be measured using any suitable techniques, e.g., described in Goulas, et al. International Dairy Journal 17 (2007) 648-656. When applied to glycans generated using this method, the purifications described in Examples 16-18 may be altered to remove residual cellular material by any well-known method including centrifugation, sterile filtration, and gel filtration chromatography. The glycans may then be used to increase growth of the particular strains, combinations, or community of strains, e.g., in the gut, e.g., upon administration of an effective amount to a human subject.

In a further embodiment, a biomass pellet containing -400 mg Bifidobacterium longum (BLO.16) grown in a media containing glucose as the sole carbohydrate source was washed with 1 ml 0.01XPBS six times to remove any residue culture media. For each wash, the pellet was resuspended by vortex and then collected by centrifugation at 10,000 g for 3 min. After the wash was complete, the biomass was re-suspended in water at ~100 mg/ml and diluted with 16 ml 0.01XPBS and 40 μl toluene for 1h at room temperature to increase the cell membrane permeability. The cell pellet was collected by 10,000 g centrifugation for 10 min and the supernatant discarded. The pellet was then washed four times with 20 ml 0.01XPBS in the same manner. After that the biomass was re-suspended in 4 ml water and lyophilized for enzymatic synthesis purposes.

After preparation of the biomass, 250 mg of maltose were weighed into a 1.5 ml microcentrifuge tube and dissolved in 250 μl 0.05 M, pH5 acetate buffer with brief heating in a 40 °C water bath. The lyophilized biomass was added into the maltose solution. The reactions were kept at 40 °C for 3 days and oligomer production was monitored. At each time interval, a 20 μl portion of a sample was heated in a boiling water bath for 10 min to inactivate the enzyme. This portion was then diluted down to ~20 mg/ml with water and 0.2 μm filtered to check the reaction progress by SEC or TLC.

**Example 10. Method for inducing hydrolase expression by pre-treatment of bacterial culture with defined glycan**

The method for producing glycans by an isolated bacterial preparation described in Example 3 may be further modified by inducing expression of specific glycosyl hydrolases in the culture growth step in order to shift enzyme expression levels. In this example, the bacterial culture conditions described in Example 8 may be modified by the addition of a glycan (e.g. from a previous synthesis), glycoside (e.g. alpha-1,6-mannobiose), oligosaccharide (e.g. lactose, GOS), or fiber (e.g. cellulose, inulin, pectin) in lieu of other carbon sources in order to induce the culture to express glycosyl hydrolases specific to the metabolism of that glycan, glycoside, oligosaccharide, or fiber as a substrate. In this fashion, the enzymatic activity of the isolated bacterial preparation may be tuned and enhanced to improve the production of the desired glycan. For example, a cellular isolate that favors the production of alpha-1,6-glucose bonds are generated by culturing the desired strain on oligosaccharides containing predominantly alpha-1,6-glucose bonds such as isomaltose. The culture is established with isomaltose as a sole carbon source until the strain achieves a growth rate that suggests upregulation of enzymes capable of digesting isomaltose. Isolates from this culture are enriched in glycosidases selective for alpha-1,6-glucose hydrolysis. This enriched isolate is then used to produce glycans by reverse hydrolysis.

The glycan can be administered to a subject, e.g., gut, to selectively boost growth of a bacterial taxa rich in alpha-1,6-glucosidase.

In a second embodiment, the biomass described in Example 9 was grown in a media containing melibiose (galactose-alpha-1,6-glucose) as the only source of carbohydrates instead of glucose, resulting in a biomass with downregulated glucosyl hydrolases and upregulated galactosylhydrolases. The procedure described in Example 9 was then modified by replacing maltose with 100 mg melibiose dissolved into pH5.5, 250 μl citric/phosphate buffer. The procedure was then followed identically to create oligomers of alpha-galactose instead of alphaglucose as described in Example 9.

**Example 11. Glycan Preparations**

To a round bottom flask equipped with an overhead stirrer and a jacketed short-path condenser was added one or more mono- or disaccharides along with 3-20% by dry weight of one or more of the catalysts e.g. acid, ionic, ionic/acid containing catalysts such as, e.g. described in U.S. patent No. 9,079,171 and WO 2016/007778, which are incorporated herein by reference in their entirety. Water or another compatible solvent (zero to 10 equiv.) was added to the dry mixture and the slurry was combined at approximately 100 rpm using a paddle sized to match the contours of the selected round bottom flask as closely as possible. The mixture was then heated to 80-185° C. Once the solids achieved a molten state, the vessel was placed under 10-1000 mbar vacuum pressure. The reaction was stirred for 30 minutes to 8 hours, constantly removing water from the reaction. Reaction progress was monitored by HPLC. When sufficient oligomerization had occurred, the stirrer was shut off, the reaction was cooled to room temperature and vented to atmospheric pressure, and the product, either as a solid or syrup, was dissolved in a volume of water sufficient to create a solution of approximately 50 Brix (grams sugar per 100 g solution). Once dissolution was complete, solid catalyst was removed by filtration and the oligomer solution was concentrated to approximately 50-75 Brix by rotary evaporation. In cases in which an organic solvent has been used, water immiscible solvents can be removed by biphasic extraction and water miscible solvents can be removed by rotary evaporation concomitant to the concentration step.

Among others, the following glycans were made in multiple batches and tested in various assays described herein:

Single glycan unit (homo-glycans): ara100, fru100, gal100, galA100, glcNac100, glu100, gluA100, Lglu100, man100, rha100, xyl100.

Two glycan units (hetero-glycans): Ara60Xyl40, Ara80Xyl20, Gal20Ara80, Gal20Xyl80, Gal40Ara60, Gal40Man60, Gal40Xyl60, Gal57Glu43, Gal60Ara40, Gal60Man40, Gal60Xyl40, Gal80Ara20, Gal80Man20, Gal80Xyl20, Glu20Ara80, Glu20Xyl80, Glu40Ara60, Glu40Gal60, Glu40Xyl60, Glu50Gal50, Glu50Lglu50, Glu60Ara40, Glu60Gal20Man20, Glu60Gal40, Glu60Man40, Glu60Xyl40, Glu66Fru33, Glu75Gala25, Glu75GluA25, Glu75GluN25, Glu80Ara20, Glu80Gal20, Glu80Lglu20, Glu80Man20, Glu80Xyl20, Glu90LGlu10, Man20Ara80, Man20Xyl80, Man40Ara60, Man40Xyl60, Man60Ara40, Man60Glu40, Man60Xyl40, Man75Gal25, Man80Ara20, Man80Gal20, Man80Glu21, Man80Xyl20, Xyl60Ara40, Xyl75Ara25, Xyl80Ara20, and the hybrid glycans glu90sor10 and glu90gly10.

Three glycan units (hetero-glycans): Gal5Xyl5Ara90, Gal5Xyl90Ara5, Gal10Xyl10Ara80, Gal10Xyl45Ara45, Gal10Xyl80Ara10, Gal20Xyl20Ara60, Gal20Xyl40Ara40, Gal20Xyl60Ara20, Gal30Xyl30Ara40, Gal30Xyl40Ara30, Gal33Man33Ara33, Gal33Man33Xyl33, Gal33Xyl33Ara33, Gal45Xyl10Ara45, Gal45Xyl45Ara10, Gal50Glu25Fru25, Gal40Xyl20Ara40, Gal40Xyl30Ara30, Gal40Xyl40Ara20, Gal60Xyl20Ara20, Gal80Xyl10Ara10, Gal90Xyl5Ara5, Glu5Gal5Man90, Glu5Gal90Man5, Glu5Xyl5Ara90, Glu5Xyl90Ara5, Glu10Gal10Man80, Glu10Gal45Man45, Glu10Gal80Man10, Glul0Xyl10Ara80, Glu10Xyl45Ara45, Glul0Xyl80Ara10, Glu20Gal20Man60, Glu20Gal40Man40, Glu20Gal60Man20, Glu20Gal80, Glu20Xyl20Ara60, Glu20Xyl40Ara40, Glu20Xyl60Ara20, Glu30Gal30Man40, Glu30Gal40Man30, Glu30Xyl30Ara40, Glu30Xyl40Ara30, Glu33Gal33Ara33, Glu33Gal33Fuc33, Glu33Gal33Man33, Glu33Gal33Xyl33, Glu33Man33Ara33, Glu33Man33Xyl33, Glu33Xyl33Ara33, Glu40Gal20Man40, Glu40Gal30Man30, Glu40Gal40Man20, Glu40Xyl20Ara40, Glu40Xyl30Ara30, Glu40Xyl40Ara20, Glu45Gal10Man45, Glu45Gal45Man10, Glu45Xyl10Ara45, Glu45Xyl45Ara10, Glu60Xyl20Ara20, Glu75GluNAc25, Glu80Gal10Man10, Glu80Xyl10Ara10, Glu90Gal5Man5, Glu90Xyl5Ara5, Man33Xyl33Ara33, Man52Glu29Gal 19.

Four glycan units (hetero-glycans): Gal25Man25Xyl25Ara25, Glu25Gal25Man25Ara25, Glu25Gal25Man25Xyl25, Glu25Gal25Xyl25Ara25, Glu25Man25Xyl25Ara25.

Five glycan units (hetero-glycans): Glu20Gal20Man20Xyl20Ara20.

Glycans are described by a three- to six-letter code representing the monomeric sugar component followed by a number out of one hundred reflecting the percentage of the material that monomer constitutes. Thus, 'glu100' is ascribed to a glycan generated from a100% D-glucose (glycan unit) input and 'glu50gal50' is ascribed to a glycan generated from 50% D-glucose and 50% D-galactose (glycan units) input or, alternatively from a lactose dimer (glycan unit) input. As used herein: xyl = D-xylose; ara = L-arabinose; gal = D-galactose; glu = D-glucose; rha = L-rhamnose; fuc = L-fucose; man = D-mannose; sor = D-sorbitol; gly = D-glycerol; neu = NAcneuraminic acid; Lglu = L-glucose; gluA = D-glucuronic acid; gluN = D-glucosamine; gluNAc = N-acetyl-D-glucosamine; galA = D-galacturonic acid. 3-Bn = benzyl; 3-Obn = 3-benzyloxy; 6-TBDPS = 6-tert-butyldiphenylsilyl; galnac = N-acetyl galactosamine; rib = D-ribose; Sor = sorbitol.

## Example 12. Purification

Oligo- and polysaccharides were dissolved in deionized water to a final concentration of 25-50 Brix. The material was then exposed to at least 2 mass equivalents of Dowex Monosphere 88 ion exchange resin. Exposure may occur by swirling in a flask at 120-170 rpm or by filtration through a wet slurry packed column as long as the residence time is sufficient for the solution to achieve a final pH between 3 and 5. The oligomer solution was isolated by filtration (as in the case of swirled reactions) or elution (as in the case of column filtration) and the process was repeated with Dowex Monosphere 77 ion exchange resin in an analogous fashion until the solution pH was above 5.5. Finally, the solution was exposed to Dowex Optipore SD-2 Adsorbent decolorizing resin until the solution was sufficiently clarified and filtered through a 0.2 micron filter to remove residual resin and resin fines. The final solution was then concentrated to 50-85 Brix by rotary evaporation or to a solid by lyophilization.

## Example 13. High-throughput preparation at small scale

The oligomers and polymers were synthesized in a parallel fashion in 24-, 48-, or 96-well plates or similarly sized arrays of 1 dram vials housed in aluminum heating blocks. In this example, all liquid transfers were handled by a programmable robot or manually using calibrated pipettes. To each vial or well was added 20-100% by dry weight of one or more catalysts e.g. acid, ionic, ionic/acid containing catalysts such as, e.g. described in U.S. patent No. 9,079,171 and WO 2016/007778. The plate or heating block was placed uncovered in a vacuum oven heated to 50 to 150 °C under a vacuum of 10-800 mbar. The oven vacuum pump was protected by a twostage condenser consisting of a recirculating chiller trap followed by a dry ice/acetone trap. The plates or blocks are heated for 30 minutes to 6 hours under elevated temperature and reduced pressure without stirring. After a pre-established period of time, the oven was vented to atmospheric pressure, the plates or blocks were cooled to room temperature, and each well or vial was diluted to approximately 50 Brix with deionized water. The solid-phase extraction steps described in Example 12 were performed by elution through sequential wet-packed columns in which the eluent from each column flows immediately into the top of the next column at a rate between 2 and 6 bed volumes/hour using a peristaltic pump or other suitable small pump. The column stack was then rinsed with deionized water and the combined effluents are concentrated by lyophilization to isolate solid powders with residual water content of 1-10% by mass.

## Example 14. Removal of low molecular weight species

Oligomers or polymers were modified so as to remove low molecular weight species.

In one embodiment the separation was achieved by osmotic separation. Approximately 45 cm of 1.0 kD MWCO Biotech CE dialysis tubing (31 mm flat width) from Spectrum Labs was placed into deionized water and soaked for 10 minutes, then one end was sealed with a dialysis tubing clip. A 25 Brix solution of 8 grams dry oligosaccharide was sterile filtered and sealed into the tube with a second clip along with a few mL of air to permit the tube to float. The filled tube was then placed in a 3 gallon tank of deionized water which was stirred with sufficient force to induce slow swirling of the sealed tubes. After 8 hours, the water in the tank was replaced and the tube was allowed to stir for an additional 16 hours. Once the dialysis was complete and the material had a DP2+ yield greater than 95% and a DP3+ yield greater than 90%, the dilute solution was sterile filtered and concentrated in vacuo to a final concentration of approximately 65 Brix or lyophilized to a solid with a residual moisture between 1 and 10%.

In a second embodiment the separation was achieved by tangential flow filtration (TFF). In this case, 100 mL of 25 Brix glycan sample dissolved in deionized water and sterile filtered was placed into the feed bottle of a Spectrum Labs KrosFlo Research Iii TFF system that was prepared according to the manufacturer's recommendation. The sample was then diafiltered through a 1 kD mPES MidiKros hollow-fiber filter at a transmembrane pressure of 25 psig. HPLC samples of the feed stock taken every 0.5 diafiltration volumes were used to determine when the material had a DP2+ yield greater than 95% and a DP3+ yield greater than 90% at which point the solution was sterile filtered and concentrated *in vacuo* to a 65 Brix syrup or lyophilized to a solid with residual water content of 1-10% by mass.

In a third embodiment the separation was achieved by ethanol precipitation. In this case, 100 mL of 25 Brix glycan sample was poured into a vigorously stirred beaker containing 900 mL of pure, USP-grade ethanol at a rate no higher than 10 mL/minute. Once the addition was complete, the precipitated solids were subjected to stirring for an additional 15 minutes at or slightly below room temperature. The precipitated solids were isolated by filtration through a fine frit sintered glass funnel under an atmosphere of nitrogen to prevent hydration and gumming. The solids were rinsed once with ethanol, then dissolved in water to a final concentration of 25 Brix and reconcentrated to >65 Brix. This syrup was then diluted back to 25 Brix and concentrated once more to ensure removal of residual ethanol.

**Example 15. Methods for analyzing preparations**

Measurement of concentration by liquid refractometry

This experiment was designed to quantitate the amount of glycan in any given aqueous solution. A Mettler-Toledo Refracto 30GS portable sugar refractometer was calibrated using high-purity reverse-osmosis deionized water. Several drops of the glycan solution were filtered through a 0.2 micron syringe filter directly onto the lens of the refractometer. The measurement was taken at room temperature and reported as Brix. The glycans were routinely concentrated to 50, 60, 70, or 75 Brix without obvious solidification or crystallization at 23 °C. Brix can then be converted to solubility assuming a specific density of water equal to 1.0 g/mL. Thus, 75 Brix (100 grams of solution consisting of 75 grams of glycan and 25 grams of water) equals an aqueous solubility of 3.0 g/mL. As a comparison, the aqueous solubility of D-glucose is reported to be 0.909 g/mL (48 Brix) at 25 °C by Sigma-Aldrich.

Monomeric composition by hydrolysis and GC-MS

This experiment was designed to quantitate the ratio of monomer content within a given oligosaccharide. Glycosyl composition analysis was performed by combined gas chromatography/mass spectrometry (GC/MS) of the per-O-trimethylsilyl (TMS) derivatives of the monosaccharide methyl glycosides produced from the sample by acidic methanolysis as described previously by Santander et al. (2013) Microbiology 159:1471. Between 100 and 200 $\mu$g of sample were lyophilized into a suitable test tube. Inositol (20 $\mu$g) was added to the sample as an internal standard, then the sample was heated to 80 °C in 1M HCl/methanol for 18 hours. The resulting monosaccharides were then re-acetylated using pyridine and acetic anhydride in MeOH, and per-O-trimethylsilylated with Tri-Sil (Pierce) at 80 °C for 30 minutes. GC/MS analysis of the TMS methyl glycosides was performed on an Agilent 7890A GC interfaced to a 5975C MSD, using a Supelco Equity-1 fused silica capillary column (30 m x 0.25 mm ID). Each peak was assigned to a component sugar based upon comparison to known standards and integration of the respective peaks allowed clean calculation of the relative percentage of monomers within an exemplified glycan. In all enumerated glycans, conditions can be routinely identified in which the monomer composition of a given oligosaccharide matched the input ratio within experimental error and the output composition matched the input composition within the precision of the measurement.

Molecular weight distribution by size-exclusion chromatography (SEC)

This experiment was designed to quantitate the distribution of molecular weights within a given oligosaccharide. The measurement was made by HPLC using the method described in Monograph of United States Pharmacopeia, 38(6) In-Process Revision: Heparin Sodium (USP37-NF32). Separations were achieved on an Agilent 1200 HPLC system via a GE superpose 12 column using 50 mM ammonium acetate as an eluent at 1.0 mL/min flow rate and an ELSD detector. The column temperature was set at 30 °C and dextran (1 kD, 5 kD, 10 kD weight) were used to draw a standard curve. A 2 mg/ml solution of the samples was prepared and passed through a 0.45 $\mu$m spin filter, followed by 40 $\mu$l injections into the HPLC. A third-order polynomial curve was constructed based on the logarithmic molecular weights and elution volumes of the listed standards. The weight-average molecular weight (Mw), the number average molecular weight (Mn), and the polydispersity index (PDI) for the sample were calculated by comparison to the standard curve. Figure 1 shows the curve generated during the SEC evaluation of a glu 100 sample in which the average molecular weight was determined to be 1212 g/mol or approximately DP7. The upper end of molecular weight of the material as defined by the point of the curve at 10% of maximum absorption leading the curve was determined to be 4559 g/mol or approximately DP28. The lower end of molecular weight of the material as defined by 10% of the maximum absorption trailing the curve was determined to be 200 g/mol or approximately DP1. Similar analysis of a glu50gal50 sample showed a MW, high mass, and low mass of 1195 g/mol (~DP7), 4331 g/mol (~DP27), and 221 g/mol (~DP1) respectively.

Molecular weight distribution by ion-affinity chromatography (IAC)

The proportion of glycan with DP greater than or equal to 2 (DP2+) and 3(DP3+) may be measured by ion-affinity chromatography. A sample of glycan was diluted out to 50-100 mg/mL and 10 $\mu$L of this solution was injected onto an Agilent 1260 BioPure HPLC equipped with a 7.8x300 mm BioRad Aminex HPX-42A column and RI detector. Using pure HPLC-grade water as an eluent, the sample was eluted at 0.6 mL/min through an 80 °C column and an RI detector maintained at 50 °C. The peaks representing DP1-6 are assigned by comparison to reference standards and integrated using the Agilent ChemStation software. Peaks are typically integrated as DP1, DP2, DP3, DP4-7, and DP8+. The DP that is achievable by the reaction described in Example 11 varies from monomer to monomer although it is consistent across batches if the procedure is followed. For example, across 17 batches of glu100, DP2+ values ranged from 77-93% and DP3+ values ranged from 80-90%. Conversely, across 6 batches of ara100, DP2+ values ranged from 63-78% and DP3+ values ranged from 48-71%. Mixtures of monomers behaved as averages of the individual components.

Alpha-/beta-distribution by 2D NMR

This experiment was designed to quantitate the ratio of alpha- and beta-glycosidic bonds within a given sample by two-dimensional NMR. Approximately 150 mg of 65 Brix oligosaccharide solution was dried to stable mass in a vacuum oven at 45-95 °C under 400 mbar pressure. The sample was subjected to two cycles of dissolution in $D_2O$ and drying to remove residual $H_2O$. Once dried, the sample was dissolved in 750 $\mu$L $D_2O$ with 0.1% acetone, placed into a 3 mm NMR tube, and analyzed in a Bruker Avance-III operating at 500.13MHz 1H (125.77MHz 13C) equipped with a Bruker BBFO probe operating at 21.1 °C. The sample was analyzed using a heteroatomic single quantum coherence pulse sequence (HSQC) using the standard Bruker pulse sequence. Anomeric protons between 4-6 ppm (1H) and 80-120 ppm (13C) were assigned by analogy to glucose as reported in Roslund, et al. (2008) Carbohydrate Res. 343:101-112. Spectra were referenced to the internal acetone signal: 1H - 2.22 ppm; 13C - 30.8 ppm. Isomers were quantitated by integration of their respective peaks using the MNova software package from Mestrelab Research (Santiago de Compostela, Spain). Figure 2 shows the anomeric region of a representative spectrum. Over 300 samples have been assayed in this fashion and Table 29 lists the distribution across a sample of combinations of monomers showing the alpha-/beta- ratio to be as high as 4:1 as in the case of rha100 and as low as 1:1 as in the case of glu50gal50.

**Table 29: Distribution of alpha- and beta-bonds across batches and types of glycans**

| glycans | alpha-bonds (%) | beta-bonds (%) | alpha/beta ratio |
|---|---|---|---|
| Glu100 | 58 | 42 | 1.4 |
| | 61 | 39 | 1.6 |
| | 64 | 36 | 1.8 |
| | 64 | 36 | 1.8 |
| | 62 | 38 | 1.6 |
| | 61 | 39 | 1.6 |
| | 62 | 38 | 1.6 |
| | 63 | 37 | 1.7 |
| | 60 | 40 | 1.5 |
| | 65 | 35 | 1.9 |
| | 65 | 35 | 1.9 |
| | 60 | 40 | 1.5 |
| Gal100 | 60 | 40 | 1.5 |
| Gal33man33ara33 | 79 | 21 | 3.8 |
| | 75 | 25 | 3.0 |
| Glu50gal50 | 50 | 50 | 1.0 |
| | 56 | 44 | 1.3 |
| | 61 | 39 | 1.6 |
| | 65 | 35 | 1.9 |
| Glu33gal33fuc33 | 55 | 45 | 1.2 |
| Man100 | 57 | 43 | 1.3 |
| Man52glu29gal19 | 76 | 24 | 3.2 |
| Ara100 | 67 | 33 | 2.0 |

(continued)

| glycans | alpha-bonds (%) | beta-bonds (%) | alpha/beta ratio |
|---|---|---|---|
| Rha100 | 80 | 20 | 4.0 |
| Xyl100 | 57 | 43 | 1.3 |
| | 59 | 41 | 1.4 |
| Xyl75gal25 | 56 | 44 | 1.5 |

Identification of composition by NMR

This experiment was designed to identify the composition of a glycan by 2D-NMR identification of the constituent monomers. Approximately 150 mg of 65 Brix oligosaccharide solution was dried to stable mass in a vacuum oven at 45-95 °C under 400 mbar pressure. The sample was subjected to two cycles of dissolution in $D_2O$ and drying to remove residual $H_2O$. Once dried, the sample was dissolved in 750 $\mu$L $D_2O$ with 0.1% acetone, placed into a 3 mm NMR tube, and analyzed in a Bruker Avance-III operating at 500.13MHz 1H (125.77MHz 13C) equipped with a Bruker BBFO probe operating at 70 °C. The sample was analyzed using a heteroatomic single quantum coherence pulse sequence (HSQC) using the standard Bruker pulse sequence. The anomeric region of each glycan spectra derived from a single sugar monomer was then examined for peaks representing specific glycosidic bonds characteristic to that monomer. For any given glycan, the HSQC spectra allow the identification of peaks that are unique to specific regio- and stereochemical bond arrangement. For example, Figure **5** shows a partial assignment of the spectra of a glu100 preparation demonstrating how these peaks may be used to identify specific glycosidic regio- and stereo-chemistries. Due to the spin-isolated nature of single carbohydrate rings within polysaccharides, the HSQC spectra of a glycan with more than one monomer is predicted to be represented by the sum of the HSQC peaks of each of its constituent sugars. Therefore, each constituent monomer has unique HSQC peaks that will appear in any glycan that contains that monomer irrespective of other constituent monomers and furthermore, the monomers used to synthesize a glycan can be determined by identifying the fingerprint peaks unique to each constituent monomer. For example, Figure **3B** shows that the HSQC spectra of glu50gal50 is a hybrid of the spectra of glu100 (Figure **3A**) and gal100 (Figure **3C**). Table 30 lists the fingerprint peaks for selected glycan units.

**Table 30: Diagnostic HSQC peaks for each component sugar.**

| Monomer | 1H shift | 13C shift | Monomer | 1H shift | 13C shift |
|---|---|---|---|---|---|
| Glucose | 5.42 | 92.5 | Xylose | 5.18 | 93.0 |
| | 5.21 | 92.8 | | 5.10 | 94.3 |
| | 5.18 | 93.9 | | 5.34 | 98.2 |
| | 5.08 | 97.0 | | 5.31 | 99.6 |
| | 5.36 | 98.4 | | 5.11 | 100.8 |
| | 5.34 | 99.8 | | 4.91 | 99.4 |
| | 5.38 | 100.3 | | 4.56 | 97.3 |
| | 4.95 | 98.6 | | 4.64 | 104.2 |
| | 4.62 | 96.6 | | 4.54 | 103.4 |
| | 4.70 | 103.6 | | 4.44 | 102.6 |
| | 4.49 | 103.4 | | 4.44 | 104.1 |
| Galactose | 5.37 | 92.9 | Arabinose | 5.22 | 93.2 |
| | 5.24 | 93.1 | | 5.13 | 93.2 |
| | 5.14 | 96.0 | | 5.29 | 96.0 |
| | 4.96 | 99.3 | | 5.26 | 97.2 |
| | 5.31 | 98.7 | | 5.12 | 96.6 |
| | 5.39 | 101.4 | | 5.18 | 99.6 |

(continued)

| Monomer | 1H shift | 13C shift | Monomer | 1H shift | 13C shift |
|---|---|---|---|---|---|
| | 5.00 | 101.8 | | 5.06 | 99.2 |
| | 4.80 | 101.3 | | 4.99 | 100.0 |
| | 4.63 | 97.0 | | 5.26 | 101.9 |
| | 4.56 | 97.2 | | 5.06 | 102.1 |
| | 4.53 | 103.1 | | 4.55 | 97.4 |
| | 4.43 | 104.1 | | 4.54 | 105.2 |
| Fucose | 5.18 | 92.9 | | 4.50 | 105.5 |
| | 5.33 | 92.4 | | 4.38 | 103.9 |
| | 5.04 | 96.3 | Rhamnose | 5.21 | 93.2 |
| | 4.90 | 99.7 | | 5.10 | 94.5 |
| | 4.52 | 97.0 | | 4.85 | 94.1 |
| | 4.39 | 103.6 | | 5.01 | 95.8 |
| Mannose | 5.37 | 93.0 | | 5.35 | 100.5 |
| | 5.16 | 94.6 | | 5.15 | 102.2 |
| | 4.88 | 94.2 | | 5.04 | 102.9 |
| | 5.39 | 101.7 | | 4.78 | 97.9 |
| | 5.24 | 101.9 | | 4.71 | 99.0 |
| | 5.13 | 102.8 | | 4.72 | 101.0 |
| | 5.03 | 102.7 | | | |
| | 5.24 | 105.6 | | | |
| | 5.09 | 108.0 | | | |
| | 4.88 | 94.2 | | | |
| | 4.89 | 100.0 | | | |
| | 4.70 | 101.1 | | | |

At least 5 peaks appeared for each glycan unit used as a starting material in the synthesis of glycans containing 3 or fewer distinct glycan units. The HSQC spectra of glycans containing 4 or more distinct glycan units have at least 4 peaks for each constituent glycan unit.

Figure 6A and 6B show the HSQC spectra for man100 and xyl100, respectively.

Glycosidic linkage analysis

This experiment was designed to quantitate the distribution of glycosidic regioisomers (branching) within a given oligosaccharide. For glycosyl linkage analysis, the samples were permethylated, depolymerized, reduced, and acetylated; and the resultant partially methylated alditol acetates (PMAAs) analyzed by gas chromatography-mass spectrometry (GC-MS) as described by Heiss et al (2009) Carbohydr. Res. 344:915. The samples were suspended in 200 μl of dimethyl sulfoxide and left to stir for 1 day. Permethylation was affected by two rounds of treatment with sodium hydroxide (15 min) and methyl iodide (45 min). The aqueous solution was hydrolyzed by addition of 2M trifluoroacetic acid and heating to 121 °C for 2 hours. Solids were isolated *in vacuo* and acetylated in acetic acid/trifluoroacetic acid. The resulting PMAAs were analyzed on an Agilent 7890A GC interfaced to a 5975C MSD (mass selective detector, electron impact ionization mode); separation was performed on a 30 m Supelco SP-2331 bonded phase fused silica capillary column. Figure **4** shows three representative GC spectra from this analysis. These analyses show that the glycans had at least 0.1%, 0.2%, 0.5%, 1%, 2%, 5%, 10% or more of the 1,2-glycoside bond type, e.g. ara100 = 3.8%, gal100 = 7.2%; at least 0.1%, 0.2%, 0.5%, 1%, 2%, 5%, 10% or more of the 1,3-glycoside bond type, e.g. 3-bn-glu100 = 1.7%, glu50gal50 = 10.4%; at least 0.1%, 0.2%, 0.5%, 1%, 2%, 5%, 10% or more of the 1,4-glycoside bond type, e.g. glu50gal50 = 5.9%, gal33man33ara33 = 10.1%; and at least 0.1%, 0.2%, 0.5%, 1%, 2%, 5%, 10%, 15%, 20%, 25% or more of the 1,6-glycoside bond type, e.g. gal33man33ara33 = 13.4%, glu100 = 25.4%. The materials also contained at least 5%, 10%, 15%, 20%, 25%, 30%, 40%, 50%, or more of the branched bond types (including but not limited to 1,3,6-; 1,4,6-; or 1,2,4-glycosides, e.g. Table 31), a degree of branching (DB) of at least 0.05. Degree of branching is defined as the average number of branched monomers relative to total number of monomer units. For example, a glu100 glycan polymer in which 20% of the glucose monomer units contain glycosidic linkages to three or more other glucose monomers would have a DB of 0.20. The glycans also have about 3-12% of the monomeric units in the furanose form. A glycan originating from a single monomer consisted of at least 12 distinct non-terminal substitution patterns. A glycan originating from two monomers consisted of at least 18 distinct non-terminal substitution patterns, e.g. glu-1,2-glu; glu-1,2-gal; gal-1,2-glu; gal-1,2-gal; glu-1,2(glu),6-glu; glu-1,3-glu; glu-1,3-gal; etc. A glycan originating from three or more monomers consisted of at least 24 distinct non-terminal substitution patterns.

**Table 31. A sample of degree of branching (DB) measurements; sample selected from 54 different preparations characterized as described herein.**

| composition | % branched monomers | |
| --- | --- | --- |
| | highest measure | lowest measure |
| glu100 | 40.4 | 10.4 |
| glu80man20 | 16.1 | |
| glu60man40 | 16.4 | |
| man80glu20 | 18.6 | |
| man60glu40 | 20.5 | |
| glu50gal50 | 22.4 | 12.6 |
| gal100 | 22.2 | |
| glu33gal33fuc33 | 41.8 | |
| ara100 | 16.6 | |
| xyl100 | 63.2 | |
| ayl75ara25 | 26.9 | |
| man52glu29gal19 | 22.7 | 9.8 |
| man100 | 40.0 | |

**Table 32. Full chemical characterization of a sample of different glycan preparations for use in the methods disclosed herein.**

| Glycan | total molar incidence of a bond (%) | | | | Misc glycoside sums (%) | | | alpha/beta ratio by HSQC NMR | | SEC data | | | | |
|---|---|---|---|---|---|---|---|---|---|---|---|---|---|---|
| | total 1,2 | total 1,3 | total 1,4 | total 1,6 | total branchin g | total furanos e | total termin al sugars | % alpha | % beta | DP2+ % | Mw | Mn | PD | DPn |
| Glu5Gal5Man90-2 | 19% | 15% | 22% | 43% | 25.9 | 12 | 34.9 | 80% | 20% | 98% | 1842 | 946 | 1.95 | 11.26 |
| Glu10Gal10Man80-1 | 15% | 16% | 24% | 45% | 22.6 | 6.7 | 33.1 | 81% | 19% | 98.60 % | 1978 | 1021 | 1.94 | 12.1 |
| Glu20Gal20Man20Xyl 20Ara 20-1 | 16% | 18% | 32% | 34% | 25.1 | 33.1 | 6.85 | 87% | 13% | 100% | 1278 | 935 | 1.37 | 7.78 |
| Glu20Gal20Man20Xyl 20Ara 20-2 | 16% | 19% | 16% | 48% | 4.8 | 35.3 | 1.68 | 63% | 37% | 100% | 1845 | 1000 | 1.85 | 11.28 |
| Gal33Man33Ara33-8 | 17% | 26% | 23% | 34% | 25.5 | 27.5 | 32.7 | 87% | 13% | 98% | 1527 | 834 | 1.83 | 9.31 |
| Gal57Glu43-1 | 4% | 7% | 73% | 16% | 2 | 2.7 | 50.9 | 33% | 67% | 94% | 374 | 349 | 1.07 | 2.20 |
| Glu100-87 | 1% | 3% | 93% | 4% | 0 | 0 | 34.7 | 69% | 31% | 100% | 416 | 399 | 1.04 | 2.46 |
| Gal57Glu43-2 | 2% | 2% | 1% | 94% | 1.3 | 1.5 | 46.6 | 65% | 35% | 98% | 390 | 374 | 1.04 | 2.3 |
| Glu50Gal50-11 | 15% | 20% | 20% | 45% | 14.8 | 12.2 | 38.3 | 64% | 36% | 91% | 1456 | 675 | 2.16 | 8.88 |
| Glu50Gal50-32 | 15% | 16% | 26% | 43% | 13.1 | 17.9 | 45.2 | 66% | 34% | 96% | 1114 | 790 | 1.41 | 6.77 |
| Glu50Gal50-14 | 13% | 17% | 25% | 44% | 13.5 | 22.4 | 43.3 | 70% | 30% | | | | | |
| Glu50Gal50-27 | 15% | 20% | 22% | 43% | 19.5 | 9.6 | 29.5 | 61% | 39% | 99% | 1776 | 945 | 1.88 | 10.85 |
| Glu50Gal50-23 | 17% | 20% | 20% | 44% | 19.2 | 17.2 | 35.5 | 71% | 29% | 99% | 1497 | 855 | 1.75 | 9.13 |
| Glu50Gal50-2 | 16% | 21% | 18% | 45% | 19.4 | 15.6 | 35.5 | 65% | 35% | | 1931 | 936 | 2.06 | 11.8 |
| Glu100-129 | 20% | 19% | 16% | 46% | 19.1 | 5.3 | 36.3 | 62% | 38% | 99% | 1411 | 1712 | 1.21 | 7.84 |
| Glu100-136 | 19% | 20% | 16% | 46% | 19.6 | 4.7 | 34.8 | 64% | 36% | 99% | 1577 | 1834 | 1.16 | 8.76 |
| Glu100-17 | 19% | 20% | 15% | 47% | 19.7 | 3.1 | 31.6 | 61% | 39% | 98% | 1523 | 1797 | 1.18 | 8.46 |
| Glu100-64 | 19% | 21% | 15% | 46% | 19.6 | 3.3 | 34.6 | 62% | 38% | 98% | 1620 | 1871 | 1.15 | 9.00 |
| Glu100-76 | 18% | 19% | 15% | 47% | 18.5 | 3.8 | 33.4 | 62% | 38% | 99% | 1410 | 1702 | 1.21 | 7.83 |
| Glu100-131 | 18% | 18% | 17% | 46% | 16.4 | 7.4 | 39.2 | 61% | 39% | 98% | 1200 | 1520 | 1.27 | 6.67 |

| Glycan | total molar incidence of a bond (%) | | | | Misc glycoside sums (%) | | | alpha/beta ratio by HSQC NMR | | SEC data | | | | |
|---|---|---|---|---|---|---|---|---|---|---|---|---|---|---|
| | total 1,2 | total 1,3 | total 1,4 | total 1,6 | total branching | total furanose | total terminal sugars | % alpha | % beta | DP2+ % | Mw | Mn | PD | DPn |
| Glu100-83 | 19% | 20% | 18% | 44% | 22.2 | 8.7 | 34.5 | 64% | 36% | 99% | 1605 | 1849 | 1.15 | 8.92 |
| Glu100-139 | 19% | 20% | 15% | 46% | 19.4 | 4.5 | 34.5 | 64% | 36% | 98% | 1542 | 1819 | 1.18 | 8.57 |
| Glu100-84 | 19% | 20% | 15% | 46% | 19 | 3.5 | 32.6 | 62% | 38% | 99% | 1431 | 1726 | 1.21 | 7.95 |
| Glu100-74 | 19% | 19% | 17% | 45% | 22.2 | 6.7 | 27.9 | 61% | 39% | 98% | 1387 | 1697 | 1.22 | 7.71 |
| Glu100-98 | 19% | 19% | 18% | 45% | 18.5 | 6.9 | 36.4 | 62% | 38% | 98% | 1383 | 1690 | 1.22 | 7.68 |
| Glu100-141 | 18% | 24% | 16% | 41% | 40.4 | 3.7 | 16.3 | 63% | 37% | 99% | 1673 | 1898 | 1.13 | 9.29 |
| Glu100-29 | 19% | 18% | 16% | 46% | 19.5 | 3.8 | 30 | 60% | 40% | 98% | 1311 | 1624 | 1.24 | 7.28 |
| Glu100-18 | 20% | 21% | 15% | 45% | 27.5 | 3.4 | 18.9 | 65% | 35% | 99% | 1748 | 1946 | 1.11 | 9.71 |
| Glu100-99 | 18% | 20% | 16% | 45% | 20.1 | 6.5 | 35.4 | 64% | 36% | 99% | 1641 | 1876 | 1.14 | 9.12 |
| Glu100-72 | 19% | 20% | 17% | 44% | 22.2 | 6.3 | 32.2 | 64% | 36% | 99% | 1716 | 1929 | 1.12 | 9.54 |
| Glu100-82 | 18% | 21% | 17% | 44% | 22 | 6.4 | 30.6 | 65% | 35% | 99% | 1711 | 1927 | 1.13 | 9.50 |
| Glu100-130 | 18% | 21% | 17% | 44% | 21.9 | 5.2 | 32.9 | 63% | 37% | 99% | 1781 | 1967 | 1.10 | 9.90 |
| Glu100-78 | 18% | 20% | 17% | 44% | 21.6 | 4.5 | 32 | 63% | 37% | 99% | 1719 | 1926 | 1.12 | 9.55 |
| Glu100-66 | 19% | 20% | 17% | 44% | 22 | 6.6 | 31.1 | 62% | 38% | 98% | 1472 | 1763 | 1.20 | 8.18 |
| Glu100-89 | 18% | 19% | 16% | 48% | 18.6 | 6.7 | 35.9 | 61% | 39% | 98% | 1326 | 1638 | 1.23 | 7.37 |
| Glu100-133 | 17% | 18% | 18% | 46% | 20.1 | 11.1 | 35.8 | 65% | 35% | 97% | 1224 | 1567 | 1.28 | 6.80 |
| Glu100-68 | 18% | 19% | 17% | 46% | 18.7 | 7.4 | 36.3 | 60% | 40% | 98% | 1394 | 1701 | 1.22 | 7.74 |
| Glu100-90 | 19% | 20% | 16% | 45% | 16.8 | 4.2 | 38.8 | 51% | 49% | 96% | 982 | 674 | 1.46 | 5.90 |
| Glu100-94 | 19% | 19% | 14% | 47% | 17.7 | 3.1 | 35.1 | 54% | 46% | 100% | 1369 | 978 | 1.40 | 8.30 |
| Glu100-5 | 19% | 19% | 14% | 48% | 16.3 | 3 | 36.6 | 57% | 43% | 100% | 1226 | 902 | 1.36 | 7.40 |
| 3-Obn Glu100-1 | 14% | 5% | 31% | 50% | 34.4 | 5.5 | 5.8 | 66% | 34% | 100% | 1014 | 486 | 2.09 | 6.15 |
| Gal100-30 | 16% | 19% | 24% | 41% | 17.2 | 32.6 | 30.4 | 74% | 26% | | | | | |

| Glycan | total molar incidence of a bond (%) | | | | Misc glycoside sums (%) | | | alpha/beta ratio by HSQC NMR | | SEC data | | | | |
|---|---|---|---|---|---|---|---|---|---|---|---|---|---|---|
| | total 1,2 | total 1,3 | total 1,4 | total 1,6 | total branchin g | total furanos e | total termin al sugars | % alpha | % beta | DP2+ % | Mw | Mn | PD | DPn |
| Glu33Gal33Fuc33-3 | 15% | 30% | 29% | 27% | 41.8 | 15.2 | 22.5 | 65% | 35% | | | | | |
| Ara100-12 | 26% | 42% | 32% | NA | 16.6 | 36.7 | 23.1 | 74% | 26% | | | | | |
| Xyl100-8 | 19% | 35% | 46% | NA | 63.2 | 3.8 | 0.3 | 70% | 30% | | | | | |
| Xyl75Ara25-3 | 25% | 32% | 43% | NA | 26.9 | 18.7 | 23.5 | 69% | 31% | | | | | |
| Glu80Man20-2 | 15% | 19% | 21% | 45% | 16.1 | 4.6 | 34 | 68% | 32% | | | | | |
| Glu60Man40-5 | 10% | 24% | 23% | 43% | 16.4 | 2.1 | 28.3 | 79% | 21% | | | | | |
| Man80Glu20-2 | 8% | 25% | 17% | 50% | 18.6 | 1.8 | 30.9 | 87% | 13% | | | | | |
| Man60Glu40-2 | 8% | 22% | 26% | 43% | 20.5 | 3.7 | 28.6 | 73% | 27% | | | | | |
| Man52Glu29Gal19-2 | 12% | 19% | 27% | 42% | 8.4 | 19 | 5 | 77% | 23% | | | | | |
| Man52Glu29Gal19-3 | 8% | 18% | 31% | 44% | 23.6 | 26.6 | 6.8 | 82% | 18% | | | | | |
| Man100-17 | 12% | 27% | 25% | 36% | 40 | 9.5 | 19.4 | 57% | 43% | | | | | |

Fluorescence-assisted carbohydrate electrophoresis (FACE)

This experiment was designed to quantitate the molecular weight of glycans within a given oligosaccharide in cases where the background signal (e.g. material from a bacterial culture, residual enzymes, buffers, etc.) interferes with traditional SEC techniques. The selected glycan was labeled with a polyanionic dye such as 8-Aminopyrene-1,3,6-trisulfonic acid (APTS) or 8-aminonaphthalene-1,3,6-trisulfonic acid (ANTS) and separated by gel electrophoresis. The selected glycans were diluted to 100 mg/mL in water and 5 $\mu$L of this solution was diluted to 1.00 mL to obtain a final solution concentration of 0.5 $\mu$g/$\mu$L. To 10 nmol of glycan dried *in vacuo* for 3 h were added 2 $\mu$l 0.1 M APTS or ANTS, 2 $\mu$l 1M NaCNBH$_3$ in THF, and 2 $\mu$L 1.7M citric acid and the mixture was kept at 70 °C for 2 h. The labeled solutions were diluted with 94 $\mu$l water and subsequently 4 $\mu$L of this labeled glycan solution were mixed with 1 $\mu$l 40% glycerol in water and loaded onto a 20% polyacrylamide precast gel (Life-Technologies). The electrophoresis was run for 5 min at 100V then 400V for 40 min at 4 °C. Maltodextrin and pullulan 12,000 were treated in the same fashion and used as standards on each gel. Gels were visualized with a Bio-Rad Gel Doc EZ Imager using the Bio-Rad Image Lab 3.0 software package. Images captured with the Bio-Rad software were processed using the GE ImageQuant TL 8.1 software.

The reaction conditions can be adjusted as described herein to achieve a desired mean degree of polymerization, degree of branching, and distribution of glycosidic linkages to generate glycan preparations that are effecutive to modulate the production or level of a metabolite when administered to, e.g., the gut of a subject, and are substrates of a glycosidic enzyme (e.g., a glycosidase enzyme that is present in a human gut microbe) and if so desired, the human gut microbe is one of a glycotaxa selected from class 1, class 2, class 3, class 4, class 5, class 6, or class 7, as described herein.

## Example 16. Purification of glycan polymer preparations by ion-exchange chromatography

If so desired, any glycan generated as described herein is purified by ion-exchange chromatography. Oligo- and polysaccharides were synthesized and dissolved in deionized water to a final concentration of 25-50 Brix. The material was then exposed to at least 2 mass equivalents of Dowex Monosphere 88 ion exchange resin. Exposure may occur by swirling in a flask at 120-170 rpm or by filtration through a wet slurry packed column with sufficient residence time for the solution to achieve a final pH between 3 and 5. The oligomer solution was isolated by filtration (as in the case of swirled reactions) or elution (as in the case of column filtration) and the process was repeated with Dowex Monosphere 77 ion exchange resin in an analogous fashion until the solution pH was above 5.5. Finally, the solution was exposed to Dowex Optipore SD-2 Adsorbent decolorizing resin until the solution was sufficiently clarified and filtered through a sterile 0.2 micron filter to remove residual solids and potential microbiological contaminants. Glycans isolated from cell matter reactions may by additionally purified by centrifugation, gel chromatography, ultrafiltration or dialysis, precipitation, or other well-known methods for removing residual cell matter.

## Example 17. Fractionation of glycan polymer preparations by activated charcoal chromatography

If so desired, any glycan generated as described herein is fractionated by activated charcoal chromatography. Oligo- and polysaccharides were synthesized and fractionated into pools of differential molecular weight by chromatography on activated charcoal. 500 mg of a 40-60 Brix aqueous solution of glycan and 6 g of activated charcoal (glycan:charcoal = 1:12, w/w) were stirred into 100 mL of a 1% v/v ethanol/water solution. The mixture was stirred at 300 rpm in a 250mL beaker at room temperature for 3 h and was vacuum filtered through a coarse glass frit pre-loaded with 6 g of celite 545 (Acros Organics). The charcoal and celite were finely mixed and loaded into an empty 20 mL Biotage Samplet cartridge. The outlet of the cartridge was connected to the inlet of a second 20 mL Biotage Samplet cartridge pre-loaded with 8 g of charcoal/celite mixture (1:1, w/w). Then 100 mL of increasing concentrations of ethanol/water eluents (0%, 1%, 3%, 5%, 7%, 10%, 15%, 20%, 25%, 30%, 35%, 40%, 45%, 50%, v/v) were run through the cartridge column at a flow rate of 3.7 mL/min using a Masterflex peristaltic pump to desorb glycan fractions. 100 mL of eluate was collected for each fraction and dried in vacuo.

In one embodiment, this process was used to remove only monomeric sugars from the glycan. In this embodiment, the charcoal was washed with 1% aqueous ethanol until monomer no longer eluted off the column. The charcoal was then washed with 50% aqueous ethanol to remove all residual glycan as shown in FIG. 11.

In a second embodiment, this process was used to split the glycan into multiple pools of increasing average molecular weight. In this embodiment, the charcoal was sequentially washed with eluents of increasing ethanol proportion (e.g. 1%, 2%, 5%, 10%, 20%, 50%). Fractions of increasing molecular weight were collected as the fraction of ethanol in the eluent increased.

## Example 18. Removal of monomeric and dimeric materials by yeast fermentation

If so desired, any glycan generated as described herein has its monomeric and dimeric materials removed by yeast fermentation. Oligo- and polysaccharides were synthesized and purified of monomeric species and some dimeric species by fermentation with a yeast culture. This procedure is primarily intended to remove materials that may be digestible by host metabolism to enrich the abundance of glycans that are exclusively available to bacterial fermentation. Bread yeast (2 g, Fleischmann's Yeast, Fenton, MO) was swollen in 10 ml deionized water for 1-16h at 4 °C. Sodium alginate (2.5 g) was dissolved in 100 ml hot deionized water by stirring at ~300 rpm for 1h on a hot stirring plate maintained at -80 °C. The final volume of sodium alginate solution was adjusted to 100 ml with deionized water. After the alginate solution temperature cooled to ~30 °C, the yeast suspension was mixed with the alginate solution at ~ 100 rpm for 10 min. The mixture was transferred into a 15 ml syringe equipped with a 21-gauge needle and was slowly added dropwise to an unstirred 4% calcium chloride (Sigma) solution in a 200 ml Erlenmeyer flask to form yeast-immobilized alginate beads. The beads were then kept at 4 °C for 1h to solidify, and were washed at least 5 times with water to remove calcium chloride. This yeast bead stock was kept at 4 °C in water for no longer than 1 week. The yeast beads were added into 20 Brix glycan solutions at 37 °C for 24 h with a ratio of ~6 ml beads per 25 ml glycan solution. The spent beads were replaced by fresh beads and incubated for an additional for another 39 h. The progression of monosaccharides removal was monitored by SEC-HPLC. A second bead replenish was applied for samples required further monosaccharide removal. After the yeast treatment, the glycan solutions were filtered through a 0.2 um sterile filter and passed through ion exchange columns as described in Example 16. The filtrates were collected and concentrated *in vacuo*. All monomeric galactose and glucose were removed from the glycans using this method. Some dimeric materials (e.g. maltose, lactose) could be removed using this method although some dimers were resistant to fermentation (e.g. melibiose, allo-lactose). FIGS. 7A-7B show a maltose-derived glycan before and after a yeast fermentation.

Bread yeast (Fleischmann's Yeast, Fenton, MO) was selected for purifying glycans made from maltose, sucrose, palatinose, cellobiose, melezitose, and melibiose. *Kluyveromyces marxianus* (ATCC 46537) was selected for purifying glycans made from raffinose, gentiobiose, lactose, and lactulose. *Kluyveromyces marxianus* beads were made in an analogous fashion, except that the yeast suspension was made by mixing 10 g freshly cultured yeast with 2.5 ml water.

## Example 19. Single Strain Growth Assays to assess Effects of low DP glycans

An *in vitro* assay was performed to assess the ability of various bacterial strains, including commensals and pathogens of the gastrointestinal tract, to utilize enzymatically synthesized glycans (i.e., glycan polymers) and the sugars from which they were derived as growth substrates. The assay was designed to determine if enzymatically synthesized glycans differ from the sugars from which they are derived in how well they support growth of potentially beneficial or harmful bacterial members of the human microbiome. Bacterial strains were handled at all steps in an anaerobic chamber (AS-580, Anaerobe Systems) featuring a palladium catalyst. The chamber was initially made anaerobic by purging with an anaerobic gas mixture of 5% hydrogen, 5% carbon dioxide and 90% nitrogen and subsequently maintained in an anaerobic state using this same anaerobic gas mixture. Anaerobicity of the chamber was confirmed daily using Oxoid anaerobic indicator strips that change color in the presence of oxygen. All culture media, assay plates, other reagents and plastic consumables were pre-reduced in the anaerobic chamber for at least 24-48 hours prior to contact with bacteria. Enzymatically synthesized glycans lacto-oligo, melib-oligo, malto-oligo, sucro-oligo, raffino-oligo and lactulo-oligo and the sugars from which they were derived, lactose, melibiose, maltose, sucrose, raffinose and lactulose were filter-sterilized, prepared at 5% w/v in sterile water and added to Costar 3370 assay plates for a final concentration of 0.5% w/v in the assay, with each glycan assayed in three non-adjacent wells and dextrose and water supplied as positive and negative controls.

Bacterial isolates were obtained from the American Type Culture Collection (ATCC) and Leibniz Institute DSMZ-German Institute of Microorganisms and Cell Cultures (DSMZ). Cultures of the Bacteroidetes *Bacteroides thetaiotaomicron* ATCC 29741 "BTH.8" and *Parabacteroides distasonis* ATCC 8503 "PDI.6"; the Clostridiales *Clostridium scindens* ATCC 35704 "CSC.32", *Dorea formicigenerans* ATCC 27755 "DF0.36", *Dorea longicatena* DSM 13814 "DLO.76" and *Blautia hansenii* ATCC 27752 "BHA.20" were grown anaerobically in Chopped Meat Glucose broth (CMG, Anaerobe Systems), a pre-reduced enriched medium including lean ground beef, enzymatic digest of casein, yeast extract, potassium phosphate, dextrose, cysteine, hemin and Vitamin K1, for 18-48 hours at 37° C. Inocula were prepared by determining the optical density of each culture at 600 nM ($OD_{600}$) in a Costar 3370 polystyrene 96-well flat-bottom assay plate using a Biotek Synergy 2 plate reader with Gen5 2.0 All-In-One Microplate Reader Software according to manufacturer's protocol, and diluting the cells to $OD_{600}$ 0.01 final in defined and semi-defined media that were prepared without sugars. *D. formicigenerans, P. distasonis, B. hansenii* and *D. longicatena* isolates were tested in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114), supplemented with 0-10% (v/v) CMG. *B. thetaiotaom-*

*icron* was tested in 100 mM potassium phosphate buffer (pH 7.2), 15 mM sodium chloride, 8.5 mM ammonium sulfate, 4 mM L-cysteine, 1.9 $\mu$M hematin, 200 $\mu$M L-histidine, 100 $\mu$M magnesium chloride, 1.4 $\mu$M iron sulfate heptahydrate, 50 $\mu$M calcium chloride, 1 $\mu$g/mL vitamin K3 and 5 ng/mL vitamin B12 (Martens EC et al. Cell Host & Microbe 2008; 4, 447-457). *C. scindens* was tested in 10 g/L tryptone peptone, 5 g/L yeast extract, 0.5 g/L L-cysteine hydrochloride, 0.1 M potassium phosphate buffer pH 7.2, 1 $\mu$g/mL vitamin K3, 0.08% w/v calcium chloride, 0.4 $\mu$g/mL iron sulfate heptahydrate, 1 $\mu$g/mL resazurin, 1.2 $\mu$g/mL hematin, 0.2 mM histidine, 0.05% Tween 80, 0.5% meat extract (Sigma), 1% trace mineral supplement (ATCC), 1% vitamin supplement (ATCC), 0.017% v/v acetic acid, 0.001% v/v isovaleric acid, 0.2% v/v propionic acid and 0.2% v/v N-butyric acid (Romano KA et al. mBio 2015; 6(2):e02481-14). Bacteria were exposed to enzymatically synthesized glycans lacto-oligo, melib-oligo, malto-oligo, sucro-oligo, raffino-oligo and lactulo-oligo and the sugars lactose, melibiose, maltose, sucrose, raffinose, lactulose and dextrose at a final concentration of 0.5% w/v in 96-well microplates, 200 $\mu$L final volume per well, at 37° C for 18-48 hours, anaerobically. $OD_{600}$ measurements for each isolate at the end of the incubation period were obtained using a Biotek Synergy2 reader with Gen5 2.0 software according to manufacturer's specifications. Measurements for each strain were blanked by subtracting the average OD600 value of the strain in the absence of glycan or sugar. Table 8 and the following three paragraphs summarize the results obtained.

**Table 8. Growth of gut commensals on sugars and enzymatically synthesized glycans**

| Sugar/Glycan | DFO.36 | CSC.32 | PDI.6 | BTH.8 | DLO.76 | BHA.20 | Key to Growth | |
|---|---|---|---|---|---|---|---|---|
| raffinose | - | - | +++ | - | - | ++ | Symbol | $OD_{600}$ |
| raffino-oligo | ++ | + | +++ | +++ | + | +++ | - | <0.1 |
| lactulose | - | - | ++ | + | - | +++ | + | 0.1-0.3 |
| lactulo-oligo | + | ++ | +++ | +++ | + | + | ++ | 0.3-0.7 |
| sucrose | - | - | ++ | +++ | +++ | - | +++ | >0.7 |
| sucro-oligo | + | - | +++ | ++ | ++ | ++ | | |

Some enzymatically-synthesized glycans support different levels of growth of gut commensal bacteria in the assay than the sugars from which they are derived. As seen in Table 8, raffino-oligo supported higher growth than raffinose of the Firmicutes *D. formicigenerans, C. scindens, D. longicatena* and *B. hansenii* and the Bacteroidete *B. thetaiotaomicron* in the assay. Lactulo-oligo supported higher growth than lactulose of *D. formicigenerans, C. scindens, P. distasonis, B. thetaiotaomicron* and *D. longicatena* in the assay, while *B. hansenii* grew less well on lactulo-oligo than lactulose in the assay. In the assay, sucro-oligo supported higher growth of *D. formicigenerans, P. distasonis* and *B. hansenii* than sucrose and less growth of *B. thetaiotaomicron* and *D. longicatena* than sucrose. The enzymatically synthesized glycans also differ in how much growth they support across bacterial strains in the assay. In the assay, raffino-oligo supported high levels of growth of *P. distasonis, B. thetaiotaomicron* and *B. hansenii* with $OD_{600}$ values greater than 0.7, moderate levels of growth of *D. formicigenerans* with $OD_{600}$ values between 0.3 and 0.7, and a lower level of growth of *C. scindens and D. longicatena* with $OD_{600}$ values between 0.1 and 0.3.

In the assay, some enzymatically synthesized glycans also support different levels of growth of enteric pathogens than the sugars from which they are derived. Sucro-oligo supported less than half the growth of *S. enterica, E. faecium* and *C. difficile* than sucrose in the assay, while raffino-oligo supported more growth of these pathogens than raffinose. Lactulo-oligo and lactulose supported similar growth levels of these pathogens in the assay.

Some enzymatically synthesized glycans support levels of bacterial growth in the assay similar to the sugars from which they are derived. In the assay, the bacterial commensals *D. formicigenerans, D. longicatena, B. hansenii, C. scindens, P. distasonis* and *B. thetaiotaomicron* grew to similar levels, with optical densities differing by 20% or less, on lacto-oligo and lactose, melib-oligo and melibiose, and malto-oligo and maltose. The enteric pathogens *S. enterica, E. faecium* and *C. difficile* also grew to roughly similar levels in the assay on melib-oligo and melibiose, malto-oligo and maltose, and lacto-oligo and lactose, with the exception of *C. difficile* on lacto-oligo and lactose, for which there was a 30% difference in growth.

The observed differences between the activity of enzymatically synthesized glycans and the sugars from which they are derived in the assay with regard to the bacterial strains that they support and the level of growth that they support for various bacterial strains suggest that an enzymatically synthesized glycan may be administered to differentially support one or more beneficial microbes *in vivo* for therapeutic benefit. For example, *C. scindens* has been associated with protection against *C. difficile-associated* diarrhea (Buffie et al, Nature 2015). Administration of an enzymatically synthesized glycan that selectively promotes growth of *C. scindens* may encourage growth of *C. scindens in vivo* and thereby be beneficial in prophylaxis or treatment of *C. difficile-associated* diarrhea.

**Example 20. Glycosidase profiles and sugar utilization of selected metabolite producers**

Linking metabolic functions encoded by gut bacteria with carbohydrate utilization genes has the potential of *in silica* screening of glycan polymer preparations for specific metabolite-linked human diseases. However, many metabolic functions have been proven to be polyphyletic and therefore, 16S rRNA gene sequencing is not a suitable analysis method (Vital et al., 2014, Martinez del Campo et al., 2015). In order to overcome this challenge, genomic and metagenomic analyses can be utilized to directly assay for genes involved in metabolite synthesis and linking these genes to charbohydrate utilization through sequenced genomes.

The Carbohydrate-Active enZYmes Database (CAZy; http://www.cazy.org/) describes families of enzymes with catalytic or carbohydrate-binding modules for enzymes that degrade, modify, or create glycosidic bonds. Glycoside hydrolases (GH) and glycosyltransferases (GT) are enzymes that are important for the utilization of carbohydrates in the gut microbiome. CAZy families are widely distributed phylogenetically (Kaoutari et al., 2013).

A total of 439 sequenced genomes isolated from the gastrointestinal tract as part of the Human Microbiome Project were utilized in this analysis (http://hmpdacc.org/reference_ genomes/reference_genomes.php). All genomes were annotated using the Carbohydrate Active Enzyme Database (CAZy; http://www.cazy.org/) using USEARCH v8 and the -usearch_global option, requiring 90% global identity to the target protein. In addition, all genomes were annotated for their predicted involvement in 1) butyrate production, 2) conversion of choline to TMA through TMA-lyase, and 3) urease conversion of urea to ammonia. For butyrate production, annotation was focused on the acetyl-CoA pathway, the most abundant pathway in encoded in the gut microbiome (Vital et al., 2014). In order to predict butyrate production, the terminal genes in the pathway (*but* and *buk*) were used, requiring 90% global identity to a target protein identified in Vital et al.'s genome analysis. In order to predict TMA-lyase potential, BLASTP to the *cutC* gene described in Martinez del Campo et al., requiring >60% identity over 40% of both the target and query proteins. Following annotation of both metabolite production and CAZy content, enrichment of carbohydrate utilization genes in metabolite producers (butyrate) or non-metabolite producers (TMA) was performed using Wilcox test with FDR correction. For urease, indole, p-cresol, propionate, and secondary bile acid conversion, all HMP genomes were annotated using the KEGG database, with greater than 70% global identity using the -usearch_global option. For urease production, the EC number 3.5.1.5 was used as a marker for the urease enzyme to identify urease-positive genomes. For indole production, the EC number 4.1.99.1 was used as a marker for the tryptophanase enzyme to indentify indole-positive genomes. For p-cresol production, the EC numbers 4.1.1.83, 2.6.1.-, 4.1.1.-, 1.2.7.5 were used to identify the pathway from tyrosine to p-cresol through 4-phenylhydroxyacetate. For propionate, the EC numbers 6.4.1.3, 2.1.3.1, 4.1.1.41, 1.2.1.27, 2.3.3.5, 1.2.1.87, 1.3.1.95, 1.3.8.7, 2.3.1.54, 2.3.1.168, 2.3.1.8, 2.3.1.222 were used to identify all pathways for propionate production. For secondary bile acid conversion, the following EC numbers were used to identify bile-acid converters: 1.1.1.159, 1.1.1.176, 1.1.1.201, .1.1.238, 1.1.1.391, 1.1.392, 1.1.393, 1.1.395, 1.1.1.52, 2.8.3.25, 4.2.1.106, 6.2.1.7.

A total of 439 sequenced genomes across an array of commensal bacterial species isolated from healthy human gut microbiomes as a part of the Human Microbiome Project were predicted for their ability to produced butyrate, convert urea to ammonia through urease, and convert choline to TMA (FIG. 15). Each of these three important bacterial functions are widely yet discontinuously distributed across phylogeny, demonstrating importance of a functional approach to characterizing the metabolic potential of a microbiome. For example, while butyrate production is predicted to be universal in *Faecalibacterium* and *Roseburia* species, *Eubacterium* and *Lacnospiraceae* species appear to be more variable in their ability to produce butyrate. In addition, the production of these three metabolic functions seems to be overall non-overlapping between species, presenting the potential for selective production of beneficial and simultaneous suppression of toxic metabolites.

A total of seven CAZy families and subfamilies were identified to be differentially abundant between predicted butyrate and non-butyrate producers (FIGS. 16A-16B; P<0.001, Wilcox Rank Sum with FDR correction). Of these families, two are almost completely absent from non-butyrate producers (GH13.36 and GH113), which are known to target glucose and mannose containing glycans, respectively (FIGS. 16A-16B; Table 9 below). Overall, enzymes that are enriched in butyrate producers target glycans composing glucose, mannose, and galactose, with glucose being the most common target composition (Table 9). This finding is supported experimentally, using both an *ex vivo* fecal community and defined *in vitro* community grown in various glycan compositions and assayed for SCFA production. To identify monomer compositions and interactions that most contribute to increased SCFA production, including butyrate, a LASSO linear regression model was used (glmnet in R with minimum lambda from cross validation for defined communities and maximum lambda within one standard error of minimum lambda for *ex vivo*) with percentage of monomer composition as input, allowing all second order interaction terms. All positive model coefficients are shown in FIGS. 22A-22B. Here, glucose is an important predictor for production of all SCFA, including butyrate, for defined communities (FIG. 22B), and the combination of glucose, galactose, and mannose increase butyrate production in *ex vivo* fecal communities, while glucose alone is predicted to increase acetate production (FIG. 22A). Further supporting using monomer target composition as predictive of metabolic potential, using a LASSO linear regression model with percent monomer composition as input to predict growth of single bacterial strains, we show that monomer composition is highly predictive of bacterial growth.

While these finding support the glycan target genes that are most differential between butyrate and non-butyrate producers, these are non-exhaustive of the most abundant glycosyl hydrolases encoded in butyrate producers (FIG. 17).

**Table 9. Glycan target composition for CAZy Families Enriched in Butyrate Producers over Non-Butyrate Producers.**

| CAZy Family | Glycan Target Composition (substrate) | CAZy Family | Glycan Target Composition (substrate) |
|---|---|---|---|
| GT5 | Glucose, alpha-1,3, alpha- 1,4 | GH13.36 | Glucose, alpha-glycosidic |
| GH94 | Beta-glucose, beta-glycosidic | GH113.0 | Mannose, beta-1,4 |
| GH13.9 | Glucose (Maltose), alpha-glycosidic | GH112 | Beta-Galactose, beta-1,3 |
| GH13.39 | Alpha-glucan debranching, alpha-glycosidic | | |

This method can be further applied to target glycans for metabolites that are harmful and desirable to decrease, including TMA and ammonia. For example, 11 CAZy families and subfamilies were identified that are exclusive and overrepresented in taxa that do not encode the *cutC* gene (FIGS. 18A-18B; P<0.05, Wilcox Rank Sum, FDR corrected), which has been shown to be responsible for converting choline to TMA in the gut (Martinez del Campo, et al). Further, at least one of these families is encoded in almost 50% of bacterial species which do not also encode the *cutC* gene. These families target a wider array of glycan compositions (FIGS. 18A-18B, Table 10), however, galactose and glucose are the most abundant target monomer compositions. These CAZy families and subfamilies are the most differentially abundant between *cutC* positive and negative genomes, however, another set of genes can be found to be the most abundant in *cutC* negative genomes and are also desirable targets for TMA reduction (FIG. 19).

**Table 10. Glycan target composition for CAZy Families in genomes lacking the TMA-lvase enzyme.**

| CAZy Family | Glycan Target Composition | CAZy Family | Glycan Target Composition |
|---|---|---|---|
| GT11.0 | Galactose/Fucose, alpha-1,2, alpha- 1,3 | GH29.0 | Fucose, alpha-glycosidic |
| GT10.0 | Galactose, alpha- 1,3 | GH28.0 | Galactose, alpha-1,4 |
| GH92.0 | Mannose, alpha-1,2, alpha- 1,3, alpha-1,4, alpha- 1,6 | GH130.0 | Mannose, beta- 1,2, beta-1,4 |
| GH51.0 | Glucose/Xylose/Arabinose, beta-1,4 | G H 13.8 | Glucose, alpha-glycosidic |
| GH35.0 | Galactose beta-1,3, beta- 1,4, beta-1,6 | GH13.14 | Glucose, alpha-glycosidic |

Extending this application to the production of ammonia cleaving of urea by the urease enzyme, 7 CAZy families and subfamilies were identified that are differentially encoded between urease positive and negative genomes (FIGS. 20A-20B; P<0.05, Wilcox Rank Sum, FDR Corrected).

Further, these enzymes are exclusive to urease negative genomes and are exclusive targets of glucose and galactose containing glycans (FIGS. 20A-20B, Table 11). These CAZy families and subfamilies are the most differentially abundant between urease positive and negative genomes, however, another set of genes can be found to be the most abundant in urease negative genomes and are therefore also desirable targets for ammonia reduction (FIG. 21).

**Table 11. Glycan target composition for CAZy Families in genomes lacking the urease enzyme.**

| urease production: | | | |
|---|---|---|---|
| CAZy Family | Glycan Target Composition | CAZy Family | Glycan Target Composition |
| GT3.0 | glucose | GH133.0 | glucose |
| GH97.0 | glucose, galactose, alpha-1,2, alpha- 1,3, alpha-1,4, alpha- 1,6 | GH13.8 | Glucose, alpha-glycosidic |

(continued)

| urease production: | | | |
|---|---|---|---|
| **CAZy Family** | **Glycan Target Composition** | **CAZy Family** | **Glycan Target Composition** |
| GH43.24 | Galactose, arabinose, xylose, alpha-1,3 | GH13.0 | Glucose, alpha-glycosidic |
| GH27.0 | Galactose, glucose | | |

Extending this application to propionate production, 7 CAZy families and subfamilies were identified that are differentially encoded between propionate producers and non-propionate producers (FIGS. 30G, P<0.01, Wilcox Rank Sum, FDR Corrected). These enzymes are enriched in non-propionate producers and are targets of N-acetylglucosamine, xylose, arabinose, glucose, galactose, and fucose (FIG. 30G, Table 25). These CAZy families and subfamilies are the most differentially abundance between non-propionate and propionate producers, however, another set of genes can be found to be the most abundance in non-propionate producers and therefore are also desirable targets for propionate reduction (FIG. 30H).

**Table 25. Glycan target composition for CAZy Families in genomes lacking propionate production pathways.**

| **CAZy Family** | **Glycan Target Composition** |
|---|---|
| GH84.0 | N-acetylglucosamine |
| GH43.8 | xylose, arabinose, galactose, alpha-1,3 |
| GH43.27 | xylose, arabinose, galactose, alpha-1,3 |
| GH43.22 | xylose, arabinose, galactose, alpha-1,3 |
| GH30.5 | xylose |
| GH13.3 | Glucose, alpha-glycosidic |
| GH121.0 | Arabinose, beta-glycosidic |

Extending this application to bile acid conversion, 6 CAZy families and subfamilies were identified that are differentially encoded between secondary bile acid converters and nonsecondary bile acid converters (FIG. 30A, P<0.05, Wilcox Rank Sum, FDR Corrected). These enzymes are enriched in secondary bile acid coverters and are exclusively targets of trehalose, sialic acid, and glucose (FIG. 30A, Table 26). These CAZy families and subfamilies are the most differentially abundance between secondary bile acid converters and non-secondary bile acid converters, however, another set of genes can be found to be the most abundance in nonpropionate producers and therefore are also desirable targets for secondary bile acid conversion (FIG. 30B).

**Table 26. Glycan target composition for CAZy Families in genomes with secondary bile acid synthesis pathways.**

| **CAZy Family** | **Glycan Target Composition** |
|---|---|
| GH37.0 | Glucose, alpha-glycosidic |
| GH33.0 | sialic acid, glucose, alpha-glycosidic |
| GH23.0 | |
| GH13.21 | Glucose, alpha-glycosidic |
| GH13.19 | Glucose, alpha-glycosidic |
| GH104.0 | |

Extending this application to indole production, 7 CAZy families and subfamilies were identified that are exclusively encoded in non-indole producing bacteria (FIG. 30C). These enzymes are exclusively encoded in non-indole producing bacteria and are enriched for targets of glucose, xylose, arabinose, and mannose (FIG. 30C, Table 27). These CAZy families and subfamilies are exclusively encoded in non-indole producing bacteria, however, another set of genes can be found to be the most abundance in non-indole producing bacteria and therefore are also desirable targets for indole reduction (FIG. 30D).

**Table 27. Glycan target composition for CAZy Families in genomes lacking an encoded tryptophanase for indole production.**

| CAZy Family | Glycan Target Composition |
|---|---|
| GH94.0 | Glucose, beta-glycosidic |
| GH5.44 | glucose, xylose, mannose, beta-glycosidic |
| GH43.11 | xylose, arabinose, galactose, alpha-1,3 |
| GH39.0 | xylose, beta-glycosidic |
| GH13.39 | Glucose, alpha-glycosidic |
| GH13.31 | Glucose, alpha-glycosidic |
| GH13.20 | Glucose, alpha-glycosidic |

Extending this application to p-cresol production, 7 CAZy families and subfamilies were identified that are differentially encoded between p-cresol producers and non-p-cresol producers (FIG. 30E, P<0.01, Wilcox Rank Sum, FDR Corrected). These enzymes are enriched in non-p-cresol producing bacteria and enriched for targets of xylose, arabinose, galactose, and glucose (FIG. 30E, Table 28). These CAZy families and subfamilies are exclusively encoded in non-indole producing bacteria, however, another set of genes can be found to be the most abundance in non-p-cresol producing bacteria and therefore are also desirable targets for p-cresol reduction (FIG. 30F).

**Table 28. Glycan target composition for CAZy Families in genomes lacking an encoded pathway for p-cresol production.**

| CAZy Family | Glycan Target Composition |
|---|---|
| GH43.8 | xylose, arabinose, galactose, alpha-1,3 |
| GH43.27 | xylose, arabinose, galactose, alpha-1,3 |
| GH15.0 | Glucose, alpha-1,2, alpha- 1,3, alpha-1,4, alpha- 1,6 |
| GH13.30 | Glucose, alpha-glycosidic |
| GH13.3 | Glucose, alpha-glycosidic |
| GH121.0 | Arabinose, beta-glycosidic |
| GH110.0 | Galactose, alpha-1,3 |

For all strains which grow well on synthesized glycans, monomer composition is strongly predictive of strain growth on a particular glycan as described in Table 12.

**Table 12. $R^2$ calculated using LASSO linear regression model on monomer composition.**

| Strain | $R^2$ | Maximum $OD_{600}$ | Strain | $R^2$ | Maximum $OD_{600}$ |
|---|---|---|---|---|---|
| PDI.6 | 0.96 | 0.3 | BHA.20 | 0.76 | 0.07 |
| PCO.72 | 0.9 | 0.2 | BTH.8 | 0.73 | 0.17 |
| BCE.85 | 0.85 | 0.14 | CSC.32 | 0.71 | 0.05 |
| BPR.22 | 0.85 | 0.19 | CDI.23 | 0.68 | 0.13 |
| BCE.71 | 0.83 | 0.12 | CNE.31 | 0.67 | 0.1 |
| BLO.16 | 0.83 | 0.21 | BLO.83 | 0.67 | 0.1 |
| AMU.73 | 0.77 | 0.04 | BVU.10 | 0.64 | 0.07 |

**Example 21. Targeted enrichment of a microorganism using an exemplary glycan polymer preparation.**

If a microbial species of interest is present in a microbial community, an increased representation of that species may be observed upon contacting the microbial community with certain glycan polymer preparations. For example, species of the genus *Bacteroides* are known to possess the largest number of carbohydrate active enzymes (CAZymes). Among those species, the genome sequence of *Bacteroides cellulolyticus* comprises the largest number of genes encoding enzymes dedicated to carbohydrate degradation of all known sequenced genomes in the genus: 503 total CAZymes that include 373 GHs, 28 carbohydrate esterases and 84 glycosyl transferases (McNulty et al, PLOS Biology, doi:10.1371/journal.pbio.1001637). To show that *Bacteroides cellulolyticus* could be selectively increased in its representation in response to administration of an exemplary glycan polymer preparation, a complex bacterial community comprising 15 bacterial strains was assembled and tested.

The composition of this defined community was: 1) Phylum Actinobacteria: 2 strains of *Bifidobacterium longum;* 2) Phylum Firmicutes: *Blautia hansenii, Clostridium nexile, Clostridium scindens, Dorea formicigenerans, Dorea longicatena, Ruminococcus obeum; 3)* Phylum Bacteroidetes: *Bacteroides caccae, Bacteroides cellulolyticus, Bacteroides thetaiotaomicron, Bacteroides vulgatus, Prevotella copri, Parabacteroides distasonis;* 4) Phylum Verrucomicrobia: *Akkermansia muciniphila.*

Each strain was grown separately in standard chopped meat glucose medium for 18 hours. Each culture was diluted to a final optical density (OD600) of 0.01, and combined into the final mixed community. This community was grown for 48 hours on a medium supplied with the following carbohydrates as sole carbon source: glucose, FOS, Glu100 (also referred to as "Glu"), Glu50Gal50 (also referred to as "GluGal"), Gal100 (also referred to as "Gal"), and Man52Glu29Gal19 (also referred to as "ManGluGal"). Water was added to a medium without any added carbon source as a control.

The resulting community, as well as individual strains, was added to a 96-well plate (flat bottom Costar, 300uL) containing growth medium without a glycan, or with each of the glycans listed above. Final concentration of each glycan in the assay was 5%. Each glycan was represented 3 times within each growth plate and was the sole carbon source for bacteria. Plates were incubated at 37 °C in anaerobic chamber AS-580 for a total of 48 hours.

At 18 and 48 hours, optical density was determined for each community incubated with a glycan, and 150uL of culture was aliquoted and immediately frozen at -80C for sequencing of 16S rRNA gene to determine changes in community composition as a function of time and glycan.

To determine representation of each strain in this defined community, genomic DNA was extracted from each community and 16S rRNA gene amplified and sequenced. As shown in FIGS. 23A-23B, the relative abundance of *Bacteroides cellulolyticus* increased from an average of 4% to 30% when a defined community composed of 15 strains was grown on Glu100 glycan, but not other carbon sources.

Moreover, when a community has been grown in the presence of a carbon source, other than Glu100, as shown in FIG. 23A, and then Glu100 was added to an established community, relative abundance of *Bacteroides cellulolyticus* increased from an average of 4% to 14%, as shown in FIG. 23B (Gal100, Glu50Gal50, Man52Glu29Gal19). Importantly, when *B.cellulolyticus* was added to a community during a stationary phase, such as communities grown in the presence of glucose or FOS, no change in *B.cellulolyticus* was observed, implying that other growth limiting nutrients have been likely consumed, such as nitrogen or phosphorus sources (FIGS. 23A-23B). The data suggest feasibility of targeted enrichment of specific taxa in a bacterial community using the glycan polymers described herein.

**Example 22. Demonstration of a synbiotic administration to an established *in vitro* defined community.**

In certain disease states a native microbiota can be perturbed by intake of antibiotics, or other agents damaging to the microbiota. Administration of probiotic species is a common attempt to restore native microbiota. However, most FDA approved probiotics are lactic acid bacteria that are present at very low abundance in the gut microbiota of adults. Moreover, administration of these species does not result in restoration of the commensal microbiota, such as members of the genus *Bacteroides.* In addition, engraftment of a probiotic species into an established complex community is often difficult. Therefore, simultanous administration of a probiotic along with a carbon source that is selectively consumed by that probiotic species may be required to guarantee a successful engraftment of this species into a resident community. Here, successful engraftment of *Bacteroides cellulolyticus* into an established community is demonstrated, wherein the community significantly improved by simultaneous administration of an exemplary glycan polymer preparation (Glu100 glycan).

A defined community composed of 14 strains (all of the 15 strains listed in Example 21 except *Bacteroides cellulolyticus),* was grown in the presence of 6 glycans or water as a sole carbon source for 18 hours. At 18th hour, a culture of *Bacteroides cellulolyticus* at OD=0.1 was added to each community. To test if engraftment of *Bacteroides cellulolyticus* was dependent on its preferred carbon source (Glu100), 20uL of 5% Glu100 or *Bacteroides cellulolyticus* and 20uL of 5% Glu100 were added to each established community, as shown in the **Table 13.**

**Table 13. Experimental design.**

| Experimental hour | 0 hours | | | 18 hours | | | | | |
|---|---|---|---|---|---|---|---|---|---|
| Number of strains in a community | 15 | **14** | | 15 | 15 | **14** | **14** | **14** | **14** |
| B.cellulolyticus at 0 hours | Present | Absent | | Present | Present | Absent | Absent | Absent | Absent |
| B.cellulolyticus added at 18 hours | | | | | | | | **+ B.cellulolyticus** | **+ B.cellulolyticus** |
| Glu added at 18 hours | | | | | **+ Glu** | | **+ Glu** | | **+ Glu** |
| Carbon source in a media at the beginning at 0 hours | FOS<br>Gal<br>Glu<br>GluGal<br>Glucose<br>ManGluGal<br>Water | | | | + Glu<br>+ Glu<br>+ Glu<br>+ Glu<br>+ Glu<br>+ Glu<br>+ Glu | | + Glu<br>+ Glu<br>+ Glu<br>+ Glu<br>+ Glu<br>+ Glu<br>+ Glu | + B.cellulolyticus<br>+ B.cellulolyticus<br>+ B.cellulolyticus<br>+ B.cellulolyticus<br>+ B.cellulolyticus<br>+ B.cellulolyticus<br>+ B.cellulolyticus | + B.cellulolyticus   + Glu<br>+ B.cellulolyticus   + Glu<br>+ B.cellulolyticus   + Glu<br>+ B.cellulolyticus   + Glu<br>+ B.cellulolyticus   + Glu<br>+ B.cellulolyticus   + Glu<br>+ B.cellulolyticus   + Glu |

At 48 hours, optical density was determined for each community, and 150uL of culture was aliquoted and immediately frozen at -80C for sequencing of 16S rRNA gene to determine changes in community composition as a function of time and glycan.

To determine representation of each strain in this defined community, genomic DNA was extracted from 150uL of cultures using MoBio Microbiome DNA/RNA extraction kit (catalogue number 27500-4-EP). V4 region of 16S rRNA gene was amplified using 515Forward and 806 Reverse primers as described in Caporaso JG et al, ISME J. Amplicons were sequenced using Illumina MiSeq instrument with 250bp long reads using paired end chemistry. Operational Taxonomic Units (OTUs) were picked using 97% sequence identity. Fifteen OTUs were obtained that matched 16 strains. Two strains of *Bifidobacterium longum* could not be resolved by 16S rRNA gene, thus 1 OTU was representing both strains.

As shown in FIG. 24A, relative abundance of *Bacteroides cellulolyticus* was 8% in a community grown on Glu100 or water and only 2% for communities grown on other glycans.

However, when Glu100 was administered simultaneously with *Bacteroides cellulolyticus*, the relative abundance of this strain increased from 2% to 10% on average if a community had been growing in the presence of a glycan polymer preparation, or 25% if a community has been growing in the absence of glycan polymer preparation (FIG. 24B). Importantly, if *B.cellulolyticus* was added to a community during a stationary phase, such as communities grown in the presence of glucose or FOS, no change in B.cellulolyticus was observed, implying that other growth limiting nutrients have been likely consumed, such as nitrogen or phosphorus sources (FIGS. 24A-24B).

**Example 23: Ex vivo assay to assess ability of glycan polymer preparations to modulate bacterial growth and metabolite production**

An ex vivo assay was performed to assess the ability of a human fecal community, in an in vitro setting, to utilize different glycans. The ex vivo assay was designed to determine if glycans can be used to differentially modulate a complex bacterial community and short chain fatty acids, including those associated with protective effects. Fecal samples and slurries were handled in an anaerobic chamber (AS-580, Anaerobe Systems) featuring a palladium catalyst. Glycans glu33gal33man33, gal33man33ara33, glu50gal50, glu33gal33ara33, gal100, glu40man60, gal33man33xyl33, glu40gal60, glu20gal80, glu60gal40, glu80ara20, gal40man60, man80xyl20, gal60ara40, glu100, gal80man20, gal40ara60, gal80ara20, gal60man40, glu80gal20, xyl60ara40, glu80xyl20, glu60man40, glu20xyl80, glu20man80, man60ara40, glu80man20, glu60xyl40, gal20man80, gal60xyl40, gal80xyl20, glu40ara60, glu60ara40, xyl20ara80, man52glu29gal19, gal40xyl60, glu40xyl60, man60xyl40, xyl100, glu20ara80, gal20ara80, gal20xyl80, man20ara80, man100, xyl40ara60, ara100, xyl80ara20 and a commercially available control, FOS (Nutraflora FOS; NOW Foods, Bloomingdale IL), were prepared at 5% w/v in water, filter-sterilized and added to 96-well deep well microplates assay plates for a final concentration of 0.5% w/v in the assay, with water supplied as positive and negative controls.

A human fecal sample donation was stored at -80°C. To prepare working stocks the fecal sample was transferred into the anaerobic chamber and allowed to thaw. The fecal sample was prepared to 20% w/v in phosphate buffered saline (PBS) pH 7.4 (P0261, Teknova Inc., Hollister, CA), 15% glycerol and stored at -80°C. The 20% w/v fecal slurry + 15% glycerol was centrifuged at 2,000xg, supernatant was removed, and the pellet was suspended in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114) to 1% w/v fecal slurry. Prepared 1% w/v fecal slurry was exposed to glycans glu33gal33man33, gal33man33ara33, glu50gal50, glu33gal33ara33, gal100, glu40man60, gal33man33xyl33, glu40gal60, glu20gal80, glu60gal40, glu80ara20, gal40man60, man80xy120, gal60ara40, glu100, gal80man20, gal40ara60, gal80ara20, gal60man40, glu80gal20, xyl60ara40, glu80xyl20, glu60man40, glu20xyl80, glu20man80, man60ara40, glu80man20, glu60xyl40, gal20man80, gal60xyl40, gal80xyl20, glu40ara60, glu60ara40, xyl20ara80, man52glu29gal19, gal40xyl60, glu40xyl60, man60xyl40, xyl100, glu20ara80, gal20ara80, gal20xyl80, man20ara80, man100, xyl40ara60, ara100, xyl80ara20 and commercial FOS at a final concentration of 0.5% w/v in 96-well deep well microplates, 500 μL final volume per well, at 37° C for 45 hours, anaerobically. Following incubation, assay samples were split, with a portion used for DNA extraction and sequencing and the other used for short chain fatty acid analysis.

Genomic DNA was extracted from the fecal slurries treated with glycans and controls, and variable region 4 of the 16S rRNA gene was amplified and sequenced (Earth Microbiome Project protocol www.earthmicrobiome.org/emp-standard-protocols/16s/ and Caporaso JG et al. 2012. Ultra-high-throughput microbial community analysis on the Illumina HiSeq and MiSeq platforms. ISME J.). Operational Taxonomic Units (OTUs) were generated by aligning 16S rRNA sequences at 97% identity.

Fecal slurries treated with glycans and controls were centrifuged at 4000xg for 10 minutes at 4° C, and the supernatants were transferred to fresh tubes. The samples were kept frozen at -80°C until analysis. The samples were removed from the freezer and thawed. 1mL of the culture supernatant was transferred to a glass vial. The pH of the suspension was adjusted to 2-3 by adding 100 uL of 50% sulfuric acid. The acidified samples were kept at room temperature and vortexed for 10 minutes. For the volatile extraction 50 uL of the internal standard (1% 2-methyl pentanoic acid solution) and 1000 uL of ethyl ether anhydrous were added. The tubes were mixed end over end for 10 minutes and then centrifuged at 2000g for 2 minutes. 1uL of the upper ether layer was injected into the chromatogram for analysis.

Chromatographic analysis of SCFA concentration was carried out using an Agilent 7890B system with a flame ionization detector (FID) (Agilent Technologies, Santa Clara, CA). A high resolution gas chromatography capillary column 30m x 0.25 mm coated with 0.25um film thickness was used (DB-FFAP) for the volatile acids (Agilent Technologies). Nitrogen was used as the carrier gas. The oven temperature was 145°C and the FID and injection port was set to 225 °C. The injected sample volume was 1 uL and the run time for each analysis was 12 minutes. Chromatograms and data integration was carried out using the OpenLab ChemStation software (Agilent Technologies). Results are summarized in Tables 14 and 15.

The relative abundances of OTUs of fecal slurry samples treated with glycans were compared to the no-added glycan group using the non-parametric Wilcoxon rank sum test for difference in microbiome compositions at the species level. In order to explore associations between the species composition and butyrate, acetate and propionate levels across different glycans, we first normalized the proportion of species present in the glycan treated group to those obtained from the no-added carbon(control) group. The fold change values, thus obtained for each glycan treatments, were then correlated to the butyrate levels obtained from the glycans using Spearman's rank correlation method [Myles Hollander & Douglas A. Wolfe (1973), Nonparametric Statistical Methods. New York: John Wiley & Sons. Pages 185-194 (Kendall and Spearman tests).]. Associations between species and butyrate levels found with statistical significance (benjamini hochberg corrected p-value < 0.05) [Benjamini, Y., and Hochberg, Y. (1995) Journal of the Royal Statistical Society Series B 57, 289-300.] were identified, and plots representing the strongest associations are shown in FIGS. 25A-25D, 26A-26F, and 27A-27D.

**Table 14. Glycans differentially modulate production of linear short chain fatty acids in the *ex vivo* assay relative to the average glycan-free control**

| | Fold Change Relative to Control | | | Key to Table | |
| --- | --- | --- | --- | --- | --- |
| Glycan | Butyrate | Acetate | Propionate | Symbol | Fold Change |
| glu33gal33man33 | +++ | ++ | +++ | +++ | >10 |
| gal33man33ara33 | ++ | + | ++ | ++ | 5-10 |
| glu50gal50 | ++ | ++ | ++ | + | 3-5 |
| glu33gal33ara33 | ++ | + | ++ | - | <3 |
| FOS | ++ | ++ | - | +++ | >10 |
| gal100 | ++ | - | - | | |
| glu40man60 | ++ | + | ++ | | |
| gal33man33xyl33 | ++ | + | +++ | | |
| glu40gal60 | ++ | ++ | ++ | | |
| glu20gal80 | ++ | + | + | | |
| glu60gal40 | ++ | ++ | + | | |
| glu80ara20 | ++ | ++ | ++ | | |
| gal40man60 | ++ | + | +++ | | |
| man80xyl20 | ++ | + | +++ | | |
| gal60ara40 | ++ | - | - | | |
| glu100 | ++ | ++ | ++ | | |
| gal80man20 | ++ | + | ++ | | |
| gal40ara60 | + | - | - | | |
| gal80ara20 | + | - | - | | |

(continued)

| Glycan | Fold Change Relative to Control | | | Key to Table | |
|---|---|---|---|---|---|
| | Butyrate | Acetate | Propionate | Symbol | Fold Change |
| gal60man40 | + | + | ++ | | |
| glu80gal20 | + | ++ | ++ | | |
| xyl60ara40 | + | - | - | | |
| glu80xyl20 | + | ++ | ++ | | |
| glu60man40 | + | + | ++ | | |
| glu20xyl80 | + | + | + | | |
| glu20man80 | + | + | ++ | | |
| man60ara40 | + | - | ++ | | |
| glu80man20 | + | + | + | | |
| glu60xyl40 | + | + | ++ | | |
| gal20man80 | + | + | ++ | | |
| gal60xyl40 | + | - | - | | |
| gal80xyl20 | + | - | - | | |
| glu40ara60 | + | + | + | | |
| glu60ara40 | + | + | + | | |
| xyl20ara80 | + | - | - | | |
| man52glu29gal19 | + | + | ++ | | |
| gal40xyl60 | + | - | - | | |
| glu40xyl60 | + | + | + | | |
| man60xyl40 | + | - | ++ | | |
| xyl100 | + | - | - | | |
| glu20ara80 | - | - | - | | |
| gal20ara80 | - | - | - | | |
| gal20xyl80 | - | - | - | | |
| man20ara80 | - | - | - | | |
| man 100 | - | + | ++ | | |
| xyl40ara60 | - | - | - | | |
| ara100 | - | - | - | | |
| xyl80ara20 | - | - | - | | |

As shown in Table 14, 48 synthetic glycans differentially modulate production of the nonbranched short chain fatty acids butyrate, acetate and propionate relative to the average no added glycan control in the assay. In the assay, the largest fold-increases were observed with butyrate and propionate, as 1 glycan increased butyrate at least 10-fold, 4 glycans increased propionate at least 10-fold and none of the glycans increased acetate at least 10-fold. In the assay, 3-10 fold shifts relative to the no glycan control were observed in butyrate with 38 glycans, in acetate with 28 glycans, and in propionate with 27 glycans. In the assay, less than 3-fold shifts relative to the no glycan control were observed in butyrate with 9 glycans, in acetate with 20 glycans, and in propionate with 17 glycans.

Butyrate was modulated in the assay to varying degrees depending on the glycan composition. Butyrate increased at least 10-fold in the assay relative to the average no added glycan control with a trimer where 3 of the inputs were one-third each mannose, galactose and glucose. Butyrate increased at least 5-fold in the assay with glycans containing glucose and galactose where glucose constituted less than 80% of the monomer input, and with glycans composed of three monomers where two of the inputs were at least one-third each mannose and galactose. Butyrate increased less than 5-fold in the assay with two out of four glycans composed of arabinose and xylose, and with four out of five glycans where the input was at least 80% arabinose.

Acetate was modulated in the assay to varying degrees depending on the glycan composition. Aside from FOS, glycans that increased acetate at least 5-fold in the assay included glucose as a monomer input. Aside from man 100 and gal100, glycans that increased acetate less than 3-fold in the assay included arabinose and/or xylose as a monomer input. Thus monomer composition appears to influence acetate production in the assay, with glucose input favoring acetate production, and arabinose and xylose inputs trending to relatively lower acetate.

Propionate was modulated in the assay to varying degrees depending on glycan composition. Three out of four glycans that increased propionate at least 10-fold in the assay included both galactose and mannose as inputs, and two out of four included both xylose and mannose as inputs. Propionate increased at least 5-fold in the assay with 14 out of 16 glycans that included mannose as a monomer input. In the assay, 16 out of 18 glycans with which propionate shifted less than 3-fold included xylose and/or arabinose as a monomer input. Thus monomer composition appears to influence propionate production in the assay, with mannose input favoring propionate production, and arabinose and xylose inputs trending to relatively lower propionate.

**Table 15. Glycans differentially modulate production of branched short chain fatty acids in the *ex vivo* assay relative to the average glycan-free control**

| Glycan | Fold Change Relative to Control | | | Key to Table | |
|---|---|---|---|---|---|
|  | Isobutyrate | Isovalerate | Isocaproate | Symbol | Fold Change |
| ga160man40 | + | - | - | + | ≥ 2-fold increase |
| glu20xyl80 | + | - | + | - | < 2-fold change |
| g1u40man60 | + | - | - | - | ≥ 2-fold decrease |
| glu33ga133man33 | + | + | ND | | |
| glu20man80 | + | - | - | | |
| glu80gal20 | - | - | ND | | |
| glu60man40 | - | - | - | | |
| man80xyl20 | - | - | ND | | |
| glu80man20 | - | - | ND | | |
| xyl60ara40 | - | - | - | | |
| gal40man60 | - | - | ND | | |
| gal20xy180 | - | - | - | | |
| xyl100 | - | - | + | | |
| man60ara40 | - | - | - | | |
| glu60gal40 | - | - | ND | | |
| glu100 | - | - | ND | | |
| xyl80ara20 | - | - | - | | |
| glu60xyl40 | - | - | - | | |
| glu80xyl20 | - | - | - | | |
| man60xyl40 | - | - | - | | |
| gal20ara80 | - | - | ND | | |
| glu80ara20 | - | - | - | | |

(continued)

| Glycan | Fold Change Relative to Control | | | Key to Table | |
| --- | --- | --- | --- | --- | --- |
| | Isobutyrate | Isovalerate | Isocaproate | Symbol | Fold Change |
| ara100 | - | - | - | | |
| xyl40ara60 | - | - | + | | |
| gal40ara60 | - | - | - | | |
| glu40ara60 | - | - | - | | |
| gal33man33xyl33 | - | - | - | | |
| glu40xyl60 | - | - | - | | |
| gal80man20 | - | -- | ND | | |
| xyl80ara20 | - | - | - | | |
| gal60xyl40 | - | - | - | | |
| glu20ara80 | - | - | - | | |
| man52glu29gal19 | - | - | ND | | |
| man100 | - | - | - | | |
| glu40gal60 | - | - | ND | | |
| ga120man80 | - | - | - | | |
| gal40xyl60 | - | - | - | | |
| man20ara80 | - | - | - | | |
| gal60ara40 | - | - | - | | |
| glu60ara40 | - | - | - | | |
| pal80xyl20 | - | - | - | | |
| ga180ara20 | - | - | - | | |
| glu50gal50 | - | - | ND | | |
| glu33gal33 ara33 | - | - | ND | | |
| gal33man33ara33 | - | - | ND | | |
| gal100 | - | - | ND | | |
| glu20gal80 | ND | - | ND | | |
| FOS | ND | ND | ND | | |

As observed in Table 15, glycans differentially modulate the branched short chain fatty acids isovalerate, isobutyrate and isocaproate in the assay relative to the average no added glycan control. In the assay, at least 2-fold increases were observed in isobutyrate with 5 glycans, in isovalerate with 1 glycan and in isocaproate with 3 glycans. In the assay, less than 2-fold shifts were observed in isobutyrate with 36 glycans, in isovalerate with 38 glycans, and in isocaproate with 25 glycans. At least 2-fold decreases were observed in the assay in isobutyrate with 6 glycans, in isovalerate with 9 glycans and in isocaproate with 4 glycans. Consistent with levels below the limit of detection, isobutyrate was not detected in the assay with 2 glycans, isovalerate was not detected with 1 glycan, and isocaproate was not detected with 16 glycans.

In the assay, modulation of branched short-chain fatty acids appears to depend on glycan composition. 4 out of 5 glycans with which isobutyrate increased at least 2-fold in the assay included mannose in combination with glucose and/or galactose. All 6 of the glycans with which isobutyrate decreased in the assay at least 2-fold included galactose. The ability of glycans to selectively increase or decrease branched short chain fatty acids suggests that they may be administered to selectively modulate short chain fatty acids *in vivo* for therapeutic benefit. Glycan modulation of short chain fatty acids in the assay is associated with shifts in a number of taxa. As indicated in FIGS. 25A-25D, 16S rRNA sequencing analysis indicates that bacteria identified as *Clostridiaceae, Turicibacter, Roseburia* and *Bacteroides fragilis* were positively correlated with butyrate in the assay. Many members of the taxa Clostridiaceae and *Roseburia* have been identified as butyrate producers based on genomic analysis reported and an increase in their relative abundance may be directly connected an increase in butyrate in the assay. The correlation of *Turicibacter* and *B. fragilis* with butyrate production may be related to indirect effects. *Turicibacter* have been reported to produce lactate (Bosshard PP et al, IJSEM 2002), and lactate is a precursor for butyrate synthesis (Bourriaud C et al, JAM 2005; Belenguer A et al, AEM 2007). *B. fragilis* produces acetate (Macfarlane S et al, PNS 2003), which has been shown to be utilized for butyrate production by bacteria including *Roseburia* (Duncan SH et al, BJN 2004). *Turicibacter* and *B. fragilis* may indirectly contribute to an increase in butyrate production by providing precursors that are transformed to butyrate by bacteria such as Clostridiaceae and *Roseburia.*

Glycan modulation of acetate in the assay was negatively correlated with some bacterial taxa and positively correlated with others. As indicated in FIGS. 26A-26F, taxa that included *Ruminococcaceae, Rickenellaceae* and *Oscillospira* were strongly negatively associated with acetate production in the assay. Taxa that were positively associated with acetate in the assay included *Bacteroides uniformis,* Clostridiaceae and *Bacteroides ovatus.* As indicated in FIGS. 27A-27D, *B. ovatus* and *B. uniformis* were also positively associated with propionate in the assay, while the taxa that were most strongly negatively associated with propionate differed from those most strongly associated with acetate and included Bifidobacterium, *Ruminococcus bromii,* and *Roseburia faecis.* Differential modulation of relative abundances of bacterial taxa by glycans in the assay thus appears to be associated with differential modulation of short chain fatty acids.

**Example 24: Ability of glycan polymer preparations to modulate bacterial growth of spore-forming bacterial taxa.**

An *in vitro* assay was performed to assess the ability of various bacterial spore-forming commensals of the gastrointestinal tract to utilize different glycans as growth substrates. This assay was designed to assess the ability of selected glycans to promote the growth of spore-forming gut commensals, which have been associated with a range of beneficial health effects. Bacterial strains were handled at all steps in an anaerobic chamber (AS-580, Anaerobe Systems) featuring a palladium catalyst. The chamber was initially made anaerobic by purging with an anaerobic gas mixture of 5% hydrogen, 5% carbon dioxide and 90% nitrogen and subsequently maintained in an anaerobic state using this same anaerobic gas mixture. Anaerobicity of the chamber was confirmed daily using Oxoid anaerobic indicator strips that change color in the presence of oxygen. All culture media, assay plates, other reagents and plastic consumables were pre-reduced in the anaerobic chamber for 24-48 hours prior to contact with bacteria. Glycans gal100, glu20gal80, glu40gal60, glu50gal50, glu60gal40, glu80gal20, glu100, glu80man20, glu60man40, glu40man60, glu20man80, man100, xyl100, xyl80ara20, xyl60ara40, xyl40ara60, xyl20ara80, ara100, glu80ara20, glu60ara40, glu40ara60, glu20ara80, glu80xyl20, glu60xyl40, glu40xyl60, glu20xyl80, gal80man20, gal60man40, gal40man60, gal20man80, gal80xyl20, gal60xyl40, gal40xyl60, gal20xyl80, gal80ara20, gal60ara40, gal40ara60, gal20ara80, man80xy120, man60xyl40, man40xyl60, man20xyl80, man80ara20, man60ara40, man40ara60, man20ara80, glu33gal33ara33, man52glu29gal19, glu33gal33man33, glu33gal33xyl33, gal33man33xyl33, gal33man33ara33 and a commercially available control, FOS (Nutraflora FOS; NOW Foods, Bloomingdale IL), were prepared at 5% w/v in water, filter-sterilized and added to Costar 3370 assay plates for a final concentration of 0.5% w/v in the assay, with each glycan assayed in two non-adjacent wells and dextrose and water supplied as positive and negative controls.

Bacterial isolates were obtained from the American Type Culture Collection (ATCC) and Leibniz Institute DSMZ-German Institute of Microorganisms and Cell Cultures (DSMZ). Cultures of *Clostridium scindens* ATCC 35704 "CSC.32", *Clostridium nexile* ATCC 27757 "CNE.31", *Blautia producta* ATCC 27340 "BPR.22", *Dorea longicatena* DSM 13814 "DLO.76", *Ruminococcus obeum* ATCC 29714 "ROB.74" and *Blautia hansenii* ATCC 27752 "BHA.20" were grown anaerobically in Chopped Meat Glucose broth (CMG, Anaerobe Systems), a pre-reduced enriched medium including lean ground beef, enzymatic digest of casein, yeast extract, potassium phosphate, dextrose, cysteine, hemin and Vitamin K1, for 18-48 hours at 37° C. Inocula were prepared by determining the optical density of each culture at 600 nM ($OD_{600}$) in a Costar 3370 polystyrene 96-well flat-bottom assay plate using a Biotek Synergy 2 plate reader with Gen5 2.0 All-In-One Microplate Reader Software according to manufacturer's protocol, and diluting the cells to $OD_{600}$ 0.01 final in defined and semi-defined media that were prepared without sugars. *B. hansenii, B. producta* and *D. longicatena* isolates were tested in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114), supplemented with 0-10% (v/v) CMG. C. *scindens, C. nexile* and *R. obeum* were tested in 10 g/L tryptone peptone, 5 g/L yeast extract, 0.5 g/L L-cysteine hydrochloride, 0.1 M potassium phosphate buffer pH 7.2, 1 μg/mL vitamin K3, 0.08% w/v calcium chloride, 0.4 μg/mL iron sulfate heptahydrate, 1 μg/mL resazurin, 1.2 μg/mL hematin, 0.2 mM histidine, 0.05% Tween 80, 0.5% meat extract (Sigma), 1% trace mineral supplement (ATCC), 1% vitamin supplement (ATCC), 0.017% v/v acetic acid, 0.001% v/v isovaleric acid, 0.2% v/v propionic acid and 0.2% v/v N-butyric acid (Romano KA et al. mBio 2015; 6(2):e02481-14). Bacteria were exposed to glycans gal100, glu20gal80, glu40gal60, glu50gal50, glu60gal40, glu80gal20, glu100, glu80man20, glu60man40, glu40man60, glu20man80, man100, xyl100, xyl80ara20, xyl60ara40, xyl40ara60, xyl20ara80, ara100, glu80ara20, glu60ara40, glu40ara60, glu20ara80, glu80xyl20, glu60xyl40, glu40xyl60, glu20xyl80, gal80man20, gal60man40, gal40man60, gal20man80, gal80xyl20, gal60xyl40, gal40xyl60, gal20xyl80, gal80ara20, gal60ara40, gal40ara60, gal20ara80, man80xyl20, man60xyl40, man40xyl60, man20xyl80, man80ara20, man60ara40, man40ara60, man20ara80, glu33gal33ara33, man52glu29gal19, glu33gal33man33, glu33gal33xyl33, gal33man33xyl33, gal33man33ara33, commercial FOS and dextrose at a final concentration of 0.5% w/v in 96-well microplates, 200 μL final volume per well, at 37° C for 18-48 hours, anaerobically. $OD_{600}$ measurements for each isolate at the end of the incubation period were obtained using a Biotek Synergy2 reader with Gen5 2.0 software according to manufacturer's specifications.

**Table 16. Glycan-supported growth of commensal spore-formers**

| Glycan | Growth of Commensal Spore-Formers | | | | | | Key to Growth | |
|---|---|---|---|---|---|---|---|---|
| | BPR.22 | DLO.76 | ROB.74 | CNE.31 | BHA.20 | CSC.32 | Symbol | $OD_{600}$ |
| glu20xyl80 | +++ | ++ | + | + | + | + | +++ | >0.15 |
| gal100 | +++ | + | + | + | + | + | ++ | 0.1-0.15 |
| glu20gal80 | +++ | + | + | + | + | + | + | 0.05-0.1 |
| xyl80ara20 | ++ | + | + | + | + | + | - | <0.05 |
| xvl60ara40 | ++ | + | + | + | + | + | | |
| glu40xyl60 | +++ | ++ | + | + | + | - | | |
| glu40gal60 | +++ | + | + | + | + | - | | |
| glu50gal50 | +++ | + | + | + | + | - | | |
| glu60gal40 | +++ | + | + | + | + | - | | |
| glu33gal33ara33 | +++ | + | + | + | + | - | | |
| xvl40ara60 | ++ | + | + | + | + | - | | |
| xyl20ara80 | ++ | + | + | + | + | - | | |
| glu80man20 | +++ | + | + | + | - | - | | |
| gal60ara40 | +++ | + | + | + | - | - | | |
| man52glu292al19 | +++ | + | + | - | - | + | | |
| glu33gal33man33 | +++ | + | + | - | + | - | | |
| xyl100 | ++ | + | + | - | + | - | | |
| glu80gal20 | +++ | + | - | - | + | - | | |
| glu100 | +++ | + | - | + | - | - | | |
| glu60man40 | +++ | + | - | + | - | - | | |
| glu60xyl40 | +++ | + | - | + | - | - | | |
| glu33gal33xyl33 | +++ | + | + | - | - | - | | |
| gal33man33ara33 | +++ | + | + | - | - | - | | |
| glu20ara80 | ++ | + | - | - | + | - | | |
| gal20ara80 | ++ | + | - | - | - | - | | |
| man40xyl60 | ++ | + | + | - | - | - | | |
| man20xv180 | ++ | + | + | - | - | - | | |
| gal33man33xyl33 | +++ | - | + | - | - | - | | |

(continued)

| | Growth of Commensal Spore-Formers | | | | | | Key to Growth | |
|---|---|---|---|---|---|---|---|---|
| Glycan | BPR.22 | DLO.76 | ROB.74 | CNE.31 | BHA.20 | CSC.32 | Symbol | OD$_{600}$ |
| glu40man60 | +++ | + | - | - | - | - | | |
| glu20man80 | +++ | + | - | - | - | - | | |
| glu80ara20 | +++ | + | - | - | - | - | | |
| glu60ara40 | +++ | + | - | - | - | - | | |
| glu80xyl20 | +++ | + | - | - | - | - | | |
| gal40man60 | +++ | + | - | - | - | - | | |
| ga180ara20 | +++ | - | - | + | - | - | | |
| man80xyl20 | +++ | + | - | - | - | - | | |
| man60xyl40 | +++ | + | - | - | - | - | | |
| man80ara20 | +++ | + | - | - | - | - | | |
| man60ara40 | ++ | + | - | - | - | - | | |
| man40ara60 | ++ | + | - | - | - | - | | |
| gal40ara60 | ++ | + | - | - | - | - | | |
| glu40ara60 | ++ | + | - | - | - | - | | |
| gal20xyl80 | ++ | + | - | - | - | - | | |
| ara100 | + | + | - | - | - | - | | |
| man20ara80 | + | + | - | - | - | - | | |
| man 100 | +++ | - | - | - | - | - | | |
| gal80man20 | +++ | - | - | - | - | - | | |
| gal60man40 | +++ | - | - | - | - | - | | |
| gal20man80 | +++ | - | - | - | - | - | | |
| gal80xyl20 | +++ | - | - | - | - | - | | |
| gal60xyl40 | ++ | - | - | - | - | - | | |
| ga140xy160 | ++ | - | - | - | - | - | | |
| FOS | +++ | +++ | +++ | +++ | +++ | + | | |

As seen in Table 16, glycans support growth of 6 spore-forming gut commensals in the assay to varying degrees. In the assay, gal100, glu20gal80, xyl80ara20, xyl60ara40, glu20xyl80 support growth of all 6 strains; glu40gal60, glu50gal50, glu60gal40, xyl40ara60, xyl20ara80, glu40xyl60 and glu33gal33ara33 support growth of 5 strains; xyl100, gal60ara40, man52glu29gal19 and glu33gal33man33 support growth of 4 strains; and glu20ara80, gal20ara80, man40xyl60, man20xyl80, glu33gal33xyl33 and gal33man33ara33 support growth of 3 strains.

Modulation of growth of spore-forming commensals in the assay varies with the composition of glycans. Oligomers with glucose and galactose inputs support at least half of the 6 strains in the assay, and the proportion of strains for which growth is supported in the assay generally increases as the proportion of galactose increases. In the assay, glu80gal20 and glu100 support 3 strains, glu50gal50 supports 5 strains, and gal100 and glu20gal80 support growth of 6 strains. Among the 4 oligomers that include glu, gal and one other monomer as inputs, 3 oligomers (glu33gal33ara33, man52glu29gal19 and glu33gal33man33) support growth of 4 strains in the assay, and 1 oligomer (glu33gal33xyl33) supports growth of 3 strains in the assay. The number of strains supported in the assay by oligomers with xylose and arabinose inputs and by oligomers with glucose and xylose inputs increases as the proportion of xylose input increases. In the assay, glu80xyl20 supports 2 strains, glu60xyl40 supports 3 strains, glu40xyl60 supports 5 strains, and glu20xyl80 supports 6 strains. In the assay, oligomers containing both xylose and arabinose inputs support growth of 5 or 6 strains, while xyl100 supports growth of 4 strains, and ara100 supports 2 strains.

The gastrointestinal tract of healthy humans contains a diverse microbial community that may include hundreds of different bacterial species. As described in U.S. publication 2016/0158294, the gut microbiota plays an important role in human health, with benefits including colonization resistance against pathogens, regulation of host immune responses, production of essential nutrients and nutrient absorption, and maintenance of gut epithelial integrity. Under dysbiotic conditions, the gut microbiome population is altered, and negative effects on the host may include altered immune responses, increased inflammation, altered metabolic responses, and increased susceptibility to pathogens. Probiotics represent only a few of the hundreds of bacterial species that are present in normal healthy human microbiota. Spore-forming commensal bacteria are abundant in the human microbiome and include species associated with various beneficial effects, including butyrate production, which is associated with gut epithelial barrier integrity and regulation of metabolic and immune responses, and protection against *Clostridium difficile* infection by *C. scindens.* Glycans may be administered to promote the growth of spore-forming commensal bacteria to provide a range of health benefits and prevent and treat diseases related to microbial dysbiosis.

**Example 25: Glycosidase profiles and sugar utilization of spore-forming bacterial taxa**

Glycoside hydrolases (GH) and glycosyltransferases (GT) that are enriched in spore-forming bacterial taxa were identified essentially as described in Example 20.

Genes of 40 glycoside hydrolase and transferase (CAZy) families and subfamilies were identified that are enriched in genomes from spore-forming bacteria when compared to non-spore forming bacteria (Fig. 28, P<0.05, Wilcox Rank Sum, FDR Corrected). Spore formers are summarized in Table: 17.

**Table 17: Genera containing spore-forming bacteria**

| Genera containing spore-forming bacteria | | | | | |
|---|---|---|---|---|---|
| Acetivibrio | Alkaliphilus | Anaerosporobacter | Anaerostipes | Bacteroides | Blautia |
| Clostridiales | Clostridium | Collinsella | Coprobacillus | Coprococcus | Dorea |
| Eubacterium | Faecalibacterium | Flavonifractor | Lachnobacterium | Lachnospira | Lachnospiraceae |
| Lutispora | Papillibacter | Pseudoflavonifractor | Ruminococcaceae | Ruminococcus | Sarcina |
| Subdoligranulum | Turicibacter | | | | |

For example, GH43, GH13, and GH28 were much more highly represented in genomes from spore-forming bacteria (FIG. 28). Over enrichment also generally corresponded to greater prevalence of detection across all of the genomes from spore-forming bacteria (Fig. 29A). Overall, the CAZyme families that were identified to be enriched in spore-forming bacteria were present in a larger portion of spore-forming bacterial genomes when compared to non-spore forming bacteria (Fig. 29 B). These results show that specific carbohydrate active enzymes are enriched in spore-forming bacteria. Table 18 shows the glycan monomers that are likely to be targeted by these enzyme families.

**Table 18. Glycan target composition for CAZy Families Enriched in Spore-Forming Bacteria over Non-spore Forming Bacteria.**

| CAZy Family | Glycan Target Composition | CAZy Family | Glycan Target Composition |
|---|---|---|---|
| GT5.0 | glucose | GH31.0 | glucose/galactose/mannose |
| GT35.0 | glucose | GH3.0 | glucose/xylose/arabinose |
| GH97.0 | glucose/galactose | GH29.0 | fructose |
| GH92.0 | mannose | GH28.0 | galactose/rhamnose |
| GH88.0 | glucose | GH27.0 | galactose/arabinose |
| GH78.0 | rhamnose | GH2.0 | galactose/mannose/ arabinose |
| GH77.0 | glucose | GH16.0 | xylose/galactose |
| GH57.0 | glucose/galactose | GH133.0 | glucose |
| GH51.0 | xylose/arabinose | GH130.0 | mannose |
| GH43.34 | xylose/arabinose/galactose | GH13.8 | glucose/maltose |
| GH43.24 | xylose/arabinose/galactose | GH13.38 | glucose/maltose |
| GH43.10 | xylose/arabinose/galactose | GH13.14 | glucose/maltose |
| GH42.0 | galactose/arabinose | GH13.0 | glucose/maltose |
| GH36.0 | galactose | GH123.0 | galactose |
| GH35.0 | galactose | GH105.0 | rhamnose |
| GH32.0 | fructose | | |

Many target glucose, xylose, galactose, and arabinose containing substrates, suggesting that glycans with these constituents could be used to promote the growth of spore-forming bacteria. Glycans comprising glucose, xylose, galactose, and/or arabinose promoted growth of spore-forming bacteria in single strain growth assays (see Example 24, Table 16).

**Example 27: Enrichment of specific taxa encoding glycosidases in fecal slurries from humans in the presence of oligosaccharides synthesized using target glycosidases**

Glycan preparations were tested for their ability to modulate the levels of target bacterial species that encode specific glycosidases in a fecal slurry from a healthy human subject *in vitro* (also referred to as an *ex vivo* assay). Fecal samples and slurries were handled in an anaerobic chamber (AS-580, Anaerobe Systems) featuring a palladium catalyst. Glycans were prepared at 5% w/v in water, filter-sterilized and added to 96-well deep well microplates assay plates for a final concentration of 0.5% w/v in the assay, with water supplied as positive and negative controls.

A human fecal sample donation was stored at -80°C. To prepare working stocks the fecal sample was transferred into the anaerobic chamber and allowed to thaw. The fecal sample was prepared to 20% w/v in phosphate buffered saline (PBS) pH 7.4 (P0261, Teknova Inc., Hollister, CA), 15% glycerol and stored at -80°C. The 20% w/v fecal slurry + 15% glycerol was centrifuged at 2,000xg, supernatant was removed, and the pellet was suspended in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114) supplemented with 750uM urea and 0.1% peptone to 1% w/v fecal slurry.

Prepared 1% w/v fecal slurry was exposed to melibiose-1, melibiose-enz19-1, melibiose-enz20-1, melibiose-enz16-1, melibiose-enz17-1, raffinose-1, raffinose-enz19-1, raffinose-enz16-1 at a final concentration of 0.5% w/v in 96-well deep well microplates, 500 $\mu$L final volume per well, at 37° C for 14 hours, anaerobically. Melibiose and raffinose denotes the substrate used to generate oligosaccharides with specific enzymes (enz19, enz20, etc.); and the number after the dash denotes a glycan preparation (e.g., -1) that has different characteristics from another glycan preparation (e.g., -3), which differ from each other within the ranges for the glycan preparations described herein.

Following incubation, genomic DNA was extracted from the fecal slurries treated with glycans and controls, and variable region 4 of the 16S rRNA gene was amplified and sequenced (Earth Microbiome Project protocol www.earthmicrobiome.org/emp-standard-protocols/16s/ and Caporaso JG et al. 2012. Ultra-high-throughput microbial community analysis on the Illumina HiSeq and MiSeq platforms. ISME J.). Raw sequences were demultiplexed, and each sample was processed separately with UNOISE2 (Edgar 2016). Briefly, paired end reads were merged and quality filtered. Unique reads were then denoised, and unfiltered merged sequences were mapped to the denoised sequences. Taxonomy was assigned to the denoised sequences using the RDP classifier (Wang 2007).

As demonstrated herein, when glycosidases used to synthesize enzymes are encoded by gut microbial species, the resulting oligosaccharides enrich for those specific species. For example, when glycosidases encoded by Lachnospiriaceae are used to generate oligosaccharides, the resulting oligosaccharides enrich Lachnospiriceae more than either the original parent substrate or oligosaccharides generated via enzymes encoded by different species (FIGS 31A-31B). This holds regardless of the substrate used to generate the oligosaccharide (FIGS 31A-31B). This demonstrates a mechanism for targeting oligosaccharides to glycosidases in the gut microbiome to specifically enrich taxa and functions of interest.

Groups of unrelated taxa may be further targeted by identifying shared glycosyl hydrolases. As demonstrated herein, when using glycosyl hydrolases that are abundant in three unique genera, Bifidoabacteria, Bacteroides, and Roseburia, all 3 taxa are simultaneously enriched in the *ex vivo* community (FIGS. 32A-32D). This demonstrates the ability to target cross-phyla species and further target functions, or glycotaxa.

EQUIVALENTS AND SCOPE

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art. Those skilled in the art will recognize or be able to ascertain using no more than routine experimentation many equivalents to the specific embodiments described herein. The scope of the present embodiments described herein is not intended to be limited to the above Description, Figures, or Examples but rather is as set forth in the appended claims. Those of ordinary skill in the art will appreciate that various changes and modifications to this description may be made without departing from the spirit or scope of the present invention, as defined in the following claims.

**Claims**

1. A glycan polymer preparation for use in a method of treating a subject having a disease or disorder associated with an unwanted level of a metabolite, wherein the metabolite is short chain fatty acid (SCFA), and wherein the disease or disorder is selected from the group consisting of acute pouchitis, allergic diseases, AIDS, atherosclerosis, asthma, atopic dermatitis, autism spectrum disorder, chronic functional constipation, celiac disease, chronic atrophic gastritis, chronic pouchitis, Clostridium difficile-associated disease (CDAD), celiac disease, pcolorectal adenoma, colorectal cancer, Crohn's disease, cystic fibrosis, depression, diabetes (Type I), diabetes (Type II), diarrhea, eczema, enterostomy, familial mediterranean fever, food hypersensitivity, graft-versus-host disease (GvHD), hepatic encephalopathy, hypertension, inflammatory bowel disease, irritable bowel disease, irritable bowel disease-constipation (IBS-C), lung cancer, microscopic colitis, multiple sclerosis, non-alcoholic fatty liver disease (NAFLD), non-alcoholic steatohepatitis (NASH), obesity-related asthma, Parkinson's disease (PD), radiation-induced acute intestinal symptoms, Shigellosis, short bowel syndrome, spinal cord injury associated bowel dysfunction, systemic inflammatory response syndrome, systemic lupus erythematosus, and. ulcerative colitis,

wherein the SCFA is optionally propionate or butyrate,
said method comprising:

selecting a glycan polymer preparation on the basis or knowledge that it modulates the production or level of the metabolite, and
administering an amount of the glycan polymer preparation effective to result in a modulation of the level of the metabolite, thereby treating the disease or disorder,
wherein the glycan polymer preparation comprises glucose and at least one alpha-glycosidic bond, optionally, wherein the alpha-glycosidic bond is alpha-1,3 glycosidic bond, alpha-1,4 glycosidic bond, or a combination thereof, and
wherein the mean degree of polymerization (DP) of the preparation is between DP3-15.

2. The glycan polymer preparation for use of claim 1, wherein the glycan polymer preparation comprises one, two, three, or all, of the following features:

i. the glycan polymer preparation further comprises glycan polymers comprising at least one beta-glycosidic bond, optionally wherein the beta-glycosidic bond is beta-1,3 glycosidic bond, beta-1,4 glycosidic bond or a combination thereof;
ii. the glycan polymer preparation further comprises glycan polymers comprising galactose (e.g., a glu-gal preparation);
iii. the glycan polymer preparation further comprises glycan polymers comprising mannose (e.g., a glu-man preparation); and
iv. the glycan polymer preparation further comprises glycan polymers comprising galactose and mannose (e.g., a glu-gal-man preparation).

3. The glycan polymer preparation for use of claims 1 or 2, wherein the glycan polymers, or at least 20, 30, 40, 50, 60, 70, 80, 90, 95, or 99 % (by weight or number) of the glycan polymers, of the glycan polymer preparation is a substrate for a glycosidase enzyme,
wherein the glycosidase enzyme is optionally present in a human gut microbe, or wherein the human gut microbe is optionally a member of glycotaxa class 2, cutC gene-negative bacterial taxa.

4. The glycan polymer preparation for use of claim 3, wherein the glycan polymer is a substrate for a glycosidase enzyme selected from one or more of, e.g., two, three, four, or more of

a) GT11, GT10, GH92, GH51, GH35, GH29, GH28, GH20, GH130, GH13 subfamily 8, or GH13 subfamily 14 CAZy family; or
b) GT2, GT4, GH2, GH23, GH3, GT8, GT51, GT9, GH1, GH92, GH73, GH31, GH20, GH28, GT25, GT28, GT35, GH18, GT0, GH13, GH36, GH97, GH105, GH25, GH4, GH32, GH78, GH29, GH0, GH51, GT10, orGH77 CKZy family.

5. The glycan polymer preparation for use of any one of claims 1 to 4, wherein the metabolite level is decreased in the subject or a suitable sample from the subject having the disease or disorder, e.g., decreased as compared to a reference, e.g., a predetermined reference value, the level in the subject prior to treatment, or a healthy control, optionally, further comprising evaluating the level of the metabolite, or a symptom of an unwanted level of the metabolite, e.g., by acquiring a level of the metabolite, optionally prior to treating the subject (e.g., as a baseline), during the treatment (e.g., to monitor treatment success), and/or post-treatment (e.g., to assess recurrence of the disease or disorder).

6. The glycan polymer preparation for use of claim 5, wherein the level (e.g., systemic level, e.g. blood or fecal levels) of SCFA is increased, e.g., relative to a subject not treated with the glycan polymer preparation.

7. The glycan polymer preparation for use of any one of claims 1-6, further comprising selecting a subject for treatment on the basis of or responsive to acquiring knowledge of any one, two, three, four or more of:

a) the subject having an unwanted level of SCFA,
b) the subject having a disease or disorder (e.g. a disease or disorder of claim 5),
c) the subject having a dysbiosis of the gut microbiota (e.g. miscalibrated levels/relative abundance of, e.g. class 2 bacterial taxa of claim 3),

d) the subject having responded to a prior treatment with a glycan polymer (e.g. a glycan polymer of any of claims 1 or 2),

e) the subject having undergone a therapy or other environment that results in a dysbiosis, e.g., antibiotic treatment, or gastric surgery prior to treating,

optionally comprising acquiring a suitable value to determine the selection criteria.

8. The glycan polymer preparation for use of claim 7, wherein a suitable value may be acquired by analyzing a suitable biological sample from the subject, wherein the sample is optionally blood, feces, urine, saliva, or an organ tissue sample.

9. The glycan polymer preparation for use of any one of claims 1-8, wherein the unwanted level of the metabolite is decreased, (e.g. in the subject or in a suitable sample taken from the treated subject) by 3%, 5%, 10%, 15%, 20%, 25%, 30%, 35%, 40%, or 50% after a treatment period (e.g. when compared to a reference, e.g., a predetermined reference value, the level in the subject prior to treatment, or a healthy control).

10. The glycan polymer preparation for use of any one of claims 1-9, wherein the treating further comprises administering a second therapeutic agent (e.g. a therapeutic agent other than the glycan polymer for treating the disease or disorder and/or for modulating the level of the metabolite), or

administering a preparation of a gut microbe (e.g., a human gut microbe),
wherein the gut microbe (e.g., a human gut microbe) is preferably a class 2 (e.g., cutC gene-negative bacterial taxa), wherein the gut microbe is optionally selected on the basis of its association with the metabolite (e.g., on the basis of its positive, negative, or lack of correlation with the metabolite), wherein the selection of the gut microbe optionally comprises choosing a gut microbe from Table 3 based on the gut microbe's association with the metabolite (e.g., on the basis of its positive, negative, or lack of correlation with the metabolite).

11. The glycan polymer preparation for use of claim 10, wherein the glycan polymer is a substrate of the gut microbe (e.g., a human gut microbe).

12. The glycan polymer preparation for use of any one of claims 1-11, wherein the glycan polymer is a substrate of a gut microbial glycosidase enzyme and promotes the growth of the gut microbe.

13. The glycan polymer preparation for use of any one of claims 1-12, wherein the glycan preparation is

a) administered daily;
b) administered for a single treatment period, or
c) administered for more than one treatment period, e.g., wherein an inter-treatment period is longer than one or both of the adjacent treatment periods or wherein an inter-treatment period is shorter than one or both of the adjacent treatment periods.

14. The glycan polymer preparation for use of any of claims 1-13, wherein the glycan polymer is a substrate for a microbial constituent of the colon or intestine, wherein the glycan polymer preparation is optionally administered orally or rectally.

**FIG. 1**

**FIG. 2**

**FIG. 3A**

**FIG. 3B**

**FIG. 3C**

EP 4 295 840 A2

FIG. 4A

FIG 4B

FIG. 4C

223

FIG. 5

FIG. 6

FIG. 7A

FIG. 7B

Extrapolated MW

FIG. 8

oligosaccharides

glucose

protein

**FIG. 9**

FIG. 10

**FIG. 11**

**FIG. 12**

FIG. 13

FIG. 14

FIG. 15

EP 4 295 840 A2

A

**FIG. 16A**

**FIG. 16B**

**FIG. 17**

**FIG. 18A**

**FIG. 18B**

**FIG. 19**

**FIG. 20A**

**FIG. 20B**

**FIG. 21**

FIG. 22A

FIG. 22B

**FIG. 23A**

**FIG. 23B**

**FIG. 24A**

**FIG. 24B**

**FIG. 25A**

**FIG. 25B**

**FIG. 25C**

**FIG. 25D**

FIG. 26A

FIG. 26B

FIG. 26C

FIG. 26D

FIG. 26E

FIG. 26F

FIG. 27A

FIG. 27B

FIG. 27C

FIG. 27D

FIG. 28

FIG. 29A

FIG. 29B

FIG. 30A

FIG. 30B

FIG. 30C

FIG. 30D

FIG. 30E

FIG. 30F

FIG. 30G

FIG. 30H

FIG. 31A

FIG. 31B

FIG. 32A

FIG. 32B

FIG. 32C

FIG. 32D

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62430895
- US 62446316
- US 62430849
- WO 2016172658 A
- WO 2016122889 A
- WO 2016172657 A
- WO 2012118767 A
- WO 2014031956 A

- WO 2016007778 A
- WO 2005003329 A
- US 20160007642 A
- US 20060257977 A
- EP 362179 A
- EP 36776 A
- US 9079171 B
- US 20160158294 A

**Non-patent literature cited in the description**

- Biotechnological Production of Oligosaccharides - Applications in the Food Industry. **MEYER.** Food technology and Industry. 2015
- **DIEZ-MUNICIO et al.** Synthesis of novel bioactive lactose-derived oligosaccharides by microbial glycoside hydrolases. *Microbial Biotecnhology,* 2014, vol. 7, 315-313
- **PETER H. SEEBERGER ; WILM-CHRISTIAN HAASE.** Solid-Phase Oligosaccharide Synthesis and Combinatorial Carbohydrate Libraries. American Chemical Society, 2000
- **THOMAS J. BOLTJE et al.** Opportunities and challenges in synthetic oligosaccharide and glycoconjugate research. *Nat Chem.,* 01 November 2009, vol. 1 (8), 611-622
- **CARLOS VERA et al.** Synthesis and Purification of Galacto-Oligosaccharides: State of the Art. *World J. Microbiol Biotechnol.,* 2016, vol. 32, 197
- **MARINA DIEZ-MUNICIO et al.** Synthesis of Novel Bioactive Lactose-Derived Oligosaccharides by Microbial Glycoside Hydrolases. *Microbial Biotechnol.,* 2014, vol. 7 (4), 315-331
- **BIRGITTE ZEUNER et al.** Methods of Improving Enzymatic Trans-Glycosylation for Synthesis of Human Milk Oligosaccharide Biomimetics. *J. Agric. Food Chem.,* 2014, vol. 62, 9615-9631
- **M.G. SAJILATA et al.** Resistant Starch - A Review. *Comprehensive Reviews in Food Science and Food Safety,* 2006, vol. 5
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25

- Molecular Cloning: A Laboratory Manual. Cold Spring Harbor Press, 2001
- **DEUTSCHER.** *Methods in Enzymology,* 1990, vol. 182
- **SCOPES.** Protein Purification: Principles and Practice. Springer-Verlag, 2010
- **GÓMEZ et al.** Purification, Characterization, and Prebiotic Properties of Pectic Oligosaccharides from Orange Peel Wastes. *J Agric Food Chem,* 2014, vol. 62, 9769
- **HOLCK et al.** Feruloylated and nonferuloylated arabino-oligosaccharides from sugar beet pectin selectively stimulate the growth of bifidobacterium spp. in human fecal in vitro fermentations. *Journal of Agricultural and Food Chemistry,* 2011, vol. 59 (12), 6511-6519
- **HAKOMORI.** *J. Biochem. (Tokyo),* 1964, vol. 55, 205
- **HARDING et al.** *Carbohydr. Res.,* 2005, vol. 340, 1107
- **GIBSON G R ; ROBERFROID M B.** Dietary modulation of the human colonic microbiota: introducing the concept of prebiotics. *J Nutr.,* June 1995, vol. 125 (6), 1401-12
- Remington's Pharmaceutical Sciences. 1985
- **BROSIUS et al.** Complete nucleotide sequence of a 16S ribosomal RNA gene from Escherichia coli. *PNAS,* 1978, vol. 75 (10), 4801-4805
- **CORDWELL.** Exploring and exploiting bacterial proteomes. *Methods in Molecular Biology,* 2004, vol. 266, 115
- **JUSTE et al.** Bacterial protein signals are associated with Crohn's disease. *Gut,* 2014, vol. 63, 1566
- **FENDT et al.** Reductive glutamine metabolism is a function of the $\alpha$-ketoglutarate to citrate ratio in cells. *Nat Commun,* 2013, vol. 4, 2236

- **FENDT et al.** Metformin decreases glucose oxidation and increases the dependency of prostate cancer cells on reductive glutamine metabolism. *Cancer Res,* 2013, vol. 73, 4429
- **METALLO et al.** Reductive glutamine metabolism by IDH1 mediates lipogenesis under hypoxia. *Nature,* 2011, vol. 481, 380
- **FERNANDEZ et al.** Correction of 13C mass isotopomer distributions for natural stable isotope abundance. *J Mass Spectrom,* 1996, vol. 31, 255
- **DUARTE et al.** H-NMR protocol for exometabolome analysis of cultured mammalian cells. *Methods Mol Biol,* 2014, 237-47
- **CHASSAING et al.** Lack of soluble fiber drives diet-induced adiposity in mice. *Am J Physiol Gastrointest Liver Physiol,* 2015
- **BAI et al.** Comparison of Storage Conditions for Human Vaginal Microbiome Studies. *PLoS ONE,* 2012, e36934
- **ORANJE, A. P. et al.** *British Journal of Dermatology,* 2007, vol. 157 (4), 645-648
- **HANIFIN et al.** *Experimental Dermatology,* 2001, vol. 10, 11
- **GIBSON G R ; ROBERFROID M B.** *J Nutr.,* June 1995, vol. 125 (6), 1401-12
- **T.E. CREIGHTON.** Proteins: Structures and Molecular Properties. W.H. Freeman and Company, 1993
- **GREEN ; SAMBROOK et al.** Molecular Cloning: A Laboratory Manual. Cold Spring Harbor Laboratory Press, 2012
- **COLOWICK ; KAPLAN.** Methods In Enzymology. Academic Press
- Remington: The Science and Practice of Pharmacy. Pharmaceutical Press, 2012
- **; SUNDBERG ; CAREY.** Advanced Organic Chemistry. Springer, 2007
- **MARTENS, E. C et al.** *Cell Host & Microbe,* 2008, vol. 4, 447
- **ROMANO, K. A. et al.** *mBio,* 2015, vol. 6, e02481-14
- **ATLAS, R. M.** Handbook of Microbiological Media. 2010
- **GOULAS et al.** *International Dairy Journal,* 2007, vol. 17, 648-656
- **SANTANDER et al.** *Microbiology,* 2013, vol. 159, 1471
- **ROSLUND et al.** *Carbohydrate Res.,* 2008, vol. 343, 101-112
- **HEISS et al.** *Carbohydr. Res.,* 2009, vol. 344, 915
- **MARTENS EC et al.** *Cell Host & Microbe,* 2008, vol. 4, 447-457
- **ROMANO KA et al.** *mBio,* 2015, vol. 6 (2), e02481-14
- **BUFFIE et al.** *Nature,* 2015
- **MCNULTY et al.** *PLOS Biology*
- **THERIOT CM et al.** *Nat Commun.,* 2014, vol. 5, 3114
- **MYLES HOLLANDER ; DOUGLAS A. WOLFE.** Nonparametric Statistical Methods. John Wiley & Sons, 1973, 185-194
- **BENJAMINI, Y. ; HOCHBERG, Y.** *Journal of the Royal Statistical Society Series B,* 1995, vol. 57, 289-300
- **BOSSHARD PP et al.** *IJSEM,* 2002
- **BOURRIAUD C et al.** *JAM,* 2005
- **BELENGUER A et al.** *AEM,* 2007
- **MACFARLANE S et al.** *PNS,* 2003
- **DUNCAN SH et al.** *BJN,* 2004